# EUROPEAN PATENT APPLICATION

(11) **EP 2 455 370 A1**
(43) Date of publication of application: **23.05.2012**
(21) Application number: 10799877.5
(22) Date of filing: 15.07.2010
(51) Int. Cl.: C07D 233/61, A61K 31/4164, A61K 31/4178, A61K 31/42, A61K 31/421, A61K 31/422, A61K 31/427, A61K 31/428, A61K 31/4439, A61K 31/454, A61K 31/4709, A61K 31/4725, A61K 31/496, A61K 31/5377, A61K 31/541, A61K 31/635, A61P 25/28, A61P 43/00, C07D 261/08, C07D 263/32

(54) **PHARMACEUTICAL PRODUCT CONTAINING LACTAM OR BENZENE SULFONAMIDE COMPOUND**

(30) Priority: 17.07.2009 JP 2009168855
(71) Applicant: Shionogi & Co., Ltd., Osaka-shi, Osaka 5410045 (JP)
(72) Inventor: KATO, Issei, Toyonaka-shi Osaka 561-0825 (JP); KANO, Kazuya, Toyonaka-shi Osaka 561-0825 (JP)
(74) Representative: Vossius & Partner
(86) International application number: PCT/JP2010/061947
(87) International publication number: WO 2011/007819

(57) **Abstract**

The present invention provides, for example, the following compounds as a pharmaceutical composition for treating diseases induced by production, secretion or deposition of amyloid β proteins.

The present invention provides a pharmaceutical composition for suppressing amyloid β production comprising a compound of the formula: 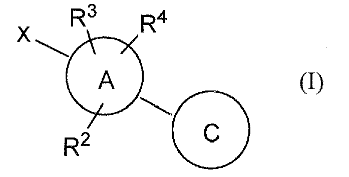 wherein A is a benzene ring, a pyridine ring or a pyrimidine ring,
X is sultam, lactam, sulfonamide or the like,
C is substituted or unsubstituted imidazolyl, substituted or unsubstituted oxazolyl or the like,
R², R³ and R⁴ are each independently hydrogen, halogen, hydroxy, carboxy, substituted or unsubstituted alkyl, substituted or unsubstituted alkoxy or the like, its pharmaceutically acceptable salt or a solvate thereof.

## Description

### [Technical Field]

The present invention relates to the compounds which have the suppressing activity of amyloid β (hereinafter referred to as Aβ) and useful for a medicament for treating the diseases induced by production, secretion, and/or deposition of Aβ.

### [Background Art]

The number of patients of Alzheimer's disease as of 2008 is said to be about 6,600,000 or more in the world. It is thought that numbers of patients continues to increase certainly with aging of society. The present treatment of Alzheimer's disease is mainly symptomatic therapy, and the treatment does not result in fundamental treatment, and development of more effective medical supplies is expected.
Aβ considered as the main morbid determination factor of this Alzheimer's disease is the metabolic product of amyloid precursor protein (APP) and some kinds of the fragments such as Aβ 40, Aβ 42 and the like are produced by the cutting position of APP. In a normal individual, Aβ 40 and Aβ 42 are detected as main Aβ and Aβ 40 is dominant. However, in patients of familial Alzheimer's disease, Aβ 42 significantly increase as compared in the normal. Moreover, in the neuritis plaque detected in the lesion area of Alzheimer's disease, Aβ 42 exists at a very high rate compared with Aβ40. It is reported that Aβ 42 accumulates rapidly and preferentially into a parenchyma plaques but Aβ 40 is unrelated to the deposition of amyloid plaque in the early stage. Aβ 42 is considered to play an major role in the deposition of amyloid plaque of familial Alzheimer's disease patient.
Thus, Aβ especially Aβ 42, closely relates to the pathogenesis and the progress of Alzheimer's disease and it is considered that compounds which suppress Aβ 42 production can be an effective prophylactic agent or therapeutic agent of Alzheimer's disease.

Some pharmaceutical agents with different mechanisms of action have been studied for the purpose of decreasing the Aβ production. B-secretase inhibitors decrease the total amount of Aβ fragment. Y-secretase inhibitors inhibit the enzymes activity of Y-secretase itself in addition to decrease the total amount of Aβ, and therefore, it is concerned about side effects. On the other hand, Y-secretase modulators do not inhibit the enzyme activity of γ-secretase itself, selectively inhibit the Aβ 42 production, and do not affect the total amount of Aβ. Then it can be become safe drugs with less side effect.
As the compounds which possess Y-secretase inhibitory activity or modulatory activity, the compounds described in the following Patent Documents 1 to 8 have been known. They have different structures with those of the compounds of the present invention.
Moreover, the compounds which are structurally similar to the compounds of the present invention are described in Patent Documents 9 to 28 and Non-patent Documents 1 to 6. These compounds have different activities from those of the compounds of the present invention.

### [Prior Art]

### [Patent Document]

[Patent Document 1] WO2009/020579 pamphlet
[patent Document 2] WO2008/097538 pamphlet
[patent Document 3] WO2009/052126 pamphlet
[patent Document 4] WO2009/075874 pamphlet
[patent Document 5] WO2009/076337 pamphlet
[Patent Document 6] WO2005/115990 pamphlet
[patent Document 7] WO2008/137102 pamphlet
[patent Document 8] WO2007/1359 pamphlet
[Patent Document 9] WO2007/088895 pamphlet
[Patent Document 10] WO2003/087071 pamphlet
[Patent Document 11] WO99/32475 pamphlet
[Patent Document 12] JP 11-504624
[Patent Document 13] WO2003/062241 pamphlet
[patent Document 14] WO2002/067939 pamphlet
[patent Document 15] WO2002/024702 pamphlet
[patent Document 16] US 2004/0077606 pamphlet
[Patent Document 17] US 2003/0114420 pamphlet
[Patent Document 18] WO2008/103636 pamphlet
[patent Document 19] WO2006/122806 pamphlet
[patent Document 20] WO2007/123853 pamphlet
[patent Document 21] WO2007/089857 pamphlet
[Patent Document 22] US 7199149 pamphlet
[patent Document 23] WO2009/133861 pamphlet
[Patent Document 24] WO2008/086188 pamphlet
[Patent Document 25] WO2007/007588 pamphlet
[Patent Document 26] WO2006/069946 pamphlet
[patent Document 27] WO2005/044192 pamphlet
[Patent Document 28] DE 1016717 pamphlet

### [Non-Patent Document]

[Non-Patent Document 1] Bioorganic&Medicinal Chemistry Letters (2007), 17 (10), 2817-2822
[Non-Patent Document 2] Bioorganic&Medicinal Chemistry Letters (2009), 19 (1), 230-233
[Non-Patent Document 3] Journal of the Indian Chemical Society (1994), 71 (11), 693-5
[Non-Patent Document 4] Bioorganic&Medicinal Chemistry Letters (2008), 18 (6), 1910-1915
[Non-Patent Document 5] Bioorganic & Medicinal Chemistry Letters (1992), 2(5), 461-6
[Non-Patent Document 6] Bulletin of the Faculty of Pharmacy (1995), 33 (Spec. Issue), 25-8

### [Summary of the invention]

### [The problems to be solved by the invention]

The present invention provides compounds which have amyloid β production suppressive activity, especially γ-secretase modulatory activity and which is useful as drugs for the diseases induced by production, secretion, or deposition of Aβ, for example, Alzheimer type dementia.

### [Means to solve the problems]

The present invention provides the following:
1) A pharmaceutical composition for suppressing amyloid β production comprising a compound of the formula (I): 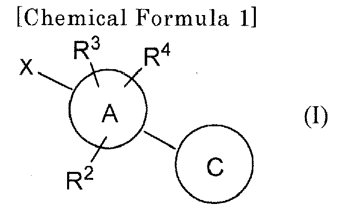 wherein X- is a group of the following formula: 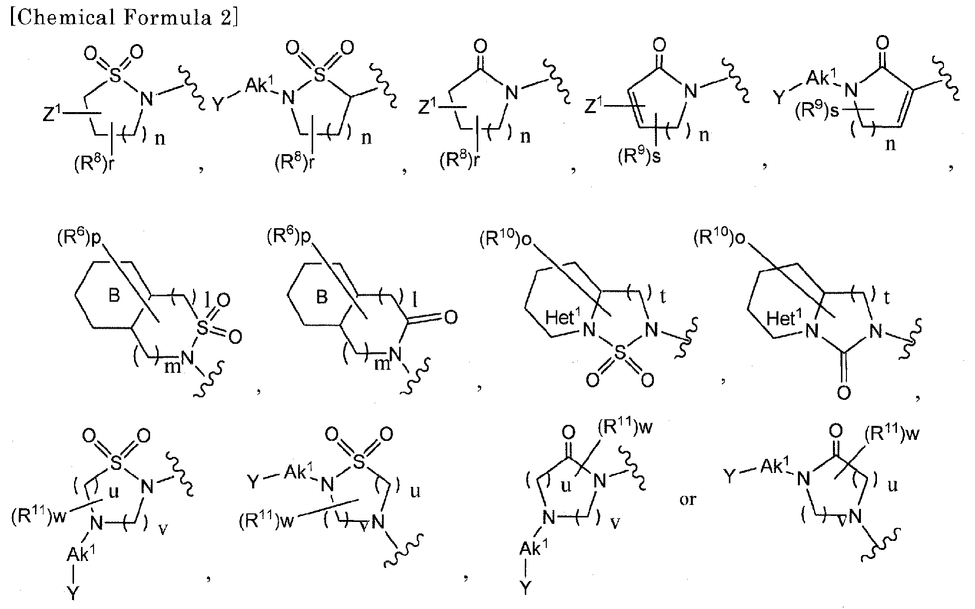 or Y-Ak¹ -SO₂ N(R⁵)-Ak² -
   wherein Y is substituted or unsubstituted phenyl which may be fused to other ring(s), substituted or unsubstituted cycloalkyl, substituted or unsubstituted cycloalkenyl, or substituted or unsubstituted pyridyl;
   provided that when X- is Y-Ak¹ -SO₂ N(R⁵)-Ak² -, then Y is substituted or unsubstituted phenyl which may be fused to other aromatic ring(s) or substituted or unsubstituted pyridyl;
   Ak¹ and Ak² are each independently a bond, or substituted or unsubstituted C1 -C3 alkylene;
   R⁵ is hydrogen, substituted or unsubstituted alkyl or substituted or unsubstituted acyl;
   Z¹ - is Y-Ak¹-D-Ak²-, Y-Ak¹ -C(=O)N(R¹²)-Ak² -, Y-Ak¹-N(R¹²)-C(=O)-Ak²-, Y-Ak¹-SO₂N(R¹²)-Ak²-, Y-Ak¹-N(R¹²)SO₂-Ak²- or Y-Ak¹-C(=O)-Ak²-;
   D is a bond, O, S or N(R¹³);
   R¹² and R¹³ are each independently hydrogen, substituted or unsubstituted alkyl or substituted or unsubstituted acyl;
   R⁶, R⁸, R⁹, R¹⁰ and R¹¹ are each independently halogen, substituted or unsubstituted alkyl, substituted or unsubstituted alkoxy, substituted or unsubstituted amino, cyano, nitro, substituted or unsubstituted phenyl, substituted or unsubstituted phenoxy, substituted or unsubstituted carbocyclyl, or substituted or unsubstituted heterocyclyl; 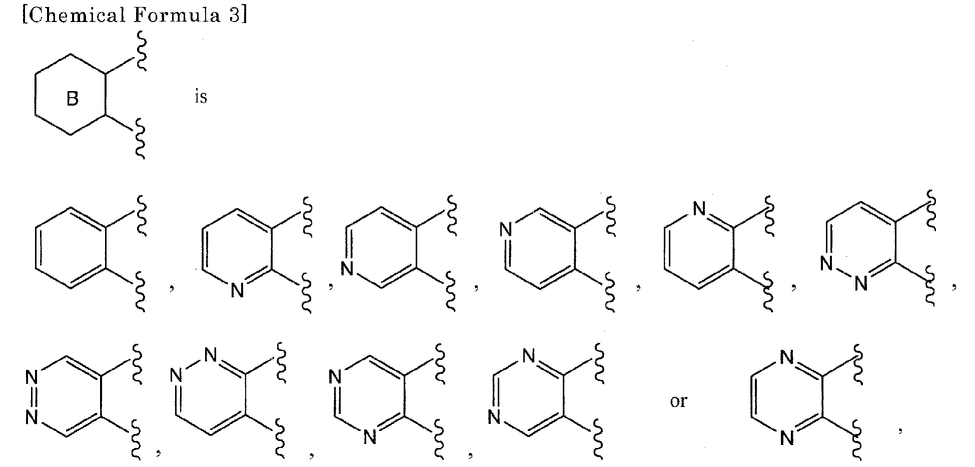 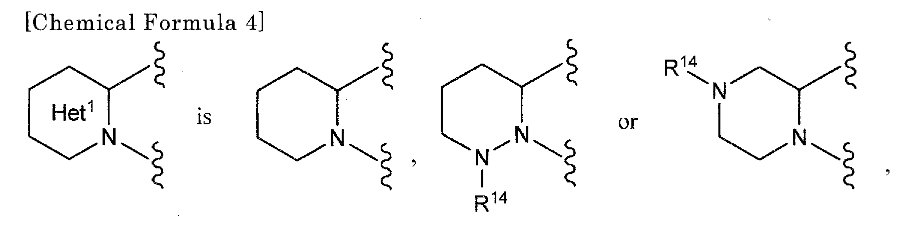
   R¹⁴ is hydrogen, substituted or unsubstituted alkyl, substituted or unsubstituted acyl, substituted or unsubstituted alkoxycarbonyl, or substituted or unsubstituted phenyl;
   n and t are each independently an integer of 0 to 5;
   u and v are each independently an integer of 1 to 5;
   m and 1 are each independently an integer of 0 to 4; and
   p, r, s, o and w are each independently an integer of 0 to 2;
   A is a benzene ring, a pyridine ring or a pyrimidine ring;
   R², R³ and R⁴ are each independently hydrogen, halogen, hydroxy, carboxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted carbocyclyloxy, substituted or unsubstituted heterocyclyloxy, substituted or unsubstituted mercapto, substituted or unsubstituted alkoxycarbonyl, substituted or unsubstituted alkenyloxycarbonyl, substituted or unsubstituted alkynyloxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted sulfamoyl, substituted or unsubstituted alkylsulfonyl, substituted or unsubstituted alkenylsulfonyl, substituted or unsubstituted alkynylsulfonyl, nitro, cyano, substituted or unsubstituted carbocyclylsulfonyl, substituted or unsubstituted heterocyclylsulfonyl, substituted or unsubstituted carbocyclyl or substituted or unsubstituted heterocyclyl; and C is substituted or unsubstituted imidazolyl, substituted or unsubstituted oxazolyl, substituted or unsubstituted isoxazolyl, substituted or unsubstituted thiazolyl or substituted or unsubstituted isothiazolyl;
   its pharmaceutically acceptable salt or a solvate thereof.
2) The pharmaceutical composition for suppressing amyloid β production of the above 1) wherein R² is a group other than hydrogen.
3) The pharmaceutical composition for suppressing amyloid β production of the above 1) or 2) wherein X- is a group of the following formula: 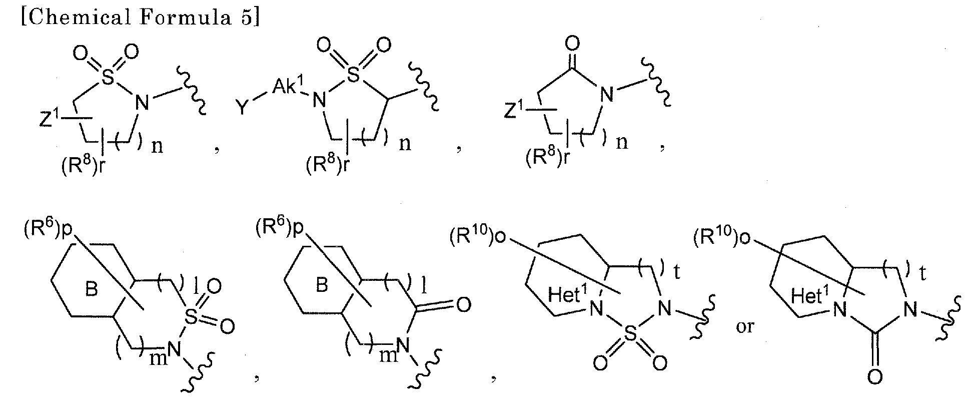 wherein Z¹, Ak¹, R⁶, R⁸, R¹⁰, B, Het¹ , l, m, n, o, p, r and t are the same as defined in the above 1).
4) The pharmaceutical composition for suppressing amyloid β production of any one of the above 1) to 3) wherein X- is a group of the following formula: 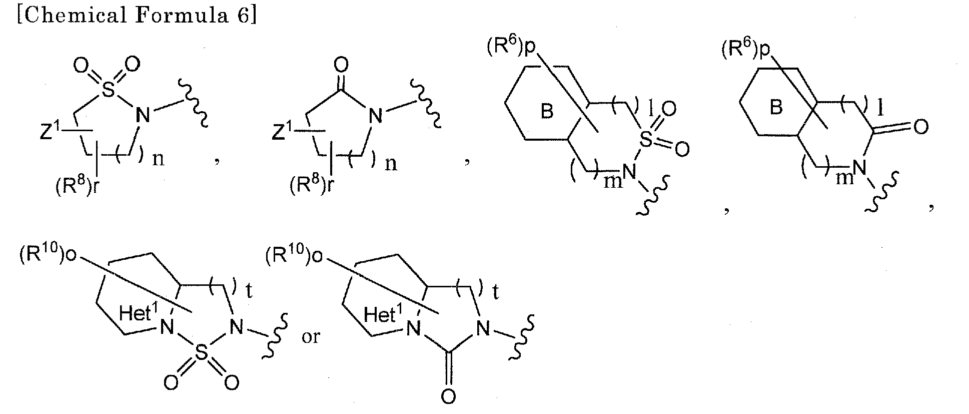 wherein Z¹, R⁶, R⁸, R¹⁰, B, Het¹ ,o, p and r are the same as defined in the above 1), n, t, and m are each independently 1 or 2 and 1 is 0.
5) The pharmaceutical composition for suppressing amyloid β production of any one of the above 1) to 4) wherein X- is a group of the following formula: 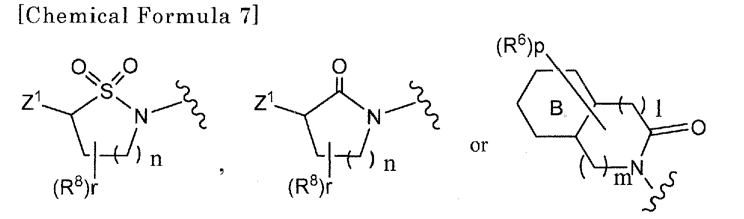 wherein Z¹ is Y-Ak¹-D-Ak²- or Y-Ak¹-C(=O)N(R¹²)-Ak²-,
   Y is substituted phenyl or substituted or unsubstituted naphthyl, Ak¹ is a bond or substituted or unsubstituted C1-C3 alkylene,
   D is a bond or N(R¹³),
   Ak² is a bond,
   R¹² and R¹³ are hydrogen,
   R⁶ and R⁸ are each independently halogen, substituted or unsubstituted alkyl, substituted or unsubstituted alkoxy, substituted or unsubstituted amino, cyano, nitro, substituted or unsubstituted phenyl, substituted or unsubstituted phenoxy, substituted or unsubstituted carbocyclyl, or substituted or unsubstituted heterocyclyl, 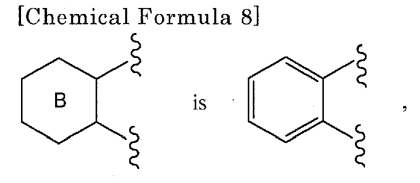 1 is 0,
   m and n are each independently 1 or 2,
   p and r are each independently an integer of 0 to 2;
   A is a benzene ring or a pyridine ring;
   R² is halogen, hydroxy, carboxy, substituted or unsubstituted alkyl, substituted or unsubstituted alkenyl, substituted or unsubstituted alkoxy, substituted or unsubstituted carbocyclyloxy, substituted or unsubstituted heterocyclyloxy, substituted or unsubstituted alkoxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted carbocyclyl or substituted or unsubstituted heterocyclyl;
   R³ and R⁴ are each independently hydrogen, halogen, hydroxy, carboxy, substituted or unsubstituted alkyl, substituted or unsubstituted alkenyl, substituted or unsubstituted alkoxy, substituted or unsubstituted carbocyclyloxy, substituted or unsubstituted heterocyclyloxy, substituted or unsubstituted alkoxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted carbocyclyl or substituted or unsubstituted heterocyclyl;
   C is substituted or unsubstituted imidazolyl, substituted or unsubstituted oxazolyl, substituted or unsubstituted isoxazolyl, substituted or unsubstituted thiazolyl or substituted or unsubstituted isothiazolyl; and
   X is in the para position relative to X on the A group.
6) The pharmaceutical composition for suppressing amyloid β production of the above 1) or 2) wherein X- is Y-Ak¹-SO₂N(R⁵)-Ak²-;
   Y is substituted phenyl or substituted or unsubstituted naphthyl;
   Ak¹ is a bond or substituted or unsubstituted methylene;
   Ak² is a bond;
   A is a benzene ring or a pyridine ring;
   R² is halogen, hydroxy, carboxy, substituted or unsubstituted alkyl, substituted or unsubstituted alkenyl, substituted or unsubstituted alkoxy, substituted or unsubstituted carbocyclyloxy, substituted or unsubstituted heterocyclyloxy, substituted or unsubstituted alkoxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted carbocyclyl or substituted or unsubstituted heterocyclyl;
   R³ and R⁴ are each independently hydrogen, halogen, hydroxy, carboxy, substituted or unsubstituted alkyl, substituted or unsubstituted alkenyl, substituted or unsubstituted alkoxy, substituted or unsubstituted carbocyclyloxy, substituted or unsubstituted heterocyclyloxy, substituted or unsubstituted alkoxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted carbocyclyl or substituted or unsubstituted heterocyclyl;
   C is substituted or unsubstituted imidazolyl, substituted or unsubstituted oxazolyl, substituted or unsubstituted isoxazolyl, substituted or unsubstituted thiazolyl or substituted or unsubstituted isothiazolyl; and
   the group of formula Y-Ak¹-SO₂N(R⁵)-Ak²- is in the para position relative to C on the A group.
7) The pharmaceutical composition for suppressing amyloid β production of any one of the above 1) to 6) wherein C is substituted or unsubstituted imidazol-1-yl.
8) The pharmaceutical composition for suppressing amyloid β production of the above 7) wherein C is imidazol-1-yl substituted with substituted or unsubstituted alkyl.
9) The pharmaceutical composition for suppressing amyloid β production of any one of the above 1) to 8) wherein both of Ak¹ and Ak² are a bond.
10) The pharmaceutical composition for suppressing amyloid β production of any one of the above 1) to 9) wherein A is a benzene ring.
11) The pharmaceutical composition for suppressing amyloid β production of any one of the above 1) to 10) wherein R² is substituted or unsubstituted alkoxy.
12) The pharmaceutical composition for suppressing amyloid β production of any one of the above 1) to 4) and 7) to 11) wherein Y is substituted or unsubstituted phenyl, substituted or unsubstituted naphthyl, substituted or unsubstituted pyridyl, substituted or unsubstituted quinolyl or substituted or unsubstituted isoquinolyl.
13) The pharmaceutical composition for suppressing amyloid β production of the above 12) wherein Y is substituted or unsubstituted phenyl, substituted or unsubstituted naphthyl, substituted or unsubstituted pyridyl, substituted or unsubstituted quinolyl or substituted or unsubstituted isoquinolyl, wherein the substituent(s) of each ring is one or more selected from the group of halogen, substituted or unsubstituted alkyl, substituted or unsubstituted alkoxy, substituted or unsubstituted amino, cyano, nitro, substituted or unsubstituted phenyl, substituted or unsubstituted phenoxy and substituted or unsubstituted heterocyclyl.
14) The pharmaceutical composition for suppressing amyloid β production of any one of the above 1) to 13) which is the pharmaceutical composition for selectively suppressing amyloid B42 production.
15) The pharmaceutical composition for suppressing amyloid β production of any one of the above 1) to 13) which is the pharmaceutical composition is for modulating Y secretase.
16) A compound of formula (II): 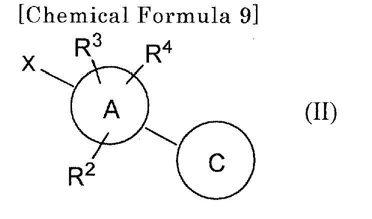 wherein X- is a group of the following formula: 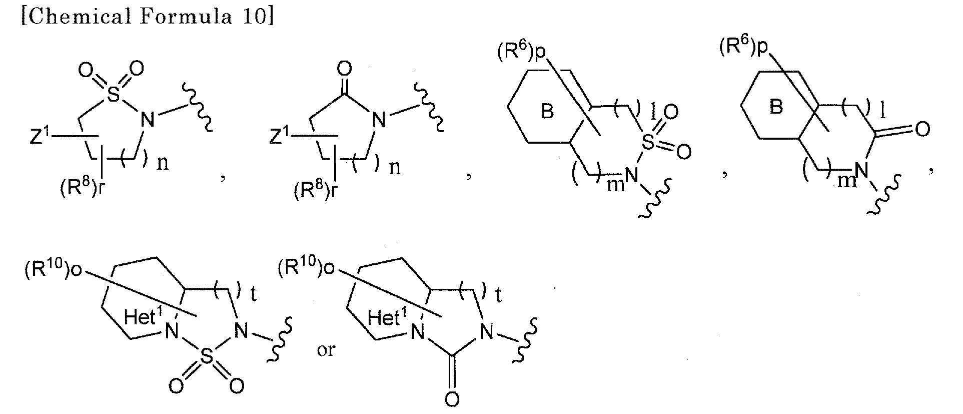
   wherein Z¹- is Y-Ak¹-D-Ak²-, Y-Ak¹-C(=O)N(R¹²)-Ak²-, Y-Ak¹-N(R¹²)-C(=O)-Ak²-, YAk¹-SO₂N(R¹²)-Ak²-, Y-Ak¹-N(R¹²)SO₂-Ak²- or Y-Ak¹-C(=O)-Ak²-,
   Y is substituted or unsubstituted phenyl which may be fused to other ring(s), substituted or unsubstituted cycloalkyl, substituted or unsubstituted cycloalkenyl, or substituted or unsubstituted pyridyl,
   Ak¹ and Ak² are each independently a bond, or substituted or unsubstituted C1-C3 alkylene,
   D is a bond, O, S or N(R¹³),
   R¹² and R¹³ are each independently hydrogen, substituted or unsubstituted alkyl or substituted or unsubstituted acyl, 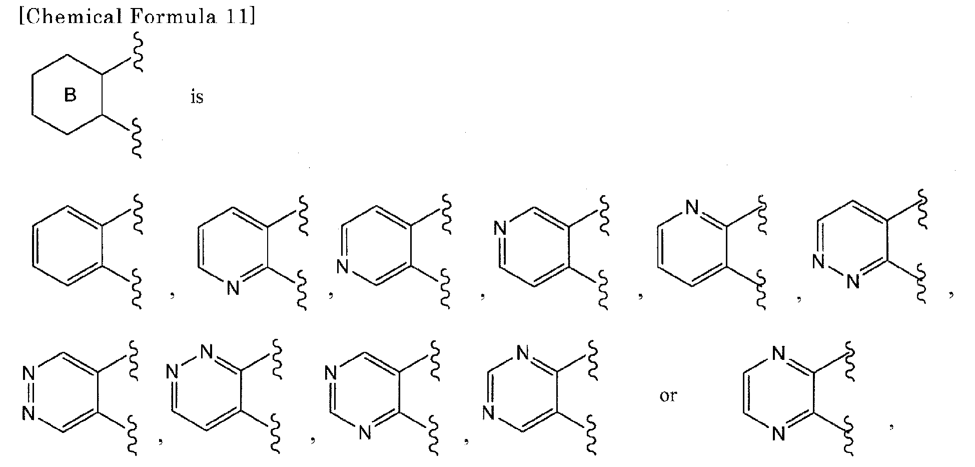 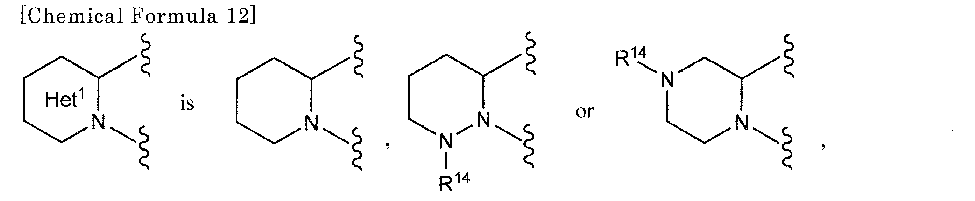
   R¹⁴ is hydrogen, substituted or unsubstituted alkyl, substituted or unsubstituted acyl, substituted or unsubstituted alkoxycarbonyl, or substituted or unsubstituted phenyl, R⁶, R⁸ and R¹⁰ are each independently halogen, substituted or unsubstituted alkyl, substituted or unsubstituted alkoxy, substituted or unsubstituted amino, cyano, nitro, substituted or unsubstituted phenyl, substituted or unsubstituted phenoxy, substituted or unsubstituted carbocyclyl, or substituted or unsubstituted heterocyclyl,
   p, r, and o are each independently an integer of 0 to 2,
   n, t, and m are each independently 1 or 2, and
   1 is 0;
   A is a benzene ring, a pyridine ring or a pyrimidine ring;
   R², R³ and R⁴ are each independently hydrogen, halogen, hydroxy, carboxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted carbocyclyloxy, substituted or unsubstituted heterocyclyloxy, substituted or unsubstituted mercapto, substituted or unsubstituted alkoxycarbonyl, substituted or unsubstituted alkenyloxycarbonyl, substituted or unsubstituted alkynyloxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted sulfamoyl, substituted or unsubstituted alkylsulfonyl, substituted or unsubstituted alkenylsulfonyl, substituted or unsubstituted alkynylsulfonyl, nitro, cyano, substituted or unsubstituted carbocyclylsulfonyl, substituted or unsubstituted heterocyclylsulfonyl, substituted or unsubstituted carbocyclyl or substituted or unsubstituted heterocyclyl; and
   C is substituted or unsubstituted imidazolyl, substituted or unsubstituted oxazolyl, substituted or unsubstituted isoxazolyl, substituted or unsubstituted thiazolyl or substituted or unsubstituted isothiazolyl;
   excluded the following compound: 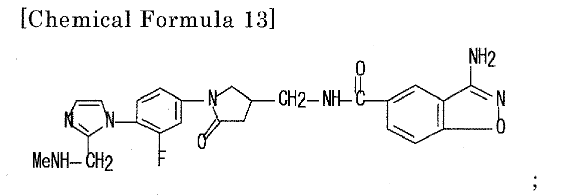 its pharmaceutically acceptable salt or a solvate thereof.
17) The compound of the above 16) wherein X- is a group of the following formula: 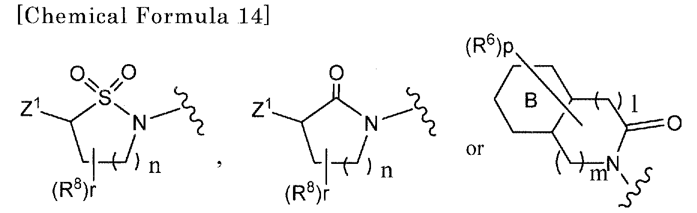 wherein Z¹- is Y-Ak¹-D-Ak²- or Y-Ak¹-C(=O)N(R¹²)-Ak²-,
   Y is substituted phenyl, or substituted or unsubstituted naphthyl,
   Ak¹ is a bond or substituted or unsubstituted C 1-C3 alkylene,
   D is a bond or N(R¹³),
   Ak² is a bond,
   R¹² and R¹³ are each independently hydrogen,
   R⁶ and R⁸ are each independently halogen, substituted or unsubstituted alkyl, substituted or unsubstituted alkoxy, substituted or unsubstituted amino, cyano, nitro, substituted or unsubstituted phenyl, substituted or unsubstituted phenoxy, substituted or unsubstituted carbocyclyl, or substituted or unsubstituted heterocyclyl, 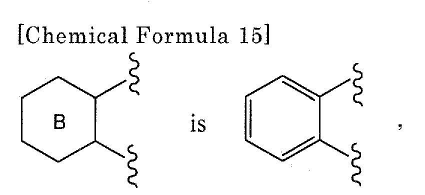 1 is 0,
   m and n are each independently 1 or 2,
   p and r are each independently an integer of 0 to 2;
   A is a benzene ring or a pyridine ring;
   R² is halogen, hydroxy, carboxy, substituted or unsubstituted alkyl, substituted or unsubstituted alkenyl, substituted or unsubstituted alkoxy, substituted or unsubstituted carbocyclyloxy, substituted or unsubstituted heterocyclyloxy, substituted or unsubstituted alkoxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted carbocyclyl or substituted or unsubstituted heterocyclyl;
   R³ and R⁴ are each independently hydrogen, halogen, hydroxy, carboxy, substituted or unsubstituted alkyl, substituted or unsubstituted alkenyl, substituted or unsubstituted alkoxy, substituted or unsubstituted carbocyclyloxy, substituted or unsubstituted heterocyclyloxy, substituted or unsubstituted alkoxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted carbocyclyl or substituted or unsubstituted heterocyclyl;
   C is substituted or unsubstituted imidazolyl, substituted or unsubstituted oxazolyl, substituted or unsubstituted isoxazolyl, substituted or unsubstituted thiazolyl or substituted or unsubstituted isothiazolyl; and
   X is in the para position relative to X on the A group;
   its pharmaceutically acceptable salt or a solvate thereof.
18) The compound of the above 16) or 17) wherein C is substituted or unsubstituted imidazolyl, its pharmaceutically acceptable salt or a solvate thereof.
19) The compound of any one of the above 16) to 18) wherein 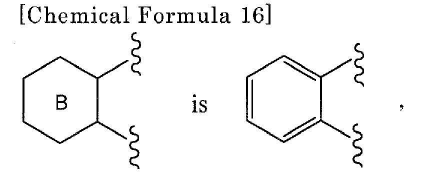 its pharmaceutically acceptable salt or a solvate thereof.
20) The compound of any one of the above 16) to 19) wherein m and n are each independently 1, its pharmaceutically acceptable salt or a solvate thereof.
21) A compound of the formula (III): 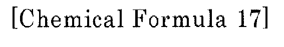 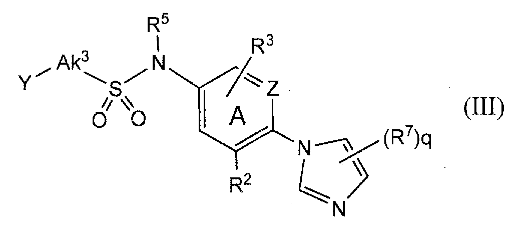 wherein Y is substituted or unsubstituted phenyl which may be fused to other aromatic ring(s), or substituted or unsubstituted pyridyl;
   Z is N or CR¹⁵;
   R² is halogen, hydroxy, carboxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted carbocyclyloxy, substituted or unsubstituted heterocyclyloxy, substituted or unsubstituted mercapto, substituted or unsubstituted alkoxycarbonyl, substituted or unsubstituted carbocyclyloxycarbonyl, substituted or unsubstituted heterocyclyloxy carbonyl, substituted or unsubstituted acyl, substituted or unsubstituted amino, substituted or unsubstituted carbamoyl, substituted or unsubstituted sulfamoyl, nitro, cyano, substituted or unsubstituted carbocyclyl or substituted or unsubstituted heterocyclyl;
   R³ and R¹⁵ are each independently hydrogen, halogen, hydroxy, carboxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted carbocyclyloxy, substituted or unsubstituted heterocyclyloxy, substituted or unsubstituted mercapto, substituted or unsubstituted alkoxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted sulfamoyl, nitro, cyano, substituted or unsubstituted carbocyclyl or substituted or unsubstituted heterocyclyl;
   R⁵ is hydrogen, substituted or unsubstituted alkyl or substituted or unsubstituted acyl; R⁷ is halogen, hydroxy, carboxy, substituted or unsubstituted alkyl, substituted or unsubstituted alkenyl, substituted or unsubstituted alkoxy, substituted or unsubstituted mercapto, substituted or unsubstituted alkoxycarbonyl, substituted or unsubstituted acyl, substituted or unsubstituted amino, substituted or unsubstituted carbamoyl or substituted or unsubstituted sulfamoyl;
   Ak³ is a bond or substituted or unsubstituted methylene; and
   q is an integer of 0 to 2;
   excluding the following compounds: 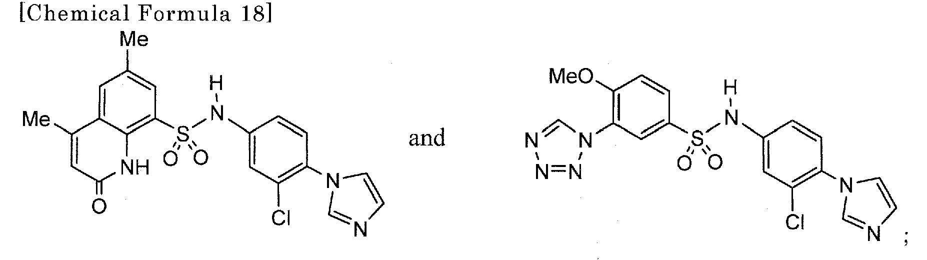 its pharmaceutically acceptable salt or a solvate thereof.
22) The compound of the above 21) wherein Z is CR¹⁵, R³ and R¹⁵ are each independently hydrogen, halogen, hydroxy, carboxy, substituted or unsubstituted alkyl, substituted or unsubstituted alkenyl, substituted or unsubstituted alkoxy, substituted or unsubstituted alkoxycarbonyl, substituted or unsubstituted acyl, substituted or unsubstituted amino or substituted or unsubstituted carbamoyl, its pharmaceutically acceptable salt or a solvate thereof.
23) The compound of the above 21) or 22) wherein Ak³ is a bond and R⁵ is hydrogen, its pharmaceutically acceptable salt or a solvate thereof.
24) The compound of any one of the above 21) to 23) wherein q is land R⁷ is substituted or unsubstituted alkyl, its pharmaceutically acceptable salt or a solvate thereof.
25) The compound of any one of the above 16) to 24) wherein A is a benzene ring, its pharmaceutically acceptable salt or a solvate thereof.
26) The compound of any one of the above 16) to 25) wherein Y is substituted or unsubstituted phenyl, substituted or unsubstituted naphthyl, substituted or unsubstituted quinolyl or substituted or unsubstituted isoquinolyl, wherein the substituent(s) of each ring is one or more selected from the group of halogen, substituted or unsubstituted alkyl, substituted or unsubstituted alkoxy, substituted or unsubstituted amino, cyano, nitro, substituted or unsubstituted phenyl, substituted or unsubstituted phenoxy and substituted or unsubstituted heterocyclyl, its pharmaceutically acceptable salt or a solvate thereof.
27) The compound of any one of the above 16) to 26) wherein R² is substituted or unsubstituted alkoxy, its pharmaceutically acceptable salt or a solvate thereof.
28) A pharmaceutical composition comprising the compound of any one of the above 16) to 27), its pharmaceutically acceptable salt or a solvate thereof.
29) A pharmaceutical composition for suppressing amyloid β production comprising the compound of any one of the above 16) to 27), its pharmaceutically acceptable salt or a solvate thereof.
30) A pharmaceutical composition for selectively suppressing amyloid β42 production comprising the compound of any one of the above 16) to 27), its pharmaceutically acceptable salt or a solvate thereof.
31) A pharmaceutical composition for modulating γ secretase comprising the compound of any one of the above 16) to 27), its pharmaceutically acceptable salt or a solvate thereof.
32) A method for suppressing amyloid β production comprising administering the compound of any one of the above 16) to 27), its pharmaceutically acceptable salt or a solvate thereof.
33) A method for selectively suppressing amyloid β42 production comprising administering the compound of any one of the above 16) to 27), its pharmaceutically acceptable salt or a solvate thereof.
34) A method for modulating γ secretase comprising administering the compound of any one of the above 16) to 27), its pharmaceutically acceptable salt or a solvate thereof.
35) A compound of any one of the above 16) to 27), its pharmaceutically acceptable salt or a solvate thereof for use in suppressing amyloid B production.
36) A compound of any one of the above 16) to 27), its pharmaceutically acceptable salt or a solvate thereof for use in selectively suppressing amyloid B42 production.
37) A compound for any one of the above 16) to 27), its pharmaceutically acceptable salt or a solvate thereof for use in modulating Y secretase.
Furthermore, the present invention provides the following:
1') A pharmaceutical composition for suppressing amyloid β production comprising a compound of the formula (I'): 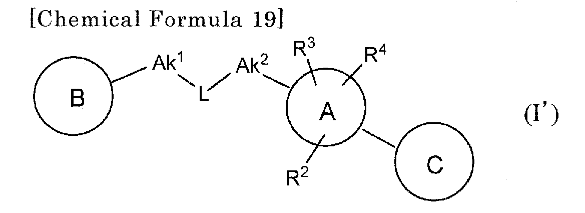 wherein A is a benzene ring, a pyridine ring or a pyrimidine ring,
   B is substituted or unsubstituted phenyl which may be fused to other aromatic ring(s) excluding unsubstituted phenyl, or substituted or unsubstituted pyridyl,
   C is substituted or unsubstituted imidazolyl, substituted or unsubstituted oxazolyl, substituted or unsubstituted isoxazolyl, substituted or unsubstituted thiazolyl or substituted or unsubstituted isothiazolyl,
   L is -SO₂N(R⁵)-, -C(=O)N(R⁵)-or -N(R⁵)-C(=O)-,
   R⁵ is hydrogen, substituted or unsubstituted alkyl or substituted or unsubstituted acyl, Ak¹ and Ak² are each independently a bond, or substituted or unsubstituted, C1-C3 alkylene which may be interposed with hetero atom(s), provided that the adjacent atom of L is a carbon atom,
   when L is -SO₂N(R⁵)-or -C(=O)N(R⁵)-, and Ak¹ is substituted or unsubstituted C1-C3 alkylene which may be interposed with hetero atom(s), then a carbon atom of Ak¹ and R⁵ taken together may form a ring,
   when L is -N(R⁵)C(=O)- and Ak² is substituted or unsubstituted C1-C3 alkylene which may be interposed with hetero atom(s), then a carbon atom of Ak² and R⁵ taken together may form a ring,
   when L is -SO₂N(R⁵)- or -C(=O)N(R⁵)-, then R⁵ and a constituent atom of B taken together may form a fused ring,
   R², R³ and R⁴ are each independently hydrogen, halogen, hydroxy, carboxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted aryloxy, substituted or unsubstituted heterocyclyloxy, substituted or unsubstituted mercapto, substituted or unsubstituted alkoxycarbonyl, substituted or unsubstituted aryloxycarbonyl, substituted or unsubstituted heterocyclyloxycarbonyl, substituted or unsubstituted acyl, substituted or unsubstituted amino, substituted or unsubstituted carbamoyl, substituted or unsubstituted sulfamoyl, nitro, cyano, substituted or unsubstituted cycloalkyl, substituted or unsubstituted cycloalkenyl, substituted or unsubstituted aryl or substituted or unsubstituted heterocyclyl,
   provided that when -Ak¹-L-Ak²- is -CH₂C(=O)NH-, then B is not phenyl substituted with CF₃,
   its pharmaceutically acceptable salt or a solvate thereof.
2') The pharmaceutical composition for suppressing amyloid β production of the above 1') wherein R² is a group other than hydrogen.
3') The pharmaceutical composition for suppressing amyloid β production of the above 1') or 2') wherein 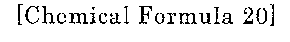 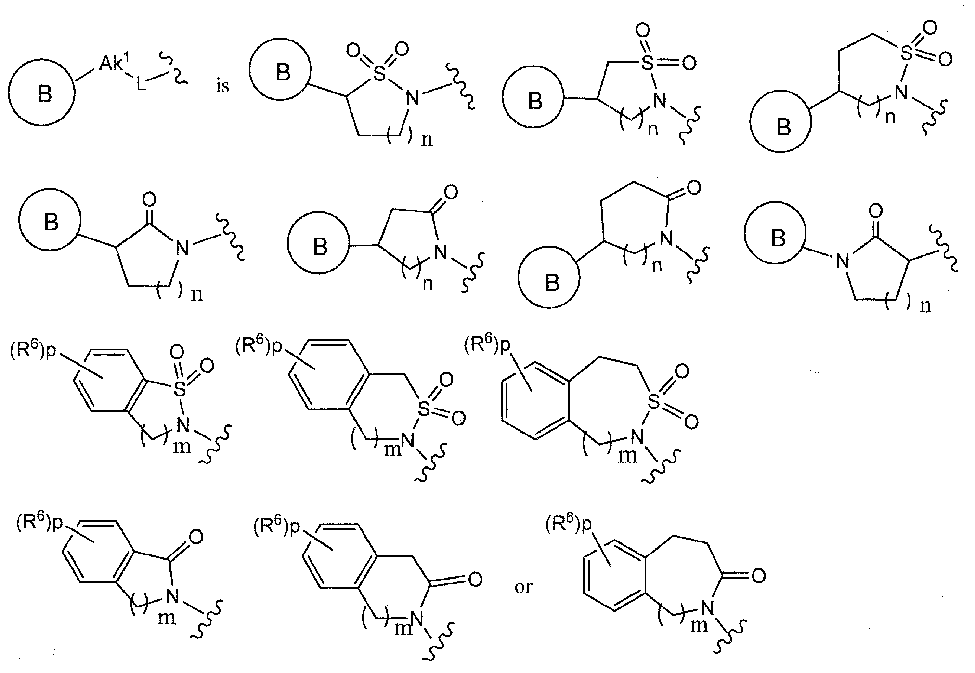 wherein R⁶ is halogen, substituted or unsubstituted alkyl, substituted or unsubstituted alkoxy, substituted or unsubstituted amino, cyano, nitro, substituted or unsubstituted phenyl, substituted or unsubstituted phenoxy or substituted or unsubstituted heteroaryl,
   p, n and m are each independently an integer of 0 to 2,
   and other symbols are the same as defined in the above 1').
4) The pharmaceutical composition for suppressing amyloid β production of the above 1') or 2') wherein A is a benzene ring or a pyridine ring,
   B is substituted phenyl or substituted or unsubstituted naphthyl,
   C is substituted or unsubstituted imidazolyl, substituted or unsubstituted oxazolyl, substituted or unsubstituted isoxazolyl, substituted or unsubstituted thiazolyl or substituted or unsubstituted isothiazolyl,
   L is -SO₂N(R⁵)-,
   R⁵ is the same as defined in the above 1'),
   L is in the para position relative to C on the A group,
   Ak¹ is a bond or substituted or unsubstituted methylene,
   Ak² is a bond,
   R² is halogen, hydroxy, carboxy, substituted or unsubstituted alkyl, substituted or unsubstituted alkenyl, substituted or unsubstituted alkoxy, substituted or unsubstituted aryloxy, substituted or unsubstituted heterocyclyloxy, substituted or unsubstituted alkoxycarbonyl, substituted or unsubstituted aryloxycarbonyl, substituted or unsubstituted heterocyclyloxycarbonyl, substituted or unsubstituted acyl, substituted or unsubstituted amino, substituted or unsubstituted carbamoyl, substituted or unsubstituted cycloalkyl, substituted or unsubstituted cycloalkenyl, substituted or unsubstituted aryl or substituted or unsubstituted heterocyclyl,
   R³ and R⁴ are each independently hydrogen, halogen, hydroxy, carboxy, substituted or unsubstituted alkyl, substituted or unsubstituted alkenyl, substituted or unsubstituted alkoxy, substituted or unsubstituted aryloxy, substituted or unsubstituted heterocyclyloxy, substituted or unsubstituted alkoxycarbonyl, substituted or unsubstituted aryloxycarbonyl, substituted or unsubstituted heterocyclyloxycarbonyl, substituted or unsubstituted acyl, substituted or unsubstituted amino, substituted or unsubstituted carbamoyl, substituted or unsubstituted cycloalkyl, substituted or unsubstituted cycloalkenyl, substituted or unsubstituted aryl or substituted or unsubstituted heterocyclyl.
5') The pharmaceutical composition for suppressing amyloid β production of any one of the above 1') to 4') wherein C is substituted or unsubstituted imidazolyl -1-yl.
6') The pharmaceutical composition for suppressing amyloid β production of the above 5') wherein C is imidazolyl -1-yl substituted with substituted or unsubstituted alkyl.
7') The pharmaceutical composition for suppressing amyloid β production of any one of the above 1'), 2') or 4') to 6') wherein L is -SO₂NH-.
8') The pharmaceutical composition for suppressing amyloid β production of any one of the above 1'), 2') and 4') to 6') wherein Ak¹ and Ak² are a bond.
9') The pharmaceutical composition for suppressing amyloid β production of any one of the above 1') to 8') wherein A is a benzene ring.
10') The pharmaceutical composition for suppressing amyloid β production of any one of the above 1') to 9') wherein R² is substituted or unsubstituted alkoxy.
11') The pharmaceutical composition for suppressing amyloid β production of any one of the above 1') to 3') and 5') to 10') wherein B is substituted or unsubstituted phenyl, substituted or unsubstituted naphthyl, substituted or unsubstituted pyridyl, substituted or unsubstituted quinolyl or substituted or unsubstituted isoquinolyl.
12') The pharmaceutical composition for suppressing amyloid β production of the above 11') wherein B is substituted or unsubstituted phenyl, substituted or unsubstituted naphthyl, substituted or unsubstituted pyridyl, substituted or unsubstituted quinolyl or substituted or unsubstituted isoquinolyl, wherein the substituent(s) of each ring is one or more selected from the group of halogen, substituted or unsubstituted alkyl, substituted or unsubstituted alkoxy, substituted or unsubstituted amino, cyano, nitro, substituted or unsubstituted phenyl, substituted or unsubstituted phenoxy and substituted or unsubstituted heteroaryl.
13') The pharmaceutical composition for suppressing amyloid β production of the above 1') wherein A is a benzene ring,
   B is substituted or unsubstituted phenyl, substituted or unsubstituted naphthyl, substituted or unsubstituted pyridyl, substituted or unsubstituted quinolyl or substituted or unsubstituted isoquinolyl,
   C is substituted or unsubstituted imidazolyl,
   L is -SO₂N(R⁵)-, -C(=O)N(R⁵)- or -N(R⁵)-C(=O)-,
   R⁵ is the same as defined in the above 1'),
   Ak¹ and Ak² are each independently a bond or substituted or unsubstituted methylene, when L is -SO₂N(R⁵)- or -C(=O)N(R⁵)- and Ak¹ is substituted or unsubstituted methylene, then a carbon atom of methylene and R⁵ are taken together may form a ring,
   when L is -N(R⁵)C(=O)- and Ak² is substituted or unsubstituted methylene, then a carbon atom of methylene and R⁵ are taken together may form a ring,
   when L is -SO₂N(R⁵)- or -C(=O)N(R⁵)-, then R⁵ and a constituent atom of B taken together may form a fused ring,
   R² is halogen, hydroxy, carboxy, substituted or unsubstituted alkyl, substituted or unsubstituted alkenyl, substituted or unsubstituted alkoxy, substituted or unsubstituted alkoxycarbonyl, substituted or unsubstituted acyl, substituted or unsubstituted amino or substituted or unsubstituted carbamoyl,
   R³ and R⁴ are each independently hydrogen, halogen, hydroxy, carboxy, substituted or unsubstituted alkyl, substituted or unsubstituted alkenyl, substituted or unsubstituted alkoxy, substituted or unsubstituted alkoxycarbonyl, substituted or unsubstituted acyl, substituted or unsubstituted amino or substituted or unsubstituted carbamoyl.
14') The pharmaceutical composition for suppressing amyloid β production of any one of the above 1') to 13') which is the pharmaceutical composition for selectively suppressing amyloid B42 production.
15') The pharmaceutical composition for suppressing amyloid β production of any one of the above 1') to 13') which is the pharmaceutical composition is for modulating Y secretase.
16') A compound of formula (II'): 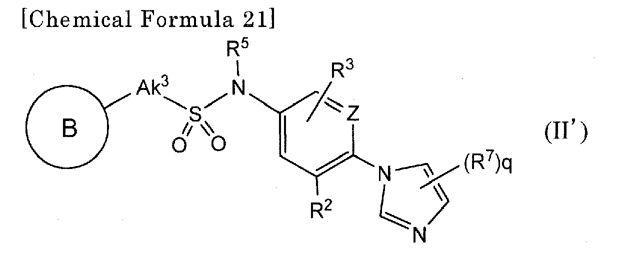 wherein B is substituted or unsubstituted phenyl which may be fused to other aromatic ring excluding unsubstituted phenyl, or substituted or unsubstituted pyridyl,
   Z is N or CR¹⁵,
   R² is halogen, hydroxy, carboxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted aryloxy, substituted or unsubstituted heterocyclyloxy, substituted or unsubstituted mercapto, substituted or unsubstituted alkoxycarbonyl, substituted or unsubstituted aryloxy carbonyl, substituted or unsubstituted heterocyclyloxy carbonyl, substituted or unsubstituted acyl, substituted or unsubstituted amino, substituted or unsubstituted carbamoyl, substituted or unsubstituted sulfamoyl, nitro, cyano, substituted or unsubstituted cycloalkyl, substituted or unsubstituted cycloalkenyl, substituted or unsubstituted aryl or substituted or unsubstituted heterocyclyl,
   R³ and R¹⁵ are each independently hydrogen, halogen, hydroxy, carboxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted aryloxy, substituted or unsubstituted heterocyclyloxy, substituted or unsubstituted mercapto, substituted or unsubstituted alkoxycarbonyl, substituted or unsubstituted aryloxy carbonyl, substituted or unsubstituted heterocyclyloxy carbonyl, substituted or unsubstituted acyl, substituted or unsubstituted amino, substituted or unsubstituted carbamoyl, substituted or unsubstituted sulfamoyl, nitro, cyano, substituted or unsubstituted cycloalkyl, substituted or unsubstituted cycloalkenyl, substituted or unsubstituted aryl or substituted or unsubstituted heterocyclyl,
   R⁵ is hydrogen, substituted or unsubstituted alkyl or substituted or unsubstituted acyl, R⁷ is halogen, hydroxy, carboxy, substituted or unsubstituted alkyl, substituted or unsubstituted alkenyl, substituted or unsubstituted alkoxy, substituted or unsubstituted mercapto, substituted or unsubstituted alkoxycarbonyl, substituted or unsubstituted acyl, substituted or unsubstituted amino, substituted or unsubstituted carbamoyl or substituted or unsubstituted sulfamoyl,
   Ak³ is a bond or substituted or unsubstituted methylene,
   q is an integer of 0 to 2,
   excluding the following compounds: 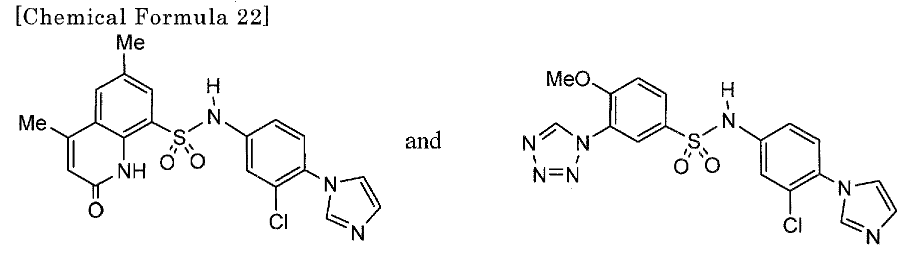 its pharmaceutically acceptable salt or a solvate thereof.
17') The compound of the above 16') wherein Z is CR¹⁵, R³ and R¹⁵ are each independently hydrogen, halogen, hydroxy, carboxy, substituted or unsubstituted alkyl, substituted or unsubstituted alkenyl, substituted or unsubstituted alkoxy, substituted or unsubstituted alkoxycarbonyl, substituted or unsubstituted acyl, substituted or unsubstituted amino or substituted or unsubstituted carbamoyl,
its pharmaceutically acceptable salt or a solvate thereof.
18') The compound of the above 16') or 17') wherein Ak³ is a bond, R⁵ is hydrogen, its pharmaceutically acceptable salt or a solvate thereof.
19') The compound of any one of the above 16') to 18') wherein q is 1, R⁷ is substituted or unsubstituted alkyl,
   its pharmaceutically acceptable salt or a solvate thereof.
20') A compound of the formula (III'): 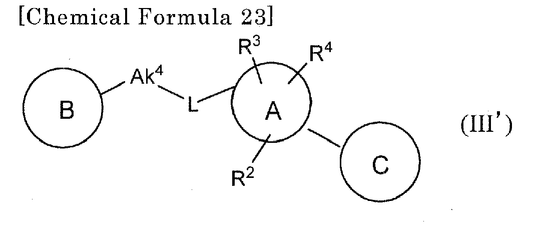 wherein 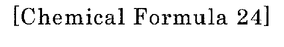 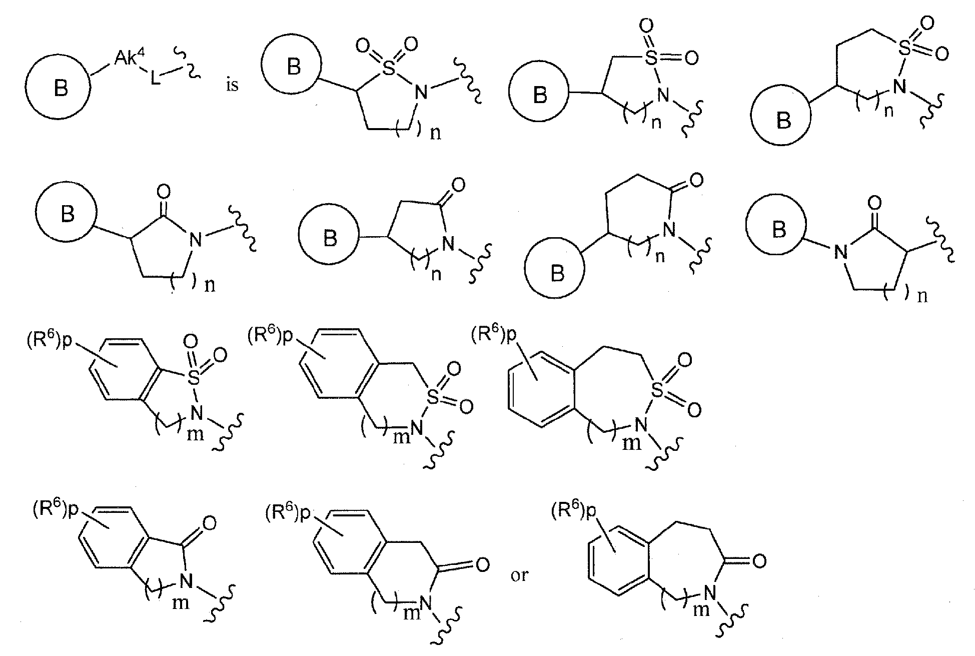 R⁶ is halogen, substituted or unsubstituted alkyl, substituted or unsubstituted alkoxy, substituted or unsubstituted amino, cyano, nitro, substituted or unsubstituted phenyl, substituted or unsubstituted phenoxy or substituted or unsubstituted heteroaryl,
   A is a benzene ring, a pyridine ring or a pyrimidine ring,
   B is substituted or unsubstituted phenyl which may be fused to other aromatic ring(s) excluding unsubstituted phenyl, or substituted or unsubstituted pyridyl,
   C is substituted or unsubstituted imidazolyl, substituted or unsubstituted oxazolyl, substituted or unsubstituted isoxazolyl, substituted or unsubstituted thiazolyl or substituted or unsubstituted isothiazolyl,
   R², R³ and R⁴ are each independently hydrogen, halogen, hydroxy, carboxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted aryloxy, substituted or unsubstituted heterocyclyloxy, substituted or unsubstituted mercapto, substituted or unsubstituted alkoxycarbonyl, substituted or unsubstituted aryloxycarbonyl, substituted or unsubstituted heterocyclyloxycarbonyl, substituted or unsubstituted acyl, substituted or unsubstituted amino, substituted or unsubstituted carbamoyl, substituted or unsubstituted sulfamoyl, nitro, cyano, substituted or unsubstituted cycloalkyl, substituted or unsubstituted cycloalkenyl, substituted or unsubstituted aryl or substituted or unsubstituted heterocyclyl,
   p, n and m are each independently an integer of 0 to 2,
   its pharmaceutically acceptable salt or a solvate thereof.
21') The compound of any one of the above 16') to 20') wherein B is substituted or unsubstituted phenyl, substituted or unsubstituted naphthyl, substituted or unsubstituted quinolyl or substituted or unsubstituted isoquinolyl, wherein the substituent(s) of each ring is one or more selected from the group of halogen, substituted or unsubstituted alkyl, substituted or unsubstituted alkoxy, substituted or unsubstituted amino, cyano, nitro, substituted or unsubstituted phenyl, substituted or unsubstituted phenoxy and substituted or unsubstituted heteroaryl,
   its pharmaceutically acceptable salt or a solvate thereof.
22') The compound of any one of the above 16) to 21) wherein R² is substituted or unsubstituted alkoxy,
   its pharmaceutically acceptable salt or a solvate thereof.
23') A pharmaceutical composition comprising the compound of any one the above 16') to 22'), its pharmaceutically acceptable salt or a solvate thereof.
24') The pharmaceutical composition for suppressing amyloid β production comprising the compound of any one of the above 16') to 22'), its pharmaceutically acceptable salt or a solvate thereof.
25') A pharmaceutical composition for selectively suppressing amyloid β42 production comprising the compound of any one of the above 16') to 22'), its pharmaceutically acceptable salt or a solvate thereof.
26') A pharmaceutical composition for modulating γ-secretase comprising the compound of any one of the above 16') to 22'), its pharmaceutically acceptable salt or a solvate thereof.
27') A method for suppressing amyloid β production comprising administering the compound of any one of the above 1') to 13'), 16') to 22'), its pharmaceutically acceptable salt or a solvate thereof.
28') A method for selectively suppressing amyloid β42 production comprising administering the compound of any one of the above 1') to 13'), 16') to 22'), its pharmaceutically acceptable salt or a solvate thereof.
29') A method for modulating γ-secretase comprising administering the compound of any one of the above 1') to 13'), 16') to 22'), its pharmaceutically acceptable salt or a solvate thereof.
30') Use of the compound of any one of the above 1') to 13'), 16') to 22'), its pharmaceutically acceptable salt or a solvate thereof for manufacturing a medicament for suppressing amyloid B production.
31') Use of the compound of any one of the above 1') to 13'), 16') to 22'), its pharmaceutically acceptable salt or a solvate thereof for manufacturing a medicament for selectively suppressing amyloid B42 production.
32') Use of the compound of any one of the above 1') to 13'), 16') to 22'), its pharmaceutically acceptable salt or a solvate thereof for manufacturing a medicament for modulating γ-secretase.
34') A compound of any one of the above 1') to 13'), 16') to 22'),its pharmaceutically acceptable salt or a solvate thereof for use a medicament for suppressing amyloid β production.
35') A compound of any one of the above 1') to 13'), 16') to 22'),its pharmaceutically acceptable salt or a solvate thereof for use a medicament for selectively suppressing amyloid β42 production.
36') A compound of any one of the above 1') to 13'), 16') to 22'),its pharmaceutically acceptable salt or a solvate thereof for use a medicament for modulating γ-secretase.

### [The effect of invention]

The compounds of the present invention are useful for a medicament for treating the diseases induced by production, secretion or deposition of aβ (e.g., Alzheimer's disease etc.).

### [Brief Description of Drawings]

[Fig. 1] Fig 1 shows change in the amount from C-terminal fragment β (CTF β) production by addition of the test compound. A vertical axis shows CTF β(%) of the test compound addition group in proportion to CTF β in DMSO only addition group.
[Fig. 2] Fig 1 shows change in the amount from C-terminal fragment β (CTF β) production by addition of the test compound. A vertical axis shows CTF β(%) of the test compound addition group in proportion to CTF β in DMSO only addition group.

### [Modes for Carrying out the Invention]

The term "halogen" as used herein includes fluorine, chlorine, bromine and iodine.
The halogen portions in "halogeno alkyl" and "halogeno alkoxy" are as defined above for the above "halogen."
The term "alkyl" includes C1-C15, preferably C1-C10, more preferably C1-C6, and further preferably C1-C3 linear or branched alkyl. Examples thereof include methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl and n-decy.
The alkyl portions in "halogeno alkyl", "alkoxy", "halogeno alkoxy", "alkoxycarbonyl", "alkylamino", "alkylcarbamoyl", "alkoxycarbonylamino", "alkyl carbamoylamino", "alkylthio", "alkylsulfonyl", "alkylsulfamoyl", "dialkyl borane" and "dialkoxy borane" are as defined above for the "alkyl."
The terms "substituted or unsubstituted alkyl", "substituted or unsubstituted alkoxy", "substituted or unsubstituted alkoxycarbonyl" and "substituted or unsubstituted alkylsulfonyl" may be substituted with one or more selected form the following Substituent Group α.

The Substituent Group α herein consists of halogen, hydroxy, alkoxy, acyl, acyloxy, carboxy, alkoxycarbonyl, amino, acylamino, alkylamino, alkoxycarbonylamino, carbamoylamino, alkylcarbamoylamino, mercapto, alkylthio, carbamoyl, alkylcarbamoyl, sulfamoyl, alkylsulfamoyl, cyano, nitro, carbocyclyl optionally substituted with the following Substituent Group β, carbocyclyloxy optionally substituted with the following Substituent Group β, carbocyclylthio optionally substituted with the following Substituent Group β, carbocyclylamino optionally substituted with the following Substituent Group β, heterocyclyl optionally substituted with the following Substituent Group β, heterocyclyloxy optionally substituted with the following Substituent Group β, heterocyclylthio optionally substituted with the following Substituent Group β, and heterocyclylamino optionally substituted with the following Substituent Group β.
The Substituent Group β herein consists of alkyl, halogenolkyl halogen, hydroxy, alkoxy, halogenoalkoxy, acyl, acyloxy, carboxy, alkoxycarbonyl, amino, acyl amino, alkylamino, alkoxycarbonylamino, mercapto, alkylthio, carbamoyl, alkyl carbamoyl, sulfamoyl, alkylsulfamoyl, cyano, nitro and phenyl.

The term "alkenyl" as used herein includes linear or branched C2-C15, preferably C2-C 10, more preferably C2-C6, more preferably C2-C4 alkenyl having one or more double bonds at optional positions. Examples include vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl and pentadecenyl.
The alkenyl portions of "alkenyloxy", "alkenyloxycarbonyl" and "alkenylsulfonyl" are as defined above for the "alkenyl."
Examples of the substituent of "substituted or unsubstituted alkenyl", "substituted or unsubstituted alkenyloxy", "alkenyloxycarbonyl" and "substituted or unsubstituted alkenylsulfonyl" include one or more selected from the above Substituent Group α.
The term "alkynyl" includes straight or branched C2-C10, preferably C2-C8, more preferably C3-C6 alkynyl having one or more triple bonds at optional positions. Examples include ethynyl, propynyl, butynyl, pentynyl, hexynyl, heptynyl, octynyl, nonyl and decynyl. These may further a double bond at an optional position.
The alkynyl portions of "alkynyloxy", "alkynyloxycarbonyl", and "alkynylsulfonyl" are as defined above for the "alkynyl."
Examples of the substituents of "substituted or unsubstituted alkynyl", "substituted or unsubstituted alkynyloxy", "substituted or unsubstituted alkynyloxycarbonyl", "substituted or unsubstituted alkynylsulfonyl" include one or more selected from the above Substituent Group α.

Examples of the substituent of "substituted or unsubstituted mercapto" includes alkyl optionally substituted with the above Substituent Group α, alkenyl optionally substituted with the above Substituent Group α, carbocyclyl optionally substituted with Substituent Group β, or heterocyclyl optionally substituted with Substituent Group β.
Examples of the substituent of "substituted or unsubstituted amino", "substituted or unsubstituted carbamoyl", "substituted or unsubstituted sulfamoyl" are one or two selected from halogen, hydroxy, alkyl optionally substituted with the above Substituent Group α, alkenyl optionally substituted with the above Substituent Group α, acy optionally substituted with the above Substituent Group α, alkoxy optionally substituted with the above Substituent Group α, alkoxycarbonyl optionally substituted with the above Substituent Group α, carbocyclyl optionally substituted with the above Substituent Group and heterocyclyl optionally substituted with the above Substituent Group β.
The term "acyl" includes C1-C10 aliphatic acyl, carbocyclylcarbonyl and heterocyclylcarbonyl. Examples include formyl, acetyl, propionyl, butyryl, isobutyryl, valeryl, pivaloyl, hexanoyl, acryloyl, propioloyl, methacryloyl, crotonoyl, benzoyl, cyclohexanecarbonyl, pyridinecarbonyl, furancarbonyl, thiophenecarbonyl, benzothiazolecarbonyl, pyrazinecarbonyl, pyrrolidinecarbonyl and piperidinecarbonyl.
The acyl portions in "acylamino" and "acyloxy" are as defined above.
Examples of the substitution of "substituted or unsubstituted acyl" includes one or more selected from the Substituent Group α. The ring portions in carbocyclylcarbonyl and heterocyclylcarbonyl may be substituted with one or more selected from alkyl, the Substituent Group α, alkyl substituted with one or more selected from the Substituent Group α.

The term "carbocycle" includes cycloalkane, cycloalkene, aromatic carbocycle and non-aromatic fused carbocycle.
The term "cycloalkane" include C3-C 10, preferably C3-C8, and more preferably C4-C8 carbocycle. Examples are cyclopropane, cyclobutane, cyclopentane, cyclohexane, cycloheptane, cyclooctane, cyclononane and cyclodecane.
The term "cycloalkene" includes a ring of the above "cycloalkane" having one or more double bonds at optional positions such as cyclopropene, cyclobutene, cyclopentene, cyclohexene, cycloheptene, cyclooctene, cyclononene, cyclodecene and bicyclohexadiene.
The term "aromatic carbocycle" includes benzene, naphthalene and anthracene.
The term "non-aromatic fused carbocycle" includes fused rings comprising two or more rings, each of ring is selected from "cycloalkane", "cycloalkene" and "aromatic carbocycle", and which is other than the above "aromatic carbocycle."

The term "carbocyclyl" includes cycloalkyl, cycloalkenyl, aryl and non-aromatic fused carbocyclyl.
The term "cycloalkyl" includes C3-C10, preferably C3-C8, and more preferably C4-C8 carbocyclyl. Examples are cyclopropyl, cyclobutyl, cyclopentyl, cyclohexyl, cycloheptyl, cyclooctyl, cyclononyl and cyclodecyl.
The term "cycloalkenyl" includes a ring of the above "cycloalkyl" having one or more double bonds at optional positions such as cyclopropenyl, cyclobutenyl, cyclopentenyl, cyclohexenyl, cycloheptynyl, cyclooctynyl and cyclononyl, cyclodecynyl and cyclohexadienyl.
The term "aryl" includes phenyl, naphthyl, anthryl and phenanthryl. Particularly phenyl is preferable.
The term "non-aromatic fused carbocyclyl" include a monovalent group comprising two or more rings, each of which is selected from the above "cycloalkane", "cycloalkene" and benzene ring, and which is other than the above "aryl." Examples are indenyl, indenyl, tetrahydronaphtyl and fluorenyl.
The carbocycle portions in "carbocyclylcarbonyl", "carbocyclyloxy", "carbocyclyloxycarbonyl", '"carbocyclylsulfonyl", "carbocyclylthio", and "carbocyclylamino" are as defined for the "carbocyclyl."
The aryl portions in "aryloxy", "arylthio" and "aryloxycarbonyl" are as defined for the above "aryl."

The term "phenyl which may be fused to other aromatic ring(s)" includes a monovalent group derived by eliminating a hydrogen atom from the fused ring comprising one or more aromatic ring selected from the above "aromatic carbocycle" and an aromatic ring in the after-mentioned "heterocyclyl" is fused to a benzene ring. In the above groups, the bond attached to the other group is located on the benzene ring. The bond attached to the other group in "substituted or unsubstituted quinolyl" or "substituted or unsubstituted isoquinolyl" as Y in the formula (I) or as B in the formula (I') is also located preferably on the benzene ring.
Examples of "phenyl fused to other aromatic ring(s)" are naphthyl, anthryrl, phenanthryl, indole (indolyl), isoindole (isoindolyl), indazole (indazolyl), indoline (indolinyl), isoindoline (isoindolinyl), quinoline (quinolyl), isoquinoline (isoquinolyl), cinnoline (cinnolinyl), phthalazine (phthalazinyl), quinazoline (quinazolinyl), quinoxaline (quinoxalinyl), benzopyran (benzopyranyl), benzimidazole (benzimidazolyl), benzotriazole (benzotriazolyl), benzisoxazole (benzisoxazolyl), benzoxazole (benzoxazolyl), benzoxadiazole (benzoxadiazolyl), benzisothiazole (benzisothiazolyl), benzothiazole (benzothiazolyl), benzothiadiazole (benzothiadiazolyl), benzofuran (benzofuranyl), isobenzofuran (isobenzofuranyl), benzothiophene (benzothiophenyl), benzotriazole (benzotriazolyl), carbazole (carbazolyl), acrydine (acridinyl), phenanthridine (phenanthridinyl), phenazine (phenazinyl) and benzisoquinoline (benzisoquinolyl).
The term "phenyl which may be fused to other ring(s)" includes a monovalent group derived by eliminating a hydrogen atom from the fused ring comprising one or more ring selected from the above "aromatic carbocycle" and after-mentioned "heterocycle" is fused to a benzene ring. The bond of these groups is located on the benzene ring. The bond of "substituted or unsubstituted quinolyl" or "substituted or unsubstituted isoquinolyl" as Y in the formula (I) is also located preferably on the benzene ring.
Examples of "phenyl fused to other ring(s)" are the above "phenyl fused to other aromatic ring(s)" and benzo[1,3]dioxole (benzo[1,3]dioxolyl).

The "heterocycle" includes a heterocycle having one or more hetero atoms optionally selected from O, S and N in a ring, and examples include 5- or 6-membered heterocycle such as pyrrole, imidazole, pyrazole, pyridine, pyridazine, pyrimidine, pyrazine, triazole, triazinyl, tetrazole, isoxazole, oxazole, oxadiazole, isothiazole, thiazole, thiadiazole, furan and thiophene;
bicyclic fused heterocycle such as indole, isoindole, indazole, indolidine, indoline, isoindoline, quinoline, isoquinoline, cinnoline, phthalazine, quinazoline, naphthrydine, quinoxaline, purine, pteridine, benzopyran, benzimidazole (benzoimidazole), benzotriazole, benzisoxazole (benzoisoxazole), benzoxazole (benzoxazole), benzoxadiazole (benzooxadiazole), benzoisothiazole, benzothiazole, benzothiadiazole, benzofuran, isobenzofurran, benzothiophene, benzotriazole, imidazopyridine, pyrazolopyridine, triazolopyridine, imidazothiazole, pyrazinopyridadine, quinazoline, quinoline, isoquinoline, naphthyridine, dihydrobenzofuran, tetrahydroquinoline, tetrahydroisoquinoline, dihydrobenzoxazine and tetrahydrobenzothiophene;
tricyclic fused heterocycle such as carbazole, acrydine, xanthene, phenothiadine, phenoxathiirane, phenoxadine, dibenzofuran and imidazoquinole; and
non-aromatic heterocycle such as dioxane, thiirane, oxirane, oxathiolane, azetidine, thiane, thiazolidine, pyrrolidine, pyrroline, imidazolidine, imidazoline, pyrazolidine, pyrazoline, piperidine, piperazine, morpholine, thiomorpholine, dihydropyridine, dihydrobenzimidazole, tetrahydropyridine, tetrahydrofuran, tetrahydropyrane, tetrahydrothiazole, tetrahydroisothiazole, dihydrooxazine, hexahydroazepine, and tetrahydrodiazepine.
The term "heterocyclyl" includes a monovalent group derived by eliminating a hydrogen atom from the above "heterocycle."
The term "heteroaryl" includes an aromatic cyclic group in the above "heterocyclyl."
The heterocyclic portions in "heterocyclyloxy", "heterocyclylthio", "heterocyclylamino", "heterocyclylcarbonyl", "heterocyclyloxycarbonyl" and "heterocyclylsulfonyl" are as defined above for the "heterocycle."

Examples of the substituent for "substituted or unsubstituted cycloalkyl", "substituted or unsubstituted cycloalkenyl", "substituted or unsubstituted aryl", "substituted or unsubstituted carbocyclyl", "substituted or unsubstituted heterocyclyl", "substituted or unsubstituted heteroaryl", "substituted or unsubstituted aryloxy", "substituted or unsubstituted carbocyclyloxy", "substituted or unsubstituted aryloxycarbonyl", "substituted or unsubstituted carbocyclyoxycarbonyl", "substituted or unsubstituted heterocyclyloxy", "substituted or unsubstituted heterocyclyoxycarbonyl", "substituted or unsubstituted carbocyclylsulfonyl", "substituted or unsubstituted heterocyclylsulfonyl", "substituted or unsubstituted phenyl", "substituted phenyl", "substituted or unsubstituted phenyl which may be fused to other aromatic ring(s)", "substituted or unsubstituted phenyl which may be fused to other ring(s)", "substituted or unsubstituted naphthyl", "substituted or unsubstituted phenoxy", "substituted or unsubstituted thiazolyl", "substituted or unsubstituted isothiazolyl", "substituted or unsubstituted oxazolyl", "substituted or unsubstituted isoxazolyl", "substituted or unsubstituted imidazolyl", "substituted or unsubstituted pyridyl", "substituted or unsubstituted quinolyl" and "substituted or unsubstituted isoquinolyl" are as follows and these may be substituted with one or more substituents selected from these groups. the Substituent Group α,
alkyl optionally substituted with one or more selected from the Substituent Group α,
alkenyl optionally substituted with one or more selected from the Substituent Group α,
alkynyl optionally substituted with one or more selected from the Substituent Group α,
alkoxy substituted with one or more selected from the Substituent Group α,
alkylthio substituted with one or more selected from the Substituent Group α,
alkylamino substituted with one or more selected from the Substituent Group α,
alkylsulfonyl optionally substituted with one or more selected from the Substituent Group α,
alkoxycarbonyl substituted with one or more selected from the Substituent Group α,
acyl substituted with one or more selected from the Substituent Group α,
alkylenedioxy optionally substituted with halogen or hydroxy, and
oxo.

The term "C1-C3 alkylene" includes C1, C2 or C3 divalent carbon chain. Examples are methylene, dimethylene and trimethylene.
The term "C1-C3 alkylene which may be interposed with hetero atom(s)" includes alkylene which may contain O, S or NR wherein R is hydrogen, alkyl, acyl or the like in the position adjacent to the alkylene constituent carbon atom or between the carbon atoms. Examples are -OCH₂-, -O(CH₂)₂-, -O(CH₂)₃-, -SCH₂-, -S(CH₂)₂-, -S(CH₂)₃-, -NHCH₂-, -NH(CH₂)₂-, -NH(CH₂)₃-, -N(Me) CH₂-, -N(Me) (CH₂)₂-, -N(Me) (CH₂)₃-, CH₂OCH₂- -CH₂SCH₂-, -CH₂NHCH₂-, -CH₂N(Me) CH₂-, -CH₂CH₂OCH₂-, -CH₂CH₂SCH₂-, - CH₂CH₂NHCH₂-, -CH₂CH₂N(Me) CH₂-, -CH₂O-, -(CH₂)₂O-, -(CH₂)₃O-, -CH₂S-, -(CH₂)₂S-, - (CH₂)₃S-, -CH₂NH-, -(CH₂)₂NH-, -(CH₂)₃NH-, -CH₂N(Me)-, -(CH₂)₂N(Me)-, -(CH₂)₃N(Me)-. The atom which is adjacent to L in Ak¹ or Ak² is necessarily a carbon atom, respectively.
The alkylene portions in "alkylenedioxy" include C1-C6 straight or branched carbon chain, and methylene, dimethylene, trimethylene, tetramethylene, pentamethylene, hexamethylene, ethylethylene, propylene and the like are included.
Examples of the substituent of "substituted or unsubstituted C1-C3 alkylene", "substituted or unsubstituted C1-C3 alkylene which may be interposed with hetero atom(s)" and "substituted or unsubstituted methylene" are halogen, hydroxy, alkyl, alkoxy, oxo, alkoxycarbonyl, phenyl optionally substituted with one or more selected from the Substituent Group α.
The term "when L is -SO₂N(R⁵)-or -C(=O)N(R⁵)-, and Ak¹ is substituted or unsubstituted C1-C3 alkylene which may be interposed with hetero atom(s), then a carbon atom of Ak¹ and R⁵ taken together may form a ring" includes, for example, the following groups: 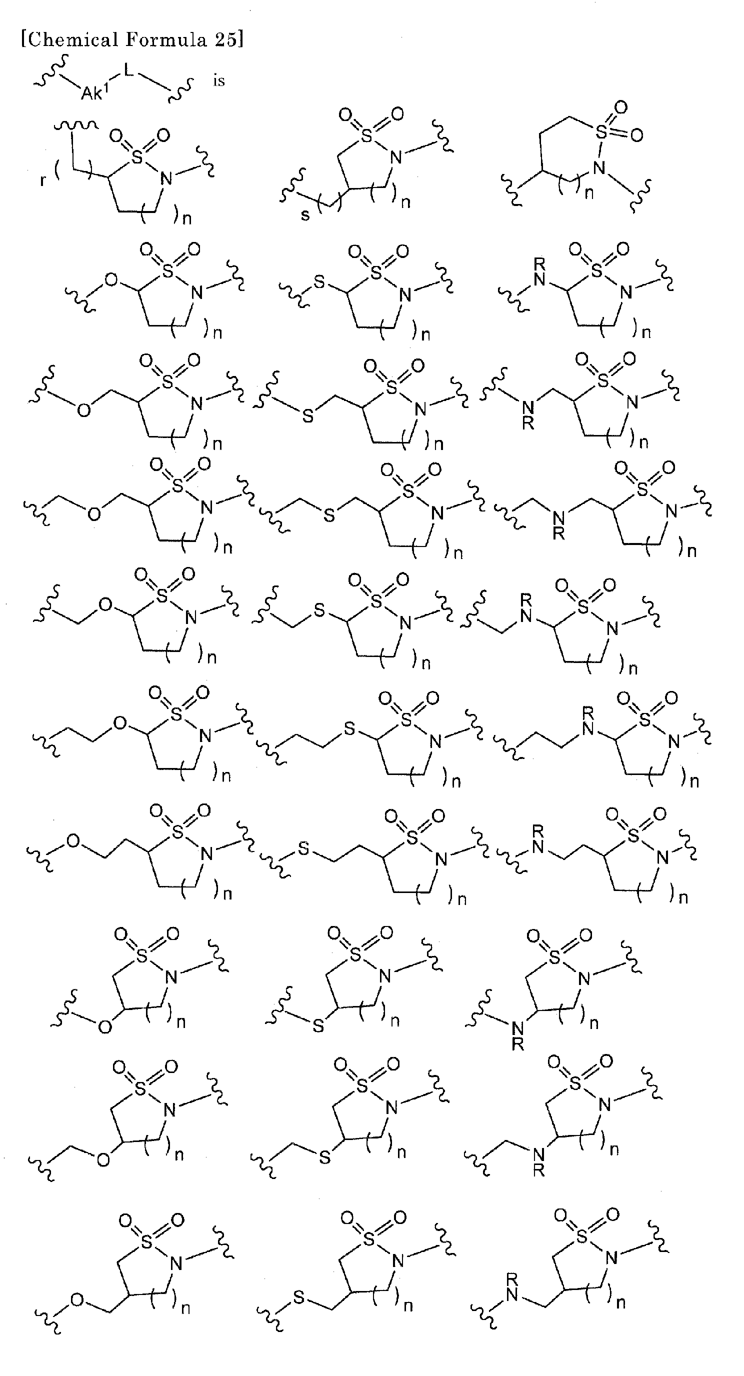 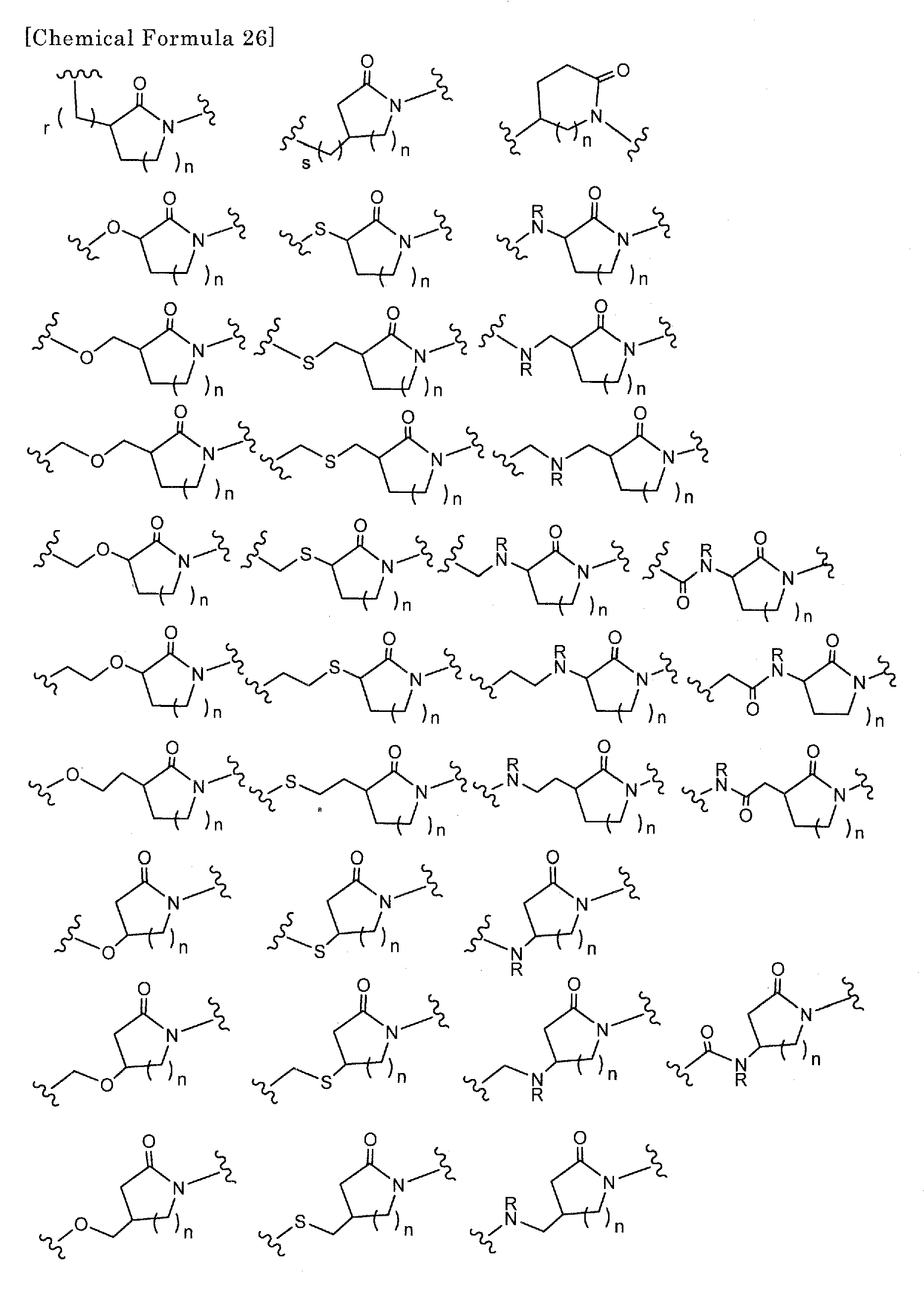 wherein R is hydrogen, alkyl, acyl, or the like, n is an integer of 0 to 2, r is an integer of 0 to 2, and s is an integer of 0 or 1.
The term "when L is -N(R⁵) C(=O)- and Ak² is substituted or unsubstituted C1-C3 alkylene which may be interposed with hetero atom(s), then a carbon atom of Ak² and R⁵ taken together may form a ring" includes, for example, the following groups: 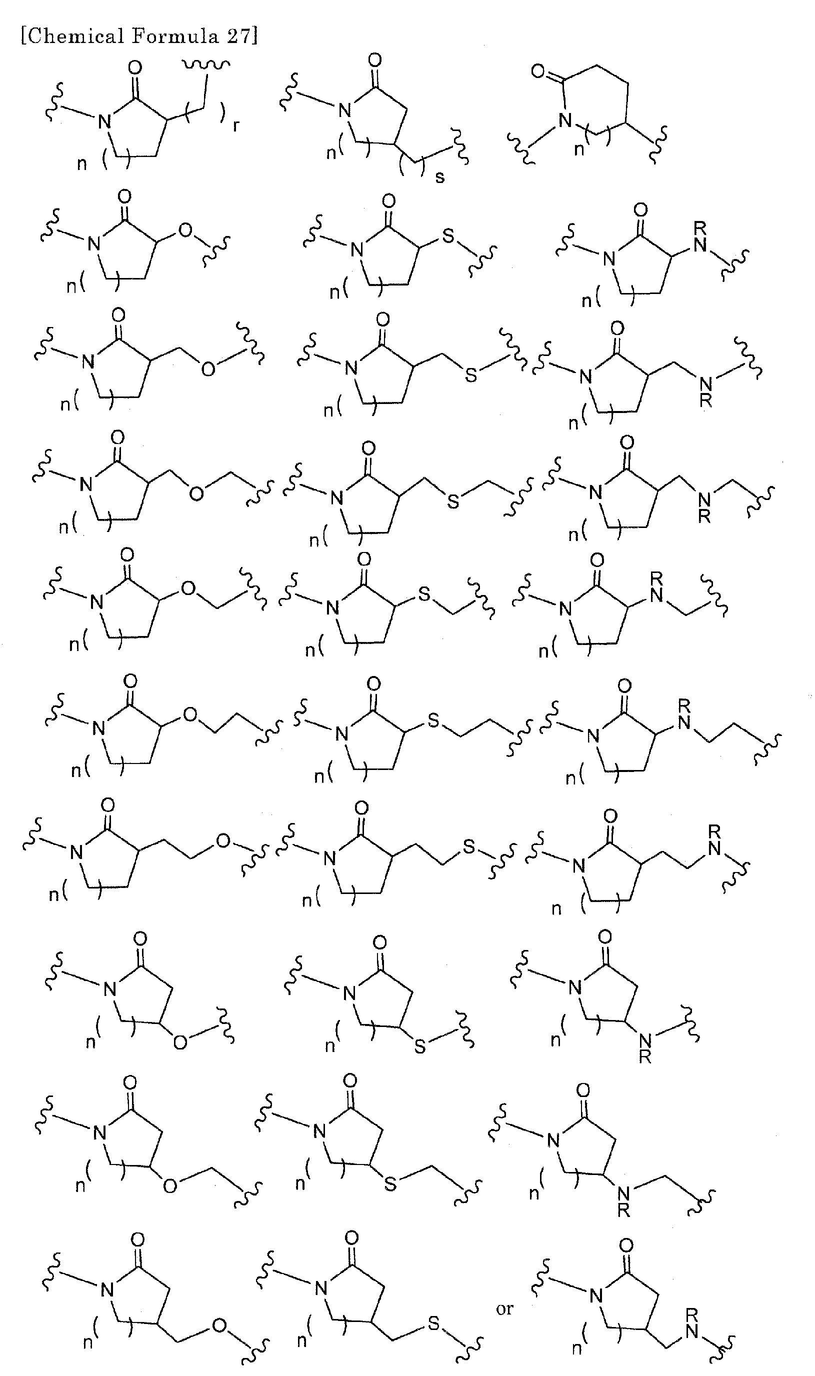 wherein R is hydrogen, alkyl, acyl or the like, n and r are each independently an integer of 0 to 2, and s is an integer of 0 or 1.

The term "when L is -SO₂N(R⁵)- or -C(=O) N(R⁵)-, then R⁵ and a constituent atom of B taken together may form a fused ring" includes, for example, the following groups: 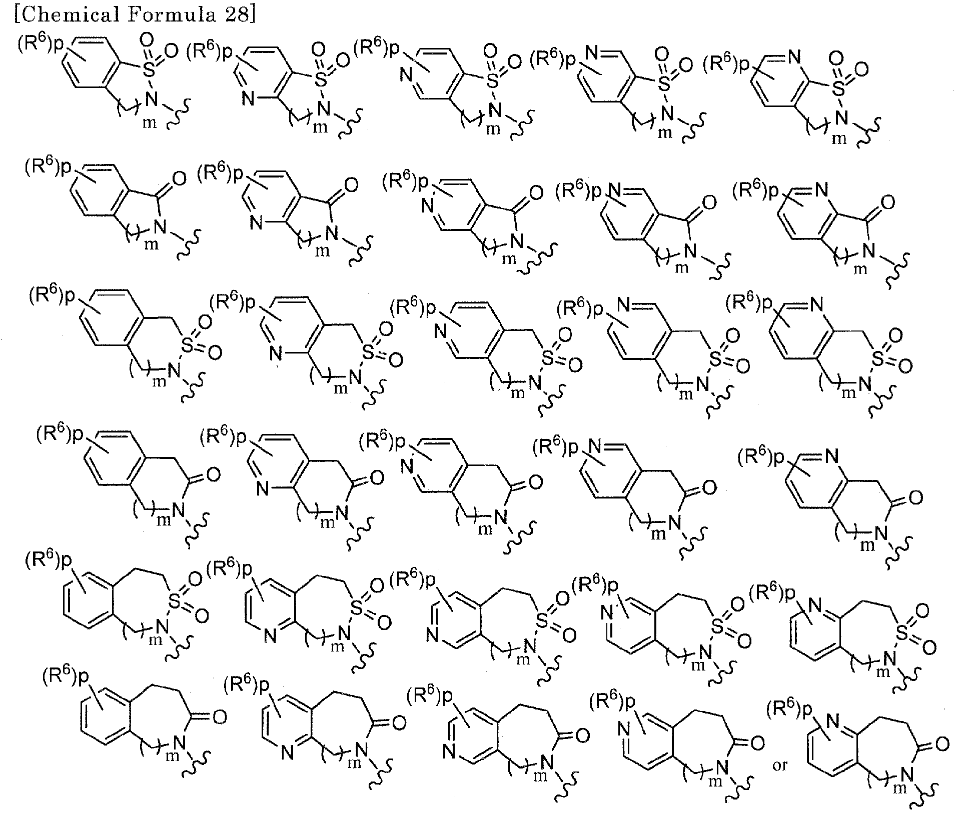 wherein m and p are each independently an integer of 0 to 2 and R⁶ is halogen, substituted or unsubstituted alkyl, substituted or unsubstituted alkoxy, substituted or unsubstituted amino, cyano, nitro, substituted or unsubstituted phenyl, substituted or unsubstituted phenoxy or substituted or unsubstituted heteroaryl.

The group of the formula: 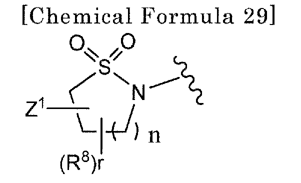 includes, for example, the following groups: 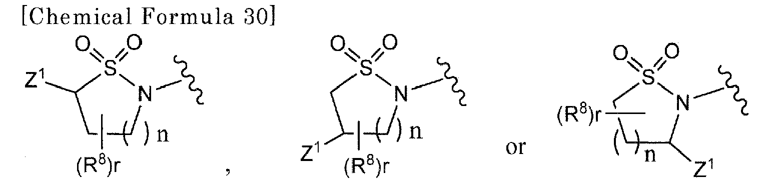 wherein Z¹, R⁸, r and n are as defined in the above 1) and R⁸ may attach to any carbon atom in the ring.

The group of the formula: 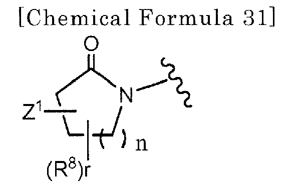 includes, for example, the following groups: 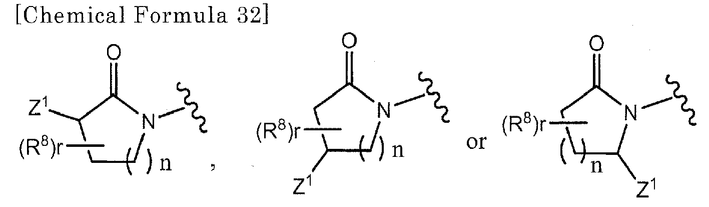 wherein Z¹, R⁸, r and n are as defined in the above 1), and R⁸ may bind to any carbon atom in the ring.

The group of the formula: 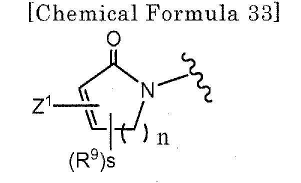 includes, for example, the following groups: 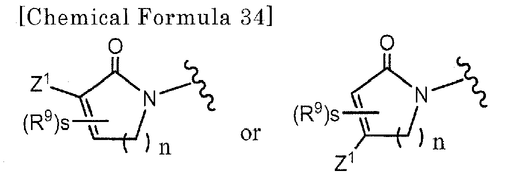 wherein Z¹, R⁹, s and n are as defined in the above 1), and R⁹ may bind to any carbon atom in the ring.

A bond of R⁶ or R¹⁰ which strike through two rings such as 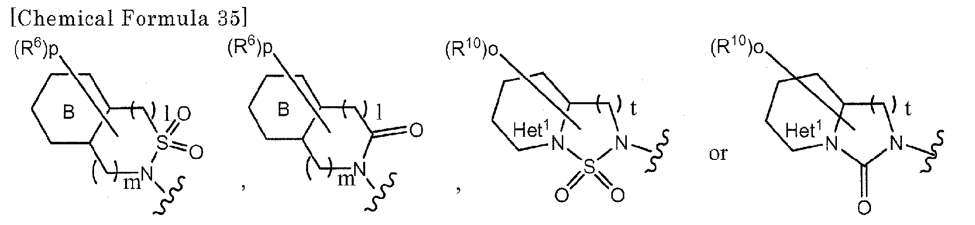 means R⁶ or R¹⁰ may bind to any atom which can be substituted in any ring.
A bond of R⁸ or R¹¹ attaches to only one ring such as 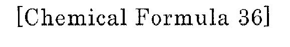 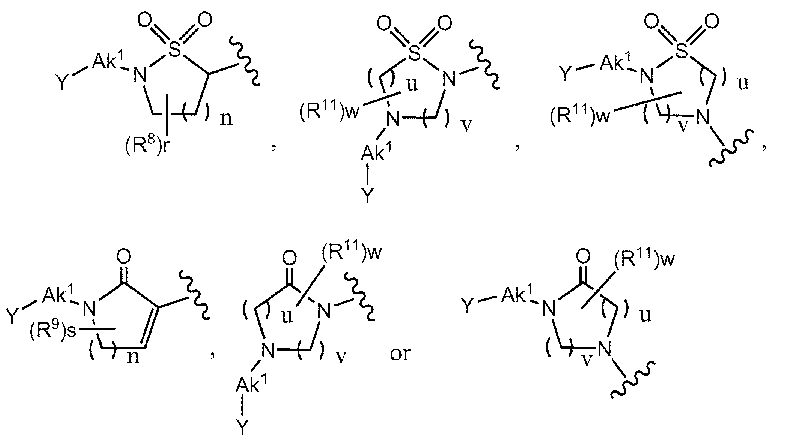 means R⁸, R⁹ or R¹¹ may bind to any atom which can be substituted in the ring.

In -Ak¹-SO₂N(R⁵)-Ak², the "S" atom binds to Ak¹ and the "N" atom binds to Ak².

In the present specification, the "solvate" includes, for example, a solvate with an organic solvent, and a hydrate. When a hydrate is formed, an arbitrary number of water molecules may be coordinated.
The compound of the present invention includes a pharmaceutically acceptable salt. Examples include salts with alkali metal such as lithium, sodium or potassium; alkaline earth metal such as magnesium or calcium; ammonium; organic base; and amino acid; salts with inorganic acid such as hydrochloric acid, sulfuric acid, nitric acid, hydrobromic acid, phosphoric acid or hydroiodic acid; or organic acid such as acetic acid, citric acid, lactic acid, tartaric acid, oxalic acid, maleic acid, fumaric acid, mandelic acid, glutaric acid, malic acid, benzoic acid, phthalic acid, benzenesulfonic acid, p-toluenesulfonic acid, methanesulfonic acid or ethanesulfonic acid. Particularly, hydrochloric acid, phosphoric acid, tartaric acid or methanesulfonic acid is preferable. These salts can be formed by ordinary methods.
The compound (I) or (I') of the present invention is not limited to a specific isomer, and include any possible isomers ,e.g. keto-enol isomers, imine-enamine isomers, diastereoisomers, optical isomers and rotamers, and racemic mixtures.
In addition, one or more hydrogen, carbon or other atoms of a compound of the formula (I) or the formula (I') can be replaced by an isotope of the hydrogen, carbon or other atoms. Compounds of the formula (I) or (I') include all radiolabeled forms of compounds of the formula (I) or the formula (I'). The "radiolabeled," "radiolabeled form", and the like of a compound of the formula (I) or the formula (I') are encompassed by the invention and useful as a research and/or diagnostic tool in metabolism pharmacokinetic studies and in binding assays. Examples of isotopes that can be incorporated into a compound of the formula (I) of the invention include isotopes of hydrogen, carbon, nitrogen, oxygen, phosphorous, sulfur, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively. Radiolabeled compounds of the invention can be prepared by methods known in the art. For example, tritiated compounds of the formula (I) or (I') can be prepared by introducing tritium into the particular compound of the formula (I) or the formula (I'), for example, by catalytic dehalogenation with tritium. This method may include reacting a suitably halogen-substituted precursor of a compound of the formula (I) or the formula (I') with tritium gas in the presence of a suitable catalyst such as Pd/C, in the presence or absence of a base. Other suitable methods for preparing tritiated compounds can be found in Isotopes in the Physical and Biomedical Sciences, Vol. 1, Labeled Compounds (Part A),Chapter 6 (1987). ¹⁴C-labeled compounds can be prepared by employing starting compounds having a ¹⁴C carbon.

General procedures for synthesis of the compound (I) or the compound (I') of the present invention are shown below. Each of starting compounds and reaction reagents is commercially available or can be prepared by known methods in the art using compounds commercially available. Compounds described in Examples were basically prepared according to the methods in Examples, but not limited to these methods. Reaction solvents, bases, palladium catalysts, and phosphine ligand which can be used for synthesis of the compounds are shown below. In general procedures, preferable ones among them are exemplified but not limited to them.
(1) Reaction solvents: DMF, NMP, DMA, dimethyl sulfoxide, aromatic hydrocarbons such as toluene, benzene and xylene, saturated hydrocarbons such as cyclohexane and hexane, halogenated hydrocarbons such as dichloromethane, chloroform and 1,2-dichloroethane, ethers such as tetrahydrofuran, dimethyl ether, dioxane and 1,2-dimethoxyethane, esters such as methyl acetate and ethyl acetate, ketones such as acetone and methyl ethyl ketone, nitriles such as acetonitrile, alcohols such as methanol, ethanol and t-butanol, water and the mixed solvents thereof and the like.
(2) Bases: metal hydrides such as sodium hydride, metal hydroxides such as sodium hydroxide, potassium hydroxide, lithium hydroxide and barium hydroxide, metal carbonates such as sodium carbonate, potassium carbonate, calcium carbonate and cesium carbonate, metal alkoxides such as sodium methoxide, sodium ethoxide, sodium t-butoxide, sodium 2-methyl butane-2-olate and potassium t-butoxide, sodium bicarbonate, metallic sodium, and organic amine such as triethylamine, diisopropylethylamine, DBU, pyridine, 4-dimethyl aminopyridine and 2, 6-lutidine, alkyl lithiums such as n-BuLi, sec-BuLi and tert-BuLi.
(3) Pd catalysts for Pd coupling: Pd(PPh₃)₄, PdCl₂(dppf), PdCl₂(PPh₃)₂, Pd(OAc)₂, Pd₂(dba)₃, PdCl₂ and Ru-Phos.
(4) Phosphine ligand: PPh₃, BINAP, Xantphos, S-Phos, X-Phos, DPPF, t-Bu₃P, Tris and o-tolylphosphine.

The compound of the formula (I) or the formula (I') and the compounds (I'a), (I'b), (I'c), (Id), (Ie), (If), (Ig), and (Ih) wherein X in the formula (I) or (I') is specified of the present invention can be prepared according to the following synthetic routes.

### (Process A)

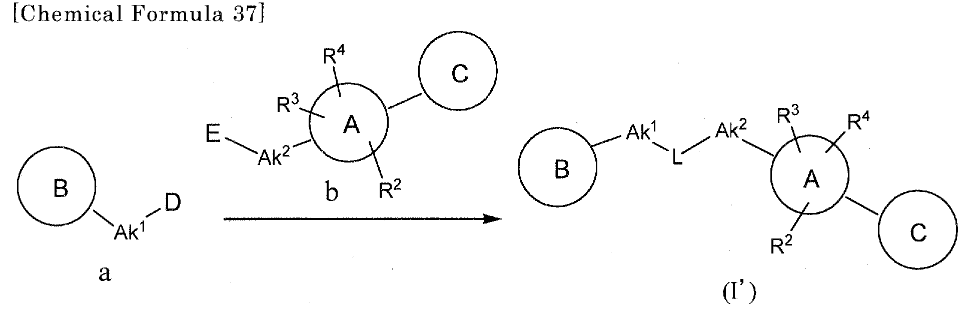

wherein D is SO₂-Hal or CO-Hal and E is NHR⁵; or D is NHR⁵ and E is CO-Hal; Hal is halogen and other symbols are as defined in the above 1').
Compound (I') can be prepared by reacting Compound a with Compound b, each of which are a known compound or compound prepared by known methods from a known compound. For example, Compound a is a halogenosulfonyl compound and Compound b is an amine compound. Compound a may be a salt such as hydrochloride or bromate. As a reaction solvent, ones shown in the above (1) can be used. Examples of preferable solvents are tetrahydrofuran, dichloromethane, chloroform, acetate ester, acetonitrile, N,N-dimethylformamide, N,N-dimethylacetamide and dimethyl sulfoxide, and a solvent is not limited if it is other than one which interferes with a reaction under the employed reaction conditions. The reaction may be carried out in the presence of a base such as DBU, triethylamine or pyridine at a temperature between room temperature and 200 °C and if the reactivity is low, the reaction may be carried out with heating. When either D or E is COOH, the reaction can be carried out in the presence of a condensing agent such as N,N' dicyclohexylcarbodiimide, N-dimethylaminopropyl-N'-ethyl carbodiimide, diethyl phosphorocyanidate or diphenylphosphorylazide.

### (Process B)

After the coupling reaction of the above Process A, it is also possible to introduce the substituent corresponding to the target compound by reactions usually used. The following scheme illustrates the case where a substituent R is introduced after a coupling reaction according to Process A. 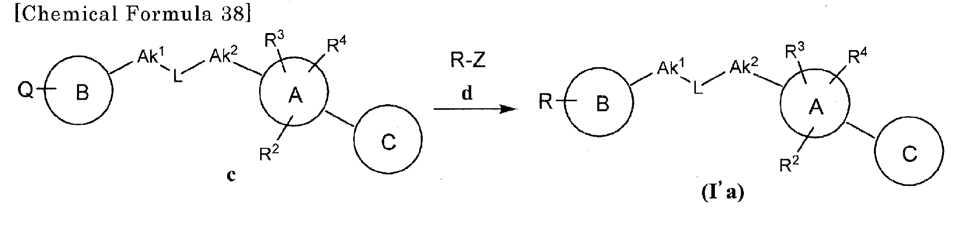 wherein either Q or Z is halogen or a leaving group and the other is dihydroxyborane, dialkoxyborane, dialkylborane, 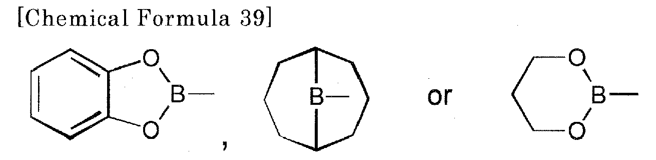 and the other symbols are as defined in the above 1').
The target compound (I'a) can be prepared by reacting Compound c which is prepared according to the above Process A with Compound d which is a known compound or compound prepared by known methods from a known compound from 50°C to under reflux, preferably under reflux, or using microwave irradiation at a temperature between 120 °C and 200 °C, preferably 130 °C and 150 °C. If necessary, a quaternary ammonium salt such as tetrabutylammonium bromide can be used as an additive.
As halogen, chlorine, iodine, and bromine are preferable and as a leaving group, -OSO₂(CₜF₂ₜ₊₁), wherein t is an integer of 1 to 4, is preferable, and OTf (trifluoromethanesulfonate) is particularly preferable. R may be an arbitrary group which is suitable as a substituent such as hydrogen, alkyl, aryl, alkenyl, acyl, amino, alkoxy, sulfamoyl or carbamoyl.
The target compound (I'a) can be also prepared by introducing a substituent R by Suzuki Coupling Reaction of Compound c with Compound d using a palladium catalyst shown in the above (3). If necessary, the reaction can be carried out in the presence of a phosphine ligand shown in the above (4) and a base shown in the above (2).
As a reaction solvent, the ones shown in the above (1) can be used. Preferable examples are dioxane, DMF, DME, lower alcohol, toluene, and the mixture thereof and a solvent is not limited if it is other than one which interferes with a reaction under these employed conditions. The reaction can be carried out at a temperature between room temperature and 200 °C but not limited to. If the reactivity is low, the reaction may be carried out with heating suitably. As a base, a solid or an aqueous solution of Na₂CO₃, K₃PO₄, K₂CO₃, NaOH, Cs₂CO₃ or the like are preferable.

### (Process C)

After the coupling reaction of the above Process A, (1) a compound wherein a carbon atom of Ak¹ and R⁵ taken together form a ring when L is -SO₂N(R⁵)- or - C(=O)N(R⁵)-, (2) a compound wherein a carbon atom of Ak² and R⁵ taken together form a ring when L is -N(R⁵)C(=O)- or (3) a compound wherein a carbon atom of Ak² and R⁵ taken together form a ring when La is -N(R⁵)C(=O)- can be prepared by methods usually used.
The following scheme is an example of a process of Compound (I'b) wherein a carbon atom of Ak¹ and R⁵ of -SO₂N(R⁵)- taken together may form a ring. 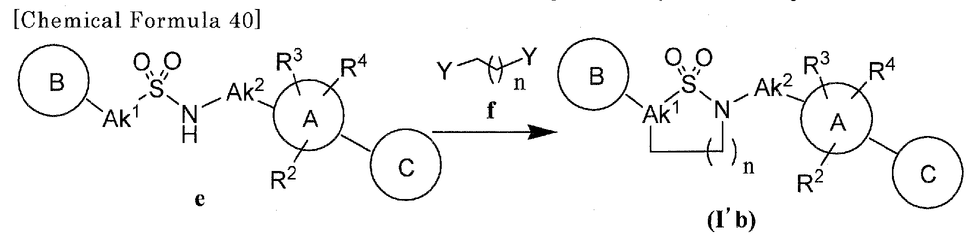 wherein Y are each independently halogen or a leaving group, n is an integer of 0 to 2 and the other symbols are as defined in the above 1').
Compound (I'b) can be prepared by reacting Compound e with a dihalogenated compound or Sulfinate f at a temperature between -78 °C and 100 °C in the presence of a base such as sodium hydride, sodium hydroxide, sodium methoxide, tert-BuLi and DBU.
After a cyclization of Process C, a substituent corresponding to the target compound can be introduced by the method of Process B.
A compound wherein a carbon atom of Ak¹ and R⁵ taken together form a ring when L is -C(=O)N(R⁵)- and a compound wherein a carbon atom of Ak² and R⁵ taken together form a ring when L s -N(R⁵)C(=O)- also can be prepared in a similar manner as described above.

### (Process D)

A compound wherein L is -C(=O)N(R⁵)- and R⁵ and a constituent atom of B taken together form a fused ring can be, for example, prepared by the following methods. 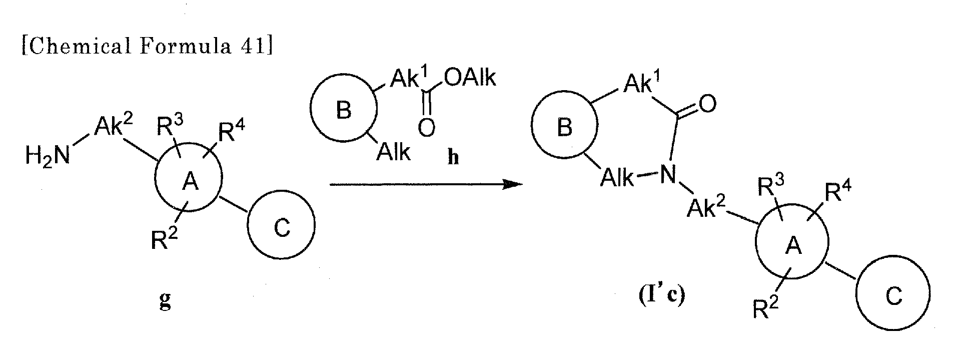 wherein Alk are each independently alkyl and the other symbols are as defined above 1').
A target compound (I'c) can be prepared by reacting Compound g with Compound h, each of which is a known compound or compound prepared by known methods from a known compound. As a reaction solvent, ones shown in the above (1) can be used. Preferable examples are tetrahydrofuran, dichloromethane, chloroform, acetate ester, acetonitrile, N,N-dimethylformamide, N,N-dimethyl acetamide, dimethyl sulfoxide and alcohols, and a solvent is not limited if it is other than one which interferes with a reaction under the employed reaction conditions. The reaction may be carried out in the presence of a base such as DBU, diisopropylethylamine, triethylamine, pyridine at a temperature between room temperature and 200 °C, and if the reactivity is low, the reaction may be carried out with heating suitably. The target compound (I'c) can be prepared by reacting using microwave irradiation at a temperature between 120 °C and 200 °C, preferably 130 °C and 150 °C.
A compound wherein L is -SO₂N(R⁵)- and R⁵ and a constituent atom of B taken together form a fused ring can be prepared in a similar manner.

### (Process E)

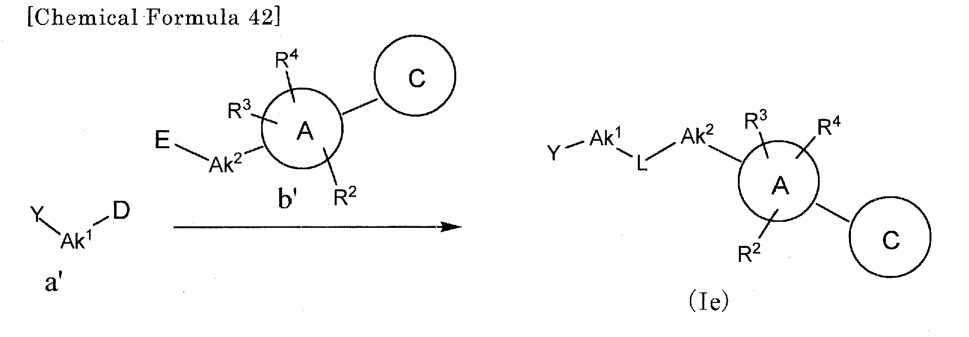

wherein D is SOz-Hal, E is -NHR⁵ and the other symbols are as defined above.
Starting Compound a' and Compound b' are known compounds or compounds prepared by known methods from known compounds, respectively.
Compound (Ie) can be prepared by reacting Compound a' with Compound b'. For example, Compound a' is a halogenosulfonyl compound and Compound b' is an amine compound. Compound a' may be a salt such as hydrochloride or bromate. As a reaction solvent, ones shown in the above (1) can be used. Preferable examples are tetrahydrofuran, dichloromethane, chloroform, acetate ester, acetonitrile, N,N-dimethylformamide, N,N-dimethylacetamide and dimethyl sulfoxide, and a solvent is not limited if it is other than one which interferes with a reaction under the employed reaction conditions. The reaction may be carried out in the presence of a base such as DBU, triethylamine or pyridine at a temperature between room temperature and 200 °C, and if the reactivity is low, the reaction may be carried out with heating suitably. When either D or E is COOH, the reaction can be carried out in the presence of a condensing agent such as N,N' dicyclohexylcarbodiimide, N-dimethylaminopropyl-N'-ethyl carbodiimide, diethyl 1 phosphorocyanidate or diphenylphosphoryl azide.

### (Process F)

After the coupling reaction of the above Process A, it is also possible to introduce a substituent corresponding to the target compound by reactions usually used. The following scheme illustrates the case where a substituent R is introduced after a coupling reaction according to Process A. 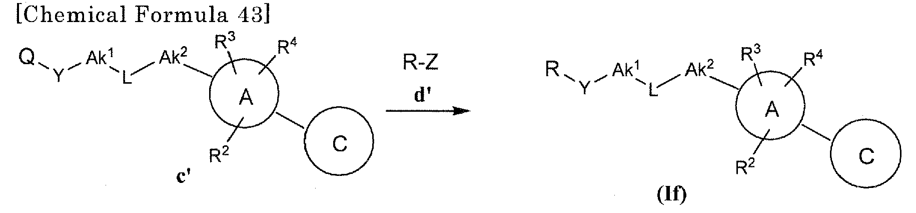 wherein either Q or Z is halogen or a leaving group and the other is dihydroxyborane, dialkoxyborane, dialkylborane, 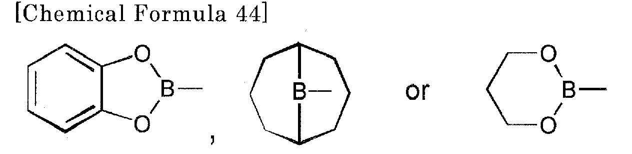 and the other symbols are as defined above.
The target compound (If) can be prepared by reacting Compound c' which is prepared according to the above Process F with Compound d' which is a known compound or compound prepared by known methods from a know compound from 50 °C to under reflux, preferably under reflux, or using microwave irradiation at a temperature between 120 °C and 200 °C, preferably 130 °C and 150 °C. If necessary, a quaternary ammonium salt such as tetrabutylammonium bromide can be used as an additive.
As halogen, chlorine, iodine, and bromine are preferable. As a leaving group, - OSO₂(CₜF₂ₜ₊₁) wherein t is an integer of 1 to 4 is preferable, and OTf (trifluoromethanesulfonate) is particularly preferable. R may be an arbitrary group which is suitable as a substituent such as hydrogen, alkyl, aryl, alkenyl, acyl, amino, alkoxy, sulfamoyl or carbamoyl.
The target compound (If) can be also prepared by introducing a substituent R by Suzuki Coupling Reaction of Compound c' with Compound d' using a palladium catalyst shown in the above (3). If necessary, the reaction can be carried out in the presence of a phosphine ligand shown in the above (4) and a base shown in the above (2).
As a reaction solvent, the ones shown in the above (1) can be used. Preferable examples are dioxane, DMF, DME, lower alcohol, toluene, and the mixture thereof and a solvent is not limited if it is other than one which interferes with a reaction under these employed conditions. The reaction can be carried out at a temperature between room temperature and 200 °C but not limited to. If the reactivity is low, the reaction may be carried out with heating suitably. As a base, a solid or an aqueous solution of Na₂CO₃, K₃PO₄, K₂CO₃, NaOH, Cs₂CO₃ or the like are preferable.

### (Process G)

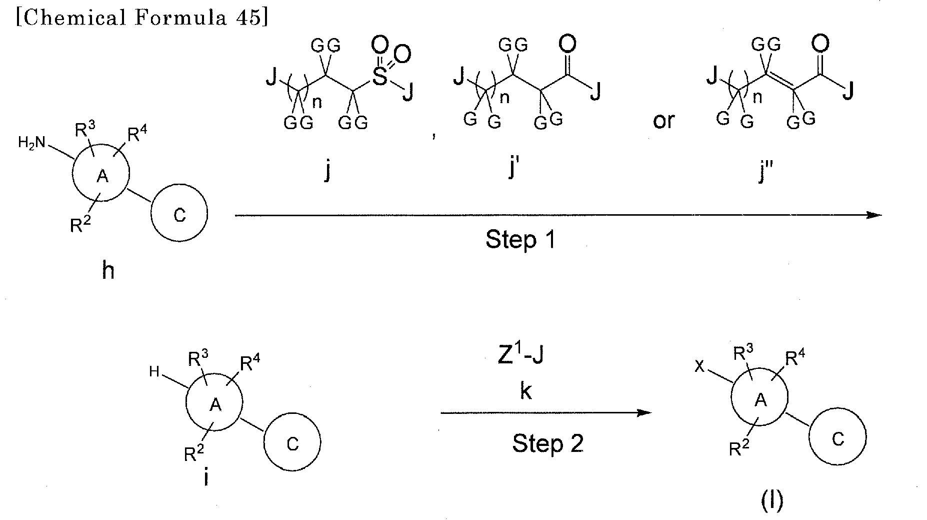

wherein J is halogen or a leaving group, 0 to 2 of G groups may be R⁸ or R⁹, the others of G groups are hydrogen, 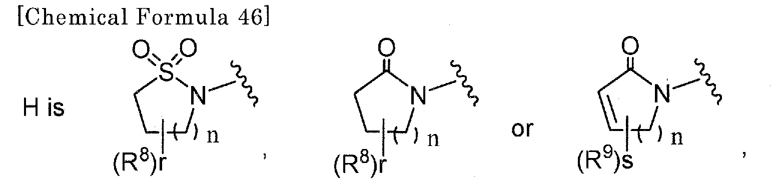 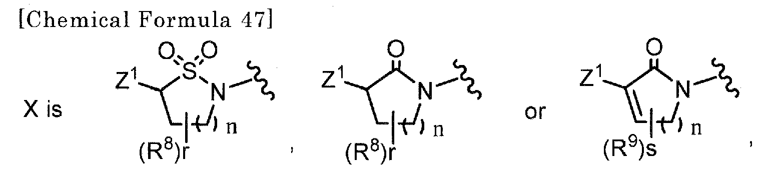 and the other symbols are as defined above 1).
Starting compounds are known compounds or compounds prepared by known methods from known compounds, respectively.

### (Step 1)

Compound i can be prepared by reacting Compound h with Compound j, Compound j' or Compound j". For example, Compound h is an aniline compound and may be salt thereof such as hydrochloride or bromate. When J is halogen, then chlorine, iodine or bromine is preferable, and when J is a leaving group, then OTf (trifluoromethanesulfonate) is preferable. As a reaction solvent, ones shown in the above (1) can be used. Preferable examples are tetrahydrofuran, dichloromethane, chloroform, acetate ester, acetonitrile, N,N-dimethylformamide, N,N-dimethylacetamide and dimethyl sulfoxide, and a solvent is not limited if it is other than one which interferes with a reaction under the employed reaction conditions. The reaction may be carried out in the presence of a base such as DBU, triethylamine or pyridine, and although the reaction temperature is not limited to, at a temperature between room temperature and 200 °C. If the reactivity is low, the reaction may be carried out with heating suitably.

### (Step 2)

Compound (I) can be prepared by reacting Compound i with Compound k from - 78 °C to under reflux, preferably at -78 °C. When J is halogen, then chlorine, iodine or bromine is preferable and when J is a leaving group, then OTf (trifluoromethanesulfonate) is preferable.

### (Process H)

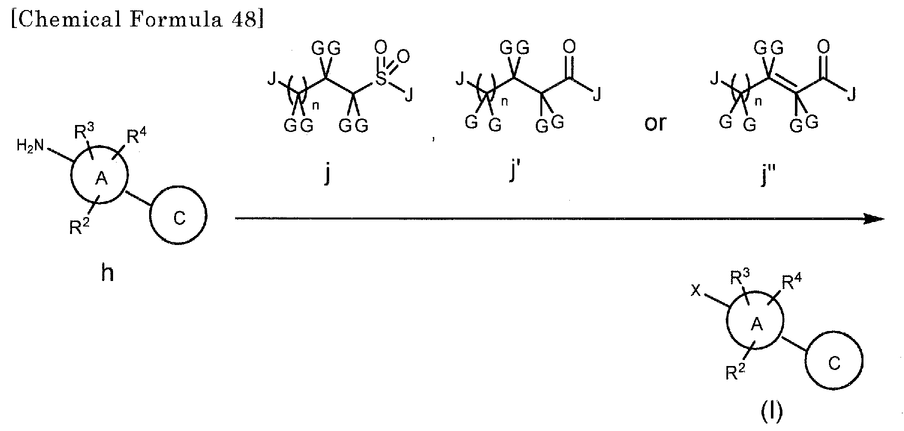

wherein J is halogen or a leaving group, one of G groups is Z¹, 0 to 2 of G groups may be R⁸ or R⁹, the others of G groups are hydrogen, 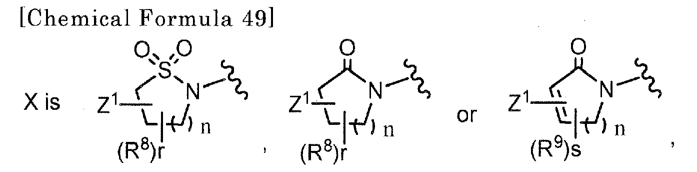 and the other symbols are as defined in the above 1).
Starting compounds are known compounds or compounds prepared by known methods from known compounds, respectively.
Compound (I) can be prepared by reacting Compound h with Compound j, Compound j' or Compound j". For example, Compound h is an aniline compound and may be salt thereof such as hydrochloride or bromate. When J is halogen, then chlorine, iodine or bromine is preferable, and when J is a leaving group, then OTf (trifluoromethanesulfonate) is preferable. As a reaction solvent, ones shown in the above (1) can be used. Preferable examples are tetrahydrofuran, dichloromethane, chloroform, acetate ester, acetonitrile, N,N-dimethylformamide, N,N-dimethylacetamide and dimethyl sulfoxide, and a solvent is not limited if it is other than one which interferes with a reaction under the employed reaction conditions. The reaction may be carried out in the presence of a base such as DBU, triethylamine or pyridine, and although the reaction temperature is not limited, at a temperature between room temperature and 200 °C. If the reactivity is low, the reaction may be carried out with heating suitably.

### (Process 1)

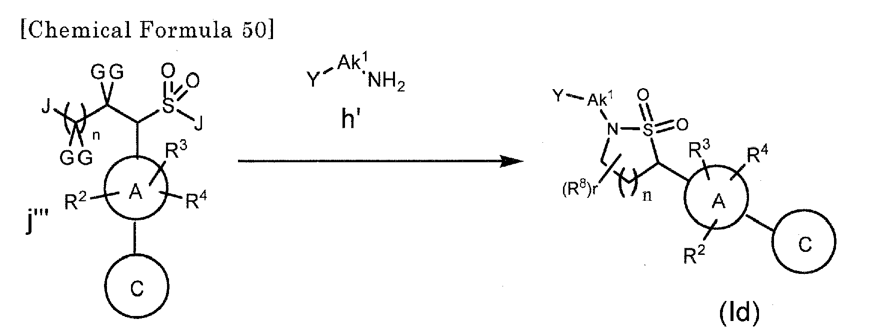

wherein J is halogen or a leaving group, 0 to 2 of G groups may be R⁸, the others of G groups are hydrogen and other symbols are as defined in the above 1).
Starting compounds are known compounds or compounds prepared by known methods from known compounds, respectively.
Compound (Id) can be prepared by reacting Compound j"' with Compound h'. For example, Compound h is an aniline compound and may be salt thereof such as hydrochloride or bromate. When J is halogen, then chlorine, iodine or bromine is preferable, and when J is a leaving group, then OTf (trifluoromethanesulfonate) is preferable. As a reaction solvent, ones shown in the above (1) can be used. Preferable examples are tetrahydrofuran, dichloromethane, chloroform, acetate ester, acetonitrile, N,N-dimethylformamide, N,N-dimethylacetamide and dimethyl sulfoxide, and a solvent is not limited if it is other than one which interferes with a reaction under the employed reaction conditions. The reaction may be carried out in the presence of a base such as DBU, triethylamine or pyridine, and although the reaction temperature is not limited to, at a temperature between room temperature and 200 °C. If the reactivity is low, the reaction may be carried out with heating suitably.

### (Process J)

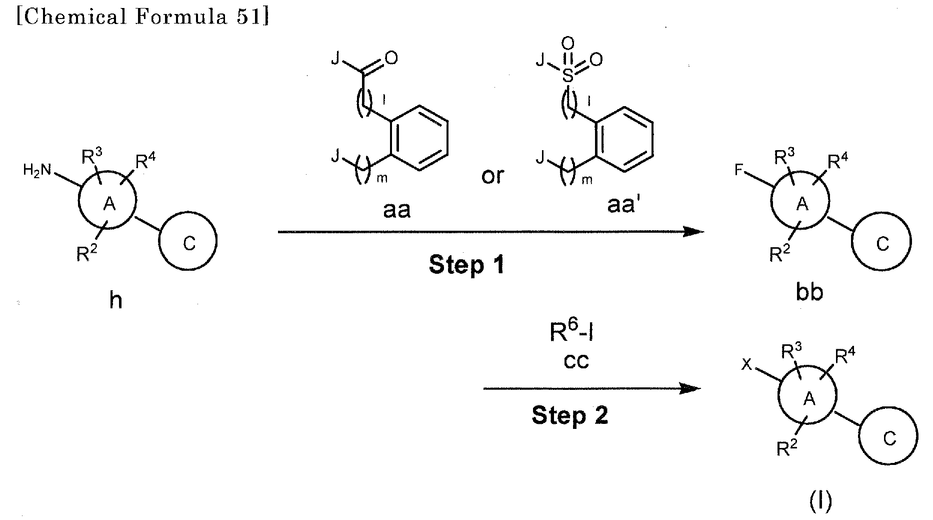

wherein J is halogen or a leaving group, 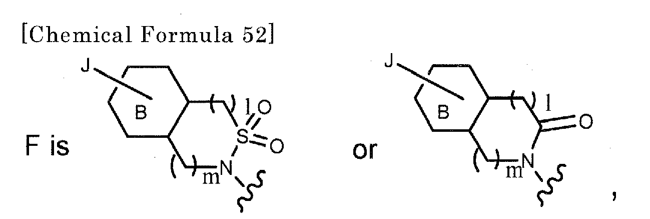 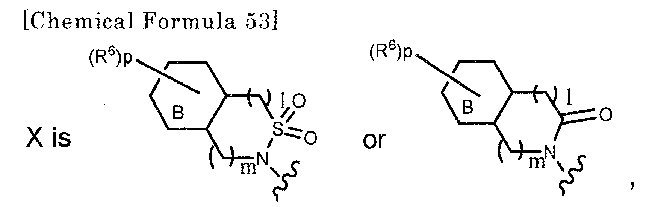 wherein I is dihydroxyborane, dialkoxyborane, dialkylborane, 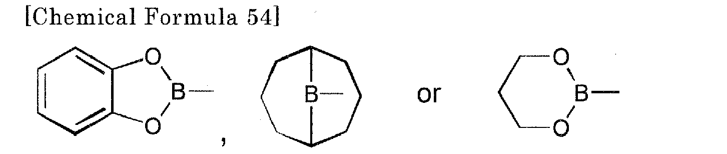 and the other symbols are as defined above 1).
Starting compounds are known compounds or compounds prepared by known methods from known compounds, respectively.
Compound bb can be prepared by reacting Compound h or hydrochloride or bromate thereof or the like with an acid halide aa or aa'. As a reaction solvent, ones shown in the above (1) can be used. Preferable examples are tetrahydrofuran, dichloromethane, chloroform, acetate ester, acetonitrile, N,N-dimethylformamide, N,N-dimethylacetamide and dimethyl sulfoxide, and a solvent is not limited if it is other than one which interferes with a reaction under the employed reaction conditions. The reaction may be carried out in the presence of a base such as DBU, triethylamine or pyridine, and although the reaction temperature is not limited to, at a temperature between room temperature and 200 °C. If the reactivity is low, the reaction may be carried out with heating suitably. When L is halogen, then chlorine, iodine or bromine are preferable, and when L is a leaving group, then OTf (trifluoromethanesulfonate) is preferable. The target compound having a substituent on a sultam or lactam ring of Compound (1) can be prepared by reactions usually used if a compound having a substituent on an alkylene side chain of an acid halide aa or aa' is used as a starting compound.

### (Step 2)

Compound (I) can be prepared by reacting Compound bb with Compound cc from 50 °C to under reflux, preferably under reflux, or using microwave irradiation at a temperature between 120 °C and 200 °C, preferably 130 °C and 150 °C.

When L is halogen, then chlorine, iodine, or bromine are preferable, and when L is a leaving group, then OTf (trifluoromethanesulfonate) is preferable. R⁶ may be an arbitrary group which is suitable as a substituent such as hydrogen, alkyl, aryl, alkenyl, acyl, amino, alkoxy, sulfamoyl or carbamoyl. Two R⁶ groups taken together may form a ring.

### (Process K)

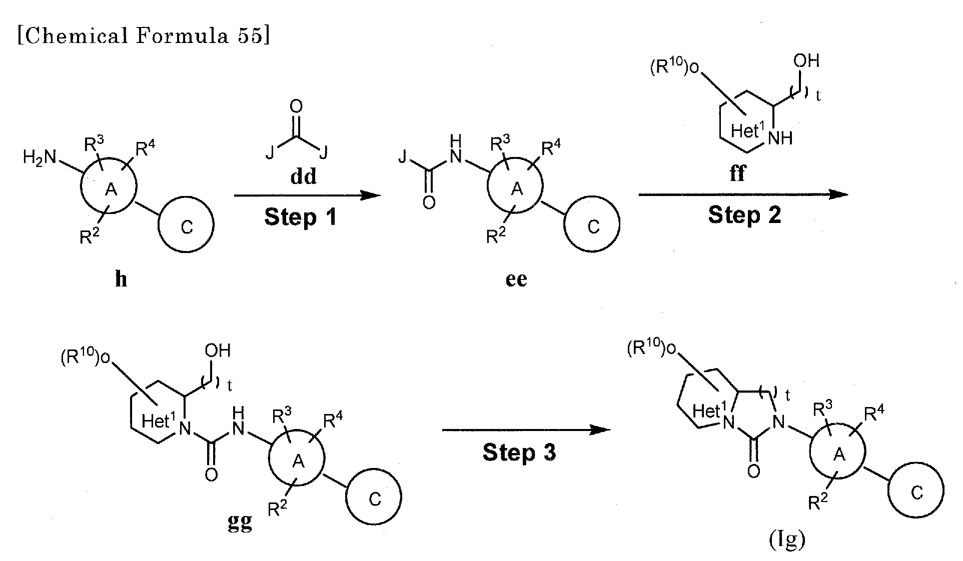

wherein J is halogen or a leaving group and other symbols are as defined in the above.
These compounds are known compounds or compounds prepared by known methods from known compounds.

### (Step 1)

Compound ee can be prepared by reacting Compound h with Compound dd. As a reaction solvent, ones shown in the above (1) can be used. Examples of preferable solvents are dioxane, tetrahydrofuran, dichloromethane, chloroform, acetate ester, acetonitrile, N,N-dimethylformamide, N,N-dimethylacetamide and dimethyl sulfoxide and a solvent is not limited if it is other than one which interferes with a reaction under the employed reaction conditions. The reaction may be carried out in the presence of a base such as DBU, triethylamine or pyridine at a temperature between -20 °C and 100 °C. If the reactivity is low, the reaction may be carried out with heating suitably.

### (Step 2)

Compound gg can be prepared by reacting Compound ee with Compound ff. For example, Compound ff is a cyclic amine compound. As a reaction solvent, ones shown in the above (1) can be used. Examples of preferable solvents are dioxane, tetrahydrofuran, dichloromethane, chloroform, acetate ester, acetonitrile, N,N-dimethylformamide, N,N-dimethylacetamide and dimethyl sulfoxide, and a solvent is not limited if it is other than one which interferes with a reaction under the employed reaction conditions. The reaction may be carried out in the presence of a base such as DBU, triethylamine or pyridine at a temperature between -20 °C and 100 °C. If the reactivity is low, the reaction may be carried out with heating suitably. The target compound having a substituent on a urea ring of Compound (Ig) can be prepared by reactions usually used if a compound having a substituent on a hydroxyalkyl side chain of a cyclic amine compound ff is used as a starting compound.

### (Step 3)

Compound (Ig) can be prepared by a ring closure reaction of Compound gg. Compound (Ig) can be prepared by reacting Compound gg with an alkylsulfonate or an arylsulfonate at a temperature between -78 °C and 100 °C in the presence of a base such as sodium hydride, sodium hydroxide, sodium methoxide, tert-BuLi, DBU or pyridine.

As a reaction solvent, ones shown in the above (1) can be used. Preferable examples are dioxane, tetrahydrofuran, dichloromethane, chloroform, acetate ester, acetonitrile, N,N-dimethylformamide, N,N-dimethylacetamide and dimethyl sulfoxide, and a solvent is not limited if it is other than one which interferes with a reaction under the employed reaction conditions. The reaction may be carried out in the presence of a base such as DBU, triethylamine or pyridine at a temperature between -20 °C and 100 °C. If the reactivity is low, the reaction may be carried out with heating suitably.

### (Process L)

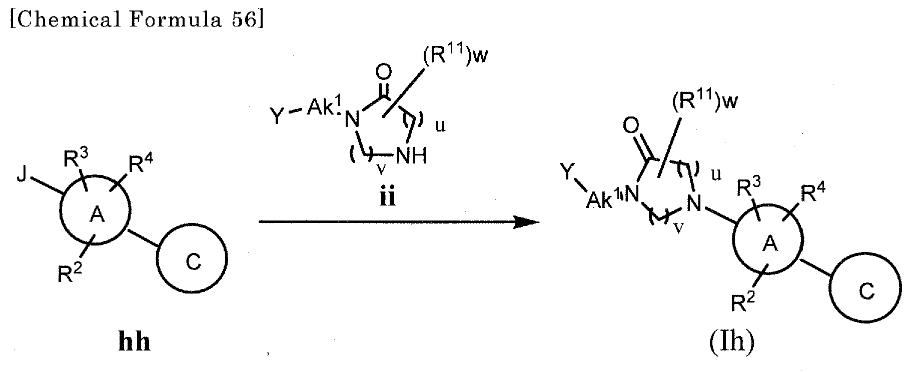

wherein J is halogen or a leaving group and the other symbols are as defined above.
Starting compounds are known compounds or compounds prepared by know methods from known compound, respectively.
Target compound (Ih) can be prepared by reacting Compound hh with Compound ii from 50 °C to under reflux, preferably under reflux, or using microwave irradiation at a temperature between 120 °C to 200 °C, preferably 100 °C and 150 °C. If necessary, a quaternary ammonium salt such as tetrabutylammonium bromide can be used as an additive.
When J is halogen, then chlorine, iodine or bromine is preferable, when J is a leaving group, then -OSO₂(CₜF₂ₜ₊₁) wherein t is an integer of 1 to 4 is preferable, and OTf (trifluoromethanesulfonate) is particularly preferable. The target compound (Ih) can be prepared by a coupling reaction of Compound hh with Compound ii using a palladium catalyst shown in the above (3) and a phosphine ligand shown in the above (4). If necessary, the reaction can be carried out in the presence of a phosphine ligand shown in the above (4) and a base shown in the above (2).
As a reaction solvent, ones shown in the above (1) can be used. Preferable examples are dioxane, DMF, DME, lower alcohol, toluene, and the mixture thereof and a solvent is not limited if it is other than one which interferes with a reaction under these employed conditions. The reaction may be carried out at a temperature between room temperature and 200 °C but not limited to. If the reactivity is low, the reaction may be carried out with heating suitably.
If the following Compound jj, Compound kk or Compound 11 is used instead of Compound ii, the corresponding target compound can be prepared.

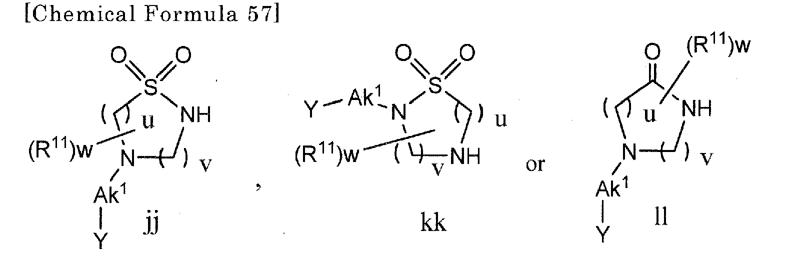

Conversion of a functional group of each substituent can be carried out in an appropriate step.
For example, a compound having a hydroxy group can be prepared by converting a compound having a formyl group to a compound having a formyloxy group using Baeyer-Villiger reaction or the like, followed by usual hydrolysis reaction in an acidic condition or a basic condition. Specifically, a compound may be reacted with peracid such as peracetic acid, perbenzoic acid, meta-chloroperbenzoic acid, trifluoro peracetic acid or peroxide in a suitable solvent such as 1,2-dichloroethane, chloroform, dichloromethane, carbon tetrachloride or benzene from -20°C to under heating for several minutes to several tens hours to afford a compound having a formyloxy group and thus-obtained compound may be hydrolyzed in an acidic condition such as heating with hydrochloric acid or in a basic condition such as heating with sodium hydroxide.
A compound having a hydroxymethyl group can be prepared by reacting a compound having a formyl group using a reductant agent such as sodium borohydride, lithium borohydride, zinc borohydride, lithium triethylborohydride, aluminum hydride or diisobutylaluminum hydride in a suitable solvent to the reductant such as methanol, ethanol, isopropanol, dimethyl sulfoxide, diethyleneglycol dimethoxyethane, tetrahydrofuran, benzene, toluene or cyclohexane at a temperature between -20 °C and 80 °C, preferably between under ice-cooling and room temperature for several tens minutes to several hours.
A compound having an alkenyl group can be prepared by subjecting a compound having a formyl group to a Wittig reaction (Organic reaction, 1965, vol. 14, p. 270).
A compound having a carboxy group can be prepared by reacting a compound having a formyl group using an oxidizing agent such as sodium chlorite, Jones reagent or chromic anhydride in a suitable solvent to the oxidizing agent such as t-butanol or acetone from 0 °C to under heating for several hours. The reaction can be preferably carried out by adding 2-methyl-2-butene, sodium dihydrogen phosphate or the like, if necessary.
A compound having an alkoxy group can be prepared by reacting a compound having a hydroxy group with a corresponding alkylating agent in the presence of a base such as sodium carbonate, sodium bicarbonate, potassium carbonate, calcium hydroxide, barium hydroxide or calcium carbonate in a suitable solvent such as tetrahydrofuran, acetone, dimethylformamide or acetonitrile.

A compound having a carbamoyl group can be prepared by reacting a compound having a carboxy group, if necessary, after activating using a suitable activating agent such as thionyl chloride, acid halide, acid anhydride or activated ester with an amine compound such as ammonia or dimethylamine, in a suitable solvent such as tetrahydrofuran, dimethylformamide, diethyl ether or dichloromethane from 0 °C to under heating for several minutes to several tens hours.
A compound having a halogen group can be prepared by reacting a compound having a hydrogen group with a halogenating agent usually used such as bromine, chlorine, iodine, sulfuryl chloride, N-bromosuccinimide or N-iodosuccinimide, if necessary, in the presence of a catalyst such as a Lewis acid, hydrochloric acid or phosphoric acid in a suitable solvent such as chloroform, dichloromethane, carbon tetrachloride, acetonitrile, nitromethane, acetic acid or acetic acid anhydride from -20 °C to under heating for several minutes to several tens hours.
A compound having a substituted amino group can be prepared by reacting a compound having a halogen group with a substituted imine compound in the presence of tris(dibenzylideneacetone) dipalladium, palladium acetate or the like and a phosphine ligand described in the above (4) in a suitable solvent such as tetrahydrofuran, toluene or xylene.
A compound having an amino group can be prepared by subjecting a compound having a nitro group to a catalytic reduction using a catalyst such as 10% palladium/carbon in a suitable solvent such as tetrahydrofuran, ethyl acetate or methanol.

In the case that a substituent which inhibits a reaction, e.g. hydroxy, mercapto, amino, formyl, carbonyl and carboxy, exists in any of the above steps, the substituent may be preliminarily protected by, for example, the method described in "Protective Groups in Organic Synthesis", and the protecting group may be removed at a desired step.

Among Compound (I) or Compound (I') of the present invention, the following compounds are especially preferable.

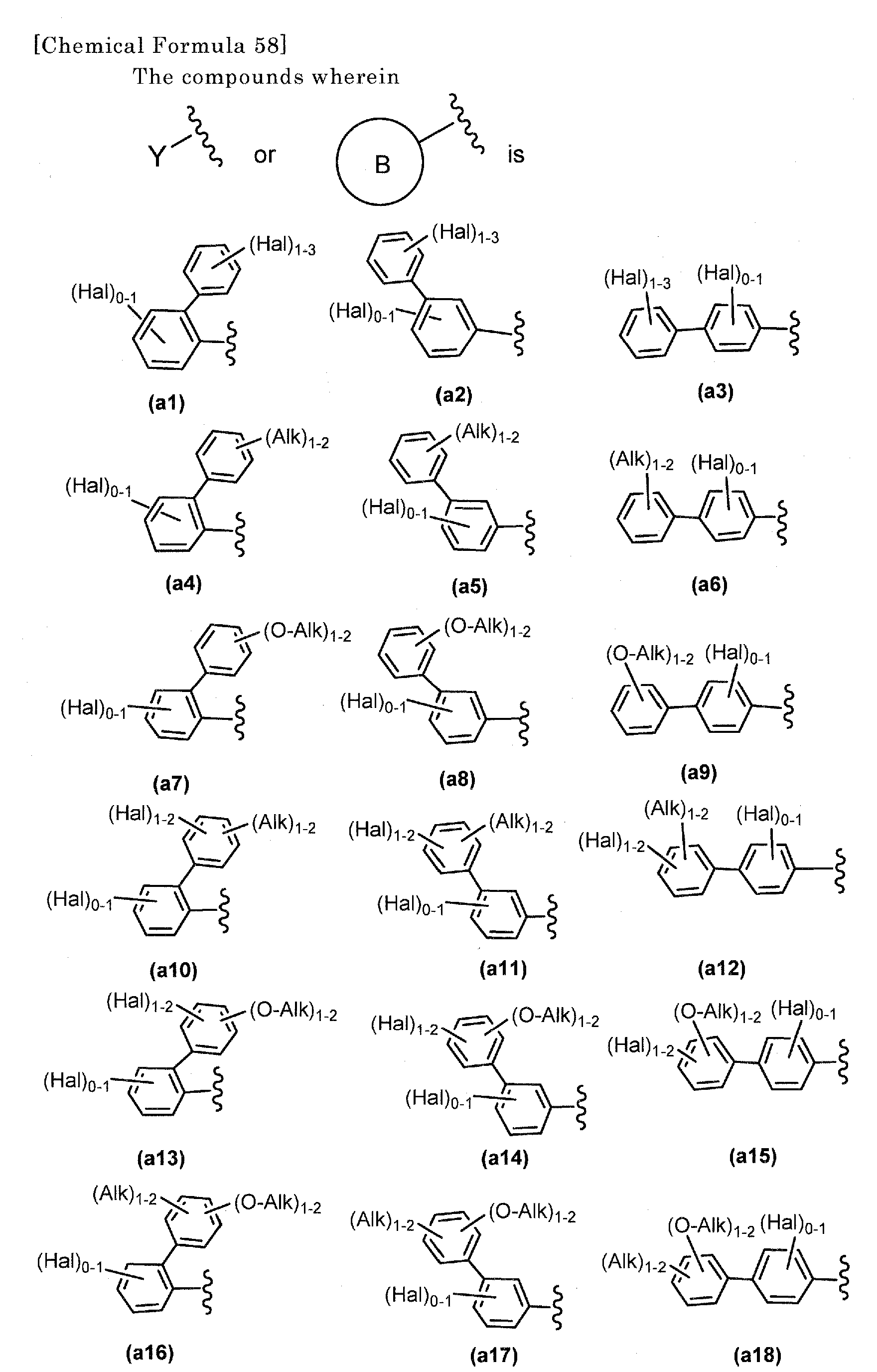

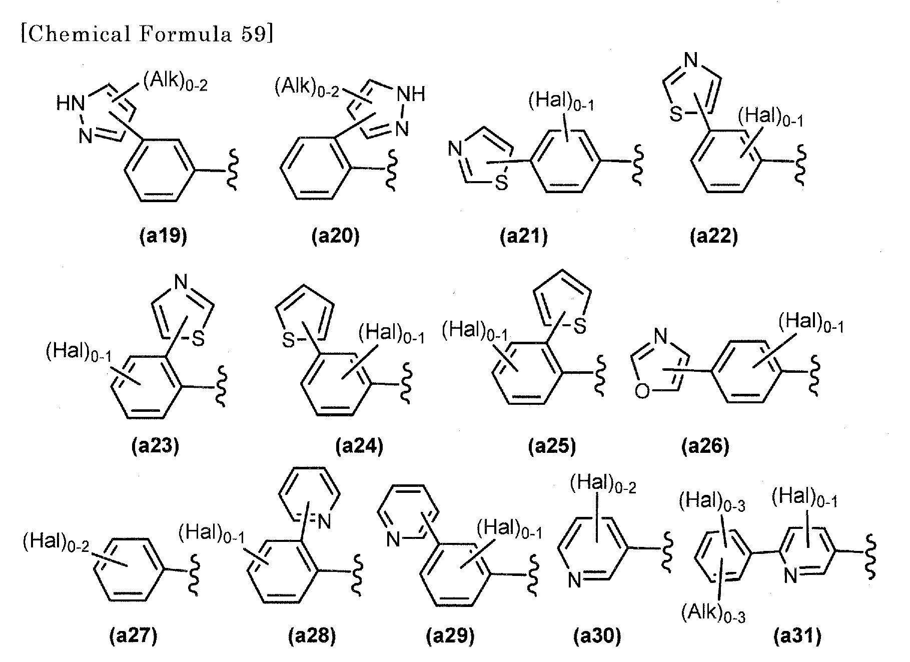

wherein Alk is alkyl, preferably methyl, ethyl, n-propyl, isopropyl or t-butyl, Hal is halogen, preferably fluorine or chlorine, each Alk and each Hal may be the same or different and each -Alk, each -O-Alk and -Hal may substitute to -NH- in the ring.

The compounds wherein 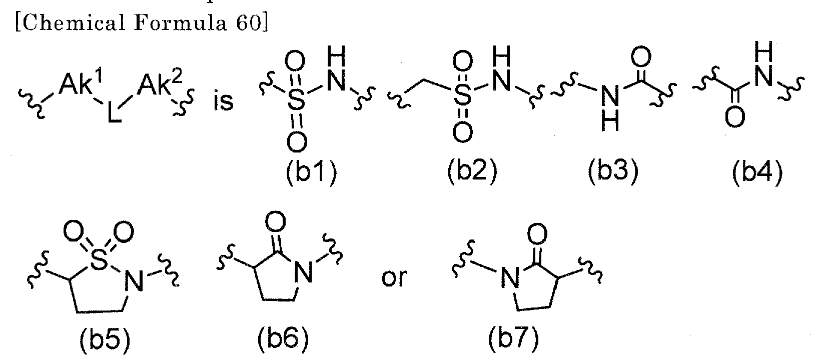 wherein the above group corresponding to -AK¹-SO₂ N(R⁵)-AK² - of Compound (I) corresponds to the group other than group represented by Y in X-,
or
Compounds wherein 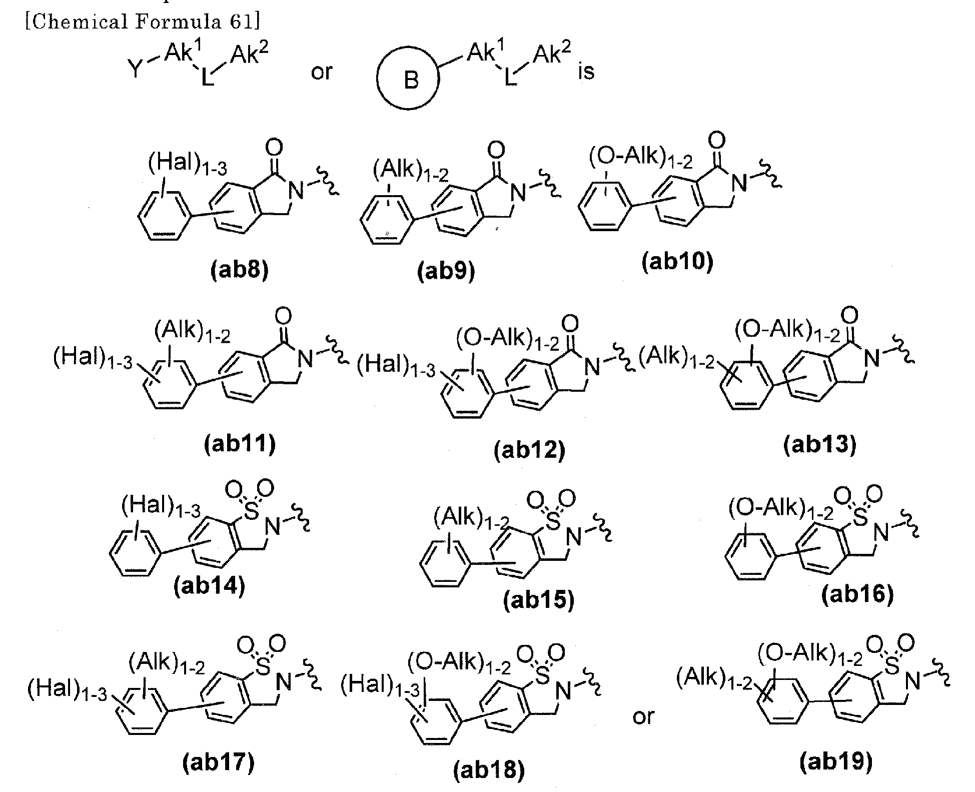 wherein the above group corresponding to Y-AK¹ -SO₂ N(R⁵)-AK² - of Compound (I) corresponds to the group represented by X,

Compounds wherein 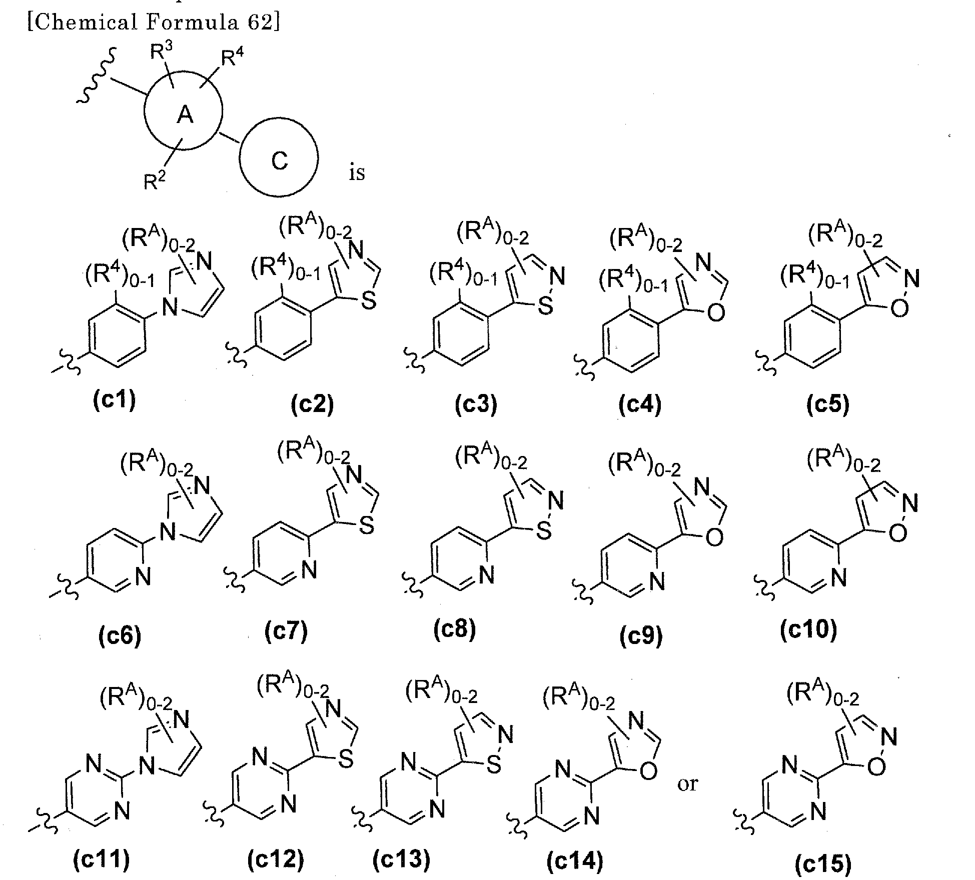 wherein R⁴ is halogen, preferably fluorine or chlorine, alkyl, preferably methyl, alkoxy, preferably methoxy or ethoxy, halogenoalkyl, preferably trifluoromethyl, or halogenoalkoxy, preferably trifluoromethoxy, R^{A} is halogen, preferably fluorine or chlorine, alkyl, preferably methyl, or halogenoalkyl, preferably trifluoromethyl, each R⁴ and each R^{A} may be the same or different and each R⁴ and each R^{A} may attach to -NH-in the ring,

In the formula (I) or (I'), compounds wherein the combination of 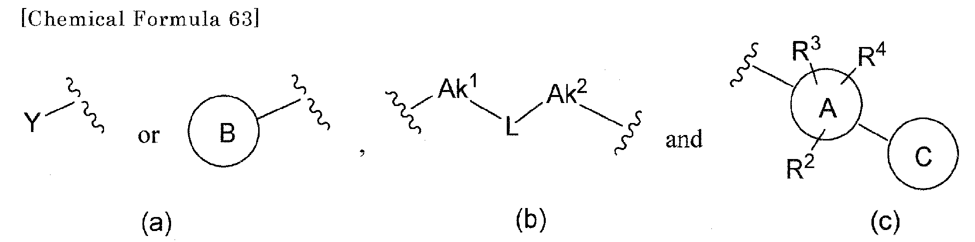 (a, b, c) are as follows:

Combination of (a,b,c)=(a1,b1,c1),(a1,b1,c2),(a1,b1,c3),(a1,b1,c4),(a1,b1,c5),(a1,b1,c6), (a1,b1,c7),(a1,b1,c8),(a1,b1,c9),(a1,b1,c10),(a1,b1,c11),(a1,b1,c12),(a1,b1,c13),(a1,b1,c14),( a1,b1,c15),(a1,b2,c1),(a1,b2,c2),(a1,b2,c3),(a1,b2,c4),(a1,b2,c5),(a1,b2,c6),(a1,b2,c7),(a1,b2, c8),(a1,b2,c9),(a1,b2,c10),(a1,b2,c11),(a1,b2,c12),(a1,b2,c13),(a1,b2,c14),(a1,b2,c15),(a1,b3, c1),(a1,b3,c2),(a1,b3,c3),(a1,b3,c4),(a1,b3,c5),(a1,b3,c6),(a1,b3,c7),(a1,b3,c8),(a1,b3,c9),(a1, b3,c10),(a1,b3,c11),(a1,b3,c12),(a1,b3,c13),(a1,b3,c14),(a1,b3,c15),(a1,b4,c1),(a1,b4,c2),(a1, b4,c3),(a1,b4,c4),(a1,b4,c5),(a1,b4,c6),(a1,b4,c7),(a1,b4,c8),(a1,b4,c9),(a1,b4,c10),(a1,b4,c1 1),(a1,b4,c12),(a1,b4,c13),(a1,b4,c14),(a1,b4,c15),(a1,b5,c1),(a1,b5,c2),(a1,b5,c3),(a1,b5,c4), (a1,b5,c5),(a1,b5,c6),(a1,b5,c7),(a1,b5,c8),(a1,b5,c9),(a1,b5,c10),(a1,b5,c11),(a1,b5,c12),(a1, b5,c13),(a1,b5,c14),(a1,b5,c15),(a1,b6,c1),(a1,b6,c2),(a1,b6,c3),(a1,b6,c4),(a1,b6,c5),(a1,b6, c6);(a1,b6,c7),(a1,b6,c8),(a1,b6,c9),(a1,b6,c10),(a1,b6,c11),(a1,b6,c12),(a1,b6,c13),(a1,b6,c1 4),(a1,b6,c15),(a1,b7,c1),(a1,b7,c2),(a1,b7,c3),(a1,b7,c4),(a1,b7,c5),(a1,b7,c6),(a1,b7,c7),(a1 ,b7,c8),(a1,b7,c9),(a1,b7,c10),(a1,b7,c11),(a1,b7,c12),(a1,b7,c13),(a1,b7,c14),(a1,b7,c15),(a2 ,b1,c1),(a2,b1,c2),(a2,b1,c3),(a2,b1,c4),(a2,b1,c5),(a2,b1,c6),(a2,b1,c7),(a2,b1,c8),(a2,b1,c9), (a2,b1,c10),(a2,b1,c11),(a2,b1,c12),(a2,b1,c13),(a2,b1,c14),(a2,b1,c15),(a2,b2,c1),(a2,b2,c2), (a2,b2,c3),(a2,b2,c4),(a2,b2,c5),(a2,b2,c6),(a2,b2,c7),(a2,b2,c8),(a2,b2,c9),(a2,b2,c10),(a2,b2 ,c11),(a2,b2,c12),(a2,b2,c13),(a2,b2,c14),(a2,b2,c15),(a2,b3,c1),(a2,b3,c2),(a2,b3,c3),(a2,b3, c4),(a2,b3,c5),(a2,b3,c6),(a2,b3,c7),(a2,b3,c8),(a2,b3,c9),(a2,b3,c10),(a2,b3,c11),(a2,b3,c12), (a2,b3,c13),(a2,b3,c14),(a2,b3,c15),(a2,b4,c1),(a2,b4,c2),(a2,b4,c3),(a2,b4,c4),(a2,b4,c5),(a2, b4,c6),(a2,b4,c7),(a2,b4,c8),(a2,b4,c9),(a2,b4,c10),(a2,b4,c11),(a2,b4,c12),(a2,b4,c13),(a2,b4 ,c14),(a2,b4,c15),(a2,b5,c1),(a2,b5,c2),(a2,b5,c3),(a2,b5,c4),(a2,b5,c5),(a2,b5,c6),(a2,b5,c7),( a2,b5,c8),(a2,b5,c9),(a2,b5,c10),(a2,b5,c11),(a2,b5,c12),(a2,b5,c13),(a2,b5,c14),(a2,b5,c15),( a2,b6,c1),(a2,b6,c2),(a2,b6,c3),(a2,b6,c4),(a2,b6,c5),(a2,b6,c6),(a2,b6,c7),(a2,b6,c8),(a2,b6,c 9),(a2,b6,c10),(a2,b6,c11),(a2,b6,c12),(a2,b6,c13),(a2,b6,c14),(a2,b6,c15),(a2,b7,c1),(a2,b7,c 2),(a2,b7,c3),(a2,b7,c4),(a2,b7,c5),(a2,b7,c6),(a2,b7,c7),(a2,b7,c8),(a2,b7,c9),(a2,b7,c10),(a2 ,b7,c11),(a2,b7,c12),(a2,b7,c13),(a2,b7,c14),(a2,b7,c15),(a3,b1,c1),(a3,b1,c2),(a3,b1,c3),(a3, b1,c4),(a3,b1,c5),(a3,b1,c6),(a3,b1,c7),(a3,b1,c8),(a3,b1,c9),(a3,b1,c10),(a3,b1,c11),(a3,b1,c 12),(a3,b1,c13),(a3,b1,c14),(a3,b1,c15),(a3,b2,c1),(a3,b2,c2),(a3,b2,c3),(a3,b2,c4),(a3,b2,c5), (a3,b2,c6),(a3,b2,c7),(a3,b2,c8),(a3,b2,c9),(a3,b2,c10),(a3,b2,c11),(a3,b2,c12),(a3,b2,c13),(a 3,b2,c14),(a3,b2,c15),(a3,b3,c1),(a3,b3,c2),(a3,b3,c3),(a3,b3,c4),(a3,b3,c5),(a3,b3,c6),(a3,b3, c7),(a3,b3,c8),(a3,b3,c9),(a3,b3,c10),(a3,b3,c11),(a3,b3,c12),(a3,b3,c13),(a3,b3,c14),(a3,b3,c 15),(a3,b4,c1),(a3,b4,c2),(a3,b4,c3),(a3,b4,c4),(a3,b4,c5),(a3,b4,c6),(a3,b4,c7),(a3,b4,c8),(a3 ,b4,c9),(a3,b4,c10),(a3,b4,c11),(a3,b4,c12),(a3,b4,c13),(a3,b4,c14),(a3,b4,c15),(a3,b5,c1),(a3 ,b5,c2),(a3,b5,c3),(a3,b5,c4),(a3,b5,c5),(a3,b5,c6),(a3,b5,c7),(a3,b5,c8),(a3,b5,c9),(a3,b5,c10 ),(a3,b5,c11),(a3,b5,c12),(a3,b5,c13),(a3,b5,c14),(a3,b5,c15),(a3,b6,c1),(a3,b6,c2),(a3,b6,c3), (a3,b6,c4),(a3,b6,c5),(a3,b6,c6),(a3,b6,c7),(a3,b6,c8),(a3,b6,c9),(a3,b6,c10),(a3,b6,c11),(a3,b 6,c12),(a3,b6,c13),(a3,b6,c14),(a3,b6,c15),(a3,b7,c1),(a3,b7,c2),(a3,b7,c3),(a3,b7,c4),(a3,b7, c5),(a3,b7,c6),(a3,b7,c7),(a3,b7,c8),(a3,b7,c9),(a3,b7,c10),(a3,b7,c11),(a3,b7,c12),(a3,b7,c13 ),(a3,b7,c14),(a3,b7,c15),(a4,b1,c1),(a4,b1,c2),(a4,b1,c3),(a4,b1,c4),(a4,b1,c5),(a4,b1,c6),(a4 ,b1,c7),(a4,b1,c8),(a4,b1,c9),(a4,b1,c10),(a4,b1,c11),(a4,b1,c12),(a4,b1,c13),(a4,b1,c14),(a4, b1,c15),(a4,b2,c1),(a4,b2,c2),(a4,b2,c3),(a4,b2,c4),(a4,b2,c5),(a4,b2,c6),(a4,b2,c7),(a4,b2,c8) ,(a4,b2,c9),(a4,b2,c10),(a4,b2,c11),(a4,b2,c12),(a4,b2,c13),(a4,b2,c14),(a4,b2,c15),(a4,b3,c1) ,(a4,b3,c2),(a4,b3,c3),(a4,b3,c4),(a4,b3,c5),(a4,b3,c6),(a4,b3,c7),(a4,b3,c8),(a4,b3,c9),(a4,b3 ,c10),(a4,b3,c11),(a4,b3,c12),(a4,b3,c13),(a4,b3,c14),(a4,b3,c15),(a4,b4,c1),(a4,b4,c2),(a4,b4 ,c3),(a4,b4,c4),(a4,b4,c5),(a4,b4,c6),(a4,b4,c7),(a4,b4,c8),(a4,b4,c9),(a4,b4,c10),(a4,b4,c11),( a4,b4,c12),(a4,b4,c13),(a4,b4,c14),(a4,b4,c15),(a4,b5,c1),(a4,b5,c2),(a4,b5,c3),(a4,b5,c4),(a4 ,b5,c5),(a4,b5,c6),(a4,b5,c7),(a4,b5,c8),(a4,b5,c9),(a4,b5,c10),(a4,b5,c11),(a4,b5,c12),(a4,b5, c13),(a4,b5,c14),(a4,b5,c15),(a4,b6,c1),(a4,b6,c2),(a4,b6,c3),(a4,b6,c4),(a4,b6,c5),(a4,b6,c6), (a4,b6,c7),(a4,b6,c8),(a4,b6,c9),(a4,b6,c10),(a4,b6,c11),(a4,b6,c12),(a4,b6,c13),(a4,b6,c14),( a4,b6,c15),(a4,b7,c1),(a4,b7,c2),(a4,b7,c3),(a4,b7,c4),(a4,b7,c5),(a4,b7,c6),(a4,b7,c7),(a4,b7, c8),(a4,b7,c9),(a4,b7,c10),(a4,b7,c11),(a4,b7,c12),(a4,b7,c13),(a4,b7,c14),(a4,b7,c15),(a5,b1, c1),(a5,b1,c2),(a5,b1,c3),(a5,b1,c4),(a5,b1,c5),(a5,b1,c6),(a5,b1,c7),(a5,b1,c8),(a5,b1,c9),(a5, b1,c10),(a5,b1,c11),(a5,b1,c12),(a5,b1,c13),(a5,b1,c14),(a5,b1,c15),(a5,b2,c1),(a5,b2,c2),(a5, b2,c3),(a5,b2,c4),(a5,b2,c5),(a5,b2,c6),(a5,b2,c7),(a5,b2,c8),(a5,b2,c9),(a5,b2,c10),(a5,b2,c1 1),(a5,b2,c12),(a5,b2,c13),(a5,b2,c14),(a5,b2,c15),(a5,b3,c1),(a5,b3,c2),(a5,b3,c3),(a5,b3,c4), (a5,b3,c5),(a5,b3,c6),(a5,b3,c7),(a5,b3,c8),(a5,b3,c9),(a5,b3,c10),(a5,b3,c11),(a5,b3,c12),(a5, b3,c13),(a5,b3,c14),(a5,b3,c15),(a5,b4,c1),(a5,b4,c2),(a5,b4,c3),(a5,b4,c4),(a5,b4,c5),(a5,b4, c6),(a5,b4,c7),(a5,b4,c8),(a5,b4,c9),(a5,b4,c10),(a5,b4,c11),(a5,b4,c12),(a5,b4,c13),(a5,b4,c1 4),(a5,b4,c15),(a5,b5,c1),(a5,b5,c2),(a5,b5,c3),(a5,b5,c4),(a5,b5,c5),(a5,b5,c6),(a5,b5,c7),(a5 ,b5,c8),(a5,b5,c9),(a5,b5,c10),(a5,b5,c11),(a5,b5,c12),(a5,b5,c13),(a5,b5,c14),(a5,b5,c15),(a5 ,b6,c1),(a5,b6,c2),(a5,b6,c3),(a5,b6,c4),(a5,b6,c5),(a5,b6,c6),(a5,b6,c7),(a5,b6,c8),(a5,b6,c9), (a5,b6,c10),(a5,b6,c11),(a5,b6,c12),(a5,b6,c13),(a5,b6,c14),(a5,b6,c15),(a5,b7,c1),(a5,b7,c2), (a5,b7,c3),(a5,b7,c4),(a5,b7,c5),(a5,b7,c6),(a5,b7,c7),(a5,b7,c8),(a5,b7,c9),(a5,b7,c10),(a5,b7 ,c11),(a5,b7,c12),(a5,b7,c13),(a5,b7,c14),(a5,b7,c15),

(a6,b1,c1),(a6,b1,c2),(a6,b1,c3),(a6,b1,c4),(a6,b1,c5),(a6,b1,c6),(a6,b1,c7),(a6,b1,c8),(a6,b1, c9),(a6,b1,c10),(a6,b1,c11),(a6,b1,c12),(a6,b1,c13),(a6,b1,c14),(a6,b1,c15),(a6,b2,c1),(a6,b2, c2),(a6,b2,c3),(a6,b2,c4),(a6,b2,c5),(a6,b2,c6),(a6,b2,c7),(a6,b2,c8),(a6,b2,c9),(a6,b2,c10),(a 6,b2,c11),(a6,b2,c12),(a6,b2,c13),(a6,b2,c14),(a6,b2,c15),(a6,b3,c1),(a6,b3,c2),(a6,b3,c3),(a6 ,b3,c4),(a6,b3,c5),(a6,b3,c6),(a6,b3,c7),(a6,b3,c8),(a6,b3,c9),(a6,b3,c10),(a6,b3,c11),(a6,b3,c 12),(a6,b3,c13),(a6,b3,c14),(a6,b3,c15),(a6,b4,c1),(a6,b4,c2),(a6,b4,c3),(a6,b4,c4),(a6,b4,c5), (a6,b4,c6),(a6,b4,c7),(a6,b4,c8),(a6,b4,c9),(a6,b4,c10),(a6,b4,c11),(a6,b4,c12),(a6,b4,c13),(a 6,b4,c14),(a6,b4,c15),(a6,b5,c1),(a6,b5,c2),(a6,b5,c3),(a6,b5,c4),(a6,b5,c5),(a6,b5,c6),(a6,b5, c7),(a6,b5,c8),(a6,b5,c9),(a6,b5,c10),(a6,b5,c11),(a6,b5,c12),(a6,b5,c13),(a6,b5,c14),(a6,b5,c 15),(a6,b6,c1),(a6,b6,c2),(a6,b6,c3),(a6,b6,c4),(a6,b6,c5),(a6,b6,c6),(a6,b6,c7),(a6,b6,c8),(a6 ,b6,c9),(a6,b6,c10),(a6,b6,c11),(a6,b6,c12),(a6,b6,c13),(a6,b6,c14),(a6,b6,c15),(a6,b7,c1),(a6 ,b7,c2),(a6,b7,c3),(a6,b7,c4),(a6,b7,c5),(a6,b7,c6),(a6,b7,c7),(a6,b7,c8),(a6,b7,c9),(a6,b7,c10 ),(a6,b7,c11),(a6,b7,c12),(a6,b7,c13),(a6,b7,c14),(a6,b7,c15),(a7,b1,c1),(a7,b1,c2),(a7,b1,c3), (a7,b1,c4),(a7,b1,c5),(a7,b1,c6),(a7,b1,c7),(a7,b1,c8),(a7,b1,c9),(a7,b1,c10),(a7,b1,c11),(a7,b 1,c12),(a7,b1,c13),(a7,b1,c14),(a7,b1,c15),(a7,b2,c1),(a7,b2,c2),(a7,b2,c3),(a7,b2,c4),(a7,b2, c5),(a7,b2,c6),(a7,b2,c7),(a7,b2,c8),(a7,b2,c9),(a7,b2,c10),(a7,b2,c11),(a7,b2,c12),(a7,b2,c13 ),(a7,b2,c14),(a7,b2,c15),(a7,b3,c1),(a7,b3,c2),(a7,b3,c3),(a7,b3,c4),(a7,b3,c5),(a7,b3,c6),(a7 ,b3,c7),(a7,b3,c8),(a7,b3,c9),(a7,b3,c10),(a7,b3,c11),(a7,b3,c12),(a7,b3,c13),(a7,b3,c14),(a7, b3,c15),(a7,b4,c1),(a7,b4,c2),(a7,b4,c3),(a7,b4,c4),(a7,b4,c5),(a7,b4,c6),(a7,b4,c7),(a7,b4,c8) ,(a7,b4,c9),(a7,b4,c10),(a7,b4,c11),(a7,b4,c12),(a7,b4,c13),(a7,b4,c14),(a7,b4,c15),(a7,b5,c1) ,(a7,b5,c2),(a7,b5,c3),(a7,b5,c4),(a7,b5,c5),(a7,b5,c6),(a7,b5,c7),(a7,b5,c8),(a7,b5,c9),(a7,b5 ,c10),(a7,b5,c11),(a7,b5,c12),(a7,b5,c13),(a7,b5,c14),(a7,b5,c15),(a7,b6,c1),(a7,b6,c2),(a7,b6 ,c3),(a7,b6,c4),(a7,b6,c5),(a7,b6,c6),(a7,b6,c7),(a7,b6,c8),(a7,b6,c9),(a7,b6,c10),(a7,b6,c11),( a7,b6,c12),(a7,b6,c13),(a7,b6,c14),(a7,b6,c15),(a7,b7,c1),(a7,b7,c2),(a7,b7,c3),(a7,b7,c4),(a7 ,b7,c5),(a7,b7,c6),(a7,b7,c7),(a7,b7,c8),(a7,b7,c9),(a7,b7,c10),(a7,b7,c11),(a7,b7,c12),(a7,b7, c13),(a7,b7,c14),(a7,b7,c15),(a8,b1,c1),(a8,b1,c2),(a8,b1,c3),(a8,b1,c4),(a8,b1,c13),(a8,b1,c6), (a8,b1,c7),(a8,b1,c8),(a8,b1,c9),(a8,b1,c10),(a8,b1,c11),(a8,b1,c12),(a8,b1,c13),(a8,b1,c14),( a8,b1,c15),(a8,b2,c1),(a8,b2,c2),(a8,b2,c3),(a8,b2,c4),(a8,b2,c5),(a8,b2,c6),(a8,b2,c7),(a8,b2, c8),(a8,b2,c9),(a8,b2,c10),(a8,b2,c11),(a8,b2,c12),(a8,b2,c13),(a8,b2,c14),(a8,b2,c15),(a8,b3, c1),(a8,b3,c2),(a8,b3,c3),(a8,b3,c4),(a8,b3,c5),(a8,b3,c6),(a8,b3,c7),(a8,b3,c8),(a8,b3,c9),(a8, b3,c10),(a8,b3,c11),(a8,b3,c12),(a8,b3,c13),(a8,b3,c14),(a8,b3,c15),(a8,b4,c1),(a8,b4,c2),(a8, b4,c3),(a8,b4,c4),(a8,b4,c,5),(a8,b4,c6),(a8,b4,c7),(a8,b4,c8),(a8,b4,c9),(a8,b4,c10),(a8,b4,c1 1),(a8,b4,c12),(a8,b4,c13),(a8,b4,c14),(a8,b4,c15),(a8,b5,c1),(a8,b5,c2),(a8,b5,c3),(a8,b5,c4), (a8,b5,c5),(a8,b5,c6),(a8,b5,c7),(a8,b5,c8),(a8,b5,c9),(a8,b5,c10),(a8,b5,c11),(a8,b5,c12),(a8, b5,c13),(a8,b5,c14),(a8,b5,c15),(a8,b6,c1),(a8,b6,c2),(a8,b6,c3),(a8,b6,c4),(a8,b6,c5),(a8,b6, c6),(a8,b6,c7),(a8,b6,c8),(a8,b6,c9),(a8,b6,c10),(a8,b6,c11),(a8,b6,c12),(a8,b6,c13),(a8,b6,c1 4),(a8,b6,c15),(a8,b7,c1),(a8,b7,c2),(a8,b7,c3),(a8,b7,c4),(a8,b7,c5),(a8,b7,c6),(a8,b7,c7),(a8 ,b7,c8),(a8,b7,c9),(a8,b7,c10),(a8,b7,c11),(a8,b7,c12),(a8,b7,c13),(a8,b7,c14),(a8,b7,c15),(a9 ,b1,c1),(a9,b1,c2),(a9,b1,c3),(a9,b1,c4),(a9,b1,c5),(a9,b1,c6),(a9,b1,c7),(a9,b1,c8),(a9,b1,c9), (a9,b1,c10),(a9,b1,c11),(a9,b1,c12),(a9,b1,c13),(a9,b1,c14),(a9,b1,c15),(a9,b2,c1),(a9,b2,c2), (a9,b2,c3),(a9,b2,c4),(a9,b2,c5),(a9,b2,c6),(a9,b2,c7),(a9,b2,c8),(a9,b2,c9),(a9,b2,c10),(a9,b2 ,c11),(a9,b2,c12),(a9,b2,c13),(a9,b2,c14),(a9,b2,c15),(a9,b3,c1),(a9,b3,c2),(a9,b3,c3),(a9,b3, c4),(a9,b3,c5),(a9,b3,c6),(a9,b3,c7),(a9,b3,c8),(a9,b3,c9),(a9,b3,c10),(a9,b3,c11),(a9,b3,c12), (a9,b3,c13),(a9,b3,c14),(a9,b3,c15),(a9,b4,c1),(a9,b4,c2),(a9,b4,c3),(a9,b4,c4),(a9,b4,c5),(a9, b4,c6),(a9,b4,c7),(a9,b4,c8),(a9,b4,c9),(a9,b4,c10),(a9,b4,c11),(a9,b4,c12),(a9,b4,c13),(a9,b4 ,c14),(a9,b4,c15),(a9,b5,c1),(a9,b5,c2),(a9,b5,c3),(a9,b5,c4),(a9,b5,c5),(a9,b5,c6),(a9,b5,c7),( a9,b5,c8),(a9,b5,c9),(a9,b5,c10),(a9,b5,c11),(a9,b5,c12),(a9,b5,c13),(a9,b5,c14),(a9,b5,c15),( a9,b6,c1),(a9,b6,c2),(a9,b6,c3),(a9,b6,c4),(a9,b6,c5),(a9,b6,c6),(a9,b6,c7),(a9,b6,c8),(a9,b6,c 9),(a9,b6,c10),(a9,b6,c11),(a9,b6,c12),(a9,b6,c13),(a9,b6,c14),(a9,b6,c15),(a9,b7,c1),(a9,b7,c 2),(a9,b7,c3),(a9,b7,c4),(a9,b7,c5),(a9,b7,c6),(a9,b7,c7),(a9,b7,c8),(a9,b7,c9),(a9,b7,c10),(a9 ,b7,c11),(a9,b7,c12),(a9,b7,c13),(a9,b7,c14),(a9,b7,c15),(a10,b1,c1),(a10,b1,c2),(a10,b1,c3),( a10,b1,c4),(a10,b1,c5),(a10,b1,c6),(a10,b1,c7),(a10,b1,c8),(a10,b1,c9),(a10,b1,c10),(a10,b1, c11),(a10,b1,c12),(a10,b1,c13),(a10,b1,c14),(a10,b1,c15),(a10,b2,c1),(a10,b2,c2),(a10,b2,c3) ,(a10,b2,c4),(a10,b2,c5),(a10,b2,c6),(a10,b2,c7),(a10,b2,c8),(a10,b2,c9),(a10,b2,c10),(a10,b2 ,c11),(a10,b2,c12),(a10,b2,c13),(a10,b2,c14),(a10,b2,c15),(a10,b3,c1),(a10,b3,c2),(a10,b3,c3 ),(a10,b3,c4),(a10,b3,c5),(a10,b3,c6),(a10,b3,c7),(a10,b3,c8),(a10,b3,c9),(a10,b3,c10),(a10,b 3,c11),(a10,b3,c12),(a10,b3,c13),(a10,b3,c14),(a10,b3,c15),(a10,b4,c1),(a10,b4,c2),(a10,b4,c 3),(a10,b4,c4),(a10,b4,c5),(a10,b4,c6),(a10,b4,c7),(a10,b4,c8),(a10,b4,c9),(a10,b4,c10),(a10, b4,c11),(a10,b4,c12),(a10,b4,c13),(a10,b4,c14),(a10,b4,c15),(a10,b5,c1),(a10,b5,c2),(a10,b5, c3),(a10,b5,c4),(a10,b5,c5),(a10,b5,c6),(a10,b5,c6),(a10,b5,c8),(a10,b5,c9),(a10,b5,c10),(a10 ,b5,c11),(a10,b5,c12),(a10,b5,c13),(a10,b5,c14),(a10,b5,c15),(a10,b6,c1),(a10,b6,c2),(a10,b6 ,c3),(a10,b6,c4),(a10,b6,c5),(a10,b6,c6),(a10,b6,c7),(a10,b6,c8),(a10,b6,c9),(a10,b6,c10),(a1 0,b6,c11),(a10,b6,c12),(a10,b6,c13),(a10,b6,c14),(a10,b6,c15),(a10,b7,c1),(a10,b7,c2),(a10,b 7,c3),(a10,b7,c4),(a10,b7,c5),(a10,b7,c6),(a10,b7,c7),(a10,b7,c8),(a10,b7,c9),(a10,b7,c10),(a 10,b7,c11),(a10,b7,c12),(a10,b7,c13),(a10,b7,c14),(a10,b7,c15),

(a11,b1,c1),(a11,b1,c2),(a11,b1,c3),(a11,b1,c4),(a11,b1,c5),(a11,b1,c6),(a11,b1,c7),(a11,b1,c 8),(a11,b1,c9),(a11,b1,c10),(a11,b1,c11),(a11,b1,c12),(a11,b1,c13),(a11,b1,c14),(a11,b1,c15) ,(a11,b2,c1),(a11,b2,c2),(a11,b2,c3),(a11,b2,c4),(a11,b2,c5),(a11,b2,c6),(a11,b2,c7),(a11,b2, c8),(a11,b2,c9),(a11,b2,c10),(a11,b2,c11),(a11,b2,c12),(a11,b2,c13),(a11,b2,c14),(a11,b2,c15 ),(a11,b3,c1),(a11,b3,c2),(a11,b3,c3),(a11,b3,c4),(a11,b3,c5),(a11,b3,c6),(a11,b3,c7),(a11,b3 ,c8),(a11,b3,c9),(a11,b3,c10),(a11,b3,c11),(a11,b3,c12),(a11,b3,c13),(a11,b3,c14),(a11,b3,c1 5),(a11,b4,c1),(a11,b4,c2),(a11,b4,c3),(a11,b4,c4),(a11,b4,c5),(a11,b4,c6),(a11,b4,c7),(a11,b 4,c8),(a11,b4,c9),(a11,b4,c10),(a11,b4,c11),(a11,b4,c12),(a11,b4,c13),(a11,b4,c14),(a11,b4,c 15),(a11,b5,c1),(a11,b5,c2),(a11,b5,c3),(a11,b5,c4),(a11,b5,c5),(a11,b5,c6),(a11,b5,c7),(a11, b5,c8),(a11,b5,c9),(a11,b5,c10),(a11,b5,c11),(a11,b5,c12),(a11,b5,c13),(a11,b5,c14),(a11,b5, c15),(a11,b6,c1),(a11,b6,c2),(a11,b6,c3),(a11,b6,c4),(a11,b6,c5),(a11,b6,c6),(a11,b6,c7),(a11 ,b6,c8),(a11,b6,c9),(a11,b6,c10),(a11,b6,c11),(a11,b6,c12),(a11,b6,c13),(a11,b6,c14),(a11,b6 ,c15),(a11,b7,c1),(a11,b7,c2),(a11,b7,c3),(a11,b7,c4),(a11,b7,c5),(a11,b7,c6),(a11,b7,c7),(a1 1,b7,c8),(a11,b7,c9),(a11,b7,c10),(a11,b7,c11),(a11,b7,c12),(a11,b7,c13),(a11,b7,c14),(a11,b 7,c15),(a12,b1,c1),(a12,b1,c2),(a12,b1,c3),(a12,b1,c4),(a12,b1,c5),(a12,b1,c6),(a12,b1,c7),(a 12,b1,c8),(a12,b1,c9),(a12,b1,c10),(a12,b1,c11),(a12,b1,c12),(a12,b1,c13),(a12,b1,c14),(a12, b1,c15),(a12,b2,c1),(a12,b2,c2),(a12,b2,c3),(a12,b2,c4),(a12,b2,c5),(a12,b2,c6),(a12,b2,c7),( a12,b2,c8),(a12,b2,c9),(a12,b2,c10),(a12,b2,c11),(a12,b2,c12),(a12,b2,c13),(a12,b2,c14),(a1 2,b2,c15),(a12,b3,c1),(a12,b3,c2),(a12,b3,c3),(a12,b3,c4),(a12,b3,c5),(a12,b3,c6),(a12,b3,c7) ,(a12,b3,c8),(a12,b3,c9),(a12,b3,c10),(a12,b3,c11),(a12,b3,c12),(a12,b3,c13),(a12,b3,c14),(a 12,b3,c15),(a12,b4,c1),(a12,b4,c2),(a12,b4,c3),(a12,b4,c4),(a12,b4,c5),(a12,b4,c6),(a12,b4,c 7),(a12,b4,c8),(a12,b4,c9),(a12,b4,c10),(a12,b4,c11),(a12,b4,c12),(a12,b4,c13),(a12,b4,c14),( a12,b4,c15),(a12,b5,c1),(a12,b5,c2),(a12,b5,c3),(a12,b5,c4),(a12,b5,c5),(a12,b5,c6),(a12,b5, c7),(a12,b5,c8),(a12,b5,c9),(a12,b5,c10),(a12,b5,c11),(a12,b5,c12),(a12,b5,c13),(a12,b5,c14) ,(a12,b5,c15),(a12,b6,c1),(a12,b6,c2),(a12,b6,c3),(a12,b6,c4),(a12,b6,c5),(a12,b6,c6),(a12,b6 ,c7),(a12,b6,c8),(a12,b6,c9),(a12,b6,c10),(a12,b6,c11),(a12,b6,c12),(a12,b6,c13),(a12,b6,c14 ),(a12,b6,c15),(a12,b7,c1),(a12,b7,c2),(a12,b7,c3),(a12,b7,c4),(a12,b7,c5),(a12,b7,c6),(a12,b 7,c7),(a12,b7,c8),(a12,b7,c9),(a12,b7,c10),(a12,b7,c11),(a12,b7,c12),(a12,b7,c13),(a12,b7,c1 4),(a12,b7,c15),(a13,b1,c1),(a13,b1,c2),(a13,b1,c3),(a13,b1,c4),(a13,b1,c5),(a13,b1,c6),(a13, b1,c7),(a13,b1,c8),(a13,b1,c9),(a13,b1,c10),(a13,b1,c11),(a13,b1,c12),(a13,b1,c13),(a13,b1,c 14),(a13,b1,c15),(a13,b2,c1),(a13,b2,c2),(a13,b2,c3),(a13,b2,c4),(a13,b2,c5),(a13,b2,c6),(a13 ,b2,c7),(a13,b2,c8),(a13,b2,c9),(a13,b2,c10),(a13,b2,c11),(a13,b2,c12),(a13,b2,c13),(a13,b2, c14),(a13,b2,c15),(a13,b3,c1),(a13,b3,c2),(a13,b3,c3),(a13,b3,c4),(a13,b3,c5),(a13,b3,c6),(a1 3,b3,c7),(a13,b3,c8),(a13,b3,c9),(a13,b3,c10),(a13,b3,c11),(a13,b3,c12),(a13,b3,c13),(a13,b3 ,c14),(a13,b3,c15),(a13,b4,c1),(a13,b4,c2),(a13,b4,c3),(a13,b4,c4),(a13,b4,c5),(a13,b4,c6),(a 13,b4,c7),(a13,b4,c8),(a13,b4,c9),(a13,b4,c10),(a13,b4,c11),(a13,b4,c12),(a13,b4,c13),(a13,b 4,c14),(a13,b4,c15),(a13,b5,c1),(a13,b5,c2),(a13,b5,c3),(a13,b5,c4),(a13,b5,c5),(a13,b5,c6),( a13,b5,c7),(a13,b5,c8),(a13,b5,c9),(a13,b5,c10),(a13,b5,c11),(a13,b5,c12),(a13,b5,c13),(a13, b5,c14),(a13,b5,c15),(a13,b6,c1),(a13,b6,c2),(a13,b6,c3),(a13,b6,c4),(a13,b6,c5),(a13,b6,c6), (a13,b6,c7),(a13,b6,c8),(a13,b6,c9),(a13,b6,c10),(a13,b6,c11),(a13,b6,c12),(a13,b6,c13),(a13 ,b6,c14),(a13,b6,c15),(a13,b7,c1),(a13,b7,c2),(a13,b7,c3),(a13,b7,c4),(a13,b7,c5),(a13,b7,c6) ,(a13,b7,c7),(a13,b7,c8),(a13,b7,c9),(a13,b7,c10),(a13,b7,c11),(a13,b7,c12),(a13,b7,c13),(a1 3,b7,c14),(a13,b7,c15),(a14,b1,c1),(a14,b1,c2),(a14,b1,c3),(a14,b1,c4),(a14,b1,c5),(a14,b1,c 6),(a14,b1,c7),(a14,b1,c8),(a14,b1,c9),(a14,b1,c10),(a14,b1,c11),(a14,b1,c12),(a14,b1,c13),(a 14,b1,c14),(a14,b1,c15),(a14,b2,c1),(a14,b2,c2),(a14,b2,c3),(a14,b2,c4),(a14,b2,c5),(a14,b2, c6),(a14,b2,c7),(a14,b2,c8),(a14,b2,c9),(a14,b2,c10),(a14,b2,c11),(a14,b2,c12),(a14,b2,c13),( a14,b2,c14),(a14,b2,c15),(a14,b3,c1),(a14,b3,c2),(a14,b3,c3),(a14,b3,c4),(a14,b3,c5),(a14,b3 ,c6),(a14,b3,c7),(a14,b3,c8),(a14,b3,c9),(a14,b3,c10),(a14,b3,c11),(a14,b3,c12),(a14,b3,c13), (a14,b3,c14),(a14,b3,c15),(a14,b4,c1),(a14,b4,c2),(a14,b4,c3),(a14,b4,c4),(a14,b4,c5),(a14,b 4,c6),(a14,b4,c7),(a14,b4,c8),(a14,b4,c9),(a14,b4,c10),(a14,b4,c11),(a14,b4,c12),(a14,b4,c13 ),(a14,b4,c14),(a14,b4,c15),(a14,b5,c1),(a14,b5,c2),(a14,b5,c3),(a14,b5,c4),(a14,b5,c5),(a14, b5,c6),(a14,b5,c7),(a14,b5,c8),(a14,b5,c9),(a14,b5,c10),(a14,b5,c11),(a14,b5,c12),(a14,b5,c1 3),(a14,b5,c14),(a14,b5,c15),(a14,b6,c1),(a14,b6,c2),(a14,b6,c3),(a14,b6,c4),(a14,b6,c5),(a14 ,b6,c6),(a14,b6,c7),(a14,b6,c8),(a14,b6,c9),(a14,b6,c10),(a14,b6,c11),(a14,b6,c12),(a14,b6,c 13),(a14,b6,c14),(a14,b6,c15),(a14,b7,c1),(a14,b7,c2),(a14,b7,c3),(a14,b7,c4),(a14,b7,c5),(a1 4,b7,c6),(a14,b7,c7),(a14,b7,c8),(a14,b7,c9),(a14,b7,c10),(a14,b7,c11),(a14,b7,c12),(a14,b7, c13),(a14,b7,c14),(a14,b7,c15),(a15,b1,c1),(a15,b1,c2),(a15,b1,c3),(a15,b1,c4),(a15,b1,c5),(a 15,b1,c6),(a15,b1,c7),(a15,b1,c8),(a15,b1,c9),(a15,b1,c10),(a15,b1,c11),(a15,b1,c12),(a15,b1 ,c13),(a15,b1,c14),(a15,b1,c15),(a15,b2,c1),(a15,b2,c2),(a15,b2,c3),(a15,b2,c4),(a15,b2,c5),( a15,b2,c6),(a15,b2,c7),(a15,b2,c8),(a15,b2,c9),(a15,b2,c10),(a15,b2,c11),(a15,b2,c12),(a15,b 2,c13),(a15,b2,c14),(a15,b2,c15),(a15,b3,c1),(a15,b3,c2),(a15,b3,c3),(a15,b3,c4),(a15,b3,c5), (a15,b3,c6),(a15,b3,c7),(a15,b3,c8),(a15,b3,c9),(a15,b3,c10),(a15,b3,c11),(a15,b3,c12),(a15, b3,c13),(a15,b3,c14),(a15,b3,c15),(a15,b4,c1),(a15,b4,c2),(a15,b4,c3),(a15,b4,c4),(a15,b4,c5 ),(a15,b4,c6),(a15,b4,c7),(a15,b4,c8),(a15,b4,c9),(a15,b4,c10),(a15,b4,c11),(a15,b4,c12),(a15 ,b4,c13),(a15,b4,c14),(a15,b4,c15),(a15,b5,c1),(a15,b5,c2),(a15,b5,c3),(a15,b5,c4),(a15,b5,c 5),(a15,b5,c6),(a15,b5,c7),(a15,b5,c8),(a15,b5,c9),(a15,b5,c10),(a15,b5,c11),(a15,b5,c12),(a1 5,b5,c13),(a15,b5,c14),(a15,b5,c15),(a15,b6,c1),(a15,b6,c2),(a15,b6,c3),(a15,b6,c4),(a15,b6, c5),(a15,b6,c6),(a15,b6,c7),(a15,b6,c8),(a15,b6,c9),(a15,b6,c10),(a15,b6,c11),(a15,b6,c12),(a 15,b6,c13),(a15,b6,c14),(a15,b6,c15),(a15,b7,c1),(a15,b7,c2),(a15,b7,c3),(a15,b7,c4),(a15,b7 ,c5),(a15,b7,c6),(a15,b7,c7),(a15,b7,c8),(a15,b7,c9),(a15,b7,c10),(a15,b7,c11),(a15,b7,c12),( a15,b7,c13),(a15,b7,c14),(a15,b7,c15),

(a16,b1,c1),(a16,b1,c2),(a16,b1,c3),(a16,b1,c4),(a16,b1,c5),(a16,b1,c6),(a16,b1,c7),(a16,b1,c 8),(a16,b1,c9),(a16,b1,c10),(a16,b1,c11),(a16,b1,c12),(a16,b1,c13),(a16,b1,c14),(a16,b1,c15) ,(a16,b2,c1),(a16,b2,c2),(a16,b2,c3),(a16,b2,c4),(a16,b2,c5),(a16,b2,c6),(a16,b2,c7),(a16,b2, c8),(a16,b2,c9),(a16,b2,c10),(a16,b2,c11),(a16,b2,c12),(a16,b2,c13),(a16,b2,c14),(a16,b2,c15 ),(a16,b3,c1),(a16,b3,c2),(a16,b3,c3),(a16,b3,c4),(a16,b3,c5),(a16,b3,c6),(a16,b3,c7),(a16,b3 ,c8),(a16,b3,c9),(a16,b3,c10),(a16,b3,c11),(a16,b3,c12),(a16,b3,c13),(a16,b3,c14),(a16,b3,c1 5),(a16,b4,c1),(a16,b4,c2),(a16,b4,c3),(a16,b4,c4),(a16,b4,c5),(a16,b4,c6),(a16,b4,c7),(a16,b 4,c8),(a16,b4,c9),(a16,b4,c10),(a16,b4,c11),(a16,b4,c12),(a16,b4,c13),(a16,b4,c14),(a16,b4,c 15),(a16,b5,c1),(a16,b5,c2),(a16,b5,c3),(a16,b5,c4),(a16,b5,c5),(a16,b5,c6),(a16,b5,c7),(a16, b5,c8),(a16,b5,c9),(a16,b5,c10),(a16,b5,c11),(a16,b5,c12),(a16,b5,c13),(a16,b5,c14),(a16,b5, c15),(a16,b6,c1),(a16,b6,c2),(a16,b6,c3),(a16,b6,c4),(a16,b6,c5),(a16,b6,c6),(a16,b6,c7),(a16 ,b6,c8),(a16,b6,c9),(a16,b6,c10),(a16,b6,c11),(a16,b6,c12),(a16,b6,c13),(a16,b6,c14),(a16,b6 ,c15),(a16,b7,c1),(a16,b7,c2),(a16,b7,c3),(a16,b7,c4),(a16,b7,c5),(a16,b7,c6),(a16,b7,c7),(a1 6,b7,c8),(a16,b7,c9),(a16,b7,c10),(a16,b7,c11),(a16,b7,c12),(a16,b7,c13),(a16,b7,c14),(a16,b 7,c15),(a17,b1,c1),(a17,b1,c2),(a17,b1,c3),(a17,b1,c4),(a17,b1,c5),(a17,b1,c6),(a17,b1,c7),(a 17,b1,c8),(a17,b1,c9),(a17,b1,c10),(a17,b1,c11),(a17,b1,c12),(a17,b1,c13),(a17,b1,c14),(a17, b1,c15),(a17,b2,c1),(a17,b2,c2),(a17,b2,c3),(a17,b2,c4),(a17,b2,c5),(a17,b2,c6),(a17,b2,c7),( a17,b2,c8),(a17,b2,c9),(a17,b2,c10),(a17,b2,c11),(a17,b2,c12),(a17,b2,c13),(a17,b2,c14),(a1 7,b2,c15),(a17,b3,c1),(a17,b3,c2),(a17,b3,c3),(a17,b3,c4),(a17,b3,c5),(a17,b3,c6),(a17,b3,c7) ,(a17,b3,c8),(a17,b3,c9),(a17,b3,c10),(a17,b3,c11),(a17,b3,c12),(a17,b3,c13),(a17,b3,c14),(a 17,b3,c15),(a17,b4,c1),(a17,b4,c2),(a17,b4,c3),(a17,b4,c4),(a17,b4,c5),(a17,b4,c6),(a17,b4,c 7),(a17,b4,c8),(a17,b4,c9),(a17,b4,c10),(a17,b4,c11),(a17,b4,c12),(a17,b4,c13),(a17,b4,c14),( a17,b4,c15),(a17,b5,c1),(a17,b5,c2),(a17,b5,c3),(a17,b5,c4),(a17,b5,c5),(a17,b5,c6),(a17,b5, c7),(a17,b5,c8),(a17,b5,c9),(a17,b5,c10),(a17,b5,c11),(a17,b5,c12),(a17,b5,c13),(a17,b5,c14) ,(a17,b5,c15),(a17,b6,c1),(a17,b6,c2),(a17,b6,c3),(a17,b6,c4),(a17,b6,c5),(a17,b6,c6),(a17,b6 ,c7),(a17,b6,c8),(a17,b6,c9),(a17,b6,c10),(a17,b6,c11),(a17,b6,c12),(a17,b6,c13),(a17,b6,c14 ),(a17,b6,c15),(a17,b7,c1),(a17,b7,c2),(a17,b7,c3),(a17,b7,c4),(a17,b7,c5),(a17,b7,c6),(a17,b 7,c7),(a17,b7,c8),(a17,b7,c9),(a17,b7,c10),(a17,b7,c11),(a17,b7,c12),(a17,b7,c13),(a17,b7,c1 4),(a17,b7,c15),(a18,b1,c1),(a18,b1,c2),(a18,b1,c3),(a18,b1,c4),(a18,b1,c5),(a18,b1,c6),(a18, b1,c7),(a18,b1,c8),(a18,b1,c9),(a18,b1,c10),(a18,b1,c11),(a18,b1,c12),(a18,b1,c13),(a18,b1,c 14),(a18,b1,c15),(a18,b2,c1),(a18,b2,c2),(a18,b2,c3),(a18,b2,c4),(a18,b2,c5),(a18,b2,c6),(a18 ,b2,c7),(a18,b2,c8),(a18,b2,c9),(a18,b2,c10),(a18,b2,c11),(a18,b2,c12),(a18,b2,c13),(a18,b2, c14),(a18,b2,c15),(a18,b3,c1),(a18,b3,c2),(a18,b3,c3),(a18,b3,c4),(a18,b3,c5),(a18,b3,c6),(a1 8,b3,c7),(a18,b3,c8),(a18,b3,c9),(a18,b3,c10),(a18,b3,c11),(a18,b3,c12),(a18,b3,c13),(a18,b3 ,c14),(a18,b3,c15),(a18,b4,c1),(a18,b4,c2),(a18,b4,c3),(a18,b4,c4),(a18,b4,c5),(a18,b4,c6),(a 18,b4,c7),(a18,b4,c8),(a18,b4,c9),(a18,b4,c10),(a18,b4,c11),(a18,b4,c12),(a18,b4,c13),(a18,b 4,c14),(a18,b4,c15),(a18,b5,c1),(a18,b5,c2),(a18,b5,c3),(a18,b5,c4),(a18,b5,c5),(a18,b5,c6),( a18,b5,c7),(a18,b5,c8),(a18,b5,c9),(a18,b5,c10),(a18,b5,c11),(a18,b5,c12),(a18,b5,c13),(a18, b5,c14),(a18,b5,c15),(a18,b6,c1),(a18,b6,c2),(a18,b6,c3),(a18,b6,c4),(a18,b6,c5),(a18,b6,c6), (a18,b6,c7),(a18,b6,c8),(a18,b6,c9),(a18,b6,c10),(a18,b6,c11),(a18,b6,c12),(a18,b6,c13),(a18 ,b6,c14),(a18,b6,c15),(a18,b7,c1),(a18,b7,c2),(a18,b7,c3),(a18,b7,c4),(a18,b7,c5),(a18,b7,c6) ,(a18,b7,c7),(a18,b7,c8),(a18,b7,c9),(a18,b7,c10),(a18,b7,c11),(a18,b7,c12),(a18,b7,c13),(a1 8,b7,c14),(a18,b7,c15),(a19,b1,c1),(a19,b1,c2),(a19,b1,c3),(a19,b1,c4),(a19,b1,c5),(a19,b1,c 6),(a19,b1,c7),(a19,b1,c8),(a19,b1,c9),(a19,b1,c10),(a19,b1,c11),(a19,b1,c12),(a19,b1,c13),(a 19,b1,c14),(a19,b1,c15),(a19,b2,c1),(a19,b2,c2),(a19,b2,c3),(a19,b2,c4),(a19,b2,c5),(a19,b2, c6),(a19,b2,c7),(a19,b2,c8),(a19,b2,c9),(a19,b2,c10),(a19,b2,c11),(a19,b2,c12),(a19,b2,c13),( a19,b2,c14),(a19,b2,c15),(a19,b3,c1),(a19,b3,c2),(a19,b3,c3),(a19,b3,c4),(a19,b3,c5),(a19,b3 ,c6),(a19,b3,c7),(a19,b3,c8),(a19,b3,c9),(a19,b3,c10),(a19,b3,c11),(a19,b3,c12),(a19,b3,c13), (a19,b3,c14),(a19,b3,c15),(a19,b4,c1),(a19,b4,c2),(a19,b4,c3),(a19,b4,c4),(a19,b4,c5),(a19,b 4,c6),(a19,b4,c7),(a19,b4,c8),(a19,b4,c9),(a19,b4,c10),(a19,b4,c11),(a19,b4,c12),(a19,b4,c13 ),(a19,b4,c14),(a19,b4,c15),(a19,b5,c1),(a19,b5,c2),(a19,b5,c3),(a19,b5,c4),(a19,b5,c5),(a19, b5,c6),(a19,b5,c7),(a19,b5,c8),(a19,b5,c9),(a19,b5,c10),(a19,b5,c11),(a19,b5,c12),(a19,b5,c1 3),(a19,b5,c14),(a19,b5,c15),(a19,b6,c1),(a19,b6,c2),(a19,b6,c3),(a19,b6,c4),(a19,b6,c5),(a19 ,b6,c6),(a19,b6,c7),(a19,b6,c8),(a19,b6,c9),(a19,b6,c10),(a19,b6,c11),(a19,b6,c12),(a19,b6,c 13),(a19,b6,c14),(a19,b6,c15),(a19,b7,c1),(a19,b7,c2),(a19,b7,c3),(a19,b7,c4),(a19,b7,c5),(a1 9,b7,c6),(a19,b7,c7),(a19,b7,c8),(a19,b7,c9),(a19,b7,c10),(a19,b7,c11),(a19,b7,c12),(a19,b7, c13),(a19,b7,c14),(a19,b7,c15),(a20,b1,c1),(a20,b1,c2),(a20,b1,c3),(a20,b1,c4),(a20,b1,c5),(a 20,b1,c6),(a20,b1,c7),(a20,b1,c8),(a20,b1,c9),(a20,b1,c10),(a20,b1,c11),(a20,b1,c12),(a20,b1 ,c13),(a20,b1,c14),(a20,b1,c15),(a20,b2,c1),(a20,b2,c2),(a20,b2,c3),(a20,b2,c4),(a20,b2,c5),( a20,b2,c6),(a20,b2,c7),(a20,b2,c8),(a20,b2,c9),(a20,b2,c10),(a20,b2,c11),(a20,b2,c12),(a20,b 2,c13),(a20,b2,c14),(a20,b2,c15),(a20,b3,c1),(a20,b3,c2),(a20,b3,c3),(a20,b3,c4),(a20,b3,c5), (a20,b3,c6),(a20,b3,c7),(a20,b3,c8),(a20,b3,c9),(a20,b3,c10),(a20,b3,c11),(a20,b3,c12),(a20, b3,c13),(a20,b3,c14),(a20,b3,c15),(a20,b4,c1),(a20,b4,c2),(a20,b4,c3),(a20,b4,c4),(a20,b4,c5 ),(a20,b4,c6),(a20,b4,c7),(a20,b4,c8),(a20,b4,c9),(a20,b4,c10),(a20,b4,c11),(a20,b4,c12),(a20 ,b4,c13),(a20,b4,c14),(a20,b4,c15),(a20,b5,c1),(a20,b5,c2),(a20,b5,c3),(a20,b5,c4),(a20,b5,c 5),(a20,b5,c6),(a20,b5,c7),(a20,b5,c8),(a20,b5,c9),(a20,b5,c10),(a20,b5,c11),(a20,b5,c12),(a2 0,b5,c13),(a20,b5,c14),(a20,b5,c15),(a20,b6,c1),(a20,b6,c2),(a20,b6,c3),(a20,b6,c4),(a20,b6, c5),(a20,b6,c6),(a20,b6,c7),(a20,b6,c8),(a20,b6,c9),(a20,b6,c10),(a20,b6,c11),(a20,b6,c12),(a 20,b6,c13),(a20,b6,c14),(a20,b6,c15),(a20,b7,c1),(a20,b7,c2),(a20,b7,c3),(a20,b7,c4),(a20,b7 ,c5),(a20,b7,c6),(a20,b7,c7),(a20,b7,c8),(a20,b7,c9),(a20,b7,c10),(a20,b7,c11),(a20,b7,c12),( a20,b7,c13),(a20,b7,c14),(a20,b7,c15),

(a21,b1,c1),(a21,b1,c2),(a21,b1,c3),(a21,b1,c4),(a21,b1,c5),(a21,b1,c6),(a21,b1,c7),(a21,b1,c 8),(a21,b1,c9),(a21,b1,c10),(a21,b1,c11),(a21,b1,c12),(a21,b1,c13),(a21,b1,c14),(a21,b1,c15) ,(a21,b2,c1),(a21,b2,c2),(a21,b2,c3),(a21,b2,c4),(a21,b2,c5),(a21,b2,c6),(a21,b2,c7),(a21,b2, c8),(a21,b2,c9),(a21,b2,c10),(a21,b2,c11),(a21,b2,c12),(a21,b2,c13),(a21,b2,c14),(a21,b2,c15 ),(a21,b3,c1),(a21,b3,c2),(a21,b3,c3),(a21,b3,c4),(a21,b3,c5),(a21,b3,c6),(a21,b3,c7),(a21,b3 ,c8),(a21,b3,c9),(a21,b3,c10),(a21,b3,c11),(a21,b3,c12),(a21,b3,c13),(a21,b3,c14),(a21,b3,c1 5),(a21,b4,c1),(a21,b4,c2),(a21,b4,c3),(a21,b4,c4),(a21,b4,c5),(a21,b4,c6),(a21,b4,c7),(a21,b 4,c8),(a21,b4,c9),(a21,b4,c10),(a21,b4,c11),(a21,b4,c12),(a21,b4,c13),(a21,b4,c14),(a21,b4,c 15),(a21,b5,c1),(a21,b5,c2),(a21,b5,c3),(a21,b5,c4),(a21,b5,c5),(a21,b5,c6),(a21,b5,c7),(a21, b5,c8),(a21,b5,c9),(a21,b5,c10),(a21,b5,c11),(a21,b5,c12),(a21,b5,c13),(a21,b5,c14),(a21,b5, c15),(a21,b6,c1),(a21,b6,c2),(a21,b6,c3),(a21,b6,c4),(a21,b6,c5),(a21,b6,c0),(a21,b6,c7),(a21 ,b6,c8),(a21,b6,c9),(a21,b6,c10),(a21,b6,c11),(a21,b6,c12),(a21,b6,c13),(a21,b6,c14),(a21,b6 ,c15),(a21,b7,c1),(a21,b7,c2),(a21,b7,c3),(a21,b7,c4),(a21,b7,c5),(a21,b7,c6),(a21,b7,c7),(a2 1,b7,c8),(a21,b7,c9),(a21,b7,c10),(a21,b7,c11),(a21,b7,c12),(a21,b7,c13),(a21,b7,c14),(a21,b 7,c15),(a22,b1,c1),(a22,b1,c2),(a22,b1,c3),(a22,b1,c4),(a22,b1,c5),(a22,b1,c6),(a22,b1,c7),(a 22,b1,c8),(a22,b1,c9),(a22,b1,c10),(a22,b1,c11),(a22,b1,c12),(a22,b1,c13),(a22,b1,c14),(a22, b1,c15),(a22,b2,c1),(a22,b2,c2),(a22,b2,c3),(a22,b2,c4),(a22,b2,c5),(a22,b2,c6),(a22,b2,c7),( a22,b2,c8),(a22,b2,c9),(a22,b2,c10),(a22,b2,c11),(a22,b2,c12),(a22,b2,c13),(a22,b2,c14),(a2 2,b2,c15),(a22,b3,c1),(a22,b3,c2),(a22,b3,c3),(a22,b3,c4),(a22,b3,c5),(a22,b3,c6),(a22,b3,c7) ,(a22,b3,c8),(a22,b3,c9),(a22,b3,c10),(a22,b3,c11),(a22,b3,c12),(a22,b3,c13),(a22,b3,c14),(a 22,b3,c15),(a22,b4,c1),(a22,b4,c2),(a22,b4,c3),(a22,b4,c4),(a22,b4,c5),(a22,b4,c6),(a22,b4,c 7),(a22,b4,c8),(a22,b4,c9),(a22,b4,c10),(a22,b4,c11),(a22,b4,c12),(a22,b4,c13),(a22,b4,c14), a22,b4,c15),(a22,b5,c1),(a22,b5,c2),(a22,b5,c3),(a22,b5,c4),(a22,b5,c5),(a22,b5,c6),(a22,b5, c7),(a22,b5,c8),(a22,b5,c9),(a22,b5,c10),(a22,b5,c11),(a22,b5,c12),(a22,b5,c13),(a22,b5,c14) ,(a22,b5,c15),(a22,b6,c1),(a22,b6,c2),(a22,b6,c3),(a22,b6,c4),(a22,b6,c5),(a22,b6,c6),(a22,b6 ,c7),(a22,b6,c8),(a22,b6,c9),(a22,b6,c10),(a22,b6,c11),(a22,b6,c12),(a22,b6,c13),(a22,b6,c14 ),(a22,b6,c15),(a22,b7,c1),(a22,b7,c2),(a22,b7,c3),(a22,b7,c4),(a22,b7,c5),(a22,b7,c6),(a22,b 7,c7),(a22,b7,c8),(a22,b7,c9),(a22,b7,c10),(a22,b7,c11),(a22,b7,c12),(a22,b7,c13),(a22,b7,c1 4),(a22,b7,c15),(a23,b1,c1),(a23,b1,c2),(a23,b1,c3),(a23,b1,c4),(a23,b1,c5),(a23,b1,c6),(a23, b1,c7),(a23,b1,c8),(a23,b1,c9),(a23,b1,c10),(a23,b1,c11),(a23,b1,c12),(a23,b1,c13),(a23,b1,c 14),(a23,b1,c15),(a23,b2,c1),(a23,b2,c2),(a23,b2,c3),(a23,b2,c4),(a23,b2,c5),(a23,b2,c6),(a23 ,b2,c7),(a23,b2,c8),(a23,b2,c9),(a23,b2,c10),(a23,b2,c11),(a23,b2,c12),(a23,b2,c13),(a23,b2, c14),(a23,b2,c15),(a23,b3,c1),(a23,b3,c2),(a23,b3,c3),(a23,b3,c4),(a23,b3,c5),(a23,b3,c6),(a2 3,b3,c7),(a23,b3,c8),(a23,b3,c9),(a23,b3,c10),(a23,b3,c11),(a23,b3,c12),(a23,b3,c13),(a23,b3 ,c14),(a23,b3,c15),(a23,b4,c1),(a23,b4,c2),(a23,b4,c3),(a23,b4,c4),(a23,b4,c5),(a23,b4,c6),(a 23,b4,c7),(a23,b4,c8),(a23,b4,c9),(a23,b4,c10),(a23,b4,c11),(a23,b4,c12),(a23,b4,c13),(a23,b 4,c14),(a23,b4,c15),(a23,b5,c1),(a23,b5,c2),(a23,b5,c3),(a23,b5,c4),(a23,b5,c5),(a23,b5,c6),( a23,b5,c7),(a23,b5,c8),(a23,b5,c9),(a23,b5,c10),(a23,b5,c11),(a23,b5,c12),(a23,b5,c13),(a23, b5,c14),(a23,b5,c15),(a23,b6,c1),(a23,b6,c2),(a23,b6,c3),(a23,b6,c4),(a23,b6,c5),(a23,b6,c6), (a23,b6,c7),(a23,b6,c8),(a23,b6,c9),(a23,b6,c10),(a23,b6,c11),(a23,b6,c12),(a23,b6,c13),(a23 ,b6,c14),(a23,b6,c15),(a23,b7,c1),(a23,b7,c2),(a23,b7,c3),(a23,b7,c4),(a23,b7,c5),(a23,b7,c6) ,(a23,b7,c7),(a23,b7,c8),(a23,b7,c9),(a23,b7,c10),(a23,b7,c11),(a23,b7,c12),(a23,b7,c13),(a2 3,b7,c14),(a23,b7,c15),(a24,b1,c1),(a24,b1,c2),(a24,b1,c3),(a24,b1,c4),(a24,b1,c5),(a24,b1,c 6),(a24,b1,c7),(a24,b1,c8),(a24,b1,c9),(a24,b1,c10),(a24,b1,c11),(a24,b1,c12),(a24,b1,c13),(a 24,b1,c14),(a24,b1,c15),(a24,b2,c1),(a24,b2,c2),(a24,b2,c3),(a24,b2,c4),(a24,b2,c5),(a24,b2, c6),(a24,b2,c7),(a24,b2,c8),(a24,b2,c9),(a24,b2,c10),(a24,b2,c11),(a24,b2,c12),(a24,b2,c13),( a24,b2,c14),(a24,b2,c15),(a24,b3,c1),(a24,b3,c2),(a24,b3,c3),(a24,b3,c4),(a24,b3,c5),(a24,b3 ,c6),(a24,b3,c7),(a24,b3,c8),(a24,b3,c9),(a24,b3,c10),(a24,b3,c11),(a24,b3,c12),(a24,b3,c13), (a24,b3,c14),(a24,b3,c15),(a24,b4,c1),(a24,b4,c2),(a24,b4,c3),(a24,b4,c4),(a24,b4,c5),(a24,b 4,c6),(a24,b4,c7),(a24,b4,c8),(a24,b4,c9),(a24,b4,c10),(a24,b4,c11),(a24,b4,c12),(a24,b4,c13 ),(a24,b4,c14),(a24,b4,c15),(a24,b5,c1),(a24,b5,c2),(a24,b5,c3),(a24,b5,c4),(a24,b5,c5),(a24, b5,c6),(a24,b5,c7),(a24,b5,c8),(a24,b5,c9),(a24,b5,c10),(a24,b5,c11),(a24,b5,c12),(a24,b5,c1 3),(a24,b5,c14),(a24,b5,c15),(a24,b6,c1),(a24,b6,c2),(a24,b6,c3),(a24,b6,c4),(a24,b6,c5),(a24 ,b6,c6),(a24,b6,c7),(a24,b6,c8),(a24,b6,c9),(a24,b6,c10),(a24,b6,c11),(a24,b6,c12),(a24,b6,c 13),(a24,b6,c14),(a24,b6,c15),(a24,b7,c1),(a24,b7,c2),(a24,b7,c3),(a24,b7,c4),(a24,b7,c5),(a2 4,b7,c6),(a24,b7,c7),(a24,b7,c8),(a24,b7,c9),(a24,b7,c10),(a24,b7,c11),(a24,b7,c12),(a24,b7, c13),(a24,b7,c14),(a24,b7,c15),(a25,b1,c1),(a25,b1,c2),(a25,b1,c3),(a25,b1,c4),(a25,b1,c5),(a 25,b1,c6),(a25,b1,c7),(a25,b1,c8),(a25,b1,c9),(a25,b1,c10),(a25,b1,c1),(a25,b1,c12),(a25,b1 ,c13),(a25,b1,c14),(a25,b1,c15),(a25,b2,c1),(a25,b2,c2),(a25,b2,c3),(a25,b2,c4),(a25,b2,c5),( a25,b2,c6),(a25,b2,c7),(a25,b2,c8),(a25,b2,c9),(a25,b2,c10),(a25,b2,c11),(a25,b2,c12),(a25,b 2,c13),(a25,b2,c14),(a25,b2,c15),(a25,b3,c1),(a25,b3,c2),(a25,b3,c3),(a25,b3,c4),(a25,b3,c5), (a25,b3,c6),(a25,b3,c7),(a25,b3,c8),(a25,b3,c9),(a25,b3,c10),(a25,b3,c11),(a25,b3,c12),(a25, b3,c13),(a25,b3,c14),(a25,b3,c15),(a25,b4,c1),(a25,b4,c2),(a25,b4,c3),(a25,b4,c4),(a25,b4,c5 ),(a25,b4,c6),(a25,b4,c7),(a25,b4,c8),(a25,b4,c9),(a25,b4,c10),(a25,b4,c11),(a25,b4,c12),(a25 ,b4,c13),(a25,b4,c14),(a25,b4,c15),(a25,b5,c1),(a25,b5,c2),(a25,b5,c3),(a25,b5,c4),(a25,b5,c 5),(a25,b5,c6),(a25,b5,c7),(a25,b5,c8),(a25,b5,c9),(a25,b5,c10),(a25,b5,c11),(a25,b5,c12),(a2 5,b5,c13),(a25,b5,c14),(a25,b5,c15),(a25,b6,c1),(a25,b6,c2),(a25,b6,c3),(a25,b6,c4),(a25,b6, c5),(a25,b6,c6),(a25,b6,c7),(a25,b6,c8),(a25,b6,c9),(a25,b6,c10),(a25,b6,c11),(a25,b6,c12),(a 25,b6,c13),(a25,b6,c14),(a25,b6,c15),(a25,b7,c1),(a25,b7,c2),(a25,b7,c3),(a25,b7,c4),(a25,b7 ,c5),(a25,b7,c6),(a25,b7,c7),(a25,b7,c8),(a25,b7,c9),(a25,b7,c10),(a25,b7,c11),(a25,b7,c12),( a25,b7,c13),(a25,b1,c14),(a25,b7,c15),

(a26,b1,c1),(a26,b1,c2),(a26,b1,c3),(a26,b1,c4),(a26,b1,c5),(a26,b1,c6),(a26,b1,c7),(a26,b1,c 8),(a26,b1,c9),(a26,b1,c10),(a26,b1,c11),(a26,b1,c12),(a26,b1,c13),(a26,b1,c14),(a26,b1,c15) ,(a26,b2,c1),(a26,b2,c2),(a26,b2,c3),(a26,b2,c4),(a26,b2,c5),(a26,b2,c6),(a26,b2,c7),(a26,b2, c8),(a26,b2,c9),(a26,b2,c10),(a26,b2,c11),(a26,b2,c12),(a26,b2,c13),(a26,b2,c14),(a26,b2,c15 ),(a26,b3,c1),(a26,b3,c2),(a26,b3,c3),(a26,b3,c4),(a26,b3,c5),(a26,b3,c6),(a26,b3,c7),(a26,b3 ,c8),(a26,b3,c9),(a26,b3,c10),(a26,b3,c11),(a26,b3,c12),(a26,b3,c13),(a26,b3,c14),(a26,b3,c1 5),(a26,b4,c1),(a26,b4,c2),(a26,b4,c3),(a26,b4,c4),(a26,b4,c5),(a26,b4,c6),(a26,b4,c7),(a26,b 4,c8),(a26,b4,c9),(a26,b4,c10),(a26,b4,c11),(a26,b4,c12),(a26,b4,c13),(a26,b4,c14),(a26,b4,c 15),(a26,b5,c1),(a26,b5,c2),(a26,b5,c3),(a26,b5,c4),(a26,b5,c5),(a26,b5,c6),(a26,b5,c7),(a26, b5,c8),(a26,b5,c9),(a26,b5,c10),(a26,b5,c11),(a26,b5,c12),(a26,b5,c13),(a26,b5,c14),(a26,b5, c15),(a26,b6,c1),(a26,b6,c2),(a26,b6,c3),(a26,b6,c4),(a26,b6,c5),(a26,b6,c6),(a26,b6,c7),(a26 ,b6,c8),(a26,b6,c9),(a26,b6,c10),(a26,b6,c11),(a26,b6,c12),(a26,b6,c13),(a26,b6,c14),(a26,b6 ,c15),(a26,b7,c1),(a26,b7,c2),(a26,b7,c3),(a26,b7,c4),(a26,b7,c5),(a26,b7,c6),(a26,b7,c7),(a2 6,b7,c8),(a26,b7,c9),(a26,b7,c10),(a26,b7,c11),(a26,b7,c12),(a26,b7,c13),(a26,b7,c14),(a26,b 7,c15),(a27,b1,c1),(a27,b1,c2),(a27,b1,c3),(a27,b1,c4),(a27,b1,c5),(a27,b1,c6),(a27,b1,c7),(a 27,b1,c8),(a27,b1,c9),(a27,b1,c10),(a27,b1,c11),(a27,b1,c12),(a27,b1,c13),(a27,b1,c14),(a27, b1,c15),(a27,b2,c1),(a27,b2,c2),(a27,b2,c3),(a27,b2,c4),(a27,b2,c5),(a27,b2,c6),(a27,b2,c7),( a27,b2,c8),(a27,b2,c9),(a27,b2,c10),(a27,b2,c11),(a27,b2,c12),(a27,b2,c13),(a27,b2,c14),(a2 7,b2,c15),(a27,b3,c1),(a27,b3,c2),(a27,b3,c3),(a27,b3,c4),(a27,b3,c5),(a27,b3,c6),(a27,b3,c7) ,(a27,b3,c8),(a27,b3,c9),(a27,b3,c10),(a27,b3,c11),(a27,b3,c12),(a27,b3,c13),(a27,b3,c14),(a 27,b3,c15),(a27,b4,c1),(a27,b4,c2),(a27,b4,c3),(a27,b4,c4),(a27,b4,c5),(a27,b4,c6),(a27,b4,c 7),(a27,b4,c8),(a27,b4,c9),(a27,b4,c10),(a27,b4,c11),(a27,b4,c12),(a27,b4,c13),(a27,b4,c14),( a27,b4,c15),(a27,b5,c1),(a27,b5,c2),(a27,b5,c3),(a27,b5,c4),(a27,b5,c5),(a27,b5,c6),(a27,b5, c7),(a27,b5,c8),(a27,b5,c9),(a27,b5,c10),(a27,b5,c11),(a27,b5,c12),(a27,b5,c13),(a27,b5,c14) ,(a27,b5,c15),(a27,b6,c1),(a27,b6,c2),(a27,b6,c3),(a27,b6,c4),(a27,b6,c5),(a27,b6,c6),(a27,b6 ,c7),(a27,b6,c8),(a27,b6,c9),(a27,b6,c10),(a27,b6,c11),(a27,b6,c12),(a27,b6,c13),(a27,b6,c14 ),(a27,b6,c15),(a27,b7,c1),(a27,b7,c2),(a27,b7,c3),(a27,b7,c4),(a27,b7,c5),(a27,b7,c6),(a27,b 7,c7),(a27,b7,c8),(a27,b7,c9),(a27,b7,c10),(a27,b7,c11),(a27,b7,c12),(a27,b7,c13),(a27,b7,c1 4),(a27,b7,c15),(a28,b1,c1),(a28,b1,c2),(a28,b1,c3),(a28,b1,c4),(a28,b1,c5),(a28,b1,c6),(a28, b1,c7),(a28,b1,c8),(a28,b1,c9),(a28,b1,c10),(a28,b1,c11),(a28,b1,c12),(a28,b1,c13),(a28,b1,c 14),(a28,b1,c15),(a28,b2,c1),(a28,b2,c2),(a28,b2,c3),(a28,b2,c4),(a28,b2,c5),(a28,b2,c6),(a28 ,b2,c7),(a28,b2,c8),(a28,b2,c9),(a28,b2,c10),(a28,b2,c11),(a28,b2,c12),(a28,b2,c13),(a28,b2, c14),(a28,b2,c15),(a28,b3,c1),(a28,b3,c2),(a28,b3,c3),(a28,b3,c4),(a28,b3,c5),(a28,b3,c6),(a2 8,b3,c7),(a28,b3,c8),(a28,b3,c9),(a28,b3,c10),(a28,b3,c11),(a28,b3,c12),(a28,b3,c13),(a28,b3 ,c14),(a28,b3,c15),(a28,b4,c1),(a28,b4,c2),(a28,b4,c3),(a28,b4,c4),(a28,b4,c5),(a28,b4,c6),(a 28,b4,c7),(a28,b4,c8),(a28,b4,c9),(a28,b4,c10),(a28,b4,c11),(a28,b4,c12),(a28,b4,c13),(a28,b 4,c14),(a28,b4,c15),(a28,b5,c1),(a28,b5,c2),(a28,b5,c3),(a28,b5,c4),(a28,b5,c5),(a28,b5,c6),( a28,b5,c7),(a28,b5,c8),(a28,b5,c9),(a28,b5,c10),(a28,b5,c11),(a28,b5,c12),(a28,b5,c13),(a28, b5,c14),(a28,b5,c15),(a28,b6,c1),(a28,b6,c2),(a28,b6,c3),(a28,b6,c4),(a28,b6,c5),(a28,b6,c6), (a28,b6,c7),(a28,b6,c8),(a28,b6,c9),(a28,b6,c10),(a28,b6,c11),(a28,b6,c12),(a28,b6,c13),(a28 ,b6,c14),(a28,b6,c15),(a28,b7,c1),(a28,b7,c2),(a28,b7,c3),(a28,b7,c4),(a28,b7,c5),(a28,b7,c6) ,(a28,b7,c7),(a28,b7,c8),(a28,b7,c9),(a28,b7,c10),(a28,b7,c11),(a28,b7,c12),(a28,b7,c13),(a2 8,b7,c14),(a28,b7,c15),(a29,b1,c1),(a29,b1,c2),(a29,b1,c3),(a29,b1,c4),(a29,b1,c5),(a29,b1,c 6),(a29,b1,c7),(a29,b1,c8),(a29,b1,c9),(a29,b1,c10),(a29,b1,c11),(a29,b1,c12),(a29,b1,c13),(a 29,b1,c14),(a29,b1,c15),(a29,b2,c1),(a29,b2,c2),(a29,b2,c3),(a29,b2,c4),(a29,b2,c5),(a29,b2, c6),(a29,b2,c7),(a29,b2,c8),(a29,b2,c9),(a29,b2,c10),(a29,b2,c11),(a29,b2,c12),(a29,b2,c13),( a29,b2,e14),(a29,b2,c15),(a29,b3,c1),(a29,b3,c2),(a29,b3,c3),(a29,b3,c4),(a29,b3,c5),(a29,b3 ,c6),(a29,b3,c7),(a29,b3,c8),(a29,b3,c9),(a29,b3,c10),(a29,b3,c11),(a29,b3,c12),(a29,b3,c13), (a29,b3,c14),(a29,b3,c15),(a29,b4,c1),(a29,b4,c2),(a29,b4,c3),(a29,b4,c4),(a29,b4,c5),(a29,b 4,c6),(a29,b4,c7),(a29,b4,c8),(a29,b4,c9),(a29,b4,c10),(a29,b4,c11),(a29,b4,c12),(a29,b4,c13 ),(a29,b4,c14),(a29,b4,c15),(a29,b5,c1),(a29,b5,c2),(a29,b5,c3),(a29,b5,c4),(a29,b5,c5),(a29, b5,c6),(a29,b5,c7),(a29,b5,c8),(a29,b5,c9),(a29,b5,c10),(a29,b5,c11),(a29,b5,c12),(a29,b5,c1 3),(a29,b5,c14),(a29,b5,c15),(a29,b6,c1),(a29,b6,c2),(a29,b6,c3),(a29,b6,c4),(a29,b6,c5),(a29 ,b6,c6),(a29,b6,c7),(a29,b6,c8),(a29,b6,c9),(a29,b6,c10),(a29,b6,c11),(a29,b6,c12),(a29,b6,c 13),(a29,b6,c14),(a29,b6,c15),(a29,b7,c1),(a29,b7,c2),(a29,b7,c3),(a29,b7,c4),(a29,b7,c5),(a2 9,b7,c6),(a29,b7,c7),(a29,b7,c8),(a29,b7,c9),(a29,b7,c10),(a29,b7,c11),(a29,b7,c12),(a29,b7, c13),(a29,b7,c14),(a29,b7,c15),(a30,b1,c1),(a30,b1,c2),(a30,b1,c3),(a30,b1,c4),(a30,b1,c5),(a 30,b1,c6),(a30,b1,c7),(a30,b1,c8),(a30,b1,c9),(a30,b1,c10),(a30,b1,c11),(a30,b1,c12),(a30,b1 ,c13),(a30,b1,c14),(a30,b1,c15),(a30,b2,c1),(a30,b2,c2),(a30,b2,c3),(a30,b2,c4),(a30,b2,c5),( a30,b2,c6),(a30,b2,c7),(a30,b2,c8),(a30,b2,c9),(a30,b2,c10),(a30,b2,c11),(a30,b2,c12),(a30,b 2,c13),(a30,b2,c14),(a30,b2,c15),(a30,b3,c1),(a30,b3,c2),(a30,b3,c3),(a30,b3,c4),(a30,b3,c5), (a30,b3,c6),(a30,b3,c7),(a30,b3,c8),(a30,b3,c9),(a30,b3,c10),(a30,b3,c11),(a30,b3,c12),(a30, b3,c13),(a30,b3,c14),(a30,b3,c15),(a30,b4,c1),(a30,b4,c2),(a30,b4,c3),(a30,b4,c4),(a30,b4,c5 ),(a30,b4,c6),(a30,b4,c7),(a30,b4,c8),(a30,b4,c9),(a30,b4,c10),(a30,b4,c11),(a30,b4,c12),(a30 ,b4,c13),(a30,b4,c14),(a30,b4,c15),(a30,b5,c1),(a30,b5,c2),(a30,b5,c3),(a30,b5,c4),(a30,b5,c 5),(a30,b5,c6),(a30,b5,c7),(a30,b5,c8),(a30,b5,c9),(a30,b5,c10),(a30,b5,c11),(a30,b5,c12),(a3 O,bS,c13),(a30,b5,c14),(a30,b5,c15),(a30,b6,c1),(a30,b6,c2),(a30,b6,c3),(a30,b6,c4),(a30,b6, c5),(a30,b6,c6),(a30,b6,c7),(a30,b6,c8),(a30,b6,c9),(a30,b6,c10),(a30,b6,c11),(a30,b6,c12),(a 30,b6,c13),(a30,b6,c14),(a30,b6,c15),(a30,b7,c1),(a30,b7,c2),(a30,b7,c3),(a30,b7,c4),(a30,b7 ,c5),(a30,b7,c6),(a30,b7,c7),(a30,b7,c8),(a30,b7,c9),(a30,b7,c10),(a30,b7,c11),(a30,b7,c12),( a30,b7,c13),(a30,b7,c14),(a30,b7,c15),(a31,b1,c1),(a31,b1,c2),(a31,b1,c3),(a31,b1,c4),(a31,b 1,c5),(a31,b1,c6),(a31,b1,c7),(a31,b1,c8),(a31,b1,c9),(a31,b1,c10),(a31,b1,c11),(a31,b1,c12), (a31,b1,c13),(a31,b1,c14),(a31,b1,c15),(a31,b2,c1),(a31,b2,c2),(a31,b2,c3),(a31,b2,c4),(a31, b2,c5),(a31,b2,c6),(a31,b2,c7),(a31,b2,c8),(a31,b2,c9),(a31,b2,c10),(a31,b2,c11),(a31,b2,c12 ),(a31,b2,c13),(a31,b2,c14),(a31,b2,c15),(a31,b3,c1),(a31,b3,c2),(a31,b3,c3),(a31,b3,c4),(a31 ,b3,c5),(a31,b3,c6),(a31,b3,c7),(a31,b3,c8),(a31,b3,c9),(a31,b3,c10),(a31,b3,c11),(a31,b3,c1 2),(a31,b3,c13),(a31,b3,c14),(a31,b3,c15),(a31,b4,c1),(a31,b4,c2),(a31,b4,c3),(a31,b4,c4),(a3 1,b4,c5),(a31,b4,c6),(a31,b4,c7),(a31,b4,c8),(a31,b4,c9),(a31,b4,c10),(a31,b4,c11),(a31,b4,c 12),(a31,b4,c13),(a31,b4,c14),(a31,b4,c15),(a31,b5,c1),(a31,b5,c2),(a31,b5,c3),(a31,b5,c4),(a 31,b5,c5),(a31,b5,c6),(a31,b5,c7),(a31,b5,c8),(a31,b5,c9),(a31,b5,c10),(a31,b5,c11),(a31,b5, c12),(a31,b5,c13),(a31,b5,c14),(a31,b5,c15),(a31,b6,c1),(a31,b6,c2),(a31,b6,c3),(a31,b6,c4),( a31,b6,c5),(a31,b6,c6),(a31,b6,c7),(a31,b6,c8),(a31,b6,c9),(a31,b6,c10),(a31,b6,c11),(a31,b6 ,c12),(a31,b6,c13),(a31,b6,c14),(a31,b6,c15),(a31,b7,c1),(a31,b7,c2),(a31,b7,c3),(a31,b7,c4), (a31,b7,c5),(a31,b7,c6),(a31,b7,c7),(a31,b7,c8),(a31,b7,c9),(a31,b7,c10),(a31,b7,c11),(a31,b 7,c12),(a31,b7,c13),(a31,b7,c14)or(a31,b7,c15).

Compounds wherein the combination of 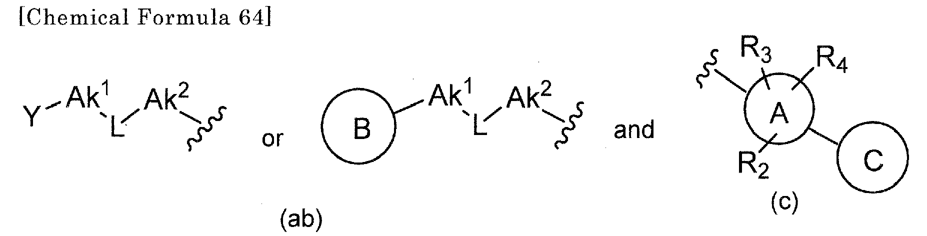 (ab,c) are as follows:
(ab,c)= (ab8,c1),(ab8,c2),(ab8,c3),(ab8,c4),(ab8,c5),(ab8,c6),(ab8,c7),(ab8,c8),(ab8,c9),(ab8,c10),(ab 8,c11),(ab8,c12),(ab8,c13),(ab8,c14),(ab8,c15),(ab9,c1),(ab9,c2),(ab9,c3),(ab9,c4),(ab9,c5),(a b9,c6),(ab9,c7),(ab9,c8),(ab9,c9),(ab9,c10),(ab9,c11),(ab9,c12),(ab9,c13),(ab9,c14),(ab9,c15) ,(ab10,c1),(ab10,c2),(ab10,c3),(ab10,c4),(ab10,c5),(ab10,c6),(ab10,c7),(ab10,c8),(ab10,c9),(a b10,c10),(ab10,c11),(ab10,c12),(ab10,c13),(ab10,c14),(ab10,c15),(ab11,c1),(ab11,c2),(ab11,c 3),(ab11,c4),(ab11,c5),(ab11,c6),(ab11,c7),(ab11,c8),(ab11,c9),(ab11,c10),(ab11,c11),(ab11,c 12),(ab11,c13),(ab11,c14),(ab11,c15),(ab12,c1),(ab12,c2),(ab12,c3),(ab12,c4),(ab12,c5),(ab1 2,c6),(ab12,c7),(ab12,c8),(ab12,c9),(ab12,c10),(ab12,c11),(ab12,c12),(ab12,c13),(ab12,c14),( ab12,c15),(ab13,c1),(ab13,c2),(ab13,c3),(ab13,c4),(ab13,c5),(ab13,c6),(ab13,c7),(ab13,c8),(a b13,c9),(ab13,c10),(ab13,c11),(ab13,c12),(ab13,c13),(ab13,c14),(ab13,c15),(ab14,c1),(ab 14,c 2),(ab14,c3),(ab14,c4),(ab14,c5),(ab14,c6),(ab14,c7),(ab14,c8),(ab14,c9),(ab14,c10),(ab14,c1 1),(ab14,c12),(ab14,c13),(ab14,c14),(ab14,c15),(ab15,c1),(ab15,c2),(ab15,c3),(ab15,c4),(ab1 5,c5),(ab15,c6),(ab15,c7),(ab15,c8),(ab15,c9),(ab15,c10),(ab15,c11),(ab15,c12),(ab15,c13),(a b15,c14),(ab15,c15),(ab16,c1),(ab16,c2),(ab16,c3),(ab16,c4),(ab16,c5),(ab16,c6),(ab16,c7),(a b16,c8),(ab16,c9),(ab16,c10),(ab16,c11),(ab16,c12),(ab16,c13),(ab16,c14),(ab16,c15),(ab17,c 1),(ab17,c2),(ab17,c3),(ab17,c4),(ab17,c5),(ab17,c6),(ab17,c7),(ab17,c8),(ab17,c9),(ab17,c10 ),(ab17,c11),(ab17,c12),(ab17,c13),(ab17,c14),(ab17,c15),(ab18,c1),(ab18,c2),(ab18,c3),(ab1 8,c4),(ab18,c5),(ab18,c6),(ab18,c7),(ab18,c8),(ab18,c9),(ab18,c10),(ab18,c11),(ab18,c12),(ab 18,c13),(ab18,c14),(ab18,c15),(ab19,c1),(ab19,c2),(ab19,c3),(ab19,c4),(ab19,c5),(ab19,c6),(a b19,c7),(ab19,c8),(ab19,c9),(ab19,c10),(ab19,c11),(ab19,c12),(ab19,c13),(ab19,c14)or (ab19,c15).

In another aspect, the following compounds are preferable.

Compounds wherein

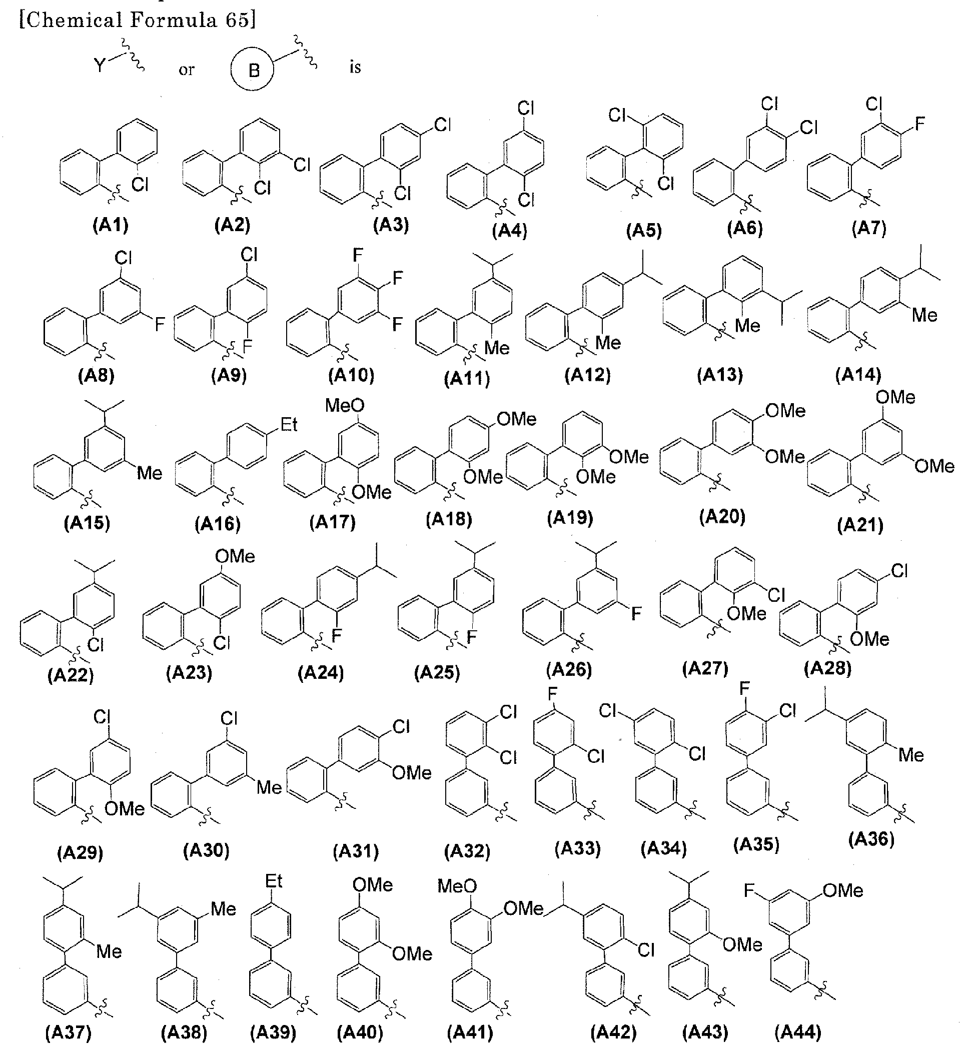

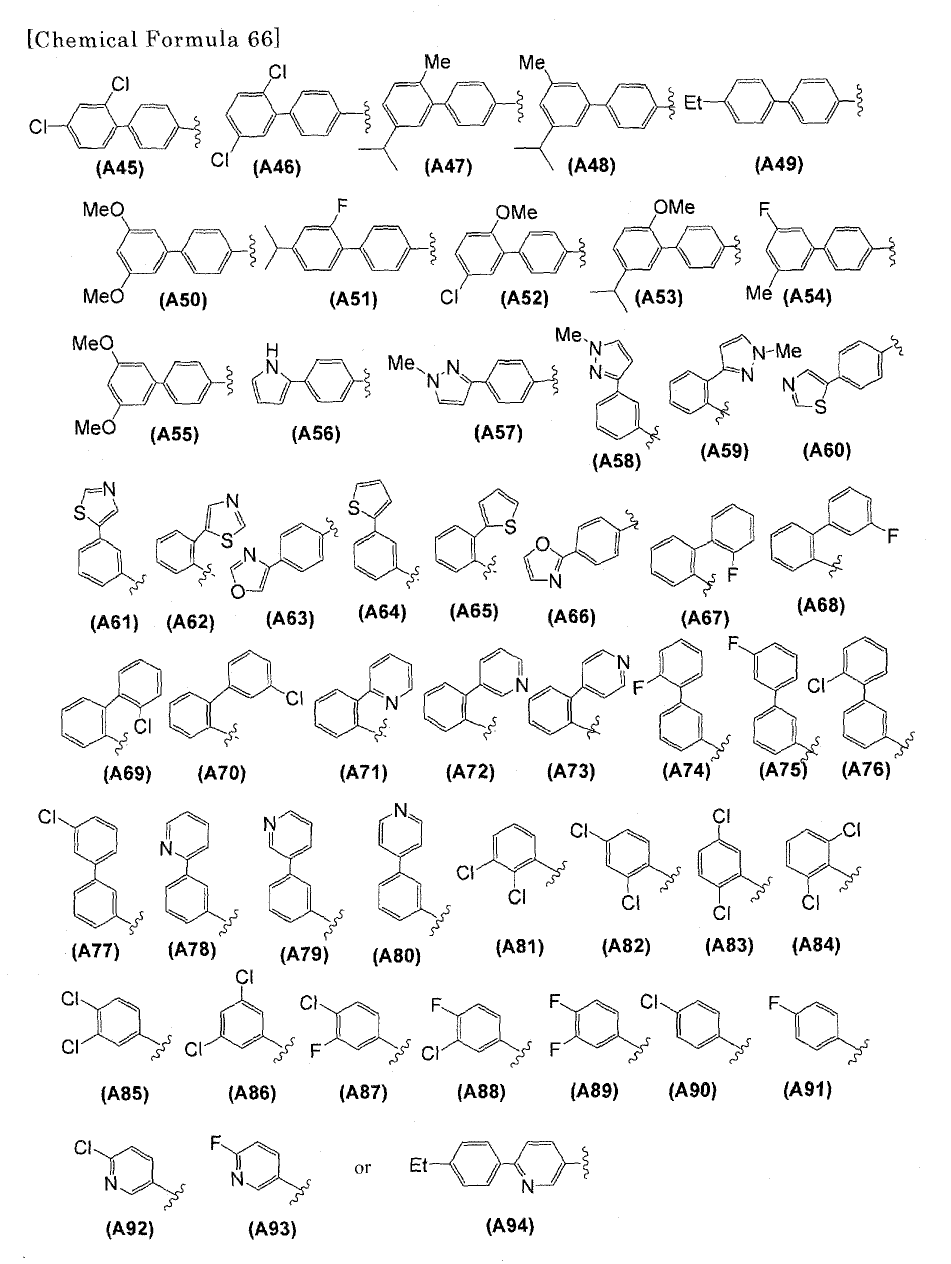

Compounds wherein 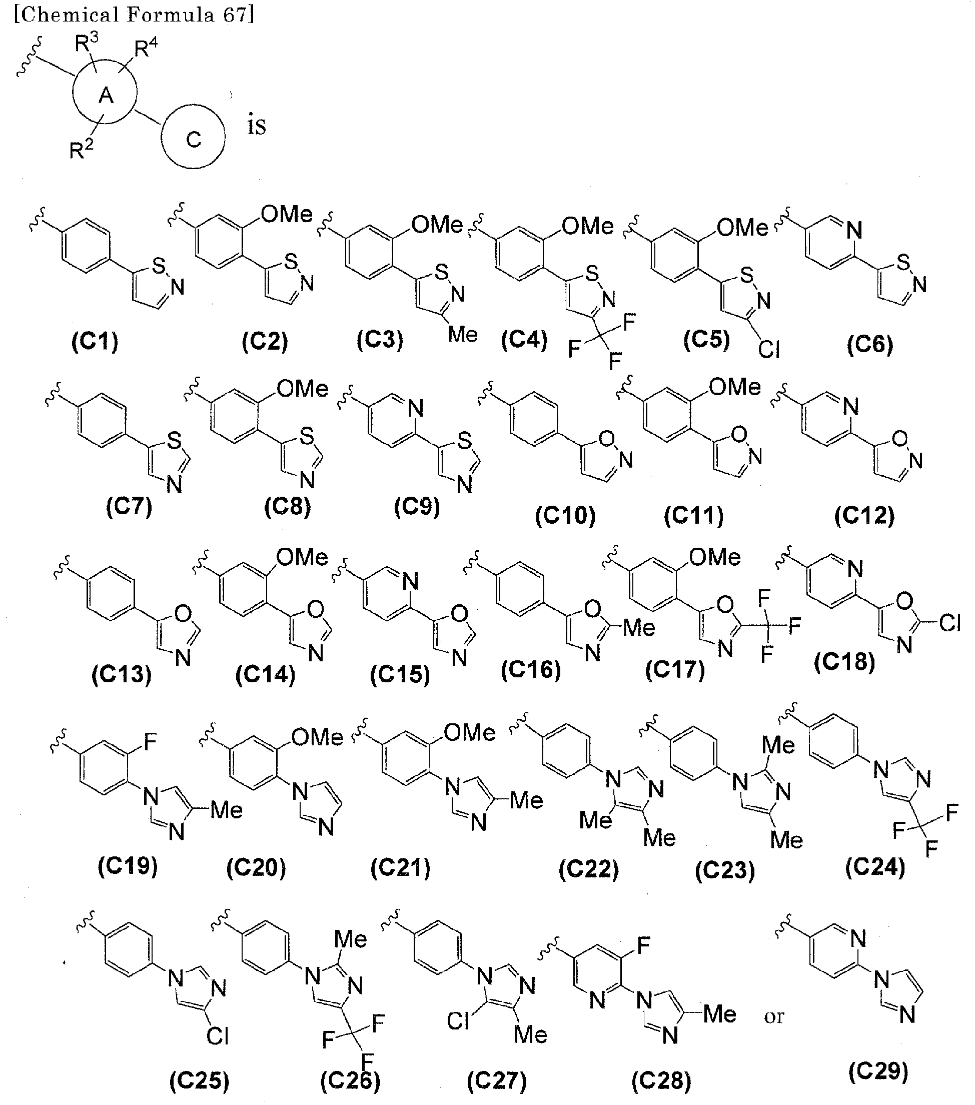 Compounds wherein the combination of 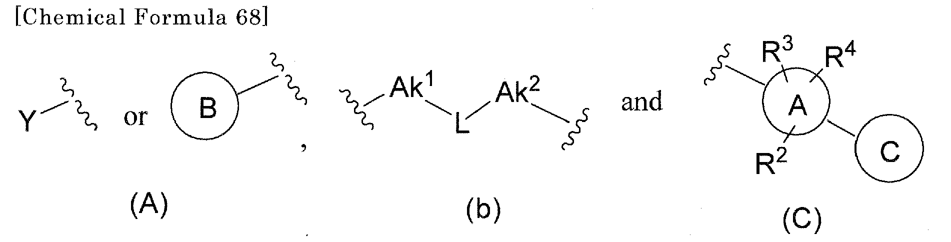 (A,b,C) are as follows:
(A,b,C)=
   (A1,b1,C1),(A1,b1,C2),(A1,b1,C3),(A1,b1,C4),(A1,b1,C5),(A1,b1,C6),(A1,b1,C7),(A1,b1,C8), (A1,b1,C9),(A1,b1,C10),(A1,b1,C11),(A1,b1,C12),(A1,b1,C13),(A1,b1,C14),(A1,b1,C15),(A1, b1,C16),(A1,b1,C17),(A1,b1,C18),(A1,b1,C19),(A1,b1,C20),(A1,b1,C21),(A1,b1,C22),(A1,b**1,** C23),(A1,b1,C24),(A1,b1,C25),(A1,b1,C26),(A1,b1,C27),(A1,b1,C28),(A1,b1,C29),(A1,b2,C1 ),(A1,b2,C2),(A1,b2,C3),(A1,b2,C4),(A1,b2,C5),(A1,b2,C6),(A1,b2,C7),(A1,b2,C8),(A1,b2,C9 ),(A1,b2,C10),(A1,b2,C11),(A1,b2,C12),(A1,b2,C13),(A1,b2,C14),(A1,b2,C15),(A1,b2,C16),( A1,b2,C17),(A1,b2,C18),(A1,b2,C19),(A1,b2,C20),(A1,b2,C21),(A1,b2,C22),(A1,b2,C23),(A1 ,b2,C24),(A1,b2,C25),(A1,b2,C26),(A1,b2,C27),(A1,b2,C28),(A1,b2,C29),(A1,b3,C1),(A1,b3, C2),(A1,b3,C3),(A1,b3,C4),(A1,b3,C5),(A1,b3,C6),(A1,b3,C7),(A1,b3,C8),(A1,b3,C9),(A1,b3, C10),(A1,b3,C11),(A1,b3,C12),(A1,b3,C13),(A1,b3,C14),(A1,b3,C15),(A1,b3,C16),(A1,b3,C1 7),(A1,b3,C18),(A1,b3,C19),(A1,b3,C20),(A1,b3,C21),(A1,b3,C22),(A1,b3,C23),(A1,b3,C24), (A1,b3,C25),(A1,b3,C26),(A1,b3,C27),(A1,b3,C28),(A1,b3,C29),(A1,b4,C1),(A1,b4,C2),(A1,b 4,C3),(A1,b4,C4),(A1,b4,C5),(A1,b4,C6),(A1,b4,C7),(A1,b4,C8),(A1,b4,C9),(A1,b4,C10),(A1, b4,C11),(A1,b4,C12),(A1,b4,C13),(A1,b4,C14),(A1,b4,C15),(A1,b4,C16),(A1,b4,C17),(A1,b4, C18),(A1,b4,C19),(A1,b4,C20),(A1,b4,C21),(A1,b4,C22),(A1,b4,C23),(A1,b4,C24),(A1,b4,C2 5),(A1,b4,C26),(A1,b4,C27),(A1,b4,C28),(A1,b4,C29),(A1,b5,C1),(A1,b5,C2),(A1,b5,C3),(A1, b5,C4),(A1,b5,C5),(A1,b5,C6),(A1,b5,C7),(A1,b5,C8),(A1,b5,C9),(A1,b5,C10),(A1,b5,C11),( A1,b5,C12),(A1,b5,C13),(A1,b5,C14),(A1,b5,C15),(A1,b5,C16),(A1,b5,C17),(A1,b5,C18),(A1 ,b5,C19),(A1,b5,C20),(A1,b5,C21),(A1,b5,C22),(A1,b5,C23),(A1,b5,C24),(A1,b5,C25),(A1,b5 ,C26),(A1,b5,C27),(A1,b5,C28),(A1,b5,C29),(A1,b6,C1),(A1,b6,C2),(A1,b6,C3),(A1,b6,C4),( A1,b6,C5),(A1,b6,C6),(A1,b6,C7),(A1,b6,C8),(A1,b6,C9),(A1,b6,C10),(A1,b6,C11),(A1,b6,C1 2),(A1,b6,C13),(A1,b6,C14),(A1,b6,C15),(A1,b6,C16),(A1,b6,C17),(A1,b6,C18),(A1,b6,C19), (A1,b6,C20),(A1,b6,C21),(A1,b6,C22),(A1,b6,C23),(A1,b6,C24),(A1,b6,C25),(A1,b6,C26),(A 1,b6,C27),(A1,b6,C28),(A1,b6,C29),(A1,b7,C1),(A1,b7,C2),(A1,b7,C3),(A1,b7,C4),(A1,b7,C5 ),(A1,b7,C6),(A1,b7,C7),(A1,b7,C8),(A1,b7,C9),(A1,b7,C10),(A1,b7,C11),(A1,b7,C12),(A1,b7 ,C13),(A1,b7,C14),(A1,b7,C15),(A1,b7,C16),(A1,b7,C17),(A1,b7,C18),(A1,b7,C19),(A1,b7,C 20),(A1,b7,C21),(A1,b7,C22),(A1,b7,C23),(A1,b7,C24),(A1,b7,C25),(A1,b7,C26),(A1,b7,C27) ,(A1,b7,C28),(A1,b7,C29),(A2,b1,C1),(A2,b1,C2),(A2,b1,C3),(A2,b1,C4),(A2,b1,C5),(A2,b1, C6),(A2,b1,C7),(A2,b1,C8),(A2,b1,C9),(A2,b1,C10),(A2,b1,C11),(A2,b1,C12),(A2,b1,C13),(A 2,b1,C14),(A2,b1,C15),(A2,b1,C16),(A2,b1,C17),(A2,b1,C18),(A2,b1,C19),(A2,b1,C20),(A2,b 1,C21),(A2,b1,C22),(A2,b1,C23),(A2,b1,C24),(A2,b1,C25),(A2,b1,C26),(A2,b1,C27),(A2,b1, C28),(A2,b1,C29),(A2,b2,C1),(A2,b2,C2),(A2,b2,C3),(A2,b2,C4),(A2,b2,C5)(A2,b2,C6),(A2, b2,C7),(A2,b2,C8),(A2,b2,C9),(A2,b2,C10),(A2,b2,C11),(A2,b2,C12),(A2,b2,C13),(A2,b2,C1 4),(A2,b2,C15),(A2,b2,C16),(A2,b2,C17),(A2,b2,C18),(A2,b2,C19),(A2,b2,C20),(A2,b2,C21), (A2,b2,C22),(A2,b2,C23),(A2,b2,C24),(A2,b2,C25),(A2,b2,C26),(A2,b2,C27),(A2,b2,C28),(A 2,b2,C29),(A2,b3,C1),(A2,b3,C2),(A2,b3,C3),(A2,b3,C4),(A2,b3,C5),(A2,b3,C6),(A2,b3,C7),( A2,b3,C8),(A2,b3,C9),(A2,b3,C10),(A2,b3,C11),(A2,b3,C12),(A2,b3,C13),(A2,b3,C14),(A2,b 3,C15),(A2,b3,C16),(A2,b3,C17),(A2,b3,C18),(A2,b3,C19),(A2,b3,C20),(A2,b3,C21),(A2,b3, C22),(A2,b3,C23),(A2,b3,C24),(A2,b3,C25),(A2,b3,C26),(A2,b3,C27),(A2,b3,C28),(A2,b3,C2 9),(A2,b4,C1),(A2,b4,C2),(A2,b4,C3),(A2,b4,C4),(A2,b4,C5),(A2,b4,C6),(A2,b4,C7),(A2,b4,C 8),(A2,b4,C9),(A2,b4,C10),(A2,b4,C11),(A2,b4,C12),(A2,b4,C13),(A2,b4,C14),(A2,b4,C15),( A2,b4,C16),(A2,b4,C17),(A2,b4,C18),(A2,b4,C19),(A2,b4,C20),(A2,b4,C21),(A2,b4,C22),(A2 ,b4,C23),(A2,b4,C24),(A2,b4,C25),(A2,b4,C26),(A2,b4,C27),(A2,b4,C28),(A2,b4,C29),(A2,b5 ,C1),(A2,b5,C2),(A2,b5,C3),(A2,b5,C4),(A2,b5,C5),(A2,b5,C6),(A2,b5,C7),(A2,b5,C8),(A2,b5 ,C9),(A2,b5,C10),(A2,b5,C11),(A2,b5,C12),(A2,b5,C13),(A2,b5,C14),(A2,b5,C15),(A2,b5,C1 6),(A2,b5,C17),(A2,b5,C18),(A2,b5,C19),(A2,b5,C20),(A2,b5,C21),(A2,b5,C22),(A2,b5,C23), (A2,b5,C24),(A2,b5,C25),(A2,b5,C26),(A2,b5,C27),(A2,b5,C28),(A2,b5,C29),(A2,b6,C1),(A2, b6,C2),(A2,b6,C3),(A2,b6,C4),(A2,b6,C5),(A2,b6,C6),(A2,b6,C7),(A2,b6,C8),(A2,b6,C9),(A2, b6,C10),(A2,b6,C11),(A2,b6,C12),(A2,b6,C13),(A2,b6,C14),(A2,b6,C15),(A2,b6,C16),(A2,b6, C17),(A2,b6,C18),(A2,b6,C19),(A2,b6,C20),(A2,b6,C21),(A2,b6,C22),(A2,b6,C23),(A2,b6,C2 4),(A2,b6,C25),(A2,b6,C26),(A2,b6,C27),(A2,b6,C28),(A2,b6,C29),(A2,b7,C1),(A2,b7,C2),(A 2,b7,C3),(A2,b7,C4),(A2,b7,C5),(A2,b7,C6),(A2,b7,C7),(A2,b7,C8),(A2,b7,C9),(A2,b7,C10),( A2,b7,C11),(A2,b7,C12),(A2,b7,C13),(A2,b7,C14),(A2,b7,C15),(A2,b7,C16),(A2,b7,C17),(A2 ,b7,C18),(A2,b7,C19),(A2,b7,C20),(A2,b7,C21),(A2,b7,C22),(A2,b7,C23),(A2,b7,C24),(A2,b7 ,C25),(A2,b7,C26),(A2,b7,C27),(A2,b7,C28),(A2,b7,C29),(A3,b1,C1),(A3,b1,C2),(A3,b1,C3),( A3,b1,C4),(A3,b1,C5),(A3,b1,C6),(A3,b1,C7),(A3,b1,C8),(A3,b1,C9),(A3,b1,C10),(A3,b1,C11 ),(A3,b1,C12),(A3,b1,C13),(A3,b1,C14),(A3,b1,C15),(A3,b1,C16),(A3,b1,C17),(A3,b1,C18),( A3,b1,C19),(A3,b1,C20),(A3,b1,C21),(A3,b1,C22),(A3,b1,C23),(A3,b1,C24),(A3,b1,C25),(A3 ,b1,C26),(A3,b1,C27),(A3,b1,C28),(A3,b1,C29),(A3,b2,C1),(A3,b2,C2),(A3,b2,C3),(A3,b2,C4 ),(A3,b2,C5),(A3,b2,C6),(A3,b2,C7),(A3,b2,C8),(A3,b2,C9),(A3,b2,C10),(A3,b2,C11),(A3,b2, C12),(A3,b2,C13),(A3,b2,C14),(A3,b2,C15),(A3,b2,C16),(A3,b2,C17),(A3,b2,C18),(A3,b2,C1 9),(A3,b2,C20),(A3,b2,C21),(A3,b2,C22),(A3,b2,C23),(A3,b2,C24),(A3,b2,C25),(A3,b2,C26), (A3,b2,C27),(A3,b2,C28),(A3,b2,C29),(A3,b3,C1),(A3,b3,C2),(A3,b3,C3),(A3,b3,C4),(A3,b3, C5),(A3,b3,C6),(A3,b3,C7),(A3,b3,C8),(A3,b3,C9),(A3,b3,C10),(A3,b3,C11),(A3,b3,C12),(A3 ,b3,C13),(A3,b3,C14),(A3,b3,C15),(A3,b3,C16),(A3,b3,C17),(A3,b3,C18),(A3,b3,C19),(A3,b3 ,C20),(A3,b3,C21),(A3,b3,C22),(A3,b3,C23),(A3,b3,C24),(A3,b3,C25),(A3,b3,C26),(A3,b3,C 27),(A3,b3,C28),(A3,b3,C29),(A3,b4,C1),(A3,b4,C2),(A3,b4,C3),(A3,b4,C4),(A3,b4,C5),(A3,b 4,C6),(A3,b4,C7),(A3,b4,C8),(A3,b4,C9),(A3,b4,C10),(A3,b4,C11),(A3,b4,C12),(A3,b4,C13),( A3,b4,C14),(A3,b4,C15),(A3,b4,C16),(A3,b4,C17),(A3,b4,C18),(A3,b4,C19),(A3,b4,C20),(A3 ,b4,C21),(A3,b4,C22),(A3,b4,C23),(A3,b4,C24),(A3,b4,C25),(A3,b4,C26),(A3,b4,C27),(A3,b4 ,C28),(A3,b4,C29),(A3,b5,C1),(A3,b5,C2),(A3,b5,C3),(A3,b5,C4),(A3,b5,C5),(A3,b5,C6),(A3, b5,C7),(A3,b5,C8),(A3,b5,C9),(A3,b5,C10),(A3,b5,C11),(A3,b5,C12),(A3,b5,C13),(A3,b5,C1 4),(A3,b5,C15),(A3,b5,C16),(A3,b5,C17),(A3,b5,C18),(A3,b5,C19),(A3,b5,C20),(A3,b5,C21), (A3,b5,C22),(A3,b5,C23),(A3,b5,C24),(A3,b5,C25),(A3,b5,C26),(A3,b5,C27),(A3,b5,C28),(A 3,b5,C29),(A3,b6,C1),(A3,b6,C2),(A3,b6,C3),(A3,b6,C4),(A3,b6,C5),(A3,b6,C6),(A3,b6,C7),( A3,b6,C8),(A3,b6,C9),(A3,b6,C10),(A3,b6,C11),(A3,b6,C12),(A3,b6,C13),(A3,b6,C14),(A3,b 6,C15),(A3,b6,C16),(A3,b6,C17),(A3,b6,C18),(A3,b6,C19),(A3,b6,C20),(A3,b6,C21),(A3,b6, C22),(A3,b6,C23),(A3,b6,C24),(A3,b6,C25),(A3,b6,C26),(A3,b6,C27),(A3,b6,C28),(A3,b6,C2 9),(A3,b7,C1),(A3,b7,C2),(A3,b7,C3),(A3,b7,C4);(A3,b7,C5),(A3,b7,C6),(A3,b7,C7),(A3,b7,C 8),(A3,b7,C9),(A3,b7,C10),(A3,b7,C11),(A3,b7,C12),(A3,b7,C13),(A3,b7,C14),(A3,b7,C15),( A3,b7,C16),(A3,b7,C17),(A3,b7,C18),(A3,b7,C19),(A3,b7,C20),(A3,b7,C21),(A3,b7,C22),(A3 ,b7,C23),(A3,b7,C24),(A3,b7,C25),(A3,b7,C26),(A3,b7,C27),(A3,b7,C28),(A3,b7,C29),(A4,b1 ,C1),(A4,b1,C2),(A4,b1,C3),(A4,b1,C4),(A4,b1,C5),(A4,b1,C6),(A4,b1,C7),(A4,b1,C8),(A4,b1 ,C9),(A4,b1,C10),(A4,b1,C11),(A4,b1,C12),(A4,b1,C13),(A4,b1,C14),(A4,b1,C15),(A4,b1,C1 6),(A4,b1,C17),(A4,b1,C18),(A4,b1,C19),(A4,b1,C20),(A4,b1,C21),(A4,b1,C22),(A4,b1,C23), (A4,b1,C24),(A4,b1,C25),(A4,b1,C26),(A4,b1,C27),(A4,b1,C28),(A4,b1,C29),(A4,b2,C1),(A4, b2,C2),(A4,b2,C3),(A4,b2,C4),(A4,b2,C5),(A4,b2,C6),(A4,b2,C7),(A4,b2,C8),(A4,b2,C9),(A4, b2,C10),(A4,b2,C11),(A4,b2,C12),(A4,b2,C13),(A4,b2,C14),(A4,b2,C15),(A4,b2,C16),(A4,b2, C17),(A4,b2,C18),(A4,b2,C19),(A4,b2,C20),(A4,b2,C21),(A4,b2,C22),(A4,b2,C23),(A4,b2,C2 4),(A4,b2,C25),(A4,b2,C26),(A4,b2,C27),(A4,b2,C28),(A4,b2,C29),(A4,b3,C1),(A4,b3,C2),(A 4,b3,C3),(A4,b3,C4),(A4,b3,C5),(A4,b3,C6),(A4,b3,C7),(A4,b3,C8),(A4,b3,C9),(A4,b3,C10),( A4,b3,C11),(A4,b3,C12),(A4,b3,C13),(A4,b3,C14),(A4,b3,C15),(A4,b3,C16),(A4,b3,C17),(A4 ,b3,C18),(A4,b3,C19),(A4,b3,C20),(A4,b3,C21),(A4,b3,C22),(A4,b3,C23),(A4,b3,C24),(A4,b3 ,C25),(A4,b3,C26),(A4,b3,C27),(A4,b3,C28),(A4,b3,C29),(A4,b4,C1),(A4,b4,C2),(A4,b4,C3),( A4,b4,C4),(A4,b4,C5),(A4,b4,C6),(A4,b4,C7),(A4,b4,C8),(A4,b4,C9),(A4,b4,C10),(A4,b4,C11 ),(A4,b4,C12),(A4,b4,C13),(A4,b4,C14),(A4,b4,C15),(A4,b4,C16),(A4,b4,C17),(A4,b4,C18),( A4,b4,C19),(A4,b4,C20),(A4,b4,C21),(A4,b4,C22),(A4,b4,C23),(A4,b4,C24),(A4,b4,C25),(A4 ,b4,C26),(A4,b4,C27),(A4,b4,C28),(A4,b4,C29),(A4,b5,C1),(A4,b5,C2),(A4,b5,C3),(A4,b5,C4 ),(A4,b5,C5),(A4,b5,C6),(A4,b5,C7),(A4,b5,C8),(A4,b5,C9),(A4,b5,C10),(A4,b5,C11),(A4,b5, C12),(A4,b5,C13),(A4,b5,C14),(A4,b5,C15),(A4,b5,C16),(A4,b5,C17),(A4,b5,C18),(A4,b5,C1 9),(A4,b5,C20),(A4,b5,C21),(A4,b5,C22),(A4,b5,C23),(A4,b5,C24),(A4,b5,C25),(A4,b5,C26), (A4,b5,C27),(A4,b5,C28),(A4,b5,C29),(A4,b6,C1),(A4,b6,C2),(A4,b6,C3),(A4,b6,C4),(A4,b6, C5),(A4,b6,C6),(A4,b6,C7),(A4,b6,C8),(A4,b6,C9),(A4,b6,C10),(A4,b6,C11),(A4,b6,C12),(A4 ,b6,C13),(A4,b6,C14),(A4,b6,C15),(A4,b6,C16),(A4,b6,C17),(A4,b6,C18),(A4,b6,C19),(A4,b6 ,C20),(A4,b6,C21),(A4,b6,C22),(A4,b6,C23),(A4,b6,C24),(A4,b6,C25),(A4,b6,C26),(A4,b6,C 27),(A4,b6,C28),(A4,b6,C29),(A4,b7,C1),(A4,b7,C2),(A4,b7,C3),(A4,b7,C4),(A4,b7,C5),(A4,b 7,C6),(A4,b7,C7),(A4,b7,C8),(A4,b7,C9),(A4,b7,C10),(A4,b7,C11),(A4,b7,C12),(A4,b7,C13),( A4,b7,C14),(A4,b7,C15),(A4,b7,C16),(A4,b7,C17),(A4,b7,C18),(A4,b7,C19),(A4,b7,C20),(A4 ,b7,C21),(A4,b7,C22),(A4,b7,C23),(A4,b7,C24),(A4,b7,C25),(A4,b7,C26),(A4,b7,C27),(A4,b7 ,C28),(A4,b7,C29),(A5,b1,C1),(A5,b1,C2),(A5,b1,C3),(A5,b1,C4),(A5,b1,C5),(A5,b1,C6),(A5, b1,C7),(A5,b1,C8),(A5,b1,C9),(A5,b1,C10),(A5,b1,C11),(A5,b1,C12),(A5,b1,C13),(A5,b1,C1 4),(A5,b1,C15),(A5,b1,C16),(A5,b1,C17),(A5,b1,C18),(A5,b1,C19),(A5,b1,C20),(A5,b1,C21), (A5,b1,C22),(A5,b1,C23),(A5,b1,C24),(A5,b1,C25),(A5,b1,C26),(A5,b1,C27),(A5,b1,C28),(A 5,b1,C29),(A5,b2,C1),(A5,b2,C2),(A5,b2,C3),(A5,b2,C4),(A5,b2,C5),(A5,b2,C6),(A5,b2,C7),( A5,b2,C8),(A5,b2,C9),(A5,b2,C10),(A5,b2,C11),(A5,b2,C12),(A5,b2,C13),(A5,b2,C14),(A5,b 2,C15),(A5,b2,C16),(A5,b2,C17),(A5,b2,C18),(A5,b2,C19),(A5,b2,C20),(A5,b2,C21),(A5,b2, C22),(A5,b2,C23),(A5,b2,C24),(A5,b2,C25),(A5,b2,C26),(A5,b2,C27),(A5,b2,C28),(A5,b2,C2 9),(A5,b3,C1),(A5,b3,C2),(A5,b3,C3),(A5,b3,C4),(A5,b3,C5),(A5,b3,C6),(A5,b3,C7),(A5,b3,C 8),(A5,b3,C9),(A5,b3,C10),(A5,b3,C11),(A5,b3,C12),(A5,b3,C13),(A5,b3,C14),(A5,b3,C15),( A5,b3,C16),(A5,b3,C17),(A5,b3,C18),(A5,b3,C19),(A5,b3,C20),(A5,b3,C21),(A5,b3,C22),(A5 ,b3,C23),(A5,b3,C24),(A5,b3,C25),(A5,b3,C26),(A5,b3,C27),(A5,b3,C28),(A5,b3,C29),(A5,b4 ,C1),(A5,b4,C2),(A5,b4,C3),(A5,b4,C4),(A5,b4,C5),(A5,b4,C6),(A5,b4,C7),(A5,b4,C8),(A5,b4 ,C9),(A5,b4,C10),(A5,b4,C11),(A5,b4,C12),(A5,b4,C13),(A5,b4,C14),(A5,b4,C15),(A5,b4,C1 6),(A5,b4,C17),(A5,b4,C18),(A5,b4,C19),(A5,b4,C20),(A5,b4,C21),(A5,b4,C22),(A5,b4,C23), (A5,b4,C24),(A5,b4,C25),(A5,b4,C26),(A5,b4,C27),(A5,b4,C28),(A5,b4,C29),(A5,b5,C1),(A5, b5,C2),(A5,b5,C3),(A5,b5,C4),(A5,b5,C5),(A5,b5,C6),(A5,b5,C7),(A5,b5,C8),(A5,b5,C9),(A5, b5,C10),(A5,b5,C11),(A5,b5,C12),(A5,b5,C13),(A5,b5,C14),(A5,b5,C5),(A5,b5,C16),(A5,b5, C17),(A5,b5,C18),(A5,b5,C19),(A5,b5,C20),(A5,b5,C21),(A5,b5,C22),(A5,b5,C23),(A5,b5,C2 4),(A5,b5,C25),(A5,b5,C26),(A5,b5,C27),(A5,b5,C28),(A5,b5,C29),(A5,b6,C1),(A5,b6,C2),(A 5,b6,C3),(A5,b6,C4),(A5,b6,C5),(A5,b6,C6),(A5,b6,C7),(A5,b6,C8),(A5,b6,C9),(A5,b6,C10),( A5,b6,C11),(A5,b6,C12),(A5,b6,C13),(A5,b6,C14),(A5,b6,C15),(A5,b6,C16),(A5,b6,C17),(A5 ,b6,C18),(A5,b6,C19),(A5,b6,C20),(A5,b6,C21),(A5,b6,C22),(A5,b6,C23),(A5,b6,C24),(A5,b6 ,C25),(A5,b6,C26),(A5,b6,C27),(A5,b6,C28),(A5,b6,C29),(A5,b7,C1),(A5,b7,C2),(A5,b7,C3), A5,b7,C4),(A5,b7,C5),(A5,b7,C6),(A5,b7,C7),(A5,b7,C8),(A5,b7,C9),(A5,b7,C10),(A5,b7,C11 ),(A5,b7,C12),(A5,b7,C13),(A5,b7,C14),(A5,b7,C15),(A5,b7,C16),(A5,b7,C17),(A5,b7,C18),( A5,b7,C19),(A5,b7,C20),(A5,b7,C21),(A5,b7,C22),(A5,b7,C23),(A5,b7,C24),(A5,b7,C25),(A5 ,b7,C26),(A5,b7,C27),(A5,b7,C28),(A5,b7,C29),(A6,b1,C1),(A6,b1,C2),(A6,b1,C3),(A6,b1,C4 ),(A6,b1,C5),(A6,b1,C6),(A6,b1,C7),(A6,b1,C8),(A6,b1,C9),(A6,b1,C10),(A6,b1,C11),(A6,b1, C12),(A6,b1,C13),(A6,b1,C14),(A6,b1,C15),(A6,b1,C16),(A6,b1,C17),(A6,b1*,*C18),(A6,b1,C1 9),(A6,b1,C20),(A6,b1,C21),(A6,b1,C22),(A6,b1,C23),(A6,b1,C24),(A6,b1,C25),(A6,b1,C26), (A6,b1,C27),(A6,b1,C28),(A6,b1,C29),(A6,b2,C1),(A6,b2,C2),(A6,b2,C3),(A6,b2,C4),(A6,b2, C5),(A6,b2,C6),(A6,b2,C7),(A6,b2,C8),(A6,b2,C9),(A6,b2,C10),(A6,b2,C11),(A6,b2,C12),(A6 ,b2,C13),(A6,b2,C14),(A6,b2,C15),(A6,b2,C16),(A6,b2,C17),(A6,b2,C18),(A6,b2,C19),(A6,b2 ,C20),(A6,b2,C21),(A6,b2,C22),(A6,b2,C23),(A6,b2,C24),(A6,b2,C25),(A6,b2,C26),(A6,b2,C 27),(A6,b2,C28),(A6,b2,C29),(A6,b3,C1),(A6,b3,C2),(A6,b3,C3),(A6,b3,C4),(A6,b3,C5),(A6,b 3,C6),(A6,b3,C7),(A6,b3,C8),(A6,b3,C9),(A6,b3,C10),(A6,b3,C11),(A6,b3,C12),(A6,b3,C13),( A6,b3,C14),(A6,b3,C15),(A6,b3,C16),(A6,b3,C17),(A6,b3,C18),(A6,b3,C19),(A6,b3,C20),(A6 ,b3,C21),(A6,b3,C22),(A6,b3,C23),(A6,b3,C24),(A6,b3,C25),(A6,b3,C26),(A6,b3,C27),(A6,b3 ,C28),(A6,b3,C29),(A6,b4,C1),(A6,b4,C2),(A6,b4,C3),(A6,b4,C4),(A6,b4,C5),(A6,b4,C6),(A6, b4,C7),(A6,b4,C8),(A6,b4,C9),(A6,b4,C10),(A6,b4,C11),(A6,b4,C12),(A6,b4,C13),(A6,b4,C1 4),(A6,b4,C15),(A6,b4,C16),(A6,b4,C17),(A6,b4,C18),(A6,b4,C19),(A6,b4,C20),(A6,b4,C21), (A6,b4,C22),(A6,b4,C23),(A6,b4,C24),(A6,b4,C25),(A6,b4,C26),(A6,b4,C27),(A6,b4,C28),(A 6,b4,C29),(A6,b5,C1),(A6,b5,C2),(A6,b5,C3),(A6,b5,C4),(A6,b5,C5),(A6,b5,C6),(A6,b5,C7),( A6,b5,C8),(A6,b5,C9),(A6,b5,C10),(A6,b5,C11),(A6,b5,C12),(A6,b5,C13),(A6,b5,C14),(A6,b 5,C15),(A6,b5,C16),(A6,b5,C17),(A6,b5,C18),(A6,b5,C19),(A6,b5,C20),(A6,b5,C21),(A6,b5, C22),(A6,b5,C23),(A6,b5,C24),(A6,b5,C25),(A6,b5,C26),(A6,b5,C27),(A6,b5,C28),(A6,b5,C2 9),(A6,b6,C1),(A6,b6,C2),(A6,b6,C3),(A6,b6,C4),(A6,b6,C5),(A6,b6,C6),(A6,b6,C7),(A6,b6,C 8),(A6,b6,C9),(A6,b6,C10),(A6,b6,C11),(A6,b6,C12),(A6,b6,C13),(A6,b6,C14),(A6,b6,C15),( A6,b6,C16),(A6,b6,C17),(A6,b6,C18),(A6,b6,C19),(A6,b6,C20),(A6,b6,C21),(A6,b6,C22),(A6 ,b6,C23),(A6,b6,C24),(A6,b6,C25),(A6,b6,C26),(A6,b6,C27),(A6,b6,C28),(A6,b6,C29),(A6,b7 ,C1),(A6,b7,C2),(A6,b7,C3),(A6,b7,C4),(A6,b7,C5),(A6,b7,C6),(A6,b7,C7),(A6,b7,C8),(A6,b7 ,C9),(A6,b7,C10),(A6,b7,C11),(A6,b7,C12),(A6,b7,C13),(A6,b7,C14),(A6,b7,C15),(A6,b7,C1 6),(A6,b7,C17),(A6,b7,C18),(A6,b7,C19),(A6,b7,C20),(A6,b7,C21),(A6,b7,C22),(A6,b7,C23), (A6,b7,C24),(A6,b7,C25),(A6,b7,C26),(A6,b7,C27),(A6,b7,C28),(A6,b7,C29),(A7,b1,C1),(A7, b1,C2),(A7,b1,C3),(A7,b1,C4),(A7,b1,C5),(A7,b1,C6),(A7,b1,C7),(A7,b1,C8),(A7,b1,C9),(A7, b1,C10),(A7,b1,C11),(A7,b1,C12),(A7,b1,C13),(A7,b1,C14),(A7,b1,C15),(A7,b1,C16),(A7,b1, C17),(A7,b1,C18),(A7,b1,C19),(A7,b1,C20),(A7,b1,C21),(A7,b1,C22),(A7,b1,C23),(A7,b1,C2 4),(A7,b1,C25),(A7,b1,C26),(A7,b1,C27),(A7,b1,C28),(A7,b1,C29),(A7,b2,C1),(A7,b2,C2),(A 7,b2,C3),(A7,b2,C4),(A7,b2,C5),(A7,b2,C6),(A7,b2,C7),(A7,b2,C8),(A7,b2,C9),(A7,b2,C10),( A7,b2,C11),(A7,b2,C12),(A7,b2,C13),(A7,b2,C14),(A7,b2,C15),(A7,b2,C16),(A7,b2,C17),(A7 ,b2,C18),(A7,b2,C19),(A7,b2,C20),(A7,b2,C21),(A7,b2,C22),(A7,b2,C23),(A7,b2,C24),(A7,b2 ,C25),(A7,b2,C26),(A7,b2,C27),(A7,b2,C28),(A7,b2,C29),(A7,b3,C1),(A7,b3,C2),(A7,b3,C3),( A7,b3,C4),(A7,b3,C5),(A7,b3,C6),(A7,b3,C7),(A7,b3,C8),(A7,b3,C9),(A7,b3,C10),(A7,b3,C11 ),(A7,b3,C12),(A7,b3,C13),(A7,b3,C14),(A7,b3,C15),(A7,b3,C16),(A7,b3,C17),(A7,b3,C18),( A7,b3,C19),(A7,b3,C20),(A7,b3,C21),(A7,b3,C22),(A7,b3,C23),(A7,b3,C24),(A7,b3,C25),(A7 ,b3,C26),(A7,b3,C27),(A7,b3,C28),(A7,b3,C29),(A7,b4,C1),(A7,b4,C2),(A7,b4,C3),(A7,b4,C4 ),(A7,b4,C5),(A7,b4,C6),(A7,b4,C7),(A7,b4,C8),(A7,b4,C9),(A7,b4,C10),(A7,b4,C11),(A7,b4, C12),(A7,b4,C13),(A7,b4,C14),(A7,b4,C15),(A7,b4,C16),(A7,b4,C17),(A7,b4,C18),(A7,b4,C1 9),(A7,b4,C20),(A7,b4,C21),(A7,b4,C22),(A7,b4,C23),(A7,b4,C24),(A7,b4,C25),(A7,b4,C26), (A7,b4,C27),(A7,b4,C28),(A7,b4,C29),(A7,b5,C1),(A7,b5,C2),(A7,b5,C3),(A7,b5,C4),(A7,b5, C5),(A7,b5,C6),(A7,b5,C7),(A7,b5,C8),(A7,b5,C9),(A7,b5,C10),(A7,b5,C11),(A7,b5,C12),(A7 ,b5,C13),(A7,b5,C14),(A7,b5,C15),(A7,b5,C16),(A7,b5,C17),(A7,b5,C18),(A7,b5,C19),(A7,b5 ,C20),(A7,b5,C21),(A7,b5,C22),(A7,b5,C23),(A7,b5,C24),(A7,b5,C25),(A7,b5,C26),(A7,b5,C 27),(A7,b5,C28),(A7,b5,C29),(A7,b6,C1),(A7,b6,C2),(A7,b6,C3),(A7,b6,C4),(A7,b6,C5),(A7,b 6,C6),(A7,b6,C7),(A7,b6,C8),(A7,b6,C9),(A7,b6,C10),(A7,b6,C11),(A7,b6,C12),(A7,b6,C13),( A7,b6,C14),(A7,b6,C15),(A7,b6,C16),(A7,b6,C17),(A7,b6,C18),(A7,b6,C19),(A7,b6,C20),(A7 ,b6,C21),(A7,b6,C22),(A7,b6,C23),(A7,b6,C24),(A7,b6,C25),(A7,b6,C26),(A7,b6,C27),(A7,b6 ,C28),(A7,b6,C29),(A7,b7,C1),(A7,b7,C2),(A7,b7,C3),(A7,b7,C4),(A7,b7,C5),(A7,b7,C6),(A7, b7,C7),(A7,b7,C8),(A7,b7,C9),(A7,b7,C10),(A7,b7,C11),(A7,b7,C12),(A7,b7,C13),(A7,b7,C1 4),(A7,b7,C15),(A7,b7,C16),(A7,b7,C17),(A7,b7,C18),(A7,b7,C19),(A7,b7,C20),(A7,b7,C21), (A7,b7,C22),(A7,b7,C23),(A7,b7,C24),(A7,b7,C25),(A7,b7,C26),(A7,b7,C27),(A7,b7,C28),(A 7,b7,C29),(A8,b1,C1),(A8,b1,C2),(A8,b1,C3),(A8,b1,C4),(A8,b1,C5),(A8,b1,C6),(A8,b1,C7),( A8,b1,C8),(A8,b1,C9),(A8,b1,C10),(A8,b1,C11),(A8,b1,C12),(A8,b1,C13),(A8,b1,C14),(A8,b 1,C15),(A8,b1,C16),(A8,b1,C17),(A8,b1,C18),(A8,b1,C19),(A8,b1,C20),(A8,b1,C21),(A8,b1, C22),(A8,b1,C23),(A8,b1,C24),(A8,b1,C25),(A8,b1,C26),(A8,b1,C26),(A8,b1,C28),(A8,b1,C2 9),(A8,b2,C1),(A8,b2,C2),(A8,b2,C3),(A8,b2,C4),(A8,b2,C5),(A8,b2,C6),(A8,b2,C7),(A8,b2,C 8),(A8,b2,C9),(A8,b2,C10),(A8,b2,C11),(A8,b2,C12),(A8,b2,C13),(A8,b2,C14),(A8,b2,C15),( A8,b2,C16),(A8,b2,C17),(A8,b2,C18),(A8,b2,C19),(A8,b2,C20),(A8,b2,C21),(A8,b2,C22),(A8 ,b2,C23),(A8,b2,C24),(A8,b2,C25),(A8,b2,C26),(A8,b2,C2?),(A8,b2,C28),(A8,b2,C29),(A8,b3 ,C1),(A8,b3,C2),(A8,b3,C3),(A8,b3,C4),(A8,b3,C5),(A8,b3,C6),(A8,b3,C7),(A8,b3,C8),(A8,b3 ,C9),(A8,b3,C10),(A8,b3,C11),(A8,b3,C12),(A8,b3,C13),(A8,b3,C14),(A8,b3,C15),(A8,b3,C1 6),(A8,b3,C17),(A8,b3,C18),(A8,b3,C19),(A8,b3,C20),(A8,b3,C21),(A8,b3,C22),(A8,b3,C23), (A8,b3,C24),(A8,b3,C25),(A8,b3,C26),(A8,b3,C27),(A8,b3,C28),(A8,b3,C29),(A8,b4,C1),(A8, b4,C2),(A8,b4,C3),(A8,b4,C4),(A8,b4,C5),(A8,b4,C6),(A8,b4,C7),(A8,b4,C8),(A8,b4,C9),(A8, b4,C10),(A8,b4,C11),(A8,b4,C12),(A8,b4,C13),(A8,b4,C14),(A8,b4,C15),(A8,b4,C16),(A8,b4, C17),(A8,b4,C18),(A8,b4,C19),(A8,b4,C20),(A8,b4,C21),(A8,b4,C22),(A8,b4,C23),(A8,b4,C2 4),(A8,b4,C25),(A8,b4,C26),(A8,b4,C27),(A8,b4,C28),(A8,b4,C29),(A8,b5,C1),(A8,b5,C2),(A 8,b5,C3),(A8,b5,C4),(A8,b5,C5),(A8,b5,C6),(A8,b5,C7),(A8,b5,C8),(A8,b5,C9),(A8,b5,C10),( A8,b5,C11),(A8,b5,C12),(A8,b5,C13),(A8,b5,C14),(A8,b5,C15),(A8,b5,C16),(A8,b5,C17),(A8 ,b5,C18),(A8,b5,C19),(A8,b5,C20),(A8,b5,C21),(A8,b5,C22),(A8,b5,C23),(A8,b5,C24),(A8,b5 ,C25),(A8,b5,C26),(A8,b5,C27),(A8,b5,C28),(A8,b5,C29),(A8,b6,C1),(A8,b6,C2),(A8,b6,C3),( A8,b6,C4),(A8,b6,C5),(A8,b6,C6),(A8,b6,C7),(A8,b6,C8),(A8,b6,C9),(A8,b6,C10),(A8,b6,C11 ),(A8,b6,C12),(A8,b6,C13),(A8,b6,C14),(A8,b6,C15),(A8,b6,C16),(A8,b6,C17),(AB,b6,C18),( A8,b6,C19),(A8,b6,C20),(A8,b6,C21),(A8,b6,C22),(A8,b6,C23),(A8,b6,C24),(A8,b6,C25),(A8 ,b6,C26),(A8,b6,C27),(A8,b6,C28),(A8,b6,C29),(A8,b7,C1),(A8,b7,C2),(A8,b7,C3),(A8,b7,C4 ),(A8,b7,C5),(A8,b7,C6),(A8,b7,C7),(A8,b7,C8),(A8,b7,C9),(A8,b7,C10),(A8,b7,C11),(A8,b7, C12),(A8,b7,C13),(A8,b7,C4),(A8,b7,C5),(A8,b7,C16),(A8,b7,C17),(A8,b7,C8),(A8,b7,C1 9),(A8,b7,C20),(A8,b7,C21),(A8,b7,C22),(A8,b7,C23),(A8,b7,C24),(A8,b7,C25),(A8,b7,C26), (A8,b7,C27),(A8,b7,C28),(A8,b7,C29),(A9,b1,C1),(A9,b1,C2),(A9,b1,C3),(A9,b1,C4),(A9,b1, C5),(A9,b1,C6),(A9,b1,C7),(A9,b1,C8),(A9,b1,C9),(A9,b1,C10),(A9,b1,C11),(A9,b1,C12),(A9 ,b1,C13),(A9,b1,C14),(A9,b1,C15),(A9,b1,C16),(A9,b1,C17),(A9,b1,C18),(A9,b1,C19),(A9,b1 ,C20),(A9,b1,C21),(A9,b1,C22),(A9,b1,C23),(A9,b1,C24),(A9,b1,C25),(A9,b1,C26),(A9,b1,C 27),(A9,b1,C28),(A9,b1,C29),(A9,b2,C1),(A9,b2,C2),(A9,b2,C3),(A9,b2,C4),(A9,b2,C5),(A9,b 2,C6),(A9,b2,C7),(A9,b2,C8),(A9,b2,C9),(A9,b2,C10),(A9,b2,C11),(A9,b2,C12),(A9,b2,C13),( A9,b2,C14),(A9,b2,C15),(A9,b2,C16),(A9,b2,C17),(A9,b2,C18),(A9,b2,C19),(A9,b2,C20),(A9 ,b2,C21),(A9,b2,C22),(A9,b2,C23),(A9,b2,C24),(A9,b2,C25),(A9,b2,C26),(A9,b2,C27),(A9,b2 ,C28),(A9,b2,C29),(A9,b3,C1),(A9,b3,C2),(A9,b3,C3),(A9,b3,C4),(A9,b3,C5),(A9,b3,C6),(A9, b3,C7),(A9,b3,C8),(A9,b3,C9),(A9,b3,C10),(A9,b3,C11),(A9,b3,C12),(A9,b3,C13),(A9,b3,C1 4),(A9,b3,C15),(A9,b3,C16),(A9,b3,C17),(A9,b3,C18),(A9,b3,C19),(A9,b3,C20),(A9,b3,C21), (A9,b3,C22),(A9,b3,C23),(A9,b3,C24),(A9,b3,C25),(A9,b3,C26),(A9,b3,C27),(A9,b3,C28),(A 9,b3,C29),(A9,b4,C1),(A9,b4,C2),(A9,b4,C3),(A9,b4,C4),(A9,b4,C5),(A9,b4,C6),(A9,b4,C7), A9,b4,C8),(A9,b4,C9),(A9,b4,C10),(A9,b4,C11),(A9,b4,C12),(A9,b4,C13),(A9,b4,C14),(A9,b 4,C15),(A9,b4,C16),(A9,b4,C17),(A9,b4,C18),(A9,b4,C19),(A9,b4,C20),(A9,b4,C21),(A9,b4, C22),(A9,b4,C23),(A9,b4,C24),(A9,b4,C25),(A9,b4,C26),(A9,b4,C27),(A9,b4,C28),(A9,b4,C2 9),(A9,b5,C1),(A9,b5,C2),(A9,b5,C3),(A9,b5,C4),(A9,b5,C5),(A9,b5,C6),(A9,b5,C7),(A9,b5,C 8),(A9,b5,C9),(A9,b5,C10),(A9,b5,C11),(A9,b5,C12),(A9,b5,C13),(A9,b5,C14),(A9,b5,C15),( A9,b5,C16),(A9,b5,C17),(A9,b5,C18),(A9,b5,C19),(A9,b5,C20),(A9,b5,C21),(A9,b5,C22),(A9 ,b5,C23),(A9,b5,C24),(A9,b5,C25),(A9,b5,C26),(A9,b5,C27),(A9,b5,C28),(A9,b5,C29),(A9,b6 ,C1),(A9,b6,C2),(A9,b6,C3),(A9,b6,C4),(A9,b6,C5),(A9,b6,C6),(A9,b6,C7),(A9,b6,C8),(A9,b6 ,C9),(A9,b6,C10),(A9,b6,C11),(A9,b6,C12),(A9,b6,C13),(A9,b6,C14),(A9,b6,C15),(A9,b6,C1 6),(A9,b6,C17),(A9,b6,C18),(A9,b6,C19),(A9,b6,C20),(A9,b6,C21),(A9,b6,C22),(A9,b6,C23), (A9,b6,C24),(A9,b6,C25),(A9,b6,C26),(A9,b6,C27),(A9,b6,C28),(A9,b6,C29),(A9,b7,C1),(A9, b7,C2),(A9,b7,C3),(A9,b7,C4),(A9,b7,C5),(A9,b7,C6),(A9,b7,C7),(A9,b7,C8),(A9,b7,C9),(A9, b7,C10),(A9,b7,C11),(A9,b7,C12),(A9,b7,C13),(A9,b7,C14),(A9,b7,C15),(A9,b7,C16),(A9,b7, C17),(A9,b7,C18),(A9,b7,C19),(A9,b7,C20),(A9,b7,C21),(A9,b7,C22),(A9,b7,C23),(A9,b7,C2 4),(A9,b7,C25),(A9,b7,C26),(A9,b7,C27),(A9,b7,C28),(A9,b7,C29),(A10,b1,C1),(A10,b1,C2), (A10,b1,C3),(A10,b1,C4),(A10,b1,C5),(A10,b1,C6),(A10,b1,C7),(A10,b1,C8),(A10,b1,C9),(A 10,b1,C10),(A10,b1,C11),(A10,b1,C12),(A10,b1,C13),(A10,b1,C14),(A10,b1,C15),(A10,b1,C 16),(A10,b1,C17),(A10,b1,C18),(A10,b1,C19),(A10,b1,C20),(A10,b1,C21),(A10,b1,C22),(A10 ,b1,C23),(A10,b1,C24),(A10,b1,C25),(A10,b1,C26),(A10,b1,C27),(A10,b1,C28),(A10,b1,C29) ,(A10,b2,C1),(A10,b2,C2),(A10,b2,C3),(A10,b2,C4),(A10,b2,C5),(A10,b2,C6),(A10,b2,C7),(A 10,b2,C8),(A10,b2,C9),(A10,b2,C10),(A10,b2,C11),(A10,b2,C12),(A10,b2,C13),(A10,b2,C14) ,(A10,b2,C15),(A10,b2,C16),(A10,b2,C17),(A10,b2,C18),(A10,b2,C19),(A10,b2,C20),(A10,b2 ,C21),(A10,b2,C22),(A10,b2,C23),(A10,b2,C24),(A10,b2,C25),(A10,b2,C26),(A10,b2,C27),(A 10,b2,C28),(A10,b2,C29),(A10,b3,C1),(A10,b3,C2),(A10,b3,C3),(A10,b3,C4),(A10,b3,C5),(A 10,b3,C6),(A10,b3,C7),(A10,b3,C8),(A10,b3,C9),(A10,b3,C10),(A10,b3,C11),(A10,b3,C12),( A10,b3,C13),(A10,b3,C14),(A10,b3,C15),(A10,b3,C16),(A10,b3,C17),(A10,b3,C18),(A10,b3, C19),(A10,b3,C20),(A10,b3,C21),(A10,b3,C22),(A10,b3,C23),(A10,b3,C24),(A10,b3,C25),(A 10,b3,C26),(A10,b3,C27),(A10,b3,C28),(A10,b3,C29),(A10,b4,C1),(A10,b4,C2),(A10,b4,C3),( A10,b4,C4),(A10,b4,C5),(A10,b4,C6),(A10,b4,C7),(A10,b4,C8),(A10,b4,C9),(A10,b4,C10),(A 10,b4,C11),(A10,b4,C,12),(A10,b4,C13),(A10,b4,C14),(A10,b4,C15),(A10,b4,C16),(A10,b4,C 17),(A10,b4,C18),(A10,b4,C19),(A10,b4,C20),(A10,b4,C21),(A10,b4,C22),(A10,b4,C23),(A10 ,b4,C24),(A10,b4,C25),(A10,b4,C26),(A10,b4,C27),(A10,b4,C28),(A10,b4,C29),(A10,b5,C1),( A10,b5,C2),(A10,b5,C3),(A10,b5,C4),(A10,b5,C5),(A10,b5,C6),(A10,b5,C7),(A10,b5,C8),(A1 0,b5,C9),(A10,b5,C10),(A10,b5,C11),(A10,b5,C12),(A10,b5,C13),(A10,b5,C14),(A10,b5,C15) ,(A10,b5,C16),(A10,b5,C17),(A10,b5,C18),(A10,b5,C19),(A10,b5,C20),(A10,b5,C21),(A10,b5 ,C22),(A10,b5,C23),(A10,b5,C24),(A10,b5,C25),(A10,b5,C26),(A10,b5,C27),(A10,b5,C28),(A 10,b5,C29),(A10,b6,C1),(A10,b6,C2),(A10,b6,C3),(A10,b6,C4),(A10,b6,C5),(A10,b6,C6),(A1 0,b6,C7),(A10,b6,C8),(A10,b6,C9),(A10,b6,C10),(A10,b6,C11),(A10,b6,C12),(A10,b6,C13),( A10,b6,C14),(A10,b6,C15),(A10,b6,C16),(A10,b6,C17),(A10,b6,C18),(A10,b6,C19),(A10,b6, C20),(A10,b6,C21),(A10,b6,C22),(A10,b6,C23),(A10,b6,C24),(A10,b6,C25),(A10,b6,C26),(A 10,b6,C27),(A10,b6,C28),(A10,b6,C29),(A10,b7,C1),(A10,b7,C2),(A10,b7,C3),(A10,b7,C4),( A10,b7,C5),(A10,b7,C6),(A10,b7,C7),(A10,b7,C8),(A10,b7,C9),(A10,b7,C10),(A10,b7,C11),( A10,b7,C12),(A10,b7,C13),(A10,b7,C14),(A10,b7,C15),(A10,b7,C16),(A10,b7,C17),(A10,b7, C18),(A10,b7,C19),(A10,b7,C20),(A10,b7,C21),(A10,b7,C22),(A10,b7,C23),(A10,b7,C24),(A 10,b7,C25),(A10,b7,C26),(A10,b7,C27),(A10,b7,C28),(A10,b7,C29),

(A11,b1,C1),(A11,b1,C2),(A11,b1,C3),(A11,b1,C4),(A11,b1,C5),(A11,b1,C6),(A11,b1,C7),(A 11,b1,C8),(A11,b1,C9),(A11,b1,C10),(A11,b1,C11),(A11,b1,C12),(A11,b1,C13),(A11,b1,C14) ,(A11,b1,C15),(A11,b1,C16),(A11,b1,C17),(A11,b1,C18),(A11,b1,C19),(A11,b1,C20),(A11,b1 ,C21),(A11,b1,C22),(A11,b1,C23),(A11,b1,C24),(A11,b1,C25),(A11,b1,C26),(A11,b1,C27),(A 11,b1,C28),(A11,b1,C29),(A11,b2,C1),(A11,b2,C2),(A11,b2,C3),(A11,b2,C4),(A11,b2,C5),(A 11,b2,C6),(A11,b2,C7),(A11,b2,C8),(A11,b2,C9),(A11,b2,C10),(A11,b2,C11),(A11,b2,C12),( A11,b2,C13),(A11,b2,C14),(A11,b2,C15),(A11,b2,C16),(A11,b2,C17),(A11,b2,C18),(A11,b2, C19),(A11,b2,C20),(A11,b2,C21),(A11,b2,C22),(A11,b2,C23),(A11,b2,C24),(A11,b2,C25),(A 11,b2,C26),(A11,b2,C21),(A11,b2,C28),(A11,b2,C29),(A11,b3,C1),(A11,b3,C2),(A11,b3,C3),( A11,b3,C4),(A11,b3,C5),(A11,b3,C6),(A11,b3,C7),(A11,b3,C8),(A11,b3,C9),(A11,b3,C10),(A 11,b3,C11),(A11,b3,C12),(A11,b3,C13),(A11,b3,C14),(A11,b3,C15),(A11,b3,C16),(A11,b3,C 17),(A11,b3,C18),(A11,b3,C19),(A11,b3,C20),(A11,b3,C21),(A11,b3,C22),(A11,b3,C23),(A11 ,b3,C24),(A11,b3,C25),(A11,b3,C26),(A11,b3,C27),(A11,b3,C28),(A11,b3,C29),(A11,b4,C1),( A11,b4,C2),(A11,b4,C3),(A11,b4,C4),(A11,b4,C5),(A11,b4,C6),(A11,b4,C7),(A11,b4,C8),(A1 1,b4,C9),(A11,b4,C10),(A11,b4,C11),(A11,b4,C12),(A11,b4,C13),(A11,b4,C14),(A11,b4,C15) ,(A11,b4,C16),(A11,b4,C17),(A11,b4,C18),(A11,b4,C19),(A11,b4,C20),(A11,b4,C21),(A11,b4 ,C22),(A11,b4,C23),(A11,b4,C24),(A11,b4,C25),(A11,b4,C26),(A11,b4,C27),(A11,b4,C28),(A 11,b4,C29),(A11,b5,C1),(A11,b5,C2),(A11,b5,C3),(A11,b5,C4),(A11,b5,C5),(A11,b5,C6),(A1 1,b5,C7),(A11,b5,C8),(A11,b5,C9),(A11,b5,C10),(A11,b5,C11),(A11,b5,C12),(A11,b5,C13),( A11,b5,C14),(A11,b5,C15),(A11,b5,C16),(A11,b5,C17),(A11,b5,C18),(A11,b5,C19),(A11,b5, C20),(A11,b5,C21),(A11,b5,C22),(A11,b5,C23),(A11,b5,C24),(A11,b5,C25),(A11,b5,C26),(A 11,b5,C27),(A11,b5,C28),(A11,b5,C29),(A11,b6,C1),(A11,b6,C2),(A11,b6,C3),(A11,b6,C4),( A11,b6,C5),(A11,b6,C6),(A11,b6,C7),(A11,b6,C8),(A11,b6,C9),(A11,b6,C10),(A11,b6,C11),( A11,b6,C12),(A11,b6,C13),(A11,b6,C14),(A11,b6,C15),(A11,b6,C16),(A11,b6,C17),(A11,b6, C18),(A11,b6,C19),(A11,b6,C20),(A11,b6,C21),(A11,b6,C22),(A11,b6,C23),(A11,b6,C24),(A 11,b6,C25),(A11,b6,C26),(A11,b6,C27),(A11,b6,C28),(A11,b6,C29),(A11,b7,C1),(A11,b7,C2) ,(A11,b7,C3),(A11,b7,C4),(A11,b7,C5),(A11,b7,C6),(A11,b7,C7),(A11,b7,C8),(A11,b7,C9),(A 11,b7,C10),(A11,b7,C11),(A11,b7,C12),(A11,b7,C13),(A11,b7,C14),(A11,b7,C15),(A11,b7,C 16),(A11,b7,C17),(A11,b7,C18),(A11,b7,C19),(A11,b7,C20),(A11,b7,C21),(A11,b7,C22),(A11 ,b7,C23),(A11,b7,C24),(A11,b7,C25),(A11,b7,C26),(A11,b7,C27),(A11,b7,C28),(A11,b7,C29) ,(A12,b1,C1),(A12,b1,C2),(A12,b1,C3),(A12,b1,C4),(A12,b1,C5),(A12,b1,C6),(A12,b1,C7),(A 12,b1,C8),(A12,b1,C9),(A12,b1,C10),(A12,b1,C11),(A12,b1,C12),(A12,b1,C13),(A12,b1,C14) ,(A12,b1,C15),(A12,b1,C16),(A12,b1,C17),(A12,b1,C18),(A12,b1,C19),(A12,b1,C20),(A12,b1 ,C21),(A12,b1,C22),(A12,b1,C23),(A12,b1,C24),(A12,b1,C25),(A12,b1,C26),(A12,b1,C27),(A 12,b1,C28),(A12,b1,C29),(A12,b2,C1),(A12,b2,C2),(A12,b2,C3),(A12,b2,C4),(A12,b2,C5),(A 12,b2,C6),(A12,b2,C7),(A12,b2,C8),(A12,b2,C9),(A12,b2,C10),(A12,b2,C11),(A12,b2,C12),( A12,b2,C13),(A12,b2,C14),(A12,b2,C15),(A12,b2,C16),(A12,b2,C17),(A12,b2,C18),(A12,b2, C19),(A12,b2,C20),(A12,b2,C21),(A12,b2,C22),(A12,b2,C23),(A12,b2,C24),(A12,b2,C25),(A 12,b2,C26),(A12,b2,C27),(A12,b2,C28),(A12,b2,C29),(A12,b3,C1),(A12,b3,C2),(A12,b3,C3),( A12,b3,C4),(A12,b3,C5),(A12,b3,C6),(A12,b3,C7),(A12,b3,C8),(A12,b3,C9),(A12,b3,C10),(A 12,b3,C11),(A12,b3,C12),(A12,b3,C13),(A12,b3,C14),(A12,b3,C15),(A12,b3,C16),(A12,b3,C 17),(A12,b3,C18),(A12,b3,C19),(A12,b3,C20),(A12,b3,C21),(A12,b3,C22),(A12,b3,C23),(A12 ,b3,C24),(A12,b3,C25),(A12,b3,C26),(A12,b3,C27),(A12,b3,C28),(A12,b3,C29),(A12,b4,C1),( A12,b4,C2),(A12,b4,C3),(A12,b4,C4),(A12,b4,C5),(A12,b4,C6),(A12,b4,C7),(A12,b4,C8),(A1 2,b4,C9),(A12,b4,C10),(A12,b4,C11),(A12,b4,C12),(A12,b4,C13),(A12,b4,C14),(A12,b4,C15) ,(A12,b4,C16),(A12,b4,C17),(A12,b4,C18),(A12,b4,C19),(A12,b4,C20),(A12,b4,C21),(A12,b4 ,C22),(A12,b4,C23),(A12,b4,C24),(A12,b4,C25),(A12,b4,C26),(A12,b4,C27),(A12,b4,C28),(A 12,b4,C29),(A12,b5,C1),(A12,b5,C2),(A12,b5,C3),(A12,b5,C4),(A12,b5,C5),(A12,b5,C6),(A1 2,b5,C7),(A12,b5,C8),(A12,b5,C9),(A12,b5,C10),(A12,b5,C11),(A12,b5,C12),(A12,b5,C13),( A12,b5,C14),(A12,b5,C15),(A12,b5,C16),(A12,b5,C17),(A12,b5,C18),(A12,b5,C19),(A12,b5, C20),(A12,b5,C21),(A12,b5,C22),(A12,b5,C23),(A12,b5,C24),(A12,b5,C25),(A12,b5,C26),(A 12,b5,C27),(A12,b5,C28),(A12,b5,C29),(A12,b6,C1),(A12,b6,C2),(A12,b6,C3),(A12,b6,C4),( A12,b6,C5),(A12,b6,C6),(A12,b6,C7),(A12,b6,C8),(A12,b6,C9),(A12,b6,C10),(A12,b6,C11),( A12,b6,C12),(A12,b6,C13),(A12,b6,C14),(A12,b6,C15),(A12,b6,C16),(A12,b6,C17),(A12,b6, C18),(A12,b6,C19),(A12,b6,C20),(A12,b6,C21),(A12,b6,C22),(412,b6,C23),(A12,b6,C24),(A 12,b6,C25),(A12,b6,C26),(A12,b6,C27),(A12,b6,C28),(A12,b6,C29),(A12,b7,C1),(A12,b7,C2) ,(A12,b7,C3),(A12,b7,C4),(A12,b7,C5),(A12,b7,C6),(A12,b7,C7),(A12,b7,C8),(A12,b7,C9),(A 12,b7,C10),(A12,b7,C11),(A12,b7,C12),(A12,b7,C13),(A12,b7,C14),(A12,b7,C15),(A12,b7,C 16),(A12,b7,C17),(A12,b7,C18),(A12,b7,C19),(A12,b7,C20),(A12,b7,C21),(A12,b7,C22),(A12 ,b7,C23),(A12,b7,C24),(A12,b7,C25),(A12,b7,C26),(A12,b7,C27),(A12,b7,C28),(A12,b7,C29) ,(A13,b1,C1),(A13,b1,C2),(A13,b1,C3),(A13,b1,C4),(A13,b1,C5),(A13,b1,C6),(A13,b1,C7),(A 13,b1,C8),(A13,b1,C9),(A13,b1,C10),(A13,b1,C11),(A13,b1,C12),(A13,b1,C13),(A13,b1,C14) ,(A13,b1,C15),(A13,b1,C16),(A13,b1,C17),(A13,b1,C18),(A13,b1,C19),(A13,b1,C20),(A13,b1 ,C21),(A13,b1,C22),(A13,b1,C23),(A13,b1,C24),(A13,b1,C25),(A13,b1,C26),(A13,b1,C27),(A 13,b1,C28),(A13,b1,C29),(A13,b2,C1),(A13,b2,C2),(A13,b2,C3),(A13,b2,C4),(A13,b2,C5),(A 13,b2,C6),(A13,b2,C7),(A13,b2,C8),(A13,b2,C9),(A13,b2,C10),(A13,b2,C11),(A13,b2,C12),( A13,b2,C13),(A13,b2,C14),(A13,b2,C15),(A13,b2,C16),(A13,b2,C17),(A13,b2,C18),(A13,b2, C19),(A13,b2,C20),(A13,b2,C21),(A13,b2,C22),(A13,b2,C23),(A13,b2,C24),(A13,b2,C25),(A 13,b2,C26),(A13,b2,C27),(A13,b2,C28),(A13,b2,C29),(A13,b3,C1),(A13,b3,C2),(A13,b3,C3),( A13,b3,C4),(A13,b3,C5),(A13,b3,C6),(A13,b3,C7),(A13,b3,C8),(A13,b3,C9),(A13,b3,C10),(A 13,b3,C11),(A13,b3,C12),(A13,b3,C13),(A13,b3,C14),(A13,b3,C15),(A13,b3,C16),(A13,b3,C 17),(A13,b3,C18),(A13,b3,C19),(A13,b3,C20),(A13,b3,C21),(A13,b3,C22),(A13,b3,C23),(A13 ,b3,C24),(A13,b3,C25),(A13,b3,C26),(A13,b3,C27),(A13,b3,C28),(A13,b3,C29),(A13,b4,C1),( A13,b4,C2),(A13,b4,C3),(A13,b4,C4),(A13,b4,C5),(A13,b4,C6),(A13,b4,C7),(A13,b4,C8),(A1 3,b4,C9),(A13,b4,C10),(A13,b4,C11),(A13,b4,C12),(A13,b4,C13),(A13,b4,C14),(A13,b4,C15) ,(A13,b4,C16),(A13,b4,C17),(A13,b4,C18),(A13,b4,C19),(A13,b4,C20),(A13,b4,C21),(A13,b4 ,C22),(A13,b4,C23),(A13,b4,C24),(A13,b4,C25),(A13,b4,C26),(A13,b4,C27),(A13,b4,C28),(A 13,b4,C29),(A13,b5,C1),(A13,b5,C2),(A13,b5,C3),(A13,b5,C4),(A13,b5,C5),(A13,b5,C6),(A1 3,b5,C7),(A13,b5,C8),(A13,b5,C9),(A13,b5,C10),(A13,b5,C11),(A13,b5,C12),(A13,b5,C13),( A13,b5,C14),(A13,b5,C15),(A13,b5,C16),(A13,b5,C17),(A13,b5,C18),(A13,b5,C19),(A13,b5, C20),(A13,b5,C21),(A13,b5,C22),(A13,b5,C23),(A13,b5,C24),(A13,b5,C25),(A13,b5,C26),(A 13,b5,C27),(A13,b5,C28),(A13,b5,C29),(A13,b6,C2),(A13,b6,C2),(A13,b0,C3),(A13,b6,C4),( A13,b6,C5),(A13,b6,C6),(A13,b6,C7),(A13,b6,C8),(A13,b6,C9),(A13,b6,C10),(A13,b6,C11),( A13,b6,C12),(A13,b6,C13),(A13,b6,C14),(A13,b6,C15),(A13,b6,C16),(A13,b6,C17),(A13,b6, C18),(A13,b6,C29),(A13,b6,C20),(A23,b6,C21),(A13,b6,C22),(A13,b6,C23),(A13,b6,C24),(A 13,b6,C25),(A13,b6,C26),(A13,b6,C27),(A13,b6,C28),(A13,b6,C29),(A13,b7,C1),(A13,b7,C2) ,(A13,b7,C3),(A13,b7,C4),(A13,b7,C5),(A13,b7,C6),(A13,b7,C7),(A13,b7,C8),(A13,b7,C9),(A 13,b7,C10),(A13,b7,C11),(A13,b7,C12),(A13,b7,C13),(A13,b7,C14),(A13,b7,C15),(A13,b7,C 16),(A13,b7,C17),(A13,b7,C18),(A13,b7,C19),(A13,b7,C20),(A13,b7,C21),(A13,b7,C22),(A13 ,b7,C23),(A13,b7,C24),(A13,b7,C25),(A13,b7,C26),(A13,b7,C27),(A13,b7,C28),(A13,b7,C29) ,(A14,b1,C1),(A14,b1,C2),(A14,b1,C3),(A14,b1,C4),(A14,b1,CS),(A14,b1,C6),(A14,b1,C7),(A 14,b1,C8),(A14,b1,C9),(A14,b1,C10),(A14,b1,C11),(A14,b1,C12),(A14,b1,C13),(A14,b1,C14) ,(A14,b1,C15),(A14,b1,C16),(A14,b1,C17),(A14,b1,C1B),(A14,b1,C19),(A14,b1,C20),(A14,b1 ,C21),(A14,b1,C22),(A14,b1,C23),(A14,b1,C24),(A14,b1,C25),(A14,b1,C26),(A14,b1,C27),(A 14,b1,C28),(A14,b1,C29),(A14,b2,C1),(A14,b2,C2),(A14,b2,C3),(A14,b2,C4),(A14,b2,C5),(A 14,b2,C6),(A14,b2,C7),(A14,b2,C8),(A14,b2,C9),(A14,b2,C10),(A14,b2,C11),(A14,b2,C12),( A14,b2,C13),(A14,b2,C14),(A14,b2,C15),(A14,b2,C16),(A14,b2,C17),(A14,b2,C18),(A14,b2, C19),(A14,b2,C20),(A14,b2,C21),(A14,b2,C22),(A14,b2,C23),(A14,b2,(C24),(A14,b2,C25),(A 14,b2,C26),(A14,b2,C27),(A14,b2,C28),(A14,b2,C29),(A14,b3,C1),(A14,b3,C2),(A14,b3,C3),( A14,b3,C4),(A14,b3,C5),(A14,b3,C6),(A14,b3,C7),(A14,b3,C8),(A14,b3,C9),(A14,b3,C10),(A 14,b3,C11),(A14,b3,C12),(A14,b3,C13),(A14,b3,C14),(A14,b3,C15),(A14,b3,C16),(A14,b3,C 17),(A14,b3,C18),(A14,b3,C19),(A14,b3,C20),(A14,b3,C21),(A14,b3,C22),(A14,b3,C23),(A14 ,b3,C24),(A14,b3,C25),(A14,b3,C26),(A14,b3,C27),(A14,b3,C28),(A14,b3,C29),(A14,b4,C1),( A14,b4,C2),(A14,b4,C3),(A14,b4,C4),(A14,b4,C5),(A14,b4,C6),(A14,b4,C7),(A14,b4,C8),(A1 4,b4,C9),(A14,b4,C10),(A14,b4,C11),(A14,b4,C12),(A14,b4,C13),(A14,b4,C14),(A14,b4,C15) ,(A14,b4,C16),(A14,b4,C17),(A14,b4,C18),(A14,b4,C19),(A14,b4,C20),(A14,b4,C21),(A14,b4 ,C22),(A14,b4,C23),(A14,b4,C24),(A14,b4,C25),(A14,b4,C26),(A14,b4,C27),(A14,b4,C28),(A 14,b4,C29),(A14,b5,C1),(A14,b5,C2),(A14,b5,C3),(A14,b5,C4),(A14,b5,C5),(A14,b5,C6),(A1 4,b5,C7),(A14,b5,C8),(A14,b5,C9),(A14,b5,C10),(A14,b5,C11),(A14,b5,C12),(A14,b5,C13),( A14,b5,C14),(A14,b5,C15),(A14,b5,C16),(A14,b5,C17),(A14,b5,C18),(A14,b5,C19),(A14,b5, C20),(A14,b5,C21),(A14,b5,C22),(A14,b5,C23),(A14,b5,C24),(A14,b5,C25),(A14,b5,C26),(A 14,b5,C27),(A14,b5,C28),(A14,b5,C29),(A14,b6,C1),(A14,b6,C2),(A14,b6,C3),(A14,b6,C4),( A14,b6,C5),(A14,b6,C6),(A14,b6,C7),(A14,b6,C8),(A14,b6,C9),(A14,b6,C10),(A14,b6,C11),( A14,b6,C12),(A14,b6,C13),(A14,b6,C14),(A14,b6,C15),(A14,b6,C16),(A14,b6,C17),(A14,b6, C18),(A14,b6,C19),(A14,b6,C20),(A14,b6,C21),(A14,b6,C22),(A14,b6,C23),(A14,b6,C24),(A 14,b6,C25),(A14,b6,C26),(A14,b6,C27),(A14,b6,C28),(A14,b6,C29),(A14,b7,C1),(A14,b7,C2) ,(A14,b7,C3),(A14,b7,C4),(A14,b7,C5),(A14,b7,C6),(A14,b7,C7),(A14,b7,C8),(A14,b7,C9),(A 14,b7,C10),(A14,b7,C11),(A14,b7,C12),(A14,b7,C13),(A14,b7,C14),(A14,b7,C15),(A14,b7,C 16),(A14,b7,C17),(A14,b7,C18),(A14,b7,C19),(A14,b7,C20),(A14,b7,C21),(A14,b7,C22),(A14 ,b7,C23),(A14,b7,C24),(A14,b7,C25),(A14,b7,C26),(A14,b7,C27),(A14,b7,C28),(A14,b7,C29) ,(A15,b1,C1),(A15,b1,C2),(A15,b1,C3),(A15,b1,C4),(A15,b1,C5),(A15,b1,C6),(A15,b1,C7),(A 15,b1,C8),(A15,b1,C9),(A15,b1,C10),(A15,b1,C12),(A15,b1,C12),(A15,b1,C13),(A15,b1,C14) ,(A15,b1,C15),(A15,b1,C16),(A15,b1,C17),(A15,b1;C18),(A15,b1,C19),(A15,b1,C20),(A15,b1 ,C21),(A15,b1,C22),(A15,b1,C23),(A15,b1,C24),(A15,b1,C25),(A15,b1,C26),(A15,b1,C27),(A 15,b1,C28),(A15,b1,C29),(A15,b2,C1),(A15,b2,C2),(A15,b2,C3),(A15,b2,C4),(A15,b2,C5),(A 15,b2,C6),(A15,b2,C7),(A15,b2,C8),(A15,b2,C9),(A15,b2,C10),(A15,b2,C11),(A15,b2,C12),( A15,b2,C13),(A15,b2,C14),(A15,b2,C15),(A15,b2,C16),(A15,b2,C17),(A15,b2,C18),(A15,b2, C19),(A15,b2,C20),(A15,b2,C21),(A15,b2,C22),(A15,b2,C23),(A15,b2,C24),(A15,b2,C25),(A 15,b2,C26),(A15,b2,C27),(A15,b2,C28),(A15,b2,C29),(A15,b3,C1),(A15,b3,C2),(A15,b3,C3),( A15,b3,C4),(A15,b3,C5),(A15,b3,C6),(A15,b3,C7),(A15,b3,C8),(A15,b3,C9),(A15,b3,C10),(A 15,b3,C11),(A15,b3,C12),(A15,b3,C13),(A15,b3,C14),(A15,b3,C15),(A15,b3,C16),(A15,b3,C 17),(A15,b3,C18),(A15,b3,C19),(A15,b3,C20),(A15,b3,C21),(A15,b3,C22),(A15,b3,C23),(A15 ,b3,C24),(A15,b3,C25),(A15,b3,C26),(A15,b3,C27),(A15,b3,C28),(A15,b3,C29),(A15,b4,C1),( A15,b4,C2),(A15,b4,C3),(A15,b4,C4),(A15,b4,C5),(A15,b4,C6),(A15,b4,C7),(A15,b4,C8),(A1 5,b4,C9),(A15,b4,C10),(A15,b4,C11),(A15,b4,C12),(A15,b4,C13),(A15,b4,C14),(A15,b4,C15) ,(A15,b4,C16),(A15,b4,C17),(A15,b4,C18),(A15,b4,C19),(A15,b4,C20),(A15,b4,C21),(A15,b4 ,C22),(A15,b4,C23),(A15,b4,C24),(A15,b4,C25),(A15,b4,C26),(A15,b4,C27),(A15,b4,C28),(A 15,b4,C29),(A15,b5,C1),(A15,b5,C2),(A15,b5,C3),(A15,b5,C4),(A15,b5,C5),(A15,b5,C6),(A1 5,b5,C7),(A15,b5,C8),(A15,b5,C9),(A15,b5,C10),(A15,b5,C11),(A15,b5,C12),(A15,b5,C13),( A15,b5,C14),(A15,b5,C15),(A15,b5,C16),(A15,b5,C17),(A15,b5,C18),(A15,b5,C19),(A15,b5, C20),(A15,b5,C21),(A15,b5,C22),(A15,b5,C23),(A15,b5,C24),(A15,b5,C25),(A15,b5,C26),(A 15,b5,C27),(A15,b5,C28),(A15,b5,C29),(A15,b6,C1),(A15,b6,C2),(A15,b6,C3),(A15,b6,C4),( A15,b6,C5),(A15,b6,C6),(A15,b6,C7),(A15,b6,C8),(A15,b6,C9),(A15,b6,C10),(A15,b6,C11),( A15,b6,C12),(A15,b6,C13),(A15,b6,C14),(A15,b6,C15),(A15,b6,C16),(A15,b6,C17),(A15,b6, C18),(A15,b6,C19),(A15,b6,C20),(A15,b6,C21),(A15,b6,C22),(A15,b6,C23),(A15,b6,C24),(A 15,b6,C25),(A15,b6,C26),(A15,b6,C27),(A15,b6,C28),(A15,b6,C29),(A15,b7,C1),(A15,b7,C2) ,(A15,b7,C3),(A15,b7,C4),(A15,b7,C5),(A15,b7,C6),(A15,b7,C7),(A15,b7,C8),(A15,b7,C9),(A 15,b7,C10),(A15,b7,C11),(A15,b7,C12),(A15,b7,C13),(A15,b7,C14),(A15,b7,C15),(A15,b7,C 16),(A15,b7,C17),(A15,b7,C18),(A15,b7,C19),(A15,b7,C20),(A15,b7,C21),(A15,b7,C22),(A15 ,b7,C23),(A15,b7,C24),(A15,b7,C25),(A15,b7,C26),(A15,b7,C27),(A15,b7,C28),(A15,b7,C29) ,(A16,b1,C1),(A16,b1,C2),(A16,b1,C3),(A16,b1,C4),(A16,b1,C5),(A16,b1,C6),(A16,b1,C7),(A 16,b1,C8),(A16,b1,C9),(A16,b1,C10),(A16,b1,C11),(A16,b1,C12),(A16,b1,C13),(A16,b1,C14) ,(A16,b1,C15),(A16,b1,C16),(A16,b1,C17),(A16,b1,C18),(A16,b1,C19),(A16,b1,C20),(A16,b1 ,C21),(A16,b1,C22),(A16,b1,C23),(A16,b1,C24),(A16,b1,C25),(A16,b1,C26),(A16,b1,C27),(A 16,b1,C28),(A16,b1,C29),(A16,b2,C1),(A16,b2,C2),(A16,b2,C3),(A16,b2,C4),(A16,b2,C5),(A 16,b2,C6),(A16,b2,C7),(A16,b2,C8),(A16,b2,C9),(A16,b2,C10),(A16,b2,C11),(A16,b2,C12),( A16,b2,C13),(A16,b2,C14),(A16,b2,C15),(A16,b2,C16),(A16,b2,C17),(A16,b2,C18),(A16,b2, C19),(A16,b2,C20),(A16,b2,C21),(A16,b2,C22),(A16,b2,C23),(A16,b2,C24),(A16,b2,C25),(A 16,b2,C26),(A16,b2,C27),(A16,b2,C28),(A16,b2,C29),(A16,b3,C1),(A16,b3,C2),(A16,b3,C3),( A16,b3,C4),(A16,b3,C5),(A16,b3,C6),(A16,b3,C7),(A16,b3,C8),(A16,b3,C9),(A16,b3,C10),(A 16,b3,C11),(A16,b3,C12),(A16,b3,C13),(A16,b3,C14),(A16,b3,C15),(A16,b3,C16),(A16,b3,C 17),(A16,b3,C18),(A16,b3,C19),(A16,b3,C20),(A16,b3,C21),(A16,b3,C22),(A16,b3,C23),(A16 ,b3,C24),(A16,b3,C25),(A16,b3,C26),(A16,b3,C27),(A16,b3,C28),(A16,b3,C29),(A16,b4,C1),( A16,b4,C2),(A16,b4,C3),(A16,b4,C4),(A16,b4,C5),(A16,b4,C6),(A16,b4,C7),(A16,b4,C8),(A1 6,b4,C9),(A16,b4,C10),(A16,b4,C11),(A16,b4,C12),(A16,b4,C13),(A16,b4,C14),(A16,b4,C15) ,(A16,b4,C16),(A16,b4,C17),(A16,b4,C18),(A16,b4,C19),(A16,b4,C20),(A16,b4,C21),(A16,b4 ,C22),(A16,b4,C23),(A16,b4,C24),(A16,b4,C25),(A16,b4,C26),(A16,b4,C27),(A16,b4,C28),(A 16,b4,C29),(A16,b5,C1),(A16,b5,C2),(A16,b5,C3),(A16,b5,C4),(A16,b5,C5),(A16,b5,C6),(A1 6,b5,C7),(A16,b5,C8),(A16,b5,C9),(A16,b5,C10),(A16,b5,C11),(A16,b5,C12),(A16,b5,C13),( A16,b5,C14),(A16,b5,C15),(A16,b5,C16),(A16,b5,C17),(A16,b5,C18),(A16,b5,C19),(A16,b5, C20),(A16,b5,C21),(A16,b5,C22),(A16,b5,C23),(A16,b5,C24),(A16,b5,C25),(A16,b5,C26),(A 16,b5,C27),(A16,b5,C28),(A16,b5,C29),(A16,b6,C1),(A16,b6,C2),(A16,b6,C3),(A16,b6,C4),( A16,b6,C5),(A16,b6,C6),(A16,b6,C7),(A16,b6,C8),(A16,b6,C9),(A16,b6,C10),(A16,b6,C11),( A16,b6,C12),(A16,b6,C13),(A16,b6,C14),(A16,b6,C15),(A16,b6,C16),(A16,b6,C17),(A16,b6, C18),(A16,b6,C19),(A16,b6,C20),(A16,b6,C21),(A16,b6,C22),(A16,b6,C23),(A16,b6,C24),(A 16,b6,C25),(A16,b6,C26),(A16,b6,C27),(A16,b6,C28),(A16,b6,C29),(A16,b7,C1),(A16,b7,C2) ,(A16,b7,C3),(A16,b7,C4),(A16,b7,C5),(A16,b7,C6),(A16,b7,C7),(A16,b7,C8),(A16,b7,C9),(A 16,b7,C10),(A16,b7,C11),(A16,b7,C12),(A16,b7,C13),(A16,b7,C14),(A16,b7,C15),(A16,b7,C 16),(A16,b7,C17),(A16,b7,C18),(A16,b7,C19),(A16,b7,C20),(A16,b7,C21),(A16,b7,C22),(A16 ,b7,C23),(A16,b7,C24),(A16,b7,C25),(A16,b7,C26),(A16,b7,C27),(A16,b7,C28),(A16,b7,C29) ,(A17,b1,C1),(A17,b1,C2),(A17,b1,C3),(A17,b1,C4),(A17,b1,C5),(A17,b1,C6),(A17,b1,C7),(A 17,b1,C8),(A17,b1,C9),(A17,b1,C10),(A17,b1,C11),(A17,b1,C12),(A17,b1,C13),(A17,b1,C14) ,(A17,b,C15),(A17,b,C16),(A17,b1,C17),(A17,b1,C18),(A17,b1,C19),(A17,b1,C20),(A17,b1 ,C21),(A17,b1,C22),(A17,b1,C23),(A17,b1,C24),(A17,b1,C25),(A17,b1,C26),(A17,b1,C27),(A 17,b1,C28),(A17,b1,C29),(A17,b2,C1),(A17,b2,C2),(A17,b2,C3),(A17,b2,C4),(A17,b2,C5),(A 17,b2,C6),(A17,b2,C7),(A17,b2,C8),(A17,b2,C9),(A17,b2,C10),(A17,b2,C11),(A17,b2,C12),( A17,b2,C13),(A17,b2,C14),(A17,b2,C15),(A17,b2,C16),(A17,b2,C17),(A17,b2,C18),(A17,b2, C19),(A17,b2,C20),(A17,b2,C21),(A17,b2,C22),(A17,b2,C23),(A17,b2,C24),(A17,b2,C25),(A 17,b2,C26),(A17,b2,C27),(A17,b2,C28),(A17,b2,C29),(A17,b3,C1),(A17,b3,C2),(A17,b3,C3),( A17,b3,C4),(A17,b3,C5),(A17,b3,C6),(A17,b3,C7),(A17,b3,C8),(A17,b3,C9),(A17,b3,C10),(A 17,b3,C11),(A17,b3,C12),(A17,b3,C13),(A17,b3,C14),(A17,b3,C15),(A17,b3,C16),(A17,b3,C 17),(A17,b3,C18),(A17,b3,C19),(A17,b3,C20),(A17,b3,C21),(A17,b3,C22),(A17,b3,C23),(A17 ,b3,C24),(A17,b3,C25),(A17,b3,C26),(A17,b3,C27),(A17,b3,C28),(A17,b3,C29),(A17,b4,C1),( A17,b4,C2),(A17,b4,C3),(A17,b4,C4),(A17,b4,C5),(A17,b4,C6),(A17,b4,C7),(A17,b4,C8),(A1 7,b4,C9),(A17,b4,C10),(A17,b4,C11),(A17,b4,C12),(A17,b4,C13),(A17,b4,C14),(A17,b4,C15) ,(A17,b4,C16),(A17,b4,C17),(A17,b4,C18),(A17,b4,C19),(A17,b4,C20),(A17,b4,C21),(A17,b4 ,C22),(A17,b4,C23),(A17,b4,C24),(A17,b4,C25),(A17,b4,C26),(A17,b4,C27),(A17,b4,C28),(A 17,b4,C29),(A17,b5,C1),(A17,b5,C2),(A17,b5,C3),(A17,b5,C4),(A17,b5,C5),(A17,b5,C6),(A1 7,b5,C7),(A17,b5,C8),(A17,b5,C9),(A17,b5,C10),(A17,b5,C11),(A17,b5,C12),(A17,b5,C13),( A17,b5,C14),(A17,b5,C15),(A17,b5,C16),(A17,b5,C17),(A17,b5,C18),(A17,b5,C19),(A17,b5, C20),(A17,b5,C21),(A17,b5,C22),(A17,b5,C23),(A17,b5,C24),(A17,b5,C25),(A17,b5,C26),(A 17,b5,C27),(A17,b5,C28),(A17,b5,C29),(A17,b6,C1),(A17,b6,C2),(A17,b6,C3),(A17,b6,C4),( A17,b6,C5),(A17,b6,C6),(A17,b6,C7),(A17,b6,C8),(A17,b6,C9),(A17,b6,C10),(A17,b6,C11),( A17,b6,C12),(A17,b6,C13),(A17,b6,C14),(A17,b6,C15),(A17,b6,C16),(A17,b6,C17),(A17,b6, C18),(A17,b6,C19),(A17,b6,C20),(A17,b6,C21),(A17,b6,C22),(A17,b6,C23),(A17,b6,C24),(A 17,b6,C25),(A17,b6,C26),(A17,b6,C27),(A17,b6,C28),(A17,b6,C29),(A17,b7,C1),(A17,b7,C2) ,(A17,b7,C3),(A17,b7,C4),(A17,b7,C5),(A17,b7,C6),(A17,b7,C7),(A17,b7,C8),(A17,b7,C9),(A 17,b7,C10),(A17,b7,C11),(A17,b7,C12),(A17,b7,C13),(A17,b7,C14),(A17,b7,C15),(A17,b7,C 16),(A17,b7,C17),(A17,b7,C18),(A17,b7,C19),(A17,b7,C20),(A17,b7,C21),(A17,b7,C22),(A17 ,b7,C23),(A17,b7,C24),(A17,b7,C25),(A17,b7,C26),(A17,b7,C27),(A17,b7,C28),(A17,b7,C29) ,(A18,b1,C1),(A18,b1,C2),(A18,b1,C3),(A18,b1,C4),(A18,b1,C5),(A18,b1,C6),(A18,b1,C7),(A 18,b1,C8),(A18,b1,C9),(A18,b1,C10),(A18,b1,C11),(A18,b1,C12),(A18,b1,C13),(A18,b1,C14) ,(A18,b1,C15),(A18,b1,C16),(A18,b1,C17),(A18,b1,C18),(A18,b1,C19),(A18,b1,C20),(A18,b1 ,C21),(A18,b1,C22),(A18,b1,C23),(A18,b1,C24),(418,b1,C25),(A18,b1,C26),(A18,b1,C27),(A 18,b1,C28),(A18,b1,C29),(A18,b2,C1),(A18,b2,C2),(A18,b2,C3),(A18,b2,C4),(A18,b2,C5),(A 18,b2,C6),(A18,b2,C7),(A18,b2,C8),(A18,b2,C9),(A18,b2,C10),(A18,b2,C11),(A18,b2,C12),( A18,b2,C13),(A18,b2,C14),(A18,b2,C15),(A18,b2,C16),(A18,b2,C17),(A18,b2,C18),(A18,b2, C19),(A18,b2,C20),(A18,b2,C21),(A18,b2,C22),(A18,b2,C23),(A18,b2,C24),(A18,b2,C25),(A 18,b2,C26),(A18,b2,C27),(A18,b2,C28),(A18,b2,C29),(A18,b3,C1),(A18,b3,C2),(A18,b3,C3),( A18,b3,C4),(A18,b3,C5),(A18,b3,C6),(A18,b3,C7),(A18,b3,C8),(A18,b3,C9),(A18,b3,C10),(A 18,b3,C11),(A18,b3,C12),(A18,b3,C13),(A18,b3,C14),(A18,b3,C15),(A18,b3,C16),(A18,b3,C 17),(A18,b3,C18),(A18,b3,C19),(A18,b3,C20),(A18,b3,C21),(A18,b3,C22),(A18,b3,C23),(A18 ,b3,C24),(A18,b3,C25),(A18,b3,C26),(A18,b3,C27),(A18,b3,C28),(A18,b3,C29),(A18,b4,C1),( A18,b4,C2),(A18,b4,C3),(A18,b4,C4),(A18,b4,C5),(A18,b4,C6),(A18,b4,C7),(A18,b4,C8),(A1 8,b4,C9),(A18,b4,C10),(A18,b4,C11),(A18,b4,C12),(A18,b4,C13),(A18,b4,C14),(A18,b4,C15) ,(A18,b4,C16),(A18,b4,C17),(A18,b4,C18),(A18,b4,C19),(A18,b4,C20),(A18,b4,C21),(A18,b4 ,C22),(A18,b4,C23),(A18,b4,C24),(A18,b4,C25),(A18,b4,C26),(A18,b4,C27),(A18,b4,C28),(A 18,b4,C29),(A18,b5,C1),(A18,b5,C2),(A18,b5,C3),(A18,b5,C4),(A18,b5,C5),(A18,b5,C6),(A1 8,b5,C7),(A18,b5,C8),(A18,b5,C9),(A18,b5,C10),(A18,b5,C11),(A18,b5,C12),(A18,b5,C13),( A18,b5,C14),(A18,b5,C15),(A18,b5,C16),(A18,b5,C17),(A18,b5,C18),(A18,b5,C19),(A18,b5, C20),(A18,b5,C21),(A18,b5,C22),(A18,b5,C23),(A18,b5,C24),(A18,b5,C25),(A18,b5,G26),(A 18,b5,C27),(A18,b5,C28),(A18,b5,C29),(A18,b6,C1),(A18,b6,C2),(A18,b6,C3),(A18,b6,C4),( A18,b6,C5),(A18,b6,C6),(A18,b6,C7),(A18,b6,C8),(A18,b6,C9),(A18,b6,C10),(A18,b6,C11),( A18,b6,C12),(A18,b6,C13),(A18,b6,C14),(A18,b6,C15),(A18,b6,C16),(A18,b6,C17),(A18,b6, C18),(A18,b6,C19),(A18,b6,C20),(A18,b6,C21),(A18,b6,C22),(A18,b6,G23),(A18,b6,C24),(A 18,b6,C25),(A18,b6,C26),(A18,b6,C27),(A18,b6,C28),(A18,b6,C29),(418,b7,C1),(A18,b7,C2) ,(A18,b7,C3),(A18,b7,C4),(A18,b7,C5),(A18,b7,C6),(A18,b7,C7),(A18,b7,C8),(A18,b7,C9),(A 18,b7,C10),(A18,b7,C11),(A18,b7,C12),(A18,b7,C13),(A18,b7,C14),(A18,b7,C15),(A18,b7,C 16),(A18,b7,C17),(A18,b7,C18),(A18,b7,C19),(A18,b7,C20),(A18,b7,C21),(A18,b7,C22),(A18 ,b7,C23),(A18,b7,C24),(A18,b7,C25),(A18,b7,C26),(A18,b7,C27),(A18,b7,C28),(A18,b7,C29) ,(A19,b1,C1),(A19,b1,C2),(A19,b1,C3),(A19,b1,C4),(A19,b1,C5),(A19,b1,C6),(A19,b1,C7),(A 19,b1,C8),(A19,b1,C9),(A19,b1,C10),(A19,b1,C11),(A19,b1,C12),(A19,b1,C13),(A19,b1,C14) ,(A19,b1,C15),(A19,b1,C16),(A19,b1,C17),(A19,b1,C18),(A19,b1,C19),(A19,b1,C20),(A19,b1 ,C21),(A19,b1,C22),(A19,b1,C23),(A19,b1,C24),(A19,b1,C25),(A19,b1,C26),(A19,b1,C27),(A 19,b1,C28),(A19,b1,C29),(A19,b2,C1),(A19,b2,C2),(A19,b2,C3),(A19,b2,C4),(A19,b2,C5),(A 19,b2,C6),(A19,b2,C7),(A19,b2,C8),(A19,b2,C9),(A19,b2,C10),(A19,b2,C11),(A19,b2,C12),( A19,b2,C13),(A19,b2,C14),(A19,b2,C15),(A19,b2,C16),(A19,b2,C17),(A19,b2,C18),(A19,b2, C19),(A19,b2,C20),(A19,b2,C21),(A19,b2,C22),(A19,b2,C23),(A19,b2,C24),(A19,b2,C25),(A 19,b2,C26),(A19,b2,C27),(A19,b2,C28),(A19,b2,C29),(A19,b3,C1),(A19,b3,C2),(A19,b3,C3),( A19,b3,C4),(A19,b3,C5),(A19,b3,C6),(A19,b3,C7),(A19,b3,C8),(A19,b3,C9),(A19,b3,C10),(A 19,b3,C11),(A19,b3,C12),(A19,b3,C13),(A19,b3,C14),(A19,b3,C15),(A19,b3,C16),(A19,b3,C 17),(A19,b3,C18),(A19,b3,C19),(A19,b3,C20),(A19,b3,C21),(A19,b3,C22),(A19,b3,C23),(A19 ,b3,C24),(A19,b3,C25),(A19,b3,C26),(A19,b3,C27),(A19,b3,C28),(A19,b3,C29),(A19,b4,C1),( A19,b4,C2),(A19,b4,C3),(A19,b4,C4),(A19,b4,C5),(A19,b4,C6),(A19,b4,C7),(A19,b4,C8),(A1 9,b4,C9),(A19,b4,C10),(A19,b4,C11),(A19,b4,C12),(A19,b4,C13),(A19,b4,C14),(A19,b4,C15) ,(A19,b4,C16),(A19,b4,C17),(A19,b4,C18),(A19,b4,C19),(A19,b4,C20),(A19,b4,C21),(A19,b4 ,C22),(A19,b4,C23),(A19,b4,C24),(A19,b4,C25),(A19,b4,C26),(A19,b4,C27),(A19,b4,C28),(A 19,b4,C29),(A19,b5,C1),(A19,b5,C2),(A19,b5,C3),(A19,b5,C4),(A19,b5,C5),(A19,b5,C6),(A1 9,b5,C7),(A19,b5,C8),(A19,b5,C9),(A19,b5,C10),(A19,b5,C11),(A19,b5,C12),(A19,b5,C13),( A19,b5,C14),(A19,b5,C15),(A19,b5,C16),(A19,b5,C17),(A19,b5,C18),(A19,b5,C19),(A19,b5, C20),(A19,b5,C21),(A19,b5,C22),(A19,b5,C23),(A19,b5,C24),(A19,b5,C25),(A19,b5,C26),(A 19,b5,C27),(A19,b5,C28),(A19,b5,C29),(A19,b6,C1),(A19,b6,C2),(A19,b6,C3),(A19,b6,C4),( A19,b6,C5),(A19,b6,C6),(A19,b6,C7),(A19,b6,C8),(A19,b6,C9),(A19,b6,C10),(A19,b6,C11),( A19,b6,C12),(A19,b6,C13),(A19,b6,C14),(A19,b6,C15),(A19,b6,C16),(A19,b6,C17),(A19,b6, C18),(A19,b6,C19),(A19,b6,C20),(A19,b6,C21),(A19,b6,C22),(A19,b6,C23),(A19,b6,C24),(A 19,b6,C25),(A19,b6,C26),(A19,b6,C27),(A19,b6,C28),(A19,b6,C29),(A19,b7,C1),(A19,b7,C2) ,(A19,b7,C3),(A19,b7,C4),(A19,b7,C5),(A19,b7,C6),(A19,b7,C7),(A19,b7,C8),(A19,b7,C9),(A 19,b7,C10),(A19,b7,C11),(A19,b7,C12),(A19,b7,C13),(A19,b7,C14),(A19,b7,C15),(A19,b7,C 16),(A19,b7,C17),(A19,b7,C18),(A19,b7,C19),(A19,b7,C20),(A19,b7,C21),(A19,b7,C22),(A19 ,b7,C23),(A19,b7,C24),(A19,b7,C25),(A19,b7,C26),(A19,b7,C27),(A19,b7,C28),(A19,b7,C29) ,(A20,b1,C1),(A20,b1,C2),(A20,b1,C3),(A20,b1,C4),(A20,b1,C5),(A20,b1,C6),(A20,b1,C7),(A 20,b1,C8),(A20,b1,C9),(A20,b1,C10),(A20,b1,C11),(420,b1,C12),(A20,b1,C13),(A20,b1,C14) ,(A20,b1,C15),(A20,b1,C16),(A20,b1,C17),(A20,b1,C18),(A20,b1,C19),(A20,b1,C20),(A20,b1 ,C21),(A20,b1,C22),(A20,b1,C23),(A20,b1,C24),(A20,b1,C25),(A20,b1,C26),(A20,b1,C27),(A 20,b1,C28),(A20,b1,C29),(A20,b2,C1),(A20,b2,C2),(A20,b2,C3),(A20,b2,C4),(A20,b2,C5),(A 20,b2,C6),(A20,b2,C7),(A20,b2,C8),(A20,b2,C9),(A20,b2,C10),(A20,b2,C11),(A20,b2,C12),( A20,b2,C13),(A20,b2,C14),(A20,b2,C15),(A20,b2,C16),(A20,b2,C17),(A20,b2,C18),(A20,b2, C19),(A20,b2,C20),(A20,b2,C21),(A20,b2,C22),(A20,b2,C23),(A20,b2,C24),(A20,b2,C25),(A 20,b2,C26),(A20,b2,C27),(A20,b2,C28),(A20,b2,C29),(A20,b3,C1),(A20,b3,C2),(A20,b3,C3),( A20,b3,C4),(A20,b3,C5),(A20,b3,C6),(A20,b3,C7),(A20,b3,C8),(A20,b3,C9),(A20,b3,C10),(A 20,b3,C 11),(A20,b3,C 12),(A20,b3,C13),(A20,b3,C14),(A20,b3,C15),(A20,b3,C16),(A20,b3,C 17),(A20,b3,C18),(A20,b3,C19),(A20,b3,C20),(A20,b3,C21),(A20,b3,C22),(A20,b3,C23),(A20 ,b3,C24),(A20,b3,C25),(A20,b3,C26),(A20,b3,C27),(A20,b3,C28),(A20,b3,C29),(A20,b4,C1),( A20,b4,C2),(A20,b4,C3),(A20,b4,C4),(A20,b4,C5),(A20,b4,C6),(A20,b4,C7),(A20,b4,C8),(A2 0,b4,C9),(A20,b4,C10),(A20,b4,C11),(A20,b4,C12),(A20,b4,C13),(A20,b4,C14),(A20,b4,C15) ,(A20,b4,C16),(A20,b4,C17),(A20,b4,C18),(A20,b4,C19),(A20,b4,C20),(A20,b4,C21),(A20,b4 ,C22),(A20,b4,C23),(A20,b4,C24),(A20,b4,C25),(A20,b4,C26),(A20,b4,C27),(A20,b4,C28),(A 20,b4,C29),(A20,b5,C1),(A20,b5,C2),(A20,b5,C3),(A20,b5,C4),(A20,b5,C5),(A20,b5,C6),(A2 0,b5,C7),(A20,b5,C8),(A20,b5,C9),(A20,b5,C10),(A20,b5,C11),(A20,b5,C12),(A20,b5,C13),( A20,b5,C14),(A20,b5,C15),(A20,b5,C16),(A20,b5,C17),(A20,b5,C18),(A20,b5,C19),(A20,b5, C20),(A20,b5,C21),(A20,b5,C22),(A20,b5,C23),(A20,b5,C24),(A20,b5,C25),(A20,b5,C26),(A 20,b5,C27),(A20,b5,C28),(A20,b5,C29),(A20,b6,C1),(A20,b6,C2),(A20,b6,C3),(A20,b6,C4),( A20,b6,C5),(A20,b6,C6),(A20,b6,C7),(A20,b6,C8),(A20,b6,C9),(A20,b6,C10),(A20,b6,C11),( A20,b6,C12),(A20,b6,C13),(A20,b6,C14),(A20,b6,C15),(A20,b6,C16),(A20,b6,C17),(A20,b6, C18),(A20,b6,C19),(A20,b6,C20),(A20,b6,C21),(A20,b6,C22),(A20,b6,C23),(A20,b6,C24),(A 20,b6,C25),(A20,b6,C26),(A20,b6,C27),(A20,b6,C28),(A20,b6,C29),(A20,b7,C1),(A20,b7,C2) ,(A20,b7,C3),(A20,b7,C4),(A20,b7,C5),(A20,b7,C6),(A20,b7,C7),(A20,b7,C8),(A20,b7,C9),(A 20,b7,C10),(A20,b7,C11),(A20,b7,C12),(A20,b7,C13),(A20,b7,C14),(A20,b7,C15),(A20,b7,C 16),(A20,b7,C17),(A20,b7,C18),(A20,b7,C19),(A20,b7,C20),(A20,b7,C21),(A20,b7,C22),(A20 ,b7,C23),(A20,b7,C24),(A20,b7,C25),(A20,b7,C26),(A20,b7,C27),(A20,b7,C28),(A20,b7,C29),

(A21,b1,C1),(A21,b1,C2),(A21,b1,C3),(A21,b1,C4),(A21,b1,C5),(A21,b1,C6),(A21,b1,C7),(A 21,b1,C8),(A21,b1,C9),(A21,b1,C10),(A21,b1,C11),(A21,b1,C12),(A21,b1,C13),(A21,b1,C14) ,(A21,b1,C15),(A21,b1,C16),(A21,b1,C17),(A21,b1,C18),(A21,b1,C19),(A21,b1,C20),(A21,b1 ,C21),(A21,b1,C22),(A21,b1,C23),(A21,b1,C24),(A21,b1,C25),(A21,b1,C26),(A21,b1,C27),(A 21,b1,C28),(A21,b1,C29),(A21,b2,C1),(A21,b2,C2),(A21,b2,C3),(A21,b2,C4),(A21,b2,C5),(A 21,b2,C6),(A21,b2,C7),(A21,b2,C8),(A21,b2,C9),(A21,b2,C10),(A21,b2,C11),(A21,b2,C12),( A21,b2,C13),(A21,b2,C14),(A21,b2,C15),(A2l,b2,C16),(A21,b2,C17),(A21,b2,C18),(A21,b2, C19),(A21,b2,C20),(A21,b2,C21),(A21,b2,C22),(A21,b2,C23),(A21,b2,C24),(A21,b2,C25),(A 21,b2,C26),(A21,b2,C27),(A21,b2,C28),(A21,b2,C29),(A21,b3,C1),(A21,b3,C2),(A21,b3,C3),( A21,b3,C4),(A21,b3,C5),(A21,b3,C6),(A21,b3,C7),(A21,b3,C8),(A21,b3,C9),(A21,b3,C10),(A 21,b3,C11),(A21,b3,C12),(A21,b3,C13),(A21,b3,C14),(A21,b3,C15),(A21,b3,C16),(A21,b3,C 17),(A21,b3,C18),(A21,b3,C19),(A21,b3,C20),(A21,b3,C21),(A21,b3,C22),(A21,b3,C23),(A21 ,b3,C24),(A21,b3,C25),(A21,b3,C26),(A21,b3,C27),(A21,b3,C28),(A21,b3,C29),(A21,b4,C1),( A21,b4,C2),(A21,b4,C3),(A21,b4,C4),(A21,b4,C5),(A21,b4,C6),(A21,b4,C7),(A21,b4,C8),(A2 1,b4,C9),(A21,b4,C10),(A21,b4,C11),(A21,b4,C12),(A21,b4,C13),(A21,b4,C14),(A21,b4,C15) ,(A21,b4,C16),(A21,b4,C17),(A21,b4,C18),(A21,b4,C19),(A21,b4,C20),(A21,b4,C21),(A21,b4 ,C22),(A21,b4,C23),(A21,b4,C24),(A21,b4,C25),(A21,b4,C26),(A21,b4,C27),(A21,b4,C28),(A 21,b4,C29),(A21,b5,C1),(A21,b5,C2),(A21,b5,C3),(A21,b5,C4),(A21,b5,C5),(A21,b5,C6),(A2 1,b5,C7),(A21,b5,C8),(A21,b5,C9),(A21,b5,C10),(A21,b5,C11),(A21,b5,C12),(A21,b5,C13),( A21,b5,C14),(A21,b5,C15),(A21,b5,C16),(A21,b5,C17),(A21,b5,C18),(A21,b5,C19),(A21,b5, C20),(A21,b5,C21),(A21,b5,C22),(A21,b5,C23),(A21,b5,C24),(A21,b5,C25),(A21,b5,C26),(A 21,b5,C27),(A21,b5,C28),(A21,b5,C29),(A21,b6,C1),(A21,b6,C2),(A21,b6,C3),(A21,b6,C4),( A21,b6,C5),(A21,b6,C6),(A21,b6,C7),(A21,b6,C8),(A21,b6,C9),(A21,b6,C10),(A21,b6,C11),( A21,b6,C12),(A21,b6,C13),(A21,b6,C14),(A21,b6,C15),(A21,b6,C16),(A21,b6,C17),(A21,b6, C18),(A21,b6,C19),(A21,b6,C20),(A21,b6,C21),(A21,b6,C22),(A21,b6,C23),(A21,b6,C24),(A 21,b6,C25),(A21,b6,C26),(A21,b6,C27),(A21,b6,C28),(A21,b6,C29),(A21,b7,C1),(A21,b7,C2) ,(A21,b7,C3),(A21,b7,C4),(A21,b7,C5),(A21,b7,C6),(A21,b7,C7),(A21,b7,C8),(A21,b7,C9),(A 21,b7,C10),(A21,b7,C11),(A21,b7,C12),(A21,b7,C13),(A21,b7,C14),(A21,b7,C15),(A21,b7,C 16),(A21,b7,C17),(A21,b7,C18),(A21,b7,C19),(A21,b7,C20),(A21,b7,C21),(A21,b7,C22),(A21 ,b7,C23),(A21,b7,C24),(A21,b7,C25),(A21,b7,C26),(A21,b7,C27),(A21,b7,C28),(A21,b7,C29) ,(A22,b1,C1),(A22,b1,C2),(A22,b1,C3),(A22,b1,C4),(A22,b1,C5),(A22,b1,C6),(A22,b1,C7),(A 22,b1,C8),(A22,b1,C9),(A22,b1,C10),(A22,b1,C11),(A22,b1,C12),(A22,b1,C13),(A22,b1,C14) ,(A22,b1,C15),(A22,b1,C16),(A22,b1,C17),(A22,b1,C18),(A22,b1,C19),(A22,b1,C20),(A22,b1 ,C21),(A22,b1,C22),(A22,b1,C23),(A22,b1,C24),(A22,b1,C25),(A22,b1,C26),(A22,b1,C27),(A 22,b1,C28),(A22,b1,C29),(A22,b2,C1),(A22,b2,C2),(A22,b2,C3),(A22,b2,C4),(A22,b2,C5),(A 22,b2,C6),(A22,b2,C7),(A22,b2,C8),(A22,b2,C9),(A22,b2,C10),(A22,b2,C11),(A22,b2,C12),( A22,b2,C13),(A22,b2,C14),(A22,b2,C15),(A22,b2,C16),(A22,b2,C17),(A22,b2,C18),(A22,b2, C19),(A22,b2,C20),(A22,b2,C21),(A22,b2,C22),(A22,b2,C23),(A22,b2,C24),(A22,b2,C25),(A 22,b2,C26),(A22,b2,C27),(A22,b2,C28),(A22,b2,C29),(A22,b3,C1),(A22,b3,C2),(A22,b3,C3),( A22,b3,C4),(A22,b3,C5),(A22,b3,C6),(A22,b3,C7),(A22,b3,C8),(A22,b3,C9),(A22,b3,C10),(A 22,b3,C11),(A22,b3,C12),(A22,b3,C13),(A22,b3,C14),(A22,b3,C15),(A22,b3,C16),(A22,b3,C 17),(A22,b3,C18),(A22,b3;C19),(A22,b3,C20),(A22,b3,C21),(A22,b3,C22),(A22,b3,C23),(A22 ,b3,C24),(A22,b3,C25),(A22,b3,C26),(A22,b3,C27),(A22,b3,C28),(A22,b3,C29),(A22,b4,C1),( A22,b4,C2),(A22,b4,C3),(A22,b4,C4),(A22,b4,C5),(A22,b4,C6),(A22,b4,C7),(A22,b4,C8),(A2 2,b4,C9),(A22,b4,C10),(A22,b4,C11),(A22,b4,C12),(A22,b4,C13),(A22,b4,C14),(A22,b4,C15) ,(A22,b4,C16),(A22,b4,C17),(A22,b4,C18),(A22,b4,C19),(A22,b4,C20),(A22,b4,C21),(A22,b4 ,C22),(A22,b4,C23),(A22,b4,C24),(A22,b4,C25),(A22,b4,C26),(A22,b4,C27),(A22,b4,C28),(A 22,b4,C29),(A22,b5,C1),(A22,b5,C2),(A22,b5,C3),(A22,b5,C4),(A22,b5,C5),(A22,b5,C6),(A2 2,b5,C7),(A22,b5,C8),(A22,b5,C9),(A22,b5,C10),(A22,b5,C11),(A22,b5,C12),(A22,b5,C13),( A22,b5,C14),(A22,b5,C15),(A22,b5,C16),(A22,b5,C17),(A22,b5,C18),(A22,b5,C19),(A22,b5, C20),(A22,b5,C21),(A22,b5,C22),(A22,b5,G23),(A22,b5,C24),(A22,b5,C25),(A22,b5,C26),(A 22,b5,C27),(A22,b5,C28),(A22,b5,C29),(A22,b6,C1),(A22,b6,C2),(A22,b6,C3),(A22,b6,C4),( A22,b6,C5),(A22,b6,C6),(A22,b6,C7),(A22,b6,C8),(A22,b6,C9),(A22,b6,C10),(A22,b6,C11),( A22,b6,C12),(A22,b6,C13),(A22,b6,C14),(A22,b6,C15),(A22,b6,C16),(A22,b6,C17),(A22,b6, C18),(A22,b6,C19),(A22,b6,C20),(A22,b6,C21),(A22,b6,C22),(A22,b6,C23),(A22,b6,C24),(A 22,b6,C25),(A22,b6,C26),(A22,b6,C27),(A22,b6,C28),(A22,b6,C29),(A22,b7,C1),(A22,b7,C2) ,(A22,b7,C3),(A22,b7,C4),(A22,b7,C5),(A22,b7,C6),(A22,b7,C7),(A22,b7,C8),(A22,b7,C9),(A 22,b7,C10),(A22,b7,C11),(A22,b7,C12),(A22,b7,C13),(A22,b7,C14),(A22,b7,C15),(A22,b7,C 16),(A22,b7,C17),(A22,b7,C18),(A22,b7,C19),(A22,b7,C20),(A22,b7,C21),(A22,b7,C22),(A22 ,b7,C23),(A22,b7,C24),(A22,b7,C25),(A22,b7,C26),(A22,b7,C27),(A22,b7,C28),(A22,b7,C29) ,(A23,b1,C1),(A23,b1,C2),(A23,b1,C3),(A23,b1,C4),(A23,b1,C5),(A23,b1,C6),(A23,b1,C7),(A 23,b1,C8),(A23,b1,C9),(A23,b1,C10),(A23,b1,C11),(A23,b1,C12),(A23,b1,C13),(A23,b1,C14) ,(A23,b1,C15),(A23,b1,C16),(A23,b1,C17),(A23,b1,C18),(A23,b1,C19),(A23,b1,C20),(A23,b ,C21),(A23,b1,C22),(A23,b1,C23),(A23,b1,C24),(A23,b1,C25),(A23,b1,C26),(A23,b1,C27),(A 23,b1,C28),(A23,b1,C29),(A23,b2,C1),(A23,b2,C2),(A23,b2,C3),(A23,b2,C4),(A23,b2,C5),(A 23,b2,C6),(A23,b2,C7),(A23,b2,C8),(A23,b2,C9),(A23,b2,C10),(A23,b2,C11),(A23,b2,C12),( A23,b2,C13),(A23,b2,C14),(A23,b2,C15),(A23,b2,C16),(A23,b2,C17),(A23,b2,C18),(A23,b2, C19),(A23,b2,C20),(A23,b2,C21),(A23,b2,C22),(A23,b2,C23),(A23,b2,C24),(A23,b2,C25),(A 23,b2,C26),(A23,b2,C27),(A23,b2,C28),(A23,b2,C29),(A23,b3,C1),(A23,b3,C2),(A23,b3,C3),( 23,b3,C11),(A23,b3,C12),(A23,b3,C13),(A23,b3,C14),(A23,b3,C15),(A23,b3,C16),(A23,b3,C 17),(A23,b3,C18),(A23,b3,C19),(A23,b3,C20),(A23,b3,C21),(A23,b3,C22),(A23,b3,C23),(A23 ,b3,C24),(A23,b3,C25),(A23,b3,C26),(A23,b3,C27),(A23,b3,C28),(A23,b3,C29),(A23,b4,C1),( A23,b4,C2),(A23,b4,C3),(A23,b4,C4),(A23,b4,C5),(A23,b4,C6),(A23,b4,C7),(A23,b4,C8),(A2 3,b4,C9),(A23,b4,C10),(A23,b4,C11),(A23,b4,C12),(A23,b4,C13),(A23,b4,C14),(A23,b4,C15) ,(A23,b4,C16),(A23,b4,C17),(A23,b4,C18),(A23,b4,C19),(A23,b4,C20),(A23,b4,C21),(A23,b4 ,C22),(A23,b4,C23),(A23,b4,C24),(A23,b4,C25),(A23,b4,C26),(A23,b4,C27),(A23,b4,C28),(A 23,b4,C29),(A23,b5,C1),(A23,b5,C2),(A23,b5,C3),(A23,b5,C4),(A23,b5,C5),(A23,b5,C6),(A2 3,b5,C77),(A23,b5,C8),(A23,b5,C9),(A23,b5,C10),(A23,b5,C11),(A23,b5,C12),(A23,b5,C13),( A23,b5,C14),(A23,b5,C15),(A23,b5,C16),(A23,b5,C17),(A23,b5,C18),(A23,b5,C19),(A23,b5, C20),(A23,b5,C21),(A23,b5,C22),(A23,b5,C23),(A23,b5,C24),(A23,b5,C25),(A23,b5,C26),(A 23,b5,C27),(A23,b5,C28),(A23,b5,C29),(A23,b6,C1),(A23,b6,C2),(A23,b6,C3),(A23,b6,C4),( A23,b6,C5),(A23,b6,C6),(A23,b6,C7),(A23,b6,C8),(A23,b6,C9),(A23,b6,C10),(A23,b6,C11),( A23,b6,C12),(A23,b6,C13),(A23,b6,C14),(A23,b6,C15),(A23,b6,C16),(A23,b6,C17),(A23,b6, C18),(A23,b6,C19),(A23,b6,C20),(A23,b6,C21),(A23,b6,C22),(A23,b6,C23),(A23,b6,C24),(A 23,b6,C25),(A23,b6,C26),(A23,b6,C27),(A23,b6,C28),(A23,b6,C29),(A23,b7,C1),(A23,b7,C2) ,(A23,b7,C3),(A23,b7,C4),(A23,b7,C5),(A23,b7,C6),(A23,b7,C7),(A23,b7,C8),(A23,b7,C9),(A 23,b7,C10),(A23,b7,C11),(A23,b7,C12),(A23,b7,C13),(A23,b7,C14),(A23,b7,C15),(A23,b7,C 16),(A23,b7,C17),(A23,b7,C18),(A23,b7,C19),(A23,b7,C20),(A23,b7,C21),(A23,b7,C22),(A23 ,b7,C23),(A23,b7,C24),(A23,b7,C25),(A23,b7,C26),(A23,b7,C27),(A23,b7,C28),(A23,b7,C29) ,(A24,b1,C1),(A24,b1,C2),(A24,b1,C3),(A24,b1,C4),(A24,b1,C5),(A24,b1,C6),(A24,b1,C7),(A 24,b1,C8),(A24,b1,C9),(A24,b1,C10),(A24,b1,C11),(A24,b1,C12),(A24,b1,C13),(A24,b1,C14) ,(A24,b1,C15),(A24,b1,C16),(A24,b1,C17),(A24,b1,C18),(A24,b1,C19),(A24,b1,C20),(A24,b1 ,C21),(A24,b1,C22),(A24,b1,C23),(A24,b1,C24),(A24,b1,C25),(A24,b1,C26),(A24,b1,C27),(A 24,b1,C28),(A24,b1,C29),(A24,b2,C1),(A24,b2,C2),(A24,b2,C3),(A24,b2,C4),(A24,b2,C5),(A 24,b2,C6),(A24,b2,C7),(A24,b2,C8),(A24,b2,C9),(A24,b2,C10),(A24,b2,C11),(A24,b2,C12),( A24,b2,C13),(A24,b2,C14),(A24,b2,C15),(A24,b2,C16),(A24,b2,C17),(A24,b2,C18),(A24,b2, C19),(A24,b2,C20),(A24,b2,C21),(A24,b2,C22),(A24,b2,C23),(A24,b2,C24),(A24,b2,C25),(A 24,b2,C26),(A24,b2,C27),(A24,b2,C28),(A24,b2,C29),(A24,b3,C1),(A24,b3,C2),(A24,b3,C3),( A24,b3,C4),(A24,b3,C5),(A24,b3,C6),(A24,b3,C7),(A24,b3,C8),(A24,b3,C9),(A24,b3,C10),(A 24,b3,C11),(A24,b3,C12),(A24,b3,C13),(A24,b3,C14),(A24,b3,C15),(A24,b3,C16),(A24,b3,C 17),(A24,b3,C18),(A24,b3,C19),(A24,b3,C20),(A24,b3,C21),(A24,b3,C22),(A24,b3,C23),(A24 ,b3,C24),(A24,b3,C25),(A24,b3,C26),(424,b3,C27),(A24,b3,C28),(A24,b3,C29),(A24,b4,C1),( A24,b4,C2),(A24,b4,C3),(A24,b4,C4),(A24,b4,C5),(A24,b4,C6),(A24,b4,C7),(A24,b4,C8),(A2 4,b4,C9),(A24,b4,C10),(A24,b4,C11),(A24,b4,C12),(A24,b4,C13),(A24,b4,C4),(A24,b4,C15) ,(A24,b4,C16),(A24,b4,C17),(A24,b4,C18),(A24,b4,C19),(A24,b4,C20),(A24,b4,C21),(A24,b4 ,C22),(A24,b4,C23),(A24,b4,C24),(A24,b4,C25),(A24,b4,C26),(A24,b4,C27),(A24,b4,C28),(A 24,b4,C29),(A24,b5,C1),(A24,b5,C2),(A24,b5,C3),(A24,b5,C4),(A24,b5,C5),(A24,b5,C6),(A2 4,b5,C7),(A24,b5,C8),(A24,b5,C9),(A24,b5,C10),(A24,b5,C11),(A24,b5,C12),(A14,b5,C13),( A24,b5,C14),(A24,b5,C15),(A24,b5,C16),(A24,b5,C17),(A24,b5,C18),(A24,b5,C19),(A24,b5, C20),(A24,b5,C21),(A24,b5,C22),(A24,b5,C23),(A24,b5,C24),(A24,b5,C25),(A24,b5,C26),(A 24,b5,C27),(A24,b5,C28),(A24,b5,C29),(A24,b6,C1),(A24,b6,C2),(A24,b6,C3),(A24,b6,C4),( A24,b6,C5),(A24,b6,C6),(A24,b6,C7),(A24,b6,C8),(A24,b6,C9),(A24,b6,C10),(A24,b6,C11),( A24,b6,C12),(A24,b6,C13),(A24,b6,C14),(A24,b6,C15),(A24,b6,C16),(A24,b6,C17),(A24,b6, C18),(A24,b6,C19),(A24,b6,C20),(A24,b6,C21),(A24,b6,C22),(A24,b6,C23),(A24,b6,C24),A 24,b6,C25),(A24,b6,C26),(A24,b6,C27),(A24,b6,C28),(A24,b6,C29),(A24,b7,C1),(A24,b7,C2) ,(A24,b7,C3),(A24,b7,C4),(A24,b7,C5),(A24,b7,C6),(A24,b7,C7),(A24,b7,C8),(A24,b7,C9),(A 24,b7,C10),(A24,b7,C11),(A24,b7,C12),(A24,b7,C3),(A24,b7,C14),(A24,b7,C5),(A24,b7,C 16),(A24,b7,C17),(A24,b7,C18),(A24,b7,C19),(A24,b7,C20),(A24,b7,C21),(A24,b7,C22),(A24 ,b7,C23),(A24,b7,C24),(A24,b7,C25),(A24,b7,C26),(A24,b7,C27),(A24,b7,C28),(A24,b7,C29) ,(A25,b1,C1),(A25,b1,C2),(A25,b1,C3),(A25,b1,C4),(A25,b1,C5),(A25,b1,C6),(A25,b1,C7),(A 25,b1,C8),(A25,b1,C9),(A25,b1,C10),(A25,b1,C11),(A25,b1,C12),(A25,b1,C13),(A25,b1,C14) ,(A25,b1,C15),(A25,b1,C16),(A25,b1,C17),(A25,b1,C18),(A25,b1,C19),(A25,b1,C20),(A25,b1 ,C21),(A25,b1,C22),(A25,b1,C23),(A25,b1,C24),(A25,b1,C25),(A25,b1,C26),(A25,b1,C27),(A 25,b1,C28),(A25,b1,C29),(A25,b2,C1),(A25,b2,C2),(A25,b2,C3),(A25,b2,C4),(A25,b2,C5),(A 25,b2,C6),(A25,b2,C7),(A25,b2,C8),(A25,b2,C9),(A25,b2,C10),(A25,b2,C11),(A25,b2,C12),( A25,b2,C13),(A25,b2,C14),(A25,b2,C15),(A25,b2,C16),(A25,b2,C17),(A25,b2,C18),(A25,b2, C19),(A25,b2,C20),(A25,b2,C21),(A25,b2,C22),(A25,b2,C23),(A25,b2,C24),(A25,b2,C25),(A 25,b2,C26),(A25,b2,C27),(A25,b2,C28),(A25,b2,C29),(A25,b3,C1),(A25,b3,C2),(A25,b3,C3),( A25,b3,C4),(A25,b3,C5),(A25,b3,C6),(A25,b3,C7),(A25,b3,C8),(A25,b3,C9),(A25,b3,C10),(A 25,b3,C11),(A25,b3,C12),(A25,b3,C13),(A25,b3,C14),(A25,b3,C15),(A25,b3,C16),(A25,b3,C 17),(A25,b3,C18),(A25,b3,C19),(A25,b3,C20),(A25,b3,C21),(A25,b3,C22),(A25,b3,C23),(A25 ,b3,C24),(A25,b3,C25),(A25,b3,C26),(A25,b3,C27),(A25,b3,C28),(A25,b3,C29),(A25,b4,C1),( A25,b4,C2),(A25,b4,C3),(A25,b4,C4),(A25,b4,C5),(A25,b4,C6),(A25,b4,C7),(A25,b4,C8),(A2 5,b4,C9),(A25,b4,C10),(A25,b4,C11),(A25,b4,C12),(A25,b4,C13),(A25,b4,C14),(A25,b4,C15) ,(A25,b4,C16),(A25,b4,C17),(A25,b4,C18),(A25,b4,C19),(A21,b4,C20),(A25,b4,C21),(A25,b4 ,C22),(A25,b4,C23),(A25,b4,C24),(A25,b4,C25),(A25,b4,C26),(A25,b4,C27),(A25,b4,C28),(A 25,b4,C29),(A25,b5,C1),(A25,b5,C2),(A25,b5,C3),(A25,b5,C4),(A25,b5,C5),(A25,b5,C6),(A2 5,b5,C7),(A25,b5,C8),(A25,b5,C9),(A25,b5,C10),(A25,b5,C11),(A25,b5,C12),(A25,b5,C13), A25,b5,C14),(A25,b5,C15),(A25,b5,C16),(A25,b5,C17),(A25,b5,C18),(A25,b5,C19),(A25,b5, C20),(A25,b5,C21),(A25,b5,C22),(A25,b5,C23),(A25,b5,C24),(A25,b5,C25),(A25,b5,C26),(A 25,b5,C27),(A25,b5,C28),(A25,b5,C29),(A25,b6,C1),(A25,b6,C2),(A25,b6,C3),(A25,b6,C4),( A25,b6,C5),(A25,b6,C6),(A25,b6,C7),(A25,b6,C8),(A25,b6,C9),(A25,b6,C10),(A25,b6,C11),( A25,b6,C12),(A25,b6,C13),(A25,b6,C14),(A25,b6,C15),(A25,b6,C16),(A25,b6,C17),(A25,b6, C18),(A25,b6,C19),(A25,b6,C20),(A25,b6,C21),(A25,b6,C22),(A25,b6,C23),(A25,b6,C24),(A 25,b6,C25),(A25,b6,C26),(A25,b6,C27),(A25,b6,C28),(A25,b6,C29),(A25,b7,C1),(A25,b7,C2) ,(A25,b7,C3),(A25,b7,C4),(A25,b7,C5),(A25,b7,C6),(A25,b7,C7),(A25,b7,C8),(A25,b7,C9),(A 25,b7,C10),(A25,b7,C11),(A25,b7,C12),(A25,b7,C13),(A25,b7,C14),(A25,b7,C15),(A25,b7,C 16),(A25,b7,C17),(A25,b7,C18),(A25,b7,C19),(A25,b7,C20),(A25,b7,C21),(A25,b7,C22),(A25 ,b7,C23),(A25,b7,C24),(A25,b7,C25),(A25,b7,C26),(A25,b7,C27),(A25,b7,C28),(A25,b7,C29) ,(A26,b1,C1),(A26,b1,C2),(A26,b1,C3),(A26,b1,C4),(A26,b1,C5),(A26,b1,C6),(A26,b1,C7),(A 26,b1,C8),(A26,b1,C9),(A26,b1,C10),(A26,b1,C11),(A26,b1,C12),(A26,b1,C13),(A26,b1,C14) ,(A26,b,C15),(A26,b1,C16),(A26,b1,C17),(A26,b1,C18),(A26,b1,C19),(A26,b1,C20),(A26,b1 ,C21),(A26,b1,C22),(A26,b1,C23),(A26,b1,C24),(A26,b1,C25),(A26,b1,C26),(A26,b1,C27),(A 26,b1,C28),(A26,b1,C29),(A26,b2,C1),(A26,b2,C2),(A26,b2,C3),(A26,b2,C4),(A26,b2,C5),(A 26,b2,C6),(A26,b2,C7),(A26,b2,C8),(A26,b2,C9),(A26,b2,C10),(A26,b2,C11),(A26,b2,C12),( A26,b2,C3),(A26,b2,C14),(A26,b2,C15),(A26,b2,C16),(A26,b2,C17),(A26,b2,C8),(A26,b2, C19),(A26,b2,C20),(A26,b2,C21),(A26,b2,C22),(A26,b2,C23),(A26,b2,C24),(A26,b2,C25),(A 26,b2,C26),(A26,b2,C27),(A26,b2,C28),(A26,b2,C29),(A26,b3,C1),(A26,b3,C2),(A26,b3,C3),( A26,b3,C4),(A26,b3,C5),(A26,b3,C6),(A26,b3,C7),(A26,b3,C8),(A26,b3,C9),(A26,b3,C10),(A 26,b3,C11),(A26,b3,C12),(A26,b3,C13),(A26,b3,C4),(A26,b3,C15),(A26,b3,C16),(A26,b3,C 17),(A26,b3,C18),(A26,b3,C19),(A26,b3,C20),(A26,b3,C21),(A26,b3,C22),(A26,b3,C23),(A26 ,b3,C24),(A26,b3,C25),(A26,b3,C26),(A26,b3,C27),(A26,b3,C28),(A26,b3,C29),(A26,b4,C1),( A26,b4,C2),(A26,b4,C3),(A26,b4,C4),(A26,b4,C5),(A26,b4,C6),(A26,b4,C7),(A26,b4,C8),(A2 6,b4,C9),(A26,b4,C10),(A26,b4,C11),(A26,b4,C12),(A26,b4,C13),(A26,b4,C14),(A26,b4,C15) ,(A26,b4,C16),(A26,b4,C17),(A26,b4,C18),(A26,b4,C9),(A26,b4,C20),(A26,b4,C21),(A26,b4 ,C22),(A26,b4,C23),(A26,b4,C24),(A26,b4,C25),(A26,b4,C26),(A26,b4,C27),(A26,b4,C28),(A 26,b4,C29),(A26,b5,C1),(A26,b5,C2),(A26,b5,C3),(A26,b5,C4),(A26,b5,C5),(A26,b5,C6),(A2 6,b5,C7),(A26,b5,C8),(A26,b5,C9),(A26,b5,C10),(A26,b5,C1),(A26,b5,C12),(A26,b5,C13),( A26,b5,C14),(A26,b5,C15),(A26,b5,C16),(A26,b5,C17),(A26,b5,C18),(A26,b5,C19),(A26,b5, C20),(A26,b5,C21),(A26,b5,C22),(A26,b5,C23),(A26,b5,C24),(A26,b5,C25),(A26,b5,C26),(A 26,b5,C27),(A26,b5,C28),(A26,b5,C29),(A26,b6,C1),(A26,b6,C2),(A26,b6,C3),(A26,b6,C4),( A26,b6,C5),(A26,b6,C6),(A26,b6,C7),(A26,b6,C8),(A26,b6,C9),(A26,b6,C10),(A26,b6,C11),( A26,b6,C12),(A26,b6,C13),(A26,b6,C14),(A26,b6,C15),(A26,b6,C16),(A26,b6,C17),(A26,b6, C18),(A26,b6,C19),(A26,b6,C20),(A26,b6,C21),(A26,b6,C22),(A26,b6,C23),(A26,b6,C24),(A 26,b6,C25),(A26,b6,C26),(A26,b6,C27),(A26,b6,C28),(A26,b6,C29),(A26,b7,C1),(A26,b7,C2) ,(A26,b7,C3),(A26,b7,C4),(A26,b7,C5),(A26,b7,C6),(A26,b7,C7),(A26,b7,C8),(A26,b7,C9),(A 26,b7,C10),(A26,b7,C11),(A26,b7,C12),(A26,b7,C13),(A26,b7,C14),(A26,b7,C15),(A26,b7,C 16),(A26,b7,C17),(A26,b7,C18),(A26,b7,C19),(A26,b7,C20),(A26,b7,C21),(A26,b7,C22),(A26 ,b7,C23),(A26,b7,C24),(A26,b7,C25),(A26,b7,C26),(A26,b7,C27),(A26,b7,C28),(A26,b7,C29) ,(A27,b1,C1),(A27,b1,C2),(A27,b1,C3),(A27,b1,C4),(A27,b1,C5),(A27,b1,C6),(A27,b1,C7),(A 27,b1,C8),(A27,b1,C9),(A27,b1,C10),(A27,b1,C11),(A27,b1,C12),(A27,b1,C13),(A27,b1,C14) ,(A27,b1,C15),(A27,b1,C16),(A27,b1,C17),(A27,b1,C18),(A27,b1,C19),(A27,b1,C20),(A27,b1 ,C21),(A27,b1,C22),(A27,b1,C23),(A27,b1,C24),(A27,b1,C25),(A27,b1,C26),(A27,b1,C27),(A 27,b1,C28),(A27,b1,C29),(A27,b2,C1),(A27,b2,C2),(A27,b2,C3),(A27,b2,C4),(A27,b2,C5),(A 27,b2,C6),(A27,b2,C7),(A27,b2,C8),(A27,b2,C9),(A27,b2,C10),(A27,b2,C11),(A27,b2,C12),( A27,b2,C13),(A27,b2,C14),(A27,b2,C15),(A27,b2,C16),(A27,b2,C17),(A27,b2,C18),(A27,b2, C19),(A27,b2,C20),(A27,b2,C21),(A27,b2,C22),(A27,b2,C23),(A27,b2,C24),(A27,b2,C25),(A 27,b2,C26),(A27,b2,C27),(A27,b2,C28),(A27,b2,C29),(A27,b3,C1),(A27,b3,C2),(A27,b3,C3),( A27,b3,C4),(A27,b3,C5),(A27,b3,C6),(A27,b3,C7),(A27,b3,C8),(A27,b3,C9),(A27,b3,C10),(A 27,b3,C11),(A27,b3,C12),(A27,b3,C13),(A27,b3,C14),(A27,b3,C15),(A27,b3,C16),(A27,b3,C 17),(A27,b3,C18),(A27,b3,C19),(A27,b3,C20),(A27,b3,C21),(A27,b3,C22),(A27,b3,C23),(A27 ,b3,C24),(A27,b3,C25),(A27,b3,C26),(A27,b3,C27),(A27,b3,C28),(A27,b3,C29),(A27,b4,C1),( A27,b4,C2),(A27,b4,C3),(A27,b4,C4),(A27,b4,C5),(A27,b4,C6),(A27,b4,C7),(A27,b4,C8),(A2 7,b4,C9),(A27,b4,C10),(A27,b4,C11),(A27,b4,C12),(A27,b4,C13),(A27,b4,C14),(A27,b4,C15) ,(A27,b4,C16),(A27,b4,C17),(A27,b4,C18),(A27,b4,C19),(A27,b4,C20),(A27,b4,C21),(A27,b4 ,C22),(A27,b4,C23),(A27,b4,C24),(A27,b4,C25),(A27,b4,C26),(A27,b4,C27),(A27,b4,C28),(A 27,b4,C29),(A27,b5,C1),(A27,b5,C2),(A27,b5,C3),(A27,b5,C4),(A27,b5,C5),(A27,b5,C6),(A2 7,b5,C7),(A27,b5,C8),(A27,b5,C9),(A27,b5,C10),(A27,b5,C11),(A27,b5,C12),(A27,b5,C13),( A27,b5,C14),(A27,b5,C15),(A27,b5,C16),(A27,b5,C17),(A27,b5,C18),(A27,b5,C19),(A27,b5, C20),(A27,b5,C21),(A27,b5,C22),(A27,b5,C23),(A27,b5,C24),(A27,b5,C25),(A27,b5,C26),(A 27,b5,C27),(A27,b5,C28),(A27,b5,C29),(A27,b6,C1),(A27,b6,C2),(A27,b6,C3),(A27,b6,C4),( A27,b6,C5),(A27,b6,C6),(A27,b6,C7),(A27,b6,C8),(A27,b6,C9),(A27,b6,C10),(A27,b6,C11),( A27,b6,C12),(A27,b6,C13),(A27,b6,C14),(A27,b6,C15),(A27,b6,C16),(A27,b6,C17),(A27,b6, C18),(A27,b6,C19),(A27,b6,C20),(A27,b6,C27),(A27,b6,C22),(A27,b6,C23),(A27,b6,C24),(A 27,b6,C25),(A27,b6,C26),(A27,b6,C27),(A27,b6,C28),(A27,b6,C29),(A27,b7,C1),(A27,b7,C2) ,(A27,b7,C3),(A27,b7,C4),(A27,b7,C5),(A27,b7,C6),(A27,b7,C7),(A27,b7,C8),(A27,b7,C9),(A 27,b7,C10),(A27,b7,C11),(A27,b7,C12),(A27,b7,C13),(A27,b7,C14),(A27,b7,C15),(A27,b7,C 16),(A27,b7,C17),(A27,b7,C18),(A27,b7,C19),(A27,b7,C20),(A27,b7,C21),(A27,b7,C22),(A27 ,b7,C23),(A27,b7,C24),(A27,b7,C25),(A27,b7,C26),(A27,b7,C27),(A27,b7,C28),(A27,b7,C29) ,(A28,b1,C1),(A28,b1,C2),(A28,b1,C3),(A28,b1,C4),(A28,b1,C5),(A28,b1,C6),(A28,b1,C7),(A 28,b1,C8),(A28,b1,C9),(A28,b1,C10),(A28,b1,C11),(A28,b1,C12),(A28,b1,C13),(A28,b1,C14) ,(A28,b1,C15),(A28,b1,C16),(A28,b1,C17),(A28,b1,C18),(A28,b1,C19),(A28,b1,C20),(A28,b1 ,C21),(A28,b1,C22),(A28,b1,C23),(A28,b1,C24),(A28,b1,C25),(A28,b1,C26),(A28,b1,C27),(A 28,b1,C28),(A28,b1,C29),(A28,b2,C1),(A28,b2,C2),(A28,b2,C3),(A28,b2,C4),(A28,b2,C5),(A 28,b2,C6),(A28,b2,C7),(A28,b2,C8),(A28,b2,C9),(A28,b2,C10),(A28,b2,C11),(A28,b2,C12),( A28,b2,C13),(A28,b2,C14),(A28,b2,C15),(A28,b2,C16),(A28,b2,C17),(A28,b2,C18),(A28,b2, C19),(A28,b2,C20),(A28,b2,C21),(A28,b2,C22),(A28,b2,C23),(A28,b2,C24),(A28,b2,C25),(A 28,b2,C26),(A28,b2,C27),(A28,b2,C28),(A28,b2,C29),(A28,b3,C1),(A28,b3,C2),(A28,b3,C3),( 8,b3,C4),(A28,b3,C5),(A28,b3,C6),(A28,b3,C7),(A28,b3,C8),(A28,b3,C9),(A28,b3,C10),(A 28,b3,C11),(A28,b3,C12),(A28,b3,C13),(A28,b3,C14),(A28,b3,C15),(A28,b3,C16),(A28,b3,C 17),(A28,b3,C18),(A28,b3,C19),(A28,b3,C20),(A28,b3,C21),(A28,b3,C22),(A28,b3,C23),(A28 ,b3,C24),(A28,b3,C25),(A28,b3,C26),(A28,b3,C27),(A28,b3,C28),(A28,b3,C29),(A28,b4,C1),( A28,b4,C2),(A28,b4,C3),(A28,b4,C4),(A28,b4,C5),(A28,b4,C6),(A28,b4,C7),(A28,b4,C8),(A2 8,b4,C9),(A28,b4,C10),(A28,b4,C11),(A28,b4,C12),(A28,b4,C13),(A28,b4,C14),(A28,b4,C15) ,(A28,b4,C16),(A28,b4,C17),(A28,b4,C18),(A28,b4,C19),(A28,b4,C20),(A28,b4,C21),(A28,b4 ,C22),(A28,b4,C23),(A28,b4,C24),(A28,b4,C25),(A28,b4,C26),(A28,b4,C27),(A28,b4,C28),(A 28,b4,C29),(A28,b5,C1),(A28,b5,C2),(A28,b5,C3),(A28,b5,C4),(A28,b5,C5),(A28,b5,C6),(A2 8,b5,C7),(A28,b5,C8),(A28,b5,C9),(A28,b5,C10),(A28,b5,C11),(A28,b5,C12),(A28,b5,C13),( A28,b5,C14),(A28,b5,C15),(A28,b5,C16),(A28,b5,C17),(A28,b5,C18),(A28,b5,C19),(A28,b5, C20),(A28,b5,C21),(A28,b5,C22),(A28,b5,C23),(A28,b5,C24),(A28,b5,C25),(A28,b5,C26),(A 28,b5,C27),(A28,b5,C28),(A28,b5,C29),(A28,b6,C1),(A28,b6,C2),(A28,b6,C3),(A28,b6,C4),( A28,b6,C5),(A28,b6,C6),(A28,b6,C7),(A28,b6,C8),(A28,b6,C9),(A28,b6,C10),(A28,b6,C11),( A28,b6,C12),(A28,b6,C13),(A28,b6,C14),(A28,b6,C15),(A28,b6,C16),(A28,b6,C17),(A28,b6, C18),(A28,b6,C19),(A28,b6,C20),(A28,b6,C21),(A28,b6,C22),(A28,b6,C23),(A28,b6,C24),(A 28,b6,C25),(A28,b6,C26),(A28,b6,C27),(A28,b6,C28),(A28,b6,C29),(A28,b7,C1),(A28,b7,C2) ,(A28,b7,C3),(A28,b7,C4),(A28,b7,C5),(A28,b7,C6),(A28,b7,C7),(A28,b7,C8),(A28,b7,C9),(A 28,b7,C10),(A28,b7,C11),(A28,b7,C12),(A28,b7,C13),(A28,b7,C14),(A28,b7,C15),(A28,b7,C 16),(A28,b7,C17),(A28,b7,C18),(A28,b7,C19),(A28,b7,C20),(A28,b7,C21),(A28,b7,C22),(A28 ,b7,C23),(A28,b7,C24),(A28,b7,C25),(A28,b7,C26),(A28,b7,C27),(A28,b7,C28),(A28,b7,C29) ,(A29,b1,C1),(A29,b1,C2),(A29,b1,C3),(A29,b1,C4),(A29,b1,C5),(A29,b1,C6),(A29,b1,C7),(A 29,b1,C8),(A29,b1,C9),(A29,b1,C10),(A29,b1,C11),(A29,b1,C12),(A29,b1,C13),(A29,b1,C14) ,(A29,b1,C15),(A29,b1,C16),(A29,b1,C17),(A29,b1,C18),(A29,b1,C19),(A29,b1,C20),(A29,b1 ,C21),(A29,b1,C22),(A29,b1,C23),(A29,b1,C24),(A29,b1,C25),(A29,b1,C26),(A29,b1,C27),(A 29,b1,C28),(A29,b1,C29),(A29,b2,C1),(A29,b2,C2),(A29,b2,C3),(A29,b2,C4),(A29,b2,C5),(A 29,b2,C6),(A29,b2,C7),(A29,b2,C8),(A29,b2,C9),(A29,b2,C10),(A29,b2,C11),(A29,b2,C12),( A29,b2,C13),(A29,b2,C14),(A29,b2,C15),(A29,b2,C16),(A29,b2,C17),(A29,b2,C18),(A29,b2, C19),(A29,b2,C20),(A29,b2,C21),(A29,b2,C22),(A29,b2,C23),(A29,b2,C24),(A29,b2,C25),(A 29,b2,C26),(A29,b2,C27),(A29,b2,C28),(A29,b2,C29),(A29,b3,C1),(A29,b3,C2),(A29,b3,C3),( A29,b3,C4),(A29,b3,C5),(A29,b3,C6),(A29,b3,C7),(A29,b3,C8),(A29,b3,C9),(A29,b3,C10),(A 29,b3,C11),(A29,b3,C12),(A29,b3,C13),(A29,b3,C14),(A29,b3,C15),(A29,b3,C16),(A29,b3,C 17),(A29,b3,C18),(A29,b3,C19),(A29,b3,C20),(A29,b3,C21),(A29,b3,C22),(A29,b3,C23),(A29 ,b3,C24),(A29,b3,C25),(A29,b3,C26),(A29,b3,C27),(A29,b3,C28),(A29,b3,C29),(A29,b4,C1),( A29,b4,C2),(A29,b4,C3),(A29,b4,C4),(A29,b4,C5),(A29,b4,C6),(A29,b4,C7),(A29,b4,C8),(A2 9,b4,C9),(A29,b4,C10),(A29,b4,C11),(A29,b4,C12),(A29,b4,C13),(A29,b4,C14),(A29,b4,C15) ,(A29,b4,C16),(A29,b4,C17),(A29,b4,C18),(A29,b4,C19),(A29,b4,C20),(A29,b4,C21),(A29,b4 ,C22),(A29,b4,C23),(A29,b4,C24),(A29,b4,C25),(A29,b4,C26),(A29,b4,C27),(A29,b4,C28),(A 29,b4,C29),(A29,b5,C1),(A29,b5,C2),(A29,b5,C3),(A29,b5,C4),(A29,b5,C5),(A29,b5,C6),(A2 9,b5,C7),(A29,b5,C8),(A29,b5,C9),(A29,b5,C10),(A29,b5,C11),(A29,b5,C12),(A29,b5,C13),( A29,b5,C14),(A29,b5,C15),(A29,b5,C16),(A29,b5,C17),(A29,b5,C18),(A29,b5,C19),(A29,b5, C20),(A29,b5,C21),(A29,b5,C22),(A29,b5,C23),(A29,b5,C24),(A29,b5,C25),(A29,b5,C26),(A 29,b5,C27),(A29,b5,C28),(A29,b5,C29),(A29,b6,C1),(A29,b6,C2),(A29,b6,C3),(A29,b6,C4),( A29,b6,C5),(A29,b6,C6),(A29,b6,C7),(A29,b6,C8),(A29,b6,C9),(A29,b6,C10),(A29,b6,C11),( A29,b6,C12),(A29,b6,C13),(A29,b6,C14),(A29,b6,C15),(A29,b6,C16),(A29,b6,C17),(A29,b6, C18),(A29,b6,C19),(A29,b6,C20),(A29,b6,C21),(A29,b6,C22),(A29,b6,C23),(A29,b6,C24),(A 29,b6,C25),(A29,b6,C26),(A29,b6,C27),(A29,b6,C28),(A29,b6,C29),(A29,b7,C1),(A29,b7,C2) ,(A29,b7,C3),(A29,b7,C4),(A29,b7,C5),(A29,b7,C6),(A29,b7,C7),(A29,b7,C8),(A29,b7,C9),(A 29,b7,C10),(A29,b7,C11),(A29,b7,C12),(A29,b7,C13),(A29,b7,C14),(A29,b7,C15),(A29,b7,C 16),(A29,b7,C17),(A29,b7,C18),(A29,b7,C19),(A29,b7,C20),(A29,b7,C21),(A29,b7,C22),(A29 ,b7,C23),(A29,b7,C24),(A29,b7,C25),(A29,b7,C26),(A29,b7,C27),(A29,b7,C28),(A29,b7,C29) ,(A30,b1,C1),(A30,b1,C2),(A30,b1,C3),(A30,b1,C4),(A30,b1,C5),(A30,b1,C6),(A30,b1,C7),(A 30,b1,C8),(A30,b1,C9),(A30,b1,C10),(A30,b1,C11),(A30,b1,C12),(A30,b1,C13),(A30,b1,C14) ,(A30,b1,C15),(A30,b1,C16),(A30,b1,C17),(A30,b1,C18),(A30,b1,C19),(A30,b1,C20),(A30,b1 ,C21),(A30,b1,C22),(A30,b1,C23),(A30,b1,C24),(A30,b1,C25),(A30,b1,C26),(A30,b1,C27),(A 30,b1,C28),(A30,b1,C29),(A30,b2,C1),(A30,b2,C,2),(A30,b2,C3),(A30,b2,C4),(A30,b2,C5),(A 30,b2,C6),(A30,b2,C7),(A30,b2,C8),(A30,b2,C9),(A30,b2,C10),(A30,b2,C11),(A30,b2,C12),( A30,b2,C13),(A30,b2,C14),(A30,b2,C15),(A30,b2,C16),(A30,b2,C17),(A30,b2,C18),(A30,b2, C19),(A30,b2,C20),(A30,b2,C21),(A30,b2,C22),(A30,b2,C23),(A30,b2,C24),(A30,b2,C25),(A 30,b2,C26),(A30,b2,C27),(A30,b2,C28),(A30,b2,C29),(A30,b3,C1),(A30,b3,C2),(A30,b3,C3),( A30,b3,C4),(A30,b3,C5),(A30,b3,C6),(A30,b3,C7),(A30,b3,C8),(A30,b3,C9),(A30,b3,C10),(A 30,b3,C11),(A30,b3,C12),(A30,b3,C13),(A30,b3,C14),(A30,b3,C15),(A30,b3,C16),(A30,b3,C 17),(A30,b3,C18),(A30,b3,C19),(A30,b3,C20),(A30,b3,C21),(A30,b3,C22),(A30,b3,C23),(A30 ,b3,C24),(A30,b3,C25),(A30,b3,C26),(A30,b3,C27),(A30,b3,C28),(A30,b3,C29),(A30,b4,C1),( A30,b4,C2),(A30,b4,C3),(A30,b4,C4),(A30,b4,C5),(A30,b4,C6),(A30,b4,C7),(A30,b4,C8),(A3 0,b4,C9),(A30,b4,C10),(A30,b4,C11),(A30,b4,C12),(A30,b4,C13),(A30,b4,C14),(A30,b4,C15) ,(A30,b4,C16),(A30,b4,C17),(A30,b4,C18),(A30,b4,C19),(A30,b4,C20),(A30,b4,C21),(A30,b4 ,C22),(A30,b4,C23),(A30,b4,C24),(A30,b4,C25),(A30,b4,C26),(A30,b4,C27),(A30,b4,C28),(A 30,b4,C29),(A30,b5,C1),(A30,b5,C2),(A30,b5,C3),(A30,b5,C4),(A30,b5,C5),(A30,b5,C6),(A3 0,b5,C7),(A30,b5,C8),(A30,b5,C9),(A30,b5,C10),(A30,b5,C11),(A30,b5,C12),(A30,b5,C13),( A30,b5,C4),(A30,b5,C15),(A30,b5,C16),(A30,b5,C17),(A30,b5,C18),(A30,b5,C19),(A30,b5, C20),(A30,b5,C21),(A30,b5,C22),(A30,b5,C23),(A30,b5,C24),(A30,b5,C25),(A30,b5,C26),(A 30,b5,C27),(A30,b5,C28),(A30,b5,C29),(A30,b6,C1),(A30,b6,C2),(A30,b6,C3),(A30,b6,C4),( A30,b6,C5),(A30,b6,C6),(A30,b6,C7),(A30,b6,C8),(A30,b6,C9),(A30,b6,C10),(A30,b6,C11),( A30,b6,C12),(A30,b6,C13),(A30,b6,C14),(A30,b6,C15),(A30,b6,C16),(A30,b6,C17),(A30,b6, C18),(A30,b6,C19),(A30,b6,C20),(A30,b6,C21),(A30,b6,C22),(A30,b6,C23),(A30,b6,C24),(A 30,b6,C25),(A30,b6,C26),(A30,b6,C27),(A30,b6,C28),(A30,b6,C29),(A30,b7,C1),(A30,b7,C2) ,(A30,b7,C3),(A30,b7,C4),(A30,b7,C5),(A30,b7,C6),(A30,b7,C7),(A30,b7,C8),(A30,b7,C9),(A 30,b7,C10),(A30,b7,C11),(A30,b7,C12),(A30,b7,C13),(A30,b7,C14),(A30,b7,C15),(A30,b7,C 16),(A30,b7,C17),(A30,b7,C18),(A30,b7,C19),(A30,b7,C20),(A30,b7,C21),(A30,b7,C22),(A30 ,b7,C23),(A30,b7,C24),(A30,b7,C25),(A30,b7,C26),(A30,b7,C27),(A30,b7,C28),(A30,b7,C29),

(A31,b1,C1),(A31,b1,C2),(A31,b1,C3),(A31,b1,C4),(A31,b1,C5),(A31,b1,C6),(A31,b1,C7),(A 31,b1,C8),(A31,b1,C9),(A31,b1,C10),(A31,b1,C11),(A31,b1,C12),(A31,b1,C13),(A31,b1,C14) ,(A31,b1,C15),(A31,b1,C16),(A31,b1,C17),(A31,b1,C18),(A31,b1,C19),(A31,b1,C20),(A31,b1 ,C21),(A31,b1,C22),(A31,b1,C23),(A31,b1,C24),(A31,b1,C25),(A31,b1,C26),(A31,b1,C21),(A 31,b1,C28),(A31,b1,C29),(A31,b2,C1),(A31,b2,C2),(A31,b2,C3),(A31,b2,C4),(A31,b2,C5),(A 31,b2,C6),(A31,b2,C7),(A31,b2,C8),(A31,b2,C9),(A31,b2,C10),(A31,b2,C11),(A31,b2,C12),( A31,b2,C13),(A31,b2,C14),(A31,b2,C15),(A31,b2,C16),(A31,b2,C17),(A31,b2,C18),(A31,b2, C19),(A31,b2,C20),(A31,b2,C21),(A31,b2,C22),(A31,b2,C23),(A31,b2,C24),(A31,b2,C25),(A 31,b2,C26),(A31,b2,C27),(A31,b2,C28),(A31,b2,C29),(A31,b3,C1),(A31,b3,C2),(A31,b3,C3),( A31,b3,C4),(A31,b3,C5),(A31,b3,C6),(A31,b3,C7),(A31,b3,C8),(A31,b3,C9),(A31,b3,C10),(A 31,b3,C11),(A31,b3,C12),(A31,b3,C13),(A31,b3,C14),(A31,b3,C15),(A31,b3,C16),(A31,b3,C 17),(A31,b3,C18),(A31,b3,C19),(A31,b3,C20),(A31,b3,C21),(A31,b3,C22),(A31,b3,C23),(A31 ,b3,C24),(A31,b3,C25),(A31,b3,C26),(A31,b3,C27),(A31,b3,C28),(A31,b3,C29),(A31,b4,C1),( A31,b4,C2),(A31,b4,C3),(A31,b4,C4),(A31,b4,C5),(A31,b4,C6),(A31,b4,C7),(A31,b4,C8),(A3 1,b4,C9),(A31,b4,C10),(A31,b4,C11),(A31,b4,C12),(A31,b4,C13),(A31,b4,C14),(A31,b4,C15) ,(A31,b4,C16),(A31,b4,C17),(A31,b4,C18),(A31,b4,C19),(A31,b4,C20),(A31,b4,C21),(A31,b4 ,C22),(A31,b4,C23),(A31,b4,C24),(A31,b4,C25),(A31,b4,C26),(A31,b4,C27),(A31,b4,C28),(A 31,b4,C29),(A31,b5,C1),(A31,b5,C2),(A31,b5,C3),(A31,b5,C4),(A31,b5,C5),(A31,b5,C6),(A3 1,b5,C7),(A31,b5,C8),(A31,b5,C9),(A31,b5,C10),(A31,b5,C11),(A31,b5,C12),(A31,b5,C13),( A31,b5,C14),(A31,b5,C15),(A31,b5,C16),(A31,b5,C17),(A31,b5,C18),(A31,b5,C19),(A31,b5, C20),(A31,b5,C21),(A31,b5,C22),(A31,b5,C23),(A31,b5,C24),(A31,b5,C25),(A31,b5,C26),(A 31,b5,C27),(A31,b5,C28),(A31,b5,C29),(A31,b6,C1),(A31,b6,C2),(A31,b6,C3),(A31,b6,C4),( A31,b6,C5),(A31,b6,C6),(A31,b6,C7),(A31,b6,C8),(A31,b6,C9),(A31,b6,C10),(A31,b6,C11),( A31,b6,C12),(A31,b6,C13),(A31,b6,C14),(A31,b6,C15),(A31,b6,C16),(A31,b6,C17),(A31,b6, C18),(A31,b6,C19),(A31,b6,C20),(A31,b6,C21),(A31,b6,C22),(A31,b6,C23),(A31,b6,C24),(A 31,b6,C25),(A31,b6,C26),(A31,b6,C27),(A31,b6,C28),(A31,b6,C29),(A31,b7,C1),(A31,b7,C2) ,(A31,b7,C3),(A31,b7,C4),(A31,b7,C5),(A31,b7,C6),(A31,b7,C7),(A31,b7,C8),(A31,b7,C9),(A 31,b7,C10),(A31,b7,C11),(A31,b7,C12),(A31,b7,C13),(A31,b7,C14),(A31,b7,C15),(A31,b7,C 16),(A31,b7,C17),(A31,b7,C18),(A31,b7,C19),(A31,b7,C20),(A31,b7,C21),(A31,b7,C22),(A31 ,b7,C23),(A31,b7,C24),(A31,b7,C25),(A31,b7,C26),(A31,b7,C27),(A31,b7,C28),(A31,b7,C29) ,(A32,b1,C1),(A32,b1,C2),(A32,b1,C3),(A32,b1,C4),(A32,b1,C5),(A32,b1,C6),(A32,b1,C7),(A 32,b1,C8),(A32,b1,C9),(A32,b1,C10),(A32,b1,C11),(A32,b1,C12),(A32,b1,C13),(A32,b1,C14) ,(A32,b1,C15),(A32,b1,C16),(A32,b1,C17),(A32,b1,C18),(A32,b1,C19),(A32,b1,C20),(A32,b1 , ,C21),(A32,b1,C22),(A32,b1,C23),(A32,b1,C24),(A32,b1,C25),(A32,b1,C26),(A32,b,C27),(A 32,b1,C28),(A32,b1,C29),(A32,b2,C1),(A32,b2,C2),(A32,b2,C3),(A32,b2,C4),(A32,b2,C5),(A 32,b2,C6),(A32,b2,C7),(A32,b2,C8),(A32,b2,C9),(A32,b2,C10),(A32,b2,C11),(A32,b2,C12),( A32,b2,C13),(A32,b2,C14),(A32,b2,C15),(A32,b2,C16),(A32,b2,C17),(A32,b2,C18),(A32,b2, C19),(A32,b2,C20),(A32,b2,C21),(A32,b2,C22),(A32,b2,C23),(A32,b2,C24),(A32,b2,C25),(A 32,b2,C26),(A32,b2,C27),(A32,b2,C28),(A32,b2,C29),(A32,b3,C1),(A32,b3,C2),(A32,b3,C3),( A32,b3,C4),(A32,b3,C5),(A32,b3,C6),(A32,b3,C7),(A32,b3,C8),(A32,b3,C9),(A32,b3,C10),(A 32,b3,C11),(A32,b3,C12),(A32,b3,C13),(A32,b3,C14),(A32,b3,C15),(A32,b3,C16),(A32,b3,C 17),(A32,b3,C18),(A32,b3,C19),(A32,b3,C20),(A32,b3,C21),(A32,b3,C22),(A32,b3,C23),(A32 ,b3,C24),(A32,b3,C25),(A32,b3,C26),(A32,b3,C27),(A32,b3,C28),(A32,b3,C29),(A32,b4,C1),( A32,b4,C2),(A32,b4,C3),(A32,b4,C4),(A32,b4,C5),(A32,b4,C6),(A32,b4,C7),(A32,b4,C8),(A3 2,b4,C9),(A32,b4,C10),(A32,b4,C11),(A32,b4,C12),(A32,b4,C13),(A32,b4,C14),(A32,b4,C15) ,(A32,b4,C16),(A32,b4,C17),(A32,b4,C18),(A32,b4,C19),(A32,b4,C20),(A32,b4,C21),(A32,b4 ,C22),(A32,b4,C23),(A32,b4,C24),(A32,b4,C25),(A32,b4,C26),(A32,b4,C27),(A32,b4,C28),(A 32,b4,C29),(A32,b5,C1),(A32,b5,C2),(A32,b5,C3),(A32,b5,C4),(A32,b5,C5),(A32,b5,C6),(A3 2,b5,C7),(A32,b5,C8),(A32,b5,C9),(A32,b5,C10),(A32,b5,C11),(A32,b5,C12),(A32,b5,C13),( A32,b5,C14),(A32,b5,C15),(A32,b5,C16),(A32,b5,C17),(A32,b5,C18),(A32,b5,C19),(A32,b5, C20),(A32,b5,C21),(A32,b5,C22),(A32,b5,C23),(A32,b5,C24),(A32,b5,C25),(A32,b5,C26),(A 32,b5,C27),(A32,b5,C28),(A32,b5,C29),(A32,b6,C1),(A32,b6,C2),(A32,b6,C3),(A32,b6,C4),( A32,b6,C5),(A32,b6,C6),(A32,b6,C7),(A32,b6,C8),(A32,b6,C9),(A32,b6,C10),(A32,b6,C11),( A32,b6,C12),(A32,b6,C13),(A32,b6,C14),(A32,b6,C15),(A32,b6,C16),(A32,b6,C17),(A32,b6, C18),(A32,b6,C19),(A32,b6,C20),(A32,b6,C21),(A32,b6,C22),(A32,b6,C23),(A32,b6,C24),(A 32,b6,C25),(A32,b6,C26),(A32,b6,C27),(A32,b6,C28),(A32,b6,C29),(A32,b7,C1),(A32,b7,C2) ,(A32,b7,C3),(A32,b7,C4),(A32,b7,C5),(A32,b7,C6),(A32,b7,C7),(A32,b7,C8),(A32,b7,C9),(4 32,b7,C10),(A32,b7,C11),(A32,b7,C12),(A32,b7,C13),(A32,b7,C14),(A32,b7,C15),(A32,b7,C 16),(A32,b7,C17),(A32,b7,C18),(A32,b7,C19),(A32,b7,C20),(A32,b7,C21),(A32,b7,C22),(A32 ,b7,C23),(A632,b7,C24),(A32,b7,C25),(A32,b7,C26),(A32,b7,C27),(A32,b7,C28),(A32,b7,C29) ,(A33,b1,C1),(A33,b1,C2),(A33,b1,C3),(A33,b1,C4),(A33,b1,C5),(A33,b1,C6),(A33,b1,C7),(A 33,b1,C8),(A33,b1,C9),(A33,b1,C10),(A33,b1,C11),(A33,b1,C12),(A33,b1,C13),(A33,b1,C14) ,(A33,b1,C15),(A33,b1,C16),(A33,b1,C17),(A33,b1,C18),(A33,b1,C19),(A33,b1,C20),(A33,b1 ,C21),(A33,b1,C22),(A33,b1,C23),(A33,b1,C24),(A33,b1,C25),(A33,b1,C26),(A33,b1,C27),(A 33,b1,C28),(A33,b1,C29),(A33,b2,C1),(A33,b2,C2),(A33,b2,C3),(A33,b2,C4),(A33,b2,C5),(A 33,b2,C6),(A33,b2,C7),(A33,b2,C8),(A33,b2,C9),(A33,b2,C10),(A33,b2,C11),(A33,b2,C12),( A33,b2,C13),(A33,b2,C14),(A33,b2,C15),(A33,b2,C16),(A33,b2,C17),(A33,b2,C18),(A33,b2, C19),(A33,b2,C20),(A33,b2,C21),(A33,b2,C22),(A33,b2,C23),(A33,b2,C24),(A33,b2,C25),(A 33,b2,C26),(A33,b2,C27),(A33,b2,C28),(A33,b2,C29),(A33,b3,C1),(A33,b3,C2),(A33,b3,C3),( A33,b3,C4),(A33,b3,C5),(A33,b3,C6),(A33,b3,C7),(A33,b3,C8),(A33,b3,C9),(A33,b3,C10),(A 33,b3,C11),(A33,b3,C12),(A33,b3,C13),(A33,b3,C14),(A33,b3,C15),(A33,b3,C16),(A33,b3,C 17),(A33,b3,C18),(A33,b3,C19),(A33,b3,C20),(A33,b3,C21),(A33,b3,C22),(A33,b3,C23),(A33 ,b3,C24),(A33,b3,C25),(A33,b3,C26),(A33,b3,C27),(A33,b3,C28),(A33,b3,C29),(A33,b4,C1),( A33,b4,C2),(A33,b4,C3),(A33,b4,C4),(A33,b4,C5),(A33,b4,C6),(A33,b4,C7),(A33,b4,C8),(A3 3,b4,C9),(A33,b4,C10),(A33,b4,C11),(A33,b4,C12),(A33,b4,C13),(A33,b4,C14),(A33,b4,C15) ,(A33,b4,C16),(A33,b4,C17),(A33,b4,C18),(A33,b4,C19),(A33,b4,C20),(A33,b4,C21),(A33,b4 ,C22),(A33,b4,C23),(A33,b4,C24),(A33,b4,C25),(A33,b4,C26),(A33,b4,C27),(A33,b4,C28),(A 33,b4,C29),(A33,b5,C1),(A33,b5,C2),(A33,b5,C3),(A33,b5,C4),(A33,b5,C5),(A33,b5,C6),(A3 3,b5,C7),(A33,b5,C8),(A33,b5,C9),(A33,b5,C10),(A33,b5,C11),(A33,b5,C12),(A33,b5,C13),( A33,b5,C14),(A33,b5,C15),(A33,b5,C16),(A33,b5,C17),(A33,b5,C18),(A33,b5,C19),(A33,b5, C20),(A33,b5,C21),(A33,b5,C22),(A33,b5,C23),(A33,b5,C24),(A33,b5,C25),(A33,b5,C26),(A 33,b5,C27),(A33,b5,C28),(A33,b5,C29),(A33,b6,C1),(A33,b6,C2),(A33,b6,C3),(A33,b6,C4),( A33,b6,C5),(A33,b6,C6),(A33,b6,C7),(A33,b6,C8),(A33,b6,C9),(A33,b6,C10),(A33,b6,C11),( A33,b6,C12),(A33,b6,C13),(A33,b6,C14),(A33,b6,C15),(A33,b6,C16),(A33,b6,C17),(A33,b6, C18),(A33,b6,C19),(A33,b6,C20),(A33,b6,C21),(A33,b6,C22),(A33,b6,C23),(A33,b6,C24),(A 33,b6,C25),(A33,b6,C26),(A33,b6,C27),(A33,b6,C28),(A33,b6,C29),(A33,b7,C1),(A33,b7,C2) ,(A33,b7,C3),(A33,b7,C4),(A33,b7,C5),(A33,b7,C6),(A33,b7,C7),(A33,b7,C8),(A33,b7,C9),(A 33,b7,C10),(A33,b7,C11),(A33,b7,C12),(A33,b7,C13),(A33,b7,C14),(A33,b7,C15),(A33,b7,C 16),(A33,b7,C17),(A33,b7,C18),(A33,b7,C19),(A33,b7,C20),(A33,b7,C21),(A33,b7,C22),(A33 ,b7,C23),(A33,b7,C24),(A33,b7,C25),(A33,b7,C26),(A33,b7,C27),(A33,b7,C28),(A33,b7,C29) ,(A34,b1,C1),(A34,b1,C2),(A34,b1,C3),(A34,b1,C4),(A34,b1,C5),(A34,b1,C6),(A34,b1,C7),(A 34,b1,C8),(A34,b1,C9),(A34,b1,C10),(A34,b1,C11),(A34,b1,C12),(A34,b1,C13),(A34,b1,C14) ,(A34,b1,C15),(A34,b1,C16),(A34,b1,C17),(A34,b1,C18),(A34,b1,C19),(A34,b1,C20),(A34,b1 ,C21),(A34,b1,C22),(A34,b1,C23),(A34,b1,C24),(A34,b1,C25),(A34,b1,C26),(A34,b1,C27),(A 34,b1,C28),(A34,b1,C29),(A34,b2,C1),(A34,b2,C2),(A34,b2,C3),(A34,b2,C4),(A34,b2,C5),(A 34,b2,C6),(A34,b2,C7),(A34,b2,C8),(A34,b2,C9),(A34,b2,C10),(A34,b2,C11),(A34,b2,C12),( A34,b2,C13),(A34,b2,C14),(A34,b2,C15),(A34,b2,C16),(A34,b2,C17),(A34,b2,C18),(A34,b2, C19),(A34,b2,C20),(A34,b2,C21),(A34,b2,C22),(A34,b2,C23),(A34,b2,C24),(A34,b2,C25),(A 34,b2,C26),(A34,b2,C27),(A34,b2,C28),(A34,b2,C29),(A34,b3,C1),(A34,b3,C2),(A34,b3,C3),( A34,b3,C4),(A34,b3,C5),(A34,b3,C6),(A34,b3,C7),(A34,b3,C8),(A34,b3,C9),(A34,b3,C10),(A 34,b3,C11),(A34,b3,C12),(A34,b13,C13),(A34,b3,C14),(A34,b3,C15),(A34,b3,C16),(A34,b3,C 17),(A34,b3,C18),(A34,b3,C19),(A34,b3,C20),(A34,b3,C21),(A34,b3,C22),(A34,b3,C23),(A34 ,b3,C24),(A34,b3,C25),(A34,b3,C26),(A34,b3,C27),(A34,b3,C28),(A34,b3,C29),(A34,b4,C1),( A34,b4,C2),(A34,b4,C3),(A34,b4,C4),(A34,b4,C5),(A34,b4;C6),(A34,b4,C7),(A34,b4,C8),(A3 4,b4,C9),(A34,b4,C10),(A34,b4,C11),(A34,b4,C12),(A34,b4,C13),(A34,b4,C14),(A34,b4,C15) ,(A34,b4,C16),(A34,b4,C17),(A34,b4,C18),(A34,b4,C19),(A34,b4,C20),(A34,b4,C21),(A34,b4 ,C22),(A34,b4,C23),(A34,b4,C24),(A34,b4,C25),(A34,b4,C26),(A34,b4,C27),(A34,b4,C28),(A 34,b4,C29),(A34,b5,C1),(A34,b5,C2),(A34,b5,C3),(A34,b5,C4),(A34,b5,C5),(A34,b5,C6),(A3 4,b5,C7),(A34,b5,C8),(A34,b5,C9),(A34,b5,C10),(A34,b5,C11),(A34,b5,C12),(A34,b5,C13),( A34,b5,C14),(A34,b5,C15),(A34,b5,C16),(A34,b5,C17),(A34,b5,C18),(A34,b5,C19),(A34,b5, C20),(A34,b5,C21),(A34,b5,C22),(A34,b5,C23),(A34,b5,C24),(A34,b5,C25),(A34,b5,C26),(A 34,b5,C27),(A34,b5,C28),(A34,b5,C29),(A34,b6,C1),(A34,b6,C2),(A34,b6,C3),(A34,b6,C4),( A34,b6,C5),(A34,b6,C6),(A34,b6,C7),(A34,b6,C8),(A34,b6,C9),(A34,b6,C10),(A34,b6,C11),( A34,b6,C12),(A34,b6,C13),(A34,b6,C14),(A34,b6,C15),(A34,b6,C16),(A34,b6,C17),(A34,b6, C18),(A34,b6,C19),(A34,b6,C20),(A34,b6,C21),(A34,b6,C22),(A34,b6,C23),(A34,b6,C24),(A 34,b6,C25),(A34,b0,C26),(A34,b6,C27),(A34,b6,C28),(A34,b6,C29),(A34,b7,C1),(A34,b7,C2) ,(A34,b7,C3),(A34,b7,C4),(A34,b7,C5),(A34,b7,C6),(A34,b7,C7),(A34,b7,C8),(A34,b7,C9),(A 34,b7,C10),(A34,b7,C11),(A34,b7,C12),(A34,b7,C13),(A34,b7,C14),(A34,b7,C15),(A34,b7,C 16),(A34,b7,C17),(A34,b7,C18),(A34,b7,C19),(A34,b7,C20),(A34,b7,C21),(A34,b7,C22),(A34 ,b7,C23),(A34,b7,C24),(A34,b7,C25),(A34,b7,C26),(A34,b7,C27),(A34,b7,C28),(A34,b7,C29) ,(A35,b1,C1),(A35,b1,C2),(A35,b1,C3),(A35,b1,C4),(A35,b1,C5),(A35,b1,C6),(A35,b1,C7),(A 35,b1,C8),(A35,b1,C9),(A35,b1,C10),(A35,b1,C11),(A35,b1,C12),(A35,b1,C13),(A35,b1,C14) ,(A35,b1,C15),(A35,b1,C16),(A35,b1,C17),(A35,b1,C18),(A35,b1,C19),(A35,b1,C20),(A35,b1 ,C21),(A35,b1,C22),(A35,b1,C23),(A35,b1,C24),(A35,b1,C25),(A35,b1,C26),(A35,b1,C27),(A 35,b1,C28),(A35,b1,C29),(A35,b2,C1),(A35,b2,C2),(A35,b2,C3),(A35,b2,C4),(A35,b2,C5),(A 35,b2,C6),(A35,b2,C7),(A35,b2,C8),(A35,b2,C9),(A35,b2,C10),(A35,b2,C11),(A35,b2,C12),( A35,b2,C13),(A35,b2,C14),(A35,b2,C15),(A35,b2,C16),(A35,b2,C17),(A35,b2,C18),(A35,b2, C19),(A35,b2,C20),(A35,b2,C21),(A35,b2,C22),(A35,b2,C23),(A35,b2,C24),(A35,b2,C25),(A 35,b2,C26),(A35,b2,C27),(A35,b2,C28),(A35,b2,C29),(A35,b3,C1),(A35,b3,C2),(A35,b3,C3),( A35,b3,C4),(A35,b3,C5),(A35,b3,C6),(A35,b3,C7),(A35,b3,C8),(A35,b3,C9),(A35,b3,C10),(A 35,b3,C11),(A35,b3,C12),(A35,b3,C13),(A35,b3,C14),(A35,b3,C15),(A35,b3,C16),(A35,b3,C 17),(A35,b3,C18),(A35,b3,C19),(A35,b3,C20),(A35,b3,C21),(A35,b3,C22),(A35,b3,C23),(A35 ,b3,C24),(A35,b3,C25),(A35,b3,C26),(A35,b3,C27),(A35,b3,C28),(A35,b3,C29),(A35,b4,C1),( A35,b4,C2),(A35,b4,C3),(A35,b4,C4),(A35,b4,C5),(A35,b4,C6),(A35,b4,C7),(A35,b4,C8),(A3 5,b4,C9),(A35,b4,C10),(A35,b4,C11),(A35,b4,C12),(A35,b4,C13),(A35,b4,C14),(A35,b4,C15) ,(A35,b4,C16),(A35,b4,C17),(A35,b4,C18),(A35,b4,C19),(A35,b4,C20),(A35,b4,C21),(A35,b4 ,C22),(A35,b4,C23),(A35,b4,C24),(A35,b4,C25),(A35,b4,C26),(A35,b4,C27),(A35,b4,C28),(A 35,b4,C29),(A35,b5,C1),(A35,b5,C2),(A35,b5,C3),(A35,b5,C4),(A35,b5,C5),(A35,b5,C6),(A3 5,b5,C7),(A35,b5,C8),(A35,b5,C9),(A35,b5,C10),(A35,b5,C11),(A35,b5,C12),(A35,b5,C13),( A35,b5,C14),(A35,b5,C15),(A35,b5,C16),(A35,b5,C17),(A35,b5,C18),(A35,b5,C19),(A35,b5, C20),(A35,b5,C21),(A35,b5,C22),(A35,b5,C23),(A35,b5,C24),(A35,b5,C25),(A35,b5,C26),(A 35,b5,C27),(A35,b5,C28),(A35,b5,C29),(A35,b6,C1),(A35,b6,C2),(A35,b6,C3),(A35,b6,C4),( A35,b6,C5),(A35,b6,C6),(A35,b6,C7),(A35,b6,C8),(A35,b6,C9),(A35,b6,C10),(A35,b6,C11),( A35,b6,C12),(A35,b6,C13),(A35,b6,C14),(A35,b6,C15),(A35,b6,C16),(A35,b6,C17),(A35,b6, C18),(A35,b6,C19),(A35,b6,C20),(A35,b6,C21),(A35,b6,C22),(A35,b6,C23),(A35,b6,C24),(A 35,b6,C25),(A35,b6,C26),(A35,b6,C27),(A35,b6,C28),(A35,b6,C29),(A35,b7,C1),(A35,b7,C2) ,(A35,b7,C3),(A35,b7,C4),(A35,b7,C5),(A35,b7,C6),(A35,b7,C6),(A35,b7,C8),(A35,b7,C9),(A 35,b7,C10),(A35,b7,C11),(A35,b7,C12),(A35,b7,C13),(A35,b7,C14),(A35,b7,C15),(A35,b7,C 16),(A35,b7,C17),(A35,b7,C18),(A35,b7,C19),(A35,b7,C20),(A35,b7,C21),(A35,b7,C22),(A35 ,b7,C23),(A35,b7,C24),(A35,b7,C25),(A35,b7,C26),(A35,b7,C27),(A35,b7,C28),(A35,b7,C29) ,(A36,b1,C1),(A36,b1,C2),(A36,b1,C3),(A36,b1,C4),(A36,b1,C5),(A36,b1,C6),(A36,b1,C7),(A 36,b1,C8),(A36,b1,C9),(A36,b1,C10),(A36,b1,C11),(A36,b1,C12),(A36,b1,C13),(A36,b1,C14) ,(A36,b1,C15),(A36,b1,C16),(A36,b1,C17),(A36,b1,C18),(A36,b1,C19),(A36,b1,C20),(A36,b1 ,C21),(A36,b1,C22),(A36,b1,C23),(A36,b1,C24),(A36,b1,C25),(A36,b1,C26),(A36,b1,C27),(A 36,b1,C28),(A36,b1,C29),(A36,b2,C1),(A36,b2,C2),(A36,b2,C3),(A36,b2,C4),(A36,b2,C5),(A 36,b2,C6),(A36,b2,C7),(A36,b2,C8),(A36,b2,C9),(A36,b2,C10),(A36,b2,C11),(A36,b2,C12),( A36,b2,C13),(A36,b2,C14),(A36,b2,C15),(A36,b2,C16),(A36,b2,C17),(A36,b2,C18),(A36,b2, C19),(A36,b2,C20),(A36,b2,C21),(A36,b2,C22),(A36,b2,C23),(A36,b2,C24),(A36,b2,C25),(A 36,b2,C26),(A36,b2,C27),(A36,b2,C28),(A36,b2,C29),(A36,b3,C1),(A36,b3,C2),(A36,b3,C3), A36,b3,C4),(A36,b3,C5),(A36,b3,C6),(A36,b3,C7),(A36,b3,C8),(A36,b3,C9),(A36,b3,C10),(A 36,b3,C11),(A36,b3,C12),(A36,b3,C13),(A36,b3,C14),(A36,b3,C15),(A36,b3,C16),(A36,b3,C 17),(A36,b3,C18),(A36,b3,C19),(A36,b3,C20),(A36,b3,C21),(A36,b3,C22),(A36,b3,C23),(A36 ,b3,C24),(A36,b3,C25),(A36,b3,C26),(A36,b3,C27),(A36,b3,C28),(A36,b3,C29),(A36,b4,C1),( A36,b4,C2),(A36,b4,C3),(A36,b4,C4),(A36,b4,C5),(A36,b4,C6),(A36,b4,C7),(A36,b4,C8),(A3 6,b4,C9),(A36,b4,C10),(A36,b4,C11),(A36,b4,C12),(A36,b4,C13),(A36,b4,C14),(A36,b4,C15) ,(A36,b4,C16),(A36,b4,C17),(A36,b4,C18),(A36,b4,C19),(A36,b4,C20),(A36,b4,C21),(A36,b4 ,C22),(A36,b4,C23),(A36,b4,C24),(A36,b4,C25),(A36,b4,C26),(A36,b4,C27),(A36,b4,C28),(A 36,b4,C29),(A36,b5,C1),(A36,b5,C2),(A36,b5,C3),(A36,b5,C4),(A36,b5,C5),(A36,b5,C6),(A3 6,b5,C7),(A36,b5,C8),(A36,b5,C9),(A36,b5,C10),(A36,b5,C11),(A36,b5,C12),(A36,b5,C13),( A36,b5,C14),(A36,b5,C15),(A36,b5,C16),(A36,b5,C17),(A36,b5,C18),(A36,b5,C19),(A36,b5, C20),(A36,b5,C21),(A36,b5,C22),(A36,b5,C23),(A36,b5,C24),(A36,b5,C25),(A36,b5,C26),(A 36,b5,C27),(A36,b5,C28),(A36,b5,C29),(A36,b6,C1),(A36,b6,C2),(A36,b6,C3),(A36,b6,C4),( A36,b6,C5),(A36,b6,C6),(A36,b6,C7),(A36,b6,C8),(A36,b6,C9),(A36,b6,C10),(A36,b6,C11),( A36,b6,C12),(A36,b6,C13),(A36,b6,C14),(A36,b6,C15),(A36,b6,C16),(A36,b6,C17),(A36,b6, C18),(A36,b6,C19),(A36,b6,C20),(A36,b6,C21),(A36,b6,C22),(A36,b6,C23),(A36,b6,C24),(A 36,b6,C25),(A36,b6,C26),(A36,b6,C27),(A36,b6,C28),(A36,b6,C29),(A36,b7,C1),(A36,b7,C2) ,(A36,b7,C3),(A36,b7,C4),(A36,b7,C5),(A36,b7,C6),(A36,b7,C7),(A36,b7,C8),(A36,b7,C9),(A 36,b7,C10),(A36,b7,C11),(A36,b7,C12),(A36,b7,C13),(A36,b7,C14),(A36,b7,C15),(A36,b7,C 16),(A36,b7,C17),(A36,b7,C18),(A36,b7,C19),(A36,b7,C20),(A36,b7,C21),(A36,b7,C22),(A36 ,b7,C23),(A36,b7,C24),(A36,b7,C25),(A36,b7,C26),(A36,b7,C27),(A36,b7,C28),(A36,b7,C29) ,(A37,b1,C1),(A37,b1,C2),(A37,b1,C3),(A37,b1,C4),(A37,b1,C5),(A37,b1,C6),(A37,b1,C7),(A 37,b1,C8),(A37,b1,C9),(A37,b1,C10),(A37,b1,C11),(A37,b1,C12),(A37,b1,C13),(A37,b1,C14) ,(A37,b1,C15),(A37,b1,C16),(A37,b1,C17),(A37,b1,C18),(A37,b1,C19),(A37,b1,C20),(A37,b1 ,C21),(A37,b1,C22),(A37,b1,C23),(A37,b1,C24),(A37,b1,C25),(A37,b1,C26),(A37,b1,C27),(A 37,b1,C28),(A37,b1,C29),(A3?,b2,C1),(A37,b2,C2),(A37,b2,C3),(A37,b2,C4),(A37,b2,C5),(A 37,b2,C6),(A37,b2,C7),(A37,b2,C8),(A37,b2,C9),(A37,b2,C10),(A37,b2,C11),(A37,b2,C12), A37,b2,C13),(A37,b2,C14),(A37,b2,C15),(A37,b2,C16),(A37,b2,C17),(A37,b2,C18),(A37,b2, C19),(A37,b2,C20),(A37,b2,C21),(A37,b2,C22),(A37,b2,C23),(A37,b2,C24),(A37,b2,C25),(A 37,b2,C26),(A37,b2,C27),(A37,b2,C28),(A37,b2,C29),(A37,b3,C1),(A37,b3,C2),(A37,b3,C3),( A37,b3,C4),(A37,b3,G5),(A37,b3,C6),(A37,b3,C7),(A37,b3,C8),(A37,b3,C9),(A37,b3,C10),(A 37,b3,C11),(A37,b3,C12),(A37,b3,C13),(A37,b3,C14),(A37,b3,C15),(A37,b3,C16),(A37,b3,C 17),(A37,b3,C18),(A37,b3,C19),(A37,b3,C20),(A37,b3,C21),(A37,b3,C22),(A37,b3,C23),(A37 ,b3,C24),(A37,b3,C25),(A37,b3,C26),(A37,b3,C27),(A37,b3,C28),(A37,b3,C29),(A37,b4,C1),( A37,b4,C2),(A37,b4,C3),(A37,b4,C4),(A37,b4,C5),(A37,b4,C6),(A37,b4,C7),(A37,b4,C8),(A3 7,b4,C9),(A37,b4,C10),(A37,b4,C10),(A37,b4,C12),(A37,b4,C13),(A37,b4,C14),(A37,b4,C15) ,(A37,b4,C16),(A37,b4,C17),(A37,b4,C18),(A37,b4,C19),(A37,b4,C20),(A37,b4,C21),(A37,b4 ,C22),(A37,b4,C23),(A37,b4,C24),(A37,b4,C25),(A37,b4,C26),(A37,b4,C27),(A37,b4,C28),(A 37,b4,C29),(A37,b5,C1),(A37,b5,C2),(A37,b5,C3),(A37,b5,C4),(A37,b5,C5),(A37,b5,C6),(A3 7,b5,C7),(A37,b5,C8),(A37,b5,C9),(A37,b5,C10),(A37,b5,C11),(A37,b5,C12),(A37,b5,C13),( A37,b5,C14),(A37,b5,C15),(A37,b5,C16),(A37,b5,C17),(A37,b5,C18),(A37,b5,C19),(A37,b5, C20),(A37,b5,C21),(A37,b5,C22),(A37,b5,C23),(A37,b5,C24),(A37,b5,C25),(A37,b5,C26),(A 37,b5,C27),(A37,b5,C28),(A37,b5,C29),(A37,b6,C1),(A37,b6,C2),(A37,b6,C3),(A37,b6,C4),( A37,b6,C5),(A37,b6,C6),(A37,b6,C7),(A37,b6,C8),(A37,b6,C9),(A37,b6,C10),(A37,b6,C11),( A37,b6,C12),(A37,b6,C13),(A37,b6,C14),(A37,b6,C15),(A37,b6,C16),(A37,b6,C17),(A37,b6, C18),(A37,b6,C19),(A37,b6,C20),(A37,b6,C21),(A37,b6,C22),(A37,b6,C23),(A37,b6,C24),(A 37,b6,C25),(A37,b6,C26),(A37,b6,C27),(A37,b6,C28),(A37,b6,C29),(A37,b7,C1),(A37,b7,C2) ,(A37,b7,C3),(A37,b7,C4),(A37,b7,C5),(A37,b7,C6),(A37,b7,C7),(A37,b7,C8),(A37,b7,C9),(A 37,b7,C10),(A37,b7,C11),(A37,b7,C12),(A37,b7,C13),(A37,b7,C14),(A37,b7,C15),(A37,b7,C 16),(A37,b7,C17),(A37,b7,C18),(A37,b7,C19),(A37,b7,C20),(A37,b7,C21),(A37,b7,C22),(A37 ,b7,C23),(A37,b7,C24),(A37,b7,C25),(A37,b7,C26),(A37,b7,C27),(A37,b7,C28),(A37,b7,C29) ,(A38,b1,C1),(A38,b1,C2),(A38,b1,C3),(A38,b1,C4),(A38,b1,C5),(A38,b1,C6),(A38,b1,C7),(A 38,b1,C8),(A38,b1,C9),(A38,b1,C10),(A38,b1,C11),(A38,b1,C12),(A38,b1,C13),(A38,b1,C14) ,(A38,b1,C15),(A38,b1,C16),(A38,b1,C17),(A38,b1,C18),(A38,b1,C19),(A38,b1,C20),(A38,b ,C21),(A38,b1,C22),(A38,b1,C23),(A38,b1,C24),(A38,b1,C25),(A38,b1,C26),(A38,b1,C27),(A 38,b1,C28),(A38,b1,C29),(A38,b2,C1),(A38,b2,C2),(A38,b2,C3),(A38,b2,C4),(A38,b2,C5),(A 38,b2,C6),(A38,b2,C7),(A38,b2,C8),(A38,b2,C9),(A38,b2,C10),(A38,b2,C11),(A38,b2,C12),( A38,b2,C13),(A38,b2,C14),(A38,b2,C15),(A38,b2,C16),(A38,b2,C17),(A38,b2,C18),(A38,b2, C19),(A38,b2,C20),(A38,b2,C21),(A38,b2,C22),(A38,b2,C23),(A38,b2,C24),(A38,b2,C25),(A 38,b2,C26),(A38,b2,C27),(A38,b2,C28),(A38,b2,C29),(A38,b3,C1),(A38,b3,C2),(A38,b3,C3),( A38,b3,C4),(A38,b3,C5),(A38,b3,C6),(A38,b3,C7),(A38,b3,C8),(A38,b3,C9),(A38,b3,C10),(A 38,b3,C11),(A38,b3,C12),(A38,b3,C13),(A38,b3,C14),(A38,b3,C15),(A38,b3,C16),(A38,b3,C 17),(A38,b3,C18),(A38,b3,C19),(A38,b3,C20),(A38,b3,C21),(A38,b3,C22),(A38,b3,C23),(A38 ,b3,C24),(A38,b3,C25),(A38,b3,C26),(A38,b3,C27),(A38,b3,C28),(A38,b3,C29),(A38,b4,C1),( A38,b4,C2),(A38,b4,C3),(A38,b4,C4),(A38,b4,C5),(A38,b4,C6),(A38,b4,C7),(A38,b4,C8),(A3 8,b4,C9),(A38,b4,C10),(A38,b4,C11),(A38,b4,C12),(A38,b4,C13),(A38,b4,C14),(A38,b4,C15) ,(A38,b4,C16),(A38,b4,C17),(A38,b4,C18),(A38,b4,C19),(A38,b4,C20),(A38,b4,C21),(A38,b4 ,C22),(A38,b4,C23),(A38,b4,C24),(A38,b4,C25),(A38,b4,C26),(A38,b4,C27),(A38,b4,C28),(A 38,b4,C29),(A38,b5,C1),(A38,b5,C2),(A38,b5,C3),(A38,b5,C4),(A38,b5,C5),(A38,b5,C6),(A3 8,b5,C7),(A38,b5,C8),(A38,b5,C9),(A38,b5,C10),(A38,b5,C11),(A38,b5,C12),(A38,b5,C13),( A38,b5,C14),(A38,b5,C15),(A38,b5,C16),(A38,b5,C17),(A38,b5,C18),(A38,b5,C19),(A38,b5, C20),(A38,b5,C21),(A38,b5,C22),(A38,b5,C23),(A38,b5,C24),(A38,b5,C25),(A38,b5,C26),(A 38,b5,C27),(A38,b5,C28),(A38,b5,C29),(A38,b6,C1),(A38,b6,C2),(A38,b6,C3),(A38,b6,C4),( A38,b6,C5),(A38,b6,C6),(A38,b6,C7),(A38,b6,C8),(A38,b6,C9),(A38,b6,C10),(A38,b6,C11),( A38,b6,C12),(A38,b6,C13),(A38,b6,C14),(A38,b6,C15),(A38,b6,C16),(A38,b6,C17),(A38,b6, C18),(A38,b6,C19),(A38,b6,C20),(A38,b6,C21),(A38,b6,C22),(A38,b6,C23),(A38,b6,C24),(A 38,b6,C25),(A38,b6,C26),(A38,b6,C27),(A38,b6,C28),(A38,b6,C29),(A38,b7,C1),(A38,b7,C2) ,(A38,b7,C3),(A38,b7,C4),(A38,b7,C5),(A38,b7,C6),(A38,b7,C7),(A38,b7,C8),(A38,b7,C9),(A 38,b7,C10),(A38,b7,C11),(A38,b7,C12),(A38,b7,C13),(A38,b7,C14),(A38,b7,C15),(A38,b7,C 16),(A38,b7,C17),(A38,b7,C18),(A38,b7,C19),(A38,b7,C20),(A38,b7,C21),(A38,b7,C22),(A38 ,b7,C23),(A38,b7,C24),(A38,b7,C25),(A38,b7,C26),(A38,b7,C27),(A38,b7,C28)(A38,b7,C29) ,(A39,b1,C1),(A39,b1,C2),(A39,b1,C3),(A39,b1,C4),(A39,b1,C5),(A39,b1,C6),(A39,b1,C7),(A 39,b1,C8),(A39,b1,C9),(A39,b1,C10),(A39,b1,C11),(A39,b1,C12),(A39,b1,C13),(A39,b1,C14) ,(A39,b1,C15),(A39,b1,C16),(A39,b1,C17),(A39,b1,C18),(A39,b1,C19),(A39,b1,C20),(A39,b1 ,C21),(A39,b1,C22),(A39,b1,C23),(A39,b1,C24),(A39,b1,C25),(A39,b1,C26),(A39,b1,C27),(A 39,b1,C28),(A39,b1,C29),(A39,b2,C1),(A39,b2,C2),(A39,b2,C3),(A39,b2,C4),(A39,b2,C5),(A 39,b2,C6),(A39,b2,C7),(A39,b2,C8),(A39,b2,C9),(A39,b2,C10),(A39,b2,C1 1),(A39,b2,C12),( A39,b2,C13),(A39,b2,C14),(A39,b2,C15),(A39,b2,C16),(A39,b2,C17),(A39,b2,C18),(A39,b2, C19),(A39,b2,C20),(A39,b2,C21),(A39,b2,C22),(A39,b2,C23),(A39,b2,C24),(A39,b2,C25),(A 39,b2,C26),(A39,b2,C27),(A39,b2,C28),(A39,b2,C29),(A39,b3,C1),(A39,b3,C2),(A39,b3,C3),( A39,b3,C4),(A39,b3,C5),(A39,b3,C6),(A39,b3,C7),(A39,b3,C8),(A39,b3,C9),(A39,b3,C10),(A 39,b3,C11),(A39,b3,C12),(A39,b3,C13),(A39,b3,C14),(A39,b3,C15),(A39,b3,C16),(A39,b3,C 17),(A39,b3,C18),(A39,b3,C19),(A39,b3,C20),(A39,b3,C21),(A39,b3,C22),(A39,b3,C23),(A39 ,b3,C24),(A39,b3,C25),(A39,b3,C26),(A39,b3,C27),(A39,b3,C28),(A39,b3,C29),(A39,b4,C1),( A39,b4,C2),(A39,b4,C3),(A39,b4,C4),(A39,b4,C5),(A39,b4,C6),(A39,b4,C7),(A39,b4,C8),(A3 9,b4,C9),(A39,b4,C10),(A39,b4,C21),(A39,b4,C12),(A39,b4,C13),(A39,b4,C14),(A39,b4,C15) ,(A39,b4,C16),(A39,b4,C17),(439,b4,C18),(A39,b4,C19),(A39,b4,C20),(A39,b4,C21),(A39,b4 ,C22),(A39,b4,C23),(A39,b4,C24),(A39,b4,C25),(A39,b4,C26),(A39,b4,C27),(A39,b4,C28),(A 39,b4,C29),(A39,b5,C1),(A39,b5,C2),(A39,b5,C3),(A39,b5,C4),(A39,b5,C5),(A39,b5,C6),(A3 9,b5,C7),(A39,b5,C8),(A39,b5,C9),(A39,b5,C10),(A39,b5,C11),(A39,b5,C12),(A39,b5,C13),( A39,b5,C14),(A39,b5,C15),(A39,b5,C16),(A39,b5,C17),(A39,b5,C18),(A39,b5,C19),(A39,b5, C20),(A39,b5,C21),(A39,b5,C22),(A39,b5,C23),(A39,b5,C24),(A39,b5,C25),(A39,b5,C26),(A 39,b5,C27),(A39,b5,C28),(A39,b5,C29),(A39,b6,C1),(A39,b6,C2),(A39,b6,C3),(A39,b6,C4),( A39,b6,C5),(A39,b6,C6),(A39,b6,C7),(A39,b6,C8),(A39,b6,C9),(A39,b6,C10),(A39,b6,C11),( A39,b6,C12),(A39,b6,C13),(A39,b6,C14),(A39,b6,C15),(A39,b6,C16),(A39,b6,C17),(A39,b6, C18),(A39,b6,C19),(A39,b6,C20),(A39,b6,C21),(A39,b6,C22),(A39,b6,C23),(A39,b6,C24),(A 39,b6,C25),(A39,b6,C26),(A39,b6,C27),(A39,b6,C28),(A39,b6,C29),(A39,b7,C1),(A39,b7,C2) ,(A39,b7,C3),(A39,b7,C4),(A39,b7,C5),(A39,b7,C6),(A39,b7,C7),(A39,b7,C8),(A39,b7,C9),(A 39,b7,C10),(A39,b7,C11),(A39,b7,C12),(A39,b7,C13),(A39,b7,C14),(A39,b7,C15),(A39,b7,C 16),(A39,b7,C17),(A39,b7,C18),(A39,b7,C19),(A39,b7,C20),(A39,b7,C21),(A39,b7,C22),(A39 ,b7,C23),(A39,b7,C24),(A39,b7,C25),(A39,b7,C26),(A39,b7,C27),(A39,b7,C28),(A39,b7,C29) ,(A40,b1,C1),(A40,b1,C2),(A40,b1,C3),(A40,b1,C4),(A40,b1,C5),(A40,b1,C6),(A40,b1,C7),(A 40,b1,C8),(A40,b1,C9),(A40,b1,C10),(A40,b1,C11),(A40,b1,C12),(A40,b1,C13),(A40,b1,C14) ,(A40,b1,C15),(A40,b1,C16),(A40,b1,C17),(A40,b1,C18),(A40,b1,C19),(A40,b1,C20),(A40,b1 ,C21),(A40,b1,C22),(A40,b1,C23),(A40,b1,C24),(A40,b1,C25),(A40,b1,C26),(A40,b1,C27),(A 40,b1,C28),(A40,b1,C29),(A40,b2,C1),(A40,b2,C2),(A40,b2,C3),(A40,b2,C4),(A40,b2,C5),(A 40,b2,C6),(A40,b2,C7),(A40,b2,C8),(A40,b2,C9),(A40,b2,C10),(A40,b2,C11),(A40,b2,C12),( A40,b2,C13),(A40,b2,C14),(A40,b2,C15),(A40,b2,C16),(A40,b2,C17),(A40,b2,C18),(A40,b2, C19),(A40,b2,C20),(A40,b2,C21),(A40,b2,C22),(A40,b2,C23),(A40,b2,C24),(A40,b2,C25),(A 40,b2,C26),(A40,b2,C27),(A40,b2,C28),(A40,b2,C29),(A40,b3,C1),(A40,b3,C2),(A40,b3,C3),( A40,b3,C4),(A40,b3,C5),(A40,b3,C6),(A40,b3,C7),(A40,b3,C8),(A40,b3,C9),(A40,b3,C10),(A 40,b3,C11),(A40,b3,C12),(A40,b3,C13),(A40,b3,C14),(A40,b3,C15),(A40,b3,C16),(A40,b3,C 17),(A40,b3,C18),(A40,b3,C19),(A40,b3,C20),(A40,b3,C21),(A40,b3,C22),(A40,b3,C23),(A40 ,b3,C24),(A40,b3,C25),(A40,b3,C26),(A40,b3,C27),(A40,b3,C28),(A40,b3,C29),(A40,b4,C1),( A40,b4,C2),(A40,b4,C3),(A40,b4,C4),(A40,b4,C5),(A40,b4,C6),(A40,b4,C7),(A40,b4,C8),(A4 0,b4,C9),(A40,b4,C10),(A40,b4,C11),(A40,b4,C12),(A40,b4,C13),(A40,b4,C4),(A40,b4,C15) ,(A40,b4,C16),(A40,b4,C 17),(A40,b4,C18),(A40,b4,C19),(A40,b4,C20),(A40,b4,C21),(A40,b4 ,C22),(A40,b4,C23),(A40,b4,C24),(A40,b4,C25),(A40,b4,C26),(A40,b4,C27),(A40,b4,C28),(A 40,b4,C29),(A40,b5,C1),(A40,b5,C2),(A40,b5,C3),(A40,b5,C4),(A40,b5,C5),(A40,b5,C6),(A4 O,b5,C7),(A40,b5,C8),(A40,b5,C9),(A40,b5,C10),(A40,b5,C 11),(A40,b5,C12),(A40,b5,C 13),( ( A40,b5,C14),(A40,b5,C15),(A40,b5,C16),(A40,b5,C17),(A40,b5,C18),(A40,b5,C19),(A40,b5, C20),(A40,b5,C21),(A40,b5,C22),(A40,b5,C23),(A40,b5,C24),(A40,b5,C25),(A40,b5,C26),(A 40,b5,C27),(A40,b5,C28),(A40,b5,C29),(A40,b6,C1),(A40,b6,C2),(A40,b6,C3),(A40,b6,C4),( A40,b6,C5),(A40,b6,C6),(A40,b6,C7),(A40,b6,C8),(A40,b6,C9),(A40,b6,C10),(A40,b6,C11),( A40,b6,C12),(A40,b6,C13),(A40,b6,C14),(A40,b6,C15),(A40,b6,C16),(A40,b6,C17),(A40,b6, C18),(A40,b6,C19),(A40,b6,C20),(A40,b6,C21),(A40,b6,C22),(A40,b6,C23),(A40,b6,C24),(A 40,b6,C25),(A40,b6,C26),(A40,b6,C27),(A40,b6,C28),(A40,b6,C29),(A40,b7,C1),(A40,b7,C2) ,(A40,b7,C3),(A40,b7,C4),(A40,b7,C5),(A40,b7,C6),(A40,b7,C7),(A40,b7,C8),(A40,b7,C9),(A 40,b7,C10),(A40,b7,C11),(A40,b7,C12),(A40,b7,C13),(A40,b7,C14),(A40,b7,C15),(A40,b7,C 16),(A40,b7,C17),(A40,b7,C18),(A40,b7,C19),(A40,b7,C20),(A40,b7,C21),(A40,b7,C22),(A40 ,b7,C23),(A40,b7,C24),(A40,b7,C25),(A40,b7,C26),(A40,b7,C27),(A40,b7,C28),(A40,b7,C29),

(A41,b1,C1),(A41,b1,C2),(A41,b1,C3),(A41,b1,C4),(A41,b1,C5),(A41,b1,C6),(A41,b1,C7),(A 41,b1,C8),(A41,b1,C9),(A41,b1,C10),(A41,b1,C11),(A41,b1,C12),(A41,b1,C13),(A41,b1,C14) ,(A41,b1,C15),(A41,b1,C16),(A41,b1,C17),(A41,b1,C18),(A41,b1,C19),(A41,b1,C20),(A41,b1 ,C21),(A41,b1,C22),(A41,b1,C23),(A41,b1,C24),(A41,b1,C25),(A41,b1,C26),(A41,b1,C27),(A 41,b1,C28),(A41,b1,C29),(A41,b2,C1),(A41,b2,C2),(A41,b2,C3),(A41,b2,C4),(A41,b2,C5),(A 41,b2,C6),(A41,b2,C7),(A41,b2,C8),(A41,b2,C9),(A41,b2,C10),(A41,b2,C11),(A41,b2,C12),( A41,b2,C13),(A41,b2,C14),(A41,b2,C15),(A41;b2,C16),(A41,b2,C17),(A41,b2,C18),(A41,b2, C19),(A41,b2,C20),(A41,b2,C21),(A41,b2,C22),(A41,b2,C23),(A41,b2,C24),(A41,b2,C25),(A 41,b2,C26),(A41,b2,C27),(A41,b2,C28),(A41,b2,C29),(A41,b3,C1),(A41,b3,C2),(A41,b3,C3),( A41,b3,C4),(A41,b3,C5),(A41,b3,C6),(A41,b3,C7),(A41,b3,C8),(A41,b3,C9),(A41,b3,C10),(A 41,b3,C11),(A41,b3,C12),(A41,b3,C13),(A41,b3,C14),(A41,b3,C15),(A41,b3,C16),(A41,b3,C 17),(A41,b3,C18),(A41,b3,C19),(A41,b3,C20),(A41,b3,C21),(A41,b3,C22),(A41,b3,C23),(A41 ,b3,C24),(A41,b3,C25),(A41,b3,C26),(A41,b3,C27),(A41,b3,C28),(A41,b3,C29),(A41,b4,C1),( A41,b4,C2),(A41,b4,C3),(A41,b4,C4),(A41,b4,C5),(A41,b4,C6),(A41,b4,C7),(A41,b4,C8),(A4 1,b4,C9),(A41,b4,C10),(A41,b4,C11),(A41,b4,C12),(A41,b4,C13),(A41,b4,C14),(A41,b4,C15) ,(A41,b4,C16),(A41,b4,C17),(A41,b4,C18),(A41,b4,C19),(A41,b4,C20),(A41,b4,C21),(A41,b4 ,C22),(A41,b4,C23),(A41,b4,C24),(A41,b4,C25),{A41,b4,C26),(A41,b4,C27),(A41,b4,C28),(A 41,b4,C29),(A41,b5,C1),(A41,b5,C2),(A41,b5,C3),(A41,b5,C4),(A41,b5,C5),(A41,b5,C6),(A4 1,b5,C7),(A41,b5,C8),(A41,b5,C9),(A41,b5,C10),(A41,b5,C11),(A41,b5,C12),(A41,b5,C13),( A41,b5,C14),(A41,b5,C15),(A41,b5,C16),(A41,b5,C17),(A41,b5,C18),(A41,b5,C19),(A41,b5, C20),(A41,b5,C21),(A41,b5,C22),(A41,b5,C23),(A41,b5,C24),(A41,b5,C25),(A41,b5,C26),(A 41,b5,C27),(A41,b5,C28),(A41,b5,C29),(A41,b6,C1),(A41,b6,C2),(A41,b6,C3),(A41,b6,C4),( A41,b6,C5),(A41,b6,C6),(A41,b6,C7),(A41,b6,C8),(A41,b6,C9),(A41,b6,C10),(A41,b6,C11),( A41,b6,C12),(A141,b6,C13),(A41,b6,C14),(A41,b6,C15),(A41,b6,C16),(A41,b6,C17),(A41,b6, C18),(A41,b6,C19),(A41,b6,C20),(A41,b6,C21),(A41,b6,C22),(A41,b6,C23),(A41,b6,C24),(A 41,b6,C25),(A41,b6,C26),(A41,b6,C27),(A41,b6,C28),(A41,b6,C29),(A41,b7,C1),(A41,b7,C2) ,(A41,b7,C3),(A41,b7,C4),(A41,b7,C5),(A41,b7,C6),(A41,b7,C7),(A41,b7,C8),(A41,b7,C9),(A 41,b7,C10),(A41,b7,C11),(A41,b7,C12),(A41,b7,C13),(A41,b7,C14),(A41,b7,C15),(A41,b7,C 16),(A41,b7,C17),(A41,b7,C18),(A41,b7,C19),(A41,b7,C20),(A41,b7,C21),(A41,b7,C22),(A41 ,b7,C23),(A41,b7,C24),(A41,b7,C25),(A41,b7,C26),(A41,b7,C27),(A41,b7,C28),(A41,b7,C29) ,(A42,b1,C1),(A42,b1,C2),(A42,b1,C3),(A42,b1,C4),(A42,b1,C5),(A42,b1,C6),(A42,b1,C7),(A 42,b1,C8),(A42,b1,C9),(A42,b1,C10),(A42,b1,C11),(A42,b1,C12),(A42,b1,C13),(A42,b1,C14) ,(A42,b1,C15),(A42,b1,C16),(A42,b1,C17),(A42,b1,C18),(A42,b1,C19),(A42,b1,C20),(A42,b1 ,C21),(A42,b1,C22),(A42,b1,C23),(A42,b1,C24),(A42,b1,C25),(A42,b1,C26),(A42,b1,C27),(A 42,b1,C28),(A42,b1,C29),(A42,b2,C1),(A42,b2,C2),(A42,b2,C3),(A42,b2,C4),(A42,b2,C5),(A 42,b2,C6),(A42,b2,C7),(A42,b2,C8),(A42,b2,C9),(A42,b2,C10),(A42,b2,C11),(A42,b2,C12),( A42,b2,C13),(A42,b2,C14),(A42,b2,C15),(A42,b2,C16),(A42,b2,C17),(A42,b2,C18),(A42,b2, C19),(A42,b2,C20),(A42,b2,C21),(A42,b2,C22),(A42,b2,C23),(A42,b2,C24),(A42,b2,C25),(A 42,b2,C26),(A42,b2,C27),(A42,b2,C28),(A42,b2,C29),(A42,b3,C1),(A42,b3,C2),(A42,b3,C3),( A42,b3,C4),(A42,b3,C5),(A42,b3,C6),(A42,b3,C7),(A42,b3,C8),(A42,b3,C9),(A42,b3,C10),(A 42,b3,C11),(A42,b3,C12),(A42,b3,C13),(A42,b3,C14),(A42,b3,C15),(A42,b3,C16),(A42,b3,C 17),(A42,b3,C18),(A42,b3,C19),(A42,b3,C20),(A42,b3,C21),(A42,b3,C22),(A42,b3,C23),(A42 ,b3_{;}C24),(A42,b3,C25),(A42,b3,C26),(A42,b3,C27),(A42,b3,C28),(A42,b3,C29),(A42,b4,C1),( A42,b4,C2),(A42,b4,C3),(A42,b4,C4),(A42,b4,C5),(A42,b4,C6),(A42,b4,C7),(A42,b4,C8),(A4 2,b4,C9),(A42,b4,C10),(A42,b4,C11),(A42,b4,C12),(A42,b4,C13),(A42,b4,C14),(A42,b4,C15) ,(A42,b4,C16),(A42,b4,C17),(A42,b4,C18),(A42,b4,C19),(A42,b4,C20),(A42,b4,C21),(A42,b4 ,C22),(A42,b4,C23),(A42,b4,C24),(A42,b4,C25),(A42,b4,C26),(A42,b4,C27),(A42,b4,C28),(A 42,b4,C29),(A42,b5,C1),(A42,b5,C2),(A42,b5,C3),(A42,b5,C4),(A42,b5,C5),(A42,b5,C6),(A4 2,b5,C7),(A42,b5,C8),(A42,b5,C9),(A42,b5,C10),(A42,b5,C11),(A42,b5,C12),(A42,b5,C13),( A42,b5,C14),(A42,b5,C15),(A42,b5,C16),(A42,b5,C17),(A42,b5,C18),(A42,b5,C19),(A42,b5, C20),(A42,b5,C21),(A42,b5,C22),(A42,b5,C23),(A42,b5,C24),(A42,b5,C25),(A42,b5,C26),(A 42,b5,C27),(A42,b5,C28),(A42,b5,C29),(A42,b6,C1),(A42,b6,C2),(A42,b6,C3),(A42,b6,C4),( A42,b6,C5),(A42,b6,C6),(A42,b6,C7),(A42,b6,C8),(A42,b6,C9),(A42,b6,C10),(A42,b6,C11),( A42,b6,C12),(A42,b6,C13),(A42,b6,C14),(A42,b6,C15),(A42,b6,C16),(A42,b6,C17),(A42,b6, C18),(A42,b6,C19),(A42,b6,C20),(A42,b6,C21),(A42,b6,C22),(A42,b6,C23),(A42,b6,C24),(A 42,b6,C25),(A42,b6,C26),(A42,b6,C27),(A42,b6,C28),(A42,b6,C29),(A42,b7,C1),(A42,b7,C2) ,(A42,b7,C3),(A42,b7,C4),(A42,b7,C5),(A42,b7,C6),(A42,b7,C7),(A42,b7,C8),(A42,b7,C9),(A 42,b7,C10),(A42,b7,C11),(A42,b7,C12),(A42,b7,C13),(A42,b7,C14),(A42,b7,C15),(A42,b7,C 16),(A42,b7,C17),(A42,b7,C18),(A42,b7,C19),(A42,b7,C20),(A42,b7,C21),(A42,b7,C22),(A42 ,b7,C23),(A42,b7,C24),(A42,b7,C25),(A42,b7,C26),(A42,b7,C27),(A42,b7,C28),(A42,b7,C29) ,(A43,b1,C1),(A43,b1,C2),(A43,b1,C3),(A43,b1,C4),(A43,b1,C5),(A43,b1,C6),(A43,b1,C7),(A 43,b1,C8),(A43,b1,C9),(A43,b1,C10),(A43,b1,C11),(A43,b1,C12),(A43,b1,C13),(A43,b1,C14) (A43,b1,C15),(A43,b1,C16),(A43,b1,C17),(A43,b1,C18),(A43,b1,C19),(A43,b1,C20),(A43,b1 ,C21),(A43,b1,C22),(A43,b1,C23),(A43,b1,C24),(A43,b1,C25),(A43,b1,C26),(A43,b1,C27),(A 43,b1,C28),(A43,b1,C29),(A43,b2,C1),(A43,b2,C2),(A43,b2,C3),(A43,b2,C4),(A43,b2,C5),(A 43,b2,C6),(A43,b2,C7),(A43,b2,C8),(A43,b2,C9),(A43,b2,C10),(A43,b2,C11),(A43,b2,C12),( A43,b2,C13),(A43,b2,C14),(A43,b2,C15),(A43,b2,C16),(A43,b2,C17),(A43,b2,C18),(A43,b2, C19),(A43,b2,C20),(A43,b2,C21),(A43,b2,C22),(A43,b2,C23),(A43,b2,C24),(A43,b2,C25),(A 43,b2,C26),(A43,b2,C27),(A43,b2,C28),(A43,b2,C29),(A43,b3,C1),(A43,b3,C2),(A43,b3,C3),( A43,b3,C4),(A43,b3,C5),(A43,b3,C6),(A43,b3,C7),(A43,b3,C8),(A43,b3,C9),(A43,b3,C10),(A 43,b3,C11),(A43,b3,C12),(A43,b3,C13),(A43,b3,C14),(A43,b3,C15),(A43,b3,C16),(A43,b3,C 17),(A43,b3,C18),(A43,b3,C19),(A43,b3,C20),(A43,b3,C21),(A43,b3,C22),(A43,b3,C23),(A43 ,b3,C24),(A43,b3,C25),(A43,b3,C26),(A43,b3,C27),(A43,b3,C28),(A43,b3,C29),(A43,b4,C1),( A43,b4,C2),(A43,b4,C3),(A43,b4,C4),(A43,b4,C5),(A43,b4,C6),(A43,b4,C7),(A43,b4,C8),(A4 3,b4,C9),(A43,b4,C10),(A43,b4,C11),(A43,b4,C12),(A43,b4,C13),(A43,b4,C14),(A43,b4,C15) ,(A43,b4,C16),(A43,b4,C17),(A43,b4,C18),(A43,b4,C19),(A43,b4,C20),(A43,b4,C21),(A43,b4 ,C22),(A43,b4,C23),(A43,b4,C24),(A43,b4,C25),(A43,b4,C26),(A43,b4,C27),(A43,b4,C28),(A 43,b4,C29),(A43,b5,C1),(A43,b5,C2),(A43,b5,C3),(A43,b5,C4),(A43,b5,C5),(A43,b5,C6),(A4 3,b5,C7),(A43,b5,C8),(A43,b5,C9),(A43,b5,C10),(A43,b5,C11),(A43,b5,C12),(A43,b5,C13), A43,b5,C14),(A43,b5,C15),(A43,b5,C16),(A43,b5,C17),(A43,b5,C18),(A43,b5,C19),(A43,b5, C20),(A43,b5,C21),(A43,b5,C22),(A43,b5,C23),(A43,b5,C24),(A43,b5,C25),(A43,b5,C26),(A 43,b5,C27),(A43,b5,C28),(A43,b5,C29),(A43,b6,C1),(A43,b6,C2),(A43,b6,C3),(A43,b6,C4),( A43,b6,C5),(A43,b6,C6),(A43,b6,C7),(A43,b6,C8),(A43,b6,C9),(A43,b6,C10),(A43,b6,C11),( A43,b6,C12),(A43,b6,C13),(A43,b6,C14),(A43,b6,C15),(A43,b6,C16),(A43,b6,C7),(A43,b6, C18),(A43,b6,C19),(A43,b6,C20),(A43,b6,C21),(A43,b6,C22),(A43,b6,C23),(A43,b6,C24),(A 43,b6,C25),(A43,b6,C26),(A43,b6,C27),(A43,b6,C28),(A43,b6,C29),(A43,b7,C1),(A43,b7,C2) ,(A43,b7,C3),(A43,b7,C4),(A43,b7,C5),(A43,b7,C6),(A43,b7,C7),(A43,b7,C8),(A43,b7,C9),(A 43,b7,C10),(A43,b7,C11),(A43,b7,C12),(A43,b7,C13),(A43,b7,C14),(A43,b7,C15),(A43,b7,C 16),(A43,b7,C17),(A43,b7,C18),(A43,b7,C19),(A43,b7,C20),(A43,b7,C21),(A43,b7,C22),(A43 ,b7,C23),(A43,b7,C24),(A43,b7,C25),(A43,b7,C26),(A43,b7,C27),(A43,b7,C28),(A43,b7,C29) ,(A44,b1,C1),(A44,b1,C2),(A44,b1,C3),(A44,b1,C4),(A44,b1,C5),(A44,b1,C6),(A44,b1,C7),(A 44,b1,C8),(A44,b1,C9),(A44,b1,C10),(A44,b1,C11),(A44,b1,C12),(A44,b1,C13),(A44,b1,C14) ,(A44,b1,C15),(A44,b1,C16),(A44,b1,C17),(A44,b1,C18),(A44,b1,C19),(A44,b1,C20),(A44,b1 ,C21),(A44,b1,C22),(A44,b1,C23),(A44,b1,C24),(A44,b1,C25),(A44,b1,C26),(A44,b1,C27),(A 44,b1,C28),(A44,b1,C29),(A44,b2,C1),(A44,b2,C2),(A44_{;}b2,C3),(A44,b2,C4),(A44,b2,C5),(A 44,b2,C6),(A44,b2,C7),(A44,b2,C8),(A44,b2,C9),(A44,b2,C10),(A44,b2,C11),(A44,b2,C12),( A44,b2,C13),(A44,b2,C14),(A44,b2,C15),(A44,b2,C16),(A44,b2,C17),(A44,b2,C18),(A44,b2, C19),(A44,b2,C20),(A44,b2,C21),(A44,b2,C22),(A144,b2,C23),(A44,b2,C24),(A44,b2,C25),(A 44,b2,C26),(A44,b2,C27),(A44,b2,C28),(A44,b2,C29),(A44,b3,C1),(A44,b3,C2),(A44,b3,C3),( A44,b3,C4),(A44,b3,C5),(A44,b3,C6),(A44,b3,C7),(A44,b3,C8),(A44,b3,C9),(A44,b3,C10),(A 44,b3,C11),(A44,b3,C12),(A44,b3,C13),(A44,b3,C14),(A44,b3,C15),(A44,b3,C16),(A44,b3,C 17),(A44,b3,C18),(A44,b3,C19),(A44,b3,C20),(A44,b3,C21),(A44,b3,C22),(A44,b3,C23),(A44 ,b3,C24),(A44,b3,C25),(A44,b3,C26),(A44,b3,C27),(A44,b3,C28),(A44,b3,C29),(A44,b4,C1),( A44,b4,C2),(A44,b4,C3),(A44,b4,C4),(A44,b4,C5),(A44,b4,C6),(A44,b4,C7),(A44,b4,C8),(A4 4,b4,C9),(A44,b4,C10),(A44,b4,C11),(A44,b4,C12),(A44,b4,C13),(A44,b4,C14),(A44,b4,C15) ,(A44,b4,C16),(A44,b4,C17),(A44,b4,C18),(A44,b4,C19),(A44,b4,C20),(A44,b4,C21),(A44,b4 ,C22),(A44,b4,C23),(A44,b4,C24),(A44,b4,C25),(A44,b4,C26),(A44,b4,C27),(A44,b4,C28),(A 44,b4,C29),(A44,b5,C1),(A44,b5,C2),(A44,b5,C3),(A44,b5,C4),(A44,b5,C5),(A44,b5,C6),(A4 4,b5,C7),(A44,b5,C8),(A44,b5,C9),(A44,b5,C10),(A44,b5,C11),(A44,b5,C12),(A44,b5,C13),( A44,b5,C14),(A44,b5,C15),(A44,b5,C16),(A44,b5,C17),(A44,b5,C18),(A44,b5,C19),(A44,b5, C20),(A44,b5,C21),(A44,b5,C22),(A44,b5,C23),(A44,b5,C24),(A44,b5,C25),(A44,b5,C26),(A 44,b5,C27),(A44,b5,C28),(A44,b5,C29),(A44,b6,C1),(A44,b6,C2),(A44,b6,C3),(A44,b6,C4),( A44,b6,C5),(A44,b6,C6),(A44,b6,C7),(A44,b6,C8),(A44,b6,C9),(A44,b6,C10),(A44,b6,C11),( A44,b6,C12),(A44,b6,C13),(A44,b6,C14),(A44,b6,C15),(A44,b6,C16),(A44,b6,C17)(A44,b6, C18),(A44,b6,C19),(A44,b6,C20),(A44,b6,C21),(A44,b6,C22),(A44,b6,C23),(A44,b6,C24),(A 44,b6,C25),(A44,b6,C26),(A44,b6,C27),(A44,b6,C28),(A44,b6,C29),(A44,b7,C1)(A44,b7,C2) ,(A44,b7,C3),(A44,b7,C4),(A44,b7,C5),(A44,b7,C6),(A44,b7,C7),(A44,b7,C8),(A44,b7,C9),(A 44,b7,C10),(A44,b7,C11),(A44,b7,C12),(A44,b7,C13),(A44,b7,C14),(A44,b7,C15),(A44,b7,C 16),(A44,b7,C17),(A44,b7,C18),(A44,b7,C19),(A44,b7,C20),(A44,b7,C21),(A44,b7,C22),(A44 ,b7,C23),(A44,b7,C24),(A44,b7,C25),(A44,b7,C26),(A44,b7,C27),(A44,b7,C28),(A44,b7,C29) ,(A45,b1,C1),(A45,b1,C2),(A45,b1,C3),(A45,b1,C4),(A45,b1,C5),(A45,b1,C6),(A45,b1,C7),(A 45,b1,C8),(A45,b1,C9),(A45,b1,C10),(A45,b1,C11),(A45,b1,C12),(A45,b1,C13),(A45,b1,C14) ,(A45,b1C15),(A45,b1C16),(A45,b1,C17),(A45,b1,C18),(A45,b1,C19),(A45,b1,C20),(A45,b1 ,C21),(A45,b1,C22),(A45,b1,C23),(A45,b1,C24),(A45,b1,C25),(A45,b1,C26),(A45,bT,C27),(A 45,b1,C28),(A45,b1,C29),(A45,b2,C1),(A45,b2,C2),(A45,b2,C3),(A45,b2,C4),(A45,b2,C5),(A 45,b2,C6),(A45,b2,C7),(A45,b2,C8),(A45,b2,C9),(A45,b2,C10),(A45,b2,C11),(A45,b2,C12),( A45,b2,C13),(A45,b2,C14),(A45,b2,C15),(A45,b2,C16),(A45,b2,C17),(A45,b2,C18),(A45,b2, C19),(A45,b2,C20),(A45,b2,C21),(A45,b2,C22),(A45,b2,C23),(A45,b2,C24),(A45,b2,C25),(A 45,b2,C26),(A45,b2,C27),(A45,b2,C28),(A45,b2,C29),(A45,b3,C1),(A45,b3,C2),(A45,b3,C3),( A45,b3,C4),(A45,b3,C5),(A45,b3,C6),(A45,b3,C7),(A45,b3,C8),(A45,b3,C9),(A45,b3,C10),(A 45,b3,C11),(A45,b3,C12),(A45,b3,C13),(A45,b3,C14),(A45,b3,C15),(A45,b3,C16),(A45,b3,C 17),(A45,b3,C18),(A45,b3,C19),(A45,b3,C20),(A45,b3,C21),(A45,b3,C22),(A45,b3,C23),(A45 ,b3,C24),(A45,b3,C25),(A45,b3,C26),(A45,b3,C27),(A45,b3,C28),(A45,b3,C29),(A45,b4,C1),( A45,b4,C2),(A45,b4,C3),(A45,b4,C4),(A45,b4,C5),(A45,b4,C6),(A45,b4,C7),(A45,b4,C8),(A4 5,b4,C9),(A45,b4,C10),(A45,b4,C11),(A45,b4,C12),(A45,b4,C13),(A45,b4,C14),(A45,b4,C15) ,(A45,b4,C16),(A45,b4,C17),(A45,b4,C18),(A45,b4,C19),(A45,b4,C20),(A45,b4,C21),(A45,b4 ,C22),(A45,b4,C23),(A45,b4,C24),(A45,b4,C25),(A45,b4,C26),(A45,b4,C27),(A45,b4,C28),(A 45,b4,C29),(A45,b5,C1),(A45,b5,C2),(A45,b5,C3),(A45,b5,C4),(A45,b5,C5),(A45,b5,C6),(A4 5,b5,C7),(A45,b5,C8),(A45,b5,C9),(A45,b5,C10),(A45,b5,C11),(A45,b5,C12),(A45,b5,C13),( A45,b5,C14),(A45,b5,C15),(A45,b5,C16),(A45,b5,C17),(A45,b5,C18),(A45,b5,C19),(A45,b5, C20),(A45,b5,C21),(A45,b5,C22),(A45,b5,C23),(A45,b5,C24),(A45,b5,C25),(A45,b5;C26),(A 45,b5,C27),(A45,b5,C28),(A45,b5,C29),(A45,b6,C1),(A45,b6,C2),(A45,b6,C3),(A45,b6,C4),( A45,b6,C5),(A45,b6,C6),(A45,b6,C7),(A45,b6,C8),(A45,b6,C9),(A45,b6,C10),(A45,b6,C11),( A45,b6,C12),(A45,b6,C13),(A45,b6,C14),(A45,b6,C15),(A45,b6,C16),(A45,b6,C17),(A45,b6, C18),(A45,b6,C19),(A45,b6,C20),(A45,b6,C21),(A45,b6,C22),(A45,b6,C23),(A45,b6,C24),(A 45,b6,C25),(A45,b6,C26),(A45,b6,C27),(A45,b6,C28),(A45,b6,C29),(A45,b7,C1),(A45,b7,C2) ,(A45,b7,C3),(A45,b7,C4),(A45,b7,C5),(A45,b7,C6),(A45,b7,C7),(A45,b7,C8),(A45,b7,C9),(A 45,b7,C10),(A45,b7,C11),(A45,b7,C12),(A45,b7,C13),(A45,b7,C14),(A45,b7,C15),(A45,b7,C 16),(A45,b7,C17),(A45,b7,C18),(A45,b7,C19),(A45,b7,C20),(A45,b7,C21),(A45,b7,C22),(A45 ,b7,C23),(A45,b7,C24),(A45,b7,C25),(A45,b7,C26),(A45,b7,C27),(A45,b7,C28),(A45,b7,C29) ,(A46,b1,C1),(A46,b1,C2),(A46,b1,C3),(A46,b1,C4),(A46,b1,C5),(A46,b1,C6),(A46,b1,C7),(A 46,b1,C8),(A46,b1,C9),(A46,b1,C10),(A46,b1,C11),(A46,b1,C12),(A46,b1,C13),(A46,b1,C14) ,(A46,b1,C15),(A46,b1,C16),(A46,b1,C17),(A46,b1,C18),(A46,b1,C19),(A46,b1,C20),(A46,b1 ,C21),(A46,b1,C22),(A46,b1,C23),(A46,b1,C24),(A46,b1,C25),(A46,b1,C26),(A46,b1,C27),(A 46,b1,C28),(A46,b1,C29),(A46,b2,C1),(A46,b2,C2),(A46,b2,C3),(A46,b2,C4),(A46,b2,C5),(A 46,b2,C6),(A46,b2,C7),(A46,b2,C8),(A46,b2,C9),(A46,b2,C10),(A46,b2,C11),(A46,b2,C12),( A46,b2,C13),(A46,b2,C14),(A46,b2,C15),(A46,b2,C16),(A46,b2,C17),(A46,b2,C18),(A46,b2, C19),(A46,b2,C20),(A46,b2,C21),(A46,b2,C22),(A46,b2,C23),(A46,b2,C24),(A46,b2,C25),(A 46,b2,C26),(A46,b2,C27),(A46,b2,C28),(A46,b2,C29),(A46,b3,C1),(A46,b3,C2),(A46,b3,C3),( A46,b3,C4),(A46,b3,C5),(A46,b3,C6),(A46,b3,C77),(A46,b3,C8),(A46,b3,C9),(A46,b3,C10),(A 46,b3,C11),(A46,b3,C12),(A46,b3,C13),(A46,b3,C14),(A46,b3,C15),(A46,b3,C16),(A46,b3,C 17),(A46,b3,C18),(A46,b3,C19),(A46,b3,C20),(A46,b3,C21),(A46,b3,C22),(A46,b3,C23),(A46 ,b3,C24),(A46,b3,C25),(A46,b3,C26),(A46,b3,C27),(A46,b3,C28),(A46,b3,C29),(A46,b4,C1),( A46,b4,C2),(A46,b4,C3),(A46,b4,C4),(A46,b4,C5),(A46,b4,C6),(A46,b4,C7),(A46,b4,C8),(A4 6,b4,C9),(A46,b4,C10),(A46,b4,C11),(A46,b4,C12),(A46,b4,C13),(A46,b4,C14),(A46,b4,C15) ,(A46,b4,C16),(A46,b4,C17),(A46,b4,C18),(A46,b4,C19),(A46,b4,C20),(A46,b4,C21),(A46,b4 ,C22),(A46,b4,C23),(A46,b4,C24),(A46,b4,C25),(A46,b4,C26),(A46,b4,C27),(A46,b4,C28),(A 46,b4,C29),(A46,b5,C1),(A46,b5,C2),(A46,b5,C3),(A46,b5,C4),(A46,b5,C5),(A46,b5,C6),(A4 6,b5,C7),(A46,b5,C8),(A46,b5,C9),(A46,b5,C10),(A46,b5,C11),(A46,b5,C12),(A46,b5,C13),(( A46,b5,C14),(A46,b5,C15),(A46,b5,C16),(A46,b5,C17),(A46,b5,C18),(A46,b5,C19),(A46,b5, C20),(A46,b5,C21),(A46,b5,C22),(A46,b5,C23),(A46,b5,C24),(A46,b5,C25),(A46,b5,C26),(A 46,b5,C27),(A46,b5,C28),(A46,b5,C29),(A46,b6,C1),(A46,b6,C2),(A46,b6,C3),(A46,b6,C4),( A46,b6,C5),(A46,b6,C6),(A46,b6,C7),(A46,b6,C8),(A46,b6,C9),(A46,b6,C10),(A46,b6,C11),( A46,b6,C12),(A46,b6,C13),(A46,b6,C14),(A46,b6,C15),(A46,b6,C16),(A46,b6,C17),(A46,b6, C18),(A46,b6,C19),(A46,b6,C20),(A46,b6,C21),(A46,b6,C22),(A46,b6,C23),(A46,b6,C24),(A 46,b6,C25),(A46,b6,C26),(A46,b6,C27),(A46,b6,C28),(A46,b6,C29),(A46,b7,C1),(A46,b7,C2) ,(A46,b7,C3),(A46,b7,C4),(A46,b7,C5),(A46,b7,C6),(A46,b7,C7),(A46,b7,C8),(A46,b7,C9),(A 46,b7,C10),(A46,b7,C11),(A46,b7,C12),(A46,b7,C13),(A46,b7,C14),(A46,b7,C15),(A46,b7,C 16),(A46,b7,C17),(A46,b7,C18),(A46,b7,C19),(A46,b7,C20),(A46,b7,C21),(A46,b7,C22),(A46 ,b7,C23),(A46,b7,C24),(A46,b7,C25),(A46,b7,C26),(A46,b7,C27),(A46,b7,C28),(A46,b7,C29) ,(A47,b1,C1),(A47,b1,C2),(A47,b1,C3),(A47,b1,C4),(A47,b1,C5),(A47,b1,C6),(A47,b1,C7),(A 47,b1,C8),(A47,b1,C9),(A47,b1,C10),(A47,b1,C11),(A47,b1,C12),(A47,b1,C13),(A47,b1,C14) ,(A47,b1,C15),(A47,b1,C16),(A47,b1,C17),(A47,b1,C18),(A47,b1,C19),(A47,b1,C20),(A47,b1 ,C21),(A47,b1,C22),(A47,b1,C23),(A47,b1,C24),(A47,b1,C25),(A47,b1,C26),(A47,b1,C27),(A 47,b1,C28),(A47,b1,C29),(A47,b2,C1),(A47,b2,C2),(A47,b2,C3),(A47,b2,C4),(A47,b2,C5),(A 47,b2,C6),(A47,b2,C7),(A47,b2,C8),(A47,b2,C9),(A47,b2,C10),(A47,b2,C11),(A47,b2,C12),( A47,b2,C13),(A47,b2,C14),(A47,b2,C15),(A47,b2,C16),(A47,b2,C17),(A47,b2,C18),(A47,b2, C19),(A47,b2,C20),(A47,b2,C21),(A47,b2,C22),(A47,b2,C23),(A47,b2,C24),(A47,b2,C25),(A 47,b2,C26),(A47,b2,C27),(A47,b2,C28),(A47,b2,C29),(A47,b3,C1),(A47,b3,C2),(A47,b3,C3),( A47,b3,C4),(A47,b3,C5),(A47,b3,C6),(A47,b3,C7),(A47,b3,C8),(A47,b3,C9),(A47,b3,C10),(A 47,b3,C11),(A47,b3,C12),(A47,b3,C13),(A47,b3,C14),(A47,b3,C15),(A47,b3,C16),(A47,b3,C 17),(A47,b3,C18),(A47,b3,C19),(A47,b3,C20),(A47,b3,C21),(A47,b3,C22),(A47,b3,C23),(A47 ,b3,C24),(A47,b3,C25),(A47,b3,C26),(A47,b3,C27),(A47,b3,C28),(A47,b3,C29),(A47,b4,C1),( A47,b4,C2),(A47,b4,C3),(A47,b4,C4),(A47,b4,C5),(A47,b4,C6),(A47,b4,C7),(A47,b4,C8),(44 7,b4,C9),(A47,b4,C10),(A47,b4,C11),(A47,b4,C12),(A47,b4,C13),(A47,b4,C14),(A47,b4,C15) ,(A47,b4,C16),(A47,b4,C17),(A47,b4,C18),(A47,b4,C19),(A47,b4,C20),(A47,b4,C21),(A47,b4 ,C22),(A47,b4,C23),(A47,b4,C24),(A47,b4,C25),(A47,b4,C26),(A47,b4,C27),(A47,b4,C28),(A 47,b4,C29),(A47,b5,C1),(A47,b5,C2),(A47,b5,C3),(A47,b5,C4),(A47,b5,C5),(A47,b5,C6),(A4 7,b5,C7),(A47,b5,C8),(A47,b5,C9),(A47,b5,C10),(A47,b5,C11),(A47,b5,C12),(A47,b5,C13), A47,b5,C14),(A47,b5,C15),(A47,b5,C16),(A47,b5,C17),(A47,b5,C18),(A47,b5,C19),(A47,b5, C20),(A47,b5,C21),(A47,b5,C22),(A47,b5,C23),(A47,b5,C24),(A47,b5,C25),(A47,b5,C26),(A 47,b5,C27),(A47,b5,C28),(A47,b5,C29),(A47,b6,C1),(A47,b6,C2),(A47,b6,C3),(A47,b6,C4),( A47,b6,C5),(A47,b6,C6),(A47,b6,C7),(A47,b6,C8),(A47,b6,C9),(A47,b6,C10),(A47,b6,C11), A47,b6,C12),(A47,b6,C13),(A47,b6,C14),(A47,b6,C15),(A47,b6,C16),(A47,b6,C17),(A47,b6, C18),(A47,b6,C19),(A47,b6,C20),(A47,b6,C21),(A47,b6,C22),(A47,b6,C23),(A47,b6,C24),(A 47,b6,C25),(A47,b6,C26),(A47,b6,C27),(A47,b6,C28),(A47,b6,C29),(A47,b7,C1),(A47,b7,C2) ,(A47,b7,C3),(A47,b7,C4),(A47,b7,C5),(A47,b7,C6),(A47,b7,C7),(A47,b7,C8),(A47,b7,C9),(A 47,b7,C10),(A47,b7,C11),(A47,b7,C12),(A47,b7,C13),(A47,b7,C14),(A47,b7,C15),(A47,b7,C 16),(A47,b7,C17),(A47,b7,C18),(A47,b7,C19),(A47,b7,C20),(A47,b7,C21),(A47,b7,C22),(A47 ,b7,C23),(A47,b7,C24),(A47,b7,C25),(A47,b7,C26),(A47,b7,C27),(A47,b7,C28),(A47,b7,C29) ,(A48,b1,C1),(A48,b1,C2),(A48,b1,C3),(A48,b1,C4),(A48,b1,C5),(A48,b1,C6),(A48,b1,C7),(A 48,b1,C8),(A48,b1,C9),(A48,b1,C10),(A48,b1,C11),(A48,b1,C12),(A48,b1,C13),(A48,b1,C14) ,(A48,b1,C15),(A48,b1,C16),(A48,b1,C17),(A48,b1,C8),(A48,b1,C9),(A48,b1,C20),(A48,b1 ,C21),(A48,b1,C22),(A48,b1,C23),(A48,b1,C24),(A48,b1,C25),(A48,b1,C26),(A48,b1,C27),(A 48,b1,C28),(A48,b1,C29),(A48,b2,C1),(A48,b2,C2),(A48,b2,C3),(A48,b2,C4),(A48,b2,C5),(A 48,b2,C6),(A48,b2,C7),(A48,b2,C8),(A48,b2,C9),(A48,b2,C10),(A48,b2,C11),(A48,b2,C12),( A48,b2,C13),(A48,b2,C14),(A48,b2,C15),(A48,b2,C16),(A48,b2,C17),(A48,b2,C18),(A48,b2, C19),(A48,b2,C20),(A48,b2,C21),(A48,b2,C22),(A48,b2,C23),(A48,b2,C24),(A48,b2,C25),(A 48,b2,C26),(A48,b2,C27),(A48,b2,C28),(A48,b2,C29),(A48,b3,C1),(A48,b3,C2),(A48,b3,C3),( A48,b3,C4),(A48,b3,C5),(A48,b3,C6),(A48,b3,C7),(A48,b3,C8),(A48,b3,C9),(A48,b3,C10),(A 48,b3,C11),(A48,b3,C12),(A48,b3,C13),(A48,b3,C14),(A48,b3,C15),(A48,b3,C16),(A48,b3,C 17),(A48,b3,C18),(A48,b3,C19),(A48,b3,C20),(A48,b3,C21),(A48,b3,C22),(A48,b3,C23),(A48 ,b3,C24),(A48,b3,C25),(A48,b3,C26),(A48,b3,C27),(A48,b3,C28),(A48,b3,C29),(A48,b4,C1),( A48,b4,C2),(A48,b4,C3),(A48,b4,C4),(A48,b4,C5),(A48,b4,C6),(A48,b4,C7),(A48,b4,C8),(A4 8,b4,C9),(A48,b4,C10),(A48,b4,C11),(A48,b4,C12),(A48,b4,C13),(A48,b4,C14),(A48,b4,C15) ,(A48,b4,C16),(A48,b4,C17),(A48,b4,C18),(A48,b4,C19),(A48,b4,C20),(A48,b4,C21),(A48,b4 ,C22),(A48,b4,C23),(A48,b4,C24),(A48,b4,C25),(A48,b4,C26),(A48,b4,C27),(A48,b4,C28),(A 48,b4,C29),(A48,b5,C1),(A48,b5,C2),(A48,b5,C3),(A48,b5,C4),(A48,b5,C5),(A48,b5,C6),(A4 8,b5,C7),(A48,b5,C8),(A48,b5,C9),(A48,b5,C10),(A48,b5,C11),(A48,b5,C12),(A48,b5,C13), A48,b5,C14),(A48,b5,C15),(A48,b5,C16),(A48,b5,C17),(A48,b5,C18),(A48,b5,C19),(A48,b5, C20),(A48,b5,C21),(A48,b5,C22),(A48,b5,C23),(A48,b5,C24),(A48,b5,C25),(A48,b5,C26),(A 48,b5,C27),(A48,b5,C28),(A48,b5,C29),(A48,b6,C1),(A48,b6,C2),(A48,b6,C3),(A48,b6,C4), A48,b6,C5),(A48,b6,C6),(A48,b6,C7),(A48,b6,C8),(A48,b6,C9),(A48,b6,C10),(A48,b6,C11), A48,b6,C12),(A48,b6,C13),(A48,b6,C14),(A48_{;}b6,C15),(A48,b6,C16),(A48,b6,C17),(A48,b6, C18),(A48,b6,C19),(A48,b6,C20),(A48,b6,C21),(A48,b6,C22),(A48,b6,C23),(A48,b6,C24),(A 48,b6,C25),(A48,b6,C26),(A48,b6,C27),(A48,b6,C28),(A48,b6,C29),(A48,b7,C1),(A48,b7,C2) ,(A48,b7,C3),(A48,b7,C4),(A48,b7,C5),(A48,b7,C6),(A48,b7,C7),(A48,b7,C8),(A48,b7,C9),(A 48,b7,C10),(A48,b7,C11),(A48,b7,C12),(A48,b7,C13),(A48,b7,C14),(A48,b7,C15),(A48,b7,C 16),(A48,b7,C17),(A48,b7,C18),(A48,b7,C19),(A48,b7,C20),(A48,b7,C21),(A48,b7,C22),(A48 ,b7,C23),(A48,b7,C24),(A48,b7,C25),(A48,b7,C26),(A48,b7,C27),(A48,b7,C28),(A48,b7,C29) ,(A49,b1,C1),(A49,b1,C2),(A49,b1,C3),(A49,b1,C4),(A49,b1,C5),(A49,b1,C6),(A49,b1,C7),(A 49,b1,C8),(A49,b1,C9),(A49,b1,C10),(A49,b1,C11),(A49,b1,C12),(A49,b1,C13),(A49,b1,C14) ,(A49,b1,C15),(A49,b1,C16),(A49,b1,C17),(A49,b1,C18),(A49,b1,C19),(A49,b1,C20),(A49,b1 ,C21),(A49,b1,C22),(A49,b1,C23),(A49,b1,C24),(A49,b1,C25),(A49,b1,C26),(A49,b1,C27),(A 49,b1,C28),(A49,b1,C29),(A49,b2,C1),(A49,b2,C2),(A49,b2,C3),(A49,b2,C4),(A49,b2,C5),(A 49,b2,C6),(A49,b2,C7),(A49,b2,C8),(A49,b2,C9),(A49,b2,C10),(A49,b2,C11),(A49,b2,C12),( A49,b2,C13),(A49,b2,C4),(A49,b2,C15),(A49,b2,C16),(A49,b2,C17),(A49,b2,C18),(A49,b2, C19),(A49,b2,C20),(A49,b2,C21),(A49,b2,C22),(A49,b2,C23),(A49,b2,C24),(A49,b2,C25),(A 49,b2,C26),(A49,b2,C27),(A49,b2,C28),(A49,b2,C29),(A49,b3,C1),(A49,b3,C2),(A49,b3,C3),( A49,b3,C4),(A49,b3,C5),(A49,b3,C6),(A49,b3,C7),(A49,b3,C8),(A49,b3,C9),(A49,b3,C 10),(A 49,b3,C11),(A49,b3,C12),(A49,b3,C13),(A49,b3,C14),(A49,b3,C15),(A49,b3,C16),(A49,b3,C 17),(A49,b3,C18),(A49,b3,C19),(A49,b3,C20),(A49,b3,C21),(A49,b3,C22),(A49,b3,C23),(A49 ,b3,C24),(A49,b3,C25),(A49,b3,C26),(A49,b3,C27),(A49,b3,C28),(A49,b3,C29),(A49,b4,C1),( A49,b4,C2),(A49,b4,C3),(A49,b4,C4),(A49,b4,C5),(A49,b4,C6),(A49,b4,C7),(A49,b4,C8),(A4 9,b4,C9),(A49,b4,C10),(A49,b4,C11),(A49,b4,C12),(A49,b4,C13),(A49,b4,C14),(A49,b4,C15) ,(A49,b4,C16),(A49,b4,C17),(A49,b4,C18),(A49,b4,C19),(A49,b4,C20),(A49,b4,C21),(A49,b4 ,C22),(A49,b4,C23),(A49,b4,C24),(A49,b4,C25),(A49,b4,C26),(A49,b4,C27),(A49,b4,C28),(A 49,b4,C29),(A49,b5,C1),(A49,b5,C2),(A49,b5,C3),(A49,b5,C4),(A49,b5,C5),(A49,b5,C6),(A4 9,b5,C7),(A49,b5,C8),(A49,b5,C9),(A49,b5,C10),(A49,b5,C11),(A49,b5,C12),(A49,b5,C13), A49,b5,C14),(A49,b5,C15),(A49,b5,C16),(A49,b5,C17),(A49,b5,C18),(A49,b5,C19),(A49,b5, C20),(A49,b5,C21),(A49,b5,C22),(A49,b5,C23),(A49,b5,C24),(A49,b5,C25),(A49,b5,C26),(A 49,b5,C27),(A49,b5,C28),(A49,b5,C29),(A49,b6,C1),(A49,b6,C2),(A49,b6,C3),(A49,b6,C4),( A49,b6,C5),(A49,b6,C6),(A49,b6,C7),(A49,b6,C8),(A49,b6,C9),(A49,b6,C10),(A49,b6,C11), A49,b6,C12),(A49,b6,C13),(A49,b6,C14),(A49,b6,C15),(A49,b6,C16),(A49,b6,C17),(A49,b6, C 18),(A49,b6,C 19),(A49,b6,C20),(A49,b6,C21),(A49,b6,C22),(A49,b6,C23),(A49,b6,C24),(A 49,b6,C25),(A49,b6,C26),(A49,b6,C27),(A49,b6,C28),(A49,b6,C29),(A49,b7,C1),(A49,b7,C2) ,(A49,b7,C3),(A49,b7,C4),(A49,b7,C5),(A49,b7,C6),(A49,b7,C7),(A49,b7,C8),(A49,b7,C9),(A 49,b7,C10),(A49,b7,C11),(A49,b7,C12),(A49,b7,C13),(A49,b7,C14),(A49,b7,C15),(A49,b7,C 16),(A49,b7,C17),(A49,b7,C18),(A49,b7,C19),(A49,b7,C20),(A49,b7,C21),(A49,b7,C22),(A49 ,b7,C23),(A49,b7,C24),(A49,b7,C25),(A49,b7,C26),(A49,b7,C27),(A49,b7,C28),(A49,b7,C29) ,(A50,b1,C1),(A50,b1,C2),(A50,b1,C3),(A50,b1,C4),(A50,b1,C5),(A50,b1,C6),(A50,b1,C7),(A 50,b1,C8),(A50,b1,C9),(A50,b1,C10),(A50,b1,C11),(A50,b1,C12),(A50,b1,C13),(A50,b1,C14) ,(A50,b1,C15),(A50,b1,C16),(A50,b1,C17),(A50,b1,C18),(A50,b1,C19),(A50,b1,C20),(A50,b1 ,C21),(A50,b1,C22),(A50,b1,C23),(A50,b1,C24),(A50,b1,C25),(A50,b1,C26),(A50,b1,C27),(A 50,b1,C28),(A50,b1,C29),(A50,b2,1),(A50,b2,C2),(A50,b2,C3),(A50,b2,C4),(A50,b2,C5),(A 50,b2,C6),(A50,b2,C7),(A50,b2,C8),(A50,b2,C9),(A50,b2,C10),(A50,b2,C11),(A50,b2,C12),( A50,b2,C13),(A50,b2,C14),(A50,b2,C15),(A50,b2,C16),(A50,b2,C17),(A50,b2,C18),(A50,b2, C19),(A50,b2,C20),(A50,b2,C21),(A50,b2,C22),(A50,b2,C23),(A50,b2,C24),(A50,b2,C25),(A 50,b2,C26),(A50,b2,C27),(A50,b2,C28),(A50,b2,C29),(A50,b3,C1),(A50,b3,C2),(A50,b3,C3),( A50,b3,C4),(A50,b3,C5),(A50,b3,C6),(A50,b3,C7),(A50,b3,C8),(A50,b3,C9),(A50,b3,C10),(A 50,b3,C11),(A50,b3,C12),(A50,b3,C13),(A50,b3,C14),(A50,b3,C15),(A50,b3,C16),(A50,b3,C 17),(A50,b3,C18),(A50,b3,C19),(A50,b3,C20),(A50,b3,C21),(A50,b3,C22),(A50,b3,C23),(A50 ,b3,C24),(A50,b3,C25),(A50,b3,C26),(A50,b3,C27),(A50,b3,C28),(A50,b3,C29),(A50,b4,C1),( A50,b4,C2),(A50,b4,C3),(A50,b4,C4),(A50,b4,C5),(A50,b4,C6),(A50,b4,C7),(A50,b4,C8),(A5 0,b4,C9),(A50,b4,C10),(A50,b4,C11),(A50,b4,C12),(A50,b4,C13),(A50,b4,C14),(A50,b4,C15) ,(A50,b4,C16),(A50,b4,C17),(A50,b4,C18),(A50,b4,C19),(A50,b4,C20),(A50,b4,C21),(A50,b4 ,C22),(A50,b4,C23),(A50,b4,C24),(A50,b4,C25),(A50,b4,C26),(A50,b4,C27),(A50,b4,C28),(A 50,b4,C29),(A50,b5,C1),(A50,b5,C2),(A50,b5,C3),(A50,b5,C4),(A50,b5,C5),(A50,b5,C6),(A5 0,b5,C7),(A50,b5,C8),(A50,b5,C9),(A50,b5,C10),(A50,b5,C1),(A50,b5,C12),(A50,b5,C13),( A50,b5,C14),(A50,b5,C15),(A50,b5,C16),(A50,b5,C17),(A50,b5,C18),(A50,b5,C19),(A50,b5, C20),(A50,b5,C21),(A50,b5,C22),(A50,b5,C23),(A50,b5,C24),(A50,b5,C25),(A50,b5,C26),(A 50,b5,C27),(A50,b5,C28),(A50,b5,C29),(A50,b6,C1),(A50,b6,C2),(A50,b6,C3),(A50,b6,C4),( A50,b6,C5),(A50,b6,C6),(A50,b6,C7),(A50,b6,C8),(A50,b6,C9),(A50,b6,C10),(A50,b6,C1),( A50,b6,C12),(A50,b6,C13),(A50,b6,C14),(A50,b6,C15),(A50,b6,C16),(A50,b6,C17),(A50,b6, C18),(A50,b6,C19),(A50,b6,C20),(A50,b6,C21),(A50,b6,C22),(A50,b6,C23),(A50,b6,C24),(A 50,b6,C25),(A50,b6,C26),(A50,b6,C27),(A50,b6,C28),(A50,b6,C29),(A50,b7,C1),(A50,b7,C2) ,(A50,b7,C3),(A50,b7,C4),(A50,b7,C5),(A50,b7,C6),(A50,b7,C7),(A50,b7,C8),(A50,b7,C9),(A 50,b7,C10),(A50,b7,C11),(A50,b7,C12),(A50,b7,C13),(A50,b7,C14),(A50,b7,C15),(A50,b7,C 16),(A50,b7,C17),(A50,b7,C18),(A50,b7,C19),(A50,b7,C20),(A50,b7,C21),(A50,b7,C22),(A50 ,b7,C23),(A50,b79C24),(A50,b7,C25),(A50,b7,C26),(A50,b7,C27),(A50,b7,C28),(A50,b7,C29) ,

(A51,b1,C1),(A51,b1,C2),(A51,b1,C3),(A51,b1,C4),(A51,b1,C5),(A51,b1,C6),(A51,b1,C7),(A 51,b1,C8),(A51,b1,C9),(A51,b1,C10),(A51,b1,C11),(A51,b1,C12),(A51,b1,C13),(A51,b1,C14) ,(A51,b1,C15),(A51,b1,C16),(A51,b1,C17),(A51,b1,C18),(A51,b1,C19),(A51,b1,C20),(A51,b1 ,C21),(A51,b1,C22),(A51,b1,C23),(A51,b1,C24),(A51,b1,C25),(A51,b1,C26),(A51,b1,C27),(A 51,b1,C28),(A51,b1,C29),(A51,b2,C1),(A51,b2,C2),(A51,b2,C3),(A51,b2,C4),(A51,b2,C5),(A 51,b2,C6),(A51,b2,C7),(A51,b2,C8),(A51,b2,C9),(A51,b2,C10),(A51,b2,C11),(A51,b2,C12),( A51,b2,C13),(A51,b2,C14),(A51,b2,C15),(A51,b2,C16),(A51,b2,C17),(A51,b2,C18),(A51,b2, C19),(A51,b2,C20),(A51,b2,C21),(A51,b2,C22),(A51,b2,C23),(A51,b2,C24),(A51,b2,C25),(A 51,b2,C26),(A51,b2yC27),(A51,b2,C28),(A51,b2;C29);(A51,b3,C1),(A51,b3,C2),(A51,b3,C3), A51,b3,C4),(A51,b3,C5),(A51,b3,C6),(A51,b3,C7),(A51,b3,C8),(A51,b3,C9),(A51,b3,C10),(A 51,b3,C11),(A51,b3,C12),(A51,b3,C13),(A51,b3,C14),(A51,b3,C15),(A51,b3,C16),(A51,b3,C 17),(A51,b3,C18),(A51,b3,C19),(A51,b3,C20),(A51,b3,C21),(A51,b3,C22),(A51,b3,C23),(A51 ,b3,C24),(A51,b3,C25),(A51,b3,C26),(A51,b3,C27),(A51,b3,C28),(A51,b3,C29),(A51,b4,C1),( A51,b4,C2),(A51,b4,C3),(A51,b4,C4),(A51,b4,C5),(A51,b4,C6),(A51,b4,C7),(A51,b4,C8),(A5 1,b4,C9),(A51,b4,C10),(A51,b4,C11),(A51,b4,C12),(A51,b4,C13),(A51,b4,C14),(A51,b4,C15) ,(A51,b4,C16),(A51,b4,C17),(A51,b4,C18),(A51,b4,C19),(A51,b4,C20),(A51,b4,C21),(A51,b4 ,C22),(A51,b4,C23),(A51,b4,C24),(A51,b4,C25),(A51,b4,C26),(A51,b4,C27),(A51,b4,C28),(A 51,b4,C29),(A51,b5,C1),(A51,b5,C2),(A51,b5,C3),(A51,b5,C4),(A51,b5,C5),(A51,b5,C6),(A5 1,b5,C7),(A51,b5,C8),(A51,b5,C9),(A51,b5,C10),(A51,b5,C11),(A51,b5,C12),(A51,b5,C13),( A51,b5,C14),(A51,b5,C15),(A51,b5,C16),(A51,b5,C17),(A51,b5,C18),(A51,b5,C19),(A51,b5, C20),(A51,b5,C21),(A51,b5,C22),(A51,b5,C23),(A51,b5,C24),(A51,b5,C25),(A51,b5,C26),(A 51,b5,C27),(A51,b5,C28),(A51,b5,C29),(A51,b6,C1),(A51,b6,C2),(A51,b6,C3),(A51,b6,C4),( A51,b6,C5),(A51,b6,C6),(A51,b6,C7),(A51,b6,C8),(A51,b6,C9),(A51,b6,C10),(A51,b6,C11),( A51,b6,C12),(A51,b6,C13),(A51,b6,C14),(A51,b6,C15),(A51,b6,C16),(A51,b6,C17),(A51,b6, C18),(A51,b6,C19),(A51,b6,C20),(A51,b6,C21),(A51,b6,C22),(A51,b6,C23),(A51,b6,C24),(A 51,b6,C25),(A51,b6,C26),(A51,b6,C27),(A51,b6,C28),(A51,b6,C29),(A51,b7,C1),(A51,b7,C2) ,(A51,b7,C3),(A51,b7,C4),(A51,b7,C5),(A51,b7,C6),(A51,b7,C7),(A51,b7,C8),(A51,b7,C9),(A 51,b7,C10),(A51,b7,C11),(A51,b7,C12),(A51,b7,C13),(A51,b7,C14),(A51,b7,C15),(A51,b7,C 16),(A51,b7,C17),(A51,b7,C18),(A51,b7,C19),(A51,b7,C20),(A51,b7,C21),(A51,b7,C22),(A51 ,b7,C23),(A51,b7,C24),(A51,b7,C25),(A51,b7,C26),(A51,b7,C27),(A51,b7,C28),(A51,b7,C29) ,(A52,b1,C1),(A52,b1,C2),(A52,b1,C3),(A52,b1,C4),(A52,b1,C5),(A52,b1,C6),(A52,b1,C7),(A 52,b1,C8),(A52,b1,C9),(A52,b1,C10),(A52,b1,C11),(A52,b1,C12),(A52,b1,C13),(A52,b1,C14) ,(A52,b1,C15),(A52,b1,C16),(A52,b1,C17),(A52,b1,C18),(A52,b1,C19),(A52,b1,C20),(A52,b1 ,C21),(A52,b1,C22),(A52,b1,C23),(A52,b1,C24),(A52,b1,C25),(A52,b1,C26),(A52,b1,C27),(A 52,b1,C28),(A52,b1,C29),(A52,b2,C1),(A52,b2,C2),(A52,b2,C3),(A52,b2,C4),(A52,b2,C5),(A 52,b2,C6),(A52,b2,C7),(A52,b2,C8),(A52,b2,C9),(A52,b2,C10),(A52,b2,C11),(A52,b2,C12),( A52,b2,C13),(A52,b2,C14),(A52,b2,C15),(A52,b2,C16),(A52,b2,C17),(A52,b2,C18),(A52,b2, C19),(A52,b2,C20),(A52,b2,C21),(A52,b2,C22),(A52,b2,C23),(A52,b2,C24),(A52,b2,C25),(A 52,b2,C26),(A52,b2,C27),(A52,b2,C28),(A52,b2,C29),(A52,b3,C1),(A52,b3,C2),(A52,b3,C3),( A52,b3,C4),(A52,b3,C5),(A52,b3,C6),(A52,b3,C7),(A52,b3,C8),(A52,b3,C9),(A52,b3,C10),(A 52,b3,C11),(A52,b3,C12),(A52,b3,C13),(A52,b3,C14),(A52,b3,C15),(A52,b3,C16),(A52,b3,C 17),(A52,b3,C18),(A52,b3,C19),(A52,b3,C20),(A52,b3,C21),(A52,b3,C22),(A52,b3,C23),(A52 ,b3,C24),(A52,b3,C25),(A52,b3,C26),(A52,b3,C27),(A52,b3,C28),(A52,b3,C29),(A52,b4,C1),( A52,b4,C2),(A52,b4,C3),(A52,b4,C4),(A52,b4,C5),(A52,b4,C6),(A52,b4,C7),(A52,b4,C8),(A5 2,b4,C9),(A52,b4,C10),(A52,b4,C11),(A52,b4,C12),(A52,b4,C13),(A52,b4,C14),(A52,b4,C15) ,(A52,b4,C16),(A52,b4,C17),(A52,b4,C18),(A52,b4,C19),(A52,b4,C20),(A52,b4,C21),(A52,b4 ,C22),(A52,b4,C23),(A52,b4,C24),(A52,b4,C25),(A52,b4,C26),(A52,b4,C27),(A52,b4,C28),(A 52,b4,C29),(A52,b5,C1),(A52,b5,C2),(A52,b5,C3),(A52,b5,C4),(A52,b5,C5),(A52,b5,C6),(A5 2,b5,C7),(A52,b5,C8),(A52,b5,C9),(A52,b5,C10),(A52,b5,C11),(A52,b5,C12),(A52,b5,C13),( A52,b5,C14),(A52,b5,C15),(A52,b5,C16),(A52,b5,C17),(A52,b5,C18),(A52,b5,C19),(A52,b5, C20),(A52,b5,C21),(A52,b5,C22),(A52,b5,C23),(A52,b5,C24),(A52,b5,C25),(A52,b5,C26),(A 52,b5,C27),(A52,b5,C28),(A52,b5,C29),(A52,b6,C1),(A52,b6,C2),(A52,b6,C3),(A52,b6,C4),( A52,b6,C5),(A52,b6,C6),(A52,b6,C7),(A52,b6,C8),(A52,b6,C9),(A52,b6,C10),(A52,b6,C11),( A52,b6,C12),(A52,b6,C13),(A52,b6,C14),(A52,b6,C15),(A52,b6,C16),(A52,b6,C17),(A52,b6, C18),(A52,b6,C19),(A52,b6,C20),(A52,b6,C21),(A52,b6,C22),(A52,b6,C23),(A52,b6,C24),(A 52,b6,C25),(A52,b6,C26),(A52,b6,C27),(A52,b6,C28),(A52,b6,C29),(A52,b7,C1),(A52,b7,C2) ,(A52,b7,C3),(A52,b7,C4),(A52,b7,C5),(A52,b7,C6),(A52,b7,C7),(A52,b7,C8),(A52,b7,C9),(A 52,b7,C10),(A52,b7,C11),(A52,b7,C12),(A52,b7,C13),(A52,b7,C14),(A52,b7,C15),(A52,b7,C 16),(A52,b7,C17),(A52,b7,C18),(A52,b7,C19),(A52,b7,C20),(A52,b7,C21),(A52,b7,C22),(A52 ,b7,C23),(A52,b7,C24),(A52,b7,C25),(A52,b7,C26),(A52,b7,C27),(A52,b7,C28),(A52,b7,C29) ,(A53,b1,C1),(A53,b1,C2),(A53,b1,C3),(A53,b1,C4),(A53,b1,C5),(A53,b1,C6),(A53,b1,C7),(A 53,b1,C8),(A53,b1,C9),(A53,b1,C10),(A53,b1,C11),(A53,b1,C12),(A53,b1,C13),(A53,b1,C14) ,(A53,b1,C15),(A53,b1,C16),(A53,b1,C17),(A53,b1,C18),(A53,b1,C19),(A53,b1,C20),(A53,b1 ,C21),(A53,b1,C22),(A53,b1,C23),(A53,b1,C24),(A53,b1,C25),(A53,b1,C26),(A53,b1,C27),(A 53,b1,C28),(A53,b1,C29),(A53,b2,C1),(A53,b2,C2),(A53,b2,C3),(A53,b2,C4),(A53,b2,C5),(A 53,b2,C6),(A53,b2,C7),(A53,b2,C8),(A53,b2,C9),(A53,b2,C10),(A53,b2,C11),(A53,b2,C12),( A53,b2,C13),(A53,b2,C14),(A53,b2,C15),(A53,b2,C16),(A53,b2,C17),(A53,b2,C18),(A53,b2, C19),(A53,b2,C20),(A53,b2,C21),(A53,b2,C22),(A53,b2,C23),(A53,b2,C24),(A53,b2,C25),(A 53,b2,C26),(A53,b2,C27),(A53,b2,C28),(A53,b2,C29),(A53,b3,C1),(A53,b3,C2),(A53,b3,C3),( A53,b3,C4),(A53,b3,C5),(A53,b3,C6),(A53,b3,C7),(A53,b3,C8),(A53,b3,C9),(A53,b3,C10),(A 53,b3,C11),(A53,b3,C12),(A53,b3,C13),(A53,b3,C14),(A53,b3,C15),(A53,b3,C16),(A53,b3,C 17),(A53,b3,C18),(A53,b3,C19),(A53,b3,C20),(A53,b3,C21),(A53,b3,C22),(A53,b3,C23),(A53 ,b3,C24),(A53,b3,C25),(A53,b3,C26),(A53,b3,C27),(A53,b3,C28),(A53,b3,C29),(A53,b4,C1),( A53,b4,C2),(A53,b4,C3),(A53,b4,C4),(A53,b4,C5),(A53,b4,C6),(A53,b4,C7),(A53,b4,C8),(A5 3,b4,C9),(A53,b4,C10),(A53,b4,C11),(A53,b4,C12),(A53,b4,C13),(A53,b4,C14),(A53,b4,C15) ,(A53,b4,C16),(A53,b4,C17),(A53,b4,C18),(A53,b4,C19),(A53,b4,C20),(A53,b4,C21),(A53,b4 ,C22),(A53,b4,C23),(A53,b4,C24),(A53,b4,C25),(A53,b4,C26),(A53,b4,C27),(A53,b4,C28),(A 53,b4,C29),(A53,b5,C1),(A53,b5,C2),(A53,b5,C3),(A53,b5,C4),(A53,b5,C5),(A53,b5,C6),(A5 3,b5,C7),(A53,b5,C8),(A53,b5,C9),(A53,b5,C10),(A53,b5,C11),(A53,b5,C12),(A53,b5,C13),( A53,b5,C14),(A53,b5,C15),(A53,b5,C16),(A53,b5,C17),(A53,b5,C18),(A53,b5,C19),(A53,b5, C20),(A53,b5,C21),(A53,b5,C22),(A53,b5,C23),(A53,b5,C24),(A53,b5,C25),(A53,b5,C26),(A 53,b5,C27),(A53,b5,C28),(A53,b5,C29),(A53,b6,C1),(A53,b6,C2),(A53,b6,C3),(A53,b6,C4),( A53,b6,C5),(A53,b6,C6),(A53,b6,C7),(A53,b6,C8),(A53,b6,C9),(A53,b6,C10),(A53,b6,C11),( A53,b6,C12),(A53,b6,C13),(A53,b6,C14),(A53,b6,C15),(A53,b6,C16),(A53,b6,C17),(A53,b6, C18),(A53,b6,C19),(A53,b6,C20),(A53,b6,C21),(A53,b6,C22),(A53,b6,C23),(A53,b6,C24),(A 53,b6,C25),(A53,b6,C26),(A53,b6,C27),(A53,b6,C28),(A53,b6,C29),(A53,b7,C1),(A53,b7,C2) ,(A53,b7,C3),(A53,b7,C4),(A53,b7,C5),(A53,b7,C6),(A53,b7,C7),(A53,b7,C8),(A53,b7,C9),(A 53,b7,C10),(A53,b7,C11),(A53,b7,C12),(A53,b7,C13),(A53,b7,C14),(A53,b7,C15),(A53,b7,C 16),(A53,b7,C17),(A53,b7,C18),(A53,b7,C19),(A53,b7,C20),(A53,b7,C21),(A53,b7,C22),(A53 ,b7,C23),(A53,b7,C24),(A53,b7,C25),(A53,b7,C26),(A53,b7,C27),(A53,b7,C28),(A53,b7,C29) ,(A54,b1,C1),(A54,b1,C2),(A54,b1,C3),(A54,b1,C4),(A54,b1,C5),(A54,b1,C6),(A54,b1,C7),(A 54,b1,C8),(A54,b1,C9),(A54,b1,C10),(A54,b1,C11),(A54,b1,C12),(A54,b1,C13),(A54,b1,C14) ,(A54,b1,C15),(A54,b1,C16),(A54,b1,C17),(A54,b1,C18),(A54,b1,C19),(A54,b1,C20),(A54,b1 ,C21),(A54,b1,C22),(A54,b1,C23),(A54,b1,C24),(A54,b1,C25),(A54,b1,C26),(A54,b1,C27),(A 54,b1,C28),(A54,b1,C29),(A54,b2,C1),(A54,b2,C2),(A54,b2,C3),(A54,b2,C4),(A54,b2,C5),(A 54,b2,C6),(A54,b2,C7),(A54,b2,C8),(A54,b2,C9), (A54,b2,C10),(A54,b2,C11),(A54,b2,C12),( A54,b2,C13),(A54,b2,C14),(A54,b2,C15),(A54,b2,C16),(A54,b2,C17),(A54,b2,C18),(A54,b2, C19),(A54,b2,C20),(A54,b2,C21),(A54,b2,C22),(A54,b2,C23),(A54,b2,C24),(A54,b2,C25),(A 54,b2,C26),(A54,b2,C27),(A54,b2,C28),(A54,b2,C29),(A54,b3,C1),(A54,b3,C2),(A54,b3,C3),( A54,b3,C4),(A54,b3,C5),(A54,b3,C6),(A54,b3,C7),(A54,b3,C8),(A54,b3,C9),(A54,b3,C10),(A 54,b3,C11),(A54,b3,C12),(A54,b3,C13),(A54,b3,C14),(A54,b3,C15),(A54,b3,C16),(A54,b3,C 17),(A54,b3,C18),(A54,b3,C19),(A54,b3,C20),(A54,b3,C21),(A54,b3,C22),(A54,b3,C23),(A54 ,b3,C24),(A54,b3,C25),(A54,b3,C26),(A54,b3,C27),(A54,b3,C28),(A54,b3,C29),(A54,b4,C1),( A54,b4,C2),(A54,b4,C3),(A54,b4,C4),(A54,b4,C5),(A54,b4,C6),(A54,b4,C7),(A54,b4,C8),(A5 4,b4,C9),(A54,b4,C10),(A54,b4,C11),(A54,b4,C12),(A54,b4,C13),(A54,b4,C14),(A54,b4,C15) ,(A54,b4,C16),(A54,b4,C17),(A54,b4,C18),(A54,b4,C19),(A54,b4,C20),(A54,b4,C21),(A54,b4 ,C22),(A54,b4,C23),(A54,b4,C24),(A54,b4,C25),(A54,b4,C26),(A54,b4,C27),(A54,b4,C28),(A 54,b4,C29),(A54,b5,C1),(A54,b5,C2),(A54,b5,C3),(A54,b5,C4),(A54,b5,C5),(A54,b5,C6),(A5 4,b5,C7),(A54,b5,C8),(A54,b5,C9),(A54,b5,C10),(A54,b5,C11),(A54,b5,C12),(A54,b5,C13),( A54,b5,C14),(A54,b5,C15),(A54,b5,C16),(A54,b5,C17),(A54,b5,C18),(A54,b5,C19),(A54,b5, C20),(A54,b5,C21),(A54,b5,C22),(A54,b5,C23),(A54,b5,C24),(A54,b5,C25),(A54,b5,G26),(A 54,b5,C27),(A54,b5,C28),(A54,b5,C29),(A54,b6,C1),(A54,b6,C2),(A54,b6,C3),(A54,b6,C4),( A54,b6,C5),(A54,b6,C6),(A54,b6,C7),(A54,b6,C8),(A54,b6,C9),(A54,b6,C10),(A54,b6,C11),( A54,b6,C12),(A54,b6,C13),(A54,b6,C14),(A54,b6,C15),(A54,b6,C16),(A54,b6,C17),(A54,b6, C18),(A54,b6,C19),(A54,b6,C20),(A54,b6,C21),(A54,b6,C22),(A54,b6,C23),(A54,b6,C24),(A 54,b6,C25),(A54,b6,C26),(A54,b6,C27),(A54,b6,C28),(A54,b6,C29),(A54,b7,C1),(A54,b7,C2) ,(A54,b7,C3),(A54,b7,C4),(A54,b7,C5),(A54,b7,C6),(A54,b7,C7),(A54,b7,C8),(A54,b7,C9),(A 54,b7,C10),(A54,b7,C11),(A54,b7,C12),(A54,b7,C13),(A54,b7,C14),(A54,b7,C15),(A54,b7,C 16),(A54,b7,C17),(A54,b7,C18),(A54,b7,C19),(A54,b7,C20),(A54,b7,C21),(A54,b7,C22),(A54 ,b7,C23),(A54,b7,C24),(A54,b7,C25),(A54,b7,C26),(A54,b7,C27),(A54,b7,C28),(A54,b7,C29) ,(A55,b1,C1),(A55,b1,C2),(A55,b1,C3),(A55,b1,C4),(A55,b1,C5),(A55,b1,C6),(A55,b1,C7),(A 55,b1,C8),(A55,b1,C9),(A55,b1,C10),(A55,b1,C11),(A55,b1,C12),(A55,b1,C13),(A55,b1,C14) (A55,b1,C15),(A55,b1,C16),(A55,b1,C17),(A55,b1,C18),(A55,b1,C19),(A55,b1,C20),(A55,b1 ,C21),(A55,b1,C22),(A55,b1,C23),(A55,b1,C24),(A55,b1,C25),(A55,b1,C26),(A55,b1,C27),(A 55,b1,C28),(A55,b1,C29),(A55,b2,C1),(A55,b2,C2),(A55,b2,C3),(A55,b2,C4),(A55,b2,C5),(A 55,b2,C6),(A55,b2,C7),(A55,b2,C8),(A55,b2,C9),(A55,b2,C10),(A55,b2,C11),(A55,b2,C12),( A55,b2,C13),(A55,b2,C14),(A55,b2,C15),(A55,b2,C16),(A55,b2,C17),(A55,b2,C18),(A55,b2, C19),(A55,b2,C20),(A55,b2,C21),(A55,b2,C22),(A55,b2,C23),(A55,b2,C24),(A55,b2,C25),(A 55,b2,C26),(A55,b2,C27),(A55,b2,C28),(A55,b2,C29),(A55,b3,C1),(A55,b3,C2),(A55,b3,C3),( A55,b3,C4),(A55,b3,C5),(A55,b3,C6),(A55,b3,C7),(A55,b3,C8),(A55,b3,C9),(A55,b3,C10),(A 55,b3,C11),(A55,b3,C12),(A55,b3,C13),(A55,b3,C14),(A55,b3,C15),(A55,b3,C16),(A55,b3,C 17),(A55,b3,C18),(A55,b3,C19),(A55,b3,C20),(A55,b3,C21),(A55,b3,C22),(A55,b3,C23),(A55 ,b3,C24),(A55,b3,C25),(A55,b3,C26),(A55,b3,C27),(A55,b3,C28),(A55,b3,C29),(A55,b4,C1),( A55,b4,C2),(A55,b4,C3),(A55,b4,C4),(A55,b4,C5),(A55,b4,C6),(A55,b4,C7),(A55,b4,C8),(A5 5,b4,C9),(A55,b4,C10),(A55,b4,C12),(A55,b4,C12),(A55,b4,C13),(A55,b4,C14),(A55,b4,C15) ,(A55,b4,C16),(A55,b4,C17),(A55,b4,C18),(A55,b4,C19),(A55,b4,C20),(A55,b4,C21),(A55,b4 ,C22),(A55,b4,C23),(A55,b4,C24),(A55,b4,C25),(A55,b4,C26),(A55,b4,C27),(A55,b4,C28),(A 55,b4,C29),(A55,b5,C1),(A55,b5,C2),(A55,b5,C3),(A55,b5,C4),(A55,b5,C5),(A55,b5,C6),(A5 5,b5,C7),(A55,b5,C8),(A55,b5,C9),(A55,b5,C10),(A55,b5,C11),(A55,b5,C12),(A55,b5,C13),( A55,b5,C14),(A55,b5,C15),(A55,b5,C16),(A55,b5,C177),(A55,b5,C18),(A55,b5,C19),(A55,b5, C20),(A55,b5,C21),(A55,b5,C22),(A55,b5,C23),(A55,b5,C24),(A55,b5,C25),(A55,b5,C26),(A 55,b5,C27),(A55,b5,C28),(A55,b5,C29),(A55,b6,C1),(A55,b6,C2),(A55,b6,C3),(A55,b6,C4),( A55,b6,C5),(A55,b6,C6),(A55,b6,C7),(A55,b6,C8),(A55,b6,C9),(A55,b6,C10),(A55,b6,C11),( A55,b6,C12),(A55,b6,C13),(A55,b6,C14),(A55,b6,C15),(A55,b6,C16),(A55,b6,C17),(A55,b6, C18),(A55,b6,C19),(A55,b6,C20),(A55,b6,C21),(A55,b6,C22),(A55,b6,C23),(A55,b6,C24),(A 55,b6,C25),(A55,bC,C26),(A55,b6,C27),(A55,b6,C28),(A55,b6,C29),(A55,b7,C1),(A55,b7,C2) ,(A55,b7,C3),(A55,b7,C4),(A55,b7,C5),(A55,b7,C6),(A55,b7,C7),(A55,b7,C8),(A55,b7,C9),(A 55,b7,C10),(A55,b7,C11),(A55,b7,C12),(A55,b7,C13),(A55,b7,C14),(A55,b7,C15),(A55,b7,C 16),(A55,b7,C17),(A55,b7,C18),(A55,b7,C19),(A55,b7,C20),(A55,b7,C21),(A55,b7,C22),(A55 ,b7,C23),(A55,b7,C24),(A55,b7,C25),(A55,b7,C26),(A55,b7,C27),(A55,b7,C28),(A55,b7,C29) ,(A56,b1,C1),(A56,b1,C2),(A56,b1,C3),(A56,b1,C4),(A56,b1,C5),(A56,b1,C6),(A56,b1,C7),(A 56,b1,C8),(A56,b1,C9),(A56,b1,C10),(A56,b1,C11),(A56,b1,C12),(A56,b1,C13),(A56,b1,C14) ,(A56,b1,C15),(A56,b1,C16),(A56,b1,C17),(A56,b1,C18),(A56,b1,C19),(A56,b1,C20),(A56,b1 ,C21),(A56,b1,C22),(A56,b1,C23),(A56,b1,C24),(A56,b1,C25),(A56,b1,C26),(A56,b1,C27),(A 56,b1,C28),(A56,b1,C29),(A56,b2,C1),(A56,b2,C2),(A56,b2,C3),(A56,b2,C4),(A56,b2,C5),(A 56,b2,C6),(A56,b2,C7),(A56,b2,C8),(A56,b2,C9),(A56,b2,C10),(A56,b2,C11),(A56,b2,C12),( A56,b2,C13),(A56,b2,C14),(A56,b2,C15),(A56,b2,C16),(A56,b2,C17),(A56,b2,C18),(A56,b2, C19),(A56,b2,C20),(A56,b2,C21),(A56,b2,C22),(A56,b2,C23),(A56,b2,C24),(A56,b2,C25),(A 56,b2,C26),(A56,b2,C27),(A56,b2,C28),(A56,b2,C29),(A56,b3,C1),(A56,b3,G2),(A56,b3,C3),( A56,b3,C4),(A56,b3,C5),(A56,b3,C6),(A56,b3,C7),(A56,b3,C8),(A56,b3,C9),(A56,b3,C10),(A 56,b3,C11),(A56,b3,C12),(A56,b3,C13),(A56,b3,C14),(A56,b3,C15),(A56,b3,C16),(A56,b3,C 17),(A56,b3,C18),(A56,b3,C19),(A56,b3,C20),(A56,b3,C21),(A56,b3,C22),(A56,b3,C23),(A56 ,b3,C24),(A56,b3,C25),(A56,b3,C26),(A56,b3,C27),(A56,b3,C28),(A56,b3,C29),(A56,b4,C1),( A56,b4,C2),(A56,b4,C3),(A56,b4,C4),(A56,b4,C5),(A56,b4,C6),(A56,b4,C7),(A56,b4,C8),(A5 6,b4,C9),(A56,b4,C10),(A56,b4,C1),(A56,b4,C12),(A56,b4,C13),(A56,b4,C14),(A56,b4,C15) ,(A56,b4,C16),(A56,b4,C17),(A56,b4,C18),(A56,b4,C19),(A56,b4,C20),(A56,b4,C21),(A56,b4 ,C22),(A56,b4,C23),(A56,b4,C24),(A56,b4,C25),(A56,b4,C26),(A56,b4,C27),(A56,b4,C28)_{;}(A 56,b4,C29),(A56,b5,C1),(A56,b5,C2),(A56,b5,C3),(A56,b5,C4),(A56,b5,C5),(A56,b5,C6),(A5 6,b5,C7),(A56,b5,C8),(A56,b5,C9),(A56,b5,C10),(A56,b5,C11),(A56,b5,C12),(A56,b1,C13),( A56,b5,C14),(A56,b5,C15),(A56,b5,C16),(A56,b5,C17),(A56,b5,C18),(A56,b5,C19),(A56,b5, C20),(A56,b5,C21),(A56,b5,C22),(A56,b5,C23),(A56,b5,C24),(A56,b5,C25),(A56,b5,C26),(A 56,b5,C27),(A56,b5,C28),(A56,b5,C29),(A56,b6,C1),(A56,b6,C2),(A56,b6,C3),(A56,b6,C4),( A56,b6,C5),(A56,b6,C6),(A56,b6,C7),(A56,b6,C8),(A56,b6,C9),(A56,b6,C10),(A56,b6,C11),( A56,b6,C12),(A56,b6,C13),(A56,b6,C14),(A56,b6,C15),(A56,b6,C16),(A56,b6,C17),(A56,b6, C18),(A56,b6,C19),(A56,b6,C20),(A56,b6,C21),(A56,b6,C22),(A56,b6,C23),(A56,b6,C24),(A 56,b6,C25),(A56,b6,C26),(A56,b6,C27),(A56,b6,C28),(A56,b6,C29),(A56,b7,C0,(A56,b7,C2) ,(A56,b7,C3),(A56,b7,C4),(A56,b7,C5),(A56,b7,C6),(A56,b7,C7),(A56,b7,C8),(A56,b7,C9),(A 56,b7,C10),(A56,b7,C11),(A56,b7,C12),(A56,b7,C13),(A56,b7,C14),(A56,b7,C15),(A56,b7,C 16),(A56,b7,C17),(A56,b7,C18),(A56,b7,C19),(A56,b7,C20),(A56,b7,C21),(A56,b7,C22),(A56 ,b7,C23),(A56,b7,C24),(A56,b7,C25),(A56,b7,C26),(A56,b7,C27),(A56,b7,C28),(A56,b7,C29) ,(A57,b1,C1),(A57,b1,C2),(A57,b1,C3),(A57,b1,C4),(A57,b1,C5),(A57,b1,C6),(A57,b1,C7),(A 57,b1,C8),(A57,b1,C9),(A57,b1,C10),(A57,b1,C11),(A57,b1,C12),(A57,b1,C13),(A57,b1,C14) (A57,b1,C15),(A57,b1,C16),(A57,b1,C17),(A57,b1,C18),(A57,b1,C19),(A57,b1,C20),(A57,b1 ,C21),(A57,b1,C22),(A57,b1,C23),(A57,b1,C24),(A57,b1,C25),(A57,b1,C26),(A57,b1,C27),(A 57,b1,C28),(A57,b1,C29),(A57,b2,C1),(A57,b2,C2),(A57,b2,C3),(A57,b2,C4),(A57,b2,C5),(A 57,b2,C6),(A57,b2,C7),(A57,b2,C8),(A57,b2,C9),(A57,b2,C10),(A57,b2,C11),(A57,b2,C12),( A57,b2,C13),(A57,b2,C14),(A57,b2,C15),(A57,b2,C16),(A57,b2,C17),(A57,b2,C18),(A57,b2, C19),(A57,b2,C20),(A57,b2,C21),(A57,b2,C22),(A57,b2,C23),(A57,b2,C24),(A57,b2,C25),(A 57,b2,C26),(A57,b2,C27),(A57,b2,C28),(A57,b2,C29),(A57,b3,C1),(A57,b3,C2),(A57,b3,C3),( A57,b3,C4),(A57,b3,C5),(A57,b3,C6),(A57,b3,C7),(A57,b3,C8),(A57,b3,C9),(A57,b3,C10),(A 57,b3,C11),(A57,b3,C12),(A57,b3,C13),(A57,b3,C14),(A57,b3,C15),(A57,b3,C16),(A57,b3,C 17),(A57,b3,C18),(A57,b3,C19),(A57,b3,C20),(A57,b3,C21),(A57,b3,C22),(A57,b3,C23),(A57 ,b3,C24),(A57,b3,C25),(A57,b3,C26),(A57,b3,C27),(A57,b3,C28),(A57,b3,C29),(A57,b4,C1),( A57,b4,C2),(A57,b4,C3),(A57,b4,C4),(A57,b4,C5),(A57,b4,C6),(A57,b4,C7),(A57,b4,C8),(A5 7,b4,C9),(A57,b4,C10),(A57,b4,C11),(A57,b4,C12),(A57,b4,C13),(A57,b4,C14),(A57,b4,C15) ,(A57,b4,C16),(A57,b4,C17),(A57,b4,C18),(A57,b4,C19),(A57,b4,C20),(A57,b4,C21),(A57,b4 ,C22),(457,b4,C23),(A57,b4,C24),(A57,b4,C25),(A57,b4,C26),(A57,b4,C27),(A57,b4,C28),(A 57,b4,C29),(A57,b5,C1),(A57,b5,C2),(A57,b5,C3),(A57,b5,C4),(A57,b5,C5),(A57,b5,C6),(A5 7,b5,C7),(A57,b5,C8),(A57,b5,C9),(A57,b5,C10),(A57,b5,C11),(A57,b5,C12),(A57,b5,C13),( A57,b5,C14),(A57,b5,C15),(A57,b5,C16),(A57,b5,C17),(A57,b5,C18),(A57,b5,C19),(A57,b5, C20),(A57,b5,C21),(A57,b5,C22),(A57,b5,C23),(A57,b5,C24),(A51,b5,C25),(A51,b5,C26),(A 57,b5,C27),(A57,b5,C28),(A57,b5,C29),(A57,b6,C1),(A57,b6,C2),(A57,b6,C3),(A57,b6,C4),( A57,b6,C5),(A57,b6,C6),(A57,b6,C7),(A57,b6,C8),(A57,b6,C9),(A57,b6,C10),(A57,b6,C11),( A57,b6,C12),(A57,b6,C13),(A57,b6,C14),(A57,b6,C15),(A57,b6,C16),(A57,b6,C17),(A57,b6, C18),(A57,b6,C19),(A57,b6,C20),(A57,b6,C21),(A57,b6,C22),(A57,b6,C23),(A57,b6,C24),(A 57,b6,C25),(A57,b6,C26),(A57,b6,C27),(A57,b6,C28),(A57,b6,C29),(A57,b7,C1),(A57,b7,C2) ,(A57,b7,C3),(A57,b7,C4),(A57,b7,C5),(A57,b7,C6),(A57,b7,C7),(A57,b7,C8),(A57,b7,C9),(A 57,b7,C10),(A57,b7,C12),(A57,b7,C12),(A57,b7,C13),(A57,b7,C14),(A57,b7,C15),(A57,b7,C 16),(A57,b7,C17),(A57,b7,C18),(A57,b7,C19),(A57,b7,C20),(A57,b7,C21),(A57,b7,C22),(A57 ,b7,C23),(A57,b7,C24),(A57,b7,C25),(A57,b7,C26),(A57,b7,C27),(A57,b7,C28),(A57,b7,C29) ,(A58,b1,C1),(A58,b1,C2),(A58,b1,C3),(A58,b1,C4),(A58,b1,C5),(A58,b1,C6),(A58,b1,C7),(A 58,b1,C8),(A58,b1,C9),(A58,b1,C10),(A58,b1,C11),(A58,b1,C12),(A58,b1,C13),(A58,b1,C14) ,(A58,b1,C15),(A58,b1,C16),(A58,b1,C17),(A58,b1,C18),(A58,b1,C19),(A58,b1,C20),(A58,b1 ,C21),(A58,b1,C22),(A58,b1,C23),(A58,b1,C24),(A58,b1,C25),(A58,b1,C26),(A58,b1,C27),(A 58,b1,C28),(A58,b1,C29),(A58,b2,C1),(A58,b2,C2),(A58,b2,C3),(A58,b2,C4),(A58,b2,C5),(A 58,b2,C6),(A58,b2,C7),(A58,b2,C8),(A58,b2,C9),(A58,b2,C10),(A58,b2,C11),(A58,b2,C12),( A58,b2,C13),(A58,b2,C14),(A58,b2,C15),(A58,b2,C16),(A58,b2,C17),(A58,b2,C18),(A58,b2, C19),(A58,b2,C20),(A58,b2,C21),(A58,b2,C22),(A58,b2,C23),(A58,b2,C24),(A58,b2,C25),(A 58,b2,C26),(A58,b2,C27),(A58,b2,C28),(A58,b2,C29),(A58,b3,C1),(A58,b3,C2),(A58,b3,C3),( A58,b3,C4),(A58,b3,C5),(A58,b3,C6),(A58,b3,C7),(A58,b3,C8),(A58,b3,C9),(A58,b3,C10),(A 58,b3,C11),(A58,b3,C12),(A58,b3,C13),(A58,b3,C14),(A58,b3,C15),(A58,b3,C16),(A58,b3,C 17),(A58,b3,C18),(A58,b3,C19),(A58,b3,C20),(A58,b3,C21),(A58,b3,C22),(A58,b3,C23),(A58 ,b3,C24),(A58,b3,C25),(A58,b3,C26),(A58,b3,C27),(A58,b3,C28),(A58,b3,C29),(A58,b4,C1),( A58,b4,C2),(A58,b4,C3),(A58,b4,C4),(A58,b4,C5),(A58,b4,C6),(A58,b4,C7),(A58,b4,C8),(A5 8,b4,C9),(A58,b4,C10),(A58,b4,C11),(A58,b4,C12),(A58,b4,C13),(A58,b4,C14),(A58,b4,C15) ,(A58,b4,C16),(A58,b4,C17),(A58,b4,C18),(A58,b4,C19),(A58,b4,C20),(A58,b4,C21),(A58,b4 ,C22),(A58,b4,C23),(A58,b4,C24),(A58,b4,C25),(A58,b4,C26),(A58,b4,C27),(A58,b4,C28),(A 58,b4,C29),(A58,b5,C1),(A58,b5,C2),(A58,b5,C3),(A58,b5,C4),(A58,b5,C5),(A58,b5,C6),(A5 8,b5,C7),(A58,b5,C8),(A58,b5,C9),(A58,b5,C10),(A58,b5,C11),(A58,b5,C12),(A58,b5,C13),( A58,b5,C14),(A58,b5,C15),(A58,b5,C16),(A58,b5,C17),(A58,b5,C18),(A58,b5,C19),(A58,b5, C20),(A58,b5,C21),(A58,b5,C22),(A58,b5,C23),(A58,b5,C24),(A58,b5,C25),(A58,b5,C26),(A 58,b5,C27),(A58,b5,C28),(A58,b5,C29),(A58,b6,C1),(A58,b6,C2),(A58,b6,C3),(A58,b6,C4),( A58,b6,C5),(A58,b6,C6),(A58,b6,C7),(A58,b6,C8),(A58,b6,C9),(A58,b6,C10),(A58,b6,C11),( A58,b6,C12),(A58,b6,C13),(A58,b6,C14),(A58,b6,C15),(A58,b6,C16),(A58,b6,C17),(A58,b6, C18),(A58,b6,C19),(A58,b6,C20),(A58,b6,C21),(A58,b6,C22),(A58,b6,C23),(A58,b6,C24),(A 58,b6,C25),(A58,b6,C26),(A58,b6,C27),(A58,b6,C28),(A58,b6,C29),(A58,b7,C1),(A58,b7,C2) ,(A58,b7,C3),(A58,b7,C4),(A58,b7,C5),(A58,b7,C6),(A58,b7,C7),(A58,b7,C8),(A58,b7,C9),(A 58,b7,C10),(A58,b7,C11),(A58,b7,C12),(A58,b7,C13),(A58,b7,C14),(A58,b7,C15),(A58,b7,C 16),(A58,b7,C17),(A58,b7,C18),(A58,b7,C19),(A58,b7,C20),(A58,b7,C21),(A58,b7,C22),(A58 ,b7,C23),(A58,b7,C24),(A58,b7,C25),(A58,b7,C26),(A58,b7,C27),(A58,b7,C28),(A58,b7,C29) ,(A59,b1,C1),(A59,b1,C2),(A59,b1,C3),(A59,b1,C4),(A59,b1,C5),(A59,b1,C6),(A59,b1,C7),(A 59,b1,C8)(A59,b1,C9),(A59,b1,C10),(A59,b1,C11),(A59,b1,C12),(A59,b1,C13),(A59,b1,C14) ,(A59,b1,C15),(A59,b1,C16),(A59,b1,C17),(A59,b1,C18),(A59,b1,C19),(A59,b1,C20),(A59,b1 ,C21),(A59,b1,C22),(A59,b1,C23),(A59,b1,C24),(A59,b1,C25),(A59,b1,C26),(A59,b1,C27),(A 59,b1,C28),(A59,b1,C29),(A59,b2,C1),(A59,b2,C2),(A59,b2,C3),(A59,b2,C4),(A59,b2,C5),(A 59,b2,C6),(A59,b2,C7),(A59,b2,C8),(A59,b2,C9),(A59,b2,C10),(A59,b2,C11),(A59,b2,C12),( A59,b2,C13),(A59,b2,C14),(A59,b2,C15),(A59,b2,C16),(A59,b2,C17),(A59,b2,C18),(A59,b2, C19),(A59,b2,C20),(A59,b2,C21),(A59,b2,C22),(A59,b2,C23),(A59,b2,C24),(A59,b2,C25),(A 59,b2,C26),(A59,b2,C27),(A59,b2,C28),(A59,b2,C29),(A59,b3,C1),(A59,b3,C2),(A59,b3,C3),( A59,b3,C4),(A59,b3,C5),(A59,b3,C6),(A59,b3,C7),(A59,b3,C8),(A59,b3,C9),(A59,b3,C10),(A 59,b3,C11),(A59,b3,C12),(A59,b3,C13),(A59,b3,C14),(A59,b3,C15),(A59,b3,C16),(A59,b3,C 17),(A59,b3,C18),(A59,b3,C19),(A59,b3,C20),(A59,b3,C21),(A59,b3,C22),(A59,b3,C23),(A59 ,b3,C24),(A59,b3,C25),(A59,b3,C26),(A59,b3,C27),(A59,b3,C28),(A59,b3,C29),(A59,b4,C1), A59,b4,C2),(A59,b4,C3),(A59,b4,C4),(A59,b4,C5),(A59,b4,C6),(A59,b4,C7),(A59,b4,C8),(A5 9,b4,C9),(A59,b4,C10),(A59,b4,C11),(A59,b4,C12),(A59,b4,C13),(A59,b4,C14),(A59,b4,C15) ,(A59,b4,C16),(A59,b4,C17),(A59,b4,C18),(A59,b4,C19),(A59,b4,C20),(A59,b4,C21),(A59,b4 ,C22),(A59,b4,C23),(A59,b4,C24),(A59,b4,C25),(A59,b4,C26),(A59,b4,C27),(A59,b4,C28),(A 59,b4,C29),(A59,b5,C1),(A59,b5,C2),(A59,b5,C3),(A59,b5,C4),(A59,b5,C5),(A59,b5,C6),(A5 9,b5,C7),(A59,b5,C8),(A59,b5,C9),(A59,b5,C10),(A59,b5,C11),(A59,b5,C12),(A50,b5,C13),( A59,b5,C14),(A59,b5,C15),(A59,b5,C16),(A59,b5,C17),(A59,b5,C18),(A59,b5,C19),(A59,b5, C20),(A59,b5,C21),(A59,b5,C22),(A59,b5,C23),(A59,b5,C24),(A59,b5,C25),(A59,b5,C26),(A 59,b5,C27),(A59,b5,C28),(A59,b5,C29),(A59,b6,C1),(A59,b6,C2),(A59,b6,C3),(A59,b6,C4), A59,b6,C5),(A59,b6,C6),(A59,b6,C7),(A59,b6,C8),(A59,b6,C9),(A59,b6,C10),(A59,b6,C11),( A59,b6,C12),(A59,b6,C13),(A59,b6,C14),(A59,b6,C15),(A59,b6,C16),(A59,b6,C17),(A59,b6, C18),(A59,b6,C19),(A59,b6,C20),(A59,b6,C21),(A59,b6,C22),(A59,b6,C23),(A59,b6,C24),(A 59,b6,C25),(A59,b6,C26),(A59,b6,C27),(A59,b6,C28),(A59,b6,C29),(A59,b7,C1),(A59,b^r,C2) ,(A59,b7,C3),(A59,b7,C4),(A59,b7,C5),(A59,b7,C6),(A59,b7,C7),(A59,b7,C8),(A59,b7,C9),(A 59,b7,C10),(A59,b 7,C11),(A59,b7,C12),(A59,b7,C13),(A59,b7,C14),(A59,b7,C15),(A59,b7,C 16),(A59,b7,C17),(A59,b7,C18),(A59,b7,C19),(A59,b7,C20),(A59,b7,C21),(A59,b7,C22),(A59 ,b7,C23),(A59,b7,C24),(A59,b7,C25),(A59,b7,C26),(A59,b7,C27),(A59,b7,C28),(A59,b7,C29) ,(A60,b1,C1),(A60,b1,C2),(A60,b1,C3),(A60,b1,C4),(A60,b1,C5),(A60,b1,C6),(A60,b1,C7),(A 60,b1,C8),(A60,b1,C9),(A60,b1,C10),(A60,b1,C11),(A60,b1,C12),(A60,b1,C13),(A60,b1,C14) ,(A60,b1,C15),(A60,b1,C16),(A60,b1,C17),(A60,b1,C 8),(A60,b1,C19),(A60,b1,C20),(A60,b1 ,C21),(A60,b1,C22),(A60,b1,C23),(A60,b1,C24),(A60,b1,C25),(A60,b1,C26),(A60,b1,C27),(A 60,b1,C28),(A60,b1,C29),(A60,b2,C1),(A60,b2,C2),(A60,b2,C3),(A60,b2,C4),(A60,b2,C5),(A 60,b2,C6),(A60,b2,C7),(A60,b2,C8),(A60,b2,C9),(A60,b2,C10),(A60,b2,C11),(A60,b2,C12), A60,b2,C13),(A60,b2,C14),(A60,b2,C15),(A60,b2,C16),(A60,b2,C17),(A60,b2,C18),(A60,b2, C19),(A60,b2,C20),(A60,b2,C21),(A60,b2,C22),(A60,b2,C23),(A60,b2,C24),(A60,b2,C25),(A 60,b2,C26),(A60,b2,C27),(A60,b2,C28),(A60,b2,C29),(A60,b3,C1),(A60,b3,C2),(A60,b3,C3),( A60,b3,C4),(A60,b3,C5),(A60,b3,C6),(A60,b3,C7),(A60,b3,C8),(A60,b3,C9),(A60,b3,C10),(A 60,b3,C1),(A60,b3,C12),(A60,b3,C13),(A60,b3,C14),(A60,b3,C15),(A60,b3,C16),(A60,b3,C 17),(A60,b3,C18),(A60,b3,C19),(A60,b3,C20),(A60,b3,C21),(A60,b3,C22),(A60,b3,C23),(A60 ,b3,C24),(A60,b3,C25),(A60,b3,C26),(A60,b3,C27),(A60,b3,C28),(A60,b3,C29),(A60,b4,C1),( A60,b4,C2),(A60,b4,C3),(A60,b4,C4))(A60,b4,C5),(A60,b4,C6),(A60,b4,C7),(A60,b4,C8),(A6 0,b4,C9),(A60,b4,C10),(A60,b4,C11),(A60,b4,C12),(A60,b4,C13),(A60,b4,C14),(A60,b4,C15) ,(A60,b4,C16),(A60,b4,C17),(A60,b4,C18),(A60,b4,C19),(A60,b4,C20),(A60,b4,C21),(A60,b4 ,C22),(A60,b4,C23),(A60,b4,C24),(A60,b4,C25),(A60,b4,C26),(A60,b4,C27),(A60,b4,C28),(A 60,b4,C29),(A60,b5,C1),(A60,b5,C2),(A60,b5,C3),(A60,b5,C4),(A60,b5,C5),(A60,b5,C6),(A6 0,b5,C7),(A60,b5,C8),(A60,b5,C9),(A60,b5,C10),(A60,b5,C11),(A60,b5,C12),(A60,b5,C13), A60,b5,C14),(A60,b5,C15),(A60,b5,C16),(A60,b5,C17),(A60,b5,C18),(A60,b5,C19),(A60,b5, C20),(A60,b5,C21),(A60,b5,C22),(A60,b5,C23),(A60,b5,C24),(A60,b5,C25),(A60,b5,C26),(A 60,b5,C27),(A60,b5,C28),(A60,b5,C29),(A60,b6,C1),(A60,b6,C2),(A60,b6,C3),(A60,b6,C4),( A60,b6,C5),(A60,b6,C6),(A60,b6,C7),(A60,b6,C8),(A60,b6,C9),(A60,b6,C10),(A60,b6,C11),( A60,b6,C12),(A60,b6,C13),(A60,b6,C14),(A60,b6,C15),(A60,b6,C16},(A60,b6,C17),(A60,b6, C18),(A60,b6,C19),(A60,b6,C20),(A60,b6,C21),(A60,b6,C22),(A60,b6,C23),(A60,b6,C24),(A 60,b6,C25),(A60,b6,C26),(A60,b6,C27),(A60,b6,C28),(A60,b6,C29),(A60,b7,C1),(A60,b7,C2) ,(A60,b7,C3),(A60,b7,C4),(A60,b7,C5),(A60,b7,C6),(A60,b7,C7),(A60,b7,C8),(A60,b7,C9),(A 60,b 7,C10),(A60,b7,C11),(A60,b7,C12),(A60,b7,C13),(A60,b7,C14),(A60,b7,C15),(A60,b7,C 16),(A60,b7,C17),(A60,b7,C18),(A60,b7,C19),(A60,b7,C20),(A60,b7,C21),(A60,b7,C22),(A60 ,b7,C23),(A60,b7,C24),(A60,b7,C25),(A60,b7,C26),(A60,b7,C27),(A60,b7,C28),(A60,b7,C29),

(A61,b1,C1),(A61,b1,C2),(A61,b1,C3),(A61,b1,C4),(A61,b1,C5),(A61,b1,C6),(A61,b1,C7),(A 61,b1,C8),(A61,b1,C9),(A61,b1,C10),(A61,b1,C11),(A61,b1,C12),(A61,b1,C13),(A61,b1,C14) (A61,b1,C15),(A61,b1,C16),(A61,b1,C17),(A61,b1,C18),(A61,b1,C19),(A61,b1,C20),(A61,b1 ,C21),(A61,b1,C22),(A61,b1,C23),(A61,b1,C24),(A61,b1,C25),(A61,b1,C26),(A61,b1,C27),(A 61,b1,C28),(A61,b1,C29),(A61,b2,C1),(A61,b2,C2),(A61,b2,C3),(A61,b2,C4),(A61,b2,C5),(A 61,b2,C6),(A61,b2,C7),(A61,b2,C8),(A61,b2,C9),(A61,b2,C10),(A61,b2,C11),(A61,b2,C12),( A61,b2,C13),(A61,b2,C14),(A61,b2,C15),(A61,b2,C16},(A61,b2,C17),(A61,b2,C18),(A61,b2, C19),(A61,b2,C20),(A61,b2,C21),(A61,b2,C22),(A61,b2,C23),(A61,b2,C24),(A61,b2,C25),(A 61,b2,C26),(A61,b2,C27),(A61,b2,C28),(A61,b2,C29),(A61,b3,C1),(A61,b3,C2),(A61,b3,C3),( A61,b3,C4),(A61,b3,C5),(A61,b3,C6),(A61,b3,C7),(A61,b3,C8),(A61,b3,C9),(A61,b3,C10),(A 61,b3,C11),(A61,b3,C12),(A61,b3,C13),(A61,b3,C14),(A61,b3,C15),(A61,b3,C16),(A61,b3,C 17),(A61,b3,C18),(A61,b3,C19),(A61,b3,C20),(A61,b3,C21),(A61,b3,C22),(A61,b3,C23),(A61 ,b3,C24),(A61,b3,C25),(A61,b3,C26),(A61,b3,C27),(A61,b3,C28),(A61,b3,C29),(A61,b4,C1),( A61,b4,C2),(A61,b4,C3),(A61,b4,C4),(A61,b4,C5),(A61,b4,C6),(A61,b4,C7),(A61,b4,C8),(A6 1,b4,C9),(A61,b4,C10),(A61,b4,C11),(A61,b4,C12),(A61,b4,C13),(A61,b4,C14),(A61,b4,C15) ,(A61,b4,C16),(A61,b4,C17),(A61,b4,C18),(A61,b4,C19),(A61,b4,C20),(A61,b4,C21),(A61,b4 ,C22),(A61,b4,C23),(A61,b4,C24),(A61,b4,C25),(A61,b4,C26),(A61,b4,C27),(A61,b4,C28),(A 61,b4,C29),(A61,b5,C1),(A61,b5,C2),(A61,b5,C3),(A61,b5,C4),(A61,b5,C5),(A61,b5,C6),(A6 1,b5,C7),(A61,b5,C8),(A61,b5,C9),(A61,b5,C10),(A61,b5,C11),(A61,b5,C12),(A61,b5,C13),( A61,b5,C14),(A61,b5,C15),(A61,b5,C16),(A61,b5,C17),(A61,b5,C18),(A61,b5,C19),(A61,b5, C20),(A61,b5,C21),(A61,b5,C22),(A61,b5,C23),(A61,b5,C24),(A61,b5,C25),(A61,b5,C26),(A 61,b5,C27),(A61,b5,C28),(A61,b5,C29),(A61,b6,C1),(A61,b6,C2),(A61,b6,C3),(A61,b6,C4),( A61,b6,C5),(A61,b6,C6),(A61,b6,C11),(A61,b6,C8),(A61,b6,C9),(A61,b6,C10),(A61,b6,C11),( A61,b6,C12),(A61,b6,C13),(A61,b6,C14),(A61,b6,C15),(A61,b6,C16),(A61,b6,C17),(A61,b6, C18),(A61,b6,C19),(A61,b6,C20),(A61,b6,C21),(A61,b6,C22),(A61,b6,C23),(A61,b6,C24),(A 61,b6,C25),(A61,b6,C26),(A61,b6,C27),(A61,b6,C28),(A61,b6,C29),(A61,b7,C1),(A61,b7,C2) ,(A61,b7,C3),(A61,b1,C4),(A61,b7,C5),(A61,b7,C6),(A61,b7,C7),(A61,b7,C8),(A61,b7,C9),(A 61,b7,C10),(A61,b7,C11),(A61,b7,C12),(A61,b7,C13),(A61,b7,C14),(A61,b7,C15),(A61,b7,C 16),(A61,b7,C17),(A61,b7,C18),(A61,b7,C9),(A61,b7,C20),(A61,b7,C21),(A61,b7,C22),(A61 ,b7,C23),(A61,b7,C24),(A61,b7,C25),(A61,b7,C26),(A61,br,C27),(A61,b7,C28),(A61,b7,C29) ,(A62,b1,C1),(A62,b1,C2),(A62,b1,C3),(A62,b1,C4),(A62,b1,C5),(A62,b1,C6),(A62,b1,C7),(A 62,b1,C8),(A62,b1,C9),(A62,b1,C10),(A62,b1,C11),(A62,b1,C12),(A62,b1,C13),(A62,b1,C14) ,(A62,b1,C15),(A62,b1,C16),(A62,b1,C17),(A62,b1,C18),(A62,b1,C19),(A62,b1,C20),(A62,b1 ,C21),(A62,b1,C22),(A62,b1,C23),(A62,b1,C24),(A62,b1,C25),(A62,b1,C26),(A62,b1,C27),(A 62,b1,C28),(A62,b1,C29),(A62,b2,C1),(A62,b2,C2),(A62,b2,C3),(A62,b2,C4),(A62,b2,C5),(A 62,b2,C6),(A62,b2,C'7),(A62,b2,C8),(A62,b2,C9),(A62,b2,C10),(A62,b2,C11),(A62,b2,C12), A62,b2,C13),(A62,b2,C14),(A62,b2,C15),(A62,b2,C16),(A62,b2,C17),(A62,b2,C18),(A62,b2, C19),(A62,b2,C20),(A62,b2,C21),(A62,b2,C22),(A62,b2,C23),(A62,b2,C24),(A62,b2,C25),(A 62,b2,C26),(A62,b2,C27),(A62,b2,C28),(A62,b2,C29),(A62,b3,C1),(A62,b3,C2),(A62,b3,C3),( A62,b3,C4),(A62,b3,C5),(A62,b3,C6),(A62,b3,C7),(A62,b3,C8),(A62,b3,C9),(A62,b3,C10),(A 62,b3,C11),(A62,b3,C12),(A62,b3,C13),(A62,b3,C14),(A62,b3,C15),(A62,b3,C16),(A62,b3,C 17),(A62,b3,C18),(A62,b3,C19),(A62,b3,C20),(A62,b3,C21),(A62,b3,C22),(A62,b3,C23),(A62 ,b3,C24),(A62,b3,C25),(A62,b3,C26),(A62,b3,C27),(A62,b3,C28),(A62,b3,C29),(A62,b4,C1),( A62,b4,C2),(A62,b4,C3),(A62,b4,C4),(A62,b4,C5),(A62,b4,C6),(A62,b4,C7),(A62,b4,C8),(A6 2,b4,C9),(A62,b4,C10),(A62,b4,C11),(A62,b4,C12),(A62,b4,C13),(A62,b4,C14),(A62,b4,C15) ,(A62,b4,C16),(A62,b4,C17),(A62,b4,C18),(A62,b4,C19),(A62,b4,C20),(A62,b4,C21),(A62,b4 ,C22),(A62,b4,C23),(A62,b4,C24),(A62,b4,C25),(A62,b4,C26),(A62,b4,C27),(A62,b4,C28),(A 62,b4,C29),(A62,b5,C1),(A62,b5,C2),(A62,b5,C3),(A62,b5,C4),(A62,b5,C5),(A62,b5,C6),(A6 2,b5,C7),(A62,b5,C8),(A62,b5,C9),(A62,b5,C10),(A62,b5,C11),(A62,b5,C12),(A62,b5,C13),( A62,b5,C14),(A62,b5,C15),(A62,b5,C16),(A62,b5,C17),(A62,b5,C18),(A62,b5,C19),(A62,b 5, C20),(A62,b5,C21),(A62,b5,C22),(A62,b5,C23),(A62,b5,C24),(A62,b5,C25),(A62,b5,C26),(A 62,b5,C27),(A62,b5,C28),(A62,b5,C29),(A62,b6,C1),(A62,b6,C2),(A62,b6,C3),(A62,b6,C4),( A62,b6,C5),(A62,b6,C6),(A62,b6,C7),(A62,b6,C8),(A62,b6,C9),(A62,b6,C10),(A62,b6,C11),( A62,b6,C12),(A62,b6,C13),(A62,b6,C14),(A62,b6,C15),(A62,b6,C16),(A62,b6,C17),(A62,b6, C18),(A62,b6,C19),(A62,b6,C20),(A62,b6,C21),(A62,b6,C22),(A62,b6,C23),(A62,b6,C24),(a 62,b6,C25),(A62,b6,C26),(A62,b6,C27),(A62,b6,C28),(A62,b6,C29),(A62,b7,C1),(A62,b7,C2) ,(A62,b7,C3),(A62,b7,C4),(A62,b7,C5),(A62,b7,C6),(A62,b7,C7),(A62,b7,C8),(A62,b7,C9),(A 62,b7,C10),(A62,b7,C11),(A62,b7,C12),(A62,b7,C13),(A62,b7,C14),(A62,b7,C15),(A62,b7,C 16),(A62,b7,C17),(A62,b7,C18),(A62,b7,C19),(A62,b7,C20),(A62,b7,C21),(A62,b7,C22),(A62 ,b7,C23),(A62,b7,C24),(A62,b7,C25),(A62,b7,C26),(A62,b7,C27),(A62,b7,C28),(A62,b7,C29) ,(A63,b1,C1),(A63,b1,C2),(A63,b1,C3),(A63,b1,C4),(A63,b1,C5),(A63,b1,C6),(A63,b1,C7),(A 63,b1,C8),(A63,b1,C9),(A63,b1,C10),(A63,b1,C11),(A63,b1,C12),(A63,b1,C13),(A63,b1,C14) ,(A63,b1,C15),(A63,b1,C16),(A63,b1,C17),(A63,b1,C18),(A63,b1,C19),(A63,b1,C20),(A63,b1 ,C21),(A63,b1,C22),(A63,b1,C23),(A63,b1,C24))(A63,b1,C25),(A63,b1,C26),(A63,b1,C27),(A 63,b1,C28),(A63,b1,C29),(A63,b2,C1),(A63,b2,C2),(A63,b2,C3),(A63,b2,C4),(A63,b2,C5),(A 63,b2,C6),(A63,b2,C7),(A63,b2,C8),(A63,b2,C9),(A63,b2,C10),(A63,b2,C11),(A63,b2,C12),( A63,b2,C13),(A63,b2,C14),(A63,b2,C15),(A63,b2,C16),(A63,b2,C17),(A63,b2,C18),(A63,b2, C19),(A63,b2,C20),(A63,b2,C21),(A63,b2,C22),(A63,b2,C23),(A63,b2,C24),(A63,b2,C25),(A 63,b2,C26),(A63,b2,C27),(A63,b2,C28),(A63,b2,C29),(A63,b3,C1),(A63,b3,C2),(A63,b3,C3),( A63,b3,C4),(A63,b3,C5),(A63,b3,C6),(A63,b3,C7),(A63,b3,C8),(A63,b3,C9),(A63,b3,C10),(A 63,b3,C11),(A63,b3,C12),(A63,b3,C13),(A63,b3,C14),(A63,b3,C15),(A63,b3,C16),(A63,b3,C 17),(A63,b3,C18),(A63,b3,C19),(A63,b3,C20),(A63,b3,C21),(A63,b3,C22),(A63,b3,C23),(A63 ,b3,C24),(A63,b3,C25),(A63,b3,C26),(A63,b3,C27),(A63,b3,C28),(A63,b3,C29),(A63,b4,C1),(( A63,b4,C2),(A63,b4,C3),(A63,b4,C4),(A63,b4,C5),(A63,b4,C6),(A63,b4,C7),(A63,b4,C8),(A6 3,b4,C9),(A63,b4,C10),(A63,b4,C11),(A63,b4,C12),(A63,b4,C13),(A63,b4,C14),(A63,b4,C15) ,(A63,b4,C16),(A63,b4,C17),(A63,b4,C18),(A63,b4,C19),(A63,b4,C20),(A63,b4,C21),(A63,b4 ,C22),(A63,b4,C23),(A63,b4,C24),(A63,b4,C25),(A63,b4,C26),(A63,b4,C27),(A63,b4,C28),(A 63,b4,C29),(A63,b5,C1),(A63,b5,C2),(A63,b5,C3),(A63,b5,C4),(A63,b5,C5),(A63,b5,C6),(A6 3,b5,C7),(A63,b5,C8),(A63,b5,C9),(A63,b5,C10),(A63,b5,C11),(A63,b5,C12),(A63,b5,C13),( A63,b5,C14),(A63,b5,C15),(A63,b5,C16),(A63,b5,C17),(A63,b5,C18),(A63,b5,C1),(A63,b5, C20),(A63,b5,C21),(A63,b5,C22),(A63,b5,C23),(A63,b5,C24),(A63,b5,C25),(A63,b5,C26),(A 63,b5,C27),(A63,b5,C28),(A63,b5,C29),(A63,b6,C1),(A63,b6,C2),(A63,b6,C3),(A63,b6,C4),( A63,b6,C5),(A63,b6,C6),(A63,b6,C7),(A63,b6,C8),(A63,b6,C9),(A63,b6,C10),(A63,b6,C11),( A63,b6,C12),(A63,b6,C13),(A63,b6,C14),(A63,b6,C15),(A63,b6,C16),(A63,b6,C17),(A63,b6, C18),(A63,b6,C19),(A63,b6,C20),(A63,b6,C21),(A63,b6,C22),(A63,b6,C23),(A63,b6,C24),(A 63,b6,C25),(A63,b6,C26),(A63,b6,C27),(A63,b6,C28),(A63,b6,C29),(A63,b7,C1),(A63,b7,C2) ,(A63,b7,C3),(A63,b7,C4),(A63,b7,C5),(A63,b7,C6),(A63,b7,C7),(A63,b7,C8),(A63,b7,C9),(A 63,b7,C10),(A63,b7,C11),(A63,b7,C12),(A63,b7,C13),(A63,b7,C14),(A63,b7,C15),(A63,b7,C 16),(A63,b7,C17),(A63,b7,C18),(A63,b7,C19),(A63,b7,C20),(A63,b7,C21),(A63,b7,C22),(A63 ,b7,C23),(A63,b7,C24),(A63,b7,C25),(A63,b7,C26)_{;}(A63,b7,C27),(A63,b7,C28),(A63,b7,C29) ,(A64,b1,C),(A64,b1,C2),(A64,b1,C3),(A64,b1,C4),(A64,b1,C5),(A64,b1,C6),(A64,b1,C7),(A 64,b1,C8),(A64,b1,C9),(A64,b1,C10),(A64,b1,C11),(A64,b1,C12),(A64,b1,C13),(A64,b1,C14) ,(A64,b1,C15),(A64,b1,C16),(A64,b1,C17),(A64,b1,C18),(A64,b1,C19),(A64,b1,C20),(A64,b1 ,C21),(A64,b1,C22),(A64,b1,C23),(A64,b1,C24),(A64,b1,C25),(A64,b1,C26),(A64,b1,C27),(A 64,b1,C28),(A64,b1,C29),(A64,b2,C1),(A64,b2,C2),(A64,b2,C3),(A64,b2,C4),(A64,b2,C5),(A 64,b2,C6),(A64,b2,C7),(A64,b2,C8),(A64,b2,C9),(A64,b2,C10),(A64,b2,C11),(A64,b2,C12),( A64,b2,C13),(A64,b2,C14),(A64,b2,C15),(A64,b2,C16),(A64,b2,C17),(A64,b2,C18),(A64,b2, C19),(A64,b2,C20),(A64,b2,C21),(A64,b2,C22),(A64,b2,C23),(A64,b2,C24),(A64,b2,C25),(A 64,b2,C26),(A64,b2,C27),(A64,b2,C28),(A64,b2,C29),(A64,b3,C1),(A64,b3,C2),(A64,b3,C3),( A64,b3,C4),(A64,b3,C5),(A64,b3,C6),(A64,b3,C7),(A64,b3,C8),(A64,b3,C9),(A64,b3,C10),(A 64,b3,C11),(A64,b3,C12),(A64,b3,C13),(A64,b3,C14),(A64,b3,C15),(A64,b3,C16),(A64,b3,C 17),(A64,b3,C18),(A64,b3,C19),(A64,b3,C20),(A64,b3,C21),(A64,b3,C22),(A64,b3,C23),(A64 ,b3,C24),(A64,b3,C25),(A64,b3,C26),(A64,b3,C27),(A64,b3,C28),(A64,b3,C29),(A64,b4,C1),( A64,b4,C2),(A64,b4,C3),(A64,b4,C4),(A64,b4,C5),(A64,b4,C6),(A64,b4,C7),(A64,b4,C8),(A6 4,b4,C9),(A64,b4,C10),(A64,b4,C11),(A64,b4,C12),(A64,b4,C13),(A64,b4,C14),(A64,b4,C15) ,(A64,b4,C16),(A64,b4,C17),(A64,b4,C18),(A64,b4,C19),(A64,b4,C20),(A64,b4,C21),(A64,b4 ,C22),(A64,b4,C23),(A64,b4,C24),(A64,b4,C25),(A64,b4,C26),(A64,b4,C27),(A64,b4,C28),(A 64,b4,C29),(A64,b5,C1),(A64,b5,C2),(A64,b5,C3),(A64,b5,C4),(A64,b5,C5),(A64,b5,C6),(A6 4,b5,C7),(A64,b5,C8),(A64,b5,C9),(A64,b5,C10),(A64,b5,C11),(A64,b5,C12),(A64,b5,C13),( A64,b5,C14),(A64,b5,C15),(A64,b5,C16),(A64,b5,C17),(A64,b5,C18),(A64,b5,C19),(A64,b5, C20),(A64,b5,C21),(A64,b5,C22),(A64,b5,C23),(A64,b5,C24),(A64,b5,C25),(A64,b5,C26),(A 64,b5,C27),(A64,b5,C28),(A64,b5,C29),(A64,b6,C1),(A64,b6,C2),(A64,b6,C3),(A64,b6,C4),( A64,b6,C5),(A64,b6,C6),(A64,b6,C7),(A64,b6,C8),(A64,b6,C9),(A64,b6,C10),(A64,b6,C11),( A64,b6,C12),(A64,b6,C13),(A64,b6,C14),(A64,b6,C15),(A64,b6,C16),(A64,b6,C17),(A64,b6, C18),(A64,b6,C19),(A64,b6,C20),(A64,b6,C21),(A64,b6,C22),(A64,b6,C23),(A64,b6,C24),(A 64,b6,C25),(A64,b6,C26),(A64,b6,C27),(A64,b6,C28),(A64,b6,C29),(A64,b7,C1),(A64,b7,C2) ,(A64,b7,C3),(A64,b7,C4),(A64,b7,C5),(A64,b7,C6),(A64,b7,C7),(A64,b7,C8),(A64,b7,C9),(A 64,b7,C10),(A64,b7,C11),(A64,b7,C12),(A64,b7,C13),(A64,b7,C14),(A64,b7,C15),(A64,b7,C 16),(A64,b7,C17),(A64,b7,C18),(A64,b7,C19),(A64,b7,C20),(A64,b7,C21),(A64,b7,C22),(A64 ,b7,C23),(A64,b7,C24),(A64,b7,C25),(A64,b7,C26),(A64,b7,C27),(A64,b7,C28),(A64,b7,C29) ,(A65,b1,C1),(A65,b1,C2),(A65,b1,C3),(A65,b1,C4),(A65,b1,C5),(A65,b1,C6),(A65,b1,C7),(A 65,b1,C8),(A65,b1,C9),(A65,b1,C10),(A65,b1,C11),(A65,b1,C12),(A65,b1,C113),(A65,b1,C14) ,(A65,b1,C15),(A65,b1,C16),(A65,b1,C17),(A65,b1,C18),(A65,b1,C19),(A65,b1,C20),(A65,b ,C21),(A65,b1,C22),(A65,b1,C23),(A65,b1,C24),(A65,b1,C25),(A65,b1,C26),(A65,b1,C27),(A 65,b1,C28),(A65,b1,C29),(A65,b2,C1),(A65,b2,C2),(A65,b2,C3),(A65,b2,C4),(A65,b2,C5),(A 65,b2,C6),(A65,b2,C7),(A65,b2,C8),(A65,b2,C9),(A65,b2,C10),(A65,b2,C11),(A65,b2,C12),( A65,b2,C13),(A65,b2,C14),(A65,b2,C15),(A65,b2,C16),(A65,b2,C17),(A65,b2,C18),(A65,b2, C19),(A65,b2,C20),(A65,b2,C21),(A65,b2,C22),(A65,b2,C23),(A65,b2,C24),(A65,b2,C25),(A 65,b2,C26),(A65,b2,C27),(A66,b2,C28),(A65,b2,C29),(A65,b3,C1),(A65,b3,C2),(A65,b3,C3),( A65,b3,C4),(A65,b3,C5),(A65,b3,C6),(A65,b3,C7),(A65,b3,C8),(A65,b3,C9),(A65,b3,C10),(A 65,b3,C11),(A65,b3,C12),(A65,b3,C13),(A65,b3,C14),(A65,b3,C15),(A65,b3,C16),(A65,b3,C 17),(A65,b3,C18),(A65,b3,C19),(A65,b3,C20),(A65,b3,C21),(A65,b3,C22),(A65,b3,C23),(A65 ,b3,C24),(A65,b3,C25),(A65,b3,C26),(A65,b3,C27),(A65,b3,C28),(A65,b3,C29),(A65,b4,C1),( A65,b4,C2),(A65,b4,C3),(A65,b4,C4),(A65,b4,C5),(A65,b4,C6),(A65_{;}b4,C7),(A65,b4,C8),(A6 5,b4,C9),(A65,b4,C10),(A65,b4,C11),(A65,b4,C12),(A65,b4,C13),(A65,b4,C14),(A65,b4,C15) ,(A65,b4,C16),(A65,b4,C17),(A65,b4,C18),(A65,b4,C19),(A65,b4,C20),(A65,b4,C21),(A65,b4 ,C22),(A65,b4,C23),(A65,b4,C24),(A65,b4,C25),(A65,b4,C26),(A65,b4,C27),(A65,b4,C28),(A 65,b4,C29),(A65,b5,C1),(A65,b5,C2),(A65,b5,C3),(A65,b5,C4),(A65,b5,C5),(A65,b5,C6),(A6 5,b5,C7),(A65,b5,C8),(A65,b5,C9),(A65,b5,C10),(A65,b5,C11),(A65,b5,C12),(A65,b5,C13),( A65,b5,C14),(A65,b5,C15),(A65,b5,C16),(A65,b5,C17),(A65,b5,C18),(A65,b5,C19),(A65,b5_{;} C20),(A65,b5,C21),(A65,b5,C22),(A65,b5,C23),(A65,b5,C24),(A65,b5,C25),(A65,b5,C26),(A 65,b5,C27),(A65,b5,C28),(A65,b5,C29),(A65,b6,C1),(A65,b6,C2),(A65,b6,C3),(A65,b6,C4),( A65,b6,C5),(A65,b6,C6),(A65,b6,C7),(A65,b6,C8),(A65,b6,C9),(A65,b6,C10),(A65,b6,C11),( A65,b6,C12),(A65,b6,C13),(A65,b6,C14),(A65,b6,C15),(A65,b6,C16),(A65,b6,C17),(A65,b6, C18),(A65,b6,C19),(A65,b6,C20),(A65,b6,C21),(A65,b6,C22),(A65,b6,C23),(A65,b6,C24),(A 65,b6,C25),(A65,b6,C26),(A65,b6,C27),(A65,b6,C28),(A65,b6,C29),(A65,b7,C1),(A65,b7,C2) ,(A65,b7,C3),(A65,b7,C4),(A65,b7,C5),(A65,b7,C6),(A65,b7,C7),(A65,b7,C8),(A65,b7,C9),(A 65,b7,C10),(A65,b7,C11),(A65,b7,C12),(A65,b7,C13),(A65,b7,C14),(A65,b7,C15),(A65,b7,C 16),(A65,b7,C17),(A65,b7,C18),(A65,b7,C19),(A65,b7,C20),(A65,b7,C21),(A65,b7,C22),(A65 ,b7,C23),(A65,b7,C24),(A65,b7,C25),(A65,b7,C26),(A65,b7,C27),(A65,b7,C28),(A65,b7,C29) ,(A66,b1,C1),(A66,b1,C2),(A66,b1,C3),(A66,b1,C4),(A66,b1,C5),(A66,b1,C6),(A66,b1,C7),(A 66,b1,C8),(A66,b1,C9),(A66,b1,C10),(A66,b1,C11),(A66,b1,C12),(A66,b1,C13),(A66,b1,C14) ,(A66,b1,C15),(A66,b1,C16),(A66,b1,C17),(A66,b1,CIS),(A66,b1,C19),(A66,b1,C20),(A66,b1 ,C21),(A66,b1,C22),(A66,b1,C23),(A66,b1,C24),(A66,b1,C25),(A66,b1,C26),(A66,b1,C27),(A 66,b1,C28),(A66,b1,C29),(A66,b2,C1),(A66,b2,C2),(A66,b2,C3),(A66,b2,C4),(A66,b2,C5),(A 66,b2,C6),(A66,b2,C7),(A66,b2,C8),(A66,b2,C9),(A66,b2,C10),(A66,b2,C11),(A66,b2,C12),( A66,b2,C13),(A66,b2,C14),(A66,b2,C15),(A66,b2,C16),(A66,b2,C17),(A66,b2,C18),(A66,b2, C19),(A66,b2,C20),(A66,b2,C21),(A66,b2,C22),(A66,b2,C23),(A66,b2,C24),(A66,b2,C25),(A 66,b2,C26),(A66,b2,C27),(A66,b2,C28),(A66,b2,C29),(A66,b3,C1),(A66,b3,C2),(A66,b3,C3),( A66,b3,C4),(A66,b3,C5),(A66,b3,C6),(A66,b3,C7),(A66,b3,C8),(A66,b3,C9),(A66,b3,C10),(A 66,b3,C11),(A66,b3,C12),(A66,b3,C13),(A66,b3,C14),(A66,b3,C15),(A66,b3,C16),(A66,b3,C 17),(A66,b3,C18),(A66,b3,C19),(A66,b3,C20),(A66,b3,C21),(A66,b3,C22),(A66,b3,C23),(A66 ,b3,C24),(A66,b3,C25),(A66,b3,C26),(A66,b3,C27),(A66,b3,C28),(A66,b3,C29),(A66,b4,C1),( A66,b4,C2),(A66,b4,C3),(A66,b4,C4),(A66,b4,C5),(A66,b4,C6),(A66,b4,C7),(A66,b4,C8),(A6 6,b4,C9),(A66,b4,C10),(A66,b4,C11),(A66,b4,C12),(A66,b4,C13),(A66,b4,C14),(A66,b4,C15) ,(A66,b4,C16),(A66,b4,C17),(A66,b4,C18),(A66,b4,C19),(A66,b4,C20),(A66,b4,C21),(A66,b4 ,C22),(A66,b4,C23),(A66,b4,C24),(A66,b4,C25),(A66,b4,C26),(A66_{;}b4,C27),(A66,b4,C28),(A 66,b4,C29),(A66,b5,C1),(A66,b5,C2),(A66,b5,C3),(A66,b5,C4),(A66,b5,C5),(A66,b5,C6),(A6 6,b5,C7),(A66,b5,C8),(A66,b5,C9),(A66,b5,C10),(A66,b5,C11),(A66,b5,C12),(A66,b5,C13),( A66,b5,C14),(A66,b5,C15),(A66,b5,C16),(A66,b5,C17),(A66,b5,C18),(A66,b5,C19),(A66,b5, C20),(A66,b5,C21),(A66,b5,C22),(A66,b5,C23),(A66,b5,C24),(A66,b5,C25),(A66,b5,C26),(A 66,b5,C27),(A66,b5,C28),(A66,b5,C29),(A66,b6,C1),(A66,b6,C2),(A66,b6,C3),(A66,b6,C4),( A66,b6,C5),(A66,b6,C6),(A66,b6,C7),(A66,b6,C8),(A66,b6,C9),(A66,b6,C10),(A66,b6,C11),( A66,b6,C12),(A66,b6,C13),(A66,b6,C14),(A66,b6,C15),(A66,b6,C16),(A66,b6,C17),(A66,b6, C18),(A66,b6,C19),(A66,b6,C20),(A66,b6,C21),(A66,b6,C22),(A66,b6,C23),(A66,b6,C24),(A 66,b6,C25),(A66,b6,C26),(A66,b6,C27),(A66,b6,C28),(A66,b6,C29),(A66,b7,C1),(A66,b7,C2) ,(A66,b7,C3),(A66,b7,C4),(A66,b7,C5),(A66,b7,C6),(A66,b7,C7),(A66,b7,C8)_{;}(A66,b7,C9),(A 66,b7,C10),(A66,b7,C11),(A66,b7,C12),(A66,b7,C13),(A66,b7,C14),(A66,b7,C15),(A66,b7,C 16),(A66,b7,C17),(A66,b7,C18),(A66,b7,C19),(A66,b7,C20),(A66,b7,C21),(A66,b7,C22),(A66 ,b7,C23),(A66,b7,C24),(A66,b7,C25),(A66,b7,C26),(A66,b7,C27),(A66,b7,C28),(A66,b7,C29) ,(A67,b1,C1),(A67,b1,C2),(A67,b1,C3),(A67,b1,C4),(A67,b1,C5),(A67,b1,C6),(A67,b1,C7),(A 67,b1,C8),(A67,b1,C9),(A67,b1,C10),(A67,b1,C11),(A67,b1,C12),(A67,b1,C13),(A67,b1,C14) ,(A67,b1,C15),(A67,b1,C16),(A67,b1,C17),(A67,b1,C18),(A67,b1,C19),(A67,b1,C20),(A67,b1 ,C21),(A67,b1,C22),(A67,b1,C23),(A67,b1,C24),(A67,b1,C25),(A67,b1,C26),(A67,b1,C27),(A 67,b1,C28),(A67,b1,C29),(A67,b2,C1),(A67,b2,C2),(A67,b2,C3),(A67,b2,C4),(A67,b2,C5),(A 67,b2,C6),(A67,b2,C7),(A67,b2,C8),(A67,b2,C9),(A67,b2,C10),(A67,b2,C11),(A67,b2,C12),( A67,b2,C13),(A67,b2,C14),(A67,b2,C15),(A67,b2,C16),(A67,b2,C17),(A67,b2,C18),(A67,b2, C19),(A67,b2,C20),(A67,b2,C21),(A67,b2,C22),(A67,b2,C23),(A67,b2,C24),(A67,b2,C25),(A 67,b2,C26),(A67,b2,C27),(A67,b2,C28),(A67,b2,C29),(A67,b3,C1),(A67,b3,C2),(A67,b3,C3),( A67,b3,C4),(A67,b3,C5),(A67,b3,C6),(A67,b3,C7),(A67,b3,C8),(A67,b3,C9),(A67,b3,C10),(A 67,b3,C11),(A67,b3,C12),(A67,b3,C13),(A67,b3,C14),(A67,b3,C15),(A67,b3,C16),(A67,b3,C 17),(A67,b3,C18),(A67,b3,C19),(A67,b3,C20),(A67,b3,C21),(A67,b3,C22),(A67,b3,C23),(A67 ,b3,C24),(A67,b3,C25),(A67,b3,C26),(A67,b3,C27),(A67,b3,C28),(A67,b3,C29),(A67,b4,C1),( A67,b4,C2),(A67,b4,C3),(A67,b4,C4),(A67,b4,C5),(A67,b4,C6),(A67,b4,C7),(A67,b4,C8),(A6 7,b4,C9),(A6 7,b4,C10),(A67,b4,C11),(A67,b4,C12),(A67,b4,C13),(A67,b4,C14),(A67,b4,C15) ,(A67,b4,C16),(A67,b4,C17),(A67,b4,C18),(A67,b4,C19),(A67,b4,C20),(A67,b4,C21),(A67,b4 ,C22),(A67,b4,C23),(A67,b4,C24),(A67,b4,C25),(A67,b4,C26),(A67,b4,C27),(A67,b4,C28),(A 67,b4,C29),(A67,b5,C1),(A67,b5,C2),(A67,b5,C3),(A67,b5,C4),(A67,b5,C5),(A67,b5,C6),(A6 7,b5,C7),(A67,b5,C8),(A67,b5,C9),(A67,b5,C10),(A67,b5,C11),(A67,b5,C12),(A67,b5,C13),( A67,b5,C14),(A67,b5,C15),(A67,b5,C16),(A67,b5,C17),(A67,b5,C18),(A67,b5,C19),(A67,b5, C20),(A67,b5,C21),(A67,b5,C22),(A67,b5,C23),(A67,b5,C24),(A67,b5,C25),(A67,b5,C26),(A 67,b5,C27),(A67,b5,C28),(A67,b5,C29),(A67,b6,C1),(A67,b6,C2),(A67,b6,C3),(A67,b6,C4),( A67,b6,C5),(A67,b6,C6),(A67,b6,C7),(A67,b6,C8),(A67,b6,C9),(A67,b6,C10),(A67,b6,C11),( A67,b6,C12),(A67,b6,C13),(A67,b6,C14),(A67,b6,C15),(A67,b6,C16),(A67,b6,C17),(A67,b6, C18),(A67,b6,C19),(A67,b6,C20),(A67,b6,C21),(A67,b6,C22),(A67,b6,C23),(A67,b6,C24),(A 67,b6,C25),(A67,b6,C26),(A67,b6,C27),(A67,b6,C28),(A67,b6,C29),(A67,b7,C1),(A67,b7,C2) ,(A67,b7,C3),(A67,b7,C4),(A67,b7,C5),(A67,b7,C6),(A67,b7,C7),(A67,b7,C8),(A67,b7,C9),(A 67,b7,C10),(A67,b7,C11),(A67,b7,C12),(A67,b7,C13),(A67,b7,C14),(A67,b7,C15),(A67,b7,C 16),(A67,b7,C17),(A67,b7,C18),(A67,b7,C19),(A67,b7,C20),(A67,b7,C21),(A67,b7,C22),(A67 ,b7,C23),(A67,b7,C24),(A67,b7,C25),(A67,b7,C26),(A67,b7,C27),(A67,b7,C28),(A67,b7,C29) ,(A68,b1,C1),(A68,b1,C2),(A68,b1,C3),(A68,b1,C4),(A68,b1,C5),(A68,b1,C6),(A68,b1,C7),(A 68,b1,C8),(A68,b1,C9),(A68,b1,C10),(A68,b1,C11),(A68,b1,C12),(A68,b1,C13),(A68,b1,C14) ,(A68,b1,C15),(A68,b1,C16),(A68,b1,C17),(A68,b1,C18),(A68,b1,C19),(A68,b1,C20),(A68,b1 ,C21),(A68,b1,C22),(A68,b1,C23),(A68,b1,C24),(A68,b1,C25),(A68,b1,C26),(A68,b1,C27),(A 68,b1,C28),(A68,b1,C29),(A68,b2,C1),(A68,b2,C2),(A68,b2,C3)_{;}(A68,b2,C4),(A68,b2,C5),(A 68,b2,C6),(A68,b2,C7),(A68,b2,C8),(A68,b2,C9),(A68,b2,C10),(A68,b2,C11),(A68,b2,C12),( A68,b2,C13),(A68,b2,C14),(A68,b2,C15),(A68,b2,C16),(A68,b2,C17),(A68,b2,C18),(A68,b2, C19),(A68,b2,C20),(A68,b2,C21),(A68,b2,C22),(A68,b2,C23),(A68,b2,C24),(A68,b2,C25),(A 68,b2,C26),(A68,b2,C27),(A68,b2,C28),(A68,b2,C29),(A68,b3,C1),(A68,b3,C2),(A68,b3,C3),( A68,b3,C4),(A68,b3,C5),(A68,b3,C6),(A68,b3,C7),(A68,b3,C8),(A68,b3,C9),(A68,b3,C10),(A 68,b3,C11),(A68,b3,C12),(A68,b3,C13),(A68,b3,C14),(A68,b3,C15),(A68,b3,C16),(A68,b3,C 17),(A68,b3,C18),(A68,b3,C19),(A68,b3,C20),(A68,b3,C21),(A68,b3,C22),(A68,b3,C23),(A68 ,b3,C24),(A68,b3,C25),(A68,b3,C26),(A68,b3,C27),(A68,b3,C28),(A68,b3,C29),(A68,b4,C1),( A68,b4,C2),(A68,b4,C3),(A68,b4,C4),(A68,b4,C5),(A68,b4,C6),(A68,b4,C7),(A68,b4,C8),(A6 8,b4,C9),(A68,b4,C10),(A68,b4,C11),(A68,b4,C12),(A68,b4,C13),(A68,b4,C14),(A68,b4,C15) ,(A68,b4,C16),(A68,b4,C17),(A68,b4,C18),(A68,b4,C19),(A68,b4,C20),(A68,b4,C21),(A68,b4 ,C22),(A68,b4,C23),(A68,b4,C24),(A68,b4,C25),(A68,b4,C26),(A68,b4,C27),(A68,b4,C28),(A 68,b4,C29),(A68,b5,C1),(A68,b5,C2),(A68,b5,C3),(A68,b5,C4),(A68,b5,C5),(A68,b5,C6),(A6 8,b5,C7),(A68,b5,C8),(A68,b5,C9),(A68,b5,C10),(A68,b5,C11),(A68,b5,C12),(A68,b5,C13),( A68,b5,C14),(A68,b5,C15),(A68,b5,C16),(A68,b5,C7),(A68,b5,C18),(A68,b5,C9),(A68,b5, C20),(A68,b5,C21),(A68,b5,C22),(A68,b5,C23),(A68,b5,C24),(A68,b5,C25),(A68,b5,C26),(A 68,b5,C27),(A68,b5,C28),(A68,b5,C29),(A68,b6,C1),(A68,b6,C2),(A68,b6,C3),(A68,b6,C4),( A68,b6,C5),(A68,b6,C6),(A68,b6,C7),(A68,b6,C8),(A68,b6,C9),(A68,b6,C10),(A68,b6,C11),( A68,b6,C12),(A68,b6,C13),(A68,b6,C14),(A68,b6,C15),(A68,b6,C16),(A68,b6,C17),(A68,b6, C18),(A68,b6,C19),(A68,b6,C20),(A68,b6,C21),(A68,b6,C22),(A68,b6,C23),(A68,b6,C24),(A 68,b6,C25),(A68,b6,C26),(A68,b6,C27),(A68,b6,C28),(A68,b6,C29),(A68,b7,C1),(A68,b7,C2) ,(A68,b7,C3),(A68,b7,C4),(A68,b7,C5),(A68,b7,C6),(A68,b7,C7),(A68,b7,C8),(A68,b7,C9),(A 68,b7,C10),(A68,b7,C11),(A68,b7,C12),(A68,b7,C13),(A68,b7,C14),(A68,b7,C15),(A68,b7,C 16),(A68,b7,C17),(A68,b7,C18),(A68,b7,C19),(A68,b7,C20),(A68,b7,C21),(A68,b7,C22),(A68 ,b7,C23),(A68,b7,C24),(A68,b7,C25),(A68,b7,C26),(A68,b7,C27),(A68,b7,C28),(A68,b7,C29) ,(A69,b1,C1),(A69,b1,C2),(A69,b1,C3),(A69,b1,C4),(A69,b1,C5),(A69,b1,C6),(A69,b1,C7),(A 69,b1,C8),(A69,b1,C9),(A69,b1,C10),(A69,b1,C11),(A69,b1,C12),(A69,b1,C13),(A69,b1,C14) ,(A69,b1,C15),(A69,b1,C16),(A69,b1,C17),(A69,b1,C18),(A69,b1,C19),(A69,b1,C20),(A69,b1 ,C21),(A69,b1,C22),(A69,b1,C23),(A69,b1,C24),(A69,b1,C25),(A69,b1,C26),(A69,b1,C27),(A 69,b1,C28),(A69,b1,C29),(A69,b2,C1),(A69,b2,C2),(A69,b2,C3),(A69,b2,C4),(A69,b2_{;}C5),(A 69,b2,C6),(A69,b2,C'7),(A69,b2,C8),(A69,b2,C9),(A69,b2,C10),(A69,b2,C11),(A69,b2,C12),( A69,b2,C13),(A69,b2,C14),(A69,b2,C15),(A69,b2,C16),(A69,b2,C17),(A69,b2,C18),(A69,b2, C19),(A69,b2,C20),(A69,b2,C21),(A69,b2,C22),(A69,b2,C23),(A69,b2,C24),(A69,b2,C25),(A 69,b2,C26),(A69,b2,C27),(A69,b2,C28),(A69,b2,C29),(A69,b3,C1),(A69,b3,C2),(A69,b3,C3),( A69,b3,C4),(A69,b3,C5),(A69,b3,C6),(A69,b3,C7),(A69,b3,C8),(A69,b3,C9),(A69,b3,C10),(A 69,b3,C11),(A69,b3,C12),(A69,b3,C13),(A69,b3,C14),(A69,b3,C15),(A69,b3,C16),(A69,b3,C 17),(A69,b3,C18),(A69,b3,C19),(A69,b3,C20),(A69,b3,C21),(A69,b3,C22),(A69,b3,C23),(A69 ,b3,C24),(A69,b3,C25),(A69,b3,C26),(A69,b3,C27),(A69,b3,C28),(A69,b3,C29),(A69,b4,C1),( A69,b4,C2),(A69,b4,C3),(A69,b4,C4),(A69,b4,C5),(A69,b4,C6),(A69,b4,C7),(A69,b4,C8),(A6 9,b4,C9),(A69,b4,C10),(A69,b4,C11),(A69,b4,C12),(A69,b4,C13),(A69,b4,C14),(A69,b4,C15) ,(A69,b4,C16),(A69,b4,C17),(A69,b4,C18),(A69,b4,C19),(A69,b4,C20),(A69,b4,C21),(A69,b4 ,C22),(A69,b4,C23),(A69,b4,C24),(A69,b4,C25),(A69,b4,C26),(A69,b4,C27),(A69,b4,C28),(A 69,b4,C29),(A69,b5,C1),(A69,b5,C2),(A69,b5,C3),(A69,b5,C4),(A69,b5,C5),(A69,b5,C6),(A6 9,b5,C7),(A69,b5,C8),(A69,b5,C9),(A69,b5,C10),(A69,b5,C11),(A69,b5,C12),(A69,b5,C13),( A69,b5,C14),(A69,b5,C15),(A69,b5,C16),(A69,b5,C17),(A69,b5,C18),(A69,b5,C19),(A69,b5, C20),(A69,b5,C21),(A69,b5,C22),(A69,b5,C23),(A69,b5,C24),(A69,b5,C25),(A69,b5,C26),(A 69,b5,C27),(A69,b5,C28),(A69,b5,C29),(A69,b6,C1),(A69,b6,C2),(A69,b6,C3),(A69,b6,C4),( A69,b6,C5),(A69,b6,C6),(A69,b6,C7),(A69,b6,C8),(A69,b6,C9),(A69,b6,C10),(A69,b6,C11),( A69,b6,C12),(A69,b6,C13),(A69,b6,C14),(A69,b6,C15),(A69,b6,C16),(A69,b6,C17),(A69,b6, C18),(A69,b6,C19),(A69,b6,C20),(A69,b6,C21),(A69,b6,C22),(A69,b6,C23),(A69,b6,C24),(A 69,b6,C25),(A69,b6,C26),(A69,b6,C27),(A69,b6,C28),(A69,b6,C29),(A69,b7,C1),(A69,b7,C2) ,(A69,b7,C3),(A69,b7,C4),(A69,b7,C5),(A69,b7,C6),(A69,b7,C7),(A69,b7,C8),(A69,b7,C9),(A 69,b7,C10),(A69,b7,C11),(A69,b7,C12),(A69,b7,C13),(A69,b7,C14),(A69,b7,C15),(A69,b7,C 16),(A69,b7,C17),(A69,b7,C18),(A69,b7,C19),(A69,b7,C20),(A69,b7,C21),(A69,b7,C22),(A69 ,b7,C23),(A69,b7,C24),(A69,b7,C25),(A69,b7,C26),(A69,b7,C27),(A69,b7,C28),(A69,b7,C29) ,(A70,b1,C1),(A70,b1,C2),(A70,b1,C3),(A70,b1,C4),(A70,b1,C5),(A70,b1,C6),(A70,b1,C7),(A 70,b1,C8),(A70,b1,C9),(A70,b1,C10),(A70,b1,C11),(A70,b1,C12),(A70,b1,C13),(A70,b1,C14) ,(A70,b1,C15),(A70,b1,C16),(A70,b1,C17),(A70,b1,C18),(A70,b1,C19),(A70,b1,C20),(A70,b1 ,C21),(A70,b1,C22),(A70,b1,C23),(A70,b1,C24),(A70,b1,C25),(A70,b1,C26),(A70,b1,C27),(A 70,b1,C28),(A70,b1,C29),(A70,b2,C1),(A70,b2,C2),(A70,b2,C3),(A70,b2,C4),(A70,b2,C5),(A 70,b2,C6),(A70,b2,C7),(A70,b2,C8),(A70,b2,C9),(A70,b2,C10),(A70,b2,C11),(A70,b2,C12),( A70,b2,13),(A70,b2,C14),(A70,b2,C15),(A70,b2,C16),(A70,b2,C17),(A70,b2,C8),(A70,b2, C19),(A70,b2,C20),(A70,b2,C21),(A70,b2,C22),(A70,b2,C23),(A70,b2,C24),(A70,b2,C25),(A 70,b2,C26),(A70,b2,C27),(A70,b2,C28),(A70,b2,C29),(A70,b3,C1),(A70,b3,C2),(A70,b3,C3),( A70,b3,C4),(A70,b3,C5),(A70,b3,C6),(A'70,b3,C7),(A70,b3,C8),(A70,b3,C9),(A70,b3,C10),(A 70,b3,C11),(A70,b3,C12),(A70,b3,C13),(A70,b3,C14),(A70,b3,C15),(A70,b3,C16),(A70,b3,C 17),(A70,b3,C18),(A70,b3,C19),(A70,b3,C20),(A70,b3,C21),(A70,b3,C22),(A70,b3,C23),(A70 ,b3,C24),(A70,b3,C25),(A70,b3,C26),(A70,b3,C27),(A70,b3,C28),(A70,b3,C29),(A70,b4,C1),( A70,b4,C2),(A70,b4,C3),(A70,b4,C4),(A70,b4,C5),(A70,b4,C6),(A70,b4,C7),(A70,b4,C8),(A7 0,b4,C9),(A70,b4,C10),(A70,b4,C11),(A70,b4,C12),(A70,b4,C13),(A70,b4,C14),(A70,b4,C15) ,(A70,b4,C16),(A70,b4,C17),(A70,b4,C18),(A70,b4,C19),(A70,b4,C20),(A70,b4,C21),(A70,b4 ,C22),(A70,b4,C23),(A70,b4,C24),(A70,b4,C25),(A70,b4,C26),(A70,b4,C27),(A70,b4,C28),(A 70,b4,C29),(A70,b5,C1),(A70,b5,C2),(A70,b5,C3),(A70,b5,C4),(A70,b5,C5),(A70,b5,C6),(A7 0,b5,C7),(A70,b1,C8),(A70,b5,C9),(A70,b5,C10),(A70,b5,C11),(A70,b5,C12),(A70,b5,C13),( A70,b5,C14),(A70,b5,C15),(A70,b5,C16),(A70,b5,C17),(A70,b5,C18),(A70,b5,C19),(A70,b5, C20),(A70,b5,C21),(A70,b5,C22),(A70,b5,C23),(A70,b5,C24),(A70,b5,C25),(A70,b5,C26),(A 70,b5,C27),(A70,b5,C28),(A70,b5,C29),(A70,b6,C1),(A70,b6,C2),(A70,b6,C3),(A70,b6,C4),( A70,b6,C5),(A70,b6,C6),(A70,b6,C7),(A70,b6,C8),(A70,b6,C9),(A70,b6,C10),(A70,b6,C11),( A70,b6,C12),(A70,b6,C13),(A70,b6,C14),(A70,b6,C15),(A70,b6,C16),(A70,b6,C17),(A70,b6, C18),(A70,b6,C19),(A70,b6,C20),(A70,b6,C21),(A70,b6,C22),(A70,b6,C23),(A70,b6,C24),(A 70,b6,C25),(A70,b6,C26),(A70,b6,C27),(A70,b6,C28),(A70,b6,C29),(A70,b7,C1),(A70,b7,C2) ,(A70,b7,C3),(A70,b7,C4),(A70,b7,C5),(A70,b7,C6),(A70,b7,C7),(A70,b7C8),(A70,b7,C9),(A 70,b7,C10),(A70,b7,C11),(A70,b7,C12),(A70,b7,C13),(A70,b7,C14),(A70,b7,C15),(A70,b7,C 16),(A70,b7,C17),(A70,b7,C18),(A70,b7,C19),(A70,b7,C20),(A70,b7,C21),(A70,b7,C22),(A70 ,b7,C23),(A70,b7,C24),(A70,b7,C25),(A70,b7,C26),(A70,b7C27),(A70,b7,C28),(A70,b7,C29),

(A71,b1,C1),(A71,b1,C2),(A71,b1,C3),(A71,b1,C4),(A71,b1,C5),(A71,b1,C6),(A71,b1,C7),(A 71,b1,C8),(A71,b1,C9),(A71,b1,C10),(A71,b1,C11),(A71,b1,C12),(A71,b1,C13),(A71,b1,C14) ,(A71,b1,C15),(A71,b1,C16),(A71,b1,C17),(A71,b1,C15),(A71,b1,C19),(A71,b1,C20),(A71,b1 ,C21),(A71,b1,C22),(A71,b1,C23),(A71,b1,C24),(A71,b1,C25),(A71,b1,C26),(A71,b1,C27),(A 71,b1,C28),(A71,b1,C29),(A71,b2,C1),(A71,b2,C2),(A71,b2,C3),(A71,b2,C4),(A71,b2,C5),(A 71,b2,C6),(A71,b2,C7),(A71,b2,C8),(A71,b2,C9),(A71,b2,C10),(A71,b2,C11),(A71,b2,C12),( A71,b2,C13),(A71,b2,C14),(A71,b2,C15),(A71,b2,C16),(A71,b2,C17),(A71,b2,C18),(A71,b2, C19),(A71,b2,C20),(A71,b2,C21),(A71,b2,C22),(A71,b2,C23),(A71,b2,C24),(A71,b2,C25),(A 71,b2,C26),(A71,b2,C27),(A71,b2,C28),(A71,b2,C29),(A71,b3,C1),(A71,b3,C2),(A71,b3,C3),( A71,b3,C4),(A71,b3,C5),(A71,b3,C6),(A71,b3,C7),(A71,b3,C8),(A71,b3,C9),(A71,b3,C10),(A 71,b3,C11),(A71,b3,C12),(A71,b3,C13),(A71,b3,C14),(A71,b3,C15),(A71,b3,C16),(A71,b3,C 17),(A71,b3,C18),(A71,b3,C19),(A71,b3,C20),(A71,b3,C21),(A71,b3,C22),(A71,b3,C23),(A71 ,b3,C24),(A71,b3,C25),(A71,b3,C26),(A71,b3,C27),(A71,b3,C28),(A71,b3,C29),(A71,b4,C1),( A71,b4,C2),(A71,b4,C3),(A71,b4,C4),(A71,b4,C5),(A71,b4,C6),(A71,b4,C7),(A71,b4,C8),(A7 1,b4,C9),(A71,b4,C10),(A71,b4,C11),(A71,b4,C12),(A71,b4,C13),(A71,b4,C14),(A71,b4,C15) ,(A71,b4,C16),(A71,b4,C17),(A71,b4,C18),(A71,b4,C19),(A71,b4,C20),(A71,b4,C21),(A71,b4 ,C22),(A71,b4,C23),(A71,b4,C24),(A71,b4,C25),(A71,b4,C26),(A71,b4,C27),(A71,b4,C28),(A 71,b4,C29),(A71,b5,C1),(A71,b5,C2),(A71,b5,C3),(A71,b5,C4),(A71,b5,C5),(A71,b5,C6),(A7 1,b5,C7),(A71,b5,C8),(A71,b5,C9),(A71,b5,C10),(A71,b5,Cl1),(A71,b5,C12),(A71,b5,C13),( A71,b5,C14),(A71,b5,C155),(A71,b5,C16),(A71.,b5,C17),(A71,b5,C18),(A71,b5,C19),(A71,b5, C20),(A71,b5,C21),(A71,b5,C22),(A71,b5,C23),(A71,b5,C24),(A71,b5,C25),(A71,b5,C26),(A 71,b5,C27),(A71,b5,C28),(A71,b5,C29),(A71,b6,Cl),(A71,b6,C2),(A71,b6,C3),(A71,b6,C4),( A71,b6,C5),(A71,b6,C6),(A71,b6,C7),(A71,b6,C8),(A71,b6,C9),(A71,b6,C10),(A71,b6,C11),( A71,b6,C12),(A71,b6,C13),(A71,b6,C14),(A71,b6,C15),(A71,b6,C16),(A71,b6,C17),(A71,b6, C18),(A71,b6,C19),(A71,b6,C20),(A71,b6,C21),(A71,b6,C22),(A71,b6,C23),(A71,b6,C24),(A 71,b6,C25),(A71,b6,C26),(A71,b6,C27),(A71,b6,C28),(A71,b6,C29),(A71,b7,C1),(A71,b7,C2) ,(A71,b7,C3),(A71,b7,C4),(A71,b7,C5),(A71,b7,C6),(A71,b7,C7),(A71,b7,C8),(A71,b7,C9),(A 71,b7,C10),(A71,b7,C11),(A71,b7,C12),(A71,b7,C13),(A71,b7,C14),(A71,b7,C15),(A71,b7,C 16),(A71,b7,C17),(A71,b7,C18),(A71,b7,C19),(A71,b7,C20),(A7,b7,C21),(A71,b7,C22),(A71 ,b7,C23),(A71,b7,C24),(A71,b7,C25),(A71,b7,C26),(A71,b7,C27),(A71,b7,C28),(A71,b7,C29) ,(A72,b1,C1),(A72,b1,C2),(A72,b1,C3),(A72,b1,C4),(A72,b1,C5),(A72,b1,C6),(A72,b1,C7),(A 72,b1,C8),(A72,b1,C9),(A72,b1,C10),(A72,b1,C11),(A72,b1,C12),(A72,b1,C13),(A72,b1,C14) ,(A72,b1,C15),(A72,b1,C16),(A72,b1,C17),(A72,b1,C18),(A72,b1,C19),(A72,b1,C20),(A72,b1 ,C21),(A72,b1,C22),(A72,b1,C23),(A72,b1,C24),(A72,b1,C25),(A72,b1,C26),(A72,b1,C27),(A 72,b1,C28),(A72,b1,C29),(A72,b2,C1),(A72,b2,C2),(A72,b2,C3),(A72,b2,C4),(A72,b2,C5),(A 72,b2,C6),(A72,b2,C7),(A72,b2,C8),(A72,b2,C9),(A72,b2,C10),(A72,b2,C11),(A72,b2,C12),( A72,b2,C13),(A72,b2,C14),(A72,b2,C15),(A72,b2,C16),(A72,b2,C17),(A72,b2,C18),(A72,b2, C19),(A72,b2,C20),(A72,b2,C21),(A72,b2,C22),(A72,b2,C23),(A72,b2,C24),(A72,b2,C25),(A 72,b2,C26),(A72,b2,C27),(A72,b2,C28),(A72,b2,C29),(A72,b3,C1),(A72,b3,C2),(A72,b3,C3),( A72,b3,C4),(A72,b3,C5),(A72,b3,C6),(A72,b3,C7),(A72,b3,C8),(A72,b3,C9),(A72,b3,C10),(A 72,b3,C11),(A72,b3,C12),(A72,b3,C13),(A72,b3,C14),(A72,b3,C15),(A72,b3,C16),(A72,b3,C 17),(A72,b3,C18),(A72,b3,C19),(A72,b3,C20),(A72,b3,C21),(A72,b3,C22),(A72,b3,C23),(A72 ,b3,C24),(A72,b3,C25),(A72,b3,C26),(A72,b3,C27),(A72,b3,C28),(A72,b3,C29),(A72,b4,C1),( A72,b4,C2),(A72,b4,C3),(A72,b4,C4),(A72,b4,C5),(A72,b4,C6),(A72,b4,C7),(A72,b4,C8),(A7 2,b4,C9),(A72,b4,C10),(A72,b4,C11),(A72,b4,C12),(A72,b4,C13),(A72,b4,C14),(A72,b4,C15) ,(A72,b4,C16),(A72,b4,C17),(A72,b4,C18),(A72,b4,C19),(A72,b4,C20),(A72,b4,C21),(A72,b4 ,C22),(A72,b4,C23),(A72,b4,C24),(A72,b4,C25),(A72,b4,C26),(A72,b4,C27),(A72,b4,C28),(A 72,b4,C29),(A72,b5,C1),(A72,b5,C2),(A72,b5,C3),(A72,b5,C4),(A72,b5,C5),(A72,b5,C6),(A7 2,b5,C7),(A72,b5,C8),(A72,b5,C9),(A72,b5,C10),(A72,b5,C11),(A72,b5,C12),(A72,b5,C13),( A72,b5,C14),(A72,b5,C15),(A72,b5,C16),(A72,b5,C17),(A72,b5,C18),(A72,b5,C19),(A72,b5, C20),(A72,b5,C21),(A72,b5,C22),(A72,b5,C23),(A72,b5,C24),(A72,b5,C25),(A72,b5,C26),(A 72,b5,C27),(A72,b5,C28),(A72,b5,C29),(A72,b6,C1),(A72,b6,C2),(A72,b6,C3),(A72,b6,C4),( A72,b6,C5),(A72,b6,C6),(A72,b6,C7),(A72,b6,C8),(A72,b6,C9),(A72,b6,C10),(A72,b6,C11),( A72,b6,C12),(A72,b6,C13),(A72,b6,C14),(A72,b6,C15),(A72,b6,C16),(A72,b6,C17),(A72,b6, C18),(A72,b6,C19),(A72,b6,C20),(A72,b6,C21),(A72,b6,C22),(A72,b6,C23),(A72,b6,C24),(A 72,b6,C25),(A72,b6,C26),(A72,b0,C27),(A72,b6,C28),(A72,b6,C29),(A72,b7,C1),(A72,b7,C2) ,(A72,b7,C3),(A72,b7,C4),(A72,b7,C5),(A72,b7,C6),(A72,b7,C7),(A72,b7,C8),(A72,b7,C9),(A 72,b7,C10),(A72,b7,C11),(A72,b7,C12),(A72,b7,C13),(A72,b7,C14),(A72,b7,C15),(A72,b7,C 16),(A72,b7,C17),(A72,b7,C18),(A72,b7,C19),(A72,b7,C20),(A72,b7,C21),(A72,b7,C22),(A72 ,b7,C23),(A72,b7,C24),(A72,b7,C25),(A72,b7,C26),(A72,b7,C27),(A72,b7,C28),(A72,b7,C29) ,(A73,b1,C1),(A73,b1,C2),(A73,b1,C3),(A73,b1,C4),(A73,b1,C5),(A73,b1,C6),(A73,b1,C7),(A 73,b1,C8),(A73,b1,C9),(A73,b1,C10),(A73,b1,C11),(A73,b1,C12),(A73,b1,C13),(A73,b1,C14) ,(A73,b1,C15),(A73,b1,C16),(A73,b1,C17),(A73,b1,C18),(A73,b1,C19),(A73,b1,C20),(A73,b1 ,C21),(A73,b1,C22),(A73,b1,C23),(A73,b1,C24),(A73,b1,C25),(A73,b1,C26),(A73,b1,C27),(A 73,b1,C28),(A73,b1,C29),(A73,b2,C1),(A73,b2,C2),(A73,b2,C3),(A73,b2,C4),(A73,b2,C5),(A 73,b2,C6),(A73,b2,C7),(A73,b2,C8),(A73,b2,C9),(A73,b2,C10),(A73,b2,C11),(A73,b2,C12),( A73,b2,C13),(A73,b2,C14),(A73,b2,C15),(A73,b2,C16),(A73,b2,C17),(A73,b2,C18),(A73,b2, C19),(A73,b2,C20),(A73,b2,C21),(A73,b2,C22),(A73,b2,C23),(A73,b2,C24),(A73,b2,C25),(A 73,b2,C26),(A73,b2,C27),(A73,b2,C28),(A73,b2,C29),(A73,b3,C1),(A73,b3,C2),(A73,b3,C3),( A73,b3,C4),(A73,b3,C5),(A73,b3,C6),(A73,b3,C7),(A73,b3,C8),(A73,b3,C9),(A73,b3,C10),(A 73,b3,C11),(A73,b3,C12),(A73,b3,C13),(A73,b3,C14),(A73,b3,C15),(A73,b3,C16),(A73,b3,C 17),(A73,b3,C18),(A73,b3,C19),(A73,b3,C20),(A73,b3,C21),(A73,b3,C22),(A73,b3,C23),(A73 ,b3,C24),(A73,b3,C25),(A73,b3,C26),(A73,b3,C27),(A73,b3,C28),(A73,b3,C29),(A73,b4,C1),( A73,b4,C2),(A73,b4,C3),(A73,b4,C4),(A73,b4,C5),(A73,b4,C6),(A73,b4,C7),(A73,b4,C8),(A7 3,b4,C9),(A73,b4,C10),(A73,b4,C11),(A73,b4,C12),(A73,b4,C13),(A73,b4,C14),(A73,b4,C15) ,(A73,b4,C16),(A73,b4,C17),(A73,b4,C18),(A73,b4,C19),(A73,b4,C20),(A73,b4,C21),(A73,b4 ,C22),(A73,b4,C23),(A73,b4,C24),(A73,b4,C25),(A73,b4,C26),(A73,b4,C27),(A73,b4,C28),(A 73,b4,C29),(A73,b5,C1),(A73,b5,C2),(A73,b5,C3),(A73,b5,C4),(.A73,b5,C5),(A73,b5,C6),(A7 3,b5,C7),(A73,b5,C8),(A73,b5,C9),(A73,b5,C10),(A73,b5,C11),(A73,b5,C12),(A73,b5,C13),( A73,b5,C14),(A73,b5,C15),(A73,b5,C16),(A73,b5,C17),(A73,b5,C18),(A73,b5,C19),(A73,b5, C20),(A73,b5,C21),(A73,b5,C22),(A73,b5,C23),(A73,b5,C24),(A73,b5,C25),(A73,b5,C26),(A 73,b5,C27),(A73,b5,C28),(A73,b5,C29),(A73,b6,C1),(A73,b6,C2),(A73,b6,C3),(A73,b6,C4),( A73,b6,C5),(A73,b6,C6),(A73,b6,C7),(A73,b6,C8),(A73,b6,C9),(A73,b6,C10),(A73,b6,C11),( A73,b6,C12),(A73,b6,C13),(A73,b6,C14),(A73,b6,C15),(A73,b6,C16),(A73,b6,C17),(A73,b6, C18),(A73,b6,C19),(A73,b6,C20),(A73,b6,C21),(A73,b6,C22),(A73,b6,C23),(A73,b6,C24),(A 73,b6,C25),(A73,b6,C26),(A73,b6,C27),(A73,b6,C28),(A73;b6,C29),(A73,b7,C1),(A73,b7,C2) ,(A73,b7,C3),(A73,b7,C4),(A73,b7,C5),(A73,b7,C6),(A73,b7,C7),(A73,b7,C8),(A73,b7,C9),(A 73,b7,C10),(A73,b7,Cl l),(A73,b7,C12),(A73,b7,C13),(A73,b7,C14),(A73,b7,C15),(A73,b7,C 16),(A73,b7,C17),(A73,b7,C18),(A73,b7,C19),(A73,b7,C20),(A73,b7,C21),(A73,b7,C22),(A73 ,b7,C23),(A73,b7,C24),(A73,b7,C25),(A73,b7,C26),(A73,b7,C27),(A73,b7,C28),(A73,b7,C29) ,(A74,b1,C1),(A74,b1,C2),(A74,b1,C3),(A74,b1,C4),(A74,b1,C5),(A74,b1,C6),(A74,b1,C7),(A 74,b1,C8),(A74,b1,C9),(A74,b1,C10),(A74,b1,C11),(A74,b1,C12),(A74,b1,C13),(A74,b1,C14) ,(A74,b1,C15),(A74,b1,C16),(A74,b1,C17),(A74,b1,C18),(A74,b1,C19),(A74,b1,C20),(A74,b1 ,C21),(A74,b1,C22),(A74,b1,C23),(A74,b1,C24),(A74,b1,C25),(A74,b1,C26),(A74,b1,C27),(A 74,b1,C28),(A7A,b1,C29),(A74,b2,C1),(A74,b2,C2),(A74,b2,C3),(A74,b2,C4),(A74,b2,C5),(A 74,b2,C6),(A74,b2,C7),(A74,b2,C8),(A74,b2,C9),(A74,b2,C10),(A74,b2,C11),(A74,b2,C12),( A74,b2,C13),(A74,b2,C14),(A74,b2,C15),(A74,b2,C16),(A74,b2,C17),(A74,b2,C18),(A74,b2, C19),(A74,b2,C20),(A74,b2,C21),(A74,b2,C22),(A74,b2,C23),(A74,b2,C24),(A74,b2,C25),(A 74,b2,C26),(A74,b2,C27),(A74,b2,C28),(A74,b2,C29),(A74,b3,C1),(A74,b3,C2),(A74,b3,C3),( A74,b3,C4),(A74,b3,C5),(A74,b3,C6),(A74,b3,C7),(A74,b3,C8),(A74,b3,C9),(A74,b3,C10),(A 74,b3,C11),(A74,b3,C12),(A74,b3,C13),(A74,b3,C14),(A74,b3,C15),(A74,b3,C16),(A74,b3,C 17),(A74,b3,C18),(A74,b3,C19),(A74,b3,C20),(A74,b3,C21),(A74,b3,C22),(A74,b3,C23),(A74 ,b3,C24),(A74,b3,C25),(A74,b3,C26),(A74,b3,C27),(A74,b3,C28),(A74,b3,C29),(A74,b4,C1),( A74,b4,C2),(A74,b4,C3),(A74,b4,C4),(A74,b4,C5),(A74,b4,C6),(A74,b4,C7),(A74,b4,C8),(A7 4,b4,C9),(A74,b4,C10),(A74,b4,C11),(A74,b4,C12),(A74,b4,C13),(A74,b4,C14),(A74,b4,C15) ,(A74,b4,C16),(A74,b4,C17),(A74,b4,C18),(A74,b4,C19),(A74,b4,C20),(A74,b4,C21),(A74,b4 ,C22),(A74,b4,C23),(A74,b4,C24),(A74,b4,C25),(A74,b4,C26),(A74,b4,C27),(A74,b4,C28),(A 74,b4,C29),(A74,b5,C1),(A74,b5,C2),(A74,b5,C3),(A74,b5,C4),(A74,b5,C5),(A74,b5,C6),(A7 4,b5,C7),(A74,b5,C8),(A74,b5,C9),(A74,b5,C10),(A74,b5,C11),(A74,b5,C12),(A74,b5,C13),( A74,b5,C14),(A74,b5,C15),(A74,b5,C16),(A74,b5,C17),(A74,b5,C18),(A74,b5,C19),(A74,b5, C20),(A74,b5,C21),(A74,b5,C22),(A74,b5,C23),(A74,b5,C24),(A74,b5,C25),(A74,b5,C26),(A 74,b5,C27),(A74,b5,C28),(474,b5,C29),(A74,b6,C1),(A74,b6,C2),(A74,b6,C3),(A74,b6,C4),( A74,b6,C5),(A74,b6,C6),(A74,b6,C7),(A74,b6,C8),(A74,b6,C9),(A74,b6,C10),(A74,b6,C11),( A74,b6,C12),(A74,b6,C13),(A74,b6,C14),(A74,b6,C15),(A74,b6,C16),(A74,b6,C17),(A74,b6, C18),(A74,b6,C19),(A74,b6,C20),(A74,b6,C21),(A74,b6,C22),(A74,b6,C23),(A74,b6,C24),(A 74,b6,C25),(A74,b6,C26),(A74,b6,C27),(A74,b6,C28),(A74,b6,C29),(A74,b7,C1),(A74,b7,C2) ,(A74,b7,C3),(A74,b7,C4),(A74,b7,C5),(A74,b7,C6),(A74,b7,C7),(A74,b7,C8),(A74,b7,C9),(A 74,b7,C10),(A74,b7,C11),(A74,b7,C12),(A74,b7,C13),(A74,b7,C14),(A74,b7,C15),(A74,b7,C 16),(A74,b7,C17),(A74,b7,C18),(A74,b7,C19),(A74,b7,C20),(A74,b7,C21),(A74,b7,C22),(A74 ,b7,C23),(A74,b7,C24),(A74,b7,C25),(A74,b7,C26),(A74,b7,C27),(A74,b7,C28),(A74,b7,C29) ,(A75,b1,C1),(A75,b1,C2),(A75,b1,C3),(A75,b1,C4),(A75,b1,C5),(A75,b1,C6),(A75,b1,C7),(A 75,b1,C8),(A75,b1,C9),(A75,b1,C10),(A75,b1,C11),(A75,b1,C12),(A75,b1,C13),(A75,b1,C14) ,(A75,b1,C15),(A75,b1,C16),(A75,bl,Cl7),(A75,b1,C18),(A75,b1,C19),(A75,b1,C20),(A75,b1 ,C21),(A75,b1,C22),(A75,b1,C23),(A75,b1,C24),(A75,b1,C25),(A75,b1,C26),(A75,b1,C27),(A 75,b1,C28),(A75,b1,C29),(A75,b2,C1),(A75,b2,C2),(A75,b2,C3),(A75,b2,C4),(A75,b2,C5),(A 75,b2,C6),(A75,b2,C7),(A75,b2,C8),(A75,b2,C9),(A75,b2,C10),(A75,b2,C11),(A75,b2,C12),( A75,b2,C13),(A75,b2,C14),(A75,b2,C15),(A75,b2,C16),(A75,b2,C17),(A75,b2,C18),(A75,b2, C19),(A75,b2,C20),(A75,b2,C21),(A75,b2,C22),(A75,b2,C23),(A75,b2,C24),(A75,b2,C25),(A 75,b2,C26),(A75,b2,C27),(A75,b2,C28),(A75,b2,C29),(A75,b3,Cl),(A75,b3,C2),(A75,b3,C3),( A75,b3,C4),(A75,b3,C5),(A75,b3,C6),(A75,b3,C7),(A75,b3,C8),(A75,b3,C9),(A75,b3,C10),(A 75,b3,C11),(A75,b3,C12),(A75,b3,C13),(A75,b3,C14),(A75,b3,C15),(A75,b3,C16),(A75,b3,C 17),(A75,b3,C18),(A75,b3,C19),(A75,b3,C20),(A75,b3,C21),(A75,b3,C22),(A75,b3,C23),(A75 ,b3,C24),(A75,b3,C25),(A75,b3,C26),(A75,b3,C27),(A75,b3,C28),(A75,b3,C29),(A75,b4,C1),( A75,b4,C2),(A75,b4,C3),(A75,b4,C4),(A75,b4,C5),(A75,b4,C6),(A75,b4,C7),(A75,b4,C8),(A7 5,b4,C9),(A75,b4,C10),(A75,b4,C11),(A75,b4,C12),(A75,b4,C13),(A75,b4,C14),(A75,b4,C15) ,(A75,b4,C16),(A75,b4,C17),(A75,b4,C18),(A75,b4,C19),(A75,b4,C20),(A75,b4,C21),(A75,b4 ,C22),(A75,b4,C23),(A75,b4,C24),(A75,b4,C25),(A75,b4,C26),(A75,b4,C27),(A75,b4,C28),(A 75,b4,C29),(A75,b5,C1),(A75,b5,C2),(A75,b5,C3),(A75,b5,C4),(A75,b5,C5),(A75,b5,C6),(A7 5,b5,C7),(A75,b5,C8),(A75,b5,C9),(A75,b5,C10),(A75,b5,C11),(A75,b5,C12),(A75,b5,C13),( A75,b5,C1),(A75,b5,C15),(A75,b5,C16),(A75,b5,C17),(A75,b5,C18),(A75,b5,C19),(A75,b5, C20),(A75,b5,C21),(A75,b5,C22),(A75,b5,C23),(A75,b5,C24),(A75,b5,C25),(A75,b5,C26),(A 75,b5,C27),(A75,b5,C28),(A75,b5,C29),(A75,b6,C1),(A75,b6,C2),(A75,b6,C3),(A75,b6,C4),( A75,b6,C5),(A75,b6,C6),(A75,b6,C7),(A75,b6,C8),(A75,b6,C9),(A75,b6,C10),(A75,b6,C11),( A75,b6,C12),(A75,b6,C13),(A75,b6,C14),(A75,b6,C15),(A75,b6,C16),(A75,b6,C17),(A75,b6, C18),(A75,b6,C19),(A75,b6,C20),(A75,b6,C21),(A75,b6,C22),(A75,b6,C23),(A75,b6,C24),(A 75,b6,C25),(A75,b6,C26),(A75,b6,C27),(A75,b6,C28),(A75,b6,C29),(A75,b7,C1),(A75,b7,C2) ,(A75,b7,C3),(A75,b7,C4),(A75,b7,C5),(A75,b7,C6),(A75,b7,C7),(A75,b7,C8),(A75,b7,C9),(A 75,b7,C10),(A75,b7,C11),(A75,b7,C12),(A75,b7,C1.3),(A75,b7,C14),(A175,b7,C15),(A75,b7,C 16),(A75,b7,C17),(A75,b7,C18),(A75,b7,C19),(A75,b7,C20),(A75,b7,C21),(A75,b7,C22),(A75 ,b7,C23),(A75,b7,C24),(A75,b7,C25),(A75,b7,C26),(A75,b7,C27),(A75,b7,C28),(A75,b7,C29) ,(A76,b1,C1),(A76,b1,C2),(A76,b1,C3),(A76,b1,C4),(A76,b1,C5),(A76,b1,C6),(A76,b1,C7),(A 76,b1,C8),(A76,b1,C9),(A76,b1,C10),(A76,b1,C11),(A76,b1,C12),(A76,b1,C13),(A76,b1,C14) ,(A76,b1,C15),(A76,b1,C16),(A76,b1,C17),(A76,b1,C18),(A76,b1,C19),(A76,b1,C20),(A76,b1 ,C21),(A76,b1,C22),(A76,b1,C23),(A76,b1,C24),(A76,b1,C25),(A76,b1,C26),(A76,b1,C27),(A 76,b1,C28),(A76,b1,C29),(A76,b2,C1),(A76,b2,C2),(A76,b2,C3),(A76,b2,C4),(A76,b2,C5),(A 76,b2,C6),(A76,b2,C7),(A76,b2,C8),(A76,b2,C9),(A76,b2,C10),(A76,b2,C11),(A76,b2,C12),( A76,b2,C13),(A76,b2,C14),(A76,b2,C15),(A76,b2,C16),(A76,b2,C17),(A76,b2,C18),(A76,b2, CI9),(A76,b2,C20),(A76,b2,C21),(A76,b2,C22),(A76,b2,C23),(A76,b2,C24),(A76,b2,C25),(A 76,b2,C26),(A76,b2,C27),(A76,b2,C28),(A76,b2,C29),(A76,b3,C1),(A76,b3,C2),(A76,b3,C3),( A76,b3,C4),(A76,b3,C5),(A76,b3,C6),(A76,b3,C7),(A76,b3,C8),(A76,b3,C9),(A76,b3,C10),(A 76,b3,C11),(A76,b3,C12),(A76,b3,C13),(A76,b3,C14),(A76,b3,C15),(A76,b3,C16),(A76,b3,C 17),(A76,b3,C18),(A76,b3,C19),(A76,b3,C20),(A76,b3,C21),(A76,b3,C22),(A76,b3,C23),(A76 ,b3,C24),(A76,b3,C25),(A76,b3,C26),(A76,b3,C27),(A76,b3,C28),(A76,b3,C29),(A76,b4,C1),( A76,b4,C2),(A76,b4,C3),(A76,b4,C4),(A76,b4,C5),(A76,b4,C6),(A76,b4,C7),(A76,b4,C8),(A7 6,b4,C9),(A76,b4,C10),(A76,b4,C11),(A76,b4,C12),(A76,b4,C13),(A76,b4,C14),(A76,b4,C15) ,(A76,b4,C16),(A76,b4,C17),(A76,b4,C18),(A76,b4,C19),(A76,b4,C20),(A76,b4,C21),(A76,b4 ,C22),(A76,b4,C23),(A76,b4,C24),(A76,b4,C25),(A76,b4,C26),(A76,b4,C27),(A76,b4,C28),(A 76,b4,C29),(A76,b5,C 1),(A76,b5,C2),(A76,b5,C3),(A76,b5,C4),(A76,b5,C5),(A76,b5,C6),(A7 6,b5,C7),(A76,b5,C8),(A76,b5,C9),(A76,b5,C10),(A76,b5,C11),(A76,b5,C12),(A76,b5,C13),( A76,b5,C14),(A76,b5,C15),(A76,b5,C16),(A76,b5,C17),(A76,b5,C18),(A76,b5,C19),(A76,b5, C20),(A76,b5,C21),(A76,b5,C22),(A76,b5,C23),(A76,b5,C24),(A76,b5,C25),(A76,b5,C26),(A 76,b5,C2'7),(A76,b5,C28),(A76,b5,C29),(A76,b6,C1),(A76,b6,C2),(A76,b6,C3),(A76,b6,C4),( A76,b6,C5),(A76,b6,C6),(A76,b6,C7),(A76,b6,C8),(A76,b6,C9),(A76,b6,C10),(A76,b6,C11),( A76,b6,C12),(A76,b6,C13),(A76,b6,C14),(A76,b6,C15),(A76,b6,C16),(A76,b6,C17),(A76,b6, C18),(A76,b6,C19),(A76,b6,C20),(A76,b6,C21),(A76,b6,C22),(A76,b6,C23),(A76,b6,C24),(A 76,b6,C25),(A76,b6,C26),(A76,b6,C27),(A76,b6,C28),(A76,b6,C29),(A76,b7,C1),(A76,b7,C2) ,(A76,b7,C3),(A76,b7,C4),(A76,b7,C5),(A76,b7,C6),(A76,b7,C7),(A76,b7,C8),(A76,b7,C9),(A 76,b7,C10),(A76,b7,C11),(A76,b7,C12),(A76,b7,C13),(A76,b7,C14),(A76,b7,C15),(A76,b7,C 16),(A76,b7,C17),(A76,b7,C18),(A76,b7,C19),(A76,b7,C20),(A76,b7,C21),(A76,b7,C22),(A76 ,b7,C23),(A76,b7,C24),(A76,b7,C25),(A76,b7,C26),(A76,b7,C27),(A76,b7,C28),(A76,b7,C29) ,(A77,b1,C1),(A77,b1,C2),(A77,b1,C3),(A77,b1,C4),(A77,b1,C5),(A77,b1,C6),(A77,b1,C7),(A 77,b1,C8),(A77,b1,C9),(A77,b1,C10),(A77,b1,C11),(A77,b1,C12),(A77,b1,C13),(A77,b1,C14) ,(A77,b1,C15),(A77,b1,C16),(A77,b1,C17),(A77,b1,C18),(A77,b1,C19),(A77,b1,C20),(A77,b1 ,C21),(A77,b1,C22),(A77,b1,C23),(A77,b1,C24),(A77,b1,C25),(A77,b1,C26),(A77,b1,C27),(A 77,b1,C28),(A77,b1,C29),(A77,b2,C1),(A77,b2,C2),(A77,b2,C3),(A77,b2,C4),(A77,b2,C5),(A 77,b2,C6),(A77,b2,C7),(A77,b2,C8),(A77,b2,C9),(A77,b2,C10),(A77,b2,C11),(A77,b2,C12),( A77,b2,C13),(A77,b2,C14),(A77,b2,C15),(A77,b2,C16),(A77,b2,C17),(A77,b2,C18),(A77,b2, C19),(A77,b2,C20),(A77,b2,C21),(A77,b2,C22),(A77,b2,C23),(A77,b2,C24),(A77,b2,C25),(A 77,b2,C26),(A77,b2,C27),(A77,b2,C28),(A77,b2,C29),(A77,b3,Cl),(A77,b3,C2),(A77,b3,C3),( A77,b3,C4),(A77,b3,C5),(A77,b3,C6),(A77,b3,C7),(A77,b3,C8),(A77,b3,C9),(A77,b3,C10),(A 77,b3,C11),(A77,b3,C12),(A77,b3,C13),(A77,b3,C14),(A77,b3,C15),(A77,b3,C16),(A77,b3,C 17),(A77,b3,C18),(A77,b3,C19),(A77,b3,C20),(A77,b3,C21),(A77,b3,C22),(A77,b3,C23),(A7 ,b3,C24),(A77,b3,C25),(A77,b3,C26),(A77,b3,C27),(A77,b3,C28),(A77,b3,C29),(A77,b4,C1),( A77,b4,C2),(A77,b4,C3),(A77,b4,C4),(A77,b4,C5),(A77,b4,C6),(A77,b4,C7),(A77,b4,C8),(A7 7,b4,C9),(A77,b4,C10),(A77,b4,C11),(A77,b4,C12),(A77,b4,C13),(A77,b4,C14),(A77,b4,C15) ,(A77,b4,C16),(A77,b4,C17),(A77,b4,C18),(A77,b4,C19),(A77,b4,C20),(A77,b4,C21),(A77,b4 ,C22),(A77,b4,C23),(A77,b4,C24),(A77,b4,C25),(A77,b4,C26),(A77,b4,C27),(A77,b4,C28),(A 77,b4,C29),(A77,b5,C1),(A77,b5,C2),(A77,b5,C3),(A77,b5,C4),(A77,b5,C5),(A77,b5,C6),(A7 7,b5,C7),(A77,b5,C8),(A77,b5,C9),(A77,b5,C10),(A77,b5,C11),(A77,b5,C12),(A77,b5,C13),( A77,b5,C14),(A77,b5,C15),(A77,b5,C16),(A77,b5,C17),(A77,b5,C18),(A77,b5,C19),(A77,b5, C20),(A77,b5,C21),(A77,b5,C22),(A77,b5,C23),(A77,b5,C24),(A77,b5,C25),(A77,b5,C26),(A 77,b5,C27),(A77,b5,C28),(A77,b5,C29),(A77,b6,Cl),(A77,b6,C2),(A77,b6,C3),(A77,b6,C4),( A77,b6,C5),(A77,b6,C6),(A77,b6,C7),(A77,b6,C8),(A77,b6,C9),(A77,b6,C10),(A77,b6,C11.),( A77,b6,C12),(A77,b6,C13),(A77,b6,C14),(A77,b6,C15),(A77,b6,C16),(A77,b6,C17),(A77,b6, C18),(A77,b6,C19),(A77,b6,C20),(A77,b6,C21),(A77,b6,C22),(A77,b6,C23),(A77,b6,C24),(A 77,b6,C25),(A77,b6,C26),(A77,b6,C27),(A77,b6,C28),(A77,b6,C29),(A77,b7,C1),(A77,b7,C2) ,(A77,b7,C3),(A77,b7,C4),(A77,b7,C5),(A77,b7,C6),(A77,b7,C7),(A77,b7,C8),(A77,b7,C9),(A 77,b7,C10),(A77,b7,C11),(A77,b7,C12),(A77,b7,C13),(A77,b7,C14),(A77,b7,C15),(A77,b7,C 16),(A77,b7,C17),(A77,b7,C18),(A77,b7,C19),(A77,b7,C20),(A77,b7,C21),(A77,b7,C22),(A77 ,b7,C23),(A77,b7,C24),(A77,b7,C25)_{;}(A77,b7,C26),(A77,b7,C27),(A77,b7,C28),(A77,b7,C29) ,(A78,b1,C1),(A78,b1,C2),(A78,b1,C3),(A78,b1,C4),(A78,b1,C5),(A78,b1,C6),(A78,b1,C7),(A 78,b1,C8),(A78,b1,C9),(A78,b1,C10),(A78,b1,C11),(A78,b1,C12),(A78,b1,C13),(A78,b1,C14) ,(A78,b1,C5),(A78,b1,C16),(A78,b1,C17),(A78,b1,C18),(A78,b1,C19),(A78,b1,C20),(A78,b1 ,C21),(A78,b1,C22),(A78,b1,C23),(A78,b1,C24),(A78,b1,C25),(A78,b1,C26),(A78,b1,C27),(A 78,b1,C28),(A78,b1,C29),(A78,b2,C1),(A78,b2,C2),(A78,b2,C3),(A78,b2,C4),(A78,b2,C5),(A 78,b2,C6),(A78,b2,C7),(A78,b2,C8),(A78,b2,C9),(A78,b2,C10),(A78,b2,C11),(A78,b2,C12),( A78,b2,C13),(A78,b2,C14),(A78,b2,C15),(A78,b2,C16),(A78,b2,C17),(A78,b2,C18),(A78,b2, C19),(A78,b2,C20),(A78,b2,C21),(A78,b2,C22),(A78,b2,C23),(A78,b2,C24),(A78,b2,C25),(A 78,b2.C26),(A78,b2,C27),(A78,b2,C28),(A78,b2,C29),(A78,b3,C1),(A78,b3,C2),(A78,b3,C3),( A78,b3,C4),(A78,b3,C5),(A78,b3,C6),(A78,b3,C7),(A78,b3,C8),(A78,b3,C9),(A78,b3,C10),(A 78,b3,C11),(A78,b3,C12),(A78,b3,C13),(A78,b3,C14),(A78,b3,C15),(A78,b3,C16),(A78,b3,C 17),(A78,b3,C18),(A78,b3,C19),(A78,b3,C20),(A78,b3,C21),(A78,b3,C22),(A78,b3,C23),(A78 ,b3,C24),(A78,b3,C25),(A78,b3,C26),(A78,b3,C27),(A78,b3,C28),(A78,b3,C29),(A78,b4,C1),( A78,b4,C2),(A78,b4,C3),(A78,b4,C4),(A78,b4,C5),(A78,b4,C6),(A78,b4,C7),(A78,b4,C8),(A7 8,b4,C9),(A78,b4,C10),(A78,b4,C11),(A78,b4,C12),(A78,b4,C13),(A78,b4,C14),(A78,b4,C15) ,(A78,b4,C16),(A78,b4,C17),(A78,b4,C18),(A78,b4,C19),(A78,b4,C20),(A78,b4,C21),(A78,b4 ,C22),(A78,b4,C23),(A78,b4,C24),(A78,b4,C25),(A78,b4,C26),(A78,b4,C27),(A78,b4,C28),(A 78,b4,C29),(A78,b5,C1),(A78,b5,C2),(A78,b5,C3),(A78,b5,C4),(A78,b5,C5),(A78,b5,C6),(A7 8,b5,C7),(A78,b5,C8),(A78,b5,C9),(A78,b5,C10),(A78,b5,C11),(A78,b5,C12),(A78,b5,C13),( A78,b5,C14),(A78,b5,C15),(A78,b5,C16),(A78,b5,C17),(A78,b5,C18),(A78,b5,C19),(A78,b5, C20),(A78,b5,C21),(A78,b5,C22),(A78,b5,C23),(A78,b5,C24),(A78,b5,C25),(A78,b5,C26),(A 78,b5,C27),(A78,b5,C28),(A78,b5,C29),(A78,b6,C1),(A78,b6,C2),(A78,b6,C3),(A78,b6,C4),( A78,b6,C5),(A78,b6,C6),(A78,b6,C7),(A78,b6,C8),(A78,b6,C9),(A78,b6,C10),(A78,b6,C11),( A78,b6,C12),(A78,b6,C13),(A78,b6,C14),(A78,b6,C15),(A78,b6,C16),(A78,b6,C7),(A78,b6, C18),(A78,b6,C19),(A78,b6,C20),(A78,b6,C21),(A78,b6,C22),(A78,b6,C23),(A78,b6,C24),(A 78,b6,C25),(A78,b6,C26),(A78,b6,C27),(A78,b6,C28),(A78,b6,C29),(A78,b7,C1),(A78,b7,C2) ,(A78,b7,C3),(A78,b7,C4),(A78,b7,C5),(A78,b7,C6),(A78,b7,C7),(A78,b7,C8),(A78,b7,C9),(A 78,b7,C10),(A78,b7,C11),(A78,b7,C12),(A78,b7,C13),(A78,b7,C14),(A78,b7,C15),(A78,b7,C 16),(A78,b7,C17),(A78,b7,C18),(A78,b7,C19),(A78,b7,C20),(A78,b7,C21),(A78,b7,C22),(A78 ,b7,C23),(A78,b7,C24),(A78,b7,C25),(A78,b7,C26),(A78,b7,C27),(A78,b7,C28),(A78,b7,C29) ,(A79,b1,C1),(A79,bl,,C2),(A79,b1,C3),(A79,b1,C4),(A79,b1,C5),(A79,b1,C6),(A79,b1,C7),(A 79,b1,C8),(A79,b1,C9),(A79,b1,C10),(A79,b1,C11),(A79,b1,C12),(A79,b1,C13),(A79,b1,C14) ,(A79,b1,C15),(A79,b1,C16),(A79,b1,C17),(A79,b1,C18),(A79,b1,C19),(A79,b1,C20),(A79,b1 ,C21),(A79,b1,C22),(A79,b1,C23),(A79,b1,C24),(A79,b1,C25),(A79,b1,C26),(A79,b1,C27),(A 79,b1,C28),(A79,b1,C29),(A79,b2,C1),(A79,b2,C2),(A79,b2,C3),(A79,b2,C4),(A79,b2,C5),(A 79,b2,C6),(A79,b2,C7),(A79,b2,C8),(A79,b2,C9),(A79,b2,C10),(A79,b2,C11),(A79,b2,C12),( A79,b2,C13),(A79,b2,C14),(A79,b2,C15),(A79,b2,C16),(A79,b2,C17),(A79,b2,C18),(A79,b2, C19),(A79,b2,C20),(A79,b2,C21),(A79,b2,C22),(A79,b2,C23),(A79,b2,C24),(A79,b2,C25),(A 9,b2,C26),(A79,b2,C27),(A79,b2,C28),(A79,b2,C29),(A79,b3,C1),(A79,b3,C2),(A79,b3,C3),( A79,b3,C4),(A79,b3,C5),(A79,b3,C6),(A79,b3,C7),(A79,b3,C8),(A79,b3,C9),(A79,b3,C10),(A 79,b3,C11),(A79,b3,C12),(A79,b3,C13),(A79,b3,C14),(A79,b3,C15),(A79,b3,C16),(A79,b3,C 17),(A79,b3,C18),(A79,b3,C19),(A79,b3,C20),(A79,b3,C21),(A79,b3,C22),(A79,b3,C23),(A79 ,b3,C24),(A79,b3,C25),(A79,b3,C26),(A79,b3,C27),(A79,b3,C28),(A79,b3,C29),(A79,b4,C1),( A79,b4,C2),(A79,b4,C3),(A79,b4,C4),(A79,b4,C5),(A79,b4,C6),(A79,b4,C7),(A79,b4,C8),(A7 9,b4,C9),(A79,b4,C10),(A79,b4,C11),(A79,b4,C12),(A79,b4,C11),(A79,b4,C14),(A79,b4,C15) ,(A79,b4,C16),(A79,b4,C17),(A79,b4,C18),(A79,b4,C19),(A79,b4,C20),(A79,b4,C21),(A79,b4 ,C22),(A79,b4,C23),(A79,b4,C24),(A79,b4,C25),(A79,b4,C26),(A79,b4,C27),(A79,b4,C28),(A 79,b4,C29),(A79,b5,C1),(A79,b5,C2),(A79,b5,C3),(A79,b5,C4),(A79,b5,C5),(A79,b5,C6),(A7 9,b5,C7),(A79,b5,C8),(A79,b5,C9),(A79,b5,C10),(A79,b5,C11),(A79,b5,C12),(A79,b5,C13),( A79,b5,C14),(A79,b5,C15),(A79,b5,C16),(A79,b5,C17),(A79,b5,C18),(A79,b5,C19),(A79,b5, C20),(A79,b5,C21),(A79,b5,C22),(A79,b5,C23),(A79,b5,C24),(A79,b5,C25),(A79,b5,C26),(A 79,b5,C27),(A79,b5,C28),(A79,b5,C29),(A79,b6,C1),(A79,b6,C2),(A79,b6,C3),(A79,b6,C4),( A79,b6,C5),(A79,b6,C6),(A79,b6,C7),(A79,b6,C8),(A79,b6,C9),(A79,b6,C10),(A79,b6,C11),( A79,b6,C12),(A79,b6,C13),(A79,b6,C14),(A79,b6,C15),(A79,b6,C16),(A79,b6,C17),(A79,b6, C18),(A79,b6,C19),(A79,b6,C20),(A79,b6,C21),(A79,b6,C22),(A79,b6,C23),(A79,b6,C24),(A 79,b6,C25),(A79,b6,C26),(A79,b6,C27),(A79,b6,C28),(A79,b6,C29),(A79,b7,C1),(A79,b7,C2) ,(A79,b7,C3),(A79,b7,C4),(A79,b7,C5),(A79,b7,C6),(A79,b7,C7),(A79,b7,C8),(A79,b7,C9),(A 79,b7,C10),(A79,b7,C11),(A79,b7,C12),(A79,b7,C13),(A79,b7,C14),(A79,b7,C15),(A79,b7,C 16),(A79,b7,C17),(A79,b7,C18),(A79,b7,C19),(A79,b7,C20),(A79,b7,C21),(A79,b7,C22),(A79 ,b7,C23),(A79,b7,C24),(A79,b7,C25),(A79,b7,C26),(A79,b7,C27),(A79,b7,C28),(A79,b7,C29) ,(A80,b1,C1),(A80,b1,C2),(A80,b1,C3),(A80,b1,C4),(A80,b1,C5),(A80_{;}b1,C6),(A80,b1,C7),(A 80,b1,C8),(A80,b1,C9),(A80,b1,C10),(A80,b1,C11),(A80,b1,C12),(A80,b1,C13),(A80,b1,C14) ,(A80,b1,C15),(A80,b1,C16),(A80,b1,C17),(A80,b1,C18),(A80,b1,C19),(A80,b1,C20),(A80,b1 ,C21),(A80,b1,C22),(A80,b1,C23),(A80,b1,C24),(A80,b1,C25),(A80,b1,C26),(A80,b1,C27),(A 80,b1,C28),(A80,b1,C29),(A80,b2,C1),(A80,b2,C2),(A80,b2,C3),(A80,b2,C4),(A80,b2,C5),(A 80,b2,C6),(A80,b2,C7),(A80,b2,C8),(A80,b2,C9),(A80,b2,C10),(A80,b2,C11),(A80,b2,C12),( A80,b2,C13),(A80,b2,C14),(A80,b2,C15),(A80,b2,C16),(A80,b2,C17),(A80,b2,C18),(A80,b2, C19),(A80,b2,C20),(A80,b2,C21),(A80,b2,C22),(A80,b2,C23),(A80,b2,C24),(A80,b2,C25),(A 80,b2,C26),(A80,b2,C27),(A80,b2,C28),(A80,b2,C29),(A80,b3,C1),(A80,b3,C2),(A80,b3,C3),( A80,b3,C4),(A80,b3,C5),(A80,b3,C6),(A80,b3,C7),(A80,b3,C8),(A80,b3,C9),(A80,b3,C10),(A 80,b3,C11),)A80,b3,C12),(A80,b3,C13),(A80,b3,C14),(A80,b3,C15),(A80,b3,C16),(A80,b3,C 17),(A80,b3,C18),(A80,b3,C19),(A80,b3,C20),(A80,b3,C21),(A80,b3,C22),(A80,b3,C23),(A80 ,b3,C24),(A80,b3,C25),(A80,b3,C26),(A80,b3,C27),(A80,b3,C28),(A80,b3,C29),(A80,b4,C1),( A80,b4,C2),(A80,b4,C3),(A80,b4,C4),(A80,b4,C5),(A80,b4,C6),(A80,b4,C7),(A80,b4,C8),(A8 0,b4,C9),(A80,b4,C10),(A80,b4,C11),(A80,b4,C12),(A80,b4,C13),(A80,b4,C14),(A80,b4,C15) ,(A80,b4,C16),(A80,b4,C17),(A80,b4,C18),(A80,b4,C19),(A80,b4,C20),(A80,b4,C21),(A80,b4 ,C22),(A80,b4,C23),(A80,b4,C24),(A80,b4,C25),(A80,b4,C26),(A80,b4,C27),(A80,b4,C28),(A 80,b4,C29),(A80,b5,C1),(A80,b5,C2),(A80,b5,C3),(A80,b5,C4),(A80,b5,C5),(A80,b5,C6),(A8 0,b5,C7),(A80,b5,C8),(A80,b5,C9),(A80,b5,C10),(A80,b5,C11),(A80,b5,C12),(A80,b5,C13),( A80,b5,G14),(A80,b5,C15),(A80,b5,C16),(A80,b5,C17),(A80,b5,C18),(A80,b5,C19),(A80,b5, C20),(A80,b5,C21),(A80,b5,C22),(A80,b5,C23),(A80,b5,C24),(A80,b5,C25),(A80,b5,C26),(A 80,b5,C27),(A80,b5,C28),(A80,b5,C29),(A80,b6,C1),(A80,b6,C2),(A80,b6,C3),(A80,b6,C4),( A80,b6,C1),(A80,b6,C6),(A80,b6,C7),(A80,b6,C8),(A80,b6,C9),(A80,b6,C10),(A80,b6,C11),( A80,b6,C12),(A80,b6,C13),(A80,b6,C14),(A80,b6,C15),(A80,b6,C16),(A80,b6,C17),(A80,b6, C18),(A80,b6,C19),(A80,b6,C20),(A80,b6,C21),(A80,b6,(C22),(A80,b6,C23),(A80,b6,C24),(A 80,b6,C25),(ASO,b6,C26),(A80,b6,C27),(A80,b6,C28),(A80,b6,C29),(A80,b7,C1),(A80,b7,C2) ,(A80,b7,C3),(A80,b7,C4),(A80,b7,C5),(A80,b7,C6),(A80,b7,C7),(A80,b7,C8),(A80,b7,C9),(A 80,b7,C10),(A80,b7,C11),(A80,b7,C12),(A80,b7,C13),(A80,b7,C14),(A80,b7,C15),(A80,b7,C 16),(A80,b7,C17),(A80,b7,C18),(A80,b7,C19),(A80,b7,C20),(A80,b7,C21),(A80,b7,C22),(A80 ,b7,C23),(A80,b7,C24),(A80,b7,C25),(A80,b7,C26),(A80,b7,C27),(A80,b7,C28),(A80,b7,C29),

(A81,b1,C1),(A81,b1,C2),(A81,b1,C3),(A81,b1,C4),(A81,b1,C5),(A81,b1,C6),(A81,b1,C7),(A 81,b1,C8),(A81,b1,C9),(A81,b1,C10),(A81,b1,C11),(A81,b1,C12),(A81,b1,C13),(A81,b1,C14) ,(A81,b1,C15),(A81,b1,C16),(A81,b1,C17),(A81,b1,C18),(A81,b1,C19),(A81,b1,C20),(A81,b1 ,C21),(A81,b1,C22),(A81,b1,C23),(A81,b1,C24),(A81,b1,C25),(A81,b1,C26),(A81,b1,C27),(A 81,b1,C28),(A81,b1,C29),(A81,b2,C1),(A81,b2,C2),(A81,b2,C3),(A81,b2,C4),(A81,b2,C5),(A 81,b2,C6),(A81,b2,C7),(A81,b2,C8),(A81,b2,C9),(A81,b2,C10),(A81,b2,C11),(A81,b2,C12),( A81,b2,C13),(A81,b2,C14),(A81,b2,C15),(A81,b2,C16),(A81,b2,C17),(A81,b2,C18),(A81,b2, C19),(A81,b2,C20),(A81,b2,C21),(A81,b2,C22),(A81,b2,C23),(A81,b2,C24),(A81,b2,C25),(A 81,b2,C26),(A81,b2,C27),(A81,b2,C28),(A81,b2,C29),(A81,b3,C1),(A81,b3,C2),(A81,b3,C3),( A81,b3,C4),(A81,b3,C5),(A81,b3,C6),(A81,b3,C7),(A81,b3,C8),(A81,b3,C9),(A81,b3,C10),(A 81,b3,C11),(A81,b3,C12),(A81,b3,C13),(A81,b3,C14),(A81,b3,C15),(A81,b3,C16),(A81,b3,C 17),(A81,b3,C18),(A81,b3,C19),(A81,b3,C20),(A81,b3,C21),(A81,b3,C22),(A81,b3,C23),(A81 ,b3,C24),(A81,b3,C25),(A81,b3,C26),(A81,b3,C27),(A81,b3,C28),(A81,b3,C29),(A81,b4,C1),( A81,b4,C2),(A81,b4,C3),(A81,b4,C4),(A81,b4,C5),(A81,b4,C6),(A81,b4,C7),(A81,b4,C8),(A8 1,b4,C9),(A81,b4,C10),(A81,b4,C11),(A81,b4,C12),(A81,b4,C13),(A81,b4,C14),(A81,b4,C15) ,(A81,b4,C16),(A81,b4,C17),(A81,b4,C18),(A81,b4,C19),(A81,b4,C20),(A81,b4,C21),(A81,b4 ,C22),(A81,b4,C23),(A81,b4,C24),(A81,b4,C25),(A81,b4,C26),(A81,b4,C27),(A81,b4,C28),(A 81,b4,C29),(A81,b5,C1),(A81,b5,C2),(A81,b5,C3),(A81,b5,C4),(A81,b5,C5),(A81,b5,C6),(A8 1,b5,C7),(A81,b5,C8),(A8,b5,C9),(A81,b5,C10),(A81,b5,C11),(A81,b5,C12),(A81,b5,C13),( A81,b5,C14),(A81,b5,C15),(A81,b5,C16),(A81,b5,C17),(A81,b5,C18),(A81,b5,C19),(A81,b5, C20),(A81,b5,C21),(A81,b5,C22),(A81,b5,C23),(A81,b5,C24),(A81,b5,C25),(A81,b5,C26),(A 81,b5,C27),(A81,b5,C28),(A81,b5,C29),(A81,b6,C1),(A81,b6,C2),(A81,b6,C3),(A81,b6,C4),( A81,b6,C5),(A81,b6,C6),(A81,b6,C7),(A81,b6,C8),(A81,b6,C9),(A81,b6,C10),(A81,b6,C11),( A81,b6,C12),(A81,b6,C13),(A81,b6,C14),(A81,b6,C15),(A81,b6,C16),(A81,b6,C17),(A81,b6, C18),(A81,b6,C19),(A81,b6,C20),(A81,b6,C21),(A81,b6,C22),(A81,b6,C23),(A81,b6,C24),(A 81,b6,C25),(A81,b6,C26),(A81,b6,C27),(A81,b6,C28),(A81,b6,C29),(A81,b7,C1),(A81,b7,C2) ,(A81,b7,C3),(A81,b7,C4),(A81,b7,C5),(A81,b7,C6),(A81,b7,C7),(A81,b7,C8),(A81,b7,C9),(A 81,b7,C10),(A81,b7,C11),(A81,b7,C12),(A81,b7,C13),(A81,b7,C14),(A81,b7,C15),(A81,b7,C 16),(A81,b7,C17),(A81,b7,C18),(A81,b7,C19),(A81,b7,C20),(A81,b7,C21),(A81,b7,C22),(A81 ,b7,C23),(A81,b7,C24),(A81,b7,C25),(A81,b7,C26),(A81,b7,C27),(A81,b7,C28),(A81,b7,C29) ,(A82,b1,C1),(A82,b1,C2),(A82,b1,C3),(A82,b1,C4),(A82,b1,C5),(A82,b1,C6),(A82,b1,C7),(A 82,b1,C8),(A82,b1,C9),(A82,b1,C10),(A82,b1,C11),(A82,b1,C12),(A82,b1,C13),(A82,b1,C14) ,(A82,b1,C15),(A82,b1,C16),(A82,b1,C17),(A82,b1,C18),(A82,b1,C19),(A82,b1,C20),(A82,b1 ,C21),(A82,b1,C22),(A82,b1,C23),(A82,b1,C24),(A82,b1,C25),(A82,b1,C26),(A82,b1,C27),(A 82,b1,C28),(A82,b1,C29),(A82,b2,C1),(A82,b2,C2),(A82,b2,C3),(A82,b2,C4),(A82,b2,C5),(A 82,b2,C6),(A82,b2,C7),(A82,b2,C8),(A82,b2,C9),(A82,b2,C10),(A82,b2,C11),(A82,b2,C12),( A82,b2,C13),(A82,b2,C14),(A82,b2,C15),(A82,b2,C16),(A82,b2,C17),(A82,b2,C18),(A82,b2, C19),(A82,b2,C20),(A82,b2,C21),(A.82,b2,C22),(A82,b2,C23),(A82,b2,C24),(A82,b2,C25),(A 82,b2,C26),(A82,b2,C27),(A82,b2,C28),(A82,b2,C29),(A82,b3,C1),(A82,b3,C2),(A82,b3,C3),( A82,b3,C4),(A82,b3,C5),(A82,b3,C6),(A82,b3,C7),(A82,b3,C8),(A82,b3,C9),(A82,b3,C10),(A 82,b3,C11),(A82,b3,C12),(A82,b3,C13),(A82,b3,C14),(A82,b3,C15),(A82,b3,C16),(A82,b3,C 17),(A82,b3,C18),(A82,b3,C19),(A82,b3,C20),(A82,b3,C21),(A82,b3,C22),(A82,b3,C23),(A82 ,b3,C24),(A82,b3,C25),(A82,b3,C26),(A82,b3,C27),(A82,b3,C28),(A82,b3,C29),(A82,b4,C1),( A82,b4,C2),(A82,b4,C3),(A82,b4,C4),(A82,b4,C5),(A82,b4,C6),(A82,b4,C7),(A82,b4,C8),(A8 2,b4,C9),(A82,b4,C10),(A82,b4,C11),(A82,b4,C12),(A82,b4,C13),(A82,b4,C14),(A82,b4,C15) ,(A82,b4,C16),(A82,b4,C17),(A82,b4,C18),(A82,b4,C19),(A82,b4,C20),(A82,b4,C21),(A82,b4 ,C22),(A82,b4,C23),(A82,b4,C24),(A82,b4,C25),(A82,b4,C26),(A82,b4,C27),(A82,b4,C28),(A 82,b4,C29),(A82,b5,C1),(A82,b5,C2),(A82,b5,C3),(A82,b5,C4),(A82,b5,C5),(A82,b5,C6),(A8 2,b5,C7),(A82,b5,C8),(A82,b5,C9),(A82,b5,C10),(A82,b5,C11),(A82,b5,C12),(A82,b5,C13),( A82,b5,C14),(A82,b5,C15),(A82,b5,C16),(A82,b5,C17),(A82,b5,C18),(A82,b5,C19),(A82,b5, C20),(A82,b5,C21),(A82,b5,C22),(A82,b5,C23),(A82,b5,C24),(A82,b5,C25),(A82,b5,C26),(A 82,b5,C27),(A82,b5,C28),(A82,b5,C29),(A82,b6,C1),(A82,b6,C2),(A82,b6,C3),(A82,b6,C4),( A82,b6,C5),(A82,b6,C6),(A82,b6,C7),(A82,b6,C8),(A82,b6,C9),(A82,b6,C10),(A82,b6,C11),( A82,b6,C12),(A82,b6,C13),(A82,b6,C14),(A82,b6,C15),(A82,b6,C16),(A82,b6,C17),(A82,b6, C18),(A82,b6,C19),(A82,b6,C20),(A82,b6,C21),(A82,b6,C22),(A82,b6,C23),(A82,b6,C24),(A 82,b6,C25),(A82,b6,C26),(A82,b6,C27),(A82,b6,C28),(1A82,b6,C29),(A82,b7,C1),(A82,b7,C2) ,(A82_{;}b7,C3),(A82,b7,C4),(A82,b7,C5),(A82,b7,C6),(A82,b7,C7),(A82,b7,C8),(A82,b7,C9),(A 82,b7,C10),(A82,b7,C11),(A82,b7,C12),(A82,b7,C13),(A82,b7,C14),(A82,b7,C15),(A82,b7,C 16),(A82,b7,C17),(A82,b7,C18),(A82,b7,C19),(A82,b7,C20),(A82,b7,C21),(A82,b7,C22),(A82 ,b7,C23),(A82,b7,C24),(A82,b7,C25),(A82,b7,C26),(A82,b7,C27),(A82,b7,C28),(A82,b7,C29) ,(A83,b1,C1),(A83,b1,C2),(A83,b1,C3),(A83,b1,C4),(A83,b1,C5),(A83,b1,C6),(A83,b1,C7),(A 83,b1,C8),(A83,b1,C9),(A83,b1,C10),(A83,b1,C11),(A83,b1,C12),(A83,b1,C13),(A83,b1,C14) ,(A83,b1,C15),(A83,b1,C16),(A83,b1,C17),(A83,b1,C18),(A83,b1,C19),(A83,b1,C20),(A83,b1 ,C21),(A83,b1,C22),(A83,b1,C23),(A83,b1,C24),(A83,b1,C25),(A83,b1,C26),(A83,b1,C27),(A 83,b1,C28),(A83,b1,C29),(A83,b2,C1),(A83,b2,C2),(A83,b2,C3),(A83,b2,C4),(A83,b2,C5),(A 83,b2,C6),(A83,b2,C7),(A83,b2,C8),(A83,b2,C9),(A83,b2,C10),(A83,b2,C11),(A83,b2,C12),( A83,b2,C13),(A83,b2,C14),(A83,b2,C15),(A83,b2,C16),(A83,b2,C17),(A83,b2,C18),(A83,b2, C19),(A83,b2,C20),(A83,b2,C21),(A83,b2,C22),(A83,b2,C23),(A83,b2,C24),(A83,b2,C25),(A 83,b2,C26),(A83,b2,C27),(A83,b2,C28),(A83,b2,C29),(A83,b3,C1),(A83,b3,C2),(A83,b3,C3),( A83,b3,C4),(A83,b3,C5),(A83,b3,C6),(A83,b3,C7),(A83,b3,C8),(A83,b3,C9),(A83,b3,C10),(A 83,b3,C11),(A83,b3,C12),(A83,b3,C13),(A83,b3,C14),(A83,b3,C15),(A83,b3,C16),(A83,b3,C 17),(A83,b3,C18),(A83,b3,C19),(A83,b3,C20),(A83,b3,C21),(A83,b3,C22),(A83,b3,C23),(A83 ,b3,C24),(A83,b3,C25),(A83,b3,C26),(A83,b3,C27),(A83,b3,C28),(A83,b3,C29),(A83,b4,C1),( A83,b4,C2),(A83,b4,C3),(A83,b4,C4),(A83,b4,C5),(A83,b4,C6),(A83,b4,C7),(A83,b4,C8),(A8 3,b4,C9),(A83,b4,C10),(A83,b4,C11),(A83,b4,C12),(A83,b4,C13),(A83,b4,C14),(A83,b4,C15) ,(A83,b4,C16),(A.83,b4,C17),(A83,b4,C18),(A83,b4,C19),(A83,b4,C20),(A83,b4,C21),(A83,b4 ,C22),(A83,b4,C23),(A83,b4,C24),(A83,b4,C25),(A83,b4,C26),(A83,b4,C27),(A83,b4,C28),(A 83,b4,C29),(A83,b5,C1),(A83,b5,C2),(A83,b5,C3),(A83,b5,C4),(A83,b5,C5),(A83,b5,C6),(A8 3,b5,C7),(A83,b5,C8),(A83,b5,C9),(A83,b5,C10),(A83,b5,C11),(A83,b5,C12),(A83,b5,C13),( A83,b5,C14),(A83,b5,C15),(A83,b5,C16),(A83,b5,C7),(A83,b5,C18),(A83,b5,C19),(A83,b5, C20),(A83,b5,C21),(A83,b5,C22),(A83,b5,C23),(A83,b5,C24),(A83,b5,C25),(A83,b5,C26),(A 83,b5,C27),(A83,b5,C28),(A83,b5,C29),(A83,b6,C1),(A83,b6,C2),(A83,b6,C3),(A83,b6,C4),( A83,b6,C5),(A83,b6,C6),(A83,b6,C7),(A83,b6,C8),(A83,b6,C9),(A83,b6,C10),(A83,b6,C11),( A83,b6,C12),(A83,b6,C13),(A83,b6,C14),(A83,b6,C1),(A83,b6,C16),(A83,b6,C17),(A83,b6, C18),(A83,b6,C19),(A83,b6,C20),(A83,b6,C21),(A83,b6,C22),(A83,b6,C23),(A83,b6,C24),(A 83,b6,C25),(A83,b6,C26),(A83,b6,C27),(A83,b6,C28),(A83,b6,C29),(A83,b7,C1),(A83,b7,C2) ,(A83,b7,C3),(A83,b7,C4),(A83,b7,C5),(A83,b7,C6),(A83,b7,C7),(A83,b7,C8),(A83,b7,C9),(A 83,b7,C10),(A83,b7,C11),(A83,b7,C12),(A83,b7,C13),(483,b7,C14),(A83,b7,C15),(A83,b7,C 16),(A83,b7,C17),(A83,b7,C18),(A83,b7,C19),(A83,b7,C20),(A83,b7,C21),(A83,b7,C22),(A83 ,b7,C23),(A83,b7,C24),(A83,b7,C25),(A83,b7,C26),(A83,b7,C27),(A83,b7,C28),(A83,b7,C29) ,(A84,b1,C1),(A84,b1,C2),(A84,b1,C3),(A84,b1,C4),(A84,b1,C5),(A84,b1,C6),(A84,b1,C7),(A 84,b1,C8),(A84,b1,C9),(A84,b1,C10),(A84,b1,C11),(A84,b1,C12),(A84,b1,C13),(A84,b1,C14) ,(A84,b1,C15),(A84,b1,C16),(A84,b1,C17),(A84,b1,C18),(A84,b1,C19),(A84,b1,C20),(A84,b1 ,C21),(A84,b1,C22),(A84,b1,C23),(A84,b1,C24),(A84,b1,*C25),*(A84,b1,C26),(A84,b1,C27),(A 84,b1,C28),(A84,b1,C29),(.A84,b2,C1),(A84,b2,C2),(A84,b2,C3),(A84,b2,C4),(A84,b2,C5),(A 84,b2,C6),(A84,b2,C7),(A84,b2,C8),(A84,b2,C9),(A84,b2,C10),(A84,b2,C11),(A84,b2,C12),( A84,b2,C13),(A84,b2,C14),(A84,b2,C15),(A84,b2,C16),(A84,b2,C17),(A84,b2,C18),(A84,b2, C19),(A84,b2,C20),(A84,b2,C21),(A84,b2,C22),(A84,b2,C23),(A84,b2,C24),(A84,b2,C25),(A 84,b2,C26),(A84,b2,C27),(A84,b2,C28),(A84,b2,C29),(A84,b3,C1),(A84,b3,C2),(A84,b3,C3),( A84,b3,C4),(A84,b3,C5),(A84,b3,C6),(A84,b3,C7),(A84,b3,C8),(A84,b3,C9),(A84,b3,C10),(A 84,b3,C11),(A84,b3,C12),(A84,b3,C13),(A84,b3,C14),(A84,b3,C15),(A84,b3,C16),(A84,b3,C 17),(A84,b3,C18),(A84,b3,C19),(A84,b3,C20),(A84,b3,C21),(A84,b3,C22),(A84,b3,C23),(A84 ,b3,C24),(A84,b3,C25),(A84,b3,C26),(A84,b3,C27),(A84,b3,C28),(A84,b3,C29),(A84,b4,C1),( A84,b4,C2),(A84,b4,C3),(A84,b4,C4),(A84,b4,C5),(A84,b4,C6),(A84,b4,C7),(A84,b4,C8),(A8 4,b4,C9),(A84,b4,C10),(A84,b4,Cll),(A84,b4,C12),(A84,b4,C13),(A84,b4,C14),(A84,b4,C5) ,(A84,b4,C16),(A84,b4,C17),(A84,b4,C18),(A84,b4,C19),(A84,b4,C20),(A84,b4,C21),(A84,b4 ,C22),(A84,b4,C23),(A84,b4,C24),(A84,b4,C25),(A84,b4,C26),(A84,b4,C27),(A84,b4,C28),(A 84,b4,C29),(A84,b5,C1),(A84,b5,C2),(A84,b5,C3),(A84,b5,C4),(A84,b5,C5),(A84,b5,C6),(A8 4,b5,C7),(A84,b5,C8),(A84,b5,C9),(A84,b5,C10),(A84,b5,C11),(A84,b5,C12),(A84,b5,C13),( A84,b5,C14),(A84,b5,C15),(A84,b5,C16),(A84,b5,C17),(A84,b5,C18),(A84,b5,C19),(A84,b5, C20),(A84,b5,C21),(A84,b5,C22),(A84,b5,C23),(A84,b5,C24),(A84,b5,C25),(A84,b5,C26),(A 84,b5,C27),(A84,b5,C28),(A84,b5,C29),(A84,b6,C1),(A84,b6,C2),(A84,b6,C3),(A84,b6,C4),( A84,b6,C5),(A84,b6,C6),(A84,b6,C7),(A84,b6,C8),(A84,b6,C9),(A84,b6,C10),(A84,b6,C11),( A84,b6,C12),(A84,b6,C13),(A84,b6,C14),(A84,b6,C15),(A84,b6,C16),(A84,b6,C17),(A84,b6, C18),(A84,b6,C19),(A84,b6,C20),(A84,b6,C21),(A84,b6,C22),(A84,b6,C23),(A84,b6,C24),(A 84,b6,C25),(A84,b6,C26),(A84,b6,C27),(A84,b6,C28),(A84,b6,C29),(A84,b7,C1),(A84,b7,C2) ,(A84,b7,C3),(A84,b7,C4),(A84,b7,C5),(A84,b7,C6),(A84,b7,C7),(A84,b7,C8),(A84,b7,C9),(A 84,b7,C10),(A84,b7,C11),(A84,b7,C12),(A84,b7,C13),(A84,b7,C14),(A84,b7,C15),(A84,b7,C 16),(A84,b7,C17),(A84,b7,C18),(A84,b7,C19),(A84,b7,C20),(A84,b7,C21),(A84,b7,C22),(A84 ,b7,C23),(A84,b7,C24),(A84,b7,C25),(A84,b7,C26),(A84,b7,C27),(A84,b7,C28),(A84,b7,C29) ,(A85,b1,C1),(A85,b1,C2),(A85,b1,C3),(A85,b1,C4),(A85,b1,C5),(A85,b1,C6),(A85,b1,C7),(A 85,b1,C8),(A85,b1,C9),(A85,b1,C10),(A85,b1,C11),(A85,b1,C12),(A85,b1,C13),(A85,b1,C14) (A85,b1,C15),(A85,b1,C16),(A85,b1,C17),(A85,b1,C18),(A85,b1,C19),(A85,b1,C20),(A85,b1 ,21),(A85,b1,C22),(A85,b1,C23),(485,b1,C24),(A85,b1,C25),(A85,b1,C26),(A85,b1,C27),(A 85,b1,C28),(A85,b1,C29),(A85,b2,C1),(A85,b2,C2),(A85,b2,C3),(A85,b2,C4),(A85,b2,C5),(A 85,b2,C6),(A85,b2,C7),(A85,b2,C8),(A85,b2,C9),(A85,b2,C10),(A85,b2,C11),(A85,b2,C12),( A85,b2,C13),(A85,b2,C14),(A85,b2,C15),(A85,b2,C16),(A85,b2,C17),(A85,b2,C18),(A85,b2, C19),(A85,b2,C20),(A85,b2,C21),(A85,b2,C22),(A85,b2,C23),(A85,b2,C24),(A85,b2,C25),(A 85,b2,C26),(A85,b2,C27),(A85,b2,C28),(A85,b2,C29),(A85,b3,C1),(A85,b3,C2),(A85,b3,C3),( A85,b3,C4),(A85,b3,C5),(A85,b3,C6),(A85,b3,C7),(A85,b3,C8),(A85,b3,C9),(A85,b3,C10),(A 85,b3,C11),(A85,b3,C12),(A85,b3,C13),(A85,b3,C14),(A85,b3,C15),(A85,b3,C16),(A85,b3,C 17),(A85,b3,C18),(A85,b3,C19),(A85,b3,C20),(A85,b3,C21),(A85,b3,C22),(A85,b3,C23),(A85 ,b3,C24),(A85,b3,C25),(A85,b3,C26),(A85,b3,C27),(A85,b3,C28),(A85,b3,C29),(A85,b4,C1),( A85,b4,C2),(A85,b4,C3),(A85,b4,C4),(A85,b4,C5),(A85,b4,C6),(A85,b4,C7),(A85,b4,C8),(A8 5,b4,C9),(A85,b4,C10),(A85,b4,C11),(A85,b4,C12),(A85,b4,C13),(A85,b4,C14),(A85,b4,C15) ,(A85,b4,C16),(A85,b4,C17),(A85,b4,C18),(A85,b4,C19),(A85,b4,C20),(A85,b4,C21),f,A85,b4 ,C22),(A85,b4,C23),(A85,b4,C24),(A85,b4,C25),(A85,b4,C26),(A85,b4,C27),(A85,b4,C28),(A 85,b4,C29),(A85,b5,C1),(A85,b5,C2),(A85,b5,C3),(A85,b5,C4),(A85,b5,C5),(A85,b5,C6),(A8 5,b5,C7),(A85,b5,C8),(A85,b5,C9),(A85,b5,C10),(A85,b5,C11),(A85,b5,C12),(A85,b5,C13),( A85,b5,C4),(A85,b5,C15),(A85,b5,C16),(A85,b5,C7),(A85,b5,C8),(A85,b5,C9),(A85,b5, C20),(A85,b5,C21),(A85,b5,C22),(A85,b5,C23),(A85,b5,C24),(A85,b5,C25),(A85,b5,C26),(A 85,b5,C27),(A85,b5,C28),(A85,b5,C29),(A85,b6,C1),(A85,b6,C2),(A85,b6,C3),(A85,b6,C4),( A85,b6,C5),(A85,b6,C6),(A85,b6,C7),(A85,b6,C8),(A85,b6,C9),(A85,b6,C10),(A85,b6,C11),( A85,b6,C12),(A85,b6,C13),(A85,b6,C14),(A85,b6,C15),(A85,b6,C16),(A85,b6,C17),(A85,b6, C18),(A85,b6,C19),(A85,b6,C20),(A85,b6,C21),(A85,b6,C22),(A85,b6,C23),(A85,b6,C24),(A 85,b6,C25),(A85,b6,C26),(A85,b6,C27),(A85,b6,C28),(A85,b6,C29),(A85,b7,C1),(A85,b7,C2) ,(A85,b7,C3),(A85,b7,C4),(A85,b7,C5),(A85,b7,C6),(A85,b7,C7),(A85,b7,C8),(A85,b7,C9),(A 85,b7,C10),(A85,b7,C11),(A85,b7,C12),(A85,b7,C13),(A85,b7,C14),(A85,b7,C15),(A85,b7,C 16),(A85,b7,C17),(A85,b7,C18),(A85,b7,C19),(A85,b7,C20),(A85,b7,C21),(A85,b7,C22),(A85 ,b7,C23),(A85,b7,C24),(A85,b7,C25),(A85,b7,C26),(A85,b7,C27),(A85,b7,C28),(A85,b7,C29) ,(A86,b1,C1),(A86,b1,C2),(A86,b1,C3),(A86,b1,C4),(A86,b1,C5),(A86,b1,C6),(A86,b1,C7),(A 86,b1,C8),(A86,b1,C9),(A86,b1,C10),(A86,b1,C11),(A86,b1,C12),(A86,b1,C13),(A86,b1,C14) ,(A86,b1,C15),(A86,b1,C16),(A86,b1,C17),(A86,b1,C18),(A86,b1,C19),(A86,b1,C20),(A86,b1 ,C21),(A86,b1,C22},(A86,b1,C23),(A86,b1,C24),(A86,b1,C25),(A86,b1,C26),(A86,b1,C27),(A 86,b1,C28),(A86,b1,C29),(A86,b2,C1),(A86,b2,C2),(A86,b2,C3),(A86,b2,C4),(A86,b2,C5),(A 86,b2,C6),(A86,b2,C7),(A86,b2,C8),(A86,b2,C9),(A86,b2,C10),(A86,b2,C11),(A86,b2,C12),( A86,b2,C13),(A86,b2,C14),(A86,b2,C15),(A86,b2,C16),(A86,b2,C17),(A86,b2,C18),(A86,b2, C19),(A86,b2,C20),(A86,b2,C21),(A86,b2,C22),(A86,b2,C23),(A86,b2,C24),(A86,b2,C25),(A 86,b2,C26),(A86,b2,C27),(A86,b2,C28),(A86,b2,C,29),(A86,b3,C1),(A86,b3,C2),(A86,b3,C3),( A86,b3,C4),(A86,b3,C5),(A86,b3,C6),(A86,b3,C7),(A86,b3,C8),(A86,b3,C9),(A86,b3,C10),(A. 86,b3,C11),(A86,b3,C12),(A86,b3,C13),(A86,b3,C14),(A86,b3,C15),(A86,b3,C16),(A86,b3,C 17),(A86,b3,C18),(A86,b3,C19),(A86,b3,C20),(A86,b3,C21),(A86,b3,C22),(A86,b3,C23),(A86 ,b3,C24),(A86,b3,C25),(A86,b3,C26),(A86,b3,C27),(A86,b3,C28),(A86,b3,C29),(A86,b4,C1),( A86,b4,C2),(A86,b4,C3),(A86,b4,C4),(A86,b4,C5),(A86,b4,C6),(A86,b4,C7),(A86,b4,C8),(A8 6,b4,C9),(A86,b4,C10),(A86,b4,C11),(A86,b4,C12),(A86,b4,C13),(A86,b4,C14),(A86,b4,C15) ,(A86,b4,C16),(A86,b4,C17),(A86,b4,C18),(A86,b4,C19),(A86,b4,C20),(A86,b4,C21),(A86,b4 ,C22),(486,b4,C23),(A86,b4,C24),(A86,b4,C25),(486,b4,C26),(A86,b4,C27),(A86,b4,C28),(A 86,b4,C29),(A86,b5,C1),(A86,b5,C2),(A86,b5,C3),(A86,b5,C4),(A86,b5,C5),(A86,b5,C6),(A8 6,b5,C7),(A86,b5,C8),(A86,b5,C9),(A86,b5,C10),(A86,b5,C11),(A86,b5,C12),(A86,b5,C13),( A86,b5,C14),(A86,b5,C15),(A86,b5,C16),(A86,b5,C17),(A86,b5,C18),(A86,b5,C19),(A86,b5, C20),(A86,b5,C21),(A86,b5,C22),(A86,b5,C23),(A86,b5,C24),(A86,b5,C25),(A86,b5,C26),(A 86,b5,C27),(A86,b5,C28),(A86,b5,C29),(A86,b6,C1),(A86,b6,C2),(A86,b6,C3),(A86,b6,C4),( A86,b6,C5),(A86,b6,C6),(A86,b6,C7),(A86,b6,C8),(A86,b6,C9),(A86,b6,C10),(A86,b6,C11),( A86,b6,C12),(A86,b6,C13),(A86,b6,C14),(A86,b6,C15),(A86,b6,C16),(A86,b6,C17),(A86,b6, C18),(A86,b6,C19),(A86,b6,C20),(A86,b6,C21),(A86,b6,C22),(A86,b6,C23),(A86,b6,C24),(A 86,b6,C25),(A86,b6,C26),(A86,b6,C27),(A86,b6,C28),(A86,b6,C29),(A86,b7,C1),(A86,b7,C2) ,(A86,b7,C3),(A86,b7,C4),(A86,b7,C5),(A86,b7,C6),(A86,b7,C7),(A86,b7,C8),(A86,b7,C9),(A 86,b7,C10),(A86,b7,C11),(A86,b7,C12),(A86,b7,C13),(A86,b7,C14),(A86,b7,C15),(A86,b7,C 16),(A86,b7,C17),(A86,b7,C18),(A86,b7,C19),(A86,b7,C20),(A86,b7,C21),(A86,b7,C22),(A86 ,b7,C23),(A86,b7,C24),(A86,b7,C25),(A86,b7,C26),(A86,b7,C27),(A86,b7,C28),(A86,b7,C29) ,(A87,b1,C1),(A87,b1,C2),(A87,b1,C3),(A87,b1,C4),(A87,b1,C5),(A87,b1,C6),(A87,b1,C7),(A 87,b1,C8),(A87,b1,C9),(A87,b1,C10),(A87,b1,C11),(A87,b1,C12),(A87,b1,C13),(A87,b1,C14) ,(A87,b1,C15),(A87,b1,C16),(A87,b1,C17),(A87,b1,C18),(A81,b1,C19),(A87,b1,C20),(A87,b1, ,C21),(A87,b.1,C22),(A87,b1,C23),(A87,b1,C24),(A87,b1,C25),(A87,b1,C26),(A87,b1,C27),(A 87,b1,C28),(A87,b1,C29),(A87,b2,C1),(A87,b2,C2),(A87,b2,C3),(A87,b2,C4),(A87,b2,C5),(A 87,b2,C6),(A87,b2,C7),(A87,b2,C8),(A87,b2,C9),(A87,b2,C10),(A87,b2,C11),(A87,b2,C12),( A87,b2,C3),(A87,b2,C14),(A87,b2,C5),(A87,b2,C16),(A87,b2,C17),(A87,b2,C8),(A87,b2, C19),(A87,b2,C20),(A87,b2,C21),(A87,b2,C22),(A87,b2,C23),(A87,b2,C24),(A87,b2,C25),(A 87,b2,C26),(A87,b2,C27),(A87,b2,C28),(A87,b2,C29),(A87,b3,C1),(A87,b3,C2),(A87,b3,C3),( A87,b3,C4),(A87,b3,C5),(A87,b3,C6),(A87,b3,C7),(A87,b3,C8),(A87,b3,C9),(A87,b3,C10),(A 87,b3,C11),(A87,b3,C12),(A87,b3,C13),(A87,b3,C14),(A87,b3,C15),(A87,b3,C16),(A87,b3,C 17),(A87,b3,C18),(A87,b3,C19),(A87,b3,C20),(A87,b3,C21),(A87,b3,C22),(A87,b3,C23),(A87 ,b3,C24),(A87,b3,C25),(A87,b3,C26),(A87,b3,C27),(A87,b3,C28),(A87,b3,C29),(A87,b4,C1),( A87,b4,C2),(A87,b4,C3),(A87,b4,C4),(A87,b4,C5),(A87,b4,C6),(A87,b4,C7),(A87,b4,C8),(A8 7,b4,C9),(A87,b4,C10),(A87,b4,C11),(A87,b4,C12),(A87,b4,C3),(A87,b4,C4),(A87,b4,C5) ,(A87,b4,C16),(A87,b4,C17),(A87,b4,C18),(A87,b4,C19),(A87,b4,C20),(A87,b4,C21),(A87,b4 ,C22),(A87,b4,C23),(A87,b4,C24),(A87,b4,C25),(A87,b4,C26),(A87,b4,C27),(A87,b4,C28),(A 87,b4,C29),(A87,b5,C1),(A87,b5,C2),(A87,b5,C3),(A87,b5,C4),(A87,b5,C5),(A87,b5,C6),(A8 7,b5,C7),(A87,b5,C8),(A87,b5,C9),(A87,b5,C10),(A87,b5,C11),(A87,b5,C12),(A87,b5,C13),( A87,b5,C14),(A87,b5,C15),(A87,b5,C16),(A87,b5,C17),(A87,b5,C18),(A87,b5,C19),(A87,b5, C20),(A87,b5,C21),(A87,b5,C22),(A87,b5,C23),(A87,b5,C24),(A87,b5,C25),(A87,b5,C26),(A 87,b5,C27),(A87,b5,C28),(A87,b5,C29),(A87,b6,C1),(A87,b6,C2),(A87,b6,C3),(A87,b6,C4),( A87,b6,C5),(A87,b6,C6),(A87,b6,C7),(A87,b6,C8),(A87,b6,C9),(A87,b6,C10),(A87,b6,C11),( A87,b6,C12),(A87,b6,C13),(A87,b6,C14),(A87,b6,C15),(A87,b6,C16),(A87,b6,C17),(A87,b6, C18),(A87,b6,C19),(A87,b6,C20),(A87,b6,C21),(A87,b6,C22),(A87,b6,C23),(A87,b6,C24),(A 87,b6,C25),(A87,b6,C26),(A87,b6,C27),(A87,b6,C28),(A87,b6,C29),(A87,b7,C1),(A87,b7,C2) ,(A87,b7,C3),(A87,b7,C4),(A87,b7,C5),(A87,b7,C6),(A87,b7,C7),(A87,b7,C8),(A87,b7,C9),(A 87,b7,C10),(A87,b7,C11),(A87,b7,C12),(A87,b7,C13),(A87,b7,C14),(A87,b7,C15),(A87,b7,C 16),(A87,b7,C17),(A87,b7,C18),(A87,b7,C19),(A87,b7,C20),(A87,b7,C21),(A87,b7,C22),(A87 ,b7,C23),(A87,b7,C24),(A87,b7,C25),(A87,b7,C26),(A87,b7,C27),(A87,b7,C28),(A87,b7,C29) ,(A88,b1,C1),(A88,b1,C2),(A88,b1,C3),(A88,b1,C4),(A88,b1,C5),(A88,b1,C6),(A88,b1,C7),(A 88,b1,C8),(A88,b1,C9),(A88,b1,C10),(A88,b1,C11),(A88,b1,C12),(A88,b1,C13),(A88,b1,C14) ,(A88,b1,C15),(A88,b1,C16),(A88,b1,C7),(A88,b1,C8),(A88,b1,C9),(A88,b1,C20),(A88,b1 ,C21),(A88,b1,C22),(A88,b1,C23),(A88,b1,C24),(A88,b1,C25),(A88,b1,C26),(A88,b1,C27),(A 88,b1,C28),(A88,b1,C29),(A88,b2,C1),(A88,b2,C2),(A88,b2,C3),(A88,b2,C4),(A88,b2,C5),(A 88,b2,C6),(A88,b2,C7),(A88,b2,C8),(A88,b2,C9),(A88,b2,C10),(A88,b2,C11),(A88,b2,C12),( A88,b2,C13),(A88,b2,C14),(A88,b2,C15),(A88,b2,C16),(A88,b2,C17),(A88,b2,C18),(A88,b2, C19),(A88,b2,C20),(A88,b2,C21),(A88,b2,C22),(A88,b2,C23),(A88,b2,C24),(A88,b2,C25),(A 88,b2,C26),(A88,b2,C27),(A88,b2,C28),(A88,b2,C29),(A88,b3,C1),(A88,b3,C2),(A88,b3,C3),( A88,b3,C4),(A88,b3,C5),(A88,b3,C6),(A88,b3,C7),(A88,b3,C8),(A88,b3,C9),(A88,b3,C10),(A 88,b3,C11),(A88,b3,C12),(A88,b3,C13),(A88,b3,C14),(A88,b3,C15),(A88,b3,C16),(A88,b3,C 17),(A88,b3,C18),(A88,b3,C19),(A88,b3,C20),(A88,b3,C21),(A88,b3,C22),(A88,b3,C23),(A88 ,b3,C24),(A88,b3,C25),(A88,b3,C26),(A88,b3,C27),(A88,b3,C28),(A88,b3,C29),(A88,b4,C1),( A88,b4,C2),(A88,b4,C3),(A88,b4,C4),(A88,b4,C5),(A88,b4,C6),(A88,b4,C7),(A88,b4,C8),(A8 8,b4,C9),(A88,b4,C10),(A88,b4,C11),(A88,b4,C12),(A88,b4,C13),(A88,b4,C14),(A88,b4,C15) ,(A88,b4,C16),(A88,b4,C17),(A88,b4,C18),(A88,b4,C19),(A88,b4,C20),(A88,b4,C21),(A88,b4 ,C22),(A88,b4,C23),(A88,b4,C24),(A88,b4,C25),(A88,b4,C26),(A88,b4,C27),(A88,b4,C28),(A 88,b4,C29),(A88,b5,C1),(A88,b5,C2),(A88,b5,C3),(A88,b5,C4),(A88,b5,C5),(A88,b5,C6),(A8 8,b5,C7),(A88,b5,C8),(A88,b5,C9),(A88,b5,C10),(A88,b5,C11),(A88,b5,C12),(A88,b5,C13),( A88,b5,C14),(A88,b5,C15),(A88,b5,C16),(A88,b5,C17),(A88,b5,C18),(A88,b5,C19),(A88,b5, C20),(A88,b5,C21),(A88,b5,C22),(A88,b5,C23),(A88,b5,C24),(A88,b5,C25),(A88,b5,C26),(A 88,b5,C27),(A88,b5,C28),(A88,b5,C29),(A88,b6,C1),(A88,b6,C2),(A88,b6,C3),(A88,b6,C4),( A88,b6,C5),(A88,b6,C6),(A88,b6,C7),(A88,b6,C8),(A88,b6,C9),(A88,b6,C10),(A88,b6,C11),( A88,b6,C12),(A88,b6,C13),(A88,b6,C14),(A88,b6,C15),(A88,b6,C16),(A88,b6,(C17),(A88,b6, C18),(A88,b6,C19),(A88,b6,C20),(A88,b6,C21),(A88,b6,C22),(A88,b6,C23),(A88,b6,C24),(A 88,b6,C25),(A88,b6,C26),(A88,b6,C27),(A88,b6,C28),(A88,b6,C29),(A88,b7,C1),(A88,b7,C2) ,(A88,b7,C3),(A88,b7,C4),(A88,b7,C5),(A88,b7,C6),(A88,b7,C7),(A88,b7,C8),(A88,b7,C9),(A 88,b7,C10),(A88,b7,C11),(A88,b7,C12),(A88,b7,C13),(A88,b7,C14),(A88,b7,C15),(A88,b7,C 16),(A88,b7,C17),(A88,b7,C18),(A88,b7,C19),(A88,b7,C20),(A88,b7,C21),(A88,b7,C22),(A88 ,b7,C23),(A88,b7,C24),(A88,b7,C25),(A88,b7,C26),(A88,b7,C27),(A88,b7,C28),(A88,b7,C29) ,(A89,b1,C1),(A89,b1,C2),(A89,b1,C3),(A89,b1,C4),(A89,b1,C5),(A89,b1,C6),(A89,b1,C7),(A 89,b1,C8),(A89,b1,C9),(A89,b1,C10),(A89,b1,C11),(A89,b1,C12),(A89,b1,C13),(A89,b1,C14) ,(A89,b1,C15),(A89,b1,C16),(A89,b1,C17),(A89,b1,C18),(A89,b1,C19),(A89,b1,C20),(A89,b1 ,C21),(A89,b1,C22),(A89,b1,C23),(A89,b1,C24),(A89,b1,C25),(A89,b1,C26),(A89,b1,C27),(A 89,b1,C28),(A89,b1,C29),(A89,b2,C1),(A89,b2,C2),(A89,b2,C3),(A89,b2,C4),(A89,b2,C5),(A 89,b2,C6),(A89,b2,C7),(A89,b2,C8),(A89,b2,C9),(A89,b2,C10),(A89,b2,C11),(A89,b2,C12),( A89,b2,C13),(A89,b2,C14),(A89,b2,C15),(A89,b2,C16),(A89,b2,C17),(A89,b2,C18),(A89,b2, C19),(A89,b2,C20),(A89,b2,C21),(A89,b2,C22),(A89,b2,C23),(A89,b2,C24),(A89,b2,C25),(A 89,b2,C26),(A89,b2,C27),(A89,b2,C28),(A89,b2,C29),(A89,b3,C1),(A89,b3,C2),(A89,b3,C3),( A89,b3,C4),(A89,b3,C5),(A89,b3,C6),(A89,b3,C7),(A89,b3,C8),(A89,b3,C9),(A89,b3,C10),(A 89,b3,C11),(A89,b3,C12),(A89,b3,C13),(A89,b3,C14),(A89,b3,C15),(A89,b3,C16),(A89,b3,C 17),(A89,b3,C18),(A89,b3,C19),(A89,b3,C20),(A89,b3,C21),(A89,b3,C22),(A89,b3,C23),(A89 ,b3,C24),(A89,b3,C25),(A89,b3,C26),(A89,b3,C27),(A89,b3,C28),(A89,b3,C29),(A89,b4,C1),( A89,b4,C2),(A89,b4,C3),(A89,b4,C4),(A89,b4,C5),(A89,b4,C6),(A89,b4,C7),(A89,b4,C8),(A8 9,b4,C9),(A89,b4,C10),(A89,b4,C11),(A89,b4,C12),(A89,b4,C13),(A89,b4,C14),(A89,b4,C15) ,(A89,b4,C16),(A89,b4,C17),(A89,b4,C18),(A89,b4,C19),(A89,b4,C20),(A89,b4,C21),(A89,b4 ,C22),(A89,b4,C23),(A89,b4,C24),(A89,b4,C25),(A89,b4,C26),(A89,b4,C27),(A89,b4,C28),(A 89,b4,C29),(A89,b5,C1),(A89,b5,C2),(A89,b5,C3),(A89,b5,C4),(A89,b5,C5),(A89,b5,C6),(A8 9,b5,C7),(A89,b5,C8),(A89,b5,C9),(A89,b5,C10),(A89,b5,C11),(A89,b5,C12),(A89,b5,C13),( A89,b5,C14),(A89,b5,C15),(A89,b5,C16),(A89,b5,C17),(A89,b5,C18),(A89,b5,C19),(A89,b5, C20),(A89,b5,C21),(A89,b5,C22),(A89,b5,C23),(A89,b5,C24),(A89,b5,C25),(A89,b5,C26),(A S9,b5,C27),(AS9,b5,C27),(A89,b5,C29),(A89,b6,C1),(A89,b6,C2),(A89,b6,C3),(A89,b6,C4),( A89,b6,C5),(A89,b6,C6),(A89,b6,C7),(A89,b6,C8),(A89,b6,C9),(A89,b6,C10),(A89,b6,C1),( A89,b6,C12),(A89,b6,C13),(A89,b6,C14),(A89,b6,C15),(A89,b6,C16),(A89,b6,C17),(A89,b6, C18),(A89,b6,C19),(A89,b6,C20),(A89,b6,C21),(A89,b6,C22),(A89,b6,C23),(A89,b6,C24),(A 89,b6,C25),(A89,b6,C26),(A89,b6,C27),(A89,b6,C28),(A89,b6,C29),(A89,b7,C1),(A89,b7,C2) ,(A89,b7,C3),(A89,b7,C4),(A89,b7,C5),(A89,b1,C6),(A89,b7,G7),(A89,b7,C8),(A89,b7,C9),(A 89,b7,C10),(A89,b7,C11),(A89,b7,C12),(A89,b7,C13),(A89,b7,C14),(A89,b7,C15),(A89,b7,C 16),(A89,b7,C17),(A89,b7,C18),(A89,b7,C19),(A89,b7,C20),(A89,b7,C21),(A89,b7,C22),(A89 ,b7,C23),(A89,b7,C24),(A89,b7,C25),(A89,b7,C26),(A89,b7,C27),(A89,b7,C28),(A89,b7,C29) ,(A90,b1,C1),(A90,b1,C2),(A90,b1,C3),(A90,b1,C4),(A90,b1,C5),(A90,b1,C6),(A90,b1,C7),(A 90,b1,C8),(A90,b1,C9),(A90,b1,C10),(A90,b1,C11),(A90,b1,C12),(A90,b1,C13),(A90,b1,C14) ,(A90,b1,C15),(A90,b1,C16),(A90,b1,C17),(A90,b1,C18),(A90,b1,C19),(A90,b1,C20),(A90,b1 ,C21),(A90,b1,C22),(A90,b1,C23),(A90,b1,C24),(A90,b1,C25),(A90,b1,C26),(A90,b1,C27),(A 90,b1,C28),(A90,b1,C29),(A90,b2,C1),(A90,b2,C2),(A90,b2,C3),(A90,b2,C4),(A90,b2,C5),(A 90,b2,C6),(A90,b2,C7),(A90,b2,C8),(A90,b2,C9),(A90,b2,C10),(A90,b2,C11),(A90,b2,C12),( A90,b2,C 13),(A90,b2,C14),(A90,b2,C15),(A90,b2,C16),(A90,b2,C17),(A90,b2,C18),(A90,b2, C19),(A90,b2,C20),(A90,b2,C21),(A90,b2,C22),(A90,b2,C23),(A90,b2,C24),(A90,b2,C25),(A 90,b2,C26),(A90,b2,C27),(A90,b2,C28),(A90,b2,C29),(A90,b3,Cl),(A90,b3,C2),(A90,b3,C3),( A90,b3,C4),(A90,b3,C5),(A90,b3,C6),(A90,b3,C7),(A90,b3,C8),(A90,b3,C9),(A90,b3,C10),(A 90,b3,C11),(A90,b3,C12),(A90,b3,C1),(A90,b3,C14),(A90,b3,C15),(A90,b3,C16),(A90,b3,C 17),(A90,b3,C18),(A90,b3,C19),(A90,b3,C20),(A90,b3,C21),(A90,b3,C22),(A90,b3,C23),(A90 ,b3,C24),(A90,b3,C25),(A90,b3,C26),(A90,b3,C27),(A90,b3,C28),(A90,b3,C29),(A90,b4,C1),( A90,b4,C2),(A90,b4,C3),(A90,b4,C4),(A90,b4,C5),(A90,b4,C6),(A90,b4,C7),(A90,b4,C8),(A9 0,b4,C9),(A90,b4,C10),(A90,b4,C11),(A90,b4,C12),(A90,b4,C13),(A90,b4,C14),(A90,b4,C15) ,(A90,b4,C16),(A90,b4,C17),(A90,b4,C18),(A90,b4,C19),(A90,b4,C20),(A90,b4,C21),(A90,b4 ,C22),(A90,b4,C23),(A90,b4,C24),(A90,b4,C25),(A90,b4,C26),(A90,b4,C27),(A90,b4,C28),(A 90,b4,C29),(A90,b5,C;1),(A90,b5,C2),(A90,b5,C3),(A90,b5,C4),(A90,b5,C5),(A90,b5,C6),(A9 0,b5,C7),(A90,b5,C8),(A90,b5,C9),(A90,b5,C10),(A90,b5,C11),(A90,b5,C12),(A90,b5,C 13),( A90,b5,C14),(A90,b5,C15),(A90,b5,C16),(A90,b5,C17),(A90,b5,C18),(A90,b5,C19),(A90,b5, C20),(A90,b5,C21),(A90,b5,C22),(A90,b5,C23),(A90,b5,C24),(A90,b5,C25),(A90,b5,C26),(A 90,b5,C27),(A90,b5,C28),(A90,b5,C29),(A90,b6,C1),(A90,b6,C2),(A90,b6,C3),(A90,b6,C4),( A90,b6,C5),(A90,b6,C6),(A90,b6,C7),(A90,b6,C8),(A90,b6,C9),(A90,b6,C10),(A90,b6,C11),( A90,b6,C12),(A90,b6,C13),(A90,b6,C14),(A90,b6,C15),(A90,b6,C16),(A90,b6,C17),(A90,b6, C18),(A90,b6,C19),(A90,b6,C20),(A90,b6,C21),(A90,b6,C22),(A90,b6,C23),(A90,b6,C24),(A 90,b6,C25),(A90,b6,C26),(A90,b6,C27),(A90,b6,C28),(A90,b6,C29),(A90,b7,C1),(A90,b7,C2) ,(A90,b7,C3),(A90,b7,C4),(A90,b7,C5),(A90,b7,C6),(A90,b7,C7),(A90,b7,C8),(A90,b7,C9),(A 90,b7,C10),(A90,b7,C11),(A90,b7,C12),(A90,b7,C13),(A90,b7,C14),(A90,b7,C15),(A90,b7,C 16),(A90,b7,C17),(A90,b7,C18),(A90,b7,C19),(A90,b7,C20),(A90,b7,C21),(A90,b7,C22),(A90 ,b7,C23),(A90,b7,C24),(A90,b7,C25),(A90,b7,C26),(A90,b7,C27),(A90,b7,C28),(A90,b7,C29) ,

(A91,b1,C1),(A91,b1,C2),(491,b1,C3),(A91,b1,C4),(A91,b1,C5),(A91,b1,C6),(A91,b1,C7),(A 91,b1,C8),(A91,b1,C9),(A91,b1,C10),(A91,b1,C11),(A91,b1,C12),(A91,b1,C13),(A91,b1,C14) ,(A91,b1,C15),(A91,b1,C16),(A91,b1,C17),(A91,b1,C18),(A91,b1,C19),(A91,b1,C20),(A91,b1 ,C21),(A91,b1,C22),(A91,b1,C23),(A91,b1,C24),(A91,b1,C25),(A91,b1,C26),(A91,b1,C27),(A 91,b1,C28),(A91,b1,C29),(A91,b2,C1),(A91,b2,C2),(A91,b2,C3),(A91,b2,C4),(A91,b2,C5),(A 91,b2,C6),(A91,b2,C7),(A91,b2,C8),(A91,b2,C9),(A91,b2,C10),(A91,b2,C11),(A91,b2,C12),( A91,b2,C13),(A91,b2,C14),(A91,b2,C15),(A91,b2,C16),(A91,b2,C17),(A91,b2,C18),(A91,b2, C19),(A91,b2,C20),(A91,b2,C21),(A91,b2,C22),(A91,b2,C23),(A91,b2,C24),(A91,b2,C25),(A 91,b2,C26),(A91,b2,C27),(A91,b2,C28),(A91,b2,C29),(A91,b3,C1),(A91,b3,C2),(A91,b3,C3),( A91,b3,C4),(A91,b3,C5),(A91,b3,C6),(A91,b3,C7),(A91,b3,C8),(A91,b3,C9),(A91,b3,C10),(A 91,b3,C11),(A91,b3,C12),(A91,b3,C13),(A91,b3,C14),(A91,b3,C15),(A91,b3,C16),(A91,b3,C 17),(A91,b3,C18),(A91,b3,C19),(A91,b3,C20),(A91,b3,C21),(A91,b3,C22),(A91,b3,C23),(A91 ,b3,C24),(A91,b3,C25),(A91,b3,C26),(A91,b3,C27),(A91,b3,C28),(A91,b3,C29),(A91,b4,C1),( A91,b4,C2),(A91,b4,C3),(A91,b4,C4),(A91,b4,C5),(A91,b4,C6),(A91,b4,C7),(A91,b4,C8),(A9 1,b4,C9),(A91,b4,C10),(A91,b4,C1l),(A91,b4,C12),(A91,b4,C13),(A91,b4,C14),(A91,b4,C15) ,(A91,b4,C16),(A91,b4,C17),(A91,b4,C18),(A91,b4,C19),(A91,b4,C20),(A91,b4,C21),(A91,b4 ,C22),(A91,b4,C23),(A91,b4,C24),(A91,b4,C25),(A91,b4,C26),(A91,b4,C27),(A91,b4,C28),(A 91,b4,C29),(A91,b5,C1),(A91,b5,C2),(A91,b5,C3),(A91,b5,C4),(A91,b5,C5),(A91,b5,C6),(A9 1,b5,C7),(A91,b5,C8),(A91,b5,C9),(A91,b5,C10),(A91,b5,C11),(A91,b5,C12),(A91,b5,C13),( A91,b5,C14),(A91,b5,C15),(A91,b5,C16),(A91,b5,C17),(A91,b5,C18),(A91,b5,C19),(A91,b5, C20),(A91,b5,C21),(A91,b5,C22),{A91,b5,C23),(A91,b5,C24),(A91,b5,C25),(A91,b5,C26),(A 91,b5,C27),(A91,b5,C28),(A91,b5,C29),(A91,b6,C1),(A91,b6,C2),(A91,b6,C3),(A91,b6,C4),( A91,b6,C5),(A91,b6,C6),(A91,b6,C7),(A91,b6,C8),(A91,b6,C9),(A91,b6,C10),(A91,b6,C11),( A91,b6,C12),(A91,b6,C13),(A91,b6,C14),(A91,b6,C15),(A91,b6,C16),(A91,b6,C17),(A91,b6, C18),(A91,b6,C19),(A91,b6,C20),(A91,b6,C21),(A91,b6,C22),(A91,b6,C23),(A91,b6,C24),(A 91,b6,C25),(A91,b6,C26),(A91,b6,C27),(A91,b6,C28),(A91,b6,C29),(A91,b7,C1),(A91,b7,C2) ,(A91,b7,C3),(A91,b7,C4),(A91,b7,C5),(A91,b7,C6),(A91,b7,C7),(A91,b7,C8),(A91,b7,C9),(A 91,b7,C10),(A91,b7,C11),(A91,b7,C12),(A91,b7,C13),(A91,b7,C14),(A91,b7,C15),(A91,b7,C 16),(A91,b7,C17),(A91,b7,C18),(A91,b7,C19),(A91,b7,C20),(A91,b7,C21),(A91,b7,C22),(A91 ,b7,C23),(A91,b7,C24),(A91,b7,C25),(A91,b7,C26),(A91,b7,C27),(A91,b7,C28),(A91,b7,C29) ,(A92,b1,C1),(A92,b1,C2),(A92,b1,C3),(A92,b1,C4),(A92,b1,C5),(A92,b1,C6),(A92,b1,C7),(A 92,b1,C8),(A92,b1,C9),(A92,b1,C10),(A92,b1,C11),(A92,b1,C12),(A92,b1,C13),(A92,b1,C14) ,(A92,b1,C15),(A92,b1,C16),(A92,b1,C17),(A92,b1,C18),(A92,b1,C19),(A92,b1,C20),(A92,b1 ,C21),(A92,b1,C22),(A92,b1,C23),(A92,b1,C24),(A92,b1,C25),(A92,b1,C26),(A92,b1,C27),(A 92,b1,C28),(A92,b1,C29),(A92,b2,C1),(A92,b2,C2),(A92,b2,C3),(A92,b2,C4),(A92,b2,C5),(A 92,b2,C6),(A92,b2,C7),(A92,b2,C8),(A92,b2,C9),(A92,b2,C10),(A92,b2,C11),(A92,b2,C12),( A92,b2,C13),(A92,b2,C14),(A92,b2,C15),(A92,b2,C16),(A92,b2,C17),(A92,b2,C18),(A92,b2, C19),(A92,b2,C20),(A92,b2,C21),(A92,b2,C22),(A92,b2,C23),(A92,b2,C24),(A92,b2,C25),(A 92,b2,C26),(A92,b2,C27),(A92,b2,C28),(A92,b2,C29),(A92,b3,C1),(A92,b3,C2),(A92,b3,C3),( A92,b3,C4),(A92,b3,C5),(A92,b3,C6),(A92,b3,C7),(A92,b3,C8),(A92,b3,C9),(A92,b3,C10),(A 92,b3,C11),(A92,b3,C12),(A92,b3,C13),(A92,b3,C14),(A92,b3,C15),(A92,b3,C16),(A92,b3,C 17),(A92,b3,C18),(A92,b3,C19),(A92,b3,C20),(A92,b3,C21),(A92,b3,C22),(A92,b3,C23),(A92 ,b3,C24),(A92,b3,C25),(A92,b3,C26),(A92,b3,C27),(A92,b3,C28),(A92,b3,C29),(A92,b4,C1),( A92,b4,C2),(A92,b4,C3),(A92,b4,C4),(A92,b4,C5),(A92,b4,C6),(A92,b4,C7),(A92,b4,C8),(A9 2,b4,C9),(A92,b4,C10),(A92,b4,C11),(A92,b4,C12),(A92,b4,C13),(A92,b4,C14),(A92,b4,C15) ,(A92,b4,C16),(A92,b4,C17),(A92,b4,C18),(A92,b4,C19),(A92,b4,C20),(A92,b4,C21),(A92,b4 ,C22),(A92,b4,C23),(A92,b4,C24),(A92,b4,C25),(A92,b4,C26),(A92,b4,C27),(A92,b4,C28),(A 92,b4,C29),(A92,b5,C1),(A92,b5,C2),(A92,b5,C3),(A92,b5,C4),(A92,b5,C5),(A92,b5,C6),(A9 2,b5,C7),(A92,b5,C8),(A92,b5,C9),(A92,b5,C10),(A92,b5,C11),(A92,b5,C12),(A92,b5,C1.3),( A92,b5,C14),(A92,b5,C15),(A92,b5,C16),(A92,b5,C17),(A92,b5,C18),(A92,b5,C19),(A92,b5, C20),(A92,b5,C21),(A92,b5,C22),(A92,b5,C23),(A92,b5,C24),(A92,b5,C25),(A92,b5,C26),(A 92,b5,C27),(A92,b5,C28),(A92,b5,C29),(A92,b6,C1),(A92,b6,C2),(A92,b6,C3),(A92,b6,C4),( A92,b6,C5),(A92,b6,C6),(A92,b6,C7),(A92,b6,C8),(A92,b6,C9),(A92,b6,C10),(A92,b6,C11),( A92,b6,C12),(A92,b6,C13),(A92,b6,C14),(A92,b6,C15),(A92,b6,C16),(A92,b6,C17),(A92,b6, C18),(A92,b6,C19),(A92,b6,C20),(A92,b6,C21),(A92,b6,C22),(A92,b6,C23),(A92,b6,C24),(A 92,b6,C25),(A92,b6,C26),(A92,b6,C27),(A92,b6,C28),(A92,b6,C29),(A92,b7,C1),(A92,b7,C2) ,(A92,b7,C3),(A92,b7,C4),(A92,b7,C5),(A92,b7,C6),(A92,b7,C7),(A92,b7,C8),(A92,b7,C9),(A 92,b7,C10),(A92,b7,C11),(A92,b7,C12),(A92,b7,C13),(A92,b7,C14),(A92,b7,C15),(A92,b7,C 16),(A92,b7,C17),(A92,b7,C18),(A92,b7,C19),(A92,b7,C20),(A92,b7,C21),(A92,b7,C22),(A92 ,b7,C23),(A92,b7,C24),(A92,b7,C25),(A92,b7,C26),(A92,b7,C27),(A92,b7,C28),(A92,b7,C29) ,(A93,b1,C1),(A93,b1,C2),(A93,b1,C3),(A93,b1,C4),(A93,b1,C5),(A93,b1,C6),(A93,b1,C7),(A 93,b1,C8),(A93,b1,C9),(A93,b1,C10),(A93,b1,C11),(A93,b1,C12),(A93,b1,C13),(A93,b1,C14) ,(A93,b1,C15),(A93,b1,C16),(A93,b1,C17),(A93,b1,C18),(A93,b1,C19),(A93,b1,C20),(A93,b1 ,C21),(A93,b1,C22),(A93,b1,C23),(A93,b1,C24),(A93,b1,C25);(A93,b1,C26),(A93,b1,C27),(A 93,b1,C28),(A93,b1,C29),(A93,b2,C1),(A93,b2,C2),(A93,b2,C3),(A93,b2,C4),(A93,b2,C5),(A 93,b2,C6),(A93,b2,C7),(A93,b2,C8),(A93,b2,C9),(A93,b2,C10),(A93,b2,C11),(A93,b2,C12),( A93,b2,C13),(A93,b2,C14),(A93,b2,C15),(A93,b2,C16),(A93,b2,C17),(A93,b2,C18),(A93,b2, C19),(A93,b2,C20),(A93,b2,C21),(A93,b2,C22),(A93,b2,C23),(A93,b2,C24),(A93,b2,C25),(A 93,b2,C26),(A93,b2,C27),(A93,b2,C28),(A93,b2,C29),(A93,b3,C1),(A93,b3,C2),(A93,b3,C3),( A93,b3,C4),(A93,b3,C5),(A93,b3_{;}C6),(A93,b3,C7),(A93,b3,C8),(A93,b3,C9),(A93,b3,C10),(A 93,b3,C11),(A93,b3,C12),(A93,b3,C3),(A93,b3,C4),(A93,b3,C5),(A93,b3,C16),(A93,b3,C 17),(A93,b3,C18),(A93,b3,C19),(A93,b3,C20),(A93,b3,C21),(A93,b3,C22),(A93,b3,C23),(A93 ,b3,C24),(A93,b3,C25),(A93,b3,C26),(A93,b3,C27),(A93,b3,C28),(A93,b3,C29),(A93,b4,C1),( A93,b4,C2),(A93,b4,C3),(A93,b4,C4),(A93,b4,C5),(A93,b4,C6),(A93,b4,C7),(A93,b4,C8),(A9 3,b4,C9),(A93,b4,C10),(A93,b4,C11),(A93,b4,C12),(A93,b4,C13),(A93,b4,C14),(A93,b4,C15) ,(A93,b4,C16),(A93,b4,C17),(A93,b4,C18),(A93,b4,C19),(A93,b4,C20),(A93,b4,C21),(A93,b4 ,C22),(A93,b4,C23),(A93,b4,C24),(A93,b4,C25),(A93,b4,C26),(A93,b4,C27),(A93,b4,C28),(A 93,b4,C29),(A93,b5,C1),(A93,b5,C2),(A93,b5,C3),(A93,b5,C4),(A93,b5,C5),(A93,b5,C6),(A9 3,b5,C7),(A93,b5,C8),(A93,b5,C9),(A93,b5,C10),(A93,b5,C11),(A93,b5,C12),(A93,b5,C13),( A93,b5,C14),(A93,b5,C15),(A93,b5,C16),(A93,b5,C17),(A93,b5,C18),(A93,b5,C19),(A93,b5, C20),(A93,b5,C21),(A93,b5,C22),(A93,b5,C23),(A93,b5,C24),(A93,b5,C25),(A93,b5,C26),(A 93,b5,C27),(A93,b5,C28),(A93,b5,C29),(A93,b6,C1),(A93,b6,C2),(A93,b6,C3),(A93,b6,C4),( A93,b6,C5),(A93,b6,C6),(A93,b6,C7),(A93,b6,C8),(A93,b6,C9),(A93,b6,C10),(A93,b6,C11),( A93,b6,C12),(A93,b6,C13),(A93,b6,C14),(A93,b6,C15),(A93,b6,C16),(A93,b6,C17),(A93,b6, C18),(A93,b6,C19),(A93,b6,C20),(A93,b6,C21),(A93,b6,C22),(A93,b6,C23),(A93,b6,C24),(A 93,b6,C25),(A93,b6,C26),(A93,b6,C27),(A93,b6,C28),(A93,b6,C29),(A93,b7,C1),(A93,b7,C2) ,(A93,b7,C3),(A93,b7,C4),(A93,b7,C5),(A93,b7,C6),(A93,b7,C7),(A93,b7,C8),(A93,b7,C9),(A 93,b7,C10),(A93,b7,C11),(A93,b7,C12),(A93,b7,C13),(A93,b7,C14),(A93,b7,C15),(A93,b7,C 16),(A93,b7,C7),(A93,b7,C8),(A93,b7,C19),(A93,b7,C20),(A93,b7,C21),(A93,b7,C22),(A93 ,b7,C23),(A93,b7,C24),(A93,b7,C25),(A93,b7,C26),(A93,b7,C27),(A93,b7,C28),(A93,b7,C29) ,(A94,b1,C1),(A94,b1,C2),(A94,b1,C3),(A94,b1,C4),(A94,b1,C5),(A94,b1,C6),(A94,b1,C7),(A 94,b1,C8),(A94,b1,C9),(A94,b1,C10),(A94,b1,C11),(A94,b1,C12),(A94,b1,C13),(A94,b1,C14) ,(A94,b1,C15),(A94,b1,C16),(A94,b1,C17),(A94,b1,C18),(A94,b1,C19),(A94,b1,C20),(A94,b1 ,C21),(A94,b1,C22),(A94,b1,C23),(A94,b1,C24),(A94,b1,C25),(A94,b1,C26),(A94,b1,C27),(A 94,b1,C28),(A94,b1,C29),(A94,b2,Cl),(A94,b2,C2),(494,b2,C3),(A94,b2,C4),(A94,b2,C5),(A 94,b2,C6),(A94,b2,C7),(A94,b2,C8),(A94,b2,C9),(A94,b2,C10),(A94,b2,C11),(A94,b2,C12),( A94,b2,C13),(A94,b2,C14),(A94,b2,C15),(A94,b2,C16),(A94,b2,C17),(A94,b2,C18),(A94,b2, C19),(A94,b2,C20),(A94,b2,C21),(A94,b2,C22),(A94,b2,C23),(A94,b2,C24),(A94,b2,C25),(A 94,b2,C26),(A94,b2,C27),(A94,b2,C28),(A94,b2,C29),(A94,b3,C1),(A94,b3,C2),(A94,b3,C3),( A94,b3,C4),(A94,b3,C5),(A94,b3,C6),(A94,b3,C7),(A94,b3,C8),(A94,b3,C9),(A94,b3,C10),(A 94,b3,C11),(A94,b3,C12),(A94,b3,C13),(A94,b3,C14),(A94,b3,C15),(A94,b3,C16),(A94,b3,C 17),(A94,b3,C18),(A94,b3,C199),(A94,b3,C20),(A94,b3,C21),(A94,b3,C22),(A94,b3,C23),(A94 ,b3,C24),(A94,b3,C25),(A94,b3,C26),(A94,b3,C27),(A94,b3,C28),(A94,b3,C29),(A94,b4,C1),( A94,b4,C2),(A94,b4,C3),(A94,b4,C4).(A94,b4,C5)(A94,b4,C6),(A94,b4,C7),(A94,b4,C8),(A9 4,b4,C9),(A94_{;}b4,C10),(A94,b4,C11),(A94,b4,C12),(A94,b4,C13),(A94,b4,C14),(A94,b4,C15) ,(A94,b4,C16),(A94,b4,C17),(A94,b4,C18),(A94,b4,C19),(A94,b4,C20),(A94,b4,C21),(A94,b4 ,C22),(A94,b4,C23),(A94,b4,C24),(A94.b4,C25),(A94,b4,C26),(A94,b4,C27),(A94,b4,C28),(A 94,b4,C29),(A94,b5,C1),(A94,b5,C2),(A94,b5,C3),(A94,b5,C4),(A94,b5,C5),(A94,b5,C6),(A9 4,b5,C7),(A94,b5,C8),(A94,b5,C9),(A94,b5,C10),(A94,b5,C11),(A94,b5,C12),(A94,b5,C13),( A94,b5,C14),(A94,b5,C15),(A94,b5,C16),(A94,b5,C17),(A94,b5,C18),(A94,b5,C19),(A94,b5, C20),(A94,b5,C21),(A94,b5,C22),(A94,b5,C23),(A94,b5,C24),(A94,b5,C25),(A94,b5,C26),(A 94,b5,C27),(A94,b5,C28),(A94,b5,C29),(A94,b6,C1),(A94,b6,C2),(A94,b6,C3),(A94,b6,C4),( A94,b6,C5),(A94,b6,C6),(A94,b6,C7),(A94,b6,C8),(A94,b6,C9),(A94,b6,C10),(A94,b6,C11), A94,b6,C12),(A94,b6,C13),(A94,b6,C14),(A94,b6,C1.5),(A94,b6,C16),(A94,b6,C17),(A94,b6, C18),(A94,b6,C19),(A94,b6,C20),(A94,b6,C21),(A94,b6,C22),(A94,b6,C23),(A94,b6,C24),(A 94,b6,C25),(A94,b6,C26),(A94,b6,C27),(A94,b6,C28),(A94,b6,C29),(A94,b7,C1),(A94,b7,C2) ,(A94,b7,C3),(A94,b7,C4),(A94,b7C5),(A94,b7,C6),(A94,b7,C7),(A94,b7,C8),(A94,b7,C9),(A 94,b7,C10),(A94,b7,C11),(A94,b7,C12),(A94,b7,C13),(A94,b7,C14),(A94,b7,C15),(A94,b7,C 16),(A94,b7,C17),(A94,b7,C18),(A94,b7,C19),(A94,b7,C20),(A94,b7,C21),(A94,b7,C22),(A94 ,b7,C23),(A94,b7,C24),(A94,b7,C25),(A94,b7,C26),(A94,b7,C27),(A94,b7,C28) or (A94,b7,C29).

Compounds wherein 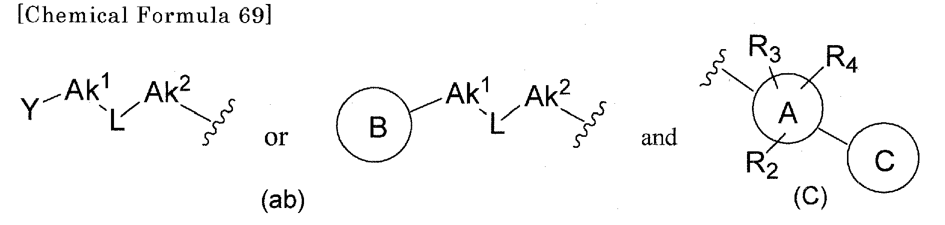 (ab,C)are as follows:
(ab,c)=
   (ab8,C1),(ab8,C2),(ab8,C3),(ab8,C4),(ab8,C5),(ab8,C6),(ab8,C7),(ab8,C8),(ab8,C9),(ab8,C1 0),(ab8,C11),(ab8,C12),(ab8,C13),(ab8,C14),(ab8,C15),(ab8,C16),(ab8,C17),(ab8,C18),(ab8, C19),(ab8,C20),(ab8,C21),(ab8,C22),(ab8,C23),(ab8,C24),(ab8,C25),(ab8,C26),(ab8,C27),(a b8,C28),(ab8,C29),(ab9,C1),(ab9,C2),(ab9,C3),(ab9,C4),(ab9,C5),(ab9,C6),(ab9,C7),(ab9,C8 ),(ab9,C9),(ab9,C10),(ab9,C 11),(ab9,C12),(ab9,C13),(ab9,C14),(ab9,C15),(ab9,C16),(ab9,C1 7),(ab9,C18),(ab9,C19),(ab9,C20),(ab9,C21),(ab9,C22),(ab9,C23),(ab9,C24),(ab9,C25),(ab9, C26),(ab9,C27),(ab9,C28),(ab9,C29),(ab10,C1),(ab10,C2),(ab10,C3),(ab10,C4),(ab10,C5),(a b10,C6),(ab10,C7),(ab10,C8),(ab10,C9),(ab10,C10),(ab10,C11),(ab10,C12),(ab10,C13),(ab1 0,C14),(ab10,C15),(ab10,C16),(ab10,C17),(ab10,C18),(ab10,C19),(ab10,C20),(ab10,C21),(a b10,C22),(ab10,C23),(ab10,C24),(ab10,C25),(ab10,C26),(ab10,C27),(ab10,C28),(ab10,C29), (ab11,C1),(ab11,C2),(ab11,C3),(ab11,C4),(ab11,C5),(ab11,C6),(ab11,C7),(ab11,C8),(ab11,C 9),(ab11,C10),(ab11,C11),(ab11,C12),(ab11,C13),(ab11,C14),(ab1l,CI5),(ab1l,C16),(ab11, C17),(ab11,C18),(ab11,C19),(ab1,C20),(ab11,C21),(ab11,C22),(ab11,C23),(ab11,C24),(ab1 1,C25),(ab11,C26),(ab11,C27),(ab11,C28),(ab11,C29),(ab12,C1),(ab12,C2),(ab12,C3),(ab12, C4),(ab12,C5),(ab12,C6),(ab12,C7),(ab12,C8),(ab12,C9),(ab12,C10),(ab12,C11),(ab12,C12), (ab12,C13),(ab12,C14),(ab12,C15),(ab12,C16),(ab12,C17),(ab12,C18),(ab12,C19),(ab12,C2 0),(ab12,C21);(ab12,C22),(ab12,C23),(ab12,C24),(ab12,C25),(ab12,C26),(ab12,C27),(ab12, C28),(ab12,C29),(ab13,C1),(ab13,C2),(ab13,C3),(ab13,C4),(ab13,C5),(ab13,C6),(ab13,C7),( ab13,C8),(ab13,C9),(ab13,C10),(ab13,C11),(ab13,C12),(ab13,C13),(ab13,C14),(ab13,C15),( ab13,C16),(ab13,C17),(ab13,C18),(ab13,C19),(ab13,C20),(ab13,C21),(ab13,C22),(ab13,C23 ),(ab13,C24),(ab13,C25),(ab13,C26),(ab13,C27),(ab13,C28),(ab13,C29),(ab14,C1),(ab14,C2) ,(ab14,C3),(ab14,C4),(ab14,C5),(ab14,C6),(ab14,C7),(ab14,C8),(ab14,C9),(ab14,C10),(ab14, C11),(ab14,C12),(ab14,C13),(ab14,C14),(ab14,C15),(ab14,C16),(ab14,C17),(ab14,C18),(ab1 4,C19),(ab14,C20),(ab14,C21),(ab14,C22),(ab14,C23),(ab14,C24),(ab14,C25),(ab14,C26),(a b14,C27),(ab14,C28),(ab14,C29),(ab15,C1),(ab15,C2),(ab15,C3),(ab15,C4),(ab15,C5),(ab15, C6),(ab15,C7),(ab15,C8),(ab15,C9),(ab15,C10),(ab15,C11),(ab15,C12),(ab15,C13),(ab15,C1 4),(ab15,C15),(ab15,C16),(ab15,C17),(ab15,C18),(ab15,C19),(ab15,C20),(ab15,C21),(ab15, C22),(ab15,C23),(ab15,C24),(ab15,C25),(ab15,C26),(ab15,C27),(ab15,C28),(ab15,C29),(ab1 6,C1),(ab16,C2),(ab16,C3),(ab16,C4),(ab16,C5),(ab16,C6),(ab16,C7),(ab16,C8),(ab16,C9),(a. b16,C10),(ab16,C11),(ab16,C12),(ab16,C13),(ab16,C14),(ab16,C15),(ab16,C16),(ab16,C17), (ab16,C18),(ab16,C19),(ab16,C20),(ab16,C21),(ab16,C22),(ab16,C23),(ab16,C24),(ab16,C2 5),(ab16,C26),(ab16,C27),(ab16,C28),(ab16,C29),(ab17,C1),(ab17,C2),(ab17,C3),(ab17;C4),( ab17,C5),(ab17,C6),(ab17,C7),(ab17,C8),(ab17,C9),(ab17,C10),(ab17,C11),(ab17,C12),(ab1 7,C13),(ab17,C14),(ab17,C15),(ab17,C16),(ab17,C17),(ab17,C18),(ab17,C19),(ab17,C20),(a b17,C21),(ab17,C22),(ab17,C23),(ab17,C24),(ab17,C25),(ab17,C26),(ab17,C27),(ab17,C28), (ab17,C29),(ab18,C1),(ab18,C2),(ab18,C3),(ab18,C4),(ab18,C5),(ab18,C6),(ab18,C7),(ab18, C8),(ab18,C9),(ab18,C10),(ab18,C11),(ab18,C12),(ab18,C13),(ab18,C14),(ab18,C15),(ab18, C16),(ab18,C17),(ab18,C18),(ab18,C19),(ab18,C20),(ab18,C21),(ab18,C22),(ab18,C23),(ab1 8,C24),(ab18,C25),(ab18,C26),(ab18,C27),(ab18;C28),(ab18,C29),(ab19,C1),(ab19,C2),(ab1 9,C3),(ab19,C4),(ab19,C5),(ab19,C6),(ab19,C7),(ab19,C8),(ab19,C9),(ab19,C10),(ab19,C11) ,(ab19,C12),(ab19,C13),(ab19,C14),(ab19,C15),(ab19,C16),(ab19,C17),(ab19,C18),(ab19,C1 9),(ab19,C20),(ab19,C1),(ab19,C22),(ab19,C23),(ab19,C24),(ab19,C25),(ab19,C26),(ab19,C 27),(ab19,C28)or(ab19,C29).

In one aspect, X in the formula (I) is a group as follows: 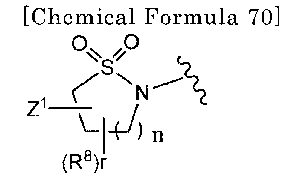 wherein Z¹, R⁸, r and n are as defined in the above 1).

In one aspect, X in the formula (I) is a group as follows: 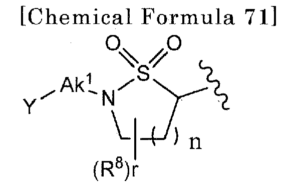 wherein Y, Ak¹, R⁸, r and n are as defined in the above 1).

In one aspect, X in the formula (I) is a group as follows: 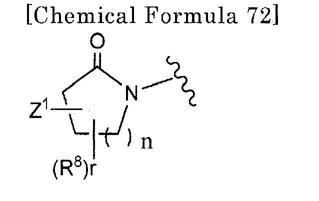 wherein Z¹, R⁸, r and n are as defined in the above 1).

In one aspect, X in the formula (I) is a group as follows: 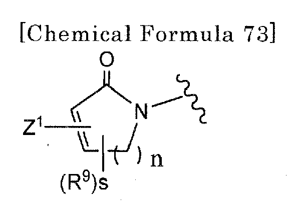 wherein Z¹, R⁹, s and n are as defined in the above 1).

In one aspect, X in the formula (I) is a group as follows: 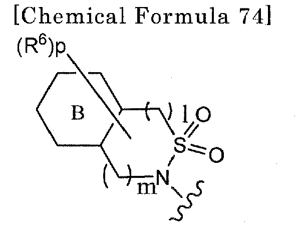 wherein R⁶, p, m and 1 are as defined in the above 1).

In one aspect, X in the formula (I) is a group as follows: 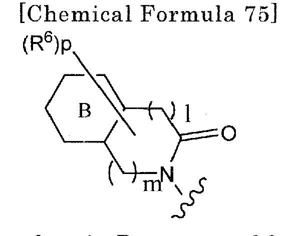 wherein R⁶, p, m and 1 are as defined in the above 1).

In one aspect, X in the formula (I) is a group as follows: 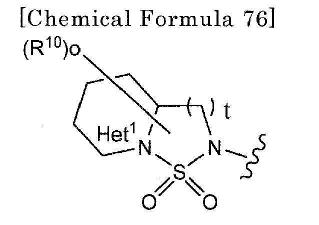 wherein R¹⁰, o, Het¹ and t are as defined in the above 1).

In one aspect, X in the formula (I) is a group as follows: 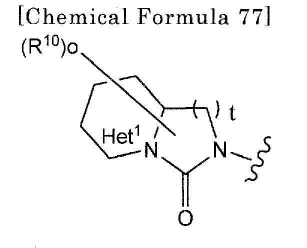 wherein R¹⁰, o, Het¹ and t are as defined in the above 1).

In one aspect, X in the formula (I) is a group as follows: 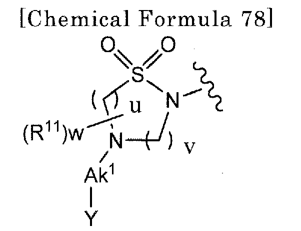 wherein u, R¹¹, w, Ak¹, Y and v are as defined in the above 1).

In one aspect, X in the formula (I) is a group as follows: 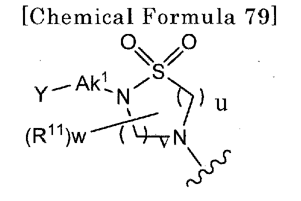 wherein Y, Ak¹, R¹¹, w, v and u are as defined in the above 1).

In one aspect, X in the formula (I) is a group as follows: 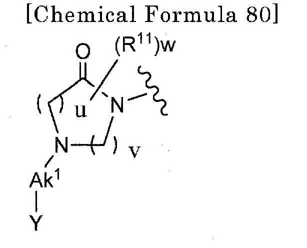 wherein u, Y, Ak¹, v, R¹¹ and w are as defined in the above 1).

In one aspect, X in the formula (I) is a group as follows: 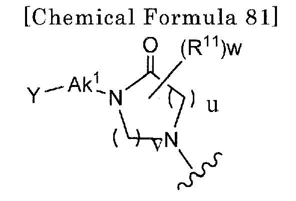 wherein Y, Ak¹, v, u, R¹¹ and w are as defined in the above 1).

In one aspect, X in the formula (I) is Y-Ak¹ -SO₂ N(R⁵)-Ak² -.

The compound (I) or (I') of the present invention is useful against diseases induced by production, secretion or deposition of amyloid-β proteins. For example, the compound is effective for treating and/or preventing, and ameliorating symptoms of diseases such as dementia of the Alzheimer's type (e.g. Alzheimer's disease and senile dementia of the Alzheimer's type), Down syndrome, memory disorder, prion diseases (e.g. Creutzfeldt-Jakob disease), mild cognitive impairment (MCI), hereditary cerebral hemorrhage with amyloidosis-Dutch type, cerebral amyloid angiopathy, other degenerative dementia, vascular and degenerative mixed dementia, dementia associated with Parkinson's disease, dementia associated with progressive supranuclear palsy, dementia associated with corticobasal degeneration, Alzheimer's disease with diffuse Lewy bodies, age-related macular degeneration, Parkinson's disease and amyloid angiopathy.

The compound (I) or (I') of the present invention selectively inhibit the production of amyloid-β proteins, and therefore, it can be pharmaceuticals with reduced side effects. In addition, the compound of the present invention has various advantages such as high selectivity against other enzymes, high metabolic stability, high solubility, high oral absorbability, high bioavailability, good clearance, high brain transferability, a long half life, high protein unbinding ratio, low hERG channel inhibition, low CYP inhibition and /or negative in Ames test.

When the compound (I) or (I') of the present invention is administered, other medicaments (e.g. other therapeutic agents for Alzheimer's disease such as acetylcholine esterase) may be used in combination. For example, anti-dementia drugs such as donepezil hydrochloride, tacrine, galantamine, rivastigmine, zanapezil, memantine and vinpocetine may be used in combination.
When the compound (I) or (I') of the present invention is administered to humans, it may be orally administered as powders, granules, tablets, capsules, pills, liquids and the like, or may be parenterally administered as injections, suppositories, transdermal systems, inhalant and the like. In addition, an effective amount of the present compound may be mixed with pharmaceutical additives such as diluents, binders, humectants, disintegrants and lubricants suitable for its dosage form, and thereby the compound may be formed into a pharmaceutical preparation.
The dosage depends on disease conditions, the route of administration, the age or the weight of a patient. In the case of oral administration to adults, the dosage is generally 0.1 µg to 1 g/day, and preferably 0.01 to 200 mg/day. In the case of parenteral administration, the dosage is generally 1 µg to 10 g/day, and preferably 0.1 to 2 g/day.

### [Examples]

The present invention will be described in more detail with reference to, but not limited to, the following examples and test examples.
In the examples, meaning of each abbreviation is as follows.
- Me:: methyl
- Et:: ethyl
- IPr:: isopropyl
- Ph:: phenyl
- Bn:: benzyl
- Boc:t: -butoxycarbonyl
- SEM:: 2-(trimethylsilyl)ethoxymethyl
- TBDPS:: t-butyldiphenylsilyl
- DMA:: dimethylacetamide
- DMF:: dimethylformamide
- DMSO:: dimethylsulfoxide
- DIPEA:: diisopropylethylamine
- NBS:: N-bromosuccinimide
- HATU:: hexafluorophosphate 2-(7-azabenzotriazol-1-yl)-1,1,3,3-tetramethyluronium
- IPA:: isopropylalcohol
- RuPhos:: 2-dicyclohexylphosphino-2', 6 '-di-i-propoxy-1,1'-biphenyl

LC/MS was performed under the following conditions.

### Analytic LCMS

### (Method A)

Column: Xbridge C18 (5 µm, i.d.4.6 x 50mm) (Waters)
Flow rate: 3 mL/min
UV detection wavelength: 254 nm
Mobile phase: [A] 0.1% formic acid-containing aqueous solution; [B] 0.1% formic acid-containing acetonitrile solution
Gradient: performing linear gradient of 10% to 100% solvent [B] for 3 minutes, and keeping 100% solvent [B] for 1 minute

### (Method B)

Column: Shim-pack XR-ODS (2.2 µm, i.d. 50 x 3.0 mm) (Shimadzu)
Flow rate: 1.6 mL/min.
UV detection wavelength: 254 nm
Mobile phase: [A] 0.1% formic acid-containing aqueous solution; [B] 0.1% formic acid-containing acetonitrile solution
Gradient: performing linear gradient of 10% to 100% solvent [B] for 3 minutes, and keeping 100% solvent [B] for 1 minute

### (Method C)

Column: Luna C18 (2) (Phenomenex) (5 µm, i.d.4.6 x 50mm) (Phenomenex)
Flow rate: 3 mL/min
UV detection wavelength: 254 nm
Mobile phase: [A] 0.1% formic acid-containing aqueous solution; [B] 0.1% formic acid-containing acetonitrile solution
Gradient: performing linear gradient of 10% to 100% solvent [B] for 3 minutes, and keeping 100% solvent [B] for 1 minute

### (Method D)

Column: Gemini-NX (5 µm, i.d.4.6 x 50mm) (Phenomenex)
Flow rate: 3 mL/min
UV detection wavelength: 254 nm
Mobile phase: [A] 0.1% formic acid-containing aqueous solution; [B] 0.1% formic acid-containing methanol solution
Gradient: performing linear gradient of 5% to 100% solvent [B] for 3.5 minutes, and keeping 100% solvent [B] for 0.5 minute

### (Method E)

Column: XBridge C18 (5 □m i.d.4.6 x 50 mm) (Waters)
Flow rates: 2 mL/min
UV detection wavelength: 254 nm
Mobile phase: [A] 10 mM ammonium bicarbonate-containing aqueous solution; [B] acetonitrile
Gradient: performing linear gradient of 10% to 100% solvent [B] for 3 minutes, and keeping 100% solvent [B] for 1 minute

### (Method F)

Column: Gemini-NX (5 µm, i.d.4.6 x 50mm) (Phenomenex)
Flow rate:3 mL/min
UV detection wavelength: 254 nm
Mobile phase: [A] 10 mM ammonium bicarbonate-containing aqueous solution; [B] methanol
Gradient: performing linear gradient of 5% to 100% solvent [B] for 3.5 minutes, and keeping 100% solvent [B] for 0.5 minute

### Example 1 Synthesis of Compound 28

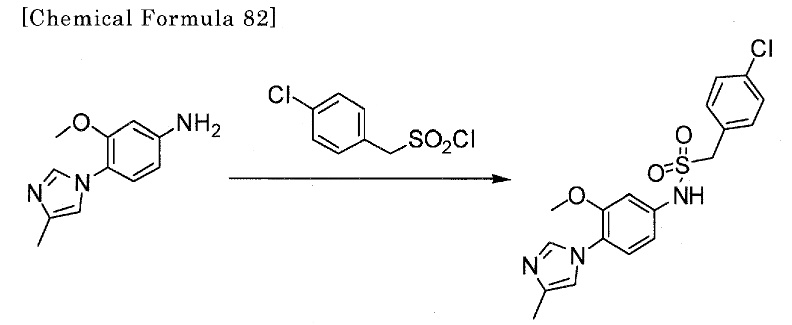

Pyridine (6.6 ml, 81 mmol) was added to a solution of 3-methoxy-4-(4-methyl-1H-imidazol-1-yl)aniline (11 g, 54.1 mmol) in CH₂Cl₂ (100 ml). The mixture was cooled with ice and (4-chlorophenyl)methanesulfonyl chloride was added. The reaction mixture was allowed to warm to room temperature and stirred for 2 hours. The mixture was concentrated, and ethyl acetate (50 ml) and water (50 ml) were added to the residue. The precipitate was filtered, washed with ethyl acetate (10 ml) and water (10 ml), and dried under reduced pressure to afford the target compound as a colorless powder (19.79 g, 93%).
LC-MS (Method D): 1.19 min, [M+H] =392.07

### Example 2 Synthesis of Compound 77

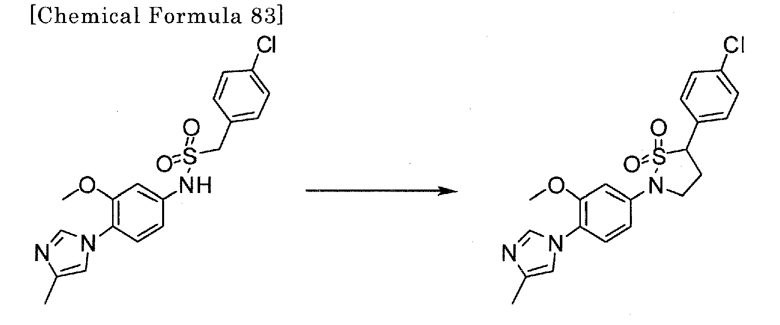

To the solution of 1-(4-chlorophenyl)-N-(3-methoxy-4-(4-methyl-1H-imidazol-1-yl)phenyl)methanesulfonamide (300 mg, 0.77 mmol) in DMF (2 ml) were added 1,2-dibromoethane (719 mg, 3.83 mmoL) and potassium carbonate (212 mg,1.53 mol), and the mixture was stirred at 60 °C for 3 hours, followed by at 100 °C for additional 2 hours. To the reaction mixture was added water (10 ml) and the mixture was extracted with ethyl acetate (20 ml). The organic layer was washed with brine (10 ml),dried over magnesium sulfate and the solvent was removed under reduced pressure. The residue was purified by silica gel chromatography (MeOH/CHCl₃: 0%-10%) to afford the target compound as a colorless powder (30 mg, 9.4%).
LC-MS (Method D): 1.81 min, [M+H] =418.09

### Example 3 Synthesis of Compound 184

### (Step 1)

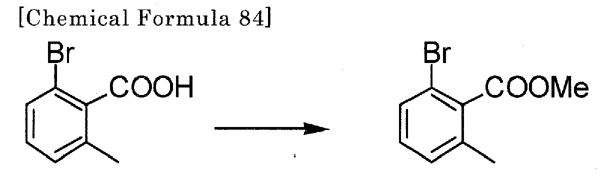

To a suspension of 2-bromo-6-methyl benzoic acid (5.05 g, 23.48 mmol) in CH₂Cl₂ (50 ml) were added oxalyl chloride (2.67 mg, 30.5 mmol) and DMF (2 drops) successively at room temperature. The mixture was stirred at room temperature for 2 hours and the solvent was concentrated. To the obtained residue was added MeOH (10 ml), and the mixture was stirred overnight. The reaction mixture was concentrated under reduced pressure A saturated aqueous solution of sodium bicarbonate (20 ml) was added to the residue and the mixture was extracted with ethyl acetate. The organic layer was dried over magnesium sulfate and the solvent was removed under reduced pressure. The obtained target compound (5.04 g, 94%) was used in the next step without further purifications.

### (Step 2)

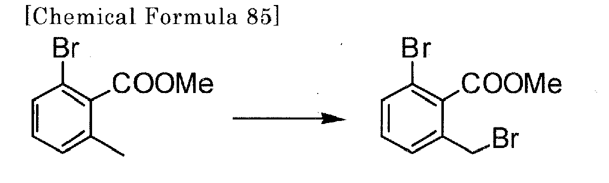

To a solution of methyl 2-bromo-6-methyl benzoate (5.04 g, 22 mmol) in carbon tetrachloride (50 ml) were added NBS (4.11 g, 23.1 mmol) and benzoyl peroxide (0.53 g, 2.2 mmol) at room temperature. The reaction mixture was heated to reflux and stirred for 4 hours. After cooling, an aqueous solution of 10% Na₂S₂O₃ (30 ml) was added to the reaction mixture and the mixture was extracted with chloroform. The organic layer was dried over magnesium sulfate and the solvent was removed under reduced pressure. The residue was purified by silica gel chromatography (AcOEt/Hexane:0%-10%) to afford the target compound as a colorless liquid (4.88 g, 72%).
LC-MS (Method D): 2.77 min, [M+H] =308.97

### (Step 3)

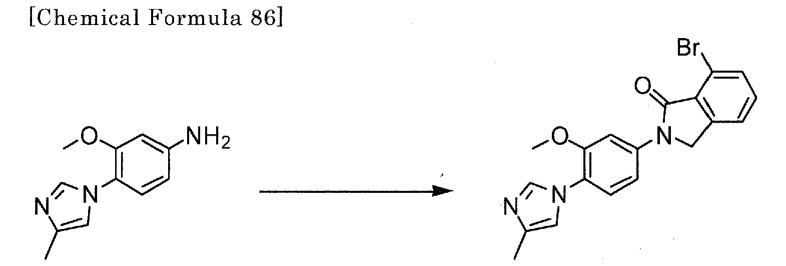

To a suspension of 3-methoxy-4-(4-methyl-1H-imidazol-1-yl)aniline (660 mg, 3.25 mmol) in isopropanol (3 ml) were added DIPEA (0.68 ml, 3.90 mmol) and 2-bromo-6-(bromomethyl)methylbenzoate (1000 mg, 3.25 mmol) at room temperature and the mixture was stirred at room temperature overnight. The reaction mixture was reacted by a microwave reactor at 150 °C for 30 minutes. To the reaction mixture was added water (10 ml) and the mixture was extracted with ethyl acetate. The organic layer was dried over magnesium sulfate and the solvent was removed under reduced pressure. The residue was purified by silica gel chromatography (MeOH/CHCl₃:0%-10%) to afford the target compound as a yellow powder (435 mg, 34%).
LC-MS (Method D): 1.73 min, [M+H] =399.25

### Example 4 Synthesis of Compound 125

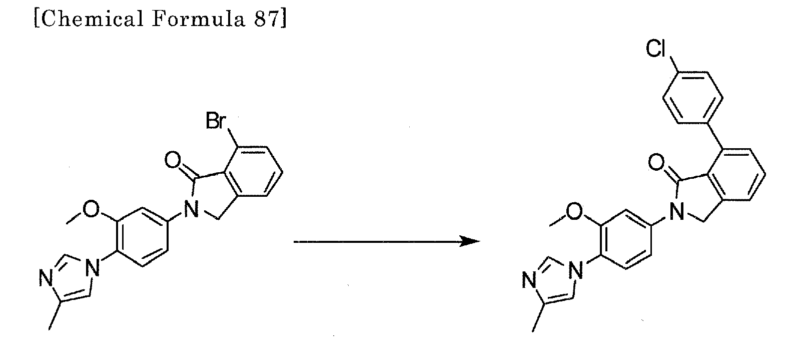

To a mixed solution of EtOH(1.6 ml)-2 mol/L Na₂CO₃ aq (0.4 ml) were added 7-bromo-2-(3-methoxy-4-(4-methyl-1H-imidazol-1-yl)phenyl)isoindolin-1-one (20 mg, 0.05 mmol), 4-chlorophenyl boronic acid (9.4 mg, 0.06 mmol) and Pd(PPh₃)₄ (5.8 mg, 5.0 □mol), and the suspension was reacted by a microreactor at 150 °C for 15 minutes. To the reaction mixture was added water (5 ml) and the mixture was extracted with ethyl acetate. The organic layer was dried over magnesium sulfate and the solvent was removed under reduced pressure. The residue was purified by silica gel chromatography (MeOH/CHCl₃:0%-10%) to afford the target compound as a yellow powder (6.4 mg, 30%). LC-MS (Method D): 2.22 min, [M+H] =430.12

### Example 5 Synthesis of Compound 90

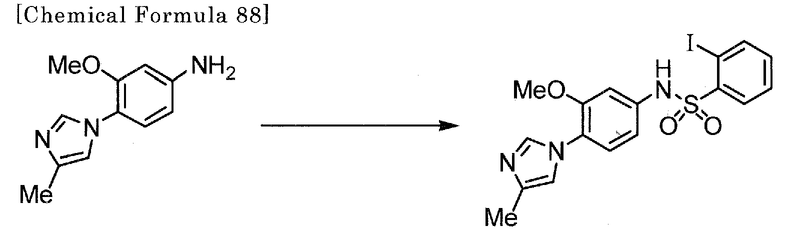

3-methoxy-4-(4-methyl-imidazol-1-yl)-phenylamine (6.72 g, 33.1 mmol) and pyridine (3.21 ml) were dissolved in dichloromethane (100 ml), and 2-iodobenzene-1-sulfonyl chloride (9.00 g, 29.8 mmol) was added under ice-cooling. The mixture was stirred at room temperature overnight. To the reaction mixture was added 0.5 mol/L hydrochloric acid solution and the mixture was extracted. The organic layer was dried over anhydrous magnesium sulfate and concentrated to dryness under reduced pressure. The residue was purified by silica gel chromatography (CHCl₃:MeOH 97:3 v/v) to afford the target compound as a pale red powder (14.4 g, 93%).
LC-MS (Method C): 1.49 min, [M+H] =470.3

### Example 6 Synthesis of Compound 143

### (Step 1)

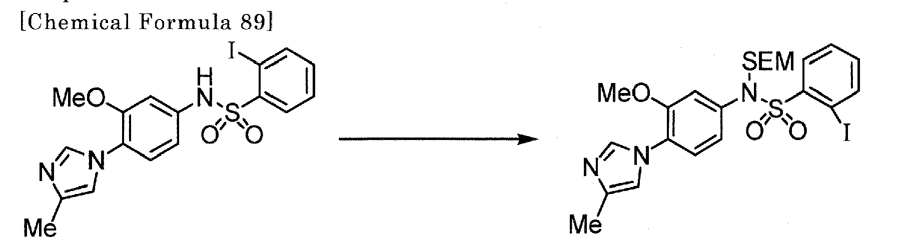

2-Iodo-N-(3-methoxy-4-(4-methyl-1H-imidazole-1-yl) phenyl) benzenesulfonamide (4.17 g, 8.9 mmol) was dissolved in dichloromethane (100 ml) and 2 mol/L NaOH (100 ml) was added, followed by stirring under ice-cooling. To the reaction mixture was added dropwise a solution of SEM-C1 (1.55 g, 9.3 mmol) in dichloromethane (10 ml). After 30 minutes, dichloromethane layer was extracted, washed with water and saturated brine and concentrated to dryness under reduced pressure. The residue was purified by silica gel chromatography (MeOH:CHCl₃ 97:3 v/v) to afford the target compound as a pale red powder (2.87 g, 54%).
LC-MS (Method E): 2.59 min, [M+H] =610.0

### (Step 2)

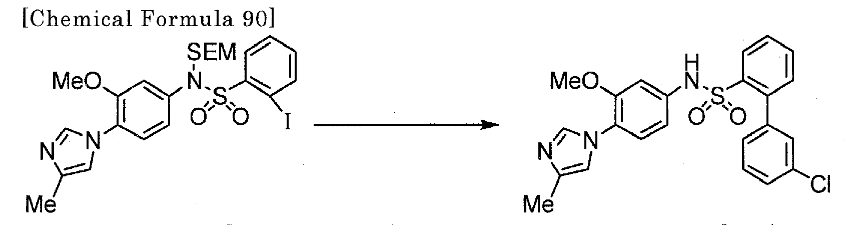

2-Iodo N-[3-methoxy-4 -(4-methylimidazol-1-yl phenyl]-N-(2-trimethyl silyl ethoxymethyl)-benzene sulfonamide (39.6 mg, 0.067 mmol) and 3-chlorophenyl boronic acid (21 mg, 0.132 mmol) were dissolved in dioxane (1.5 ml). To the mixture were added PdEnCat (registered trademark) TPP30 (20 mg) and 2 mol/L Na₂CO₃ (0.15 ml) under nitrogen atmosphere, and the mixture was heated with stirring at 130 °C for 4 hours. The reaction mixture was filtered off, the filtrate was concentrated and extracted with dichloromethane and water. The organic layer was concentrated under reduced pressure, 20% trifluoroaceticacid-dichloromethane (2 ml) was added, and the mixture was stirred at room temperature. The reaction mixture was concentrated under reduced pressure and purified by LC/MS (Method E) to afford the target compound as a pale red solid (21 mg, 79%).
LC-MS (Method C): 2.01 min, [M+H] =45409

### Example 7 Synthesis of Compound 112

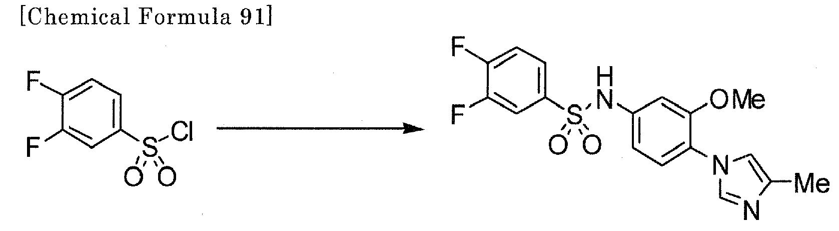

3,4-Difluorobenzene 1-sulfonylchloride (0.15 g, 0.706 mmol) and 3-methoxy-4-(4-methyl-imidazol-1-yl)-phenylamine (0.158 g, 0.776 mmol) were dissolved in pyridine (3.00 ml) and stirred at room temperature overnight. The reaction mixture was concentrated to dryness under reduced pressure. To the residue were added AcOEt 20 ml and an aqueous solution of saturated sodium bicarbonate 15 ml and the mixture was partitioned between an organic layer and an aqueous layer. The organic layer was washed twice with water 20 ml, dried over anhydrous magnesium sulfate and concentrated to dryness under reduced pressure to afford the residue 0.287 g as a yellow solid.

The residue was purified by silica gel chromatography (CHCl₃:MeOH 100:0 v/v → 95:5 v/v). To the residue was added Et₂O 10 ml and filtered to afford the target compound as a white powder (0.234 g, 87%).
LC-MS (Method B): 1.19 min, [M+H] =380.08
1H NMR (300 MHz, DMSO-d6) δ 10.67 (bs, 1H), 7.89 (m, 1H), 7.75 - 7.63 (m, 2H), 7.65 (s, 1H), 7.23 (d, J = 8.4 Hz, 1H), 7.01 (s, 1H), 6.92 (d, J = 2.1 Hz, 1H), 6.73 (dd, J = 8.4 Hz, J = 2.1 Hz, 1H), 3.73 (s, 3H), 2.11 (s, 3H).

### Example 8 Synthesis of Compound 2

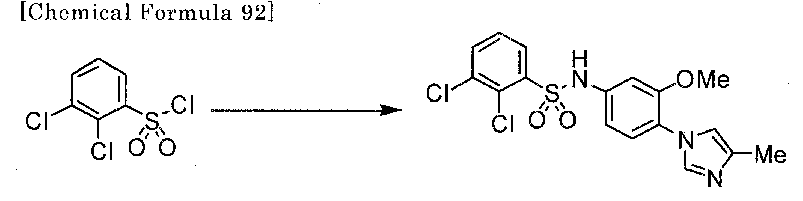

2,3-Dichlorobenzene-1-sulfonylchloride (0.15 g, 0.611 mmol) and 3-methoxy-4-(4-methyl imidazol-1-yl)phenylamine (0.137 g, 0.672 mmol) were dissolved in pyridine (3.00 ml) and the mixture was stirred at room temperature overnight. The reaction mixture was concentrated to dryness under reduced pressure. To the residue were added AcOEt 30 ml and water 15 ml, and the mixture was partitioned between an organic layer and an aqueous layer. The organic layer was washed twice with water 15 ml, dried over anhydrous magnesium sulfate and concentrated to dryness under reduced pressure. To the residue was added Et₂O 5.0 ml and filtered to afford the target compound as a white powder (0.200 g, 79%).
LC-MS (Method B): 1.28 min, [M+H] =412.02
1H NMR (300 MHz, DMSO-d6) δ 11.0 (bs, 1H), 8.13 (dd, J = 8.1 Hz, J = 1.5 Hz, 1H), 7.95 (dd, J = 8.1 Hz, J = 1.5 Hz, 1H), 7.63 (s, 3H), 7.58 (t, J = 8.1 Hz, 1H), 7.20 (d, J = 8.7 Hz, 1H), 6.99 (s, 1H), 6.90 (d, J = 2.4 Hz, 1H), 6.71 (dd, J = 8.7 Hz, J = 2.4 Hz, 1H), 3.70 (s, 3H), 2.10 (s, 3H).

### Example 9 Synthesis of Compound 5

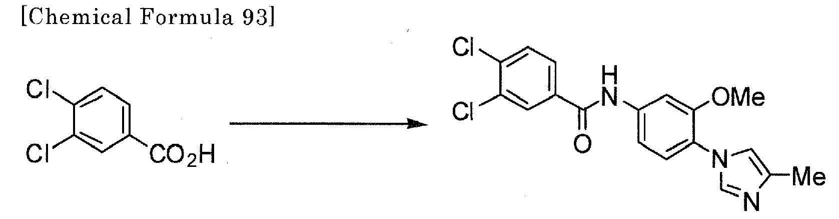

To a solution of 3,4-dichlorobenzoic acid (0.300 g, 1.57 mmol) in DMF (5.0 ml) were added HATU (0.717 g, 1.89 mmol), Et₃N (0.327 ml, 2.36 mmol) and 3-methoxy-4-(4-methylimidazol-1-yl)phenylamine and the mixture was stirred at room temperature overnight. To the mixture were added AcOEt 30 ml and a saturated aqueous solution of sodium bicarbonate 15 ml and the mixture was partitioned between an organic layer and an aqueous layer. The organic layer was washed twice with water 15 ml, dried over anhydrous magnesium sulfate and concentrated to dryness under reduced pressure. The residue was purified by silica gel chromatography (MeOH:CHCl₃ 95:5 v/v) to afford the target compound as a white solid (0.404 g, 68%).
LC-MS (Method B): 1.47 min, [M+H] =376.05

### Example 10 Synthesis of Compound 128

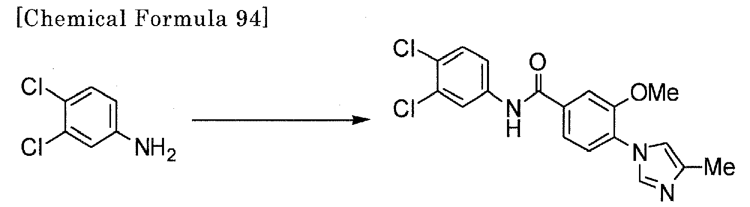

To a solution of 3-methoxy-4-(4-methyl imidazol-1-yl)benzoate (0.350 g, 1.51 mmol) in DMF (5.0 ml) were added HATU (0.688 g, 1.81 mmol), Et₃N (0.313 ml, 2.26 mmol) and 3,4-dichloroaniline (0.244 g, 1.51 mmol) and the mixture was stirred at room temperature overnight. To the mixture were added AcOEt 30 ml and saturated aqueous solution of sodium bicarbonate 15 ml and the mixture was partitioned between an organic layer and an aqueous layer. The organic layer was washed twice with water 15 ml, dried over anhydrous magnesium sulfate and concentrated to dryness under reduced pressure. The residue was purified by silica gel chromatography (MeOH:CHCl₃ 95:5 v/v) to afford the target compound as a yellow solid (0.391 g, 69%).
LC-MS (Method B): 1.49 min, [M+H] =37605
1H NMR(300 MHz, DMSO-d6) delta 10.54 (s, 1H), 8.13 (d and J=3.0 Hz, 1H), 8.12 (br, 1H), 7.78-7.58 (m, 5H), 7.31 (s, 1H), 3.94 (s, 3H), 2.19 (s, 3H).

### Example 11 Synthesis of Compound 201

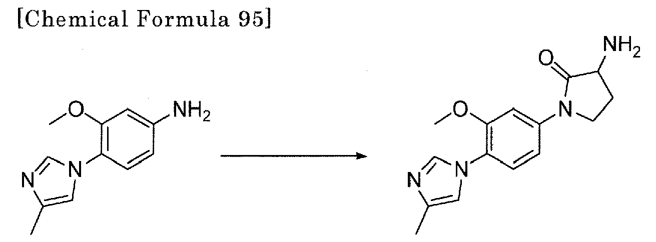

3-Methoxy-4-(4-methyl-1H-imidazol-1-yl)aniline (5.00 g, 24.60 mmol) and DIPEA (5.20 mL, 29.5 mmoL) were dissolved in dichloromethane (50 mL), and 2,4-dibromobutanoyl chloride (6.50 g, 24.60 mmoL) was added under ice-cooling, followed by stirring at room temperature for 1 hour. To the mixture were added ethyl acetate (100 ml) and water (50 ml), and the mixture was partitioned between an organic layer and and aqueous layer. The organic layer was washed with saturated brine, dried over anhydrous magnesium sulfate and concentrated to dryness under reduced pressure. The obtained residue was dissolved in DMF (50 mL), potassium carbonate (3.40 g, 24.60 mmoL) was added, and the mixture was stirred at room temperature for 3 hours. To the reaction mixture were added sodium azide (2.40 g, 36.9 mmol) and sodium iodide (369 mg, 2.46 mmol), and the mixture was stirred at room temperature overnight. To the mixture were added ethyl acetate (100 ml) and water (50 ml), and the mixture was partitioned between an organic layer and an aqueous layer. The organic layer was washed with saturated brine, dried over anhydrous magnesium sulfate and concentrated to dryness under reduced pressure. The obtained residue was dissolved in tetrahydrofuran (50 mL) and water (4.43 ml, 0.25 mol) and triphenylphosphine (7.74 g, 29.5 mmol) were added. The mixture was stirred at 50 °C for 4 hours and concentrated. The residue was purified by silica gel chromatography (MeOH/CHCl₃ 0-20%) to afford the target compound as a yellow powder (7.04 g, 80%).
LC-MS (Method E): 1.82 min, [M+H] =287.14

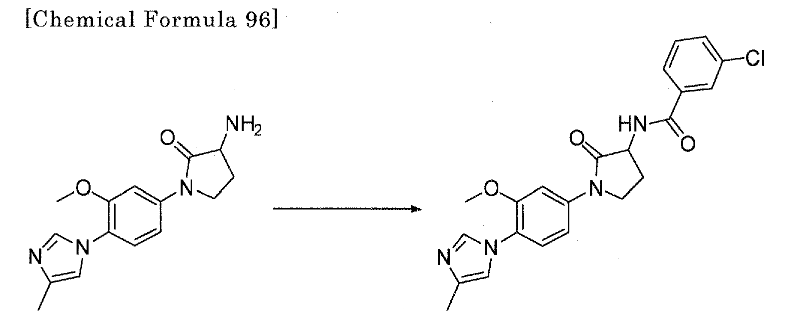

3-Amino-1-(3-methoxy-4-(4-methyl-1H-imidazol-1-yl)phenyl)pyrrolidin-2-one (30 mg, 0.11 mmol) and pyridine (13 ul, 0.158 mmol) were dissolved in dichloromethane (1 mL) and 3-chlorobenzoyl chloride (24 mg, 0.14 mmol) was added under ice-cooling, followed by stirring at room temperature for 30 minutes. To the mixture were added ethyl acetate (5 ml) and water (5 ml) and the mixture was partitioned between an organic layer and an aqueous layer. The organic layer was washed with saturated brine, dried over anhydrous magnesium sulfate and concentrated to dryness under reduced pressure. To the residue was added ethyl acetate, and the mixture was filtered to afford the target compound as a white powder (24 mg, 56%).
LC-MS (Method C): 1.60 min, [M+H] =425.13

### Example 12 Synthesis of Compound 229

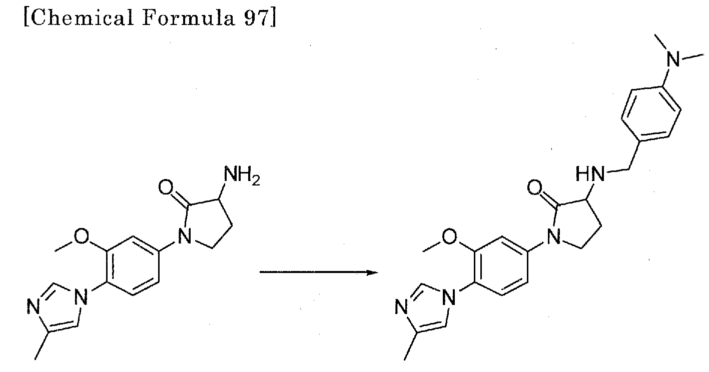

3-Amino-1-(3-methoxy-4-(4-methyl-1H-imidazol-1-yl phenyl)pyrrolidin-2-one (30 mg, 0.11 mmol) and 4-(dimethylamino)benzaldehyde (18 mg, 0.12 mmol) were dissolved in a mixed solution of DMF (0.9 mL) and acetic acid (0.1 mL) and stirred at 65 °C for 1.5 hours. After ice-cooling, sodium borohydride (8 mg, 0.20 mmol) was added, followed by stirring at room temperature for 30 minutes. After added 2 mol/L hydrochloric acid (0.5 mL) and stirred for 10 minutes, a saturated aqueous solution of sodium bicarbonate (2 mL) was added to the mixture. The reaction mixture was extracted with chloroform (5 mL). The organic layer was washed with saturated brine, dried over anhydrous magnesium sulfate and concentrated to dryness under reduced pressure. The residue was purified by silica gel chromatography (MeOH/CHCl₃ 0-20%) to afford the target compound (4.5 mg, 11%).
LC-MS(method E): 2.80 min, [M+H] =420.23

### Example 13 Synthesis of Compound 196

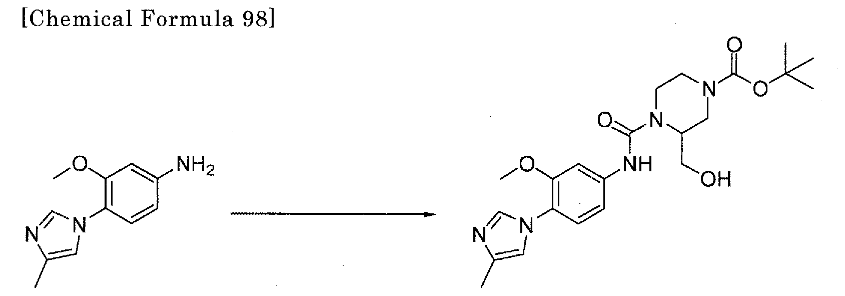

3-Methoxy-4-(4-methyl-1H-imidazol-1-yl)aniline (2.00 g, 9.84 mmoL) was dissolved in 1,4-dioxane (10 ml), and di(1H-imidazol-1-yl)methanone (3.20 g, 19.68 mmoL) was added at 5 °C, followed by stirring at room temperature for 2 hours. The reaction mixture was poured into water and the precipitate was collected by filtration and dried. The obtained solid was dissolved in 1,4-dioxane (10 ml), and the tert-butyl 3-(hydroxymethyl)piperazine-1-carboxylate (1.53 g, 7.10 mmoL) was added. The reaction mixture was stirred at 80°C for 2 hours. To the mixture were added ethyl acetate (30 ml) and water (15 ml), and the mixture was partitioned between an organic layer and an aqueous layer. The organic layer was washed with saturated brine, dried over anhydrous magnesium sulfate and concentrated to dryness under reduced pressure. The residue was purified by silica gel chromatography (MeOH/CHCl₃:0%-10%) to afford the target compound (3.41 g, 87%).
LC-MS (Method E): 2.52 min, [M+H] =44623

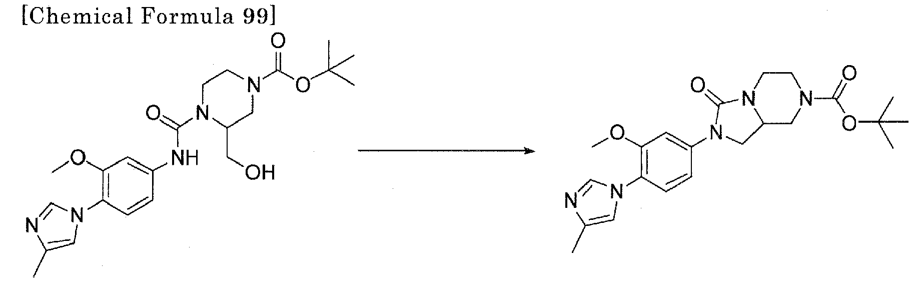

Tert-butyl 3-(hydroxymethyl)-4-(3-methoxy-4-(4-methyl-1H-imidazol-1-yl)phenylcarbonyl piperazin-1-carboxylate (2.89 g, 6.49 mmol) and pyridine (0.79 ml, 9.73 mmol) were dissolved in dichloromethane (15 ml) and methane sulfonylchloride (966 mg, 8.43 mmol) was added, followed by stirring at room temperature for 1 hour. To the mixture were added ethyl acetate (50 ml) and water (30 ml), and the mixture was partitioned between an organic layer and an aqueous layer. The organic layer was washed with saturated brine, dried over anhydrous magnesium sulfate and concentrated under reduced pressure. The residue was dissolved in DMF (15 ml), potassium carbonate (1.97 mg, 14.27 mmol) was added, and the mixture was stirred at 60 °C for 4 hours. Ethyl acetate (50 ml) and water (30 ml) were added and the mixture was partitioned between an organic layer and an aqueous layer. The organic layer was washed with saturated brine, dried over anhydrous magnesium sulfate and concentrated under reduced pressure. The residue was purified by silica gel chromatography (MeOH/CHCl₃:0%-5%) to afford the target compound (696 mg, 25%).
LC-MS (Method C): 1.83 min, [M+H] =428.22

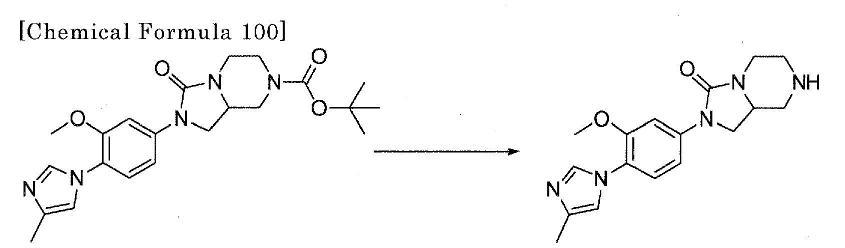

Tert-butyl 2-(3-methoxy-4-(4-methyl-1H-imidazol-1-yl)phenyl)-3-oxohexahydroimidazo[1,5-a]pyrazin-7(1H)-carboxylate (440 mg, 1.03 mmol) was dissolved in a solution of 4 mol/L hydrochloric acid in dioxane and stirred at room temperature overnight. The precipitate was collected by filtration and washed with 1,4-dioxane. To the obtained solid were added ethyl acetate (10 ml) and saturated aqueous solution of sodium bicarbonate (5 ml), and the mixture was partitioned between an organic layer and an aqueous layer. The organic layer was washed with saturated brine, dried over anhydrous magnesium sulfate and concentrated under reduced pressure to afford the target compound (337 mg, 95%).
LC-MS (Method E): 1.97 min, [M+H] =328.17

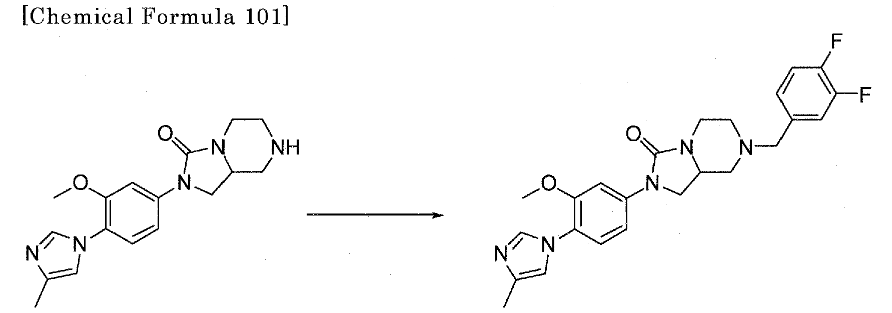

2-(3-Methoxy- 4-(4-methyl-1H-imidazol-1-yl) phenyl)hexahydroimidazo[1,5-a]pyrazin-3(5H)-one (26 mg, 0.079 mmol) and DIPEA (21 ul and 0.12 mmol) were dissolved in isopropylalcohol (1 ml) and 4-(bromomethyl)-1,2-difluorobenzene (18 mg, 0.087 mmol) was added under ice-cooling, followed by stirring at room temperature for 3 days. To the mixture were added ethyl acetate (10 ml) and water (5 ml), and the mixture was partitioned between an organic layer and an aqueous layer. The organic layer was washed with saturated brine, dried over anhydrous magnesium sulfate and concentrated under reduced pressure. The residue was purified by silica gel chromatography (MeOH/CHCl₃:0%-20%) to afford the target compound (24 mg, 67%). LC-MS (Method E): 2.88 min, [M+H] =454.20

### Example 14 Synthesis of Compound 199

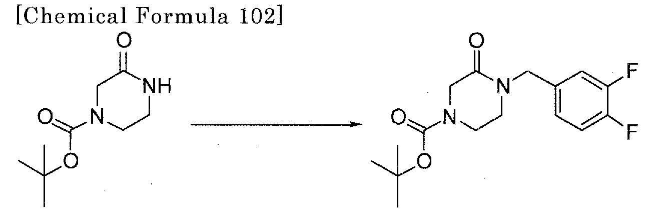

Sodium hydride (120 mg, 3.00 mmol) was suspended in 1,4-dioxane and tert-butyl 3-oxopiperazin-1-carboxylate (500 mg, 2.50 mmol) was added under ice-cooling, followed by stirring at room temperature for 30 minutes. After ice-cooling, 4-(bromomethyl)-1,2-difluorobenzene (620 mg, 3.00 mmol) was added, followed by stirring at room temperature for 3 hours. Ethyl acetate (20 ml) and water (10 ml) were added and the mixture was partitioned between an organic layer and an aqueous layer. The organic layer was washed with saturated brine, dried over anhydrous magnesium sulfate and concentrated under reduced pressure. The residue was purified by silica gel chromatography (AcOEt/Hexane:0%-20%) to afford the target compound (815 mg, 98%).
LC-MS (Method C): 2.62 min, [M+H] =327.14

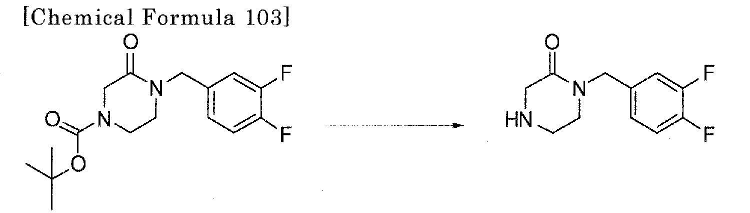

Tert-butyl 4-(3,4-difluorobenzyl)-3-oxopiperazin-1-carboxylate (800 mg, 2.45 mmol) was dissolved in a solution of 4 mol/L hydrochloric acid in dioxane (10 ml) and stirred at room temperature for 3 hours. The precipitate was collected by filtration and washed with 1,4-dioxane (10 ml). To the obtained solid were ethyl acetate (10 ml) and a saturated aqueous solution of sodium bicarbonate (5 ml), and the mixture was partitioned between an organic layer and an aqueous layer. The organic layer was washed with saturated brine, dried over anhydrous magnesium sulfate and concentrated under reduced pressure to afford the target compound (555 mg, 70%).
LC-MS (Method E): 1.71 min, [M+H] =227.09

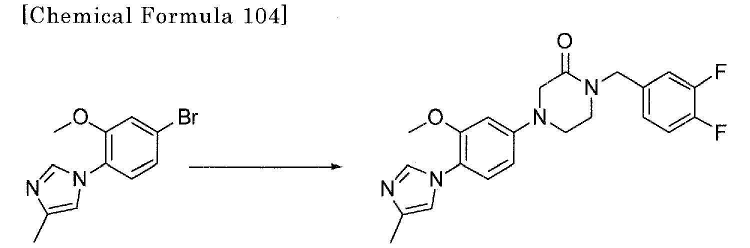

1-(4-Bromo-2-methoxyphenyl)-4-methyl-1H-imidazole (100 mg, 0.37 mmol) was dissolved in 1,4-dioxane (3 ml). To the mixture were added 1-(3,4-difluorobenzyl)piperazin-2-one (93 mg, 0.41 mmol), sodium 2-methyl butan-2-olate (58 mg, 0.52 mmol), palladium acetate (II) (8.4 mg, 0.037 mmol) and RuPhos (26 mg, 0.056 mmol), and the mixture was stirred at 100 °C for 6 hours. Ethyl acetate (20 ml) and water (10 ml) were added, and the mixture was partitioned between an organic layer and an aqueous layer. The organic layer was washed with saturated brine, dried over anhydrous magnesium sulfate and concentrated under reduced pressure. The residue was purified by silica gel chromatography (MeOH/CHCl₃:0%-20%) to afford the target compound (139 mg, 90%).
LC-MS (Method E): 2.68 min, [M+H] =413.17

### Example 14 Synthesis of Compound 255

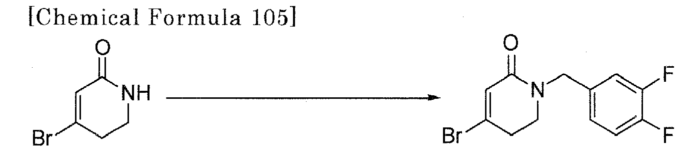

Potassium tert-butoxide (30 mg, 0.17 mmol) was suspended in tetrahydrofuran (1 ml). To the suspension was added 4-promo-5, 6-dihydropyridin-2(1H)-one (30 mg, 0.17 mmol) under ice-cooling and stirred at the same temperature for 15 minutes. After 4-(bromomethyl)-1,2-difluorobenzene (42.3 mg, 0.21 mmol) was added under ice-cooling, the mixture was allowed to warm to room temperature and stirred for 1 hour. Ethyl acetate (10 ml) and water (5 ml) were added, and the mixture was partitioned between an organic layer and an aqueous layer. The organic layer was washed with saturated brine, dried over anhydrous magnesium sulfate and concentrated under reduced pressure. The residue was purified by silica gel chromatography (AcOEt/Hexane:0%-20%) to afford the target compound (5 mg, 10%).
LC-MS(method C): 2.23 min, [M+H] =301.99

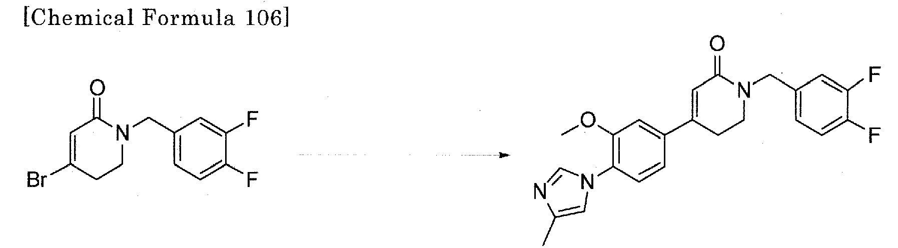

4-Bromo-1-(3,4-di.fluorobenzyl)-5, 6-dihydropyridine-2(1H)-one (5 mg, 0.017mmol) was dissolved in a mixed solvent of ethanol (1.6 ml) and 2 mol/L aqueous solution of sodium carbonate (0.4ml). To the mixture were added 1-(2-methoxy-4-(4,4,5,5-tetramethyl-1,3,2-dioxaborolan-2-yl)phenyl)-4-methyl-1H-imidazole (8 mg, 0.025 mmo) and tetrakis(triphenylphosphine)palladium (0) (2 mg, 1.7umol). The mixture was reacted by a microwave reactor at 120°C for 30 minutes. To the reaction mixture were added ethyl acetate (10 ml) and water (5 ml), and the mixture was partitioned between an organic layer and an aqueous layer. The organic layer was washed with saturated brine, dried over anhydrous magnesium sulfate and concentrated under reduced pressure. The residue was purified by silica gel chromatography (MeOH/CHCl₃:0%-10%) to afford the target compound (3.5 mg, 52%).
LC-MS (Method E): 2.85 min, [M+H] =41016

### Example 15 Synthesis of Compound 283

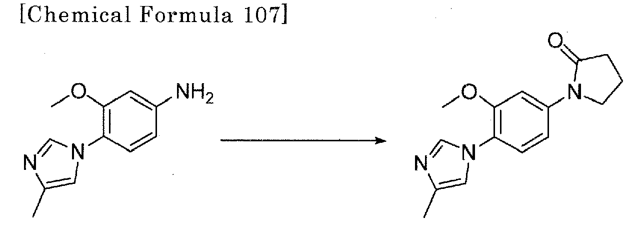

3-Methoxy-4-(4-methyl-1H-imidazol-1-yl)aniline (2.00 g, 9.84 mmol) and pyridine (0.96 ml, 11.81 mmol) were dissolved in dichloromethane (20 ml). 4-Chlorobutanoyl chloride (1.46 g, 10.33 mmol) was added under ice-cooling and the mixture was allowed to warm to room temperature and stirred for 1 hour. To the reaction mixture were added ethyl acetate (50 ml) and water (30 ml), and the mixture was partitioned between an organic layer and an aqueous layer. The organic layer was washed with saturated brine, dried over anhydrous magnesium sulfate and concentrated under reduced pressure. The residue was dissolved in DMF (10 ml) and potassium carbonate (2.99 g, 21.65 mmol) was added, followed by stirring at 60°C for 3 hours. To the reaction mixture were added ethyl acetate (40 ml) and water (30 ml), and the mixture was partitioned between an organic layer and an aqueous layer. The organic layer was washed with saturated brine, dried over anhydrous magnesium sulfate and concentrated under reduced pressure. The residue was purified by silica gel chromatography (MeOH/CHCl₃:0%-5%) to afford the target compound (1.01 g, 38%).
LC-MS (Method C): 1.06 min, [M+H] =27213

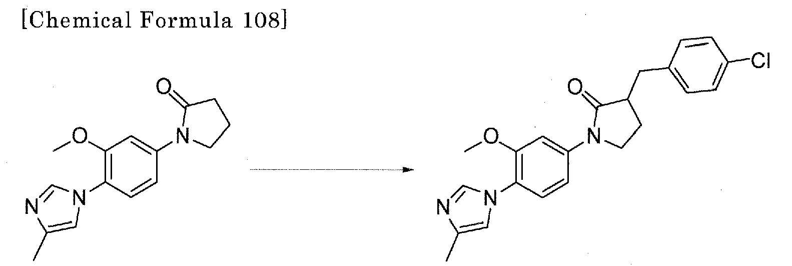

1-(3-Methoxy- 4-(4-methyl-1H-imidazol-1-yl)phenyl)pyrrolidin-2-one (100 mg, 0.369 mmol) was dissolved in tetrahydrofuran (3 ml). A solution of lithium hexamethyldisilazide in tetrahydrofuran (442 ul, 0.442 mmol, 1 mol/L) was added at -78 °C and stirred at the same temperature for 30 minutes. 1-(Bromomethyl)-4-chlorobenzene (91 mg, 0.44 mmol) was added at -78°C and stirred at the same temperature for 90 minutes. To the reaction mixture was added water (5 ml), and the mixture was extracted with ethyl acetate (20 ml). The organic layer was washed with saturated brine, dried over anhydrous magnesium sulfate, and concentrated under reduced pressure. The residue was purified by silica gel chromatography (MeOH/CHCl₃:0%-5%) to afford the target compound (30 mg, 21%).
LC-MS (Method C): 2.05 min, [M+H] =396.14

The following compounds were synthesized in similar manners. The structures and physical properties (LC/MS retention time, mass spectrums and/or NMR spectrum, measurement conditions) are shown below.

**[Table 1]**

| | |
|---|---|
| 1 | 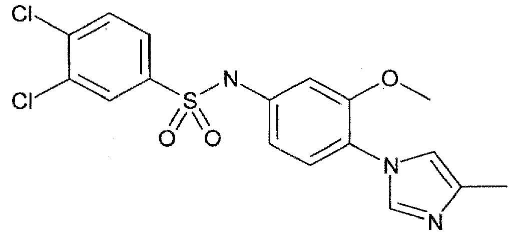 |
| 2 | 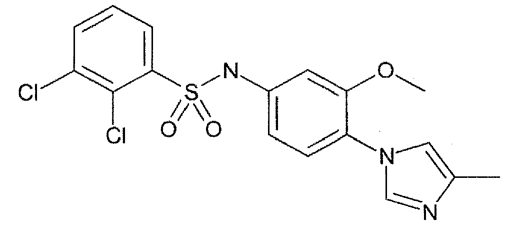 |
| 3 | 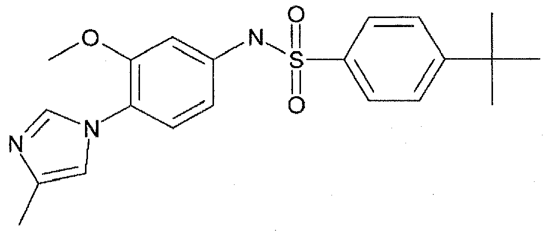 |
| 4 | 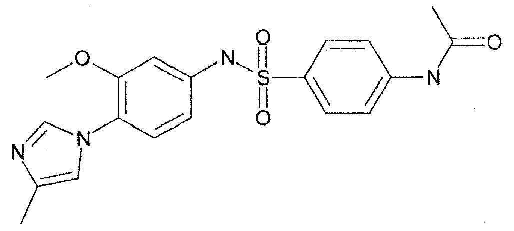 |
| 5 | 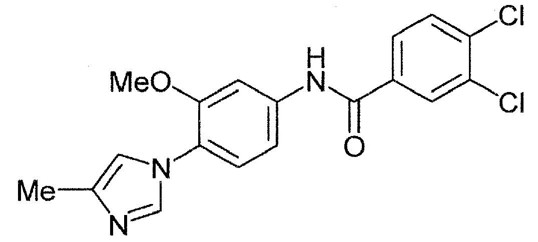 |

**[Table 2]**

| | |
|---|---|
| 6 | 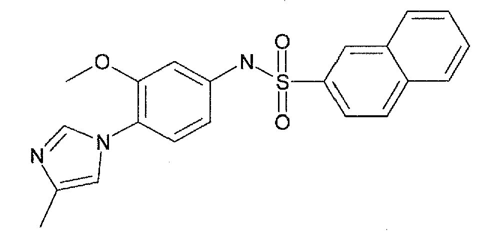 |
| 7 | 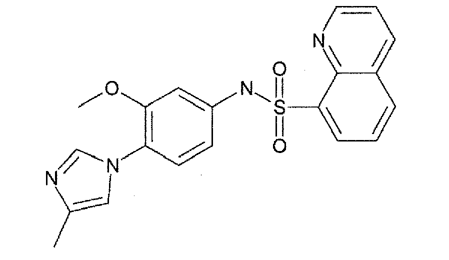 |
| 8 | 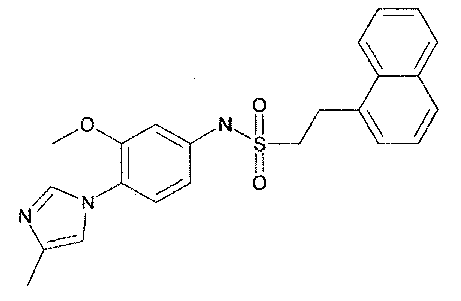 |
| 9 | 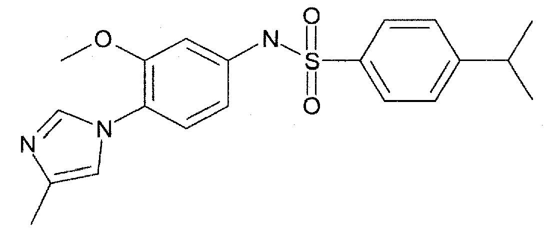 |
| 10 | 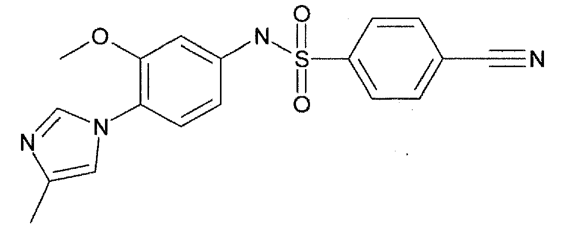 |

**[Table 3]**

| | |
|---|---|
| 11 | 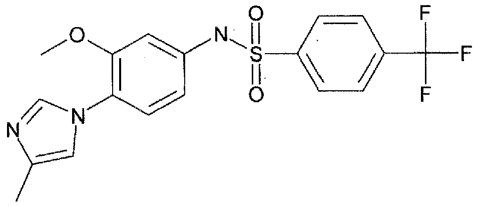 |
| 12 | 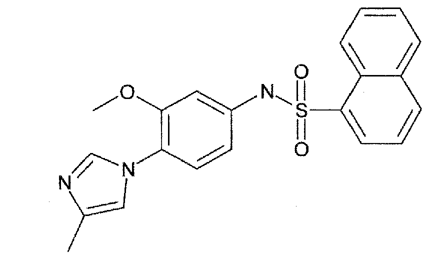 |
| 13 | 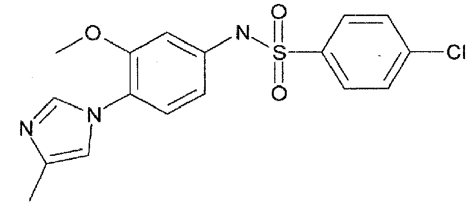 |
| 14 | 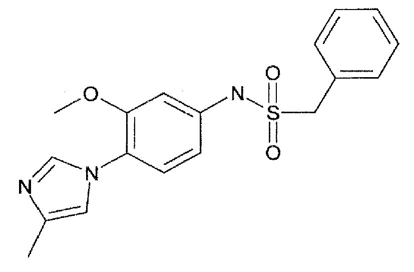 |
| 15 | 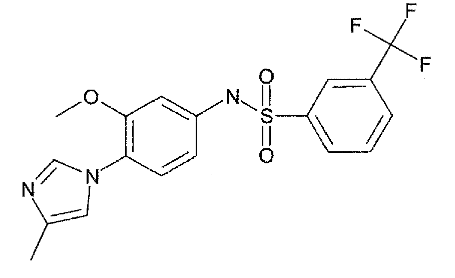 |

**[Table 4]**

| | |
|---|---|
| 16 | 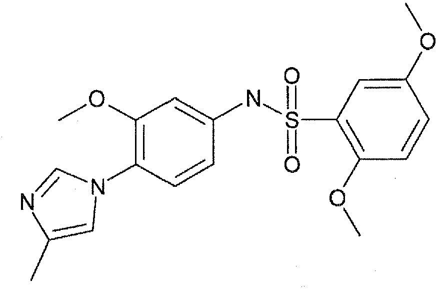 |
| 17 | 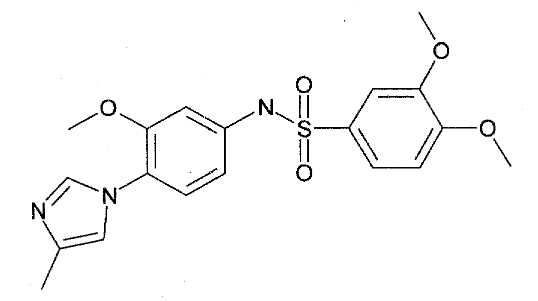 |
| 18 | 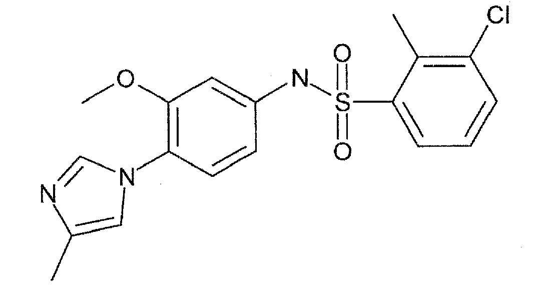 |
| 19 | 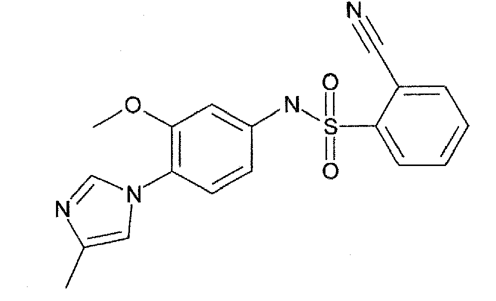 |

**[Table 5]**

| | |
|---|---|
| 20 | 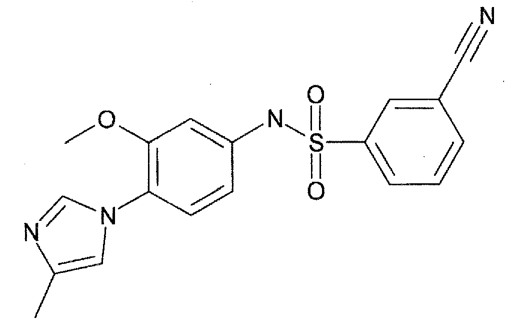 |
| 21 | 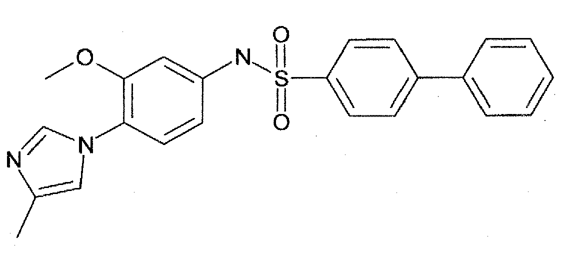 |
| 22 | 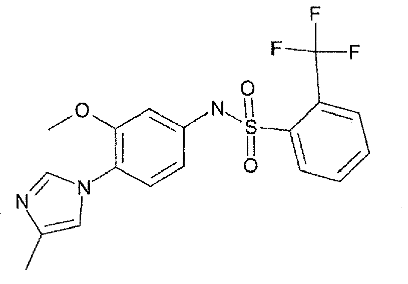 |
| 23 | 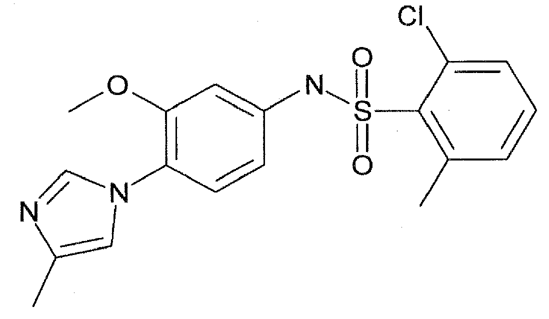 |

**[Table 6]**

| | |
|---|---|
| 24 | 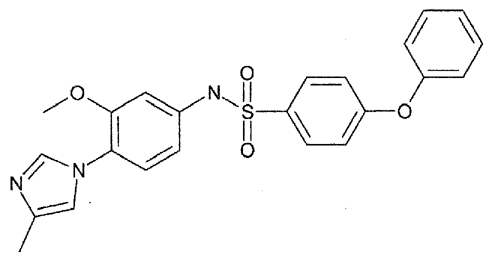 |
| 25 | 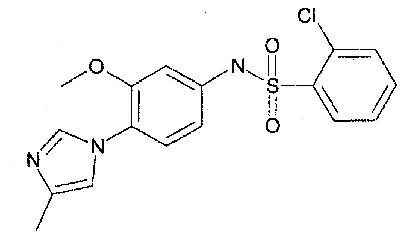 |
| 26 | 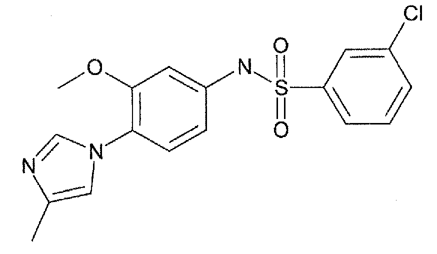 |
| 27 | 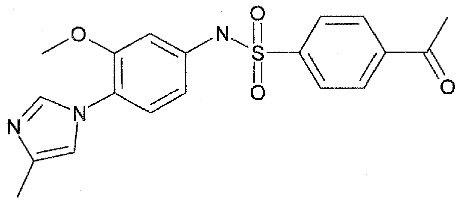 |
| 28 | 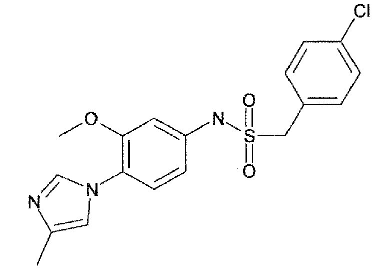 |

**[Table 7]**

| | |
|---|---|
| 29 | 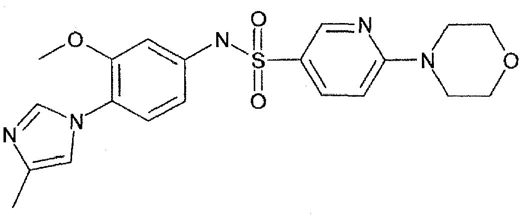 |
| 30 | 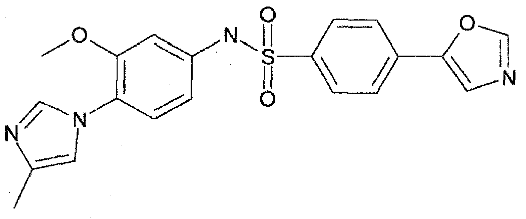 |
| 31 | 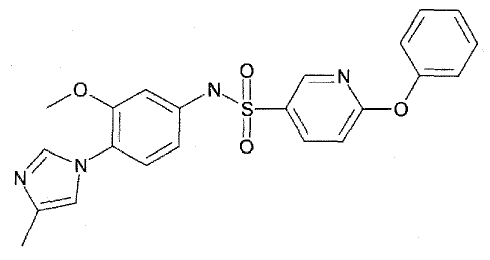 |
| 32 | 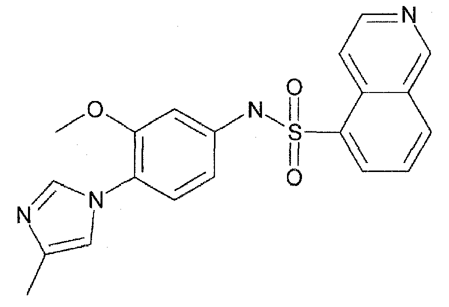 |
| 33 | 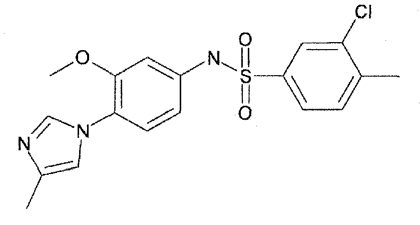 |

**[Table 8]**

| | |
|---|---|
| 34 | 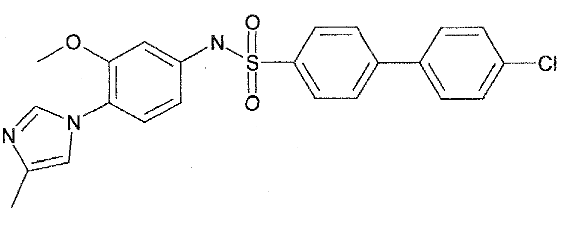 |
| 35 | 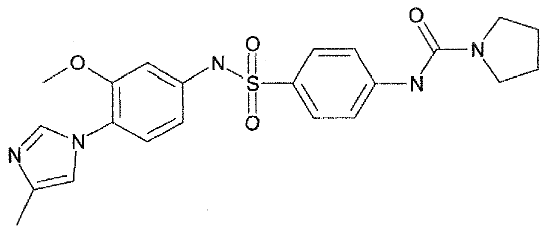 |
| 36 | 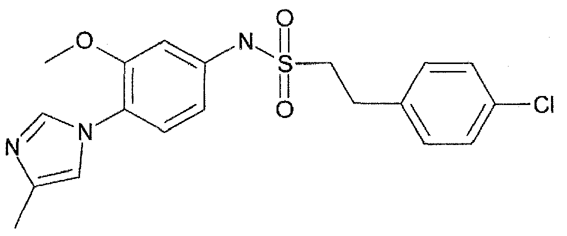 |
| 37 | 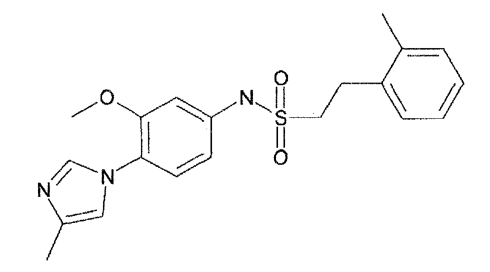 |
| 38 | 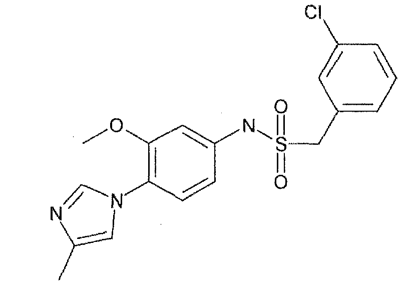 |

**[Table 9]**

| | |
|---|---|
| 39 | 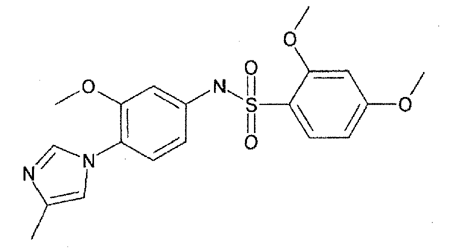 |
| 40 | 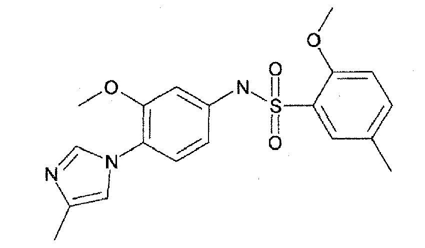 |
| 41 | 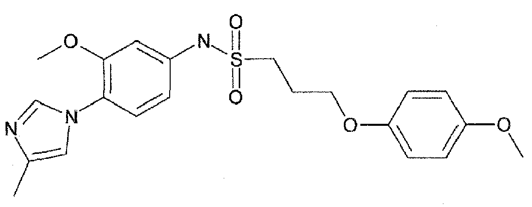 |
| 42 | 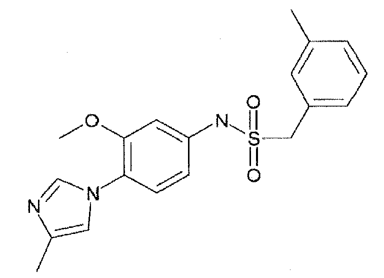 |
| 43 | 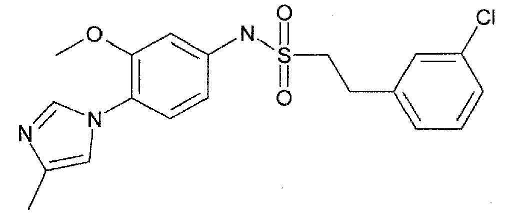 |

**[Table 10]**

| | |
|---|---|
| 44 | 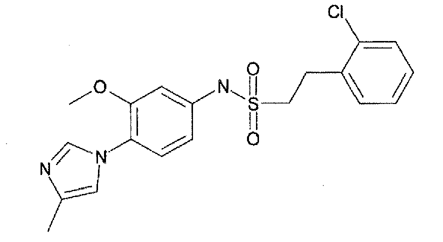 |
| 45 | 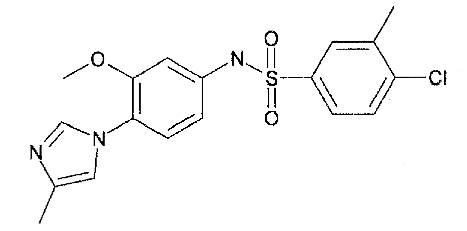 |
| 46 | 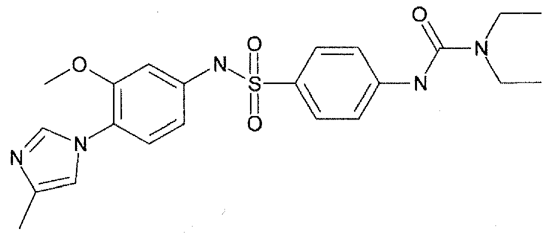 |
| 47 | 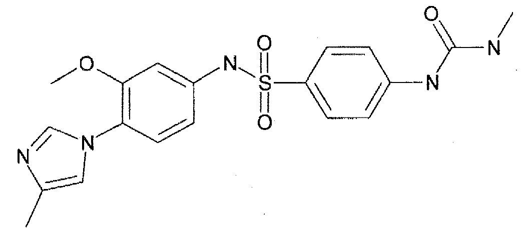 |
| 48 | 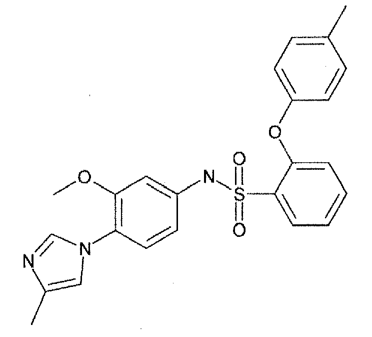 |

**[Table 11]**

| | |
|---|---|
| 49 | 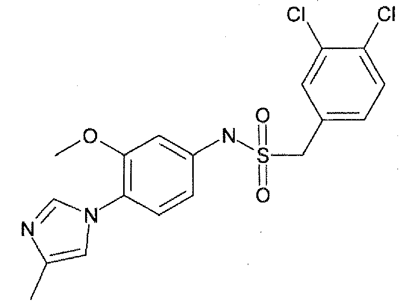 |
| 50 | 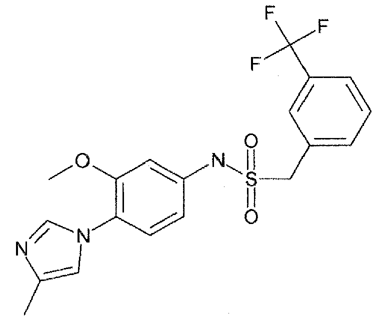 |
| 51 | 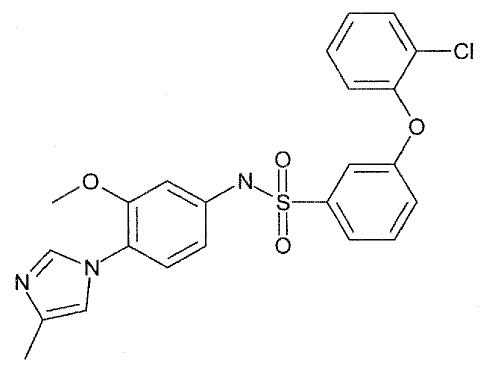 |
| 52 | 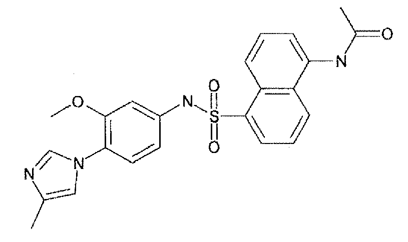 |

**[Table 12]**

| | |
|---|---|
| 53 | 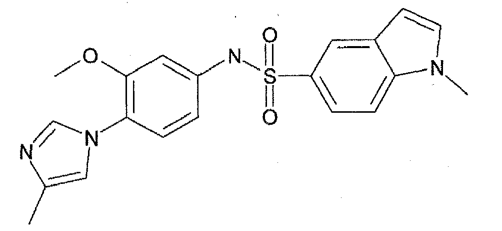 |
| 54 | 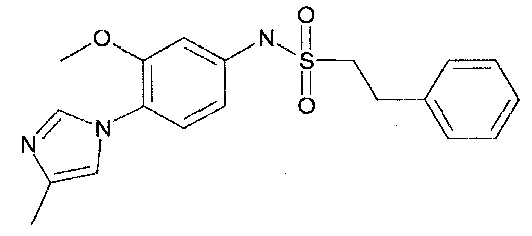 |
| 55 | 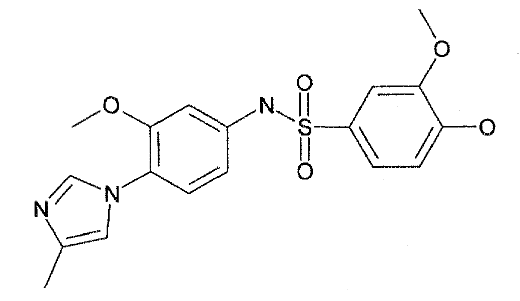 |
| 56 | 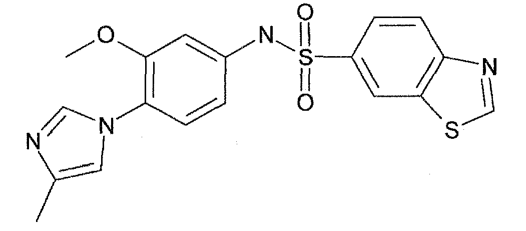 |
| 57 | 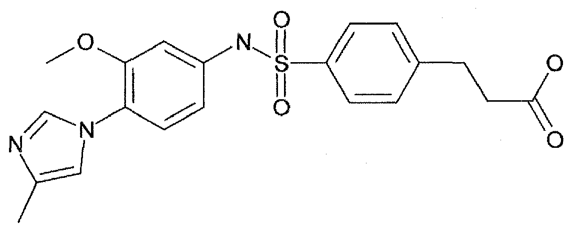 |

**[Table 13]**

| | |
|---|---|
| 58 | 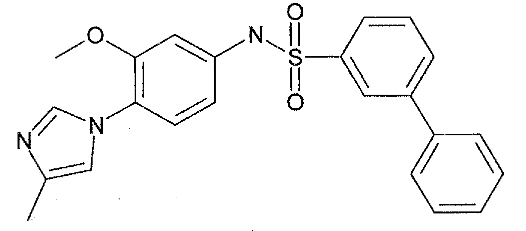 |
| 59 | 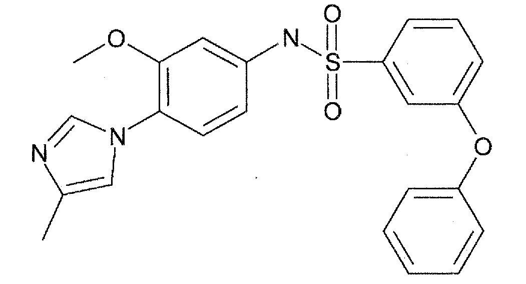 |
| 60 | 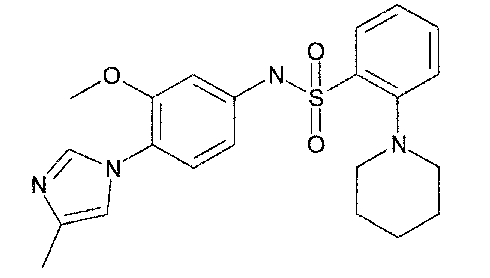 |
| 61 | 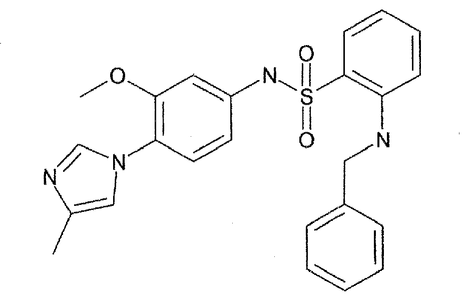 |

**[Table 14]**

| | |
|---|---|
| 62 | 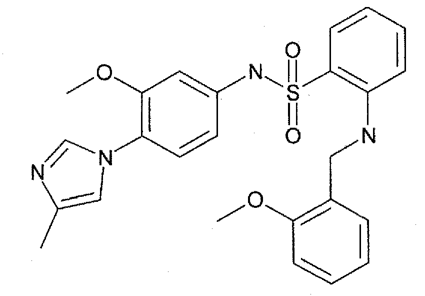 |
| 63 | 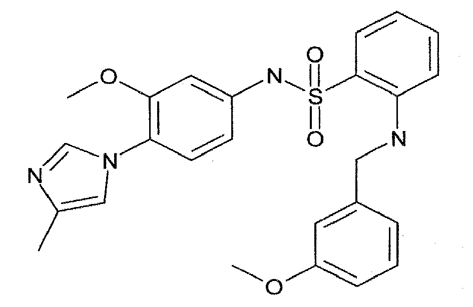 |
| 64 | 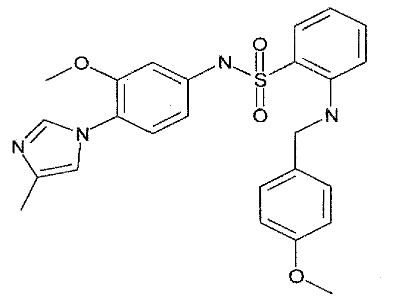 |
| 65 | 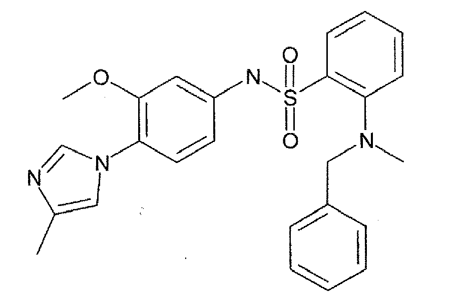 |

**[Table 15]**

| | |
|---|---|
| 66 | 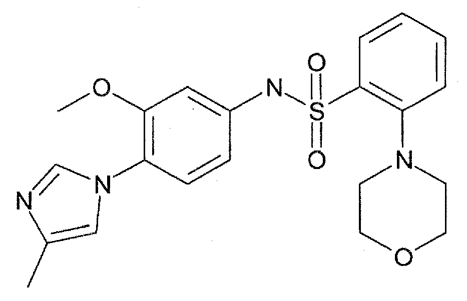 |
| 67 | 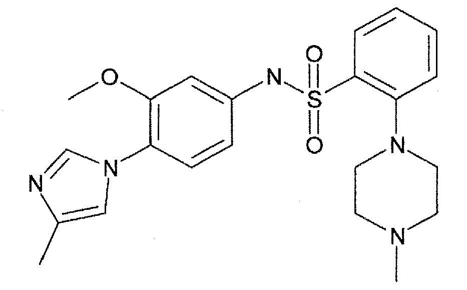 |
| 68 | 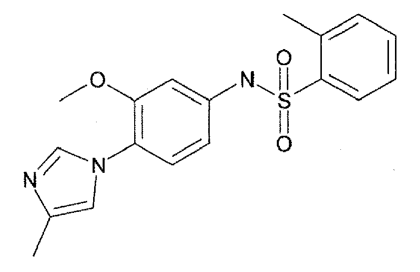 |
| 69 | 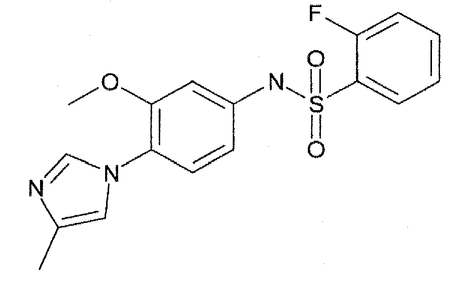 |

**[Table 16]**

| | |
|---|---|
| 70 | 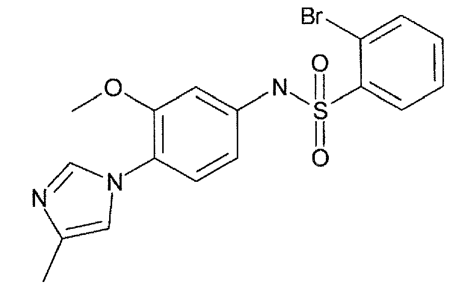 |
| 71 | 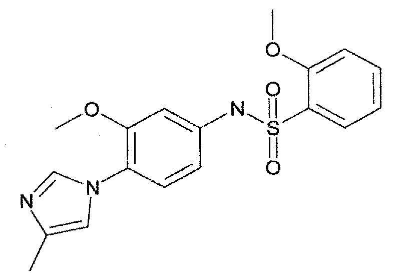 |
| 72 | 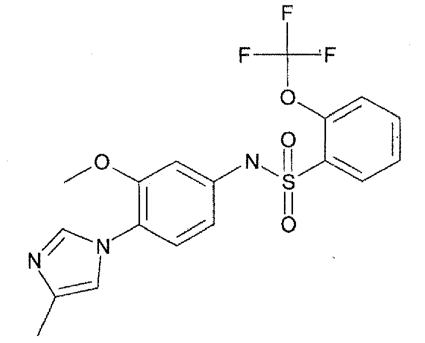 |
| 73 | 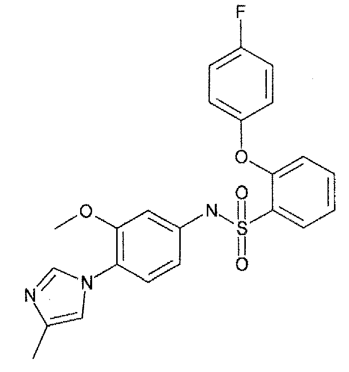 |

**[Table 17]**

| | |
|---|---|
| 74 | 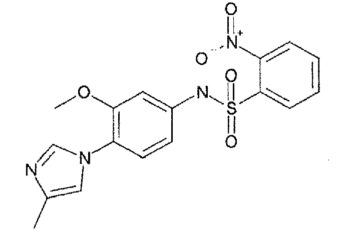 |
| 75 | 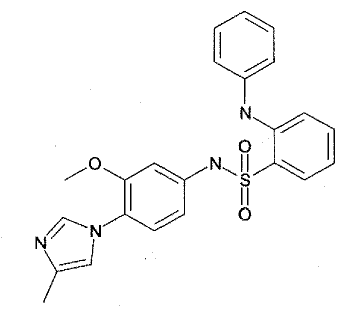 |
| 76 | 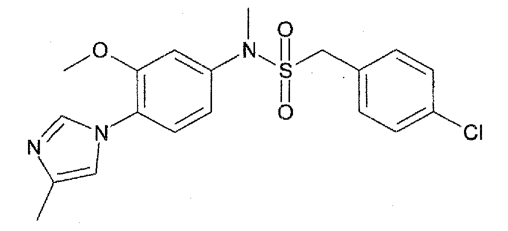 |
| 77 | 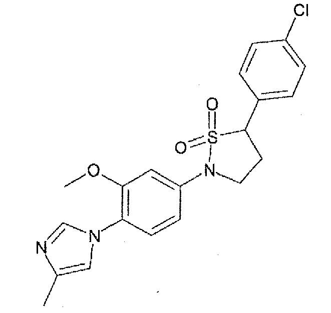 |

**[Table 18]**

| | |
|---|---|
| 78 | 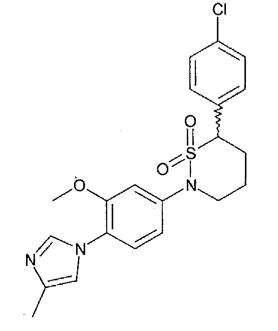 |
| 79 | 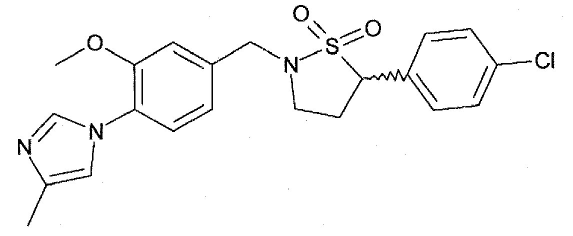 |
| 80 | 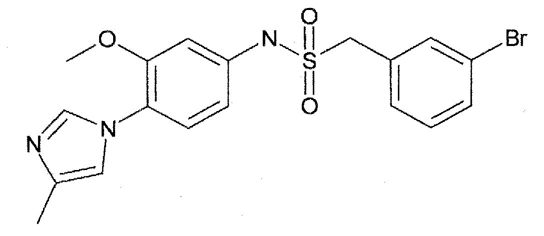 |
| 81 | 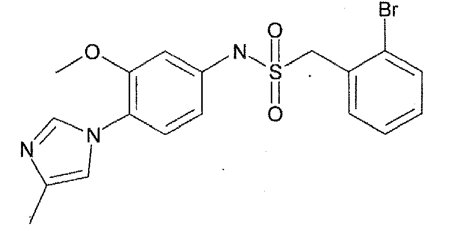 |
| 82 | 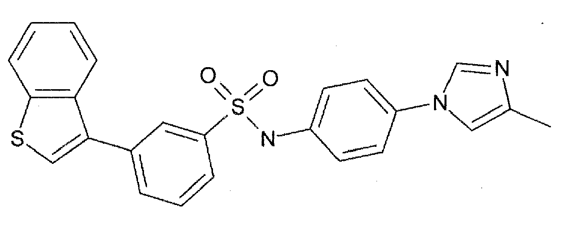 |

**[Table 19]**

| | |
|---|---|
| 83 | 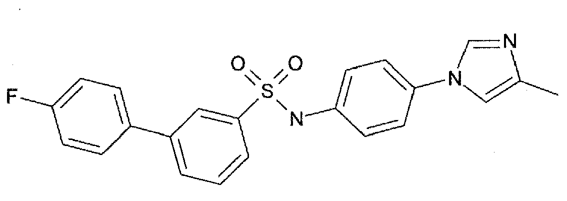 |
| 84 | 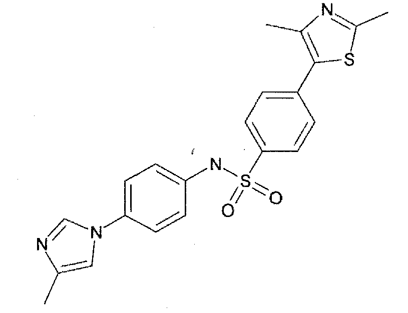 |
| 85 | 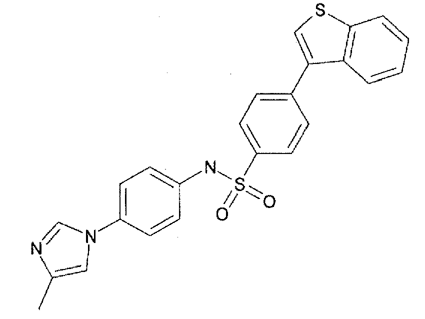 |
| 86 | 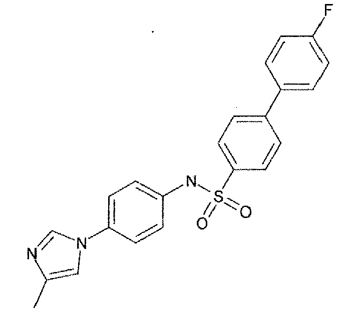 |

**[Table 20]**

| | |
|---|---|
| 87 | 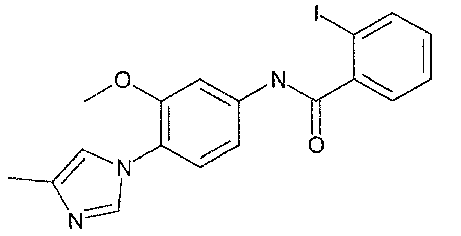 |
| 88 | 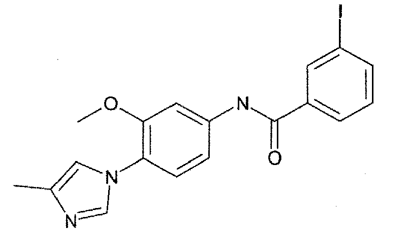 |
| 89 | 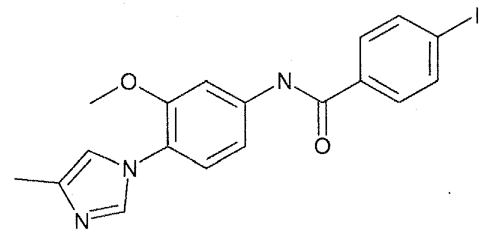 |
| 90 | 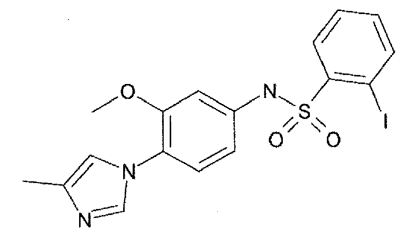 |
| 91 | 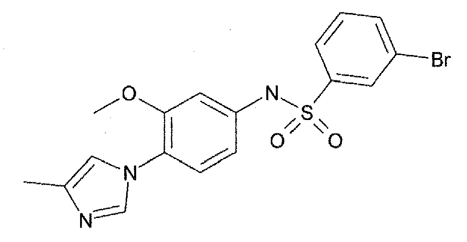 |

**[Table 21]**

| | |
|---|---|
| 92 | 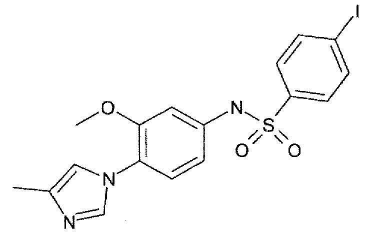 |
| 93 | 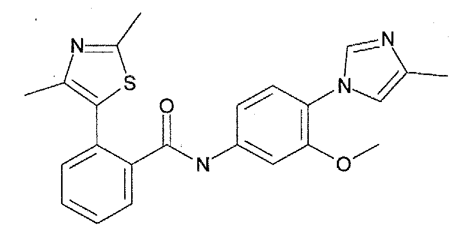 |
| 94 | 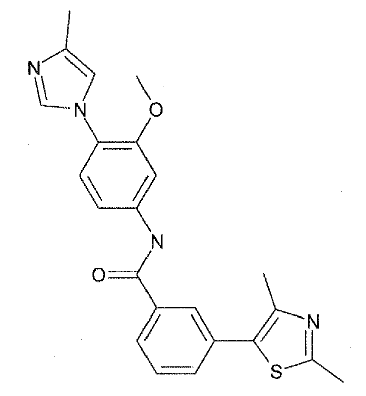 |
| 95 | 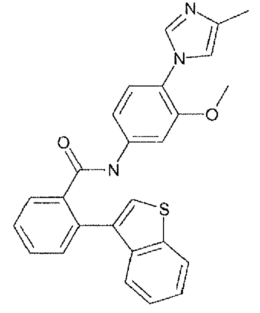 |

**[Table 22]**

| | |
|---|---|
| 96 | 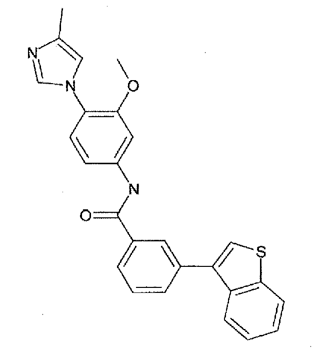 |
| 97 | 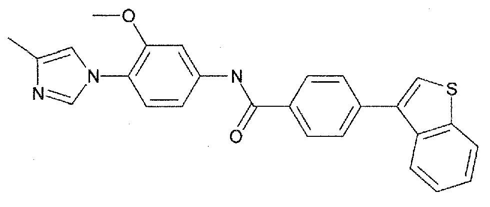 |
| 98 | 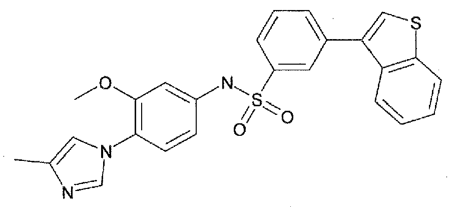 |
| 99 | 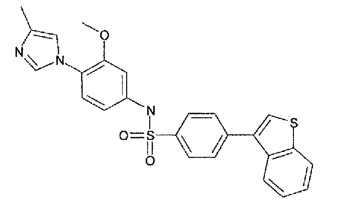 |
| 100 | 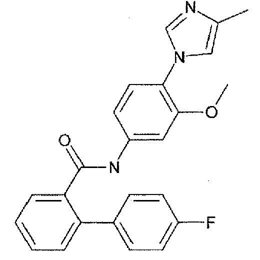 |

**[Table 23]**

| | |
|---|---|
| 101 | 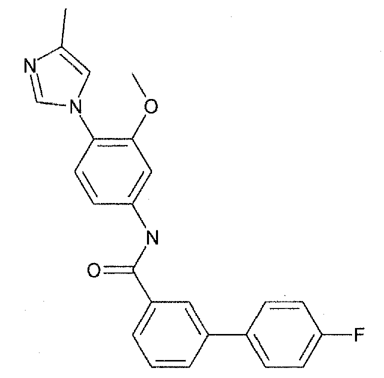 |
| 102 | 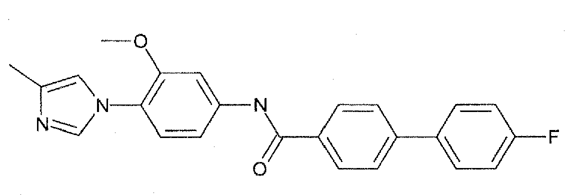 |
| 103 | 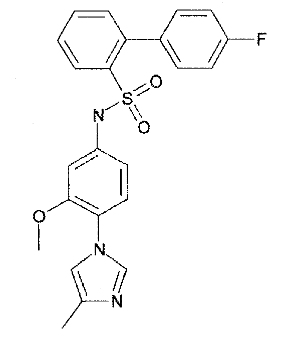 |
| 104 | 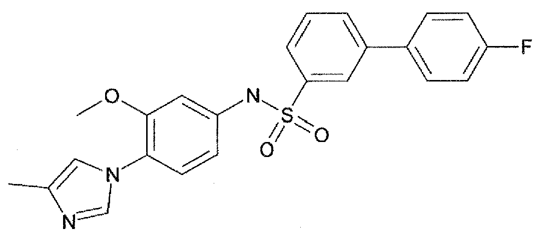 |

**[Table 24]**

| | |
|---|---|
| 105 | 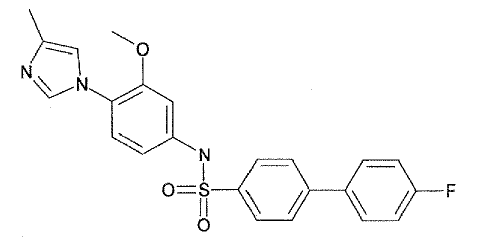 |
| 106 | 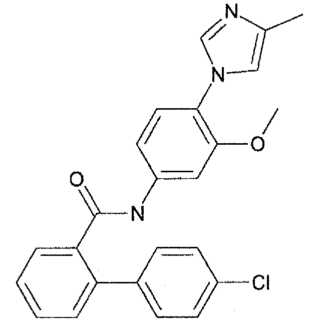 |
| 107 | 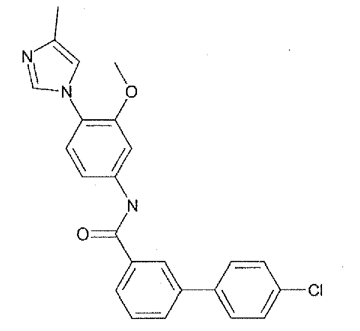 |
| 108 | 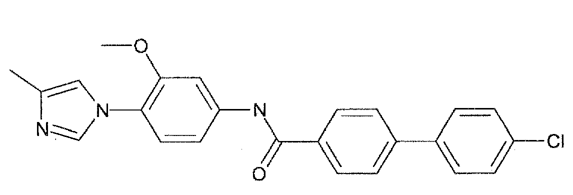 |

**[Table 25]**

| | |
|---|---|
| 109 | 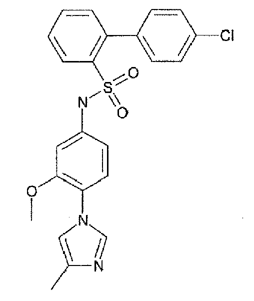 |
| 110 | 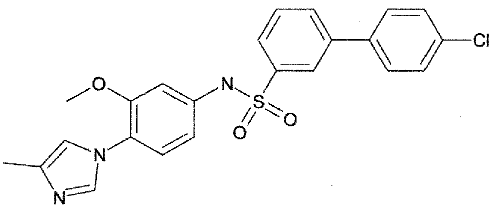 |
| 111 | 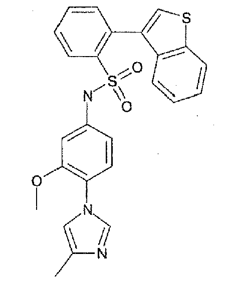 |
| 112 | 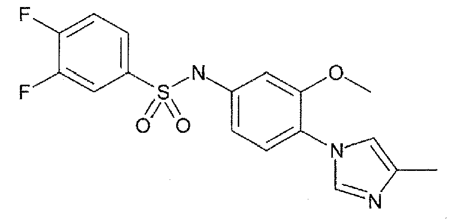 |
| 113 | 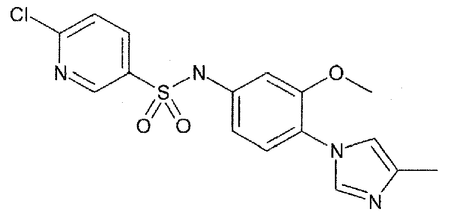 |

**[Table 26]**

| | |
|---|---|
| 114 | 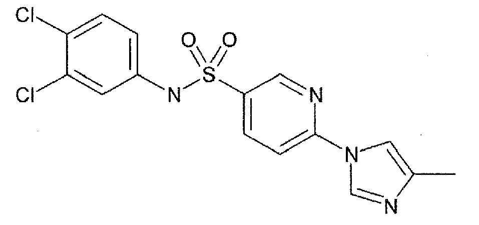 |
| 115 | 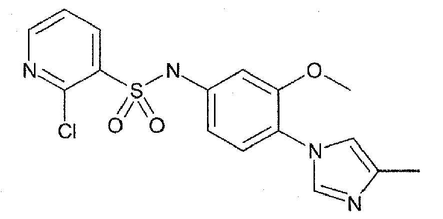 |
| 116 | 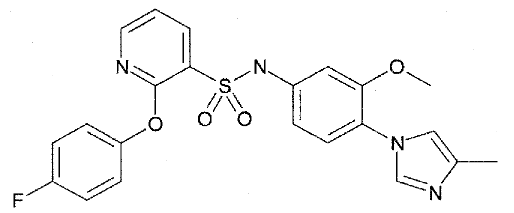 |
| 117 | 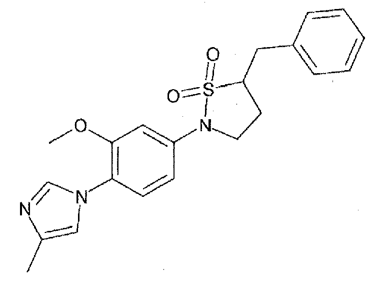 |
| 118 | 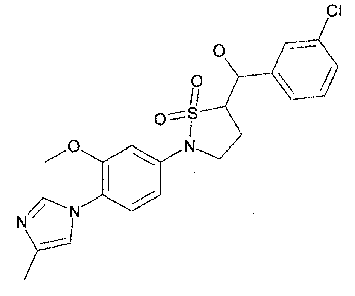 |

**[Table 27]**

| | |
|---|---|
| 119 | 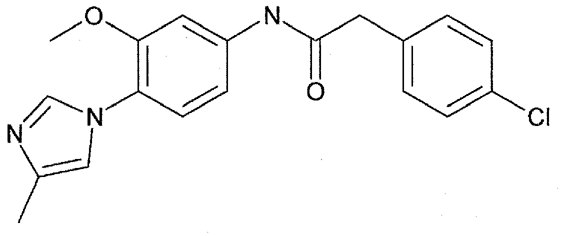 |
| 120 | 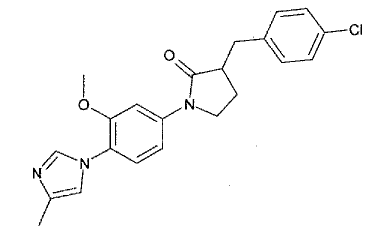 |
| 121 | 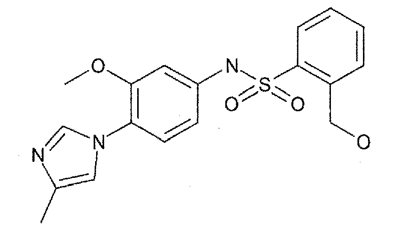 |
| 122 | 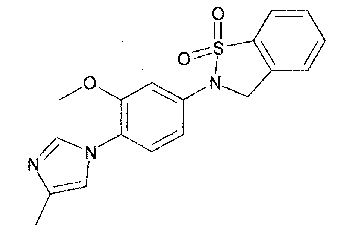 |
| 123 | 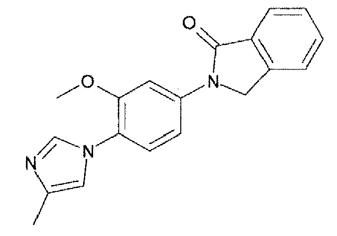 |

**[Table 28]**

| | |
|---|---|
| 124 | 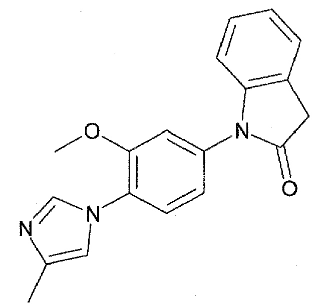 |
| 125 | 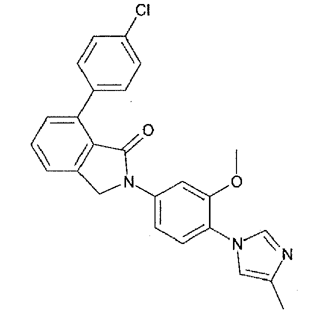 |
| 126 | 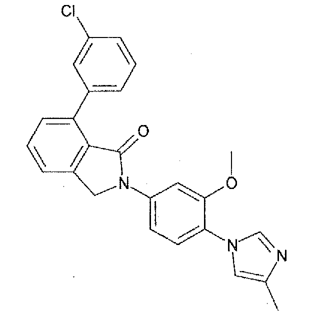 |
| 127 | 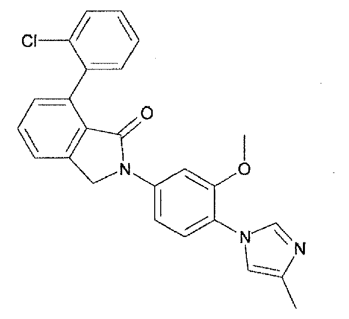 |

**[Table 29]**

| | |
|---|---|
| 128 | 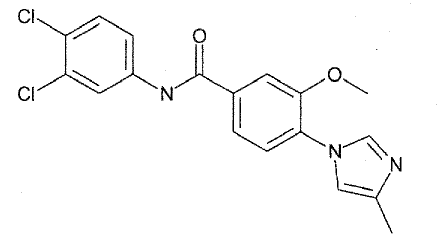 |
| 129 | 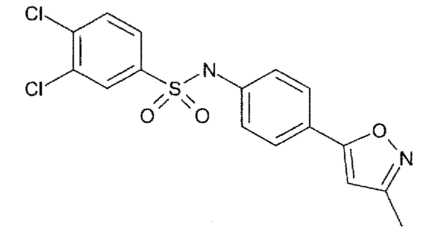 |
| 130 | 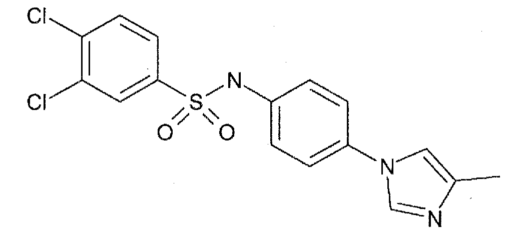 |
| 131 | 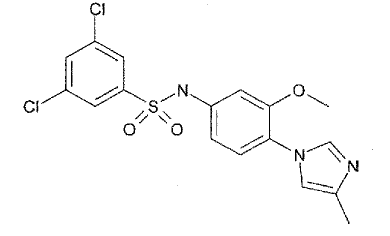 |
| 132 | 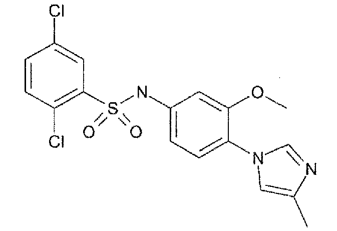 |

**[Table 30]**

| | |
|---|---|
| 133 | 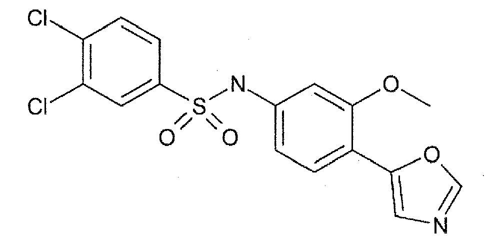 |
| 134 | 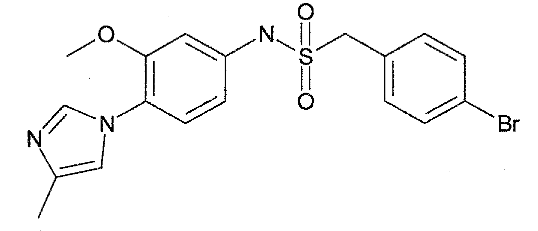 |
| 135 | 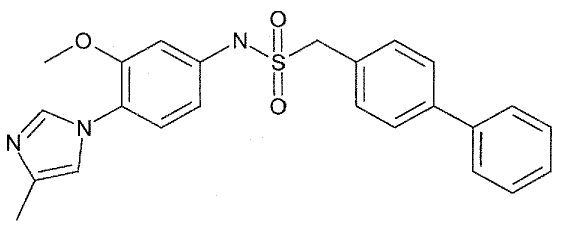 |
| 136 | 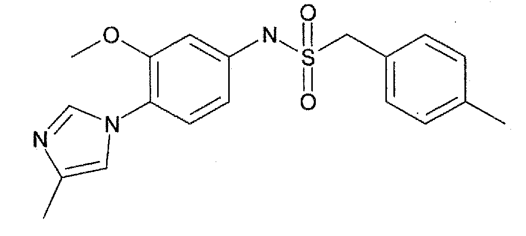 |
| 137 | 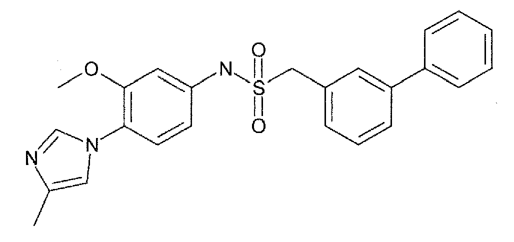 |

**[Table 31]**

| | |
|---|---|
| 138 | 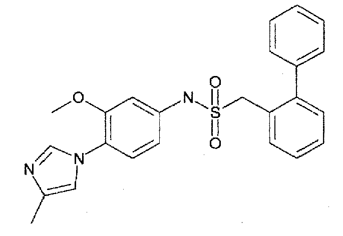 |
| 139 | 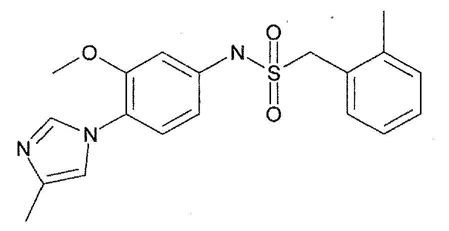 |
| 140 | 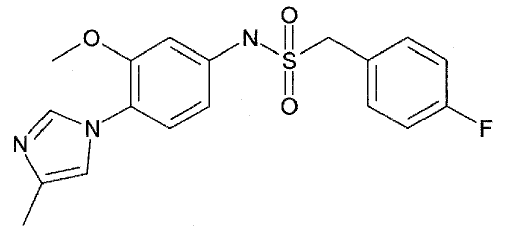 |
| 141 | 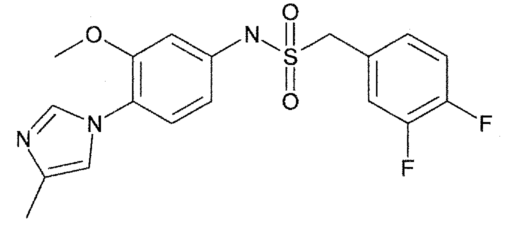 |
| 142 | 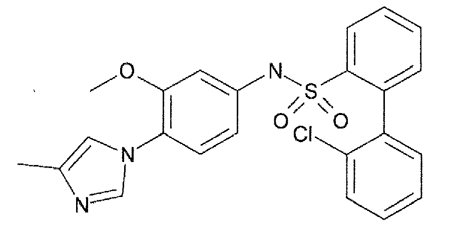 |

**[Table 32]**

| | |
|---|---|
| 143 | 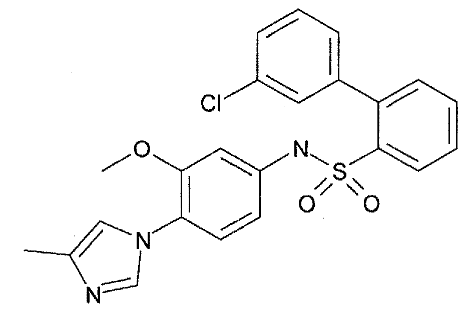 |
| 144 | 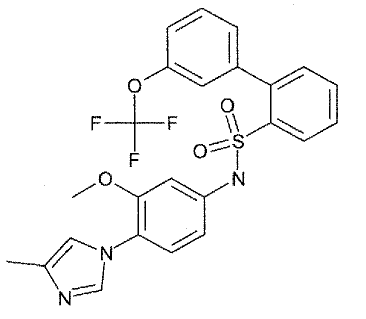 |
| 145 | 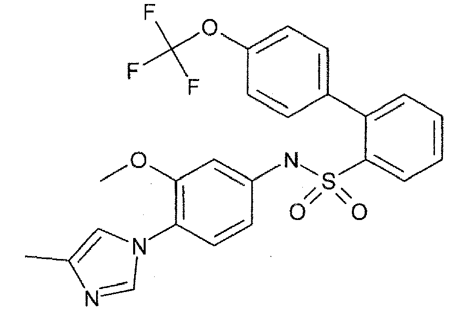 |
| 146 | 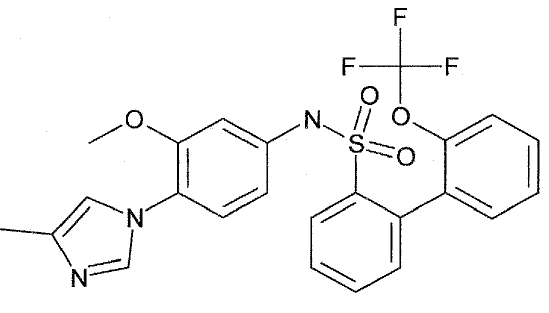 |

**[Table 33]**

| | |
|---|---|
| 147 | 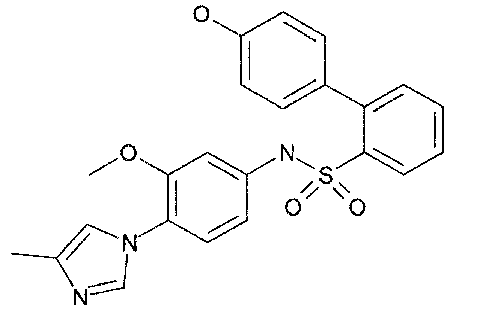 |
| 148 | 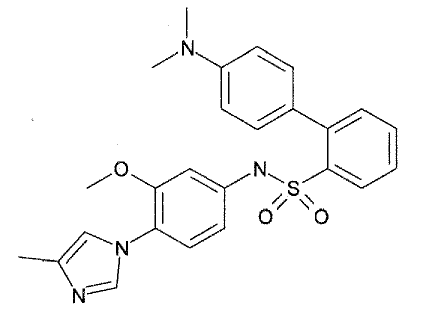 |
| 149 | 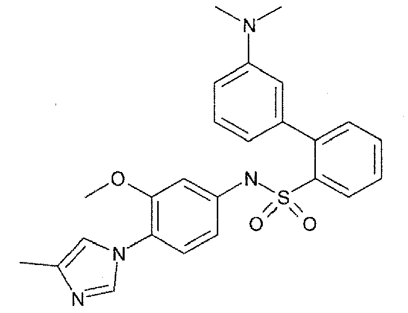 |
| 150 | 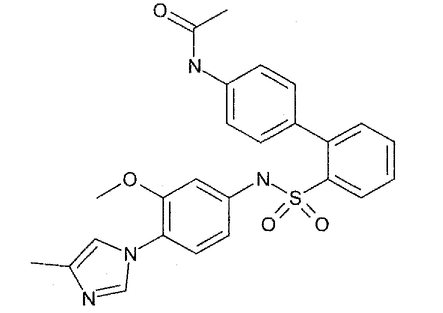 |

**[Table 34]**

| | |
|---|---|
| 151 | 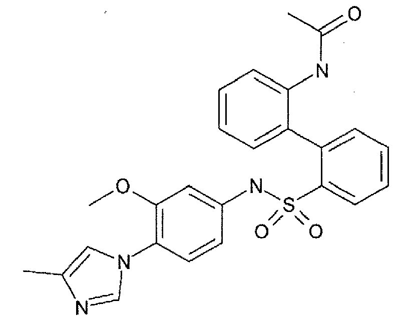 |
| 152 | 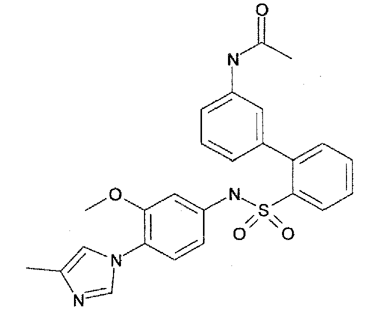 |
| 153 | 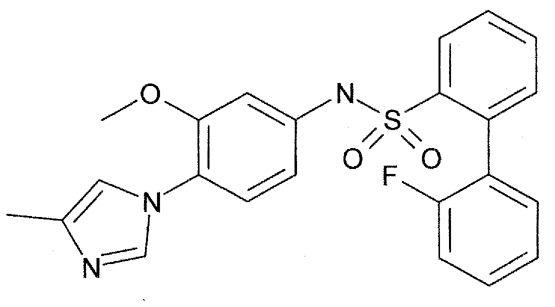 |
| 154 | 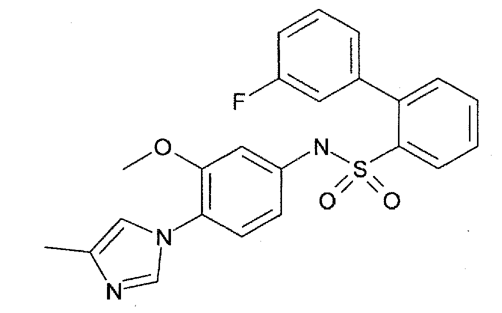 |

**[Table 35]**

| | |
|---|---|
| 155 | 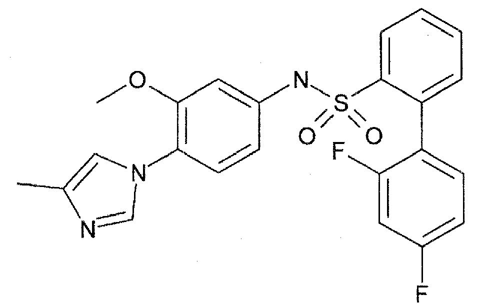 |
| 156 | 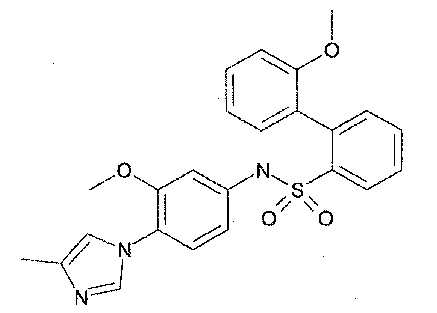 |
| 157 | 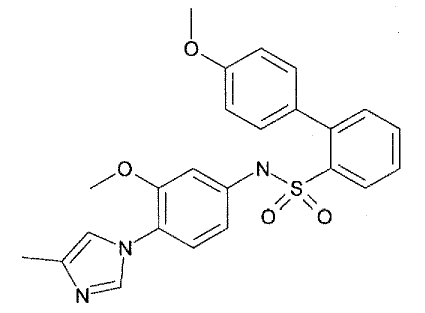 |
| 158 | 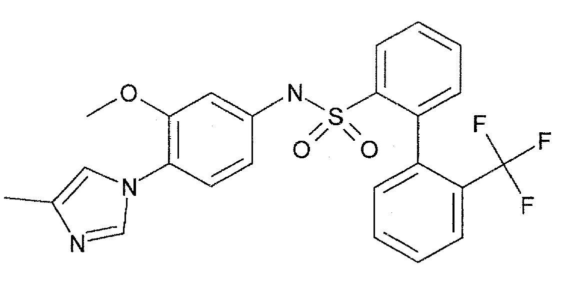 |

**[Table 36]**

| | |
|---|---|
| 159 | 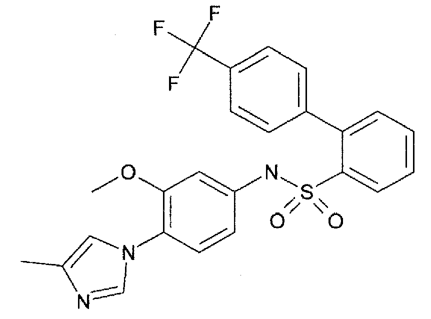 |
| 160 | 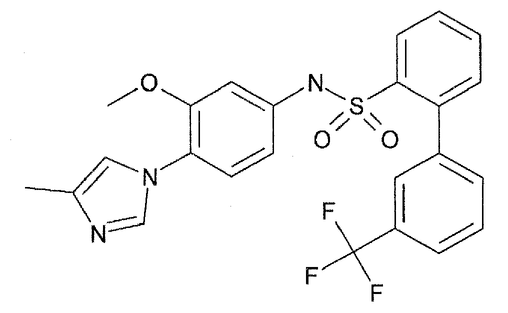 |
| 161 | 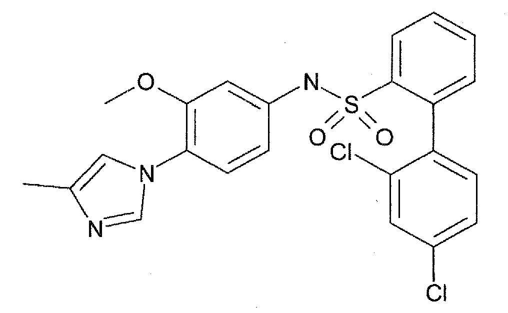 |
| 162 | 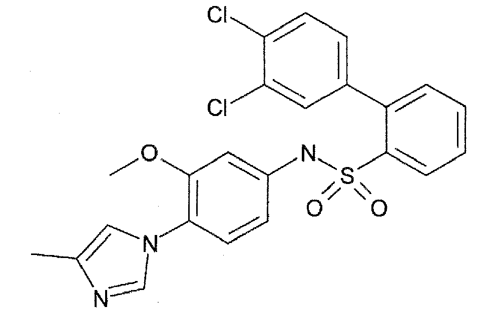 |

**[Table 37]**

| | |
|---|---|
| 163 | 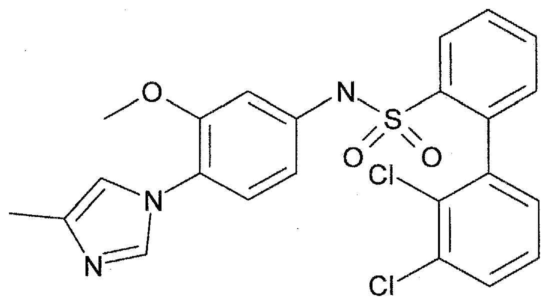 |
| 164 | 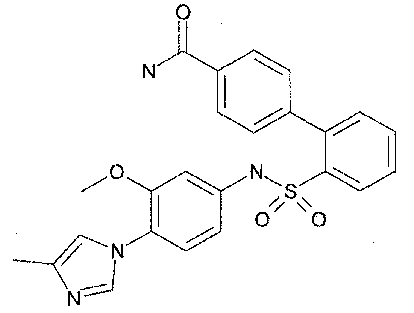 |
| 165 | 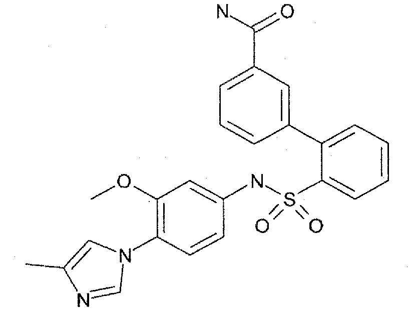 |
| 166 | 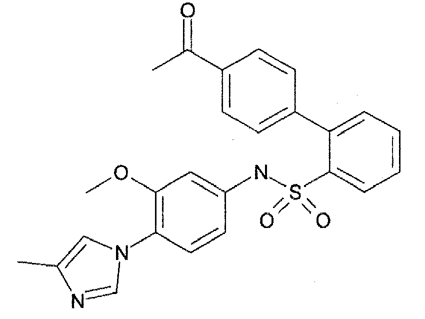 |

**[Table 38]**

| | |
|---|---|
| 167 | 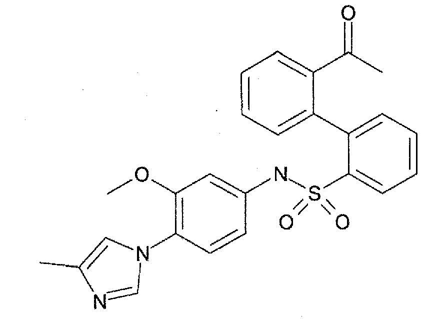 |
| 168 | 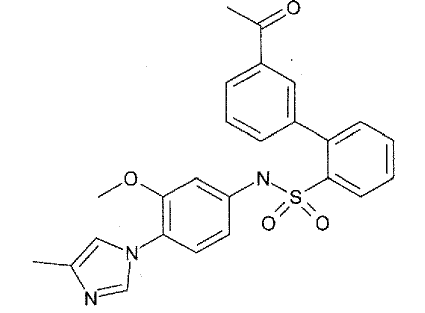 |
| 169 | 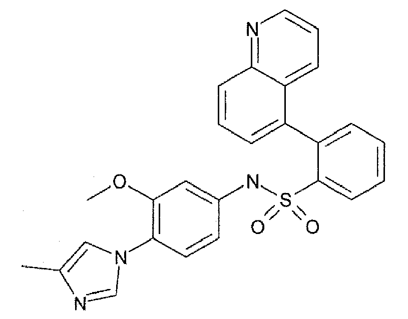 |
| 170 | 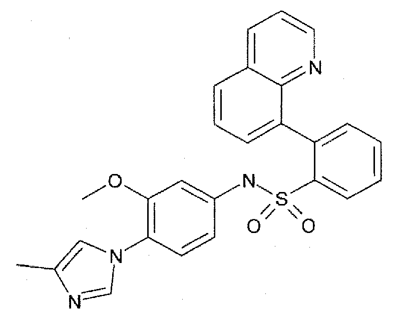 |

**[Table 39]**

| | |
|---|---|
| 171 | 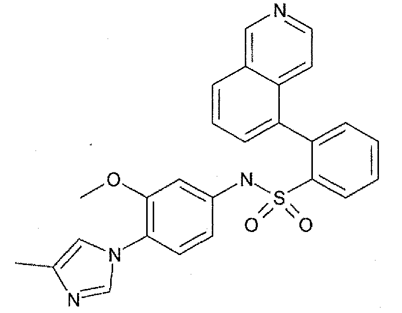 |
| 172 | 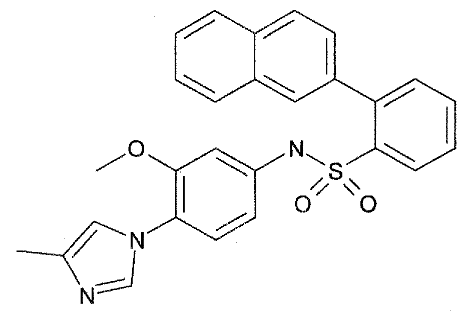 |
| 173 | 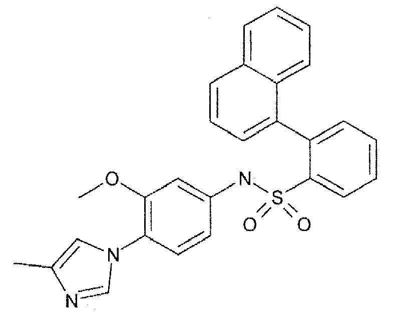 |
| 174 | 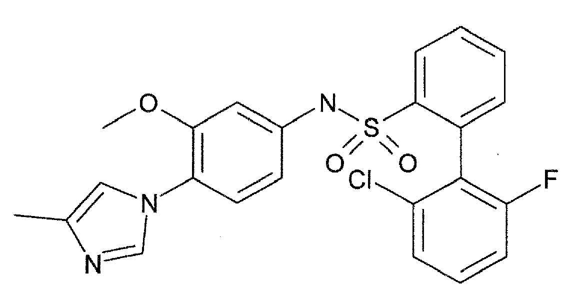 |

**[Table 40]**

| | |
|---|---|
| 175 | 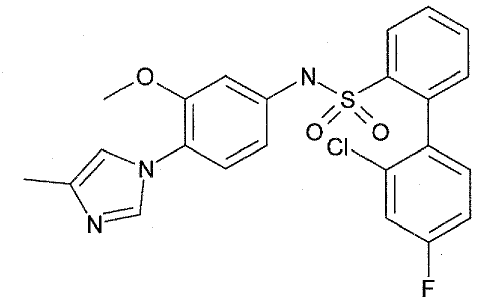 |
| 176 | 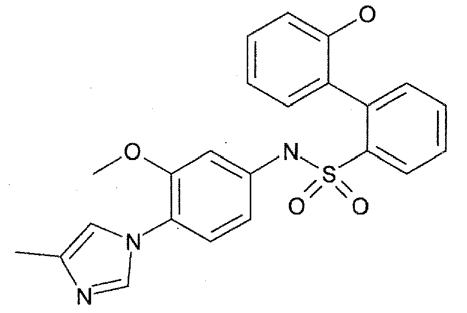 |
| 177 | 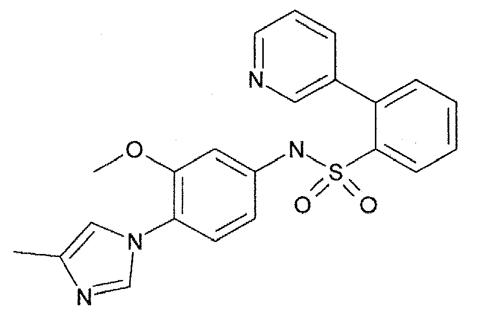 |
| 178 | 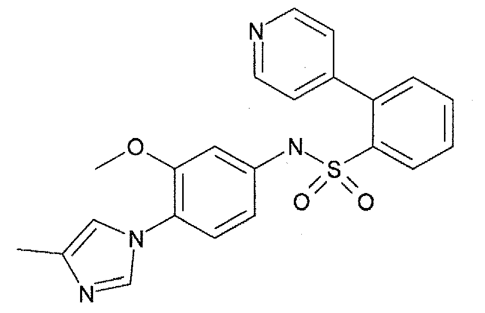 |

**[Table 41]**

| | |
|---|---|
| 179 | 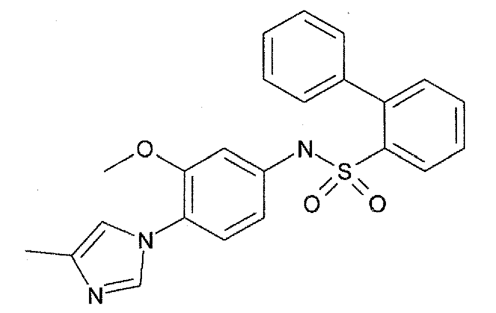 |
| 180 | 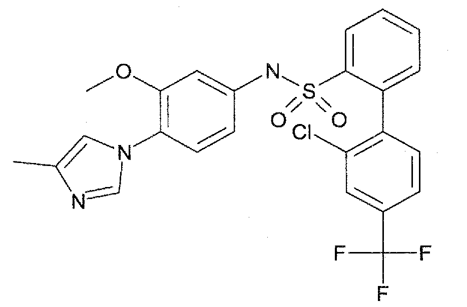 |
| 181 | 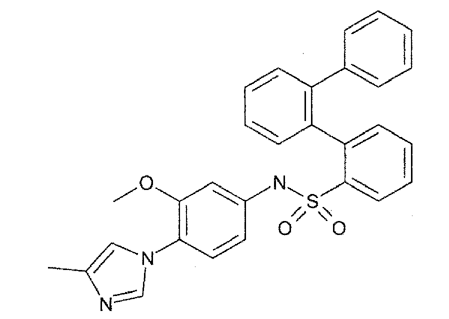 |
| 182 | 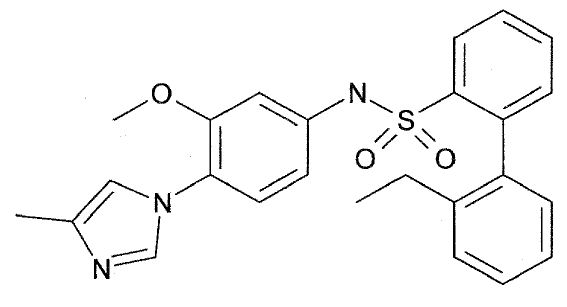 |

**[Table 42]**

| | |
|---|---|
| 183 | 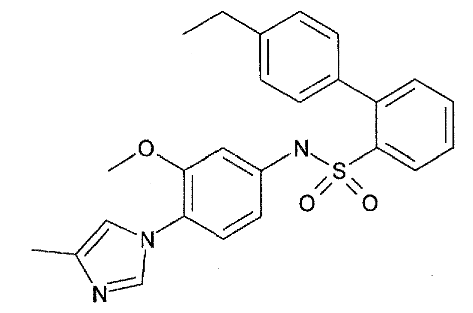 |
| 184 | 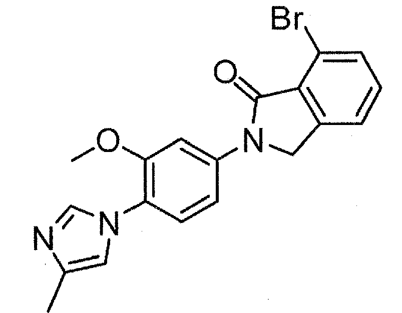 |
| 185 | 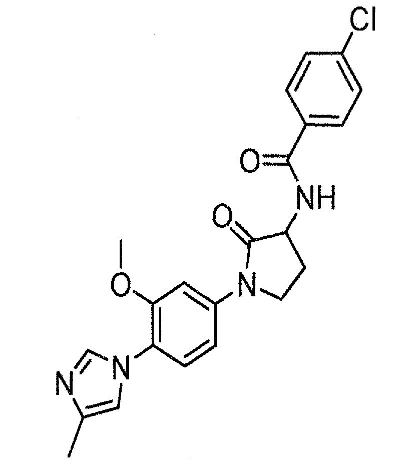 |

**[Table 43]**

| | |
|---|---|
| 186 | 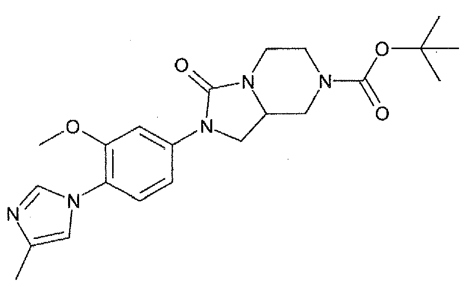 |
| 187 | 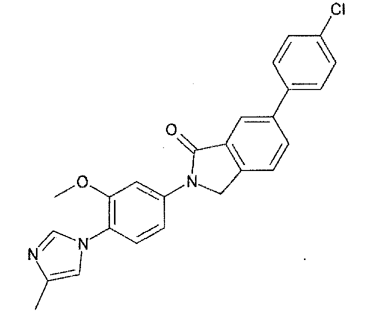 |
| 188 | 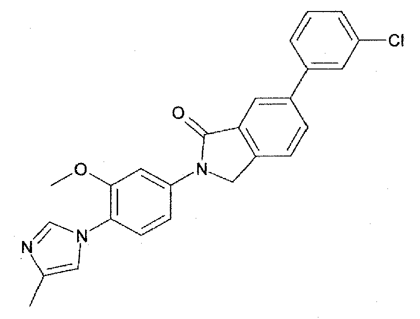 |
| 189 | 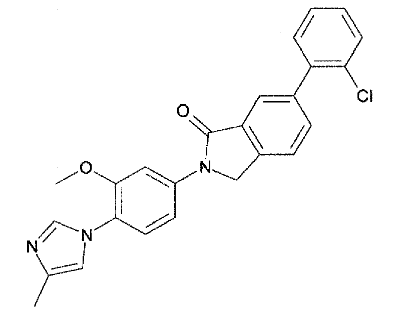 |

**[Table 44]**

| | |
|---|---|
| 190 | 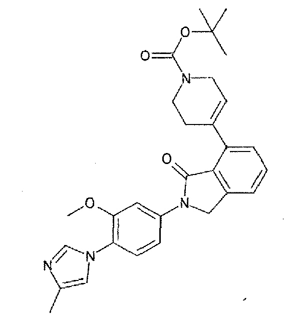 |
| 191 | 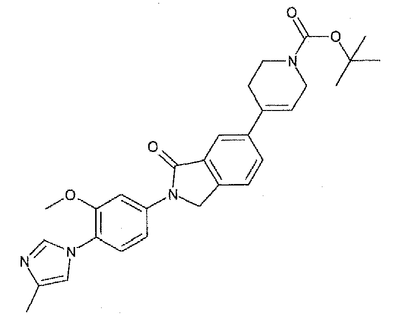 |
| 192 | 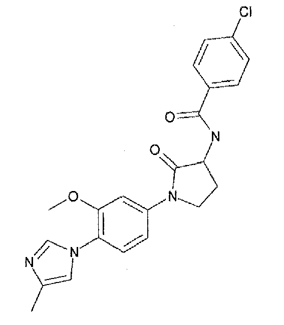 |
| 193 | 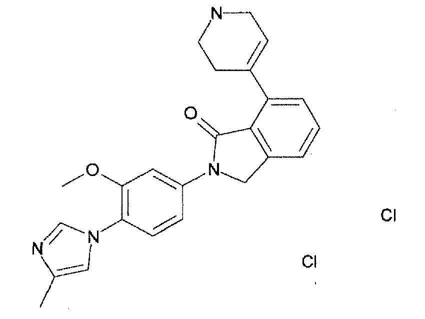 |

**[Table 45]**

| | |
|---|---|
| 194 | 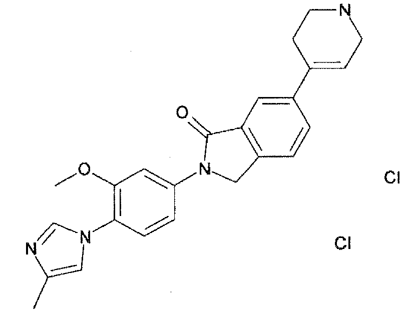 |
| 195 | 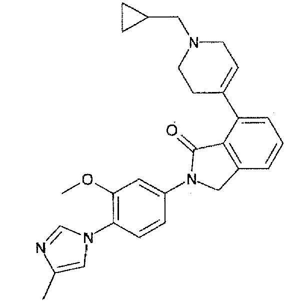 |
| 196 | 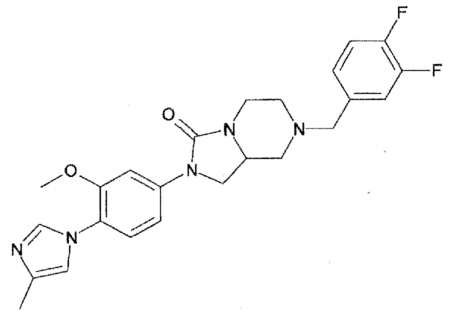 |
| 197 | 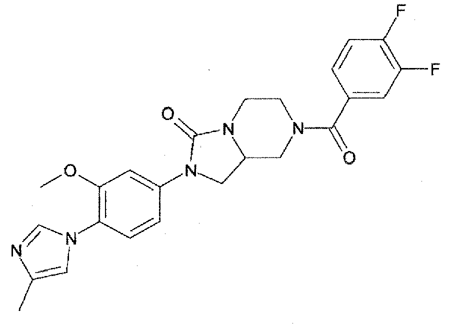 |

**[Table 46]**

| | |
|---|---|
| 198 | 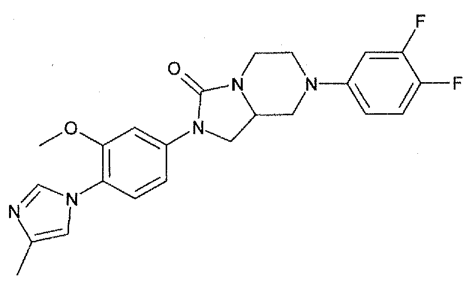 |
| 199 | 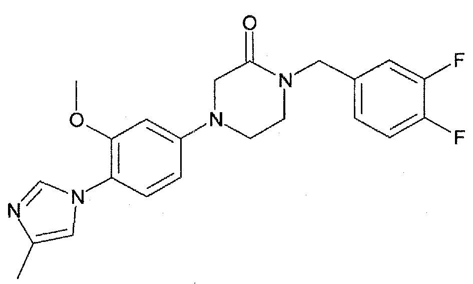 |
| 200 | 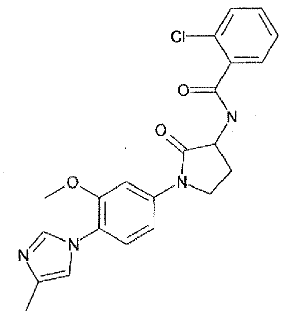 |
| 201 | 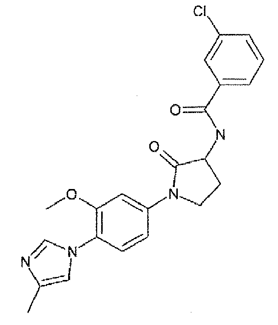 |

**[Table 47]**

| | |
|---|---|
| 202 | 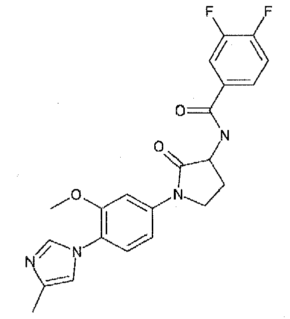 |
| 203 | 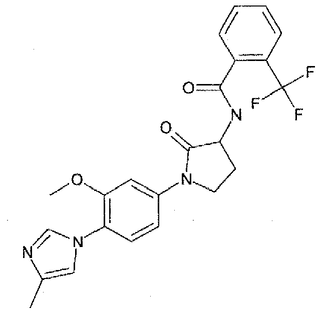 |
| 204 | 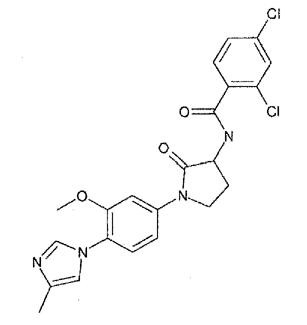 |
| 205 | 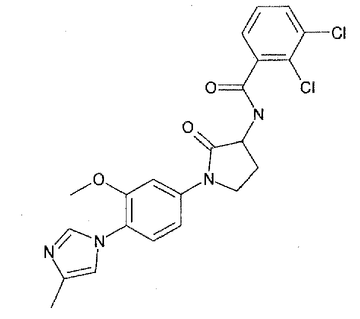 |

**[Table 48]**

| | |
|---|---|
| 206 | 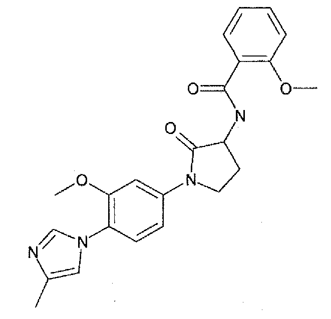 |
| 207 | 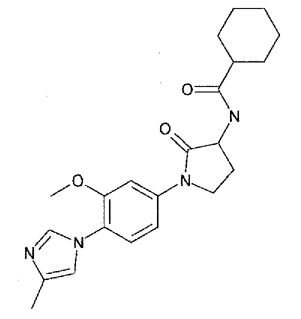 |
| 208 | 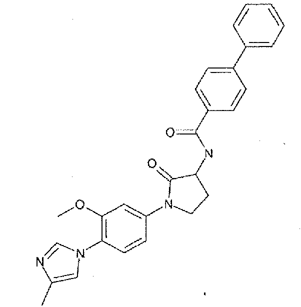 |
| 209 | 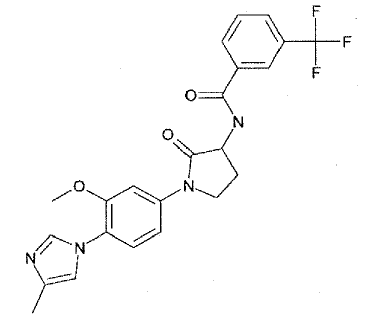 |

**[Table 49]**

| | |
|---|---|
| 210 | 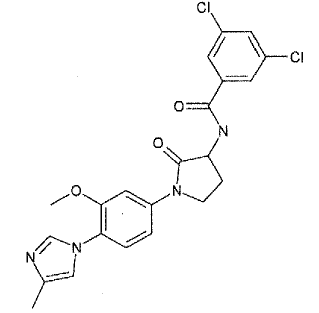 |
| 211 | 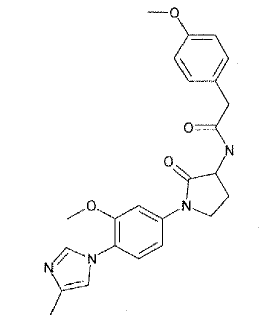 |
| 212 | 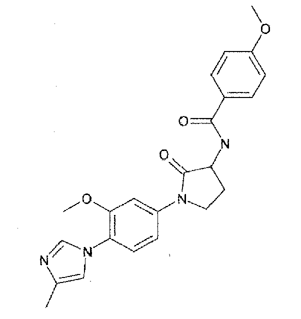 |
| 213 | 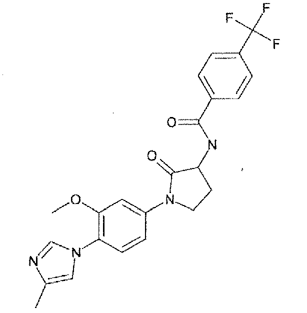 |

**[Table 50]**

| | |
|---|---|
| 214 | 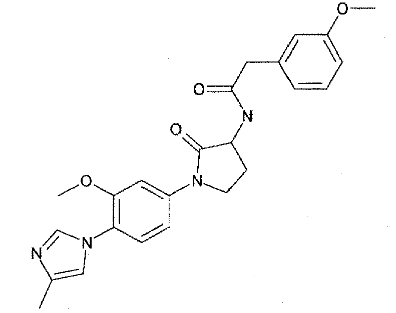 |
| 215 | 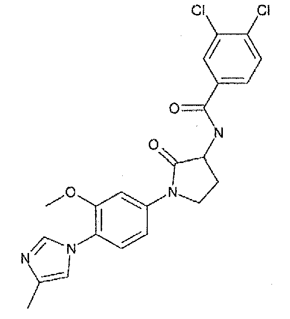 |
| 216 | 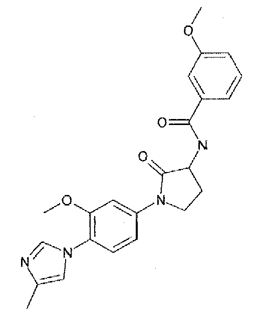 |
| 217 | 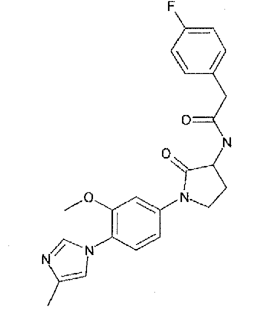 |

**[Table 51]**

| | |
|---|---|
| 218 | 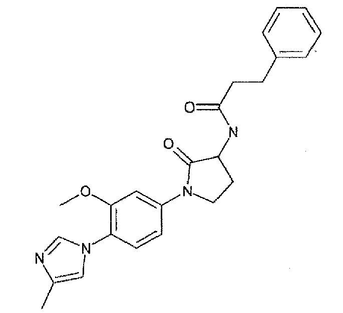 |
| 219 | 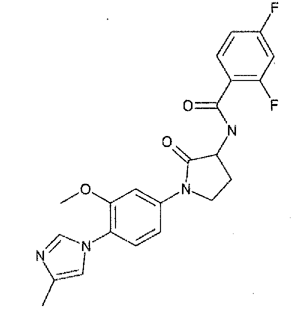 |
| 220 | 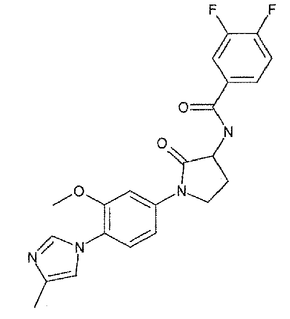 |
| 221 | 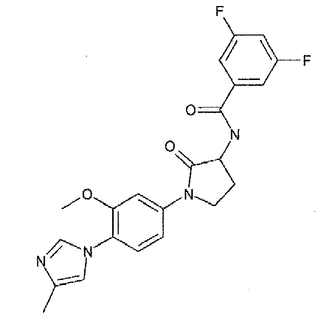 |

**[Table 52]**

| | |
|---|---|
| 222 | 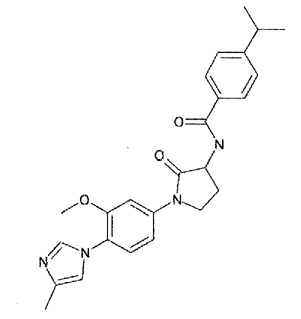 |
| 223 | 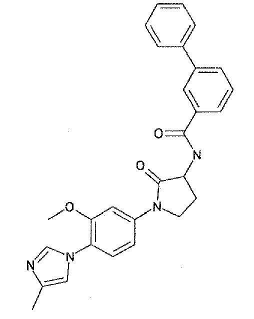 |
| 224 | 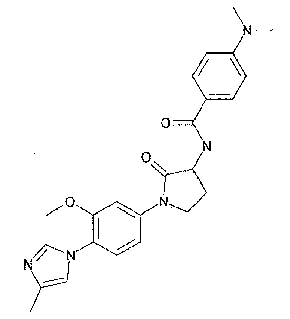 |
| 225 | 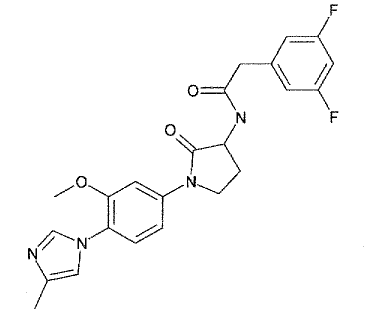 |

**[Table 53]**

| | |
|---|---|
| 226 | 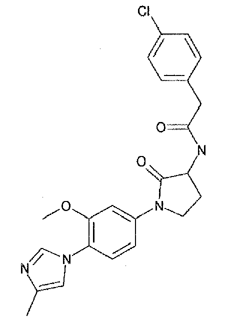 |
| 227 | 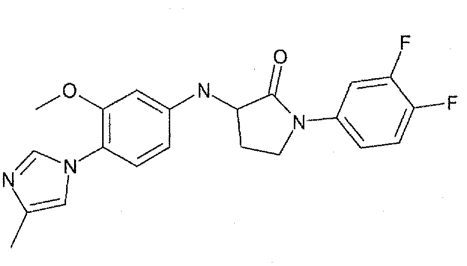 |
| 228 | 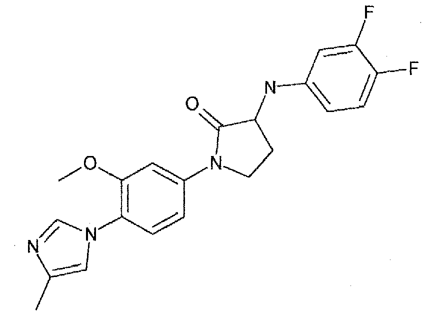 |
| 229 | 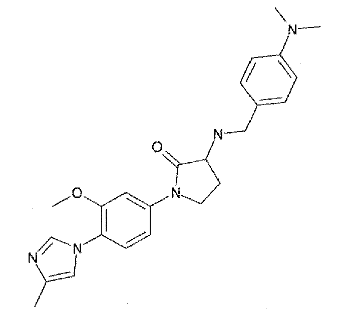 |

**[Table 54]**

| | |
|---|---|
| 230 | 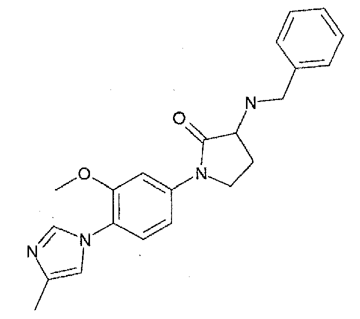 |
| 231 | 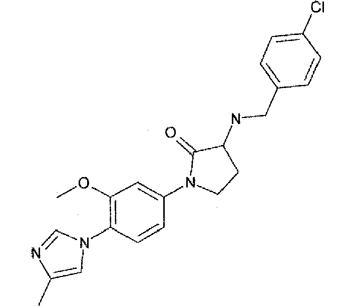 |
| 232 | 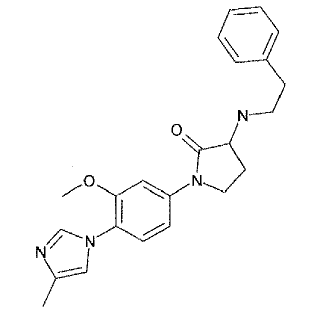 |
| 233 | 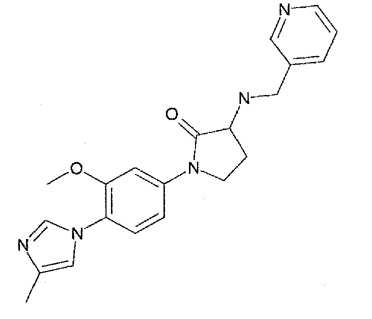 |

**[Table 55]**

| | |
|---|---|
| 234 | 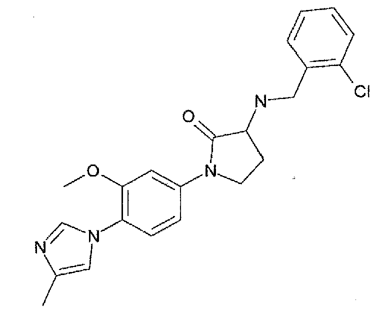 |
| 235 | 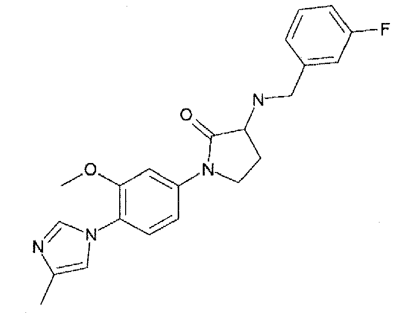 |
| 236 | 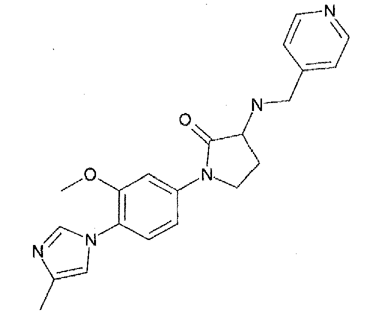 |
| 237 | 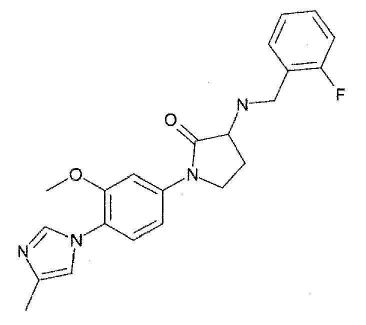 |

**[Table 56]**

| | |
|---|---|
| 238 | 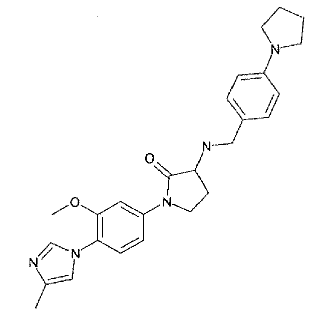 |
| 239 | 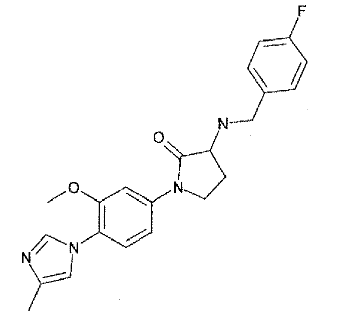 |
| 240 | 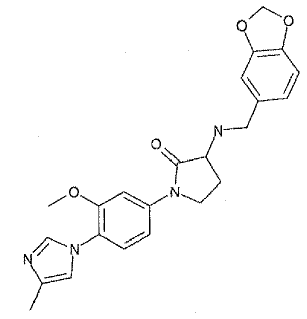 |
| 241 | 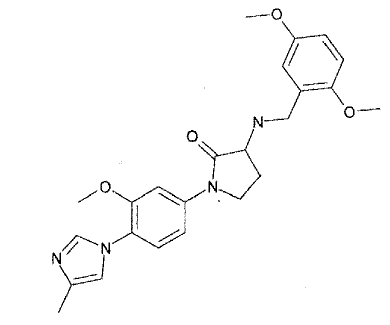 |

**[Table 57]**

| | |
|---|---|
| 242 | 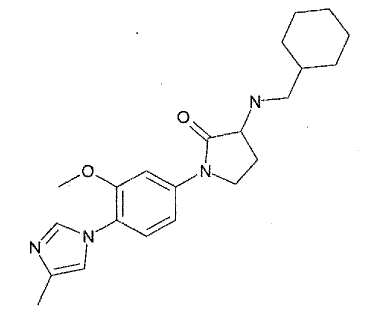 |
| 243 | 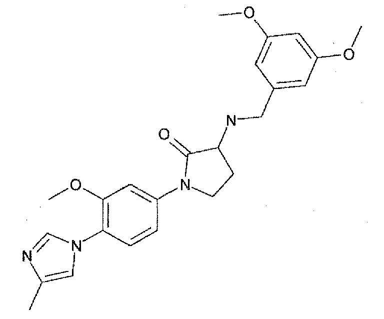 |
| 244 | 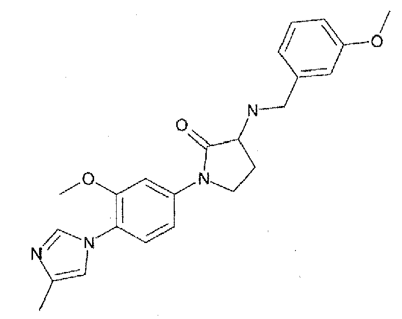 |
| 245 | 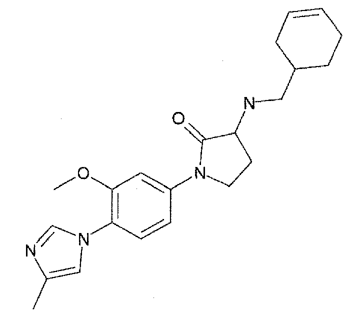 |

**[Table 58]**

| | |
|---|---|
| 246 | 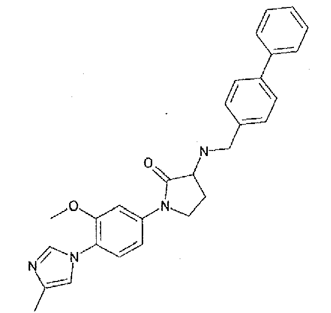 |
| 247 | 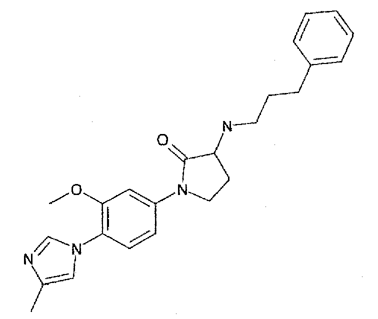 |
| 248 | 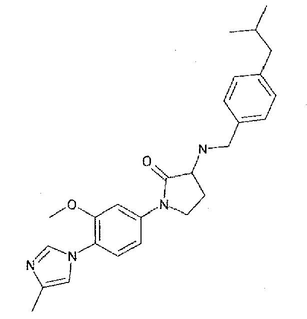 |
| 249 | 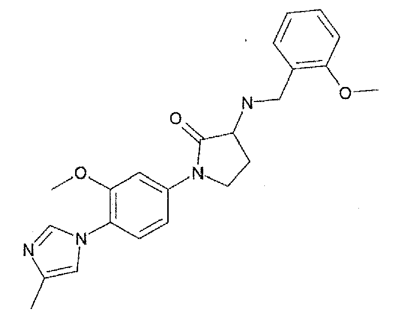 |

**[Table 59]**

| | |
|---|---|
| 250 | 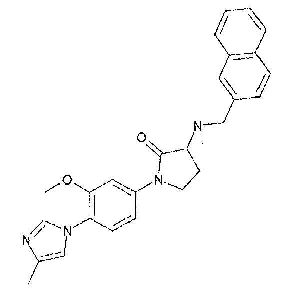 |
| 251 | 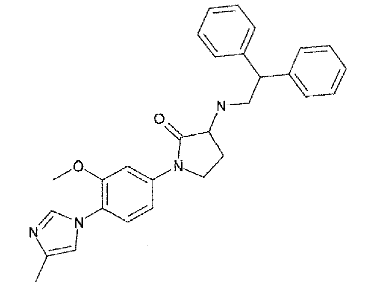 |
| 252 | 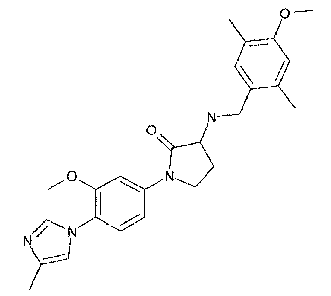 |
| 253 | 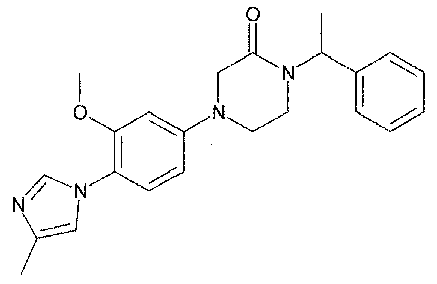 |
| 254 | canceled |

**[Table 60]**

| | |
|---|---|
| 255 | 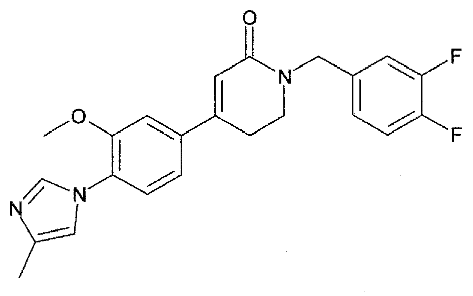 |
| 256 | 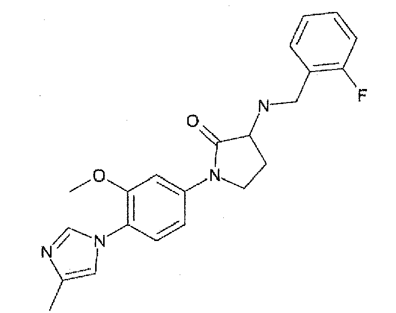 |
| 257 | 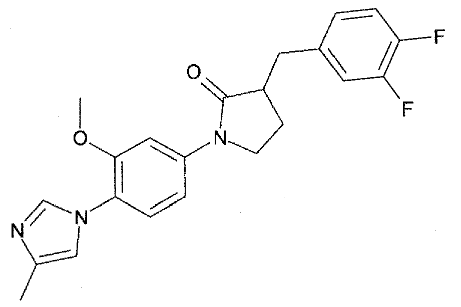 |
| 258 | 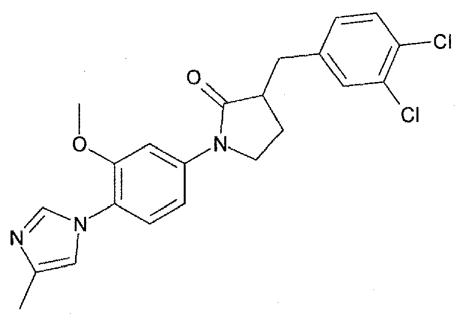 |

**[Table 61]**

| | |
|---|---|
| 259 | 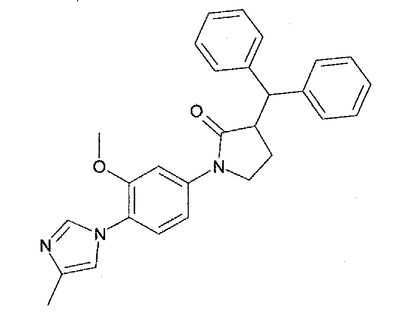 |
| 260 | 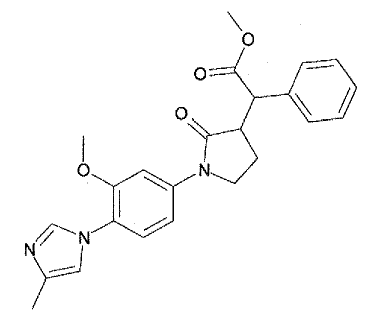 |
| 261 | 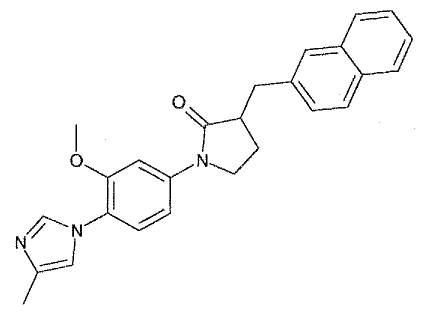 |
| 262 | 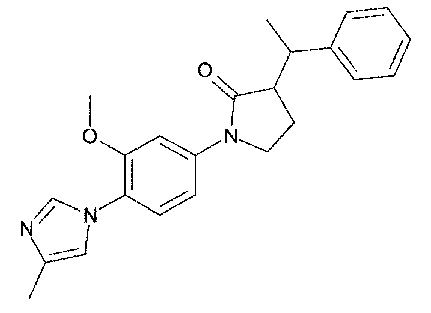 |

**[Table 62]**

| | |
|---|---|
| 263 | 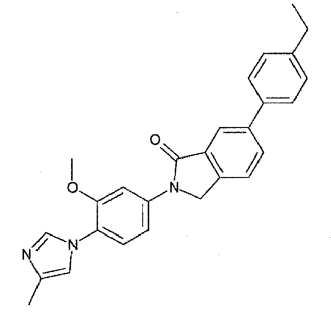 |
| 264 | 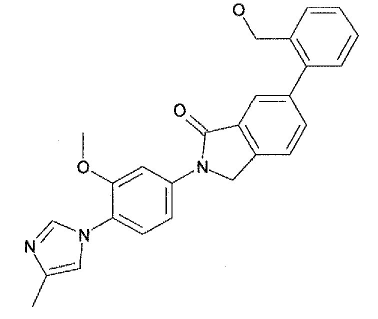 |
| 265 | 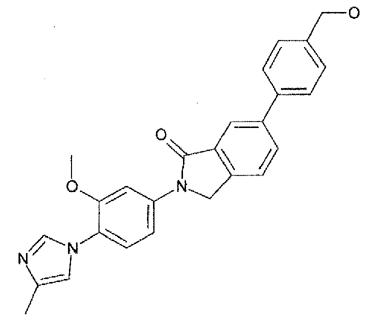 |
| 266 | 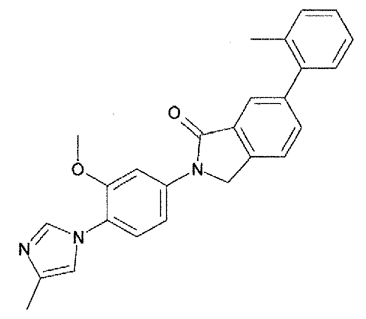 |

**[Table 63]**

| | |
|---|---|
| 267 | 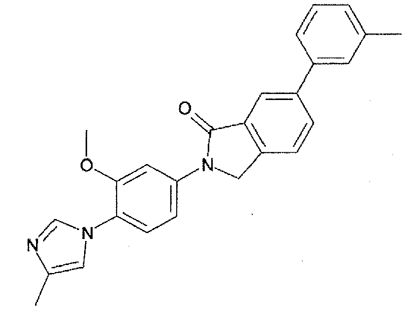 |
| 268 | 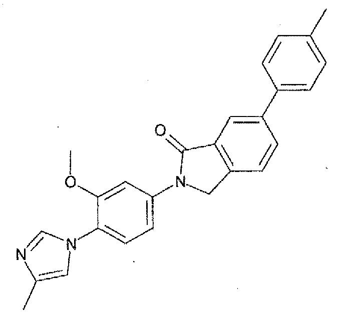 |
| 269 | 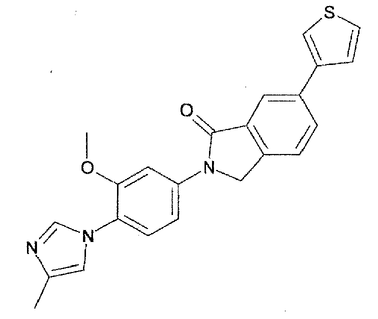 |
| 270 | 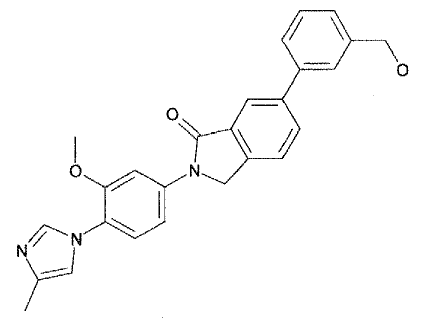 |

**[Table 64]**

| | |
|---|---|
| 271 | 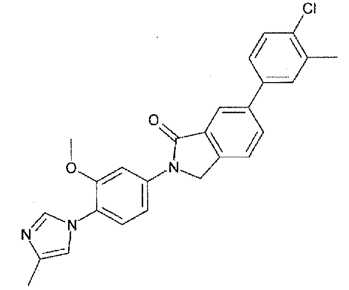 |
| 272 | 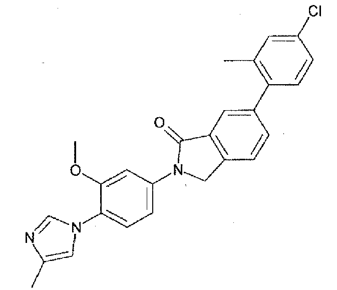 |
| 273 | 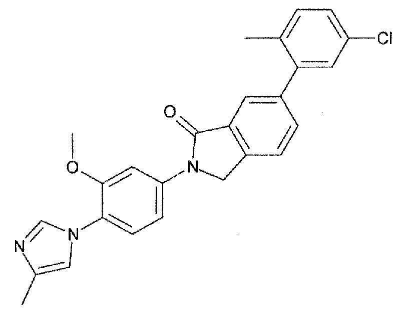 |
| 274 | 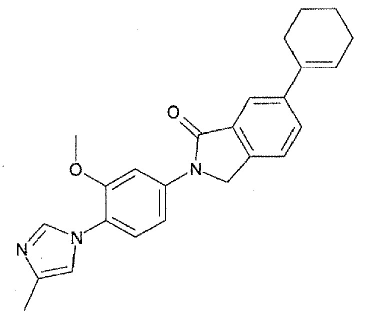 |

**[Table 65]**

| | |
|---|---|
| 275 | 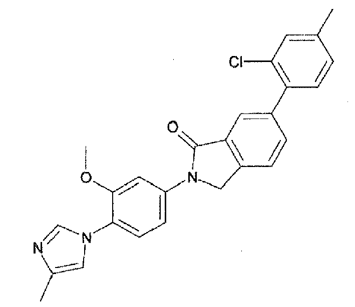 |
| 276 | 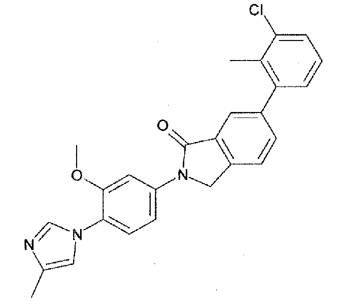 |
| 277 | 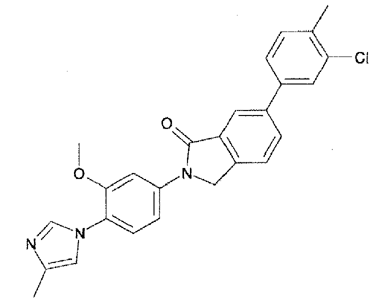 |
| 278 | 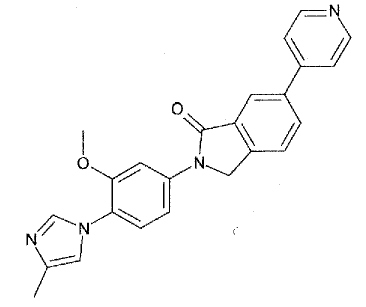 |

**[Table 66]**

| | |
|---|---|
| 279 | 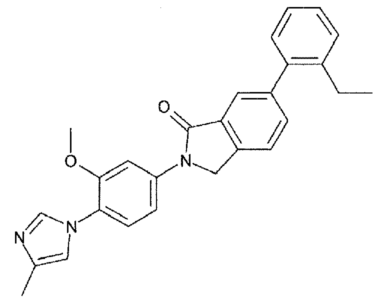 |
| 280 | 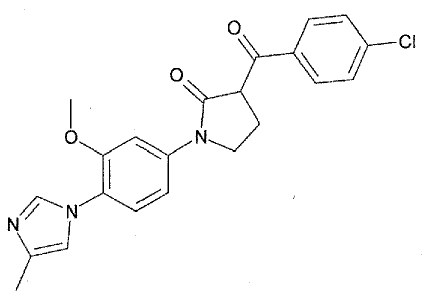 |
| 281 | 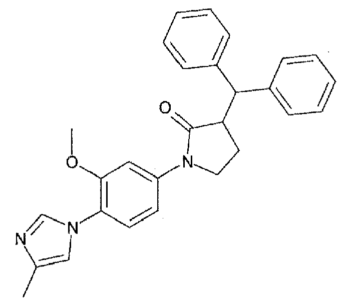 |
| 282 | 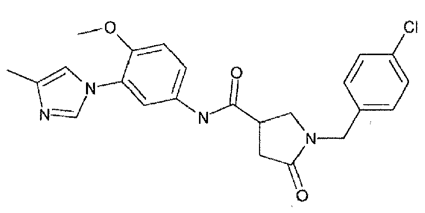 |

**[Table 67]**

| | |
|---|---|
| 283 | 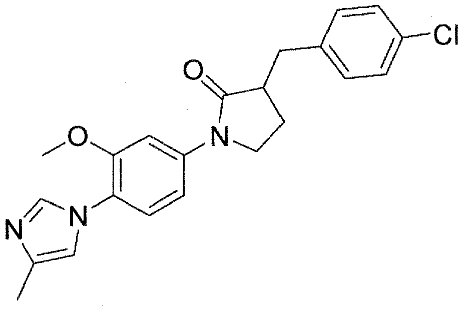 |

**[Table 68]**

| | |
|---|---|
| E1 | 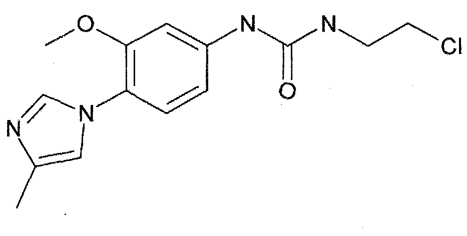 |
| E2 | 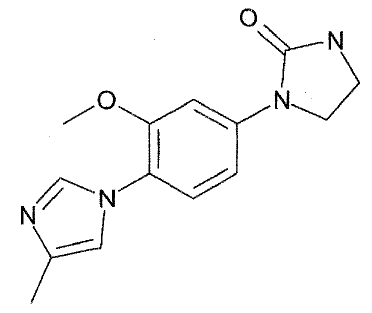 |
| E3 | 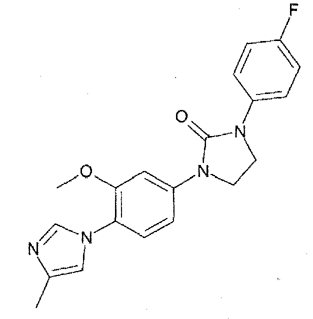 |
| E4 | 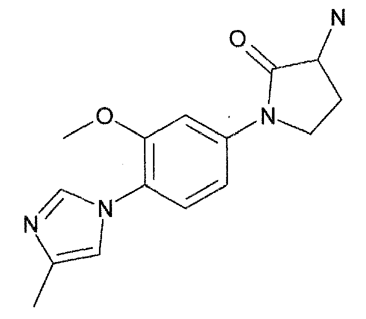 |

**[Table 69]**

| | |
|---|---|
| E5 | 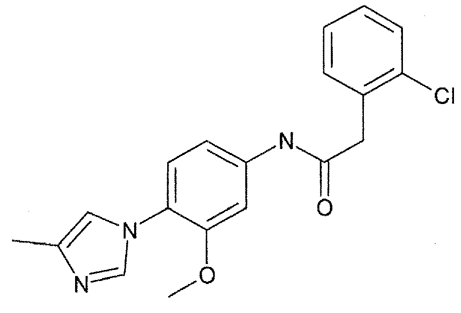 |
| E6 | 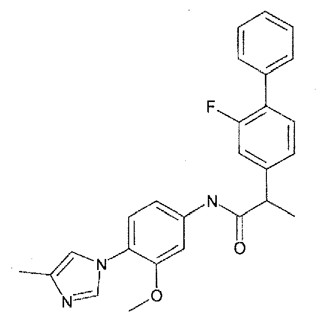 |
| E7 | 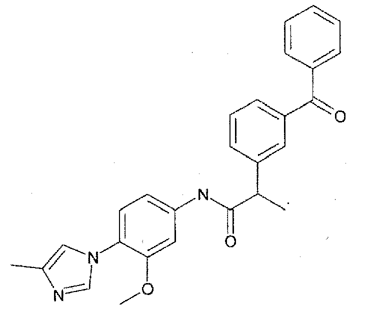 |
| E8 | 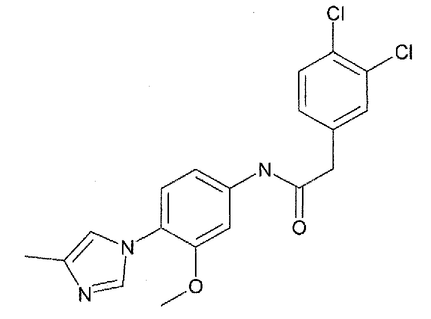 |

**[Table 70]**

| | |
|---|---|
| E9 | 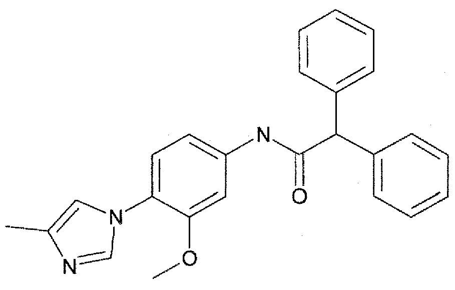 |
| E10 | 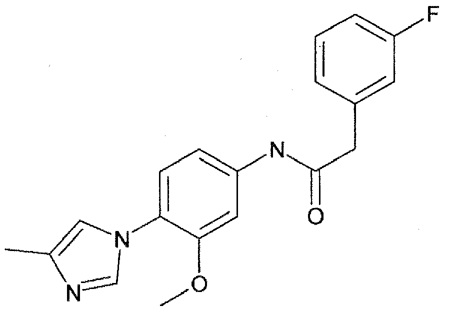 |
| E11 | 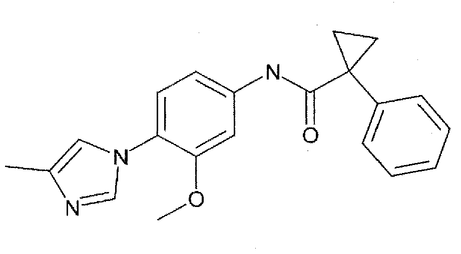 |
| E12 | 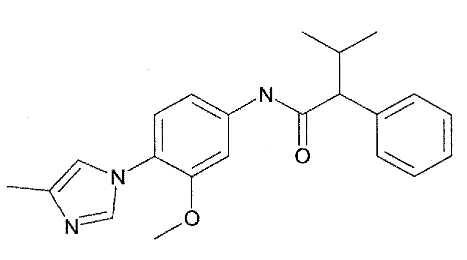 |

**[Table 71]**

| | |
|---|---|
| E13 | 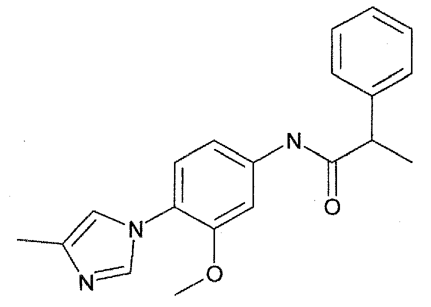 |
| E14 | 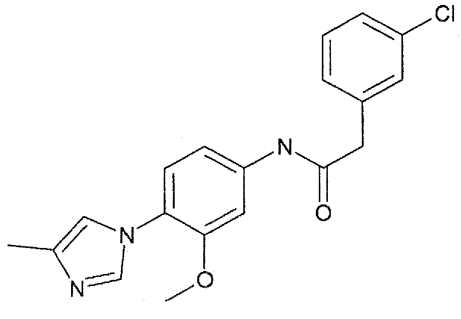 |
| E15 | 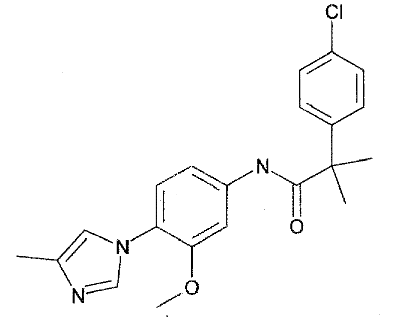 |
| E16 | 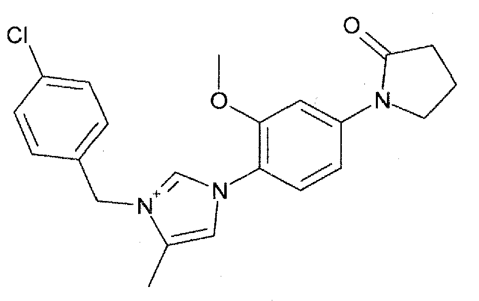 |

**[Table 72]**

| | |
|---|---|
| E17 | 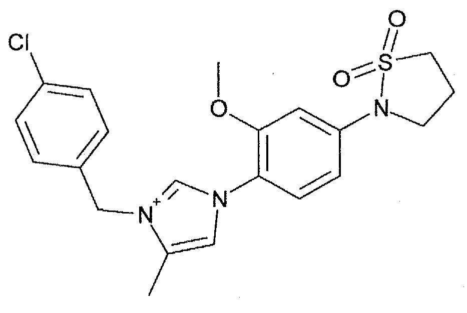 |
| E18 | 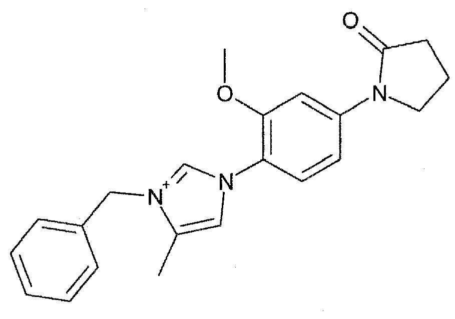 |
| E19 | 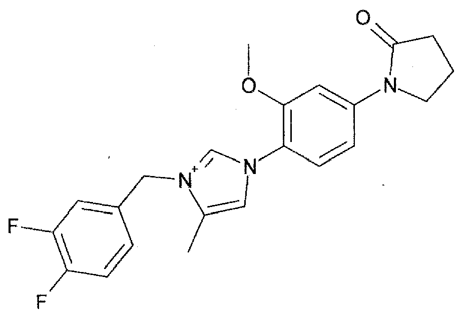 |
| E20 | 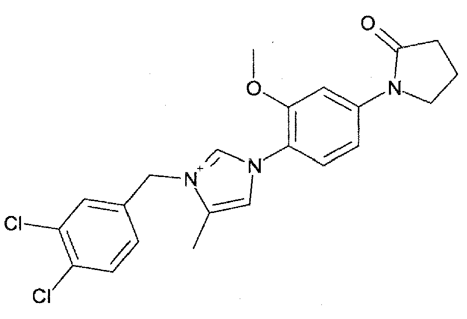 |

**[Table 73]**

| | |
|---|---|
| E21 | 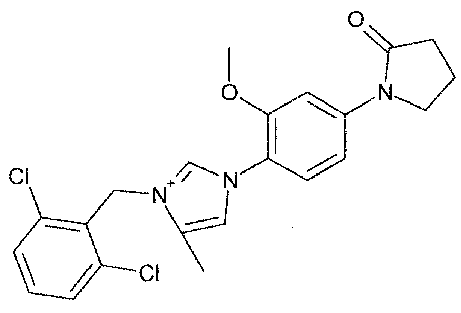 |
| E22 | 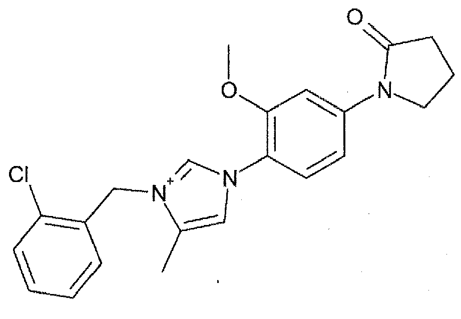 |
| E23 | 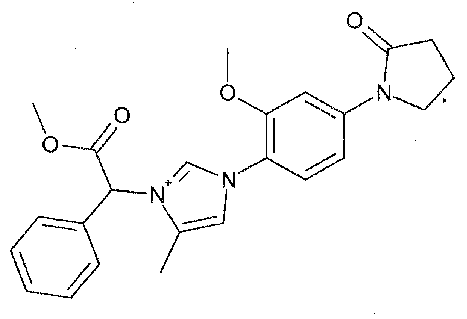 |
| E24 | 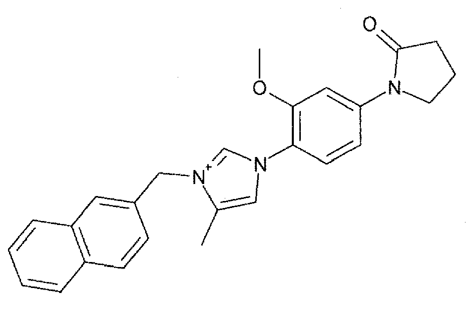 |

**[Table 74]**

| | |
|---|---|
| E25 | 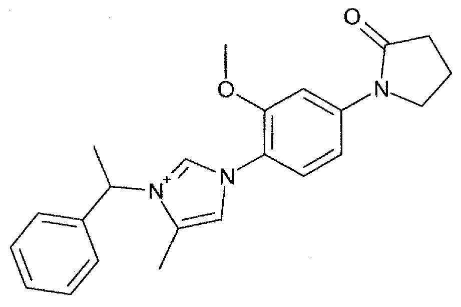 |
| E26 | 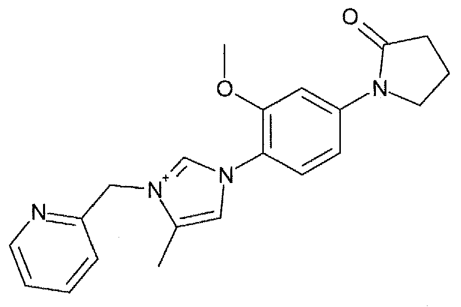 |
| E27 | 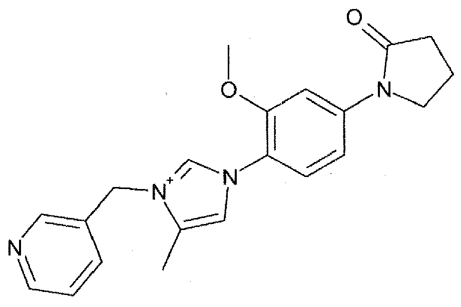 |
| E28 | 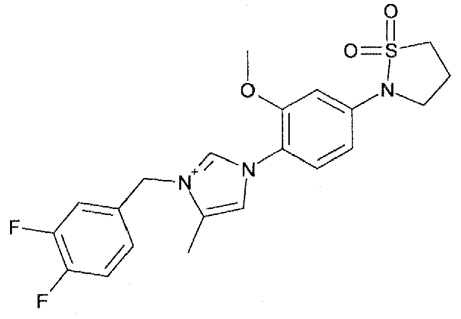 |

**[Table 75]**

| | |
|---|---|
| E29 | 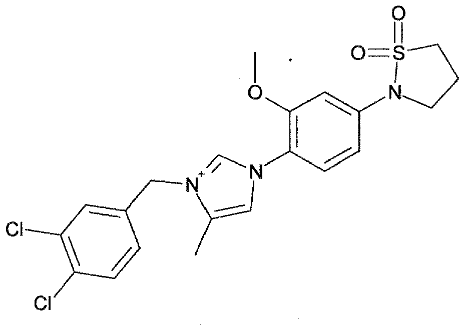 |
| E30 | 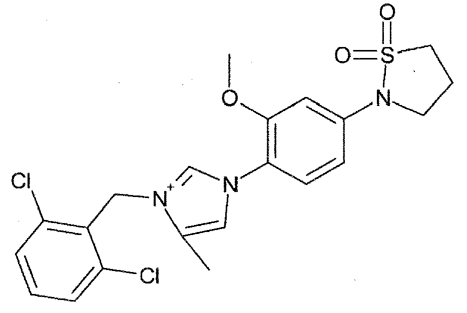 |
| E31 | 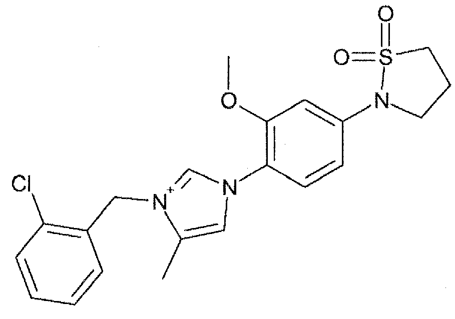 |
| E32 | 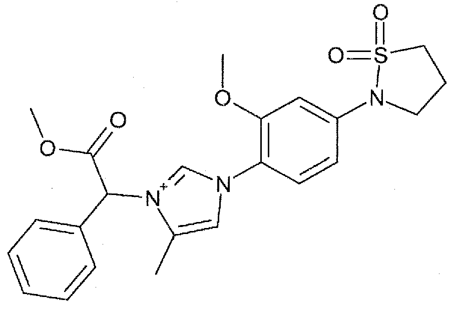 |

**[Table 76]**

| | |
|---|---|
| E33 | 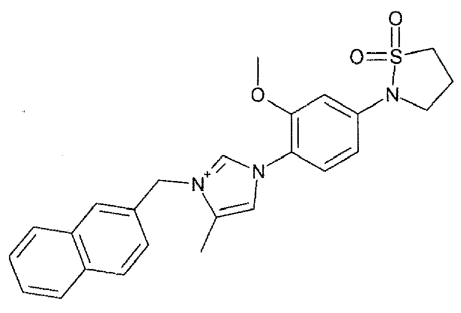 |
| E34 | 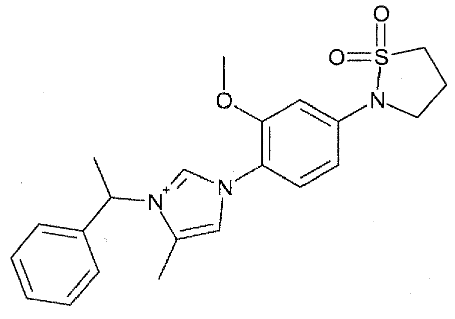 |
| E35 | 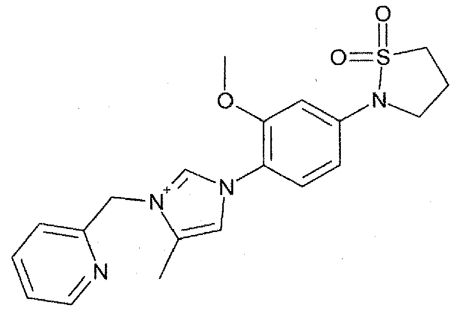 |
| E36 | 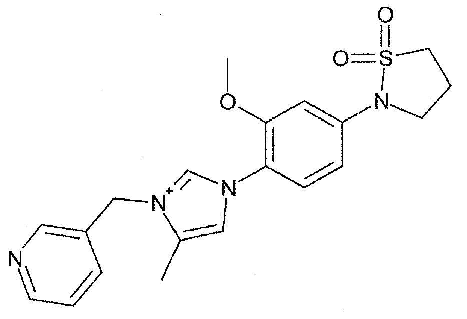 |

**[Table 77]**

| | |
|---|---|
| E37 | 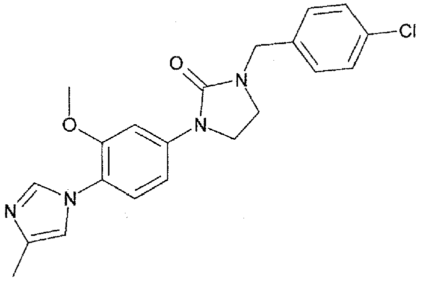 |
| E38 | 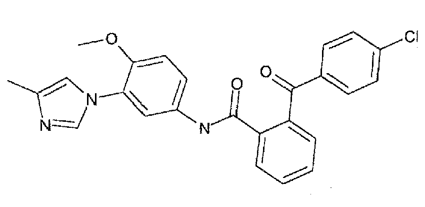 |
| E39 | 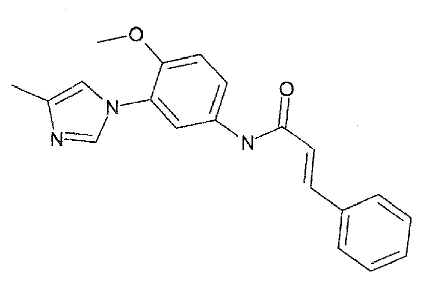 |
| E40 | 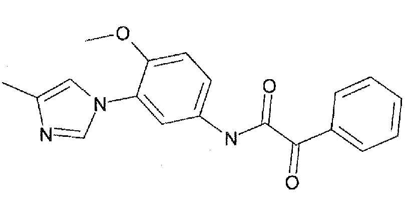 |

**[Table 78]**

| | |
|---|---|
| E41 | 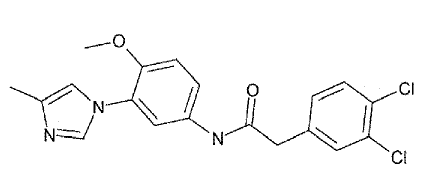 |
| E42 | 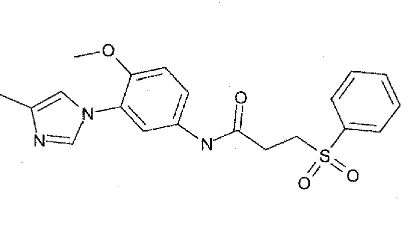 |
| E43 | 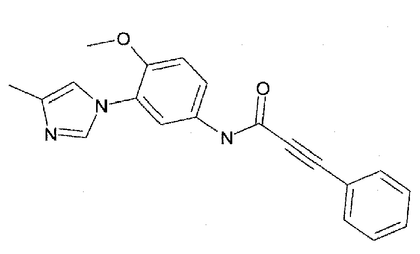 |
| E44 | 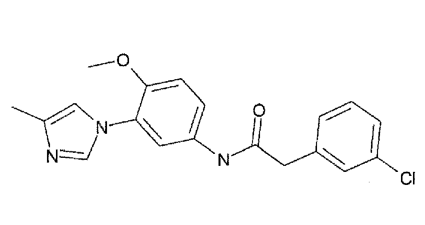 |

**[Table 79]**

| | |
|---|---|
| E45 | 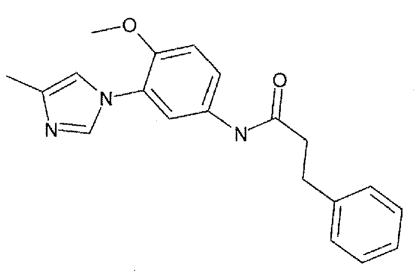 |
| E46 | 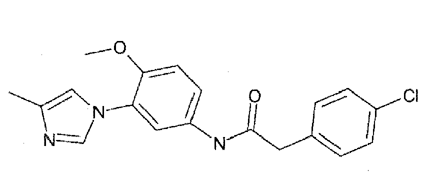 |
| E47 | 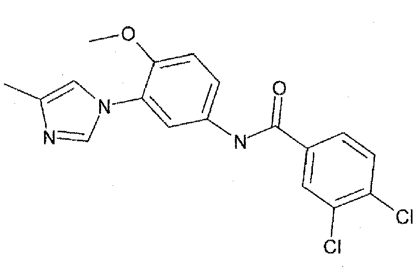 |
| E48 | 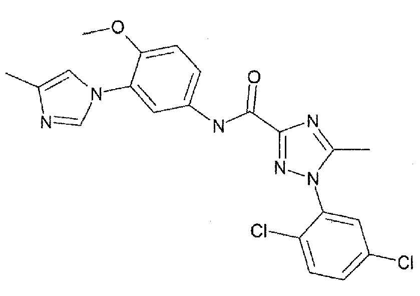 |

**[Table 80]**

| | |
|---|---|
| E49 | 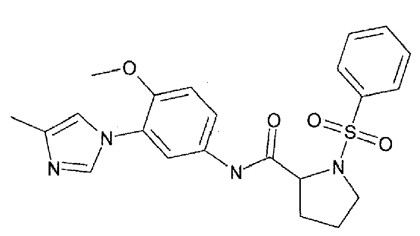 |
| E50 | 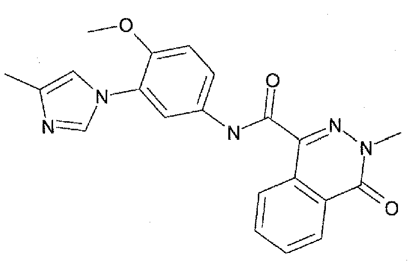 |
| E51 | 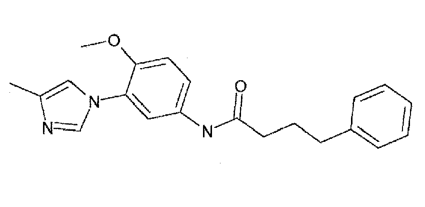 |
| E52 | 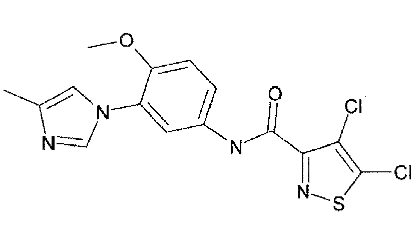 |

**[Table 81]**

| | |
|---|---|
| E53 | 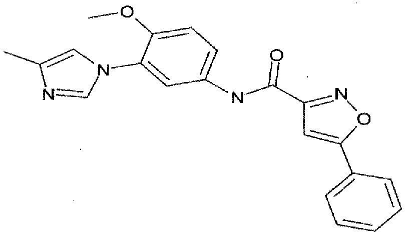 |
| E54 | 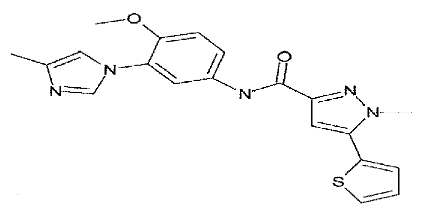 |
| E55 | 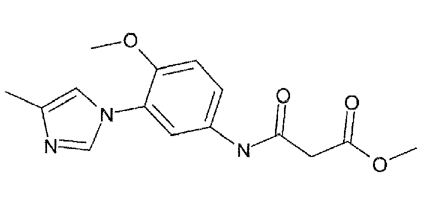 |
| E56 | 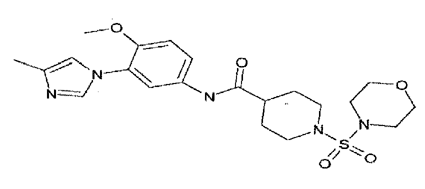 |
| E57 | 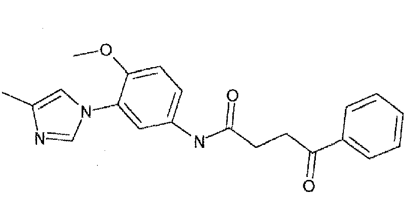 |

**[Table 82]**

| Compound No. | [M+H]+ | Retention time | LC/MS Method | **NMR** |
|---|---|---|---|---|
| 1 | 412.02 | 1.38 | B | 1H NMR (300 MHz, DMSO-d6) d 10.7 (bs, 1H), 8.01 (d, J = 2.1 Hz, 1H), 7.88 (d, J = 8.4 Hz, 1H), 7.75 (dd, J = 8.4 Hz, J = 2.1 Hz, 1H), 7.66 (s, 1H), 7.24 (d, J = 8.7 Hz, 1H), 7.01 (s; 1H), 6.92 (d, J = 2.1 Hz, 1H), 6.73 (dd, J = 8.7 Hz, J = 2.1 Hz, 1H), 3.74 (s, 3H), 2.11 (s, 3H). |
| 2 | 412.02 | 1.28 | B | 1H NMR (300 MHz, DMSO-d6) d 11.0 (bs, 1H), 8.13 (dd, J = 8.1 Hz, J = 1.5 Hz, 1H), 7.95 (dd, J = 8.1 Hz, J = 1.5 Hz, 1H), 7.63 (s, 3H), 7.58 (t, J = 8.1 Hz, 1H), 7.20 (d, J = 8.7 Hz, 1H), 6.99 (s, 1H), 6.90 (d, J = 2.4 Hz, 1H), 6.71 (dd, J = 8.7 Hz, J = 2.4 Hz, 1H), 3.70 (s, 3H), 2.10 (s, 3H). |
| 3 | | | C | |
| 4 | 401.12 | 1.17 | C | |
| 5 | 376.05 | 1.47 | C | |
| 6 | 394.11 | 1.4 | C | |
| 7 | 395.11 | 1.22 | C | |
| 8 | 422.15 | 1.53 | C | |
| 9 | 386.15 | 1.47 | C | |
| 10 | 369.09 | 1.23 | C | |
| 11 | 412.09 | 1.42 | C | |
| 12 | 394.11 | 1.32 | C | |
| 13 | 378.06 | 2.2 | C | |
| 14 | 358.11 | 1.22 | C | |
| 15 | 412.09 | 1.4 | C | |
| 16 | 404.12 | 1.22 | C | |
| 17 | 404.12 | 1.14 | C | |
| 18 | 392.08 | 1.33 | C | |
| 19 | 369.09 | 1.1 | C | |
| 20 | 369.09 | 1.15 | C | |
| 21 | 420.13 | 1.46 | C | |
| 22 | 412.09 | 1.27 | C | |
| 23 | 392.08 | 1.24 | C | |
| 24 | 436.13 | 1.45 | C | |
| 25 | 378.06 | 1.05 | C | |
| 26 | 378.06 | 1.17 | C | |
| 27 | 386.11 | 1.01 | C | |
| 28 | 392.08 | 1.19 | C | |
| 29 | 430.15 | 0.95 | C | |
| 30 | 411.10 | 1.08 | C | |
| 31 | 437.12 | 1.26 | C | |
| 32 | 395.11 | 0.92 | C | |
| 33 | 392.08 | 1.21 | C | |
| 31 | 454.09 | 1.46 | C | |
| 35 | 456.16 | 0.98 | C | |
| 36 | 406.09 | 1.21 | C | |

**[Table 83]**

| | | | | |
|---|---|---|---|---|
| 37 | 386.15 | 1.2 | C | |
| 38 | 392.08 | 1.1 | C | |
| 39 | 404.12 | 0.97 | C | |
| 40 | | | C | |
| 41 | 432.15 | 1.13 | C | |
| 42 | 372.13 | 1.1 | C | |
| 43 | 406.09 | 1.2 | C | |
| 44 | 406.09 | 1.2 | C | |
| 45 | 392.08 | 1.21 | C | |
| 46 | 458.18 | 1.07 | C | |
| 47 | 416.13 | 0.85 | C | |
| 48 | 450.14 | 1.35 | C | |
| 49 | 426.04 | 1.23 | C | |
| 50 | 426.10 | 1.18 | C | |
| 51 | 470.09 | 1.38 | C | |
| 52 | 451.14 | 0.91 | C | |
| 53 | 397.13 | 1.05 | C | |
| 54 | 372.13 | 1.1 | C | |
| 55 | 390.10 | 0.88 | C | |
| 56 | 401.07 | 0.94 | C | |
| 57 | 416.12 | 1.05 | C | |
| 58 | 420.13 | 1.3 | C | |
| 59 | 436.13 | 1.33 | C | |
| 60 | 427.17 | 1.39 | C | |
| 61 | 449.16 | 1.4 | C | |
| 62 | 479.17 | 1.4 | C | |
| 63 | 479.17 | 1.34 | C | |
| 64 | 479.17 | 1.33 | C | |
| 65 | 463.17 | 1.49 | C | |
| 66 | 429.15 | 1 | C | |
| 67 | 442.18 | 0.63 | C | |
| 68 | 358.11 | 1 | C | |
| 69 | 362.09 | 0.9 | C | |
| 70 | 422.01 | 0.96 | C | |
| 71 | 374.11 | 0.89 | C | |
| 72 | 428.08 | 1.1 | C | |
| 73 | 454.12 | 1.19 | C | |
| 74 | 389.08 | 0.95 | C | |
| 75 | 435.14 | 1.32 | C | |
| 76 | 406.09 | 1.7 | C | |
| 77 | 418.09 | 1.81 | C | |
| 78 | 432.11 | 1.93 | D | |
| 79 | 432.11 | 1.84 | D | |
| 80 | | | A | |
| 81 | | | A | |
| 82 | 446.70 | 1.94 | E | |
| 83 | 408.40 | 1.76 | E | |
| 84 | 425.40 | 1.38 | E | |
| 85 | 446.40 | 2 | E | |

**[Table 84]**

| | | | | |
|---|---|---|---|---|
| 86 | 408.50 | 1.78 | E | |
| 87 | 434.00 | 1.62 | C | |
| 88 | 434.00 | 1.84 | C | |
| 89 | 434.00 | 1.86 | C | |
| 90 | 470.00 | 1.49 | C | |
| 91 | 424.00 | 1.64 | C | |
| 92 | 470.00 | 1.71 | C | |
| 93 | 419.60 | 1.53 | E | |
| 94 | 419.70 | 1.7 | E | |
| 95 | 440.40 | 2 | E | |
| 96 | 440.60 | 2.29 | E | |
| 97 | 440.70 | 2.26 | E | |
| 98 | 476.60 | 1.97 | E | |
| 99 | 476.60 | 1.99 | E | |
| 100 | 402.60 | 1.81 | E | |
| 101 | 402.50 | 2.07 | E | |
| 102 | 402.60 | 2.06 | E | |
| 103 | 438.50 | 1.81 | E | |
| 104 | 438.40 | 1.77 | E | |
| 105 | 438.40 | 1.79 | E | |
| 106 | 418.90 | 1.97 | E | |
| 107 | 418.90 | 2.26 | E | |
| 108 | 418.80 | 2.25 | E | |
| 109 | 454.80 | 1.97 | E | |
| 110 | 454.70 | 1.93 | E | |
| 111 | 476.60 | 1.99 | E | |
| 112 | 380.08 | 1.19 | B | 1H NMR (300 MHz, DMSO-d6) d 10.67 (bs, 1H), 7.89 (m, 1H 7.75 - 7.63 (m, 2H), 7.65 (s, 1H), 7.23 (d, J = 8.4 Hz, 1H), 7.01 (s, 1H), 6.92 (d, J = 2.1 Hz, 1H), 6.73 (dd, J = 8.4 Hz, J = 2.1 Hz, 1H), 3.73 (s, 3H), 2.1 (s, 3H). |
| 113 | 379.06 | 1.07 | B | 1H NMR (300 MHz, DMSO-d6) d 10.78 (bs, 1H), 8.79 (dd, J = 2.4 Hz, J = 0.9 Hz, 1H), 8.20 (dd, J = 8.4 Hz, J = 2.4 Hz, 1H), 7.77 (dd, J = 8.4 Hz, J = 0.9 Hz, 1H), 7.67 (d, J = 1.2 Hz, 1H), 7.24 (d, J = 8.4 Hz, 1H), 7.02 (d, J = 1.2 Hz, 1H), 6.93 (d, J = 2.4 Hz, 1H), 6.74 (dd, J = 8.4 Hz, J = 2.4 Hz, 1H), 3.74 (s, 3H), 2.11 (s, 3H). |
| 114 | 383.01 | 1.66 | B | 1H NMR (300 MHz, DMSO-d6) d 10.91 (bs, 1H), 8.78 (d, J = 2.4 Hz, 1H), 8.48 (s. 1H), 8.26 (dd, J = 8.7 Hz, J = 2.4 Hz, 1H), 7.92 (d, J = 8.7 Hz, 1H). 7.68 (s, 1H), 7.53 (d, J = 8.7 Hz, 1H). 7.32 (d, J = 2.7 Hz. 1H), 7.13 (dd, J = 8.7 Hz, J = 2.7 Hz, 1H), 2.16 (s, 3H). |
| 115 | 379.06 | 0.91 | B | 1H NMR (300 MHz, DMSO-d6) d 11.22 (bs, 1H). 8.64 (dd, J = 5.4 Hz, J = 1.8 Hz, 1H), 8.54 (dd, J = 7.8 Hz, J = 1.8 Hz, 1H), 7.66 (dd, J = 7.8 Hz, J = 5.4 Hz, 1H), 7.64 (s, 1H), 7.21 (d. J = 8.4 Hz, 1H), 6.99 (s, 1H), 6.91 (d, J = 2.1 Hz. 1H), 6.73 (dd, J = 8.4 Hz, J = 2.1 Hz, 1H), 3.71 (s, 3H), 2.10 (s, 3H). |

**[Table 85]**

| | | | | |
|---|---|---|---|---|
| 116 | 455.11 | 1.26 | B | 1H NMR (300 MHz, DMSO-d6) d 10.85 (bs, 1H), 8.41 (dd, J = 4.5 Hz, J = 1.8 Hz, 1H), 8.30 (dd, J = 4.5 Hz, J = 1.8 Hz, 1H), 7.61 (s, 1H), 7.35 - 7.15 (m, 4H), 7.10 - 7.05 (m, 2H), 6.98 (d, J = 2.1 Hz, 1H). 6.98 (s, 1H), 6.81 (dd, J = 8.4 Hz, J = 2.1 Hz, 1H), 3.68 (s, 3H), 2.10 (s, 3H). |
| 117 | 398.15 | 1.76 | D | |
| 118 | 448.10 | 1.74 | D | |
| 119 | 356.11 | 1.5 | D | |
| 120 | 396.14 | 2.05 | D | |
| 121 | 374.11 | 1.3 | D | |
| 122 | 356.10 | 1.68 | D | |
| 123 | 320.13 | 1.63 | D | |
| 124 | 320.13 | 1.46 | D | |
| 125 | 430.12 | 2.22 | D | |
| 126 | 430.12 | 2.22 | D | |
| 127 | 430.12 | 2.06 | D | |
| 128 | 376.05 | 1.49 | B | 1H NMR (300 MHz, DMSO-d6) d 10.54 (s, 1H), 8.13 (d, J = 3.0 Hz, 1H), 8.12 (br, 1H), 7.78 - 7.58 (m, 5H), 7.31 (s, 1H), 3.94 (s, 3H), 2.19 (s, 3H). |
| 129 | 382.99 | 2.39 | B | 1H NMR (300 MHz, DMSO-d6) d 10.80 (s, 1H), 7.97 (d, J = 3.0 Hz, 1H), 7.85 (d, J = 9.0 Hz, 1H), 7.73 - 7.68 (m, 3H), 7.22 (d, J = 9.0 Hz, 1H). 6.73 (s, 1H), 2.24 (s, 3H). |
| 130 | 382.01 | 1.34 | B | 1H NMR (300 MHz, DMSO-d6) d 10.57 (s, 1H), 8.01 (m, 1H), 7.94 (d, J = 3.0 Hz, 1H), 7.85 (d, J = 9.0 Hz, 1H), 7.66 (dd, J = 9.0 Hz, J = 3.0 Hz, 1H), 7.49 (d, J = 9.0Hz, 2H), 7.32 (s, 1H), 7.16 (d, J = 9.0 Hz, 2H), 2.12 (s, 3H). |
| 131 | 412.02 | 1.4 | B | 1H NMR (300 MHz, DMSO-d6) d 10.2 - 10.4 (br, 1H), 7.96 (t, J = 3.0 Hz, 1H). 7.78 (d, J = 3.0 Hz, 2H), 7.66 (s, 1H), 7.24 (d, J = 9.0 Hz, 1H), 7.01 (s, 1H), 6.90 (d, J = 3.0 Hz, 1H), 6.73 (dd, J = 9.0 Hz, J = 3.0 Hz, 1H), 3.73 (s, 3H), 2.11 (s, 3H). |
| 132 | 412.02 | 1.32 | B | 1H NMR (300 MHz, DMSO-d6) d 10.2 - 10.4 (br, 1H), 7.90 (d. J = 3.0 Hz, 1H), 7.56 (dd, J = 9.0 Hz, d = 3.0 Hz, 1H), 7.51 (d, J = 9.0 Hz, 1H), 7.46 (m, 1H), 7.02 (d, J = 9.0 Hz, 1H). 6.80 (s, 1H), 6.71 (d, J = 3.0 Hz, 1H), 6.53 (dd, J = 9.0 Hz, J = 3.0 Hz, 1H), 3.51 (s, 3H), 1.90 (s, 3H). |
| 133 | 398.99 | 2.24 | B | 1H NMR (300 MHz, DMSO-d6) d 10.71 (br, 1H), 8.38 (s, 1H), 8.01 (d, J = 3.0 Hz, 1), 7.85 (d, J = 9.0 Hz, 1H), 7.76 (dd, J = 9.0 Hz, J = 3.0 Hz, 1H), 7.65 (d, J = 9.0 Hz, 1H), 7.44 (s, 1H), 6.91 (d, J = 3.0 Hz, 1H), 6.82 (dd, J = 9.0 Hz, J = 3.0 Hz. 1H), 3.87 (s, 3H). |
| 134 | 436.03 | 1.16 | D | |
| 135 | 434.15 | 1.95 | D | |
| 136 | 372.13 | 1.56 | D | |
| 137 | 434.15 | 1.87 | D | |
| 138 | 434.15 | 1.86 | D | |
| 139 | 372.13 | 1.56 | D | |
| 140 | 376.11 | 1.45 | D | |
| 141 | 394.10 | 1.48 | D | |

**[Table 86]**

| | | | | |
|---|---|---|---|---|
| 142 | 454.09 | 1.99 | D | |
| 143 | 454.09 | 2.01 | D | |
| 144 | 504.11 | 2.19 | D | |
| 145 | 504.11 | 2.18 | D | |
| 146 | 504.11 | 2.08 | D | |
| 147 | 436.13 | 1.73 | D | |
| 148 | 463.17 | 1.79 | D | |
| 149 | 463.17 | 1.72 | D | |
| 150 | 477.15 | 1.78 | D | |
| 151 | 477.15 | 1.51 | D | |
| 152 | 477.15 | 1.75 | D | |
| 153 | 438.12 | 1.82 | D | |
| 154 | 438.12 | 1.97 | D | |
| 155 | 456.11 | 1.87 | D | |
| 156 | 450.14 | 1.83 | D | |
| 157 | 450.14 | 1.91 | D | |
| 158 | 488.12 | 2.02 | D | |
| 159 | 488.12 | 2.16 | D | |
| 160 | 488.12 | 2.08 | D | |
| 161 | 488.05 | 2.21 | D | |
| 162 | 488.05 | 2.19 | D | |
| 163 | 488.05 | 2.09 | D | |
| 164 | 463.14 | 1.61 | D | |
| 165 | 463.14 | 1.59 | D | |
| 166 | 462.14 | 1.86 | D | |
| 167 | 462.14 | 1.71 | D | |
| 168 | 462.14 | 1.79 | D | |
| 169 | 471.14 | 1.6 | D | |
| 170 | 471.14 | 1.53 | D | |
| 171 | 471.14 | 1.46 | D | |
| 172 | 470.15 | 2.14 | D | |
| 173 | 470.15 | 2.07 | D | |
| 174 | 472.08 | 1.58 | D | |
| 175 | 472.08 | 2.01 | D | |
| 176 | 436.13 | 1.65 | D | |
| 177 | 421.13 | 1.31 | D | |
| 178 | 421.13 | 1.23 | D | |
| 179 | 420.13 | 1.84 | D | |
| 180 | 522.08 | 2.22 | D | |
| 181 | 496.16 | 2.22 | D | |
| 182 | 448.16 | 2.09 | D | |
| 183 | 448.16 | 2.15 | D | |
| 184 | 399.25 | 1.73 | D | |
| 185 | 425.13 | 2.51 F | | |

**[Table 87]**

| | | | | |
|---|---|---|---|---|
| 186 | 428.22 | 1.80 | Method D | |
| 187 | 430.12 | 2.30 | Method D | |
| 188 | 430.12 | 2.27 | Method D | |
| 189 | 430.12 | 2.22 | Method D | |
| 190 | 501.24 | 3.20 | Method D | |
| 191 | 501.24 | 3.15 | Method D | |
| 192 | 425.13 | 2.51 | Method D | |
| 193 | 401.19 | 2.40 | Method D | |
| 194 | 401.19 | 2.42 | Method D | |
| 195 | 455.24 | 1.28 | Method D | |
| 196 | 454.20 | 2.88 | Method D | |
| 197 | 468.18 | 2.50 | Method D | |
| 198 | 440.18 | 2.84 | Method D | |
| 199 | 413.17 | 2.68 | Method D | |
| 200 | 425.13 | 1.45 | Method D | |
| 201 | 425.13 | 1.60 | Method D | |
| 202 | 427.15 | 1.55 | Method D | |
| 203 | 459.16 | 1.64 | Method D | |
| 204 | 459.09 | 1.53 | Method D | |
| 205 | 459.09 | 1.61 | Method D | |
| 206 | 421.18 | 1.52 | Method D | |
| 207 | 397.22 | 1.49 | Method D | |
| 208 | 467.20 | 1.93 | Method D | |
| 209 | 459.16 | 1.80 | Method D | |
| 210 | 459.09 | 1.86 | Method D | |
| 211 | 435.20 | 1.51 | Method D | |
| 212 | 421.18 | 1.44 | Method D | |
| 213 | 459.16 | 1.73 | Method D | |
| 214 | 435.20 | 1.43 | Method D | |
| 215 | 459.09 | 1.88 | Method D | |
| 216 | 421.18 | 1.52 | Method D | |
| 217 | 423.18 | 1.45 | Method D | |
| 218 | 419.20 | 1.54 | Method D | |
| 219 | 427.15 | 1.44 | Method D | |
| 220 | 427.15 | 1.52 | Method D | |
| 221 | 427.15 | 1.55 | Method D | |
| 222 | 433.22 | 1.84 | Method D | |
| 223 | 467.20 | 1.93 | Method D | |
| 224 | 434.21 | 1.48 | Method D | |
| 225 | 441.17 | 1.54 | Method D | |
| 226 | 439.15 | 1.61 | Method D | |
| 227 | 399.16 | 1.74 | Method D | |
| 228 | 399.16 | 1.78 | Method D | |
| 229 | 420.23 | 2.80 | Method D | |
| 230 | 377.19 | 2.71 | Method D | |
| 231 | 411.15 | 2.92 | Method D | |
| 232 | 391.21 | 2.77 | Method D | |
| 233 | 378.19 | 2.28 | Method D | |
| 234 | 411.15 | 2.86 | Method D | |
| 235 | 395.18 | 2.77 | Method D | |
| 236 | 378.19 | 2.25 | Method D | |
| 237 | 395.18 | 2.75 | Method D | |
| 238 | 446.25 | 3.06 | Method D | |
| 239 | 395.18 | 2.75 | Method D | |

**[Table 88]**

| | | | | |
|---|---|---|---|---|
| 240 | 421.18 | 2.68 | Method D | |
| 241 | 437.21 | 2.76 | Method D | |
| 242 | 383.24 | 3.02 | Method D | |
| 243 | 437.21 | 2.75 | Method D | |
| 244 | 407.20 | 2.72 | Method D | |
| 245 | 381.22 | 2.93 | Method D | |
| 246 | 453.22 | 3.10 | Method D | |
| 247 | 405.22 | 2.91 | Method D | |
| 248 | 433.25 | 3.25 | Method D | |
| 249 | 407.20 | 2.78 | Method D | |
| 250 | 427.21 | 2.97 | Method D | |
| 251 | 467.24 | 3.10 | Method D | |
| 252 | 435.23 | 2.99 | Method D | |
| 253 | 391.21 | 2.80 | Method D | |
| 254 | canceled | | | |
| 255 | 410.16 | 2.85 | Method D | |
| 256 | 395.18 | 0.95 | Method D | |
| 257 | 398.16 | 1.86 | E | |
| 258 | 430.10 | 2.12 | E | |
| 259 | 438.21 | 1.83 | E | |
| 260 | 420.18 | 1.79 | E | |
| 261 | 412.19 | 2.08 | E | |
| 262 | 376.19 | 1.90 | E | |
| 263 | 424.19 | 2.47 | E | |
| 264 | 426.17 | 1.94 | E | |
| 265 | 426.17 | 1.95 | E | |
| 266 | 410.18 | 2.26 | E | |
| 267 | 410.18 | 2.33 | E | |
| 268 | 410.18 | 2.31 | E | |
| 269 | 402.12 | 2.06 | E | |
| 270 | 426.17 | 1.92 | E | |
| 271 | 444.14 | 2.46 | E | |
| 272 | 444.14 | 2.44 | E | |
| 273 | 444.14 | 2.42 | E | |
| 274 | 400.19 | 2.37 | E | |
| 275 | 444.14 | 2.37 | E | |
| 276 | 444.14 | 2.44 | E | |
| 277 | 444.14 | 2.35 | E | |
| 278 | 397.16 | 1.23 | E | |
| 279 | 424.19 | 2.35 | E | |
| 280 | 410.12 | | | 1H-NMR (DMSO-d6) δ: 2.15 (3H, s), 3.80 (3H, s), 3.99 (2H, s), 5.05 (1H, s), 7.10 (1H, s), 7.21 (1H, d. J = 7.3 Hz), 7.38 (1H, d, J = 10.9 Hz), 7.67 (2H, d, J = 13.1 Hz), 7.74 (1H, s), 8.11 (2H, s), 8.18 (1H, s). |
| 281 | 438.21 | 1.51 | A | |
| 282 | 439.15 | 1.79 | E | |
| 283 | 396.14 | 2.05 | C | |
| E1 | 309.104 | 2.17 | Method F | |
| E2 | 273.13 | 1.98 | Method F | |
| E3 | 367.1492 | 1.82 | Method D | |
| E4 | 287.143 | 1.84 | Method D | |
| E5 | 356.11 | 2.67 | E | |
| E6 | 430.19 | 3.14 | E | |
| E7 | 440.19 | 2.91 | E | |
| E8 | 390.07 | 2.96 | E | |
| E9 | 398.18 | 2.93 | E | |

**[Table 89]**

| | | | | |
|---|---|---|---|---|
| E10 | 340.14 | 2.62 | E | |
| E11 | 348.16 | 2.84 | E | |
| E12 | 364.19 | 2.93 | E | |
| E13 | 336.16 | 2.71 | E | |
| E14 | 356.11 | 2.78 | E | |
| E15 | 384.14 | 2.97 | E | |
| E16 | 397.15 | 1.28 | A | |
| E17 | 433.11 | 1.29 | A | |
| E18 | 363.19 | 1.50 | A | |
| E19 | 399.17 | 1.59 | A | |
| E20 | 431.11 | 1.90 | A | |
| E21 | 431.11 | 1.61 | A | |
| E22 | 397.15 | 1.63 | A | |
| E23 | 421.19 | 1.73 | A | |
| E24 | 413.20 | 1.81 | A | |
| E25 | 377.20 | 1.87 | A | |
| E26 | 364.18 | 1.22 | A | |
| E27 | 364.18 | 1.03 | A | |
| E28 | 435.14 | 1.61 | A | |
| E29 | 467.08 | 1.78 | A | |
| E30 | 467.08 | 1.83 | A | |
| E31 | 433.11 | 1.52 | A | |
| E32 | 457.16 | 1.42 | A | |
| E33 | 449.17 | 1.78 | A | |
| E34 | 413.17 | 1.49 | A | |
| E35 | 400.15 | 1.14 | A | |
| E36 | 400.15 | 0.92 | A | |
| E37 | 397.14 | 1.83 | E | |
| E38 | 446.12 | 2.13 | E | |
| E39 | 334.15 | 1.81 | E | |
| E40 | 336.13 | 1.59 | E | |
| E41 | 390.07 | 1.97 | E | |
| E42 | 400.13 | 1.36 | E | |
| E43 | 332.13 | 1.79 | E | |
| E44 | 356.11 | 1.75 | E | |
| E45 | 336.16 | 1.63 | E | |
| E46 | 356.11 | 1.77 | E | |
| E47 | 376.05 | 1.98 | E | |
| E48 | 457.09 | 1.80 | E | |
| E49 | 441.15 | 1.51 | E | |
| E50 | 390.15 | 1.67 | E | |
| E51 | 350.18 | 1.83 | E | |
| E52 | 383.01 | 1.73 | E | |
| E53 | 375.14 | 1.94 | E | |
| E54 | 394.13 | 1.83 | E | |
| E55 | 304.12 | 0.99 | E | |
| E56 | 464.19 | 1.45 | E | |
| E57 | 364.16 | 1.59 | E | |

In the tables, 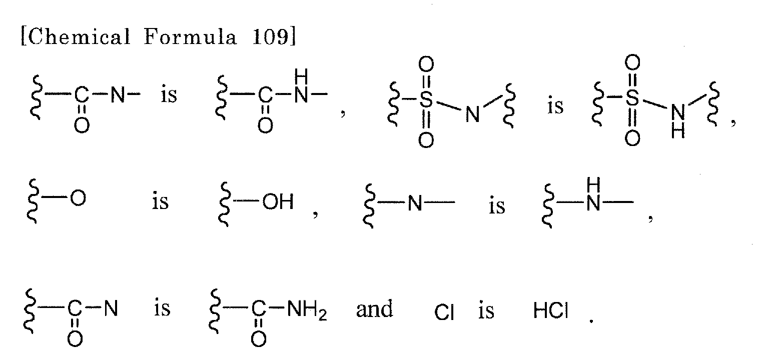 HCl means hydrochloride.

### Test example 1. Suppressive activity of Aβ42 production in neuroblast expressing human APP695

Dose dependent effect of each test compound was evaluated by measurement of secreted Aβ42 and cell viability in SH-SY5Y-APPwt cells. Secreted Aβ42 was quantified by an ELISA assay and cell viability was measured using a cell count reagent.
SH-SY5Y-APPwt cells were generated from human SH-SY5Y neuroblast by transfection of human APP695 DNA as a stable cell-line and cultured in DMEM supplemented with 10% FCS.
SH-SY5Y-APPwt cells were plated at 1.2×10⁵ cells/well/150 µl in 96-well plates. A solution of the test compound in DMSO was diluted with DMEM to a concentration 4-fold higher than the final concentration. 50 µl of the diluted solution was sufficiently mixed with the cells and the final DMEM solution was 200 µl/well. The final DMSO concentration was 1%. As a control compound, a γ-secretase inhibitor, DAPT (Calbiochem) was used. 24 hours after, the culture supernatant was collected and appropriately diluted for ELISA quantification of Aβ42. Cells were used for cell viability assay.
Human/Rat β Amyloid(42) ELISA Kit WAKO (WAKO Pure Chemical Industries) was used for ELISA. ELISA was carried out according to the protocols recommended by the manufacturer's instructions. The results were shown as percentage to the Aβ concentration in the medium of control group (% of Control) and then IC₅₀ values of each test compounds were calculated. Cell viability was measured using Cell Count Reagent SF (NACALAI TESQUE) according to the protocols recommended by the manufacturer's instructions. The results were shown as percentage to cell viability of control group (% of Control) and reduction of cell viability was not seen.
The results are shown as below.

**[Table 90]**

| Compound No. | IC50 (µM) |
|---|---|
| E8 | 1.1 |
| 189 | 1.6 |
| 259 | 0.7 |
| 201 | 0.8 |
| 231 | 1.8 |
| 38 | 6.6 |
| 1 | 0.9 |

### Test example 2 Inhibition of γ-secretase activity of the test compounds

Inhibition of γ-secretase activity of the test compounds were evaluated by assessment of APP processing by γ-secretase, i.e., by measurement of a γ-secretase substrate C-terminal fragment β (CTFβ) in SH-SY5Y-APPwt cells. Cells were treated in test compound concentration 1-fold and 10-fold higher than its IC₅₀ value for Aβ42 inhibition. CTFβ accumulated by inhibition of γ-secretase activity was quantified by an ELISA assay. SH-SY5Y-APPwt cells were generated from human SH-SY5Y neuroblast by transfection of human APP695 DNA as a stable cell-line and cultured in DMEM supplemented with 10% FCS. SHSY5Y-APPwt cells were plated at 1.0×10⁶ cells/well/1ml DMEM in 6-well plates. A solution of the test compound in DMSO was diluted with DMEM to a concentration 2-fold higher than the final concentration. 1 ml of the diluted solution was sufficiently mixed with the cell and the final DMEM solution was 2 mL/well. The final DMSO concentration was 1%. As a control compound, a γ-secretase inhibitor, DAPT (Calbiochem) was used. 24 hours after, the cells were collected, extracted by TBS buffer containing 1% Triton X100 and centrifuged at 14000rpm for 10 minutes at 4°C. Protein concentrations in the supernatants were measured by Protein assay Kit (Pierce). The supernatants were diluted and used for CTFβ quantification by βCTF ELISA Kit (IBL) The results were shown as percentage to the CTFβ concentration of control group (added only DMSO) (% of Control), and increasing and decreasing CTFβ of the test compound was calculated. The test compounds which inhibit γ-secretase activity dose-dependently increase CTFβ, γ-secretase substrate.

### (Results)

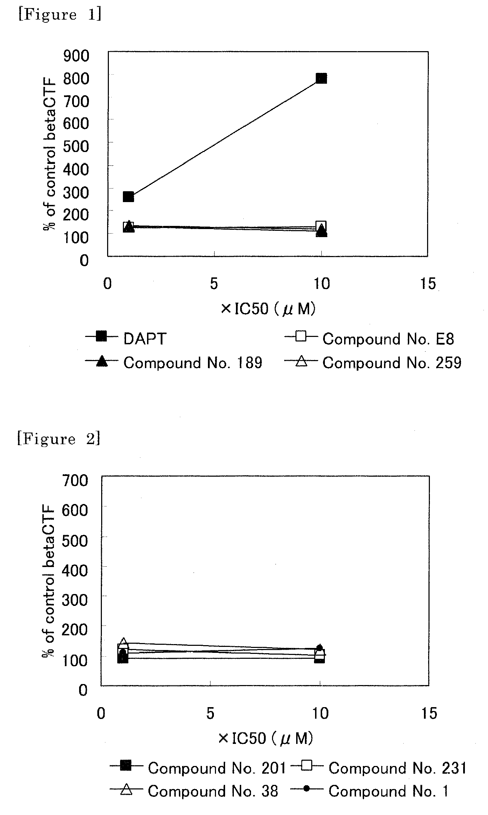

The results are shown in Figure 1 and Figure 2. A control compound, γ-secretase inhibitor DAPT dose-dependently increased CTFβ whereas the compounds of the present invention did not increase of CTFβ. It became clear that the compounds of the present invention inhibited production of Aβ42 without inhibition of γ-secretase activity.
These results indicate that the compounds of the present invention are not γ-secretase inhibitors but γ-secretase modulators which inhibit production of Aβ42.

### Test Example 3 CYP3A4 fluorescent MBI test

The CYP3A4 fluorescent MBI test is a test to examine enhancement of CYP3A4 inhibition by metabolic reaction of the compound. Here, E. coli-expressing CYP3A4 was used as an enzyme, and a reaction wherein 7-benzyloxy trifluoromethylcumarin (BFC) was debenzylated by the CYP3A4 enzyme into a fluorescent metabolite 7-hydroxytrifluoromethylcumarin (HFC) was employed as an indicator reaction.
The reaction conditions are as follows.
Substrate: 5.6 µmol/L 7-BFC; Pre-reaction time: 0 or 30 minutes; Reaction time: 15 minutes; Reaction temperature: 25°C (room temperature); CYP3A4 content (E. coli-expressing enzyme): 62.5 pmol/mL at pre-reaction, 6.25 pmol/mL at reaction (10-fold diluted); Test compound concentration: 0.625, 1.25, 2.5, 5, 10, and 20 µmol/L (six different concentrations).
The enzyme and a test compound solution are added to a K-Pi buffer (pH 7.4) in the aforementioned amounts for the pre-reaction so that a pre-reaction solution is prepared, and this solution is put into a 96-well plate. Part of this solution is transferred to another 96-well plate such that it is diluted with the substrate and the K-Pi buffer to get a 10-fold diluted solution. NADPH, which is a coenzyme, is added so as to trigger the indicator reaction (without pre-reaction). As the reaction proceeded for a predetermined time period, acetonitrile/0.5 mol/L Tris (tris hydroxyaminomethane) = 4/1 is added to terminate the reaction. NADPH is also added to the residual pre-reaction solution so as to trigger the pre-reaction (with pre-reaction). As the pre-reaction proceeded for a predetermined time period, part of the solution is transferred to another plate so that the solution is diluted with the substrate and the K-Pi buffer to get a 10-fold diluted solution, and then the indicator reaction is triggered. As the reaction proceeded for a predetermined time period, acetonitrile/0.5 mol/L Tris (tris hydroxyaminomethane) = 4/1 is added so as to terminate the reaction. With respect to each of the plates on which the indicator reaction is performed, the fluorescence value of the metabolite 7-HFC is measured with a fluorescence plate reader (Ex = 420 nm, Em = 535 nm).
Control (100%) is prepared by adding only DMSO, a solvent to dissolve the test compound, to the reaction system. The remaining activity (%) at each concentration after adding the test compound solution is calculated, and the IC₅₀ value is calculated from the concentration and inhibition percentage by inverse estimation with the logistic model. When the difference of the IC₅₀ values is 5 µM or higher, it is regarded as (+), and when the difference is 3 µM or lower, it is regarded as (-).

### (Results)

Compound No.1: (-)
Compound No.111: (-)

### Test Example 4 CYP inhibition test

Using commercially available pooled human liver microsomes and employing typical substrate metabolic reactions of 5 major species of human CYP enzymes (CYP1A2, 2C9, 2C19, 2D6, 3A4), namely, O-deethylation of 7-ethoxyresorufin (CYP1A2), methyl-hydroxylation of tolbutamide (CYP2C9), 4'-hydroxylation of mephenytoin (CYP2C19), O-demethylation of dextromethorphan (CYP2D6), and hydroxylation of terfenadine (CYP3A4), as indicator reactions, the degree of inhibition of each metabolite production by each test compound is evaluated.
The reaction conditions are as follows:
Substrate: 0.5 µmol/L of ethoxyresorufin (CYP1A2), 100 µmol/L of tolbutamide (CYP2C9), 50 µmol/L of S-mephenytoin (CYP2C19), 5 µmol/L of dextromethorphan (CYP2D6), 1 µmol/L of terfenadine (CYP3A4); Reaction time period: 15 minutes; Reaction temperature: 37°C; Enzyme: pooled human liver microsomes 0.2 mg protein/mL; Test compound concentration: 1, 5, 10 and 20 µmol/L (four different concentrations).
As a reaction solution, the respective five substrates, human liver microsomes, and test compound in the aforementioned amounts are added to a 50 mM Hepes buffer in a 96-well plate. NADPH, which is a coenzyme, is added thereto to start the indicator metabolic reaction. The reaction proceeds for 15 minutes at 37°C, and the solution of methanol/acetonitrile = 1/1 (v/v) is added so as to terminate the reaction.
Centrifugation is performed for 15 minutes at 3000 rpm, and each metabolite in the centrifuged supernatant is quantified. Namely, resorufin (CYP1A2 metabolite) is quantified with a fluorescence multilabel counter, and hydroxylated tolbutamide (CYP2C9 metabolite), 4'-hydroxylated mephenytoin (CYP2C19 metabolite), dextrorphan (CYP2D6 metabolite) and alcoholized terfenadine (CYP3A4 metabolite) are quantified with LC/MS/MS.
Control (100%) is prepared by adding only DMSO, a solvent to dissolve the test compound, to the reaction system. The remaining activity (%) at each concentration after adding the test compound solution is calculated, and the IC₅₀ value is calculated from the concentration and inhibition percentage by inverse estimation with the logistic model.

### (Results)

Compound No.121: five species >10µM

### Test Example 5 FAT test

20 µL of each strain of cryopreserved Salmonella typhimurium (TA98, TA100) is inoculated to 10 mL of a nutrient broth (2.5% Oxoid nutrient broth No.2), and cultured for 10 hours at 37°C before shaking. With respect to TA98, 9 mL of the bacterial culture is centrifuged (2000xg, 10 minutes) so that the culture medium is removed. The bacteria are suspended in 9 mL of a Micro F buffer (K₂HPO₄: 3.5 g/L, KH₂PO₄: 1 g/L, (NH₄)₂SO₄: 1g/L, trisodium citrate dihydrate: 0.25 g/L, MgSO₄ ·7H₂0: 0.1g/L), and added to 110 mL of an Exposure medium (Micro F buffer containing: biotin (8 µg/mL), histidine (0.2 µg/mL), and glucose (8 mg/mL)) to prepare a test bacterial culture. With respect to TA100, 3.16 mL of the bacterial culture is added to 120 mL of an Exposure medium to prepare a test bacterial culture. 588 µL of each test bacterial culture (mixed solution of 498 µL of the test bacterial culture and 90 µL of S9 mix for a metabolic activation condition) is mixed with 12 µL each of a solution of the test compound in DMSO (8-step 2-fold serial dilution from the maximum dosage of 50 mg/mL), DMSO as negative control, and positive controls (non-metabolic activation condition: a 50 µg/mL solution of 4-nitroquinoline-1-oxide in DMSO for TA98, a 0.25 µg/mL solution of 2-(2-furyl)-3-(5-nitro-2-furyl)acrylamide in DMSO for TA100; metabolic activation condition: a 40 µg/mL solution of 2-aminoanthracene in DMSO for TA98, a 20 µg/mL solution of 2-aminoanthracene in DMSO for TA100). The mixture is shake-cultured for 90 minutes at 37°C. 460 µL of the bacterial culture exposed to the test compound is added to 2300 µL of an Indicator medium (Micro F buffer containing biotin (8 µg/mL), histidine (0.2 µg/mL), glucose (8 mg/mL), and bromocresol purple (37.5 µg/mL)), and 50 µL portions of the mixture are dispensed in a microplate 48 wells/dosage. The mixture is stationarily cultured for 3 days at 37°C. In the wells containing bacteria which acquired proliferation potency due to mutation of the amino acid (histidine) synthase gene, the color of the mixture changes from purple to yellow due to pH change. Thus, the number of bacterial proliferation wells in which the color of the mixture has turned into yellow is counted among the 48 wells for each dosage, and evaluation is performed in comparison with the negative control group.

### (Results)

Compound No. 1: (-)
Compound No. 110: (-)

### Test Example 6 Solubility test

The solubility of each compound is determined under 1% DMSO addition conditions. A 10 mM solution of the compound is prepared with DMSO, and 6 µL of the compound solution is added to 594 µL of an artificial intestinal juice (water and 118 mL of 0.2 mol/L NaOH reagent are added to 250 mL of 0.2 mol/L potassium dihydrogen phosphate reagent to reach 1000 mL) with a pH of 6.8. The mixture is left standing for 16 hours at 25°C, and the mixture is vacuum-filtered. The filtrate is two-fold diluted with methanol/water = 1/1, and the compound concentration in the filtrate is measured with HPLC or LC/MS/MS by the absolute calibration method.

### (Results)

Compound No.74: >50µM
Compound No.178: >50µM

### Test Example 7 Metabolic stability test

The test compound is reacted with commercially available pooled human liver microsomes for a predetermined time period. The residual rate is calculated by comparing the reacted sample and unreacted sample, and thus the degree of metabolism in liver is evaluated. In 0.2 mL of a buffer (50 mmol/L tris-HCl pH 7.4, 150 mmol/L potassium chloride, 10 mmol/L magnesium chloride) containing human liver microsomes 0.5 mg protein/mL, reaction is carried out in the presence of 1 mmol/L NADPH for 0 minute or 30 minutes at 37°C (oxidative reaction). After the reaction, 50 µL of the reaction solution is added to and mixed with 100 µL of a solution of methanol/acetonitrile = 1/1 (v/v), and the mixture is centrifuged for 15 minutes at 3000 rpm. The test compound in the centrifuged supernatant is quantified by LC/MS/MS, and the residual amount of the test compound after the reaction is calculated based on the compound amount at 0-minute reaction set as 100%.

### (Results)

Compound No. 113: 97.0%
Compound No.115: 97.8%

### Test Example 8 hERG test

For the purpose of risk assessment for QT prolongation on an electrocardiogram, the action on delayed rectifier K+ current (I_{Kr}) which plays an important role on a process of ventricular repolarization is studied using a HEK293 cell with a human ether-a-go-go related gene (hERG) channel expressed therein.
A cell is maintained at a membrane potential of -80 mV, and then +50 mV of a depolarizing stimulus is applied to the cell for 2 seconds and further -50 mV of a repolarizing stimulus is applied thereto for 2 seconds by a whole-cell patch clamp technique with a fully-automatic patch clamp system (PatchXpress 7000A, Axon Instruments Inc.), and the induced I_{Kr} is recorded. As the current generated is stabilized, an extracellular fluid with the test compound dissolved therein at a target concentration (NaCl: 137 mmol/L, KCl: 4 mmol/L, CaCl₂ ·2H₂O: 1.8 mmol/L, MgCl₂ ·6H₂O: 1 mmol/L, glucose: 10 mmol/L, HEPES (4-(2-hydroxyethyl)-1-piperazineethanesulfonic acid): 10 mmol/L, pH = 7.4) is applied to the cell for 10 minutes under room temperature conditions. The absolute value of the maximum tail current is determined from the obtained I_{Kr} with an analysis software (DataXpress ver. 1, Molecular Devices Corporation) based on a current value at a maintained membrane potential. Further, an inhibitory percentage relative to the maximum tail current before application of the test compound is calculated, and influence of the test compound on I_{Kr} is evaluated in comparison with the medium application group (0.1% dimethyl sulfoxide solution).

### (Results)

Compound No.69: 6.4%
Compound No.74: 1.1%
Compound No.113: 8.5%

### Test Example 9 Powder solubility test

Appropriate amounts of the test substances are put into appropriate containers. To the respective containers are added 200 µL of JP-1 fluid (sodium chloride 2.0 g, hydrochloric acid 7.0 mL and water to reach 1000 mL), 200 µL of JP-2 fluid (phosphate buffer (pH 6.8) 500 mL and water 500 mL), and 200 µL of 20 mmol/L TCA (sodium taurocholate)/JP-2 fluid (TCA 1.08 g and water to reach 100 mL). In the case that the test compound is dissolved after the addition of the test fluid, the bulk powder is added as appropriate. The containers are sealed, and shaken for 1 hour at 37°C. The mixtures are filtered, and 100 µL of methanol is added to each of the filtrate (100 µL) so that the filtrates are two-fold diluted. The dilution ratio may be changed if necessary. The dilutions are observed for bubbles and precipitates, and then the containers are sealed and shaken. Quantification is performed by HPLC with an absolute calibration method.

### Test Example 10 BA test

Materials and methods for studies on oral absorption
(1) Animal: mice or rats
(2) Breeding conditions: mice or rats are allowed to freely take solid feed and sterilized tap water
(3) Dose and grouping: orally or intravenously administered at a predetermined dose; grouping is as follows (Dose depends on the compound)
   Oral administration: 1 to 30 mg/kg (n=2 to 3)
   Intravenous administration: 0.5 to 10 mg/kg (n=2 to 3)
(4) Preparation of dosing solution: for oral administration, in a solution or a suspension state; for intravenous administration, in a solubilized state
(5) Administration method: in oral administration, forcedly administer into ventriculus with oral probe; in intravenous administration, administer from caudal vein with a needle-equipped syringe
(6) Evaluation items: blood is collected over time, and the plasma concentration of drug is measured by LC/MS/MS
(7) Statistical analysis: regarding the transition of the plasma concentration, the area under the plasma concentration-time curve (AUC) is calculated by non-linear least squares program WinNonlin (Registered trademark), and the bioavailability (BA) is calculated from the AUCs of the oral administration group and intravenous administration group

### Formulation Example 1

Granules containing the following ingredients are prepared.

| | | |
|---|---|---|
| Ingredients | Compound of formula (I) or (I') | 10 mg |
| | Lactose | 700 mg |
| | Corn starch | 274 mg |
| | HPC-L | 16 mg |

Compound of formula (I) and lactose are screened through a 60-mesh sieve. Corn starch is screened through a 120-mesh sieve. These ingredients are mixed by a V-shaped mixer. An aqueous solution of HPC-L (low-viscosity hydroxypropyl cellulose) is added to the mixed powders, and the mixture is kneaded, granulated (extrusion-granulation, pore-size: 0.5 to 1 mm), and dried. The dried granules obtained are screened through a vibrating sieve (12/60 meshes) to afford granules.

### Formulation Example 2

Granules to be capsulated containing the following ingredients are prepared.

| | | |
|---|---|---|
| Ingredients | Compound of formula (I) or (I') | 15 mg |
| | Lactose | 90 mg |
| | Corn starch | 42 mg |
| | HPC-L | 3 mg |

Compound of formula (I) and lactose are screened through a 60-mesh sieve. Corn starch is screened through a 120-mesh sieve. Then, these ingredients are mixed with each other. An HPC-L solution is added to the mixed powders, and the mixture is kneaded, granulated, and dried. The dried granules obtained are subjected to sizing, and 150 mg of the sized granules are capsulated into a size #4 hard gelatin capsule.

### Formulation Example 3

A tablet containing the following ingredients is prepared.

| | | |
|---|---|---|
| Ingredients | Compound of formula (I) or (I') | 10 mg |
| | Lactose | 90 mg |
| | Fine crystal cellulose | 30 mg |
| | CMC-Na | 15 mg |
| | Magnesium stearate | 5 mg |

Compound of formula (I), lactose, fine crystal cellulose, and CMC-Na (carboxymethyl cellulose sodium salt) are screened through a 60-mesh sieve, and they are mixed with each other. Magnesium stearate is added to the mixed powders, and thereby mixed powders for tablet are obtained. The mixed powders are directly compressed, so that a 150-mg tablet is obtained.

### Formulation Example 4

The following ingredients are mixed under heating, and then sterilized to be an injection.

| | | |
|---|---|---|
| Ingredients | Compound of formula (I) or (I') | 3 mg |
| | Nonionic surfactant | 15 mg |
| | Purified water for injection | 1 ml |

### Formulation Example 5

A cataplasm containing the following ingredients is prepared.

| | | |
|---|---|---|
| Ingredient | Compound of formula (I) or (I') | 50 mg |
| | aqueous-based (5% ethanol/5% butylene glycol/90% purified water) | 950 mg |
| | glycerin | |
| | kaoline | |
| | aqueous polyvinyl alcohol | |

Compound of formula (I) or (I') is added to aqueous-based. The mixture is irradiated by ultrasonic for about 15 minutes and then is sufficiently stirred to obtain a solution. 5 parts of glycerin, 1 part of kaoline and 5 parts of aqueous polyvinyl alcohol are homogeneously mixed and 1 part of the resulting solution is added. The obtained solution is mixed to give a paste form and the resulting paste is applied to an unwoven fabric. The resulting composition is covered by polyester film to give a cataplasm.

### [Industrial Applicability]

The compounds of the present invention is useful for a medicament for treating the diseases induced by production, secretion, or deposition of amyloid β protein.

## Claims

1. A pharmaceutical composition for suppressing amyloid β production comprising a compound of the formula (I): 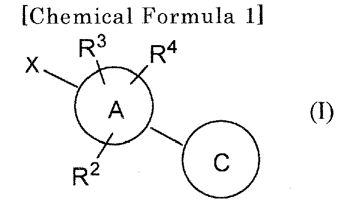 wherein X- is a group of the following formula: 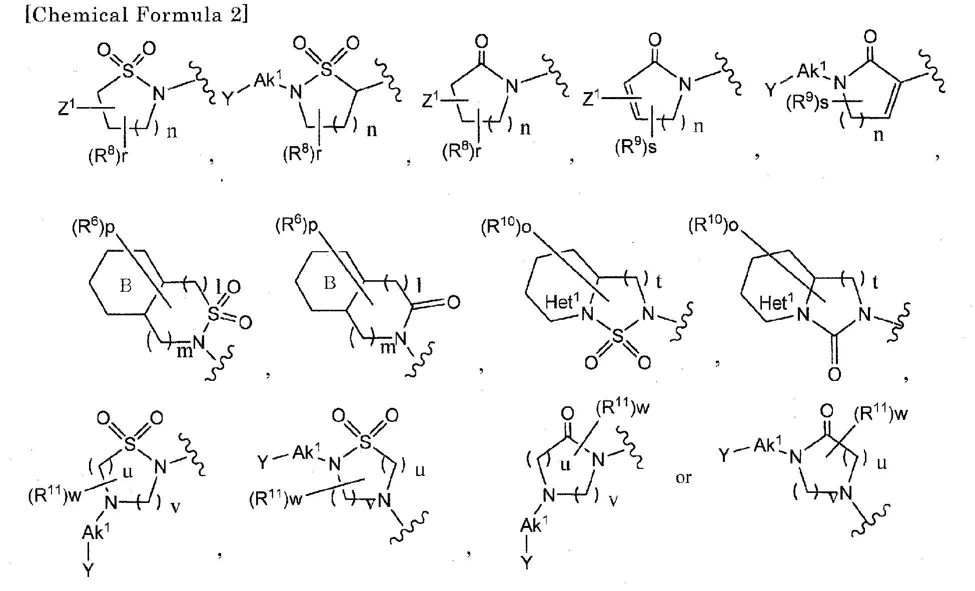 or Y-Ak¹-SO₂N(R⁵)-Ak²-
wherein Y is substituted or unsubstituted phenyl which may be fused to other ring(s), substituted or unsubstituted cycloalkyl, substituted or unsubstituted cycloalkenyl, or substituted or unsubstituted pyridyl;
provided that when X- is Y-Ak¹-SO₂N(R⁵)-Ak²-, then Y is substituted or unsubstituted phenyl which may be fused to other aromatic ring(s) or substituted or unsubstituted pyridyl;
Ak¹ and Ak² are each independently a bond, or substituted or unsubstituted C1-C3 alkylene;
R⁵ is hydrogen, substituted or unsubstituted alkyl or substituted or unsubstituted acyl;
Z¹- is Y-Ak¹-D-Ak²-, Y-Ak¹-C(=O)N(R¹²)-Ak² -, Y-Ak¹-N(R¹²)-C(=O)-Ak²-, Y-Ak¹- SO₂N(R¹²)-Ak²-, Y-Ak¹-N(R¹²)SO₂-Ak²- or Y-Ak¹-C(=O)-Ak²-;
D is a bond, O, S or N(R¹³);
R¹² and R¹³ are each independently hydrogen, substituted or unsubstituted alkyl or substituted or unsubstituted acyl;
R⁶, R⁸, R⁹, R¹⁰ and R¹¹ are each independently halogen, substituted or unsubstituted alkyl, substituted or unsubstituted alkoxy, substituted or unsubstituted amino, cyano, nitro, substituted or unsubstituted phenyl, substituted or unsubstituted phenoxy, substituted or unsubstituted carbocyclyl, or substituted or unsubstituted heterocyclyl; 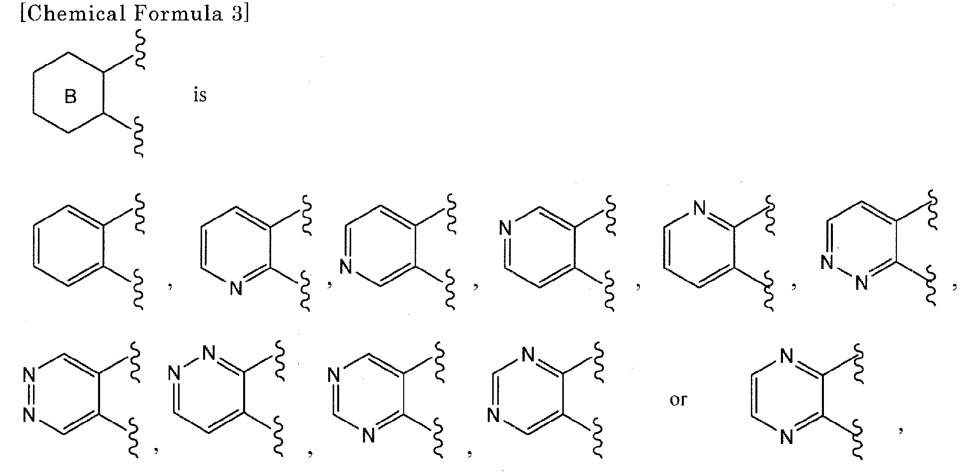 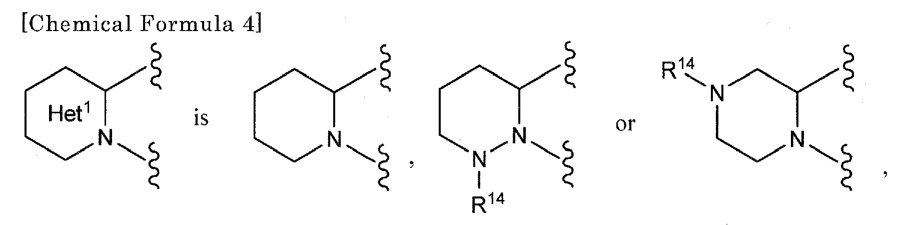
R¹⁴ is hydrogen, substituted or unsubstituted alkyl, substituted or unsubstituted acyl, substituted or unsubstituted alkoxycarbonyl, or substituted or unsubstituted phenyl;
n and t are each independently an integer of 0 to 5;
u and v are each independently an integer of 1 to 5;
m and 1 are each independently an integer of 0 to 4; and
p, r, s, o and w are each independently an integer of 0 to 2;
A is a benzene ring, a pyridine ring or a pyrimidine ring;
R², R³ and R⁴ are each independently hydrogen, halogen, hydroxy, carboxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted carbocyclyloxy, substituted or unsubstituted heterocyclyloxy, substituted or unsubstituted mercapto, substituted or unsubstituted alkoxycarbonyl, substituted or unsubstituted alkenyloxycarbonyl, substituted or unsubstituted alkynyloxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted sulfamoyl, substituted or unsubstituted alkylsulfonyl, substituted or unsubstituted alkenylsulfonyl, substituted or unsubstituted alkynylsulfonyl, nitro, cyano, substituted or unsubstituted carbocyclylsulfonyl, substituted or unsubstituted heterocyclylsulfonyl, substituted or unsubstituted carbocyclyl or substituted or unsubstituted heterocyclyl; and
C is substituted or unsubstituted imidazolyl, substituted or unsubstituted oxazolyl, substituted or unsubstituted isoxazolyl, substituted or unsubstituted thiazolyl or substituted or unsubstituted isothiazolyl;
its pharmaceutically acceptable salt or a solvate thereof.

2. The pharmaceutical composition for suppressing amyloid β production of Claim 1 wherein R² is a group other than hydrogen.

3. The pharmaceutical composition for suppressing amyloid β production of Claim 1 or 2 wherein X- is a group of the following formula: 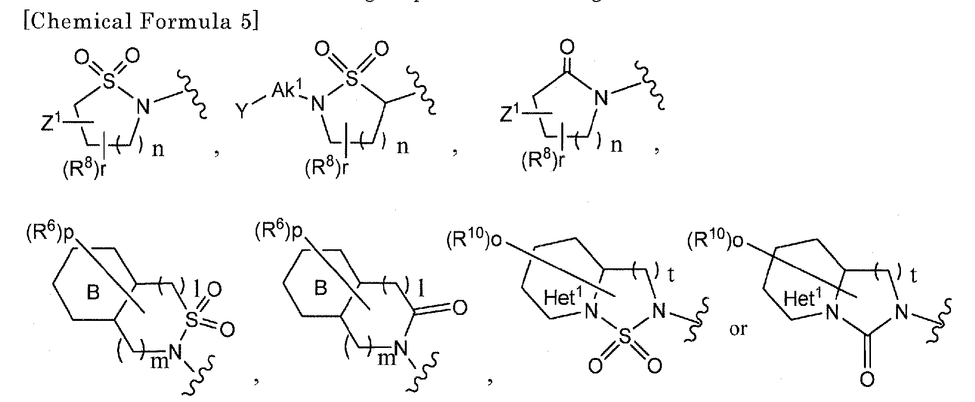 wherein Z¹, Ak¹, R⁶, R⁸, R¹⁰, B, Het¹, l, m, n, o, p, r and t are the same as defined in Claim 1.

4. The pharmaceutical composition for suppressing amyloid β production of any one of Claims 1 to 3 wherein X- is a group of the following formula: 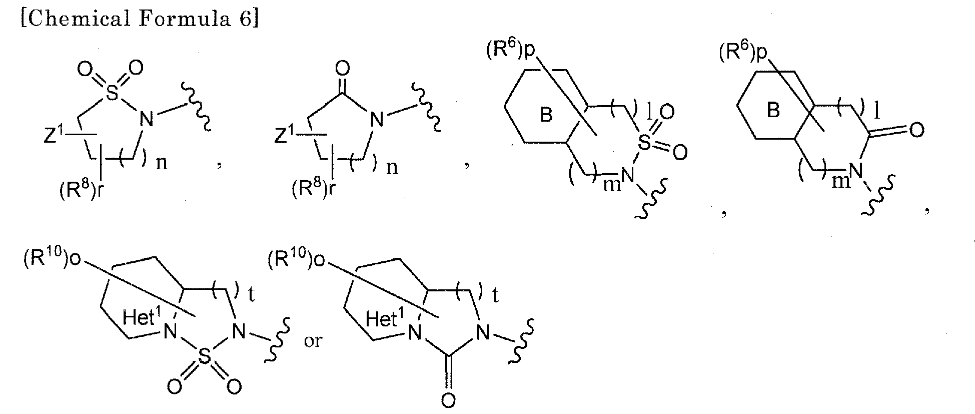 wherein Z¹, R⁶, R⁸, R¹⁰, B, Het¹,o, p and r are the same as defined in Claim 1, n, t, and m are each independently 1 or 2 and 1 is 0.

5. The pharmaceutical composition for suppressing amyloid β production of any one of Claims 1 to 4 wherein X- is a group of the following formula: 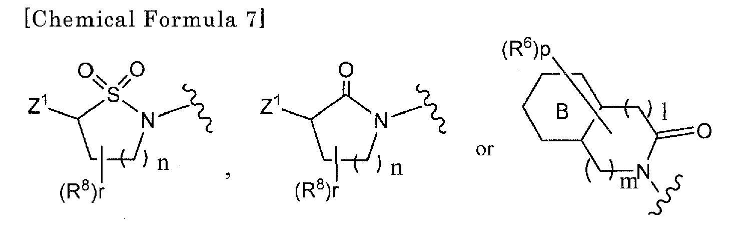 wherein Z¹ is Y-Ak¹-D-Ak²- or Y-Ak¹-C(=O)N(R¹²)-Ak²-,
Y is substituted phenyl or substituted or unsubstituted naphthyl,
Ak¹ is a bond or substituted or unsubstituted C1-C3 alkylene,
D is a bond or N(R¹³),
Ak² is a bond,
R¹² and R¹³ are hydrogen,
R⁶ and R⁸ are each independently halogen, substituted or unsubstituted alkyl, substituted or unsubstituted alkoxy, substituted or unsubstituted amino, cyano, nitro, substituted or unsubstituted phenyl, substituted or unsubstituted phenoxy, substituted or unsubstituted carbocyclyl, or substituted or unsubstituted heterocyclyl, 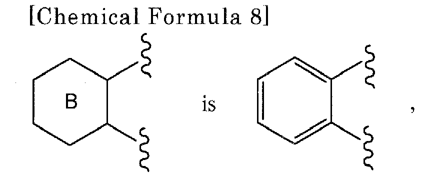 1 is 0,
m and n are each independently 1 or 2,
p and r are each independently an integer of 0 to 2;
A is a benzene ring or a pyridine ring;
R² is halogen, hydroxy, carboxy, substituted or unsubstituted alkyl, substituted or unsubstituted alkenyl, substituted or unsubstituted alkoxy, substituted or unsubstituted carbocyclyloxy, substituted or unsubstituted heterocyclyloxy, substituted or unsubstituted alkoxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted carbocyclyl or substituted or unsubstituted heterocyclyl;
R³ and R⁴ are each independently hydrogen, halogen, hydroxy, carboxy, substituted or unsubstituted alkyl, substituted or unsubstituted alkenyl, substituted or unsubstituted alkoxy, substituted or unsubstituted carbocyclyloxy, substituted or unsubstituted heterocyclyloxy, substituted or unsubstituted alkoxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted carbocyclyl or substituted or unsubstituted heterocyclyl;
C is substituted or unsubstituted imidazolyl, substituted or unsubstituted oxazolyl, substituted or unsubstituted isoxazolyl, substituted or unsubstituted thiazolyl or substituted or unsubstituted isothiazolyl; and
X is in the para position relative to X on the A group.

6. The pharmaceutical composition for suppressing amyloid β production of Claim 1 or 2 wherein X- is Y-Ak¹-SO₂N(R⁵)-Ak²-;
Y is substituted phenyl or substituted or unsubstituted naphthyl;
Ak¹ is a bond or substituted or unsubstituted methylene;
Ak² is a bond;
A is a benzene ring or a pyridine ring;
R² is halogen, hydroxy, carboxy, substituted or unsubstituted alkyl, substituted or unsubstituted alkenyl, substituted or unsubstituted alkoxy, substituted or unsubstituted carbocyclyloxy, substituted or unsubstituted heterocyclyloxy, substituted or unsubstituted alkoxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted carbocyclyl or substituted or unsubstituted heterocyclyl;
R³ and R⁴ are each independently hydrogen, halogen, hydroxy, carboxy, substituted or
unsubstituted alkyl, substituted or unsubstituted alkenyl, substituted or unsubstituted alkoxy, substituted or unsubstituted carbocyclyloxy, substituted or unsubstituted heterocyclyloxy, substituted or unsubstituted alkoxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted carbocyclyl or substituted or unsubstituted heterocyclyl;
C is substituted or unsubstituted imidazolyl, substituted or unsubstituted oxazolyl, substituted or unsubstituted isoxazolyl, substituted or unsubstituted thiazolyl or substituted or unsubstituted isothiazolyl; and
the group of formula Y-Ak¹-SO₂N(R⁵)-Ak²- is in the para position relative to C on the A group.

7. The pharmaceutical composition for suppressing amyloid β production of any one of Claims 1 to 6 wherein C is substituted or unsubstituted imidazol-1-yl.

8. The pharmaceutical composition for suppressing amyloid β production of Claim 7 wherein C is imidazol-1-yl substituted with substituted or unsubstituted alkyl.

9. The pharmaceutical composition for suppressing amyloid β production of any one of Claims 1 to 8 wherein both of Ak¹ and Ak² are a bond.

10. The pharmaceutical composition for suppressing amyloid β production of any one of Claims 1 to 9 wherein A is a benzene ring.

11. The pharmaceutical composition for suppressing amyloid β production of any one of Claims 1 to 10 wherein R² is substituted or unsubstituted alkoxy.

12. The pharmaceutical composition for suppressing amyloid β production of any one of Claims 1 to 4 and 7 to 11 wherein Y is substituted or unsubstituted phenyl, substituted or unsubstituted naphthyl, substituted or unsubstituted pyridyl, substituted or unsubstituted quinolyl or substituted or unsubstituted isoquinolyl.

13. The pharmaceutical composition for suppressing amyloid β production of Claim12 wherein Y is substituted or unsubstituted phenyl, substituted or unsubstituted naphthyl, substituted or unsubstituted pyridyl, substituted or unsubstituted quinolyl or substituted or unsubstituted isoquinolyl, wherein the substituent(s) of each ring is one or more selected from the group of halogen, substituted or unsubstituted alkyl, substituted or unsubstituted alkoxy, substituted or unsubstituted amino, cyano, nitro, substituted or unsubstituted phenyl, substituted or unsubstituted phenoxy and substituted or unsubstituted heterocyclyl.

14. The pharmaceutical composition for suppressing amyloid β production of any one of Claims 1 to 13 which is the pharmaceutical composition for selectively suppressing amyloid β42 production.

15. The pharmaceutical composition for suppressing amyloid β production of any one of Claims 1 to 13 which is the pharmaceutical composition is for modulating Y secretase.

16. A compound of formula (II): 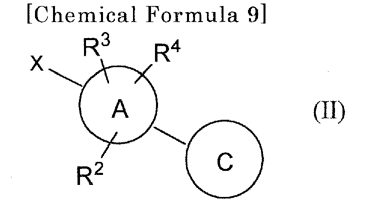 wherein X- is a group of the following formula: 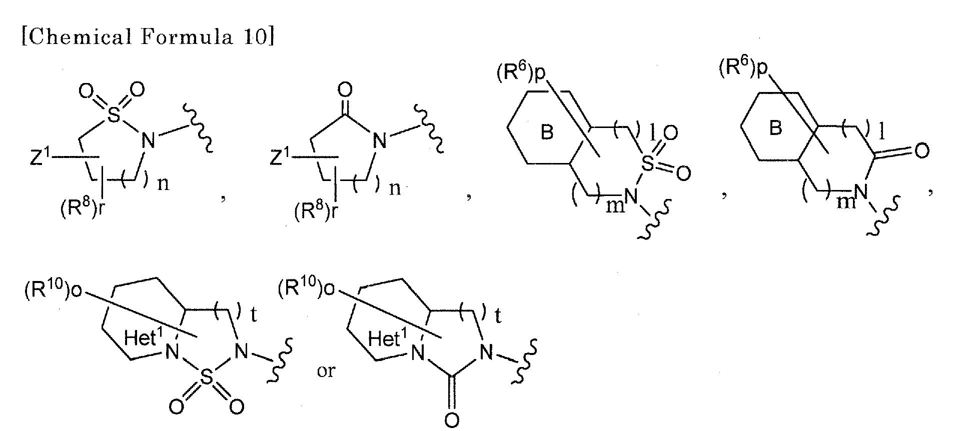
wherein Z¹- is Y-Ak¹-D-Ak²-, Y-Ak¹-C(=O)N(R¹²)-Ak²-, Y-Ak¹-N(R¹²)-C(=O)-Ak²-, YAk¹-SO₂N(R¹²)-Ak²-, Y-Ak¹-N(R¹²)SO₂-Ak²- or Y-Ak¹-C(=O)-Ak²-,
Y is substituted or unsubstituted phenyl which may be fused to other ring(s), substituted or unsubstituted cycloalkyl, substituted or unsubstituted cycloalkenyl, or substituted or unsubstituted pyridyl,
Ak¹ and Ak² are each independently a bond, or substituted or unsubstituted C1-C3 alkylene,
D is a bond, O, S or N(R¹³),
R¹² and R¹³ are each independently hydrogen, substituted or unsubstituted alkyl or substituted or unsubstituted acyl, 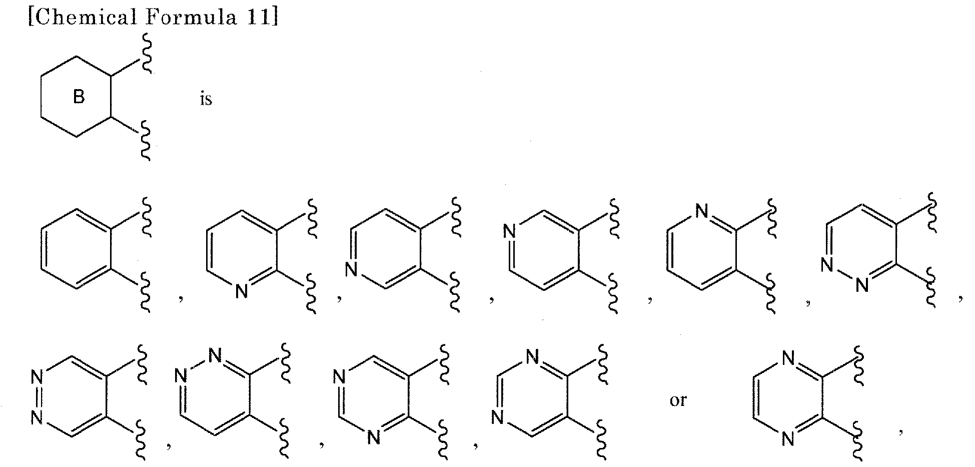 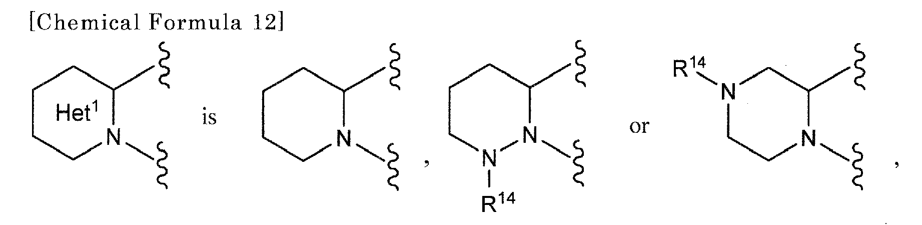
R¹⁴ is hydrogen, substituted or unsubstituted alkyl, substituted or unsubstituted acyl, substituted or unsubstituted alkoxycarbonyl, or substituted or unsubstituted phenyl, R⁶, R⁸ and R¹⁰ are each independently halogen, substituted or unsubstituted alkyl, substituted or unsubstituted alkoxy, substituted or unsubstituted amino, cyano, nitro, substituted or unsubstituted phenyl, substituted or unsubstituted phenoxy, substituted or unsubstituted carbocyclyl, or substituted or unsubstituted heterocyclyl,
p, r, and o are each independently an integer of 0 to 2,
n, t, and m are each independently 1 or 2, and
1 is 0;
A is a benzene ring, a pyridine ring or a pyrimidine ring;
R², R³ and R⁴ are each independently hydrogen, halogen, hydroxy, carboxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted carbocyclyloxy, substituted or unsubstituted heterocyclyloxy, substituted or unsubstituted mercapto, substituted or unsubstituted alkoxycarbonyl, substituted or unsubstituted alkenyloxycarbonyl, substituted or unsubstituted alkynyloxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted sulfamoyl, substituted or unsubstituted alkylsulfonyl, substituted or unsubstituted alkenylsulfonyl, substituted or unsubstituted alkynylsulfonyl, nitro, cyano, substituted or unsubstituted carbocyclylsulfonyl, substituted or unsubstituted heterocyclylsulfonyl, substituted or unsubstituted carbocyclyl or substituted or unsubstituted heterocyclyl; and
C is substituted or unsubstituted imidazolyl, substituted or unsubstituted oxazolyl, substituted or unsubstituted isoxazolyl, substituted or unsubstituted thiazolyl or substituted or unsubstituted isothiazolyl;
provided that the following compound is excluded: 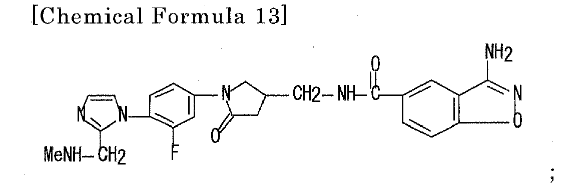 its pharmaceutically acceptable salt or a solvate thereof.

17. The compound of Claim 16 wherein X- is a group of the following formula: 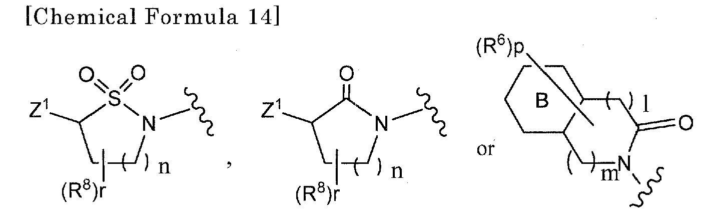 wherein Z¹- is Y-Ak¹-D-Ak²- or Y-Ak¹-C(=O)N(R¹²)-Ak²-,
Y is substituted phenyl, or substituted or unsubstituted naphthyl,
Ak¹ is a bond or substituted or unsubstituted C1-C3 alkylene,
D is a bond or N(R¹³),
Ak² is a bond,
R¹² and R¹³ are each independently hydrogen,
R⁶ and R⁸ are each independently halogen, substituted or unsubstituted alkyl, substituted or unsubstituted alkoxy, substituted or unsubstituted amino, cyano, nitro, substituted or unsubstituted phenyl, substituted or unsubstituted phenoxy, substituted or unsubstituted carbocyclyl, or substituted or unsubstituted heterocyclyl, 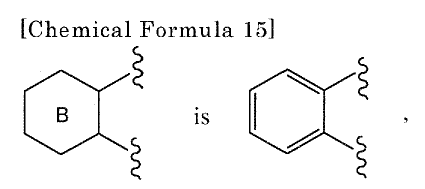 1 is 0,
m and n are each independently 1 or 2,
p and r are each independently an integer of 0 to 2;
A is a benzene ring or a pyridine ring;
R² is halogen, hydroxy, carboxy, substituted or unsubstituted alkyl, substituted or unsubstituted alkenyl, substituted or unsubstituted alkoxy, substituted or unsubstituted carbocyclyloxy, substituted or unsubstituted heterocyclyloxy, substituted or unsubstituted alkoxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted carbocyclyl or substituted or unsubstituted heterocyclyl;
R³ and R⁴ are each independently hydrogen, halogen, hydroxy, carboxy, substituted or unsubstituted alkyl, substituted or unsubstituted alkenyl, substituted or unsubstituted alkoxy, substituted or unsubstituted carbocyclyloxy, substituted or unsubstituted heterocyclyloxy, substituted or unsubstituted alkoxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted carbocyclyl or substituted or unsubstituted heterocyclyl;
C is substituted or unsubstituted imidazolyl, substituted or unsubstituted oxazolyl, substituted or unsubstituted isoxazolyl, substituted or unsubstituted thiazolyl or substituted or unsubstituted isothiazolyl; and
X is in the para position relative to X on the A group;
its pharmaceutically acceptable salt or a solvate thereof.

18. The compound of Claim 16 or 17 wherein C is substituted or unsubstituted imidazolyl, its pharmaceutically acceptable salt or a solvate thereof.

19. The compound of any one of Claims 16 to 18 wherein 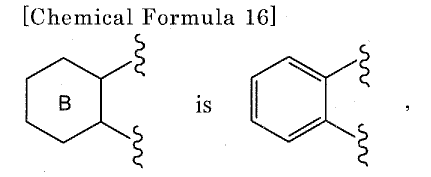 its pharmaceutically acceptable salt or a solvate thereof.

20. The compound of any one of Claims 16 to 19 wherein m and n are each independently 1, its pharmaceutically acceptable salt or a solvate thereof.

21. A compound of the formula (III): 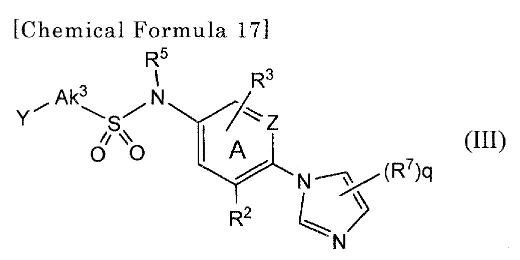 wherein Y is substituted or unsubstituted phenyl which may be fused to other aromatic ring(s), or substituted or unsubstituted pyridyl;
Z is N or CR¹⁵;
R² is halogen, hydroxy, carboxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted carbocyclyloxy, substituted or unsubstituted heterocyclyloxy, substituted or unsubstituted mercapto, substituted or unsubstituted alkoxycarbonyl, substituted or unsubstituted carbocyclyloxycarbonyl, substituted or unsubstituted heterocyclyloxy carbonyl, substituted or unsubstituted acyl, substituted or unsubstituted amino, substituted or unsubstituted carbamoyl, substituted or unsubstituted sulfamoyl, nitro, cyano, substituted or unsubstituted carbocyclyl or substituted or unsubstituted heterocyclyl;
R³ and R¹⁵ are each independently hydrogen, halogen, hydroxy, carboxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted carbocyclyloxy, substituted or unsubstituted heterocyclyloxy, substituted or unsubstituted mercapto, substituted or unsubstituted alkoxycarbonyl, substituted or unsubstituted carbocyclyloxycarbonyl, substituted or unsubstituted heterocyclyloxycarbonyl, substituted or unsubstituted acyl, substituted or unsubstituted amino, substituted or unsubstituted carbamoyl, substituted or unsubstituted sulfamoyl, nitro, cyano, substituted or unsubstituted carbocyclyl or substituted or unsubstituted heterocyclyl;
R⁵ is hydrogen, substituted or unsubstituted alkyl or substituted or unsubstituted acyl; R⁷ is halogen, hydroxy, carboxy, substituted or unsubstituted alkyl, substituted or unsubstituted alkenyl, substituted or unsubstituted alkoxy, substituted or unsubstituted mercapto, substituted or unsubstituted alkoxycarbonyl, substituted or unsubstituted acyl, substituted or unsubstituted amino, substituted or unsubstituted carbamoyl or substituted or unsubstituted sulfamoyl;
Ak³ is a. bond or substituted or unsubstituted methylene; and
q is an integer of 0 to 2;
provide that the following compounds are excluded: 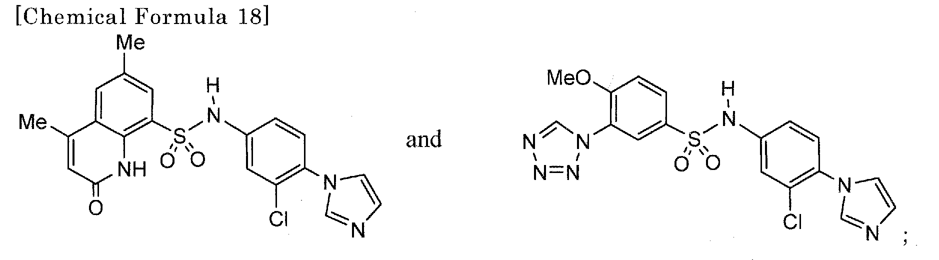 its pharmaceutically acceptable salt or a solvate thereof.

22. The compound of Claim 21 wherein Z is CR¹⁵, R³ and R¹⁵ are each independently hydrogen, halogen, hydroxy, carboxy, substituted or unsubstituted alkyl, substituted or unsubstituted alkenyl, substituted or unsubstituted alkoxy, substituted or unsubstituted alkoxycarbonyl, substituted or unsubstituted acyl, substituted or unsubstituted amino or substituted or unsubstituted carbamoyl, its pharmaceutically acceptable salt or a solvate thereof.

23. The compound of Claim 21 or 22 wherein Ak³ is a bond and R⁵ is hydrogen, its pharmaceutically acceptable salt or a solvate thereof.

24. The compound of any one of Claims 21 to 23 wherein q is land R⁷ is substituted or unsubstituted alkyl, its pharmaceutically acceptable salt or a solvate thereof.

25. The compound of any one of Claims 16 to 24 wherein A is a benzene ring, its pharmaceutically acceptable salt or a solvate thereof.

26. The compound of any one of Claims 16 to 25 wherein Y is substituted or unsubstituted phenyl, substituted or unsubstituted naphthyl, substituted or unsubstituted quinolyl or substituted or unsubstituted isoquinolyl, wherein the substituent(s) of each ring is one or more selected from the group of halogen, substituted or unsubstituted alkyl, substituted or unsubstituted alkoxy, substituted or unsubstituted amino, cyano, nitro, substituted or unsubstituted phenyl, substituted or unsubstituted phenoxy and substituted or unsubstituted heterocyclyl, its pharmaceutically acceptable salt or a solvate thereof.

27. The compound of any one of Claims 16 to 26 wherein R² is substituted or unsubstituted alkoxy, its pharmaceutically acceptable salt or a solvate thereof.

28. A pharmaceutical composition comprising the compound of any one of Claims 16 to 27, its pharmaceutically acceptable salt or a solvate thereof.

29. A pharmaceutical composition for suppressing amyloid β production comprising the compound of any one of Claims 16 to 27, its pharmaceutically acceptable salt or a solvate thereof.

30. A pharmaceutical composition for selectively suppressing amyloid β42 production comprising the compound of any one of Claims 16 to 27, its pharmaceutically acceptable salt or a solvate thereof.

31. A pharmaceutical composition for modulating γ secretase comprising the compound of any one of Claims 16 to 27, its pharmaceutically acceptable salt or a solvate thereof.

32. A method for suppressing amyloid β production comprising administering the compound of any one of Claims 16 to 27, its pharmaceutically acceptable salt or a solvate thereof.

33. A method for selectively suppressing a myloid β42 production comprising administering the compound of any one of Claims 16 to 27, its pharmaceutically acceptable salt or a solvate thereof.

34. A method for modulating γ secretase comprising administering the compound of any one of Claims 16 to 27, its pharmaceutically acceptable salt or a solvate thereof.

35. A compound of any one of Claims 16 to 27, its pharmaceutically acceptable salt or a solvate thereof for use in suppressing amyloid β production.

36. A compound of any one of Claims 16 to 27, its pharmaceutically acceptable salt or a solvate thereof for use in selectively suppressing amyloid 642 production.

37. A compound for any one of Claims 16 to 27, its pharmaceutically acceptable salt or a solvate thereof for use in modulating Y secretase.
